# EUROPEAN PATENT SPECIFICATION

(11) **EP 1 169 312 B1**
(45) Date of publication and mention of the grant of the patent: **06.10.2004**
(21) Application number: 00918816.0
(22) Date of filing: 20.03.2000
(51) Int. Cl.: C07D 277/46, A61K 31/426, C07D 277/56, C07D 213/75, A61K 31/42, A61P 3/10

(54) **GLUCOKINASE ACTIVATORS**
GLUKOKINASE AKTIVATOREN
ACTIVATEURS DE GLUCOKINASE

(30) Priority: 29.03.1999 US 126707 P; 17.11.1999 US 165948 P; 17.11.1999 US 165944 P
(43) Date of publication of application: 09.01.2002
(73) Proprietor: F. HOFFMANN-LA ROCHE AG, 4070 Basel (CH)
(72) Inventor: BIZZARRO, Fred, Thomas, Colonia, NJ 07067 (US); CORBETT, Wendy, Lea, Randolph, NJ 07869-3754 (US); FOCELLA, Antonino, Wayne, NJ 07470 (US); GRIPPO, Joseph, Francis, Stirling, NJ 07980 (US); HAYNES, Nancy-Ellen, Cranford, NJ 07016 (US); HOLLAND, George, William, North Caldwell, NJ 07006 (US); KESTER, Robert, Francis, West Orange, NJ 07052 (US); MAHANEY, Paige, E., Montclair, NJ 07042 (US); SARABU, Ramakanth, Towaco, NJ 07082 (US)
(74) Representative: Witte, Hubert, Dr.
(86) International application number: PCT/EP2000/002450
(87) International publication number: WO 2000/058293

(56) References cited:
- DE-C- 249 241
- US-A- 3 776 917
- SPIELMAN M. A. ET AL.: "Anticonvulsant Drugs. II. Some Acylureas" JOURNAL OF AMERICAN CHEMICAL SOCIETY, vol. 70, 1948, pages 4189-4191, XP000940775

## Description

Glucokinase (GK) is one of four hexokinases that are found in mammals [Colowick, S.P., in *The Enzymes*, Vol. 9 (P. Boyer, ed.) Academic Press, New York, NY, pages 1-48, 1973]. The hexokinases catalyze the first step in the metabolism of glucose, i.e., the conversion of glucose to glucose-6-phosphate. Glucokinase has a limited cellular distribution, being found principally in pancreatic β-cells and liver parenchymal cells. In addition, GK is a rate-controlling enzyme for glucose metabolism in these two cell types that are known to play critical roles in whole-body glucose homeostasis [Chipkin, S.R., Kelly, K.L., and Ruderman, N.B. in *Joslin's Diabetes* (C.R. Khan and G.C. Wier, eds.), Lea and Febiger, Philadelphia, PA, pages 97-115, 1994]. The concentration of glucose at which GK demonstrates half-maximal activity is approximately 8 mM. The other three hexokinases are saturated with glucose at much lower concentrations (<1 mM). Therefore, the flux of glucose through the GK pathway rises as the concentration of glucose in the blood increases from fasting (5 mM) to postprandial (=10-15 mM) levels following a carbohydrate-containing meal [Printz, R.G., Magnuson, M.A., and Granner, D.K. in *Ann. Rev. Nutrition* Vol. 13 (R.E. Olson, D.M. Bier, and D.B. McCormick, eds.), Annual Review, Inc., Palo Alto, CA, pages 463-496, 1993]. These findings contributed over a decade ago to the hypothesis that GK functions as a glucose sensor in β-cells and hepatocytes (Meglasson, M.D. and Matschinsky, F.M. *Amer*. *J*. *Physiol*. **246**, E1-E13, 1984). In recent years, studies in transgenic animals have confirmed that GK does indeed play a critical role in whole-body glucose homeostasis. Animals that do not express GK die within days of birth with severe diabetes while animals overexpressing GK have improved glucose tolerance (Grupe, A., Hultgren, B., Ryan, A. et al., *Cell* **83**, 69-78, 1995; Ferrie, T., Riu, E., Bosch, F. et al., *FASEB J.,* **1**0, 1213-1218, 1996). An increase in glucose exposure is coupled through GK in β-cells to increased insulin secretion and in hepatocytes to increased glycogen deposition and perhaps decreased glucose production.

The finding that type II maturity-onset diabetes of the young (MODY-2) is caused by loss of function mutations in the GK gene suggests that GK also functions as a glucose sensor in humans (Liang, Y., Kesavan, P., Wang, L. et al., *Biochem. J.* **309**, 167-173, 1995). Additional evidence supporting an important role for GK in the regulation of glucose metabolism in humans was provided by the identification of patients that express a mutant form of GK with increased enzymatic activity. These patients exhibit a fasting hypoglycemia associated with an inappropriately elevated level of plasma insulin (Glaser, B., Kesavan, P., Heyman, M. et al., *New England J. Med.* **338**, 226-230, 1998). While mutations of the GK gene are not found in the majority of patients with type II diabetes, compounds that activate GK and, thereby, increase the sensitivity of the GK sensor system will still be useful in the treatment of the hyperglycemia characteristic of all type II diabetes. Glucokinase activators will increase the flux of glucose metabolism in β-cells and hepatocytes, which will be coupled to increased insulin secretion. Such agents would be useful for treating type II diabetes.

This invention provides a compound, comprising an amide of the formula I: 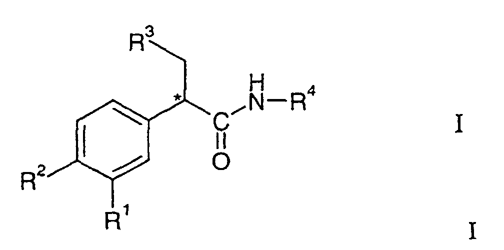 wherein
- R¹ and R²: are independently hydrogen, halo, amino, hydroxyamino, nitro, cyano, sulfonamido, lower alkyl,-OR⁵, -C(O)OR⁵, perfluoro-lower alkyl, lower alkyl thio, perfluoro-lower alkyl thio, lower alkyl sulfonyl, perfluoro-lower alkyl sulfonyl or lower alkyl sulfinyl;
- R³: is cycloalkyl having from 3 to 7 carbon atoms ;
- R⁴: is -C(O)NHR⁴⁰; or an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, - (CH₂)ₙ-C(O)OR⁷, -(CH₂)ₙ-C(O)NHR⁶, -C(O)-C(O)OR⁸, -(CH₂)ₙ-NHR⁶;
- R⁴⁰: is hydrogen, lower alkyl, lower alkenyl, hydroxy lower alkyl, halo lower alkyl, -(CH₂)ₙ-C(O)OR⁵ or -C(O)-(CH₂)ₙ-C(O)OR⁶;
- R⁵: is hydrogen, lower alkyl or perfluoro-lower alkyl;
- R⁶, R⁷ and R⁸: are independently hydrogen or lower alkyl; and
- n: is 0, 1, 2, 3 or 4;
or a pharmaceutically acceptable salt thereof.

In the compound of formula I, the * indicates the asymmetric carbon atom in this compound. The compound of formula I may be present either as a racemate or in the "R" configuration at the asymmetric carbon shown. The "R" enantiomers are preferred.

The compounds of formula I have been found to activate glucokinase *in vitro.* Glucokinase activators are useful for increasing insulin secretion in the treatment of type II diabetes.

The present invention also relates to a pharmaceutical composition comprising a compound of formula I and a pharmaceutically acceptable carrier and/or adjuvant. Furthermore the present invention relates to the use of such compounds for the preparation of medicaments for the treatment of type II diabetes. The present invention also relates to processes for the preparation of the compounds of formula I. In addition, the present invention relates to a method for the therapeutic treatment of type II diabetes, which method comprises administering a compound of formula I to a human being or an animal.

As used throughout this application, the term "halogen" and the term "halo", unless otherwise stated, designate all four halogens, i.e. fluorine, chlorine, bromine and iodine. A preferred halogen is chlorine.

As used herein, the term "lower alkyl" includes both straight chain and branched chain alkyl groups having from 1 to 7 carbon atoms, such as methyl, ethyl, propyl, isopropyl, preferably methyl and ethyl. With regard to R³, isopropyl and n-propyl are preferred. "Halo lower alkyl" as used herein designates a lower alkyl group wherein one of the hydrogens is replaced by a halogen as defined above, which replacement can be at any site on the lower alkyl, including the end. A preferred halo lower alkyl group is chloroethyl. Similarly, "hydroxy lower alkyl" designates a lower alkyl group where one of the hydrogens is replaced by a hydroxy, at any site including the end. Preferred hydroxy lower alkyl groups include ethanol, isopropanol, and n-propanol. As used herein, "perfluoro-lower alkyl" means any lower alkyl group wherein all of the hydrogens of the lower alkyl group are substituted or replaced by fluoro. Among the preferred perfluoro-lower alkyl groups are trifluoromethyl, pentafluoroethyl, heptafluoropropyl, etc.

As used herein, "lower alkyl thio" means a lower alkyl group as defined above where a thio group is bound to the rest of the molecule. Similarly "perfluoro-lower alkyl thio" means a perfluoro-lower alkyl group as defined above where a thio group is bound to the rest of the molecule.

As used herein, "lower alkyl sulfonyl" means a lower alkyl group as defined above where a sulfonyl group is bound to the rest of the molecule. Similarly "perfluoro-lower alkyl sulfonyl" means a perfluoro-lower alkyl group as defined above where a sulfonyl group is bound to the rest of the molecule.

As used herein, "lower alkyl sulfinyl" means a lower alkyl group as defined above where a sulfinyl group is bound to the rest of the molecule.

As used herein, "hydroxyamino" designates an amino group where one of the hydrogens is replaced by a hydroxy.

As used herein, "cycloalkyl" means a saturated hydrocarbon ring having from 3 to 10 carbon atoms, preferably from 3 to 7 carbon atoms, such as cyclopropyl, cyclobutyl, cyclopentyl, cyclohexyl, cycloheptyl, etc. A preferred cycloalkyl is cyclopentyl.

As used herein, the term "lower alkenyl" denotes an alkylene group having from 2 to 6 carbon atoms with a double bond located between any two adjacent carbons of the group. Preferred lower alkenyl groups are allyl and crotyl.

As used herein, the term "lower alkoxy" includes both straight chain and branched chain alkoxy groups having from 1 to 7 carbon atoms, such as methoxy, ethoxy, propoxy, isopropoxy, preferably methoxy and ethoxy.

As used herein, the term "aryl" signifies aryl mononuclear aromatic hydrocarbon groups such as phenyl, tolyl, etc. which can be unsubstituted or substituted in one or more positions with halogen, nitro, lower alkyl, or lower alkoxy substituents and polynuclear aryl groups, such as naphthyl, anthryl, and phenanthryl, which can be unsubstituted or substituted with one or more of the aforementioned groups. Preferred aryl groups are the substituted and unsubstituted mononuclear aryl groups, particularly phenyl. The term "arylalkyl" denotes an alkyl group, preferably lower alkyl, in which one of the hydrogen atoms is replaced by an aryl group. Examples of arylalkyl groups are benzyl, 2-phenylethyl, 3-phenylpropyl, 4-chlorobenzyl, 4-methoxybenzyland the like.

As used herein, the term "lower alkanoic acid" denotes lower alkanoic acids containing from 2 to 7 carbon atoms such as propionic acid, acetic acid and the like. The term "lower alkanoyl" denotes monovalent alkanoyl groups having from 2 to 7 carbon atoms such as propionoyl, acetyl and the like. The term "aroic acids" denotes aryl alkanoic acids where aryl is as defined above and alkanoic contains from 1 to 6 carbon atoms. The term "aroyl" denotes aroic acids wherein aryl is as defined hereinbefore, with the hydrogen group of the COOH moiety removed. Among the preferred aroyl groups is benzoyl.

The heteroaromatic ring in R⁴ can be an unsubstituted or mono-substituted five- or six-membered heteroaromatic ring having from 1 to 3 heteroatoms selected from the group consisting of oxygen, nitrogen or sulfur and connected by a ring carbon to the amine of the amide group shown. The heteroaromatic ring contains a first nitrogen heteroatom adjacent to the connecting ring carbon atom and if present, the other heteroatoms can be sulfur, oxygen or nitrogen. Such heteroaromatic rings include, for example, pyrazinyl, pyridazinyl, isoxazolyl, isothiazolyl, and pyrazolyl. Among the preferred heteroaromatic rings are included pyridinyl, pyrimidinyl, thiazolyl, oxazolyl and imidazolyl. These heteroaromatic rings which constitute R⁴ are connected *via* a ring carbon atom to the amide group to form the amides of formula I. The ring carbon atom of the heteroaromatic ring which is connected *via* the amide linkage to form the compound of formula I cannot contain any substituent. When R⁴ is an unsubstituted or mono-substituted five-membered heteroaromatic ring, the preferred rings are those which contain a nitrogen heteroatom adjacent to the connecting ring carbon and a second heteroatom adjacent to the connecting ring carbon or adjacent to said first heteroatom. The preferred five-membered heteroaromatic rings contain 2 or 3 heteroatom with thiazolyl, imidazolyl, oxazolyl and thiadiazolyl being especially preferred. When the heteroaromatic ring is a six-membered heteroaromatic, the ring is connected by a ring carbon to the amine group shown, with one nitrogen heteroatom being adjacent to the connecting ring carbon atom. The preferred six-membered heteroaromatic rings include, for example, pyridinyl, pyrimidinyl, pyrazinyl, pyridazinyl, and triazinyl.

During the course of the reaction the various functional groups such as the free carboxylic acid or hydroxy groups will be protected *via* conventional hydrolyzable ester or ether protecting groups. As used herein the term "hydrolyzable ester or ether protecting groups" designates any ester or ether conventionally used for protecting carboxylic acids or alcohols which can be hydrolyzed to yield the respective hydroxyl or carboxyl group. Exemplary ester groups useful for those purposes are those in which the acyl moieties are derived from a lower alkanoic, aryl lower alkanoic, or lower alkane dicarboxcyclic acid. Among the activated acids which can be utilized to form such groups are acid anhydrides, acid halides, preferably acid chlorides or acid bromides derived from aryl or lower alkanoic acids. Example of anhydrides are anhydrides derived from monocarboxylic acid such as acetic anhydride, benzoic acid anhydride, and lower alkane dicarboxcyclic acid anhydrides, e.g. succinic anhydride as well as chloro formates e.g. trichloro, ethylchloro formate being preferred. A suitable ether protecting group for alcohols are, for example, the tetrahydropyranyl ethers such as 4-methoxy-5,6-dihydroxy-2H-pyranyl ethers. Others are aroylmethylethers such as benzyl, benzhydryl or trityl ethers or α-lower alkoxy lower alkyl ethers, for example, methoxymethyl or allylic ethers or alkyl silylethers such as trimethylsilylether.

The term "amino protecting group" designates any conventional amino protecting group which can be cleaved to yield the free amino group. The preferred protecting groups are the conventional amino protecting groups utilized in peptide synthesis. Especially preferred are those amino protecting groups which are cleavable under mildly acidic conditions from about pH 2.0 to 3. Particularly preferred amino protecting groups are t-butoxycarbonyl (BOC), carbobenzyloxy (CBZ) and 9-flurorenylmethoxycarbonyl (FMOC).

The term "pharmaceutically acceptable salts" as used herein include any salt with both inorganic or organic pharmaceutically acceptable acids such as hydrochloric acid, hydrobromic acid, nitric acid, sulfuric acid, phosphoric acid, citric acid, formic acid, maleic acid, acetic acid, succinic acid, tartaric acid, methanesulfonic acid, para-toluene sulfonic acid and the like. The term "pharmaceutically acceptable salts" also includes any pharmaceutically acceptable base salt such as amine salts, trialkyl amine salts and the like. Such salts can be formed quite readily by those skilled in the art using standard techniques.

In one preferable embodiment, the present invention relates to a compound comprising an amide of the above formula I, wherein
- R¹ and R²: are independently hydrogen, halo, amino, hydroxyamino, cyano, nitro, lower alkyl, -OR⁵, -C(O)OR⁵, perfluoro-lower alkyl, lower alkyl thio, perfluoro-lower alkyl thio, lower alkyl sulfonyl, perfluoro-lower alkyl sulfonyl, lower alkyl sulfinyl, or sulfonamido;
- R³: is cycloalkyl having from 3 to 7 carbon atoms;
- R⁴: is an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, -(CH₂)ₙ-C(O)OR⁷, - (CH₂)ₙ-C(O)NHR⁶, -C(O)-C(O)OR⁸ or -(CH₂)ₙ-NHR⁶;
- n: is 0, 1, 2, 3 or 4;
- R⁵: is hydrogen, lower alkyl, or perfluoro-lower alkyl; and
- R⁶, R⁷ and R⁸: are independently hydrogen or lower alkyl;
or a pharmaceutically acceptable salt thereof.

In another preferable emodiment the present invention relates to a compound comprising an amide of the above formula I, wherein
- R¹ and R²: are independently hydrogen, halo, amino, nitro, cyano, sulfonamido, lower alkyl, perfluoro-lower alkyl, lower alkyl thio, perfluoro-lower alkyl thio, lower alkyl sulfonyl, or perfluoro-lower alkyl sulfonyl;
- R³: is cycloalkyl having from 3 to 7 carbon atoms;
- R⁴: is -C(O)NHR⁴⁰;
- R⁴⁰: is hydrogen, lower alkyl, lower alkenyl, hydroxy lower alkyl, halo lower alkyl, - (CH₂)ₙ-C(O)OR⁵ or -C(O)-(CH₂)ₙ-C(O)OR⁶;
- R⁵ and R⁶: are hydrogen or lower alkyl; and
- n: is 0, 1,2,3 or 4;
or a pharmaceutically acceptable salt thereof.

Preferable heteroaromatic residues in R⁴ are unsubstituted or mono-substituted five- or six-membered heteroaromatic rings selected from the group consisting of thiazolyl, pyridinyl, imidazolyl, isoxazolyl, oxazolyl, pyridazinyl, pyrimidinyl or thiadiazolyl. Especially preferred are unsubstituted thiazolyl, unsubstituted pyridinyl or pyridinyl substituted by halogen, lower alkyl, hydroxy lower alkyl or -C(O)OR⁵, wherein R⁵ is lower alkyl.

Preferable residues R⁴⁰ in accordance with the present invention are lower alkyl or lower alkenyl.

Preferable residues R¹ in accordance with the present invention are hydrogen, halo, nitro and cyano, more preferable are hydrogen or halo.

Preferable residues R² in accordance with the present invention are hydrogen, lower alkyl sulfonyl, perfluoro-lower alkyl, perfluoro-lower alkyl sulfonyl, halo or -OR⁵ wherein R⁵ is perfluoro-lower alkyl; more preferable are halo or lower alkyl sulfonyl.

Preferable residues R³ in accordance with the present invention are cyclopentyl, cyclohexyl or cycloheptyl, more preferable is cyclopentyl.

In the compounds described below, unless otherwise indicated, R⁴ is a group -C(O)NHR⁴⁰, wherein R⁴⁰ is as defined above.

In certain of such compounds, R⁴⁰ of the amide is hydrogen, lower alkyl, or lower alkenyl. Such amides are preferred when R³ is cyclopentyl, especially when the amide is in the "R" configuration at the asymmetric carbon shown.

In certain amides of the above compound, R¹ and R² of the amide are hydrogen. Such an amide is 1-(3-cyclopentyl-2-phenyl-propionyl)-3-methyl-urea. In other of the above compounds, one of R¹ and R² is hydrogen and the other is cyano or halo. Examples of such amides are:
1-[2-(3-chloro-phenyl)-3 cyclopentyl-propionyl]-3-methyl-urea;
1-[2-(4-chloro-phenyl)-3-cyclopentyl-propionyl]-3-methyl-urea;
1-[2-(4-cyano-phenyl)-3-cyclopentyl-propionyl]-3-methyl-urea;
1-[2-(4-bromo-phenyl)-3-cyclopentyl-propionyl]-3-methyl-urea.

In other amides of the above compound, R¹ and R² of the amide are each independently halo (preferably chloro). Examples of such amides are:
[3-cyclopentyl-2-(3,4-dichloro-phenyl)-propionyl]-urea;
1-[3-cyclopentyl-2-(3,4-dichloro-phenyl)-propionyl]-3-methyl-urea;
1-[3-cyclopentyl-2(R)-(3,4-dichloro-phenyl)-propionyl]-3-ethyl-urea;
1-allyl-3-[3-cyclopentyl-2-(3,4-dichloro-phenyl)-proprionyl]-urea;
1-allyl-3-[3-cyclopentyl-2(R)-(3, 4-dichloro-phenyl)-proprionyl]-urea;
1-[3-cyclopentyl-2-(3,4-dichloro-phenyl)-propionyl]-3-ethyl-urea;
1-[3-cyclopentyl-2(R)-(3,4-dichloro-phenyl)-propionyl]-3-methyl-urea;
1-[3-cyclopentyl-2-(3,4-dichloro-phenyl)-propionyl]-3-isopropyl-urea;
1-[3-cyclopentyl-2-(3,4-dichloro-phenyl)-propionyl]-3-propyl-urea;
1-[3-cyclopentyl-2-(3,4-difluoro-phenyl)-propionyl]-3-methyl-urea.

In yet other amides of the above compound, one of R¹and R² of the amide is hydrogen or halo and the other is nitro. Examples of such amides are:
1-[2-(4-chloro-3-nitro-phenyl)-3-cyclopentyl-proprionyl]-3-methyl-urea;
1-[3-cyclopentyl-2-(4-nitro-phenyl)-propionyl]-3-methyl-urea.

In further amides of the above compound, one of R¹ and R² is hydrogen, lower alkyl thio or perfluoro-lower alkyl thio and the other is lower alkyl thio or perfluoro-lower alkyl thio. Examples of such amides are:
1-[3-cyclopentyl-2-(4-trifluoromethylsulfanyl-phenyl)-propionyl]-3-methyl urea;
1-[3-cyclopentyl-2-(4-methylsulfanyl-phenyl)-propionyl]-3-methyl urea.

In yet further amides of the above compound, one of R¹ and R² is hydrogen or perfluoro-lower alkyl sulfonyl and the other is perfluoro-lower alkyl sulfonyl. Examples of such amides are:
1-[3-cyclopentyl-2-(4-trifluoromethanesulfonyl-phenyl)-propionyl]-3-methyl urea;
1-[3-cyclopentyl-2-(3-trifluoromethanesulfonyl-phenyl)-propionyl]-3-methyl urea.

In certain amides of the above compound, at least one of R¹ and R² is lower alkyl sulfonyl. Preferably one of R¹ and R² is hydrogen or lower alkyl sulfonyl and the other is lower alkyl sulfonyl, and more preferably R² is lower alkyl sulfonyl. Examples of such amides are:
1-[3-cyclopentyl-2-(4-methanesulfonyl-phenyl)-propionyl]-3-methyl urea;
1-{2-[4-(butane-1-sulfonyl)-phenyl]-3-cyclopentyl-proprionyl}-3-methyl-urea;
1-[3-cyclopentyl-2-(4-ethanesulfonyl-phenyl)-proprionyl]-3-methyl-urea;
1-[2-(3,4-bis-methanesulfonyl-phenyl)-3-cyclopentyl-proprionyl]-3-methyl-urea.

In other amides of the above compound, at least one of R¹ and R² is lower alkyl sulfonyl, one of R¹ and R² is cyano or halo and the other, preferably R², is lower alkyl sulfonyl. Examples of such amides are:
1-[2-(3-bromo-4-methanesulfonyl-phenyl)-3-cyclopentyl-proprionyl]-3-methyl-urea;
1-[3-cyclopentyl-2-(3-fluoro-4-methanesulfonyl-phenyl)-proprionyl]-3-methyl-urea;
1-[2-(3-chloro-4-methanesulfonyl-phenyl)-3-cyclopentyl-proprionyl]-3-methyl-urea;
1-[2(R)-(3-chloro-4-methanesulfonyl-phenyl)-3-cyclopentyl-proprionyl]-3-methyl-urea;
1-[2-(3-chloro-4-methanesulfonyl-phenyl)-3-cyclopentyl-proprionyl]-3-ethyl-urea;
1-[2-(3-cyano-4-methanesulfonyl-phenyl)-3-cyclopentyl-proprionyl]-3-methyl-urea.

In other amides of the above compound, at least one of R¹ and R² is lower alkyl sulfonyl, one of R¹ and R² is perfluoro-lower alkyl and the other, preferably R², is lower alkyl sulfonyl. An Example of such an amide is 1-[3-cyclopentyl-2-(4-methanesulfonyl-3-trifluoromethyl-phenyl)-proprionyl]-3-methyl-urea.

In further amides of the above compound, at least one of R¹ and R² is perfluoro-lower alkyl and the other is halo. Examples of such amides are:
1-[3-cyclopentyl-2-(4-fluoro-3-trifluoromethyl-phenyl)-propionyl]-3-methyl urea;
1-[3-cyclopentyl-2-(3-fluoro-4-trifluoromethyl-phenyl)-propionyl]-3-methyl urea.

In yet further amides of the above compound, at least one of R¹ and R² is lower alkyl sulfonyl, one of R¹ and R² is nitro and the other is lower alkyl sulfonyl. An example of such an amide is 1-[3-cyclopentyl-2-(4-methanesulfonyl-3-nitrophenyl)-propionyl]-3-methyl-urea.

In the compounds of this invention described above, R⁴⁰ of the amide is hydrogen, lower alkyl, or lower alkenyl and R³ is cyclopentyl. In the compounds described below, R⁴⁰ is the same but R³ is not cyclopentyl.

In certain such compounds, one of R¹ and R² is halo or hydrogen and the other is hydrogen. An example of such an amide is [2-(4-chloro-phenyl)-4-methyl-pentanoyl]-urea. In particular R¹ and R² may each be chlorine. Examples of such amides are:
[3-cyclopropyl-2-(3,4-dichloro-phenyl)-propionyl]-urea;
[3-cyclobucyl-2-(3,4-dichloro-phenyl)-propionyl]-urea;
R-[2-(3,4-dichloro-phenyl)-4-methyl-pentanoyl]-urea;
1-[2-(3,4-dichloro-phenyl)-4-methyl-pentanoyl]-3-methyl-urea;
1-[2-(3,4-dichloro-phenyl)-hexanoyl]-3-methyl-urea.

In other such compounds, R⁴⁰ is as described above and R³ is cyclohexyl. In some such amides, one of R¹ and R² is halo or hydrogen and the other is halo. Examples of such amides are:
3-[cyclohexyl-2-(3,4-dichloro-phenyl)-propionyl]-urea;
[3-cyclohexyl-2-(3,4-dichloro-phenyl)-propionyl]-3-methyl-urea.

In other such compounds, R⁴⁰ is as described above and R³ is cycloheptyl. In some such amides, one of R¹ and R² is halo or hydrogen and the other is halo. An example of such an amide is [3-cycloheptyl-2-(3,4-dichloro-phenyl)-propionyl]-urea.

In certain compounds of this invention, R⁴⁰ is -(CH₂)ₙ-C(O)OR⁵ or -C(O)-(CH₂)ₙ-C(O)OR⁶. In some such compounds, R³ of the amide is cyclopentyl. Preferably R¹ and R² are independently halo. Examples of the above amides are: 3-{3-[3-cyclopentyl-2-(3,4-dichloro-phenyl)-propionyl]-ureido}-propionic acid ethyl ester;
{3-[3-cyclopentyl-2-(3,4-dichloro-phenyl)-propionyl]-ureido}-acetic acid ethyl ester;
{3-[3-cyclopentyl-2-(3,4-dichloro-phenyl)-propionyl]-ureido}-acetic acid methyl ester;
3-{3-[3-cyclopentyl-2-(3,4-dichloro-phenyl)-propionyl]-ureido}-propionic acid methyl ester;
{3-[3-cyclopentyl-2(R)-(3,4-dichloro-phenyl)-propionyl]-ureido}-acetic acid ethyl ester;
3-{3-[3-cyclopentyl-2-(3,4-dichloro-phenyl)-propionyl]-ureido}-3-oxo-propionic acid ethyl ester.

In certain compounds of this invention, R⁴⁰ is hydroxy lower alkyl, or halo lower alkyl. In some such compounds, R³ of the amide is cyclopentyl. Preferably R¹ and R² are independently halo, and in addition the amide is in the "R" configuration at the asymmetric carbon shown. Examples of the above amides are:
1-[3-cyclopentyl-2-(3,4-dichloro-phenyl)-proprionyl]-3-(2-hydroxy-ethyl)-urea;
1-[3-cyclopentyl-2-(3,4-dichloro-phenyl)-proprionyl]-3-(2-hydroxy-propyl)-urea;
1-[3-cyclopentyl-2-(3,4-dichloro-phenyl)-proprionyl]-3-(3-hydroxy-propyl)-urea;
1-[3-cyclopentyl-2(R)-(3,4-dichloro-phenyl)-proprionyl]-3-(2-hydroxy-propyl)-urea;
1-(2-chloro-ethyl)-3-[3-cyclopentyl-2-(3,4-dichloro-phenyl)-proprionyl]-urea;
1-[3-cyclopentyl-2(R)-(3,4-dichloro-phenyl)-proprionyl]-3-(3-hydroxy-propyl)-urea.

In the compounds described below, unless otherwise indicated, R⁴ is an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, - (CH₂)ₙ-OR⁶, -(CH₂)ₙ-C(O)OR⁷, -(CH₂)ₙ-C(O)NHR⁶, -C(O)-C(O)OR⁸, -(CH₂)ₙ-NHR⁶;.

In certain of such compounds, R³ is cyclopentyl (the compound I-D). The embodiments of the compound I-D are those compounds where R⁴ is an unsubstituted thiazole (Compound I-D1). Among the various embodiments of the compound of I-D1 are included those compounds where:
a) one of R¹ and R² is hydrogen, halogen, perfluoro-lower alkyl and the other of said R¹ and R² is halo or perfluoro-lower alkyl;
b) one of R¹ and R² is amino, nitro, halo, nitro or hydrogen and the other of said R¹ and R² is amino, cyano or nitro;
c) one of R¹ and R² is lower alkylthio, perfluoro-lower alkyl thio, halo or hydrogen and the other of said R¹ and R² is perfluoro-lower alkylthio, lower alkylsulfinyl or lower alkylthio;
d) one of R¹ and R² is lower alkyl sulfonyl, hydrogen, nitro, cyano, amino, hydroxyamino, sulfonamido or halo, and the other of said R¹ and R² is lower alkyl sulfonyl;
e) one of R¹ and R² is lower alkyl sulfonyl, and the other of said R¹ and R² is halo or perfluoro-lower alkyl;
f) one of R¹ and R² is perfluoro-lower alkyl sulfonyl or hydrogen and the other of said R¹ and R² is perfluoro-lower alkyl sulfonyl;
g) one of R¹ and R² is -OR⁵, or -C(O)OR⁵ and the other of said R¹ and R² is hydrogen or -OR⁵; and R⁵ is as above; and
h) one of R¹ and R² is -OR⁵ and the other is halo.

In accordance with another embodiment of this invention where R³ is cyclopentyl, the embodiments are those compounds where R⁴ is a mono-substituted thiazole (compounds I-D2). Among the embodiments of compounds I-D2, are those compounds where the mono-substitution is -(CH₂)ₙ-OR⁶ and n and R⁶ are as above (compounds I-D2(a)). Among the embodiments of compounds I-D2 (a) are those compounds where:
a) one of R¹ and R² is halo and the other of said R¹ and R² is hydrogen or halo;
b) one of R¹ and R² is lower alkyl sulfonyl, and the other of said R¹ and R² is lower alkyl sulfonyl or hydrogen; and
c) one of R¹ and R² is hydrogen and the other of said R¹ and R² is lower alkyl or perfluoro-lower alkyl.

In accordance with another embodiment of the invention where R³ is cyclopentyl and R⁴ is a mono-substituted thiazole (Compounds I-D2), are those compounds where the mono-substitution is lower alkyl and one of R¹ and R² are hydrogen or halogen and the other of R¹ and R² is halogen (Compounds I-D2(b)).

Among another embodiment of the compounds I-D are those compounds where the mono-substituted thiazole is substituted with -(CH₂)ₙ-C(O)OR⁷, wherein n is 0 or 1 and R⁷ is hydrogen, or lower alkyl (Compounds of I-D2(c)). Among the embodiments of compounds of formula I-D2(c) are those compounds where:
a) one of R¹ and R² is hydrogen and the other of said R¹ and R² is halo;
b) R¹ and R² are each independently halo;
c) one of R¹ or R² is nitro, amino or hydrogen and the other of said R¹ and R² is nitro or amino; and
d) one of R¹ and R² is lower alkyl sulfonyl, perfluoro-lower alkyl, halogen or hydrogen and the other of said R¹ andR² is lower alkyl sulfonyl.

In accordance with another embodiment of this invention, where R³ and cyclopentyl and R⁴ is a mono-substituted thiazole (Compounds I-D2) are those compounds where the mono-substituted thiazole is substituted with -C(O)-C(O)OR⁸, wherein R⁸ is as above (Compounds I-D2(d)). Among the embodiments of compound I-D2(d) are those compounds where:
a) one of R¹ and R² are hydrogen and the other of said R¹ and R² is nitro or amino;
b) one of R¹ and R² is halo or perfluoro-lower alkyl and the other of said R¹ and R² is perfluoro-lower alkyl, halogen or hydrogen; and
c) one of R¹ and R² is hydrogen or halogen and the other of said R¹ and R² is lower alkylsulfonyl.

In accordance with another embodiment of this invention, where R³ is cyclopentyl and R⁴ is a mono-substituted thiazole, compounds I-D2, are those compounds where the mono-substitution on the thiazole ring is a nitro group and one of R¹ and R² are hydrogen and halogen and the other of R¹ and R² is halogen or lower alkyl sulfonyl (compound of formula I-D2(e)).

In accordance with another embodiment of this invention, where R³ is cyclopentyl (Compound I-D) and R⁴ is an unsubstituted pyridine (Compounds I-D3). Among the embodiments of compound I-D3 are those compounds where:
a) one of R¹ and R² are halo, perfluoro-lower alkyl or hydrogen and the other of said R¹ and R² is halo, perfluoro-lower alkyl, amino, cyano or nitro;
b) one of R¹ and R² is lower alkyl thio, perfluoro-lower alkyl thio or cyano, and the other is hydrogen;
c) one of R¹ and R² is lower alkyl sulfonyl, halo, cyano, nitro or hydrogen and the other of said R¹ and R² is lower alkyl sulfonyl; and
d) one of R¹ and R² is perfluoro-lower alkyl sulfonyl, lower alkyl sulfonyl or hydrogen and the other of said R¹ and R² is perfluoro-lower alkyl sulfonyl or perfluoro-lower alkyl.

In accordance with another embodiment of the invention, where R³ is cyclopentyl (Compounds I-D) are those compounds where R⁴ is a mono-substituted pyridine ring. Among the embodiments of the mono-substituted pyridine (Compounds I-D4) are those compounds where the mono-substitution is -(CH₂)ₙ-C(O)OR⁷, wherein n and R⁷ are as above (compound I-D4(a)). Among the embodiments of compounds I-D4(a) are those compounds where:
a) wherein R¹ and R² are each independently halo;
b) wherein one of R¹ and R² is hydrogen and the other of said R¹ and R² is halo, amino, cyano or nitro; and
c) one of R¹ and R² is perfluoro-lower alkyl sulfonyl, lower alkyl sulfonyl or hydrogen and the other of said R¹ and R² is perfluoro-lower alkyl sulfonyl or lower alkyl sulfonyl.

Other embodiments of the compounds of formula I-D4(b) are those compounds where the pyridine ring is mono-substituted with -(CH₂)ₙ-OR⁶ wherein n and R⁶ are as above (Compounds I-D4(b)). Among the embodiments of the compound I-D4(b) are those compounds where:
a) one of R¹ and R² are halo and the other of said R¹ and R² is hydrogen or halo; and
b) one of R¹ and R² is lower alkyl sulfonyl or hydrogen and the other of said R¹ and R² is lower alkyl sulfonyl.

Another embodiment of compounds where R³ is cyclopentyl and R⁴ is a mono-substituted pyridine ring are those compounds where the pyridine ring is mono-substituted with a halo or perfluoro-lower alkyl substituent, the compound of formula I-D4(c). Among the embodiments of the compound of formula I-D4(c) are those compounds where:
a) one of R¹ and R² is halo or hydrogen and the other is halo; and
b) one of R¹ and R² is halo, nitro or hydrogen and the other is perfluoro-lower alkyl sulfonyl or lower alkyl sulfonyl.

In accordance with another embodiment of this invention are compounds of where R³ is cyclopentyl and R⁴ is a mono-substituted pyridine are those compounds where the pyridine is mono-substituted with a nitro substituent, (Compound I-D4(d)). The embodiments of the compound I-D4(d) include compounds where one of R¹ and R² is halo and other of said R¹ or R² is hydrogen, halo, or lower alkyl sulfonyl.

In accordance with another embodiment of this invention are compounds of formula I where R³ is cyclopentyl and R⁴ is mono-substituted pyridine and the mono-substitution is a lower alkyl group (Compounds I-D4(e)). Among the embodiments of compounds I-D4(e) are those compounds where one of R¹ and R² is halo or hydrogen and the other of R¹ and R² is halo, perfluoro-lower alkyl, perfluoro-lower alkyl sulfonyl, or lower alkyl sulfonyl.

In accordance with another embodiment of this invention where R³ is cyclopentyl and R⁴ is a mono-substituted pyridine are those compounds where the mono-substituent is - (CH₂)ₙ-C(O)-NHR⁶, wherein n and R are as above (Compound I-D4(f)). Among the embodiments of compound I-D4(f) are those compounds wherein one of R¹ and R² are independently selected from the group consisting of halo or hydrogen and the other of said R¹ and R² is halo, or lower alkyl sulfonyl.

Another embodiment of this invention where R³ is cyclopentyl are those compounds where R⁴ is an unsubstituted imidazolyl (Compound I-D5). Among the embodiments of compounds I-D5 are those compounds wherein one of R¹ and R² is selected from the group consisting of halo and hydrogen and the other of said R¹ and R² is halo, or lower alkyl sulfonyl.

Another embodiment of the compounds of this invention are those compounds where R³ is cyclopentyl and R⁴ is an isoxazolyl ring (the compound I-D6). The embodiments of compound I-D6 are those compounds where the isoxazolyl ring is unsubstituted or substituted, preferably mono-substituted. Among the mono-substituted substituents, the preferred substituents substituted on the isoxazolyl ring is lower alkyl. An embodiment of the compound I-D6, either where the isoxazolyl ring is unsubstituted or substituted with a lower alkyl substituent are those compounds where one of R¹ and R² is halo, nitro, lower alkyl sulfonyl or perfluoro-lower alkyl and the other of R¹ and R² is hydrogen or halo.

Another embodiment of this invention where R³ is cyclopentyl are those compounds where R⁴ is either an unsubstituted oxazolyl, or an oxazolyl mono-substituted with a lower alkyl group. Another embodiment with respect to either of those compounds are those compounds where one of R¹ or R² is halo, nitro, lower alkyl sulfonyl or perfluoro-lower alkyl and the other is of R¹ or R² is hydrogen or halo.

Another embodiment of this invention where R³ is cyclopentyl are those compounds where R⁴ is pyridazinyl which is either unsubstituted or substituted with a lower alkyl group (Compound I-D7). Embodiments of the compound I-D7 are encompassed by this invention include those compounds where one of R¹ or R² is halo, nitro, lower alkyl sulfonyl or perfluoro-lower alkyl and the other of said R¹ or R² is hydrogen or halo.

Another embodiment of this invention where R³ is cyclopentyl include compounds where R⁴ is unsubstituted pyrimidinyl. The embodiments of those compounds where R³ is cyclopentyl and R⁴ is unsubstituted pyrimidinyl include those compounds where one of R¹ or R² is halo, nitro, lower alkyl sulfonyl or perfluoro-lower alkyl and the other is hydrogen or halo.

Another embodiment of this invention includes compounds where R³ is cyclopentyl where R⁴ is an unsubstituted thiadiazolyl ring. Among the embodiments included within those compounds where R³ is cyclopentyl and R⁴ is an unsubstituted thiadiazolyl ring are those compounds wherein one of R¹ or R² is halo, nitro, lower alkyl sulfonyl or perfluoro-lower alkyl and the other of said R¹ and R² is hydrogen or halo.

In accordance with other embodiments of this invention, R³ in the compound of formula I can be cycloheptyl or cyclohexyl. The embodiments of the compound of formula I where R³ is cycloheptyl or cyclohexyl include those compounds where R⁴ is thiazolyl which can be mono-substituted or unsubstituted. Embodiments included within such compounds where R³ is cycloheptyl or cyclohexyl and R⁴ is an unsubstituted thiazolyl include those compounds wherein one of R¹ and R² is halo, lower alkyl sulfinyl, perfluoro-lower alkyl sulfinyl, perfluoro-lower alkyl or lower alkyl sulfonyl and the other is of said R¹ and R² with halo, perfluoro-lower alkyl or hydrogen.

Examples of compounds of formula I according to the present invention, wherein R⁴ is a heteroaromatic ring are:
2-(3-chloro-phenyl)-3-cyclopentyl-N-thiazol-2-yl-propionamide,
2-(4-bromo-phenyl)-3-cyclopentyl-N-thiazol-2-yl-propionamide,
2-(4-chloro-phenyl)-3-cyclopentyl-N-thiazol-2-yl-propionamide,
3-cyclopentyl-2-(3,4-dichlorophenyl)-N-thiazol-2-yl-propionamide,
3-cyclopentyl-N-thiazol-2-yl-2-(4-trifluoromethyl-phenyl)-propionamide,
3-cyclopentyl-2-(3-fluoro-4-trifluoromethyl-phenyl)-N-thiazol-2-yl-propionamide,
3-cyclopentyl-2-(4-fluoro-3-trifluoromethyl-phenyl)-N-thiazol-2-yl-propionamide,
3-cyclopentyl-N-thiazol-2-yl-2-(3-trifluoromethyl-phenyl)-propionamide,
3-cyclopentyl-2(R)-(3,4-dichloro-phenyl)-N-thiazol-2-yl-propionamide,
3-cyclopentyl-2-(4-nitrophenyl)-N-thiazol-2-yl-propionamide,
2-(4-amino-phenyl)-3-cyclopentyl-N-thiazol-2-yl-propionamide,
2-(3-amino-phenyl)-3-cyclopentyl-N-thiazol-2-yl-propionamide,
2-(4-chloro-3-nitro-phenyl)-3-cyclopentyl-N-thiazol-2-yl-propionamide,
2-(4-cyano-phenyl)-3-cyclopentyl-N-thiazol-2-yl-propionamide,
3-cyclopentyl-N-thiazol-2-yl-2-(4-trifluoromethylsulfanyl-phenyl)-propionamide,
3-cyclopentyl-2-(4-methylsulfanyl-phenyl)-N-thiazol-2-yl-propionamide,
3-cyclopentyl-2-(4-methylsulfanyl-3-trifluoromethyl-phenyl)-N-thiazol-2-yl-propionamide,
3-cyclopentyl-2-(4-methanesulfinyl-phenyl)-N-thiazol-2-yl-propionamide,
3-cyclopentyl-2-(4-methanesulfonyl-phenyl)-N-thiazol-2-yl-propionamide,
3-cyclopentyl-2-(4-methanesulfonyl-3-nitrophenyl)-N-thiazol-2-yl-propionamide,
2-(3-amino-4-methanesulfonyl-phenyl)-3-cyclopentyl-N-thiazol-2-yl-propionamide,
3-cyclopentyl-2-(3-hydroxyamino-4-methanesulfonyl-phenyl)-N-thiazol-2-yl-propionamide,
2-(3-cyano-4-methanesulfonyl-phenyl)-3-cyclopentyl-N-thiazol-2-yl-propionamide,
3-cyclopentyl-2-(4-ethanesulfonyl-phenyl)-N-thiazol-2-yl-propionamide
2-(3,4-bis-methanesulfonyl-phenyl)-3-cyclopentyl-N-thiazol-2-yl-propionamide,
3-cyclopentyl-2-(4-sulfamoyl-phenyl)-N-thiazol-2-yl-propionanude,
2-[4-(butane-1-sulfonyl)-phenyl]-3-cyclopentyl-N-thiazol-2-yl-propionamide,
3-cyclopentyl-2-[4-(propane-1-sulfonyl)-phenyl]-N-thiazol-2-yl-propionamide,
3-cyclopentyl-2-(4-methanesulfonyl-3-trifluoromethyl-phenyl)-N-thiazol-2-yl-propionamide,
2(R)-(3-chloro-4-methanesulfonyl-phenyl)-3-cyclopentyl-N-thiazol-2-yl-propionamide,
2-[3-chloro-4-methanesulfonyl-phenyl]-3-cyclopentyl-N-thiazol-2-yl-propionamide,
2-(3-bromo-4-methanesulfonyl-phenyl)-3-cyclopentyl-N-thiazol-2-yl-propionamide,
3-cyclopentyl-N-thiazol-2-yl-2-(4-trifluoromethanesulfonyl-phenyl)-propionamide,
3-cyclopentyl-N-thiazol-2-yl-2-(3-trifluoromethanesulfonyl-phenyl)-propionamide,
3-cyclopentyl-N-thiazol-2-yl-2-(4-tnfluoromethoxy-phenyl)-propionamide,
3-cyclopentyl-2-(3-methoxy-phenyl)-N-thiazol-2-yl-propionamide,
3-cyclopentyl-2-(3-hydroxy-phenyl)-N-thiazol-2-yl-propionamide,
3-cyclopentyl-2-(3,4-dimethoxy-phenyl)-N-thiazol-2-yl-propionamide,
3-cyclopentyl-2-(3,4-dihydroxy-phenyl)-N-thiazol-2-yl-propionamide,
3-cyclopentyl-2-(4-methoxy-phenyl)-N-thiazol-2-yl-propionamide,
3-cyclopentyl-2-(4-hydroxy-phenyl)-N-thiazol-2-yl-propionamide,
4-[2-cyclopentyl-1-(thiazol-2-ylcarbamoyl)-ethyl]-benzoic acid methyl ester,
3-cyclopentyl-2-(3-fluoro-4-methoxy-phenyl)-N-thiazol-2-yl-propionamide,
3-cyclopentyl-2-(3-fluoro-4-hydroxy-phenyl)-N-thiazol-2-yl-propionamide,
3-cyclopentyl-2-(3,4-dichlorophenyl)-N-(5-hydroxymethyl-thiazol-2-yl)-propionamide,
3-cyclopentyl-2-(3,4-dichlorophenyl)-N-[4-(2-hydroxyethyl)-thiazol-2-yl]-propionamide,
2-(4-chloro-phenyl)-3-cyclopentyl-N-(5-hydroxymethyl-thiazol-2-yl)-propionamide,
3-cyclopentyl-2-(3,4-dichlorophenyl)-N-(4-hydroxymethyl-thiazol-2-yl)-propionamide,
3-cyclopentyl-N-(4-hydroxymethyl-thiazol-2-yl)-2-(4-methanesulfonyl-phenyl)-propionamide,
3-cyclopentyl-N-[4-(2-hydroxyethyl)-thiazol-2-yl]-2-(4-methanesulfonyl-phenyl)-propionamide,
3-cyclopentyl-2-(3,4-dichlorophenyl)-N-(4-methyl-thiazol-2-yl)-propionamide,
3-cyclopentyl-2-(3,4-dichlorophenyl)-N-(5-methyl-thiazol-2-yl)-propionamide,
{2-[2-(3-chloro-phenyl)-3-cyclopentyl-propionylamino]-thiazol-4-yl}-acetic acid ethyl ester,
{2-[2-(3-chloro-phenyl)-3-cyclopentyl-propionylamino]-thiazol-4-yl}-acetic acid methyl ester,
2-[2-(3-chloro-phenyl}-3-cyclopentyl-propionylamino]-thiazole-4-carboxylic acid methyl ester,
2-[2-(3-chloro-phenyl)-3-cyclopentyl-propionylamino]-thiazole-4-carboxylic acid ethyl ester,
{2-[2-(4-chloro-phenyl)-3-cyclopentyl-propionylamino]-thiazol-4-yl}-acetic acid ethyl ester,
2-[2-(4-chloro-phenyl)-3-cyclopentyl-propionylamino]-thiazole-4-carboxylic acid methyl ester,
2-[2-(4-chloro-phenyl)-3-cyclopentyl-propionylamino]-thiazole-4-carboxylic acid ethyl ester,
{2-[2-(4-chloro-phenyl)-3-cyclopentyl-propionylamino]-thiazol-4-yl}-acetic acid methyl ester,
{2-[3-cyclopentyl-2-(3,4-dichlorophenyl)-propionylamino]-thiazol-4-yl}-acetic acid,
{2-[3-cyclopentyl-2-(3,4-dichlorophenyl)-propionylamino]-thiazol-4-yl}-acetic acid ethyl ester,
2-[3-cyclopentyl-2-(3,4-dichlorophenyl)-propionylamino]-thiazole-5-carboxylic acid,
2-[3-cyclopentyl-2-(3,4-dichlorophenyl)-propionylamino]-thiazole-4-carboxylic acid,
{2-[3-cyclopentyl-2-(3,4-dichlorophenyl)-propionylamino]-thiazol-4-yl}-acetic acid methyl ester,
(2R)-2-[3-cyclopentyl-2-(3,4-dichloro-phenyl)-propionylamino]-thiazole-4-carboxylic acid methyl ester,
2-[3-cyclopentyl-2-(3,4-dichlorophenyl)-propionylamino]-thiazole-5-carboxylic acid methyl ester,
2-[3-cyclopentyl-2-(3,4-dichlorophenyl)-propionylamino]-thiazole-5-carboxylic acid ethyl ester,
{2-[3-cyclopentyl-2-(4-nitro-phenyl)-propionylamino]-thiazol-4-yl}-acetic acid ethyl ester.
{2-[3-cyclopentyl-2-(4-nitro-phenyl)-propionylamino]-thiazol-4-yl}-acetic acid methyl ester,
2-[3-cyclopentyl-2-(4-nitro-phenyl)-propionylamino]-thiazole-4-carboxylic acid methyl ester,
2-[3-cyclopentyl-2-(4-nitro-phenyl)-propionylamino]-thiazole-4-carboxylic acid ethyl ester,
{2-[2-(4-amino-phenyl)-3-cyclopentyl-propionylamino]-thiazol-4-yl }-acetic acid methyl ester,
2-[2-(4-amino-phenyl)-3-cyclopentyl-propionylamino]-thiazole-4-carboxylic acid methyl ester,
{2-[3-cyclopentyl-2-(4-methanesulfonyl-phenyl)-propionylamino]-thiazole-4-carboxylic acid ethyl ester,
{2-[3-cyclopentyl-2-(4-methanesulfonyl-phenyl)-propionylamino]-thiazol-4-yl}-4-acetic acid methyl ester,
{2-[3-cyclopentyl-2-(4-methanesulfonyl-3-trifluoromethyl-phenyl)-propionylamino]-thiazol-4-yl}-acetic acid methyl ester,
2-[3-cyclopentyl-2-(4-methanesulfonyl-3-trifluoromethyl-phenyl)-propionylamino]-thiazole-4-carboxylic acid methyl ester,
{2-[3-cyclopentyl-2-(4-nitro-phenyl)-propionylamino]-thiazol-4-yl}-oxo-acetic acid ethyl ester,
{2-[3-cyclopentyl-2-(3,4-dichlorophenyl)-propionylamino]thiazol-5-yl}-oxo-acetic ethyl ester,
{2-[3-cyclopentyl-2-(4-fluoro-3-trifluoromethyl-phenyl)-propionylamino]-thiazol-4-yl}-oxo-acetic acid ethyl ester,
{2-[2-(3-chloro-4-methanesulfonyl-phenyl)-3-cyclopentyl-propionylamino]-thiazol-4-yl}-oxo-acetic acid ethyl ester,
3-cyclopentyl-2-(3,4-dichlorophenyl)-N-(5-nitro-thiazol-2-yl)-propionamide,
3-cyclopentyl-2-(3-fluoro-4-trifluoromethyl-phenyl)-N-pyridin-2-yl-propionamide,
3-cyclopentyl-2-(3,4-dichloro-phenyl)-N-pyridin-2-yl-propionamide,
3-cyclopentyl-N-pyridin-2-yl-2-(4-trifluoromethyl-phenyl)-2-propionamide,
3-cyclopentyl-N-thiazol-2-yl-2-(3-trifluoromethyl-phenyl)-propionamide,
2-(3-chloro-phenyl)-3-cyclopentyl-N-pyridin-2-yl-propionamide,
2-(4-amino-phenyl)-3-cyclopentyl-N-pyridin-2-yl-propionamide,
2-(4- cyano-phenyl)-3-cyclopentyl-N-pyridin-2-yl-propionamide,
2-(4-chloro-phenyl)-3-cyclopentyl-N-pyridin-2-yl-propionamide,
2-(4-chloro-3-nitro-phenyl)-3-cyclopentyl-N-pyridin-2-yl-propionamide,
3-cyclopentyl-2-(4-nitro-phenyl)-N-pyridin-2-yl-propionamide,
2-(4-cyano-phenyl)-3-cyclopentyl-N-pyridin-2-yl-propionamide,
3-cyclopentyl-2-(4-methylsulfanyl-phenyl)-N-pyridin-2-yl-propionamide,
3-cyclopentyl-N-pyridin-2-yl-2-(4-trifluoromethylsulfanyl-phenyl)-propionamide,
3-cyclopentyl-2-(4-methanesulfonyl-3-nitrophenyl)-N-pyridin-2-yl-propionamide,
3-cyclopentyl-2-(4-methanesulfonyl-phenyl)-N-pyridin-2-yl-propionamide,
2-(3-bromo-4-methanesulfonyl-phenyl)-3-cyclopentyl-N-pyridin-2-yl-propionamide,
2-(3-cyano-4-methanesulfonyl-phenyl)-3-cyclopentyl-N-pyridin-2-yl- propionamide,
3-cyclopentyl-2-(4-ethanesulfonyl-phenyl)-N-pyridin-2-yl-propionamide,
2-(3,4-bis-methanesulfonyl-phenyl)-3-cyclopentyl-N-pyridin-2-yl-propionamide,
2-(3-chloro-4-methanesulfonyl-phenyl)-3-cyclopentyl-N-pyridin-2-yl-propionamide,
3-cyclopentyl-2-(4-methanesulfonyl-3-trifluoromethyl-phenyl)-N-pyridin-2-yl-propionamide,
3-cyclopentyl-N-pyridin-2-yl-2-(4-trifluoromethanesulfonyl-phenyl)-propionamide,
3-cyclopentyl-2-(3,4-dichloro-phenyl)-N-(5-carboxymethylpyridin)-2-yl-propionamide,
6-[3-cyclopentyl-2(R)-(3,4-dichloro-phenyl)-propionylamino)-nicotinic acid methyl ester,
6-[2-(4-chlaro-phenyl)-3-cyclopentyl-propionylamino]-nicotinic acid,
6-[2-(3-chloro-phenyl)-3-cyclopentyl-propionylamino]-nicotinic acid methyl ester,
6-[2-(4-chloro-phenyl)-3-cyclopentyl-propionylamino]-nicotinic acid methyl ester,
6-[3-cyclopentyl-2-(4-nitro-phenyl)-propionylamina]-nicotinic acid methyl ester,
6-[2-(4-amino-phenyl)-3-cyclopentyl-propionylamino]-nicotinic acid methyl ester,
6-[2-(3-chloro-phenyl)-3-cyclopentyl-propionylamino]-nicotinic acid,
6-[2-(4-cyano-phenyl)-3-cyclopentyl-propionylamino]-nicotinic acid,
6-[3-cyclopentyl-2-(4-trifluoromethanesulfonyl-phenyl)-propionylamino]-nicotinic acid methyl ester,
6-[3-cyclopentyl-2-(4-trifluoromethanesulfonyl-phenyl)-propionylamino)-nicotinic acid,
6-[3-cyclopentyl-2-(4-methanesulfonyl-phenyl)-propionylamino]-nicotinic acid methyl ester,
3-cyclopentyl-2(R)-(3,4-dichloro-phenyl)-N-(5-hydroxymethyl-pyridin-2-yl)-propionamide,
3-cyclopentyl-2-(3,4-dichloro-phenyl)-N-(5-hydroxy-pyridin-2-yl)-propionamide,
2-(4-chloro-phenyl)-3-cyclopentyl-N-(5-hydroxymethyl-pyridin-2-yl)-propionamide,
3-cyclopentyl-N-(5-hydroxymethyl-pyridin-2-yl)-2-(4-methanesulfonyl-phenyl)-propionamide,
N-(5-chloro-pyridin-2-yl)-3-cyclopentyl-2-(3,4-dichloro-phenyl)-propionamide,
3-cyclopentyl-2-(3,4-dichloro-phenyl)-N-(5-brompyridin)-2-yl-propionamide,
3-cyclopentyl-2(R)-(3,4-dichloro-phenyl)-N-(5-trifluoromethyl-pyridin-2-yl)-propionamide,
N-(5-bromo-pyridin-2-yl)-3-cyclopentyl-2(R)-(3,4-dichloro-phenyl)-propionamide,
N-(5-chloro-pyridin-2-yl)-3-cyclopentyl-2-(4-trifluoromethanesulfonyl-phenyl)-propionamide,
N-(5-bromo-pyridin-2-yl)-3-cyclopentyl-2-(4-trifluoromethanesulfonyl-phenyl)-propionamide,
N-(5-bromo-pyridin-2-yl)-3-cyclopentyl-2-(4-methanesulfonyl-3-nitro-phenyl)-propionamide,
2-(3-bromo-4-methanesulfonyl-phenyl)-N-(5-bromo-pyridin-2-yl)-3-cyclopentylpropionamide,
N-(5- bromo-pyridin-2-yl)-2-(3-chloro-4-methanesulfonyl-phenyl)-3-cyclopentylpropionamide,
2-(3-chloro-4-methanesulfonyl-phenyl)-3-cyclopentyl-N-(5-trifluoromethyl-pyridin-2-yl)-propionamide,
N-(5-chloro-pyridin-2-yl)-2-(3-chloro-4-methanesulfonyl-phenyl)-3-cyclopentylpropionamide,
3-cyclopentyl-2-(3,4-dichloro-phenyl)-N-(5-nitropyridin)-2-yl-propionamide,
3-cyclopentyl-2-(3,4-dichloro-phenyl)-N-(5-methylpyridin)-2-yl-propionamide,
3-cyclopentyl-2-(3,4-dichloro-phenyl)-N-(4-methylpyridin)-2-yl-propionamide,
3-cyclopentyl-2-(3,4-dichloro-phenyl)-N-(6-methylpyridin)-2-yl-propionamide,
3-cyclapentyl-N-(5-methyl-pyridin-2-yl)-2-(4-trifluoromethanesulfonyl-phenyl)-propionamide,
3-cyclopentyl-2-(4-fluoro-3-trifluoromethyl-phenyl)-N-(5-methyl-pyridin-2-yl)-propionamide,
6-[3-cyclopentyl-2-(3,4-dichloro-phenyl)-propionylamino]-N-methyl-nicotinamide,
3-cyclopentyl-2-(3,4-dichlorophenyl)-N-(1H-imidazol-2-yl)-propionamide,
3-cyclopentyl-2-(3,4-dichlorophenyl)-N-(5-methyl-isoxazol-3-yl)-propionamide,
3-cyclopentyl-2-(3,4-dichlorophenyl)-N-oxazol-2-yl-propionamide,
3-cyclopentyl-2-(3,4-dichlorophenyl)-N-pyridazin-3-yl-propionamide,
3-cyclopentyl-2-(3,4-dichlorophenyl)-N-pyrimidin-2-yl-propionamide,
3-cyclopentyl-2-(3,4-dichlorophenyl)-N-pyrimidin-6-yl-propionamide,
3-cyclopentyl-2-(4-nitro-phenyl)-N-pyrimidin-4-yl-propionamide,
3-cyclopentyl-2-(3,4-dichlorophenyl)-N-[1,3,4]thiadiazol-2-yl-propionamide,
2-[4-methanesulfonyl phenyl]-3-cyclohexyl N-thiazol-2-yl-propionamide, and
2-[4-methanesulfonyl phenyl]-3-cycloheptyl N-thiazol-2-yl-propionamide.

Examples of compounds of formula I according to the present invention, wherein R⁴ is a residue -C(O)NHR⁴⁰ and R⁴⁰ is as defined above, are:
1-(3-cyclopentyl-2-phenyl-propionyl)-3-methyl-urea,
1-[2-(3-chloro-phenyl)-3 cyclopentyl-propionyl]-3-methyl-urea,
1-[2-(4-chloro-phenyl)-3-cyclopentyl-propionyl]-3-methyl-urea,
1-[2-(4-cyano-phenyl)-3-cyclopentyl-propionyl]-3-methyl-urea,
1-[2-(4-bromo-phenyl)-3-cyclopentyl-propionyl]-3-methyl urea,
[3-cyclopentyl-2-(3,4-dichloro-phenyl)-propionyl]-urea,
1-[3-cyclopentyl-2-(3,4-dichloro-phenyl)-propionyl]-3-methyl-urea,
1-[3-cyclopentyl-2(R)-(3,4-dichlaro-phenyl)-propionyl]-3-ethyl-urea,
1-allyl-3-[3-cyclopentyl-2-(3,4-dichloro-phenyl)-proprionyl]-urea,
1-allyl-3-[3-cyclopentyl-2(R)-(3,4-dichloro-phenyl)-proprionyl]-urea,
1-[3-cyclopentyl-2-(3,4-dichloro-phenyl)-propionyl]-3-ethyl-urea,
1-[3-cyclopentyl-2(R)-(3,4-dichloro-phenyl)-propionyl]-3-methyl-urea,
1-[3-cyclopentyl-2-(3,4-dichloro-phenyl)-propionyl]-3-isopropyl-urea,
1-[3-cyclopentyl-2-(3,4-dichloro-phenyl)-propionyl]-3-propyl-urea,
1-[3-cyclopentyl-2-(3,4-difluoro-phenyl)-propionyl]-3-methyl-urea,
1-[2-(4-chloro-3-nitro-phenyl)-3-cyclopentyl-proprionyl]-3-methyl-urea,
1-[3-cyclopentyl-2-(4-nitro-phenyl)-propionyl]-3-methyl-urea,
1-[3-cyclopentyl-2-(4-trifluoromethylsulfanyl-phenyl)-propionyl]-3-methyl urea,
1-[3-cyclopentyl-2-(4-methylsulfanyl-phenyl)-propionyl]-3-methyl urea,
1-[3-cyclopentyl-2-(4-trifluoromethanesulfonyl-phenyl)-propionyl]-3-methyl urea,
1-[3-cyclopentyl-2-(3-trifluoromethanesulfonyl-phenyl)-propionyl]-3-methyl urea,
1-[3-cyclopentyl-2-(4-methanesulfonyl-phenyl)-propionyl]-3-methyl urea,
1-{2-[4-(butane-1-sulfonyl)-phenyl]-3-cyclopentyl-proprionyl}-3-methyl-urea,
1-[3-cyclopentyl-2-(4-ethanesulfonyl-phenyl)-proprionyl]-3-methyl-urea,
1-[2-(3,4-bis-methanesulfonyl-phenyl)-3-cyclopentyl-proprionyl]-3-methyl-urea,
1-[2-(3-bromo-4-methanesulfonyl-phenyl)-3-cyclopentyl-proprionyl]-3-methyl-urea,
1-[3-cyclopentyl-2-(3-fluoro-4-methanesulfonyl-phenyl)-proprionyl]-3-methyl-urea,
1-[2-(3-chloro-4-methanesulfonyl-phenyl)-3-cyclopentyl-proprionyl]-3-methyl-urea,
1-[2(R)-(3-chloro-4-methanesulfonyl-phenyl)-3-cyclopentyl-proprionyl]-3-methyl-urea, 1-[2-(3-chloro-4-methanesulfonyl-phenyl)-3-cyclopentyl-proprionyl]-3-ethyl-urea,
1-[2-(3-cyano-4-methanesulfonyl-phenyl)-3-cyclopentyl-proprionyl]-3-methyl-urea,
1-[3-cyclopentyl-2-(4-methanesulfonyl-3-trifluoromethyl-phenyl)-proprionyl]-3-methyl-urea,
1-[3-cyclopentyl-2-(4-fluoro-3-trifluoromethyl-phenyl)-propionyl]-3-methyl urea,
1-[3-cyclopentyl-2-(3-fluoro-4-trifluoromethyl-phenyl)-propionyl]-3-methyl urea,
1-[3-cyclopentyl-2-(4-methanesulfonyl-3-nitrophenyl)-propionyl]-3-methyl-urea,
[3-cyclopropyl-2-(3,4-dichloro-phenyl)-propionyl]-urea,
[3-cyclobutyl-2-(3,4-dichloro-phenyl)-propionyl]-urea,
3-[cyclohexyl-2-(3,4-dichloro-phenyl)-propionyl]-urea,
[3-cyclohexyl-2-(3,4-dichloro-phenyl)-propionyl]-3-methyl-urea,
[3-cycloheptyl-2-(3,4-dichloro-phenyl)-propionyl]-urea,
3-{3-[3-cyclopentyl-2-(3,4-dichloro-phenyl)-propionyl]-ureido}-propionic acid ethyl ester,
{3-[3-cyclopentyl-2-(3,4-dichloro-phenyl)-propionyl]-ureido}-acetic acid ethyl ester,
{3-[3-cyclopentyl-2-(3,4-dichloro-phenyl)-propionyl]-ureido }-acetic acid methyl ester,
3- {3-[3-cyclopentyl-2-(3,4-dichloro-phenyl)-propionyl]-ureido}-propionic acid methyl ester,
{3-[3-cyclopentyl-2(R)-(3,4-dichloro-phenyl)-propionyl]-ureido}-acetic acid ethyl ester,
3-{3-[3-cyclopentyl-2-(3,4-dichloro-phenyl)-propionyl]-ureido}-3-oxo-propionic acid ethyl ester,
1-[3-cyclopentyl-2-(3,4-dichloro-phenyl)-proprionyl]-3-(2-hydroxy-ethyl)-urea,
1-[3-cyclopentyl-2-(3,4-dichloro-phenyl)-proprionyl]-3-(2-hydroxy-propyl)-urea,
1-[3-cyclopentyl-2-(3,4-dichloro-phenyl)-proprionyl]-3-(3-hydroxy-propyl)-urea,
1-[3-cyclopentyl-2(R)-(3,4-dichloro-phenyl)-proprionyl]-3-(2-hydroxy-propyl)-urea,
1-(2-chloro-ethyl)-3-[3-cyclopentyl-2-(3,4-dichloro-phenyl)-proprionyl]-urea, and
1-[3-cyclopentyl-2(R)-(3,4-dichloro-phenyl)-proprionyl]-3-(3-hydroxy-propyl)-urea.

It will be appreciated that the compounds of formula I may be derivatised at functional groups to provide derivatives which are capable of conversion back to the parent compounds in vivo. Additionally, any physiologically acceptable equivalents of the compounds of formula I, which are capable of producing the parent compounds of formula I in vivo, are within the scope of this invention.

The compound of formula I can be prepared starting from the compound of formula V by the following Reaction Scheme: 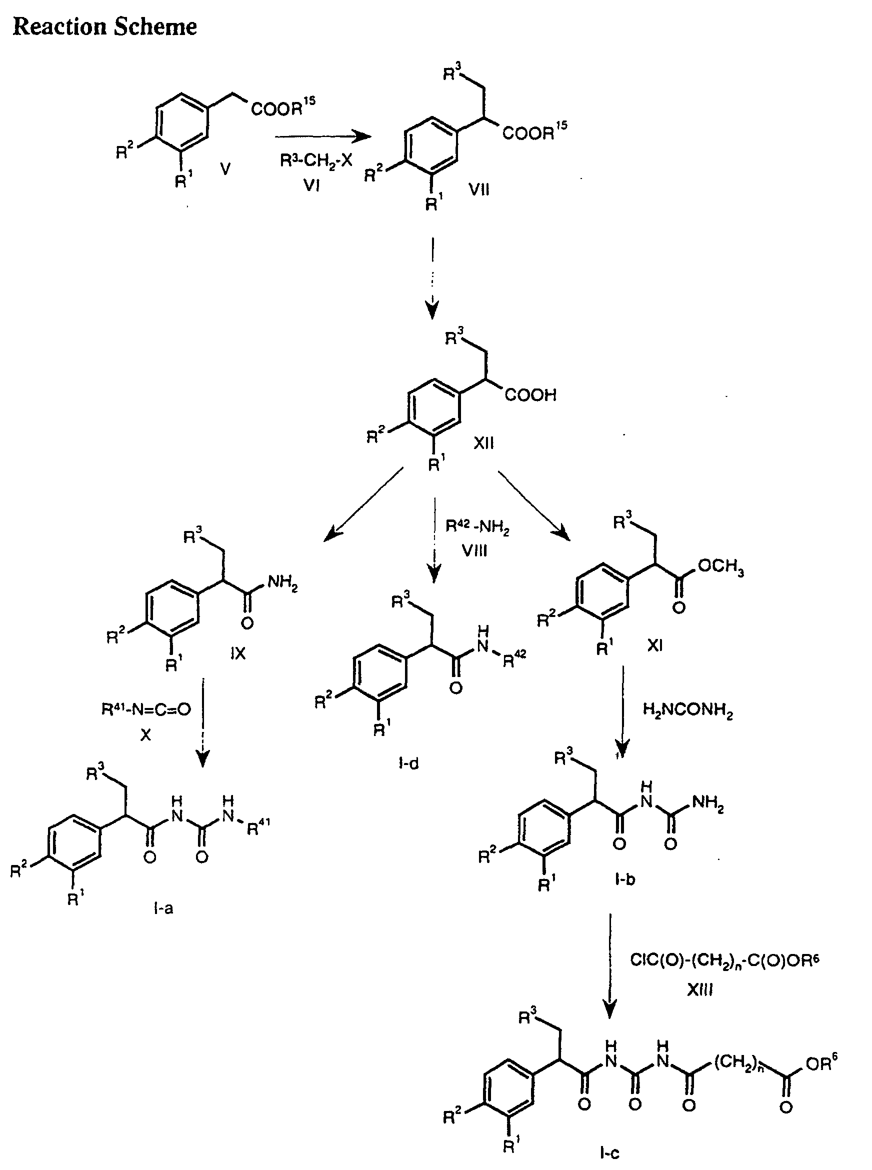 wherein R¹, R² and R³ are as above, R⁴² is an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, -(CH₂)ₙ-C(O)OR⁷, - (CH₂)ₙ-C(O)NHR⁶, -C(O)-C(O)OR⁸ or -(CH₂)ₙ-NHR⁶, wherein R⁶, R⁷, R⁸ and n are as defined above; R¹⁵ is hydrogen or lower alkyl; and R⁴¹ is lower alkyl, lower alkenyl, hydroxy lower alkyl, halo lower alkyl or -(CH₂)ₙ-C(O)OR⁵, wherein R⁵ is hydrogen or lower alkyl and n is as defined above.

The carboxylic acids and their lower alkyl esters of formula V wherein one of R¹ and R² is nitro, cyano, thio, amino, chloro, bromo, or iodo and the other is hydrogen are commercially available. In cases, where only the carboxylic acids are available, they can be converted to the corresponding esters of lower alkyl alcohols using any conventional esterification methods. All the reactions hereto forward are to be carried out on lower alkyl esters of the carboxylic acids of formula V. The amino substituted compounds of formula V can be converted to other substituents either before or after conversion to the compounds of formulae I-a, I-b, I-c or I-d. In this respect, the amino groups can be diazotized to yield the corresponding diazonium compound, which *in situ* can be reacted with the desired lower alkyl thiol, perfluoro-lower alkyl thiol (see for example, Baleja, J.D. *Synth. Comm.* **1984**, *14,* 215; Giam, C. S.; Kikukawa, K., *J. Chem*. *Soc, Chem*. *Comm.* **1980**, 756; Kau, D.; Krushniski, J. H.; Robertson, D. W, *J. Labelled Compd Rad.* **1985**, 22, 1045; Oade, S.; Shinhama, K.; Kim, Y. H., *Bull Chem Soc. Jpn*. **1980**, *53,* 2023; Baker, B. R.; et al, *J. Org. Chem.* **1952**, *17*, 164), or alkaline earth metal cyanide, to yield corresponding compounds of formula V, where one of the substituents is lower alkyl thio, perfluoro-lower alkyl thio, or cyano, and the other is hydrogen. If desired, the lower alkyl thio or perfluoro-lower alkyl thio compounds can then be converted to the corresponding lower alkyl sulfonyl or perfluoro-lower alkyl sulfonyl substituted compounds of formula V by oxidation. Any conventional method of oxidizing alkyl thio substituents to sulfones can be utilized to effect this conversion.

If it is desired to produce compounds of formula V wherein one of R¹ and R² is a lower alkyl or perfluoro-lower alkyl group, the corresponding halo substituted compounds of formula V can be used as starting materials. Any conventional method of converting an aromatic halo group to the corresponding alkyl group (see for example, Katayama, T.; Umeno, M., *Chem. Lett*. **1991**, 2073; Reddy, G. S.; Tam., *Organometallics*, **1984**, *3*, 630; Novak, J.; Salemink, C. A., *Synthesis*, **1983**, *7*, 597; Eapen, K. C.; Dua, S. S.; Tamboroski, C., *J. Org. Chem.* **1984**, *49*, 478; Chen, Q, -Y.; Duan, J. -X. *J. Chem. Soc. Chem. Comm.* **1993**, 1389; Clark, J. H.; McClinton, M. A.; Jone, C. W.; Landon, P.; Bisohp, D.; Blade, R. J., *Tetrahedron Lett*. **1989**, 2133; Powell, R. L.; Heaton, C. A, US patent 5113013) can be utilized to effect this conversion. On the other hand, the thio substituent can be oxidized to a -SO₃H group which then can be converted to -SO₂Cl which is reacted with ammonia to form the sulfonamide substituent -S(O)₂-NH₂. If it is desired to produce compounds of formula V wherein one or both of R¹ and R² is hydroxyamino, the corresponding nitro compounds can be used as starting material and can be converted to the corresponding compounds where R¹ and/or R² are hydroxyamino. Any conventional method of converting a nitro group to the corresponding aromatic hydroxyamino compound can be used to affect this conversion.

The carboxylic acids or lower alkyl esters of formula V wherein both of R¹ and R² are chloro or fluoro are commercially available. In cases, where only the carboxylic acids are available, they can converted to the corresponding esters of lower alkyl alcohols using any conventional esterification method. To produce the compound of formula V where both R¹ and R² are nitro, 3,4-dinitrotoluene can be used as starting material. This compound can be converted to the corresponding 3,4-dinitrophenyl acetic acid. This conversion can take place either before or after the compound of formula V is converted to the compounds of formulae I-a, I-b, I-c or I-d. Any conventional method of converting an aryl methyl group to the corresponding aryl acetic acid can be utilized to effect this conversion (see for example, Clark, R. D.; Muchowski, J. M.; Fisher, L. E.; Flippin, L. A.; Repke, D. B.; Souchet, M, *Synthesis,* **1991**, 871). The compounds of formula V where both R¹ and R² substituents are amino can be obtained from the corresponding dinitro compound of formula V, described above. Any conventional method of reducing a nitro group to an amine can be utilized to effect this conversion. The compound of formula V where both R¹ and R² are amine groups can be used to prepare the corresponding compound of formula V where both R¹ and R² are iodine or bromine via a diazotization reaction. Any conventional method of converting amino group to an iodo or bromo group (see for example, Lucas, H. J.; Kennedy, E. R. *Org. Synth. Coll. Vol, II* **1943**, 351) can be utilized to effect this conversion. If it is desired to produce compounds of formula V, where both R¹ and R² are lower alkyl thio or perfluoro-lower alkyl thio groups, the compound of formula V where R¹ and R² are amino can be used as starting material. Any conventional method of converting aryl amino group to aryl thioalkyl group can be utilized to effect this conversion. If it is desired to produce compounds of formula V where R¹ and R² are lower alkyl sulfonyl or lower perfluoro alkyl sulfonyl, the corresponding compounds of formula V where R¹ and R² are lower alkyl thio or perfluoro-lower alkyl thio can be used as starting material. Any conventional method of oxidizing alkyl thio substituents to sulfones can be utilized to effect this conversion.

If it is desired to produce compounds of formula V, where both R¹ and R² are substituted with lower alkyl or perfluoro-lower alkyl groups, the corresponding halo substituted compounds of formula V can be used as starting materials. Any conventional method of converting an aromatic halo group to the corresponding alkyl or perfluoro-lower alkyl group can be utilized to effect this conversion.

If it is desired to produce compounds of formula V, where one or both of R¹ and R² are substituted with sulfonamido, the corresponding compounds where one or both of R¹ and R² are substituted with nitro can be used as starting materials. Any standard method of converting a nitrophenyl compound to the corresponding sulfonamidophenyl compound can be used to effect this conversion.

The carboxylic acids corresponding to the compounds of formula V where one of R¹ and R² is nitro and the other is halo are known from the literature (see for 4-chloro-3-nitrophenyl acetic acid, Tadayuki, S.; Hiroki, M.; Shinji, U.; Mitsuhiro, S. Japanese patent, JP 71-99504, *Chemical Abstracts* 80:59716; see for 4-nitro-3-chlorophenyl acetic acid, Zhu, J.; Beugelmans, R.; Bourdet, S.; Chastanet, J.; Rousssi, G. *J*. *Org. Chem.* **1995,** *60*, 6389; Beugelmans, R.; Bourdet, S.; Zhu, J. *Tetrahedron Lett*. **1995,** *36*, 1279). These carboxylic acids can be converted to the corresponding lower alkyl esters using any conventional esterification methods. Thus, if it is desired to produce the compound of formula V where one of R¹ and R² is nitro and the other is lower alkyl thio or perfluoro-lower alkyl thio, the corresponding compound where one of R¹ and R² is nitro and the other is chloro can be used as starting material. In this reaction, any conventional method of nucleophilic displacement of aromatic chlorine group with a lower alkyl thiol can be used (see for example, Singh, P.; Batra, M. S.; Singh, H, *J. Chem. Res.-S* **1985** (6), S204; Ono, M.; Nakamura, Y.; Sata, S.; Itoh, I, *Chem*. *Lett,* **1988**, 1393; Wohrle, D.; Eskes, M.; Shigehara, K.; Yamada, A, *Synthesis*, **1993**, 194; Sutter, M.; Kunz, W, US patent, US 5169951). Once the compounds of formula V where one of R¹ and R² is nitro and the other is lower alkyl thio or perfluoro-lower alkyl thio are available, they can be converted to the corresponding compounds of formula V where one of R¹ and R² is nitro and the other is lower alkyl sulfonyl or perfluoro-lower alkyl sulfonyl using conventional oxidation procedures.

If it is desired to produce compounds of formula V where one of R¹ and R² is amino and the other is lower alkyl thio or perfluoro-lower alkyl thio, the corresponding compound where one of R¹ and R² is nitro and the other is lower alkyl thio or perfluoro-lower alkyl thio can be used as starting materials. Any conventional method of reducing an aromatic nitro group to an amine can be utilized to effect this conversion.

If it is desired to produce compounds of formula V where one of R¹ and R² is lower alkyl thio and the other is perfluoro-lower alkyl thio, the corresponding compound where one of R¹ and R² is amino and the other is lower alkyl thio or perfluoro-lower alkyl thio can be used as starting materials. Any conventional method of diazotizing aromatic amino group and reacting it *in situ* with the desired lower alkyl thiol can be utilized to effect this conversion.

If it is desired to produce compounds of formula V where one of R¹ and R² is lower alkyl sulfonyl and the other is perfluoro-lower alkyl sulfonyl, the corresponding compounds where one of R¹ and R² is lower alkyl thio and the other is perfluoro-lower alkyl thio, can be used as starting materials. Any conventional method of oxidizing an aromatic thio ether group to the corresponding sulfone group can be utilized to effect this conversion.

If it is desired to produce compounds of formula V where one of R¹ and R² is halo and the other is lower alkyl thio or perfluoro-lower alkyl thio, the corresponding compounds where one of R¹ and R² is amino and the other is lower alkyl thio or perfluoro-lower alkyl thio can be used as starting materials. Any conventional method of diazotizing an aromatic amino group and conversion of it *in situ* to an aromatic halide can be utilized to effect this conversion.

If it is desired to produce compounds of formula V where one of R¹ and R² is halo and the other is lower alkyl sulfonyl or perfluoro-lower alkyl sulfonyl, the corresponding compounds where one of R¹ and R² is halo and the other is lower alkyl thio or perfluoro-lower alkyl thio can be used as starting materials. Any conventional method of oxidizing an aromatic thio ether to the corresponding sulfone can be utilized to effect this conversion. If it is desired to produce compounds of various combinations of lower alkyl and perfluoro-lower alkyl groups of compounds of formula V, the corresponding halo substituted compounds of formula V can be used as starting materials. Any conventional method of converting an aromatic halo group to the corresponding alkyl group can be utilized to effect this conversion.

If one wishes to prepare the compound formula V where one of R¹ and R² is nitro and the other is amino, the compound of formula V where one of R¹ and R² is nitro and other is chloro can be used as a starting material. The chloro substituent on the phenyl ring can be converted to an iodo substituent (see for example, Bunnett, J. F.; Conner, R. *M.; Org. Synth. Coll Vol V*, **1973**, 478; Clark, J. H.; Jones, C. W. *J. Chem. Soc. Chem. Commun.* **1987**, 1409), which in turn can be reacted with an azide transferring agent to form the corresponding azide (see for example, Suzuki, H.; Miyoshi, K.; Shinoda, M. *Bull. Chem. Soc. Jpn*, **1980**, *53*, 1765). This azide can then be reduced in a conventional manner to form the amine substituent by reducing it with commonly used reducing agent for converting azides to amines (see for example, Soai, K.; Yokoyama, S.; Ookawa, A. *Synthesis*, **1987**, 48).

In order to prepare the compound of formula V where one of R¹ and R² is cyano and the other is amino, the compound of formula V where one of R¹ and R² is nitro and other is amino can be used as a starting material. The amino group is converted to cyano by conventional means of converting aryl-amino to aryl-cyano, for example by diazotization with a cyanide-transferring agent such as cuprous cyanide. The nitro group is converted to an amino group as described above. If it is desired to convert commercially available compounds to compounds of formula V where one of R¹ and R² is cyano and the other is any other desired substituent as defined above, the compound of formula V where one of R¹ and R² is nitro and the other is halo is used as starting material. With this starting material, the nitro is converted to the cyano and the halo is converted to the desired R¹ or R² substituent.

If it is desired to produce the compound of formula V where both R¹ and R² are cyano, this can be prepared as described hereinbefore from compounds where R¹ and R² are amino via diazotization and reaction with a cyanide-transferring agent such as cuprous cyanide.

If it is desired to produce the compound of formula V wherein one of R¹ or R² is - C(O)OR⁵, this compound can be formed from the corresponding compound where R¹ and R² is an amino group by converting the amino group to a diazonium salt reacting the diazonium salt with a hydrohalic acid to form the corresponding halide and then reacting this halide with a Grignard reagent to produce the corresponding acid which can be esterified. On the other hand, if one wants to produce the compound of formula V where both R¹ and R² are carboxylic acid groups. This compound can be produced as described above from the corresponding compound of formula V where both R¹ and R² are amino groups. In the same manner, the amino groups in the compound of formula V can be converted to the corresponding compound where R¹ or both of R¹ or R² is -OR⁵ by simply reacting the amino group with sodium nitrate in sulfuric acid to convert the amino group to a hydroxy group and thereafter etherifying, if desired, the hydroxy group.

The substituents which form R¹ and R² can be added to the ring after conversion of the compound of formula XII to the compounds of formulae I-a, I-b, I-c or I-d. Hence, all of the reactions described to produce various substituents of R¹ and R² in the compound of formula I can also be carried out on the compounds of formulae I-a, I-b I-c or I-d.

In the first step of this Reaction Scheme, the alkyl halide of formula VI is reacted with the compound of formula V, to produce the compound of formula VII. In this reaction, if in the compounds of formula V, R¹ or R² is an amino group, such amino group(s) have to be protected before carrying out the alkylation reaction with the alkyl halide of formula VI. The amino group can be protected with any conventional acid removable group (for example, for t-butyloxycarbonyl group see, Bodanszky, M. *Principles of Peptide Chemistry, Springer -Verlag, New York*, **1984**, p 99). The protecting group has to be removed from the amino groups after preparing the corresponding amine protected compounds of formulae I-a, I-b, I-c or I-d to obtain the corresponding amines. The compound of formula V is an organic acid having an alpha carbon atom and the compound of formula VI is an alkyl halide so that alkylation occurs at the alpha carbon atom of this carboxylic acid. This reaction is carried out by any conventional means of alkylation of the alpha carbon atom of a lower alkyl ester of a carboxylic acid. Generally, in these alkylation reactions any alkyl halide is reacted with the anion generated from any acetic acid ester. The anion can be generated by using a strong organic base such as lithium diisopropylamide, *n*-butyl lithium as well as other organic lithium bases. In carrying out this reaction, low boiling ether solvents are utilized such as tetrahydrofuran at low temperatures from -80°C to about -10°C being preferred. However, any temperature from -80°C to room temperature can be used.

The compound of formula VII can be converted to the compound of formula XII by any conventional procedure to convert a carboxylic acid ester to an acid. The compound of formula XIII can then be condensed with the compound of formula VIII *via* conventional peptide coupling to produce the compound of formula I-d. In carrying out this reaction, any conventional method of condensing a primary amine with a carboxylic acid can be utilized to effect this conversion. The required amino heteroaromatic compounds of formula VIII, are commercially available or can be prepared from the reported literature. The heteroaromatics of formula VIII, wherein one of the substitutions is -(CH₂)ₙCOOR⁷, where n = 0, 1, 2, 3, or 4 can be prepared from the corresponding carboxylic acid. Any conventional carbon homologation methods to convert a lower carboxylic acid to its higher homologs, (see for example, Skeean, R. W.; Goel, O. P. *Synthesis*, **1990**, 628) which then can be converted to the corresponding lower alkyl esters using any conventional esterification methods can be utilized. The heteroaromatics of formula VIII, wherein one of the claimed substitutions is -(CH₂)ₙOR⁶, where n = 0, 1, 2, 3, or 4 can be prepared from the corresponding carboxylic acid. Any conventional carbon homologation methods to convert a lower carboxylic acid to its higher homologs, which then can be converted to the corresponding alcohols using any conventional ester reduction methods can be utilized. The heteroaromatics of formula VIII, wherein one of the substituents is -COCOOR⁸, can be prepared from the corresponding halogen. Any conventional acylation method to convert an aromatic or heteroaromatic halogen to its oxoacetic acid lower ester derivative (see for example, Hayakawa, K.; Yasukouchi, T.; Kanematsu, K. *Tetrahedron Lett*, **1987**, *28*, 5895) can be utilized.

On the other hand, the carboxylic acid of the formula XII can be converted to the amide of the formula IX. This reaction is carried out by using conventional means for converting the acid of formula XII to an acid chloride and thereafter treating this acid chloride with ammonia or an ammonia-producing compound such as hexamethyl disilazane. Conditions which are conventional for converting an acid to an acid chloride can be utilized in this procedure. This acid chloride when reacted under conventional conditions with ammonia as described will produce the amide of formula IX. The compound of formula IX when reacted with an alkyl, alkenyl, or -(CH₂)ₙC(O)OR⁵ isocyanate of formula X forms the urea adduct of formula I-a. Any conventional method of reacting alkyl, alkenyl, or -(CH₂)ₙC(O)OR⁵ isocyanate with an amide to form a urea linkage can be utilized to form the compound of formula I-a.

When R⁴¹ is a lower alkenyl group in the compound of formula I-a, this compound can be converted to the corresponding hydroxy lower alkyl group by conventional hydroboration at the olefinic group to produce a corresponding hydroxy group. The hydroxy group, if desired, can be converted to a halo group. Any method of halogenating a hydroxy group can be used in accordance with this invention.

On the other hand, if it is desired to produce the compound of formula I-b the compound of formula XII is first converted to the methyl ester of formula XI, and thereafter reacted with urea to produce the compound of forumula I-b. This reaction is carried out by utilizing any conventional means of reacting a methyl ester with urea to form the corresponding condensation product.

The compound of formula I-c, i.e. the compound of formula I wherein R⁴ -C(O)NHR⁴⁰ and R⁴⁰ is -CO-(CH₂)ₙ-C(O)OR⁶, is produced from the monoacid chloride XIII of the monoester of the corresponding dicarboxylic acid. The monoacid chloride XIII is coupled with the compounds of formula I-b using standard coupling methods.

The compound of formula VII has an asymmetric carbon atom through which the group -CH₂R³ and the acid amide substituents are connected. In accordance with this invention, the preferred stereoconfiguration of this group is R.

If it is desired to produce the R or the S isomer of the compound of formula I, this compound can be separated into these isomers by any conventional chemical means. Among the preferred chemical means is to react the compound of formula XII with an optically active base. Any conventional optically active base can be utilized to carry out this resolution. Among the preferred optically active bases are the optically active amine bases such as alpha-methylbenzylamine, quinine, dehydroabietylamine and alpha-methylnaphthylamine. Any of the conventional techniques utilized in resolving organic acids with optically active organic amine bases can be utilized in carrying out this reaction.

In the resolution step, the compound of formula XII is reacted with the optically active base in an inert organic solvent medium to produce salts of the optically active amine with both the R and S isomers of the compound of formula XII. In the formation of these salts, temperatures and pressure are not critical and the salt formation can take place at room temperature and atmospheric pressure. The R and S salts can be separated by any conventional method such as fractional crystallization. After crystallization, each of the salts can be converted to the respective compounds of formula XII in the R and S configuration by hydrolysis with an acid. Among the preferred acids are dilute aqueous acids, i.e., from about 0.001N to 2N aqueous acids, such as aqueous sulfuric or aqueous hydrochloric acid. The configuration of formula XII which is produced by this method of resolution is carried out throughout the entire reaction scheme to produce the desired R or S isomer of formula I. The separation of R and S isomers can also be achieved using an enzymatic ester hydrolysis of any lower alkyl esters corresponding to the compound of the formula XII (see for example, Ahmar, M.; Girard, C.; Bloch, R, *Tetrahedron Lett*, **1989**, 7053), which results in the formation of corresponding chiral acid and chiral ester. The ester and the acid can be separated by any conventional method of separating an acid from an ester. The preferred method of resolution of racemates of the compounds of the formula XII is via the formation of corresponding diastereomeric esters or amides. These diastereomeric esters or amides can be prepared by coupling the carboxylic acids of the formula XII with a chiral alcohol, or a chiral amine. This reaction can be carried out using any conventional method of coupling a carboxylic acid with an alcohol or an amine. The corresponding diastereomers of compounds of the formula XII can then be separated using any conventional separation methods. The resulting pure diastereomeric esters or amides can then be hydrolyzed to yield the corresponding pure R or S isomers. The hydrolysis reaction can be carried out using conventional known methods to hydrolyze an ester or an amide without racemization.

All of the compounds of formula I which include the compounds set forth in the Examples, activated glucokinase *in vitro* by the procedure of Example A. In this manner, they increase the flux of glucose metabolism which causes increased insulin secretion. Therefore, the compounds of formula I are glucokinase activators useful for increasing insulin secretion.

The following compounds were tested and found to have excellent glucokinase activator *in vivo* activity when administered orally in accordance with the assay described in Example B:
3-Cyclopentyl-2-(4-methanesulfonyl-phenyl)-N-thiazol-2-yl-propionamide;
3-Cyclopentyl-N-thiazol-2-yl-2-(4-trifluoromethoxy-phenyl)-propionamide;
3-Cyclopentyl-N-thiazol-2-yl-2-(4-trifluoromethanesulfonyl-phenyl)-propionamide;
3-Cyclopentyl-2(R)-(3,4-dichloro-phenyl)-N-pyridin-2-yl-propionamide;
6-[3-Cyclopentyl-2(R)-(3,4-dichloro-phenyl)-prapionylamino]-nicotinic acid methyl ester;
N-(5-Chloro-pyridin-2-yl)-3-cyclopentyl-2(R)-(3,4-dichloro-phenyl)-propionamide;
3-Cyclopentyl-N-pyridin-2-yl-2-(4-trifluoromethanesulfonyl-phenyl)-propionamide;
3-Cyclopentyl-N-(5-methyl-pyridin-2-yl)-2-(4-trifluoromethanesulfonyl-phenyl)-propionamide;
3-Cyclopentyl-2(R)-(3,4-dichloro-phenyl)-N-(5-hydroxymethyl-pyridin-2-yl) propionamide;
6-[3-Cyclopentyl-2-(4-trifluoromethanesulfonyl-phenyl)-propionylamino]-nicotinic acid methyl ester;
3-Cyclopentyl-2-(3-fluoro-4-trifluoromethyl-phenyl)-N-pyridin-2-yl-propionamide;
3-Cyclopentyl-2-(4-methanesulfonyl-3-nitrophenyl)-N-pyridin-2-yl-propionamide;
2-(3-Bromo-4-methanesulfonyl-phenyl)-3-cyclopentyl-N-pyridin-2-yl-propionamide;
2-(3-Cyano-4-methanesulfonyl-phenyl)-3-cyclopentyl-N-pyridin-2-yl-propionamide;
2-(4-Chloro-3-nitro-phenyl)-3-cyclopentyl-N-pyridin-2-yl-propionamide;
2-(3-Chloro-4-methanesulfonyl-phenyl)-3-cyclopentyl-N-pyridin-2-yl-propionamide;
N-(5-Bromo-pyridin-2-yl)-2-(3-chloro-4-methanesulfonyl-phenyl)-3-cyclopentylpropionamide;
2-[3-Chloro-4-methanesulfonyl-phenyl]-3-cyclopentyl-N-thiazol-2-yl-propionamide;
(2R)-3-Cyclopentyl-2-(4-methanesulfonylphenyl)-N-thiazol-2-yl-propionamide;
2-(3-Bromo-4-methanesulfonyl-phenyl)-3-cyclopentyl-N-thiazol-2-yl-propionamide;
2-(3-Cyano-4-methanesulfonyl-phenyl)-3-cyclopentyl-N-thiazol-2-yl-propionamide;
3-Cyclopentyl-2-(4-ethanesulfonyl-phenyl)-N-thiazol-2-yl-propionamide;
3-Cyclopentyl-2-(4-methanesulfonyl-3-trifluoromethyl-phenyl)-N-thiazol-2-yl-propionamide; and
N-(5-Bromo-pyridin-2-yl)-2(R)-(3-chloro-4-methanesulfonyl-phenyl)-3-cyclopentylpropionamide.

Furthermore, the following compounds were tested and found to have excellent glucokinase activator *in vivo* activity when administered orally in accordance with the assay described in Example B:
1-[3-cyclopentyl-2(R)-(3,4-dichloro-phenyl)-propionyl]-3-ethyl-urea;
1-[3-cyclopentyl-2(R)-(3,4-dichloro-phenyl)-propionyl]-3-methyl-urea;
1-[3-cyclopentyl-2-(3,4-dichloro-phenyl)-propionyl]-3-methyl urea;
1-[3-cyclopentyl-2-(4-methanesulfonyl-phenyl)-propionyl]-3-methyl urea;
1-Allyl-3-[3-cyclopentyl-2(R)-(3,4-dichloro-phenyl)-propionyl]-urea;
1-[2-(3-Chloro-4-methanesulfonyl-phenyl)-3-cyclopentyl-propionyl]-3-methyl-urea;
1-[2-(3-Bromo-4-methanesulfonyl-phenyl)-3-cyclopentyl-propionyl]-3-methyl-urea; and
1-[2(R)-(3-Chloro-4-methanesulfonyl-phenyl)-3-cyclopentyl-propionyl]-3-methyl-urea.

On the basis of their capability of activating glucokinase, the compounds of formula I can be used as medicaments for the treatment of type II diabetes. Therefore, as mentioned earlier, medicaments containing a compound of formula I are also an object of the present invention, as is a process for the manufacture of such medicaments, which process comprises bringing one or more compounds of formula I and, if desired, one or more other therapeutically valuable substances into a galenical administration form.

The pharmaceutical compositions may be administered orally, for example in the form of tablets, coated tablets, dragées, hard or soft gelatine capsules, solutions, emulsions or suspensions. Administration can also be carried out rectally, for example using suppositories; locally or percutaneously, for example using ointments, creams, gels or solutions; or parenterally, e.g. intravenously, intramuscularly, subcutaneously, intrathecally or transdermally, using for example injectable solutions. Furthermore, administration can be carried out sublingually or as an aerosol, for example in the form of a spray. For the preparation of tablets, coated tablets, dragées or hard gelatine capsules the compounds of the present invention may be admixed with pharmaceutically inert, inorganic or organic excipients. Examples of suitable excipients for tablets, dragées or hard gelatine capsules include lactose, maize starch or derivatives thereof, talc or stearic acid or salts thereof. Suitable excipients for use with soft gelatine capsules include for example vegetable oils, waxes, fats, semi-solid or liquid polyols etc.; according to the nature of the active ingredients it may however be the case that no excipient is needed at all for soft gelatine capsules. For the preparation of solutions and syrups, excipients which may be used include for example water, polyols, saccharose, invert sugar and glucose. For injectable solutions, excipients which may be used include for example water, alcohols, polyols, glycerine, and vegetable oils. For suppositories, and local or percutaneous application, excipients which may be used include for example natural or hardened oils, waxes, fats and semi-solid or liquid polyols.

The pharmaceutical compositions may also contain preserving agents, solubilising agents, stabilising agents, wetting agents, emulsifiers, sweeteners, colorants, odorants, salts for the variation of osmotic pressure, buffers, coating agents or antioxidants. As mentioned earlier, they may also contain other therapeutically valuable agents. It is a prerequisite that all adjuvants used in the manufacture of the preparations are non-toxic.

Preferred forms of use are intravenous, intramuscular or oral administration, most preferred is oral administration. The dosages in which the compounds of formula (I) are administered in effective amounts depend on the nature of the specific active ingredient, the age and the requirements of the patient and the mode of application. In general, dosages of about 1-100 mg/kg body weight per day come into consideration.

This invention will be better understood from the following examples, which are for purpose of illustration and are not intended to limit the invention defined in the claims that follow thereafter.

### Example 1

### (A) 3-Cyclopentyl-2-(3,4-dichlorophenyl)-N-thiazol-2-yl-propionamide:

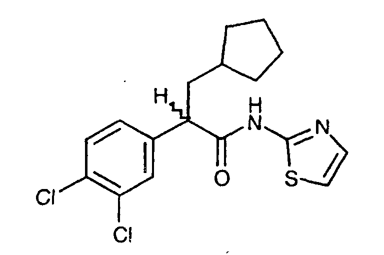

A solution of 3-cyclopentyl-2-(3,4-dichlorophenyl)-propionic acid (prepared in Example **38**, 2.0 g, 6.96 mmol), benzotriazol-1-yloxy-tris(dimethylamino)phosphonium hexafluorophosphate (4.62 g, 10.44 mmol), and 2-aminothiazole (1.05 g, 10.44 mmol) in methylene chloride (50 mL) at 25°C was treated with triethylamine (2.9 mL, 20.88 mmol). The reaction mixture was stirred for 14 h. The reaction mixture was then diluted with water (10 mL) and extracted with methylene chloride (3 x 10 mL). The combined organic layers were sequentially washed with water (1 x 10 mL), a 1N aqueous sodium hydroxide solution (1 x 10 mL), a 1N aqueous hydrochloric acid solution (1 x 10 mL), and a saturated aqueous sodium chloride solution (1 x 10 mL). The organic layer was dried over sodium sulfate, filtered, and concentrated *in vacuo.* Flash chromatography (Merck Silica gel 60, 230-400 mesh, 80/20 hexanes/ethyl acetate) afforded 3-cyclopentyl-2-(3,4-dichlorophenyl)-N-thiazol-2-yl-propionamide (2.48 g, 96%) as a white solid: mp 143.5-145.5°C; EI-HRMS m/e calcd for C₁₇H₁₈Cl₂N₂OS (M⁺) 368.0516, found 368.0516.

### (B) In an analogous manner, there were obtained:

(a) From 3-cyclopentyl-2-(3,4-dichlorophenyl)-propionic acid and 2-(amino-thiazol-4-yl)-oxo-acetic acid ethyl ester: {2-[3-Cyclopentyl-2-(3,4-dichloro-phenyl)-propionylamino]-thiazol-4-yl}-oxo-acetic acid ethyl ester as a white solid: mp 134-136°C; FAB-HRMS m/e calcd for C₂₁H₂₂Cl₂N₂O₄S (M+H)⁺ 469.0755, found 469.0746.
(b) From 3-cyclopentyl-2-(3,4-dichlorophenyl)-propionic acid and 2-(amino-thiazol-5-yl)-oxo-acetic acid ethyl ester: {2-[3-Cyclopentyl-2-(3,4-dichlorophenyl)-propionylamino]-thiazol-5-yl}-oxo-acetic acid ethyl ester as a white solid: mp 129-131°C; FAB-HRMS m/e calcd for C₂₁H₂₂Cl₂N₂O₄S (M+H)⁺ 469.0755, found 469.0765.
(c) From 3-cyclopentyl-2-(3,4-dichlorophenyl)-propionic acid and (2-amino-thiazol-4-yl)-acetic acid ethyl ester: {2-[3-Cyclopentyl-2-(3,4-dichlorophenyl)-propionylamino]-thiazol-4-yl }-acetic acid ethyl ester as a yellow solid: mp 138-139°C; FAB-HRMS m/e calcd for C₂₁H₂₄Cl₂N₂O₃S (M+H)⁺ 455.0963, found 455.0960.
(d) From 3-cyclopentyl-2-(3,4-dichlorophenyl)-propionic acid and 2-amino-5-methylthiazole: 3-Cyclopentyl-2-(3,4-dichlorophenyl)-N-(5-methyl-thiazol-2-yl)-propionamide as a white solid: mp 142-143°C; EI-HRMS m/e calcd for C₁₈H₂₀Cl₂N₂OS (M⁺) 382.0673, found 382.0679.
(e) From 3-cyclopentyl-2-(3,4-dichlorophenyl)-propionic acid and 2-amino-4-methylthiazole: 3-Cyclopentyl-2-(3,4-dichlorophenyl)-N-(4-methyl-thiazol-2-yl)-propionamide as a white foam: mp 151-152°C; FAB-HRMS m/e calcd for C₁₈H₂₀Cl₂N₂OS (M+H)⁺ 383.0751, found 383.0758.
(f) From 3-cyclopentyl-2-(3,4-dichlorophenyl)-propionic acid and 2-amino-thiazole-4-carboxylic acid ethyl ester: 2-[3-Cyclopentyl-2-(3,4-dichlorophenyl)-propionylamino]-thiazole-4-carboxylic acid ethyl ester as a white solid: mp 104-107°C; FAB-HRMS m/e calcd for C₂₀H₂₂Cl₂N₂O₃S (M+H)⁺ 441.0807, found 441.0808.
(g) From 3-cyclopentyl-2-(3,4-dichlorophenyl)-propionic acid and 2-amino-thiazole-5-carboxylic acid ethyl ester: 2-[3-Cyclopentyl-2-(3,4-dichlorophenyl)-propionylamino]-thiazole-5-carboxylic acid ethyl ester as a light yellow solid: mp 136-137°C; FAB-HRMS m/e calcd for C₂₀H₂₂Cl₂N₂O₃S (M+H)⁺ 441.0807, found 441.0803.
(h) From 3-cyclopentyl-2-(3,4-dichlorophenyl)-propionic acid and 2-amino-5-nitrothiazole: 3-Cyclopentyl-2-(3,4-dichlorophenyl)-N-(5-nitro-thiazol-2-yl)-propionamide as an orange solid: mp 67-71°C; FAB-HRMS m/e calcd for C₁₇H₁₇Cl₂N₃O₃S (M+H)⁺ 414.0446, found 414.0442.
(i) From 3-cyclopentyl-2-(3,4-dichlorophenyl)-propionic acid and 2-amino-thiazole-4-carboxylic acid amide: 2-[3-Cyclopentyl-2-(3,4-dichlorophenyl)-propionylamino]-thiazole-4-carboxylic acid amide as a light orange solid: mp 120-122°C; EI-HRMS m/e calcd for C₁₈H₁₉Cl₂N₃O₂S (M⁺) 411.0575, found 411.0572.

### Example 2

### 2-(4-Bromo-phenyl)-3-cyclopentyl-N-thiazol-2-yl-propionamide

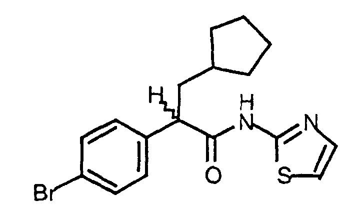

A solution of diisopropylamine (7.7 mL, 54.88 mmol) in dry tetrahydrofuran (23 mL) and 1,3-dimethyl-3,4,5,6-tetrahydro-2(1H)-pyrimidinone (10 mL) was cooled to -78°C under nitrogen and then treated with a 2.5M solution of *n*-butyllithium in hexanes (22.0 mL, 54.88 mmol). The resulting reaction mixture was stirred at -78°C for 30 min and then treated dropwise with a solution of 4-bromophenylacetic acid (5.62 g, 26.13 mmol) in dry tetrahydrofuran (23 mL) and 1,3-dimethyl-3,4,5,6-tetrahydro-2(1H)-pyrimidinone (10 mL). The reaction mixture turned dark in color and was allowed to stir at -78°C for 1 h, at which time, a solution of iodomethylcyclopentane (5.76 g, 27.44 mmol) in a small amount of dry tetrahydrofuran was added dropwise. The reaction mixture was allowed to warm to 25°C where it was stirred for 24 h. The reaction mixture was quenched with water and then concentrated *in vacuo* to remove tetrahydrofuran. The aqueous residue was acidified using a 10% aqueous hydrochloric acid solution. The resulting aqueous layer was extracted with ethyl acetate (2 x 100 mL). The combined organic extracts were dried over sodium sulfate, filtered, and concentrated *in vacuo*. Flash chromatography (Merck Silica gel 60, 230-400 mesh, 3/1 hexanes/ethyl acetate) afforded 2-(4-bromo-phenyl)-3-cyclopentyl-propionic acid (3.88 g, 50%) as a light yellow solid: mp 91-93°C; EI-HRMS m/e calcd for C₁₄H₁₇BrO₂ (M⁺) 296.0412, found 296.0417.

A solution of 2-(4-bromo-phenyl)-3-cyclopentyl-propionic acid (1.01 g, 3.39 mmol) in methylene chloride (8.5 mL) was treated with 2 drops of dry *N*,*N*-dimethylformamide. The reaction mixture was cooled to 0°C and then treated with oxalyl chloride (3 mL, 33.98 mmol). The reaction mixture was stirred at 0°C for 10 min and then stirred at 25°C for 15 h. The reaction mixture was concentrated *in vacuo*. The resulting yellow oil was dissolved in a small amount of methylene chloride and slowly added to a cooled solution (0°C) of 2-aminothiazole (680.6 mg, 6.79 mmol) and *N*,*N*-diisopropylethylamine (1.2 mL, 6.79 mmol) in methylene chloride (17 mL). The resulting reaction mixture was stirred at 0°C for 10 min and then at 25°C for 15 h. The reaction mixture was concentrated *in vacuo* to remove methylene chloride. The resulting residue was diluted with ethyl acetate (200 mL). The organic phase was washed with a 10% aqueous hydrochloric acid solution (2 x 100 mL), washed with a saturated aqueous sodium bicarbonate solution (2 x 100 mL), and washed with a saturated aqueous sodium chloride solution (1 x 100 mL). The organic layer was then dried over sodium sulfate, filtered, and concentrated *in vacuo* to afford 2-(4-bromo-phenyl)-3-cyclopentyl-N-thiazol-2-yl-propionamide (1.23 g, 95%) as an orange solid which was used in subsequent reactions without further purification. An analytical sample was recrystallized from ethyl acetate to provide a cream solid: mp 201-202°C; EI-HRMS m/e calcd for C₁₇H₁₉BrN₂OS (M⁺) 378.0401, found 378.0405.

### Example 3

### (A) 3-Cyclopentyl-2-(4-methanesulfonyl-phenyl)-N-thiazol-2-yl-propionamide

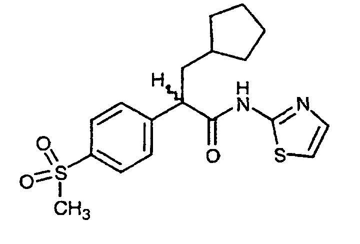

A solution of diisopropylamine (3.3 mL, 23.5 mmol) in dry tetrahydrofuran (50 mL) and 1,3-dimethyl-3,4,5,6-tetrahydro-2(1H)-pyrimidinone (10 mL) was cooled to -78°C under nitrogen and then treated with a 10M solution of *n*-butyllithium in hexanes (2.35 mL, 23.5 mmol). The yellow reaction mixture was stirred at -78°C for 30 min and then treated dropwise with a solution of 4-methylsulfonylphenylacetic acid (2.40 g, 11.2 mmol) in a small amount of dry tetrahydrofuran. After approximately one-half of the 4-methylsulfonylphenylacetic acid in dry tetrahydrofuran was added, a precipitate formed. Upon further addition of the remaining 4-methylsulfonylphenylacetic acid in dry tetrahydrofuran, the reaction mixture became thick in nature. After complete addition of the 4-methylsulfonylphenylacetic acid in dry tetrahydrofuran, the reaction mixture was very thick and became difficult to stir. An additional amount of dry tetrahydrofuran (20 mL) was added to the thick reaction mixture, and the reaction mixture was stirred at - 78°C for 45 min, at which time, a solution of iodomethylcyclopentane (2.35 g, 11.2 mmol) in a small amount of dry tetrahydrofuran was added dropwise. The reaction mixture was allowed to warm to 25°C where it was stirred for 15 h. The reaction mixture was quenched with water (100 mL), and the resulting yellow reaction mixture was concentrated *in vacuo* to remove tetrahydrofuran. The aqueous residue was acidified to pH = 2 using concentrated hydrochloric acid. The aqueous layer was extracted with ethyl acetate. The organic phase was dried over magnesium sulfate, filtered, and concentrated *in vacuo*. Flash chromatography (Merck Silica gel 60, 230-400 mesh, 1/3 hexanes/ethyl acetate) afforded 3-cyclopentyl-2-(4-methanesulfonyl-phenyl)propionic acid (1.80 g, 52%) as a white solid: mp 152-154°C; EI-HRMS m/e calcd for C₁₅H₂₀O₄S (M⁺) 296.1082, found 296.1080.

A solution of 3-cyclopentyl-2-(4-methanesulfonyl-phenyl)propionic acid (4.91 g, 16.56 mmol) and triphenylphosphine (6.52 g, 24.85 mmol) in methylene chloride (41 mL) was cooled to 0°C and then treated with *N*-bromosuccinimide (5.01 g, 28.16 mmol) in small portions. The reaction mixture color changed from light yellow to a darker yellow then to brown. After the complete addition of *N*-bromosuccinimide, the reaction mixture was allowed to warm to 25°C over 30 min. The brown reaction mixture was then treated with 2-aminothiazole (4.98 g, 49.69 mmol). The resulting reaction mixture was stirred at 25°C for 19 h. The reaction mixture was then concentrated *in vacuo* to remove methylene chloride. The remaining black residue was diluted with a 10% aqueous hydrochloric acid solution (400 mL) and then extracted with ethyl acetate (3 x 200 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 3/1 hexanes/ethyl acetate then 1/1 hexanes/ethyl acetate) afforded 3-cyclopentyl-2-(4-methanesulfonyl-phenyl)-N-thiazol-2-yl-propionamide (4.49 g, 72%) as a white solid: mp 216-217°C; EI-HRMS m/e calcd for C₁₈H₂₂N₂O₃S₂ (M⁺) 378.1072, found 378.1071.

### (B) In an analogous manner, there were obtained:

(a) From 3-cyclopentyl-2-(4-methanesulfonyl-phenyl)propionic acid and 2-aminothiazole-4-carboxylic acid methyl ester: 2-[3-Cyclopentyl-2-(4-methanesulfonylphenyl)-gropionylamino]-thiazole-4-carboxylic acid methyl ester as a tan solid: mp 126-128°C; EI-HRMS m/e calcd for C₂₀H₂₄N₂O₅S₂ (M⁺) 436.1127, found 436.1119.
(b) From 3-cyclopentyl-2-(4-methanesulfonyl-phenyl)propionic acid and 2-aminothiazole-4-carboxylic acid ethyl ester: 2-[3-Cyclopentyl-2-(4-methanesulfonylphenyl)-propionylamino]-thiazole-4-carboxylic acid ethyl ester as a light yellow solid: mp 101-103°C; EI-HRMS m/e calcd for C₂₁H₂₆N₂O₅S₂ (M⁺) 450.1283, found 450.1284.
(c) From 3-cyclopentyl-2-(4-methanesulfonyl-phenyl)propionic acid and methyl 2-amino-4 thiazoleacetate: {2-[3-Cyclopentyl-2-(4-methanesulfonyl-phenyl)-propionylamino]-thiazol-4-yl}-acetic acid methyl ester as a yellow solid: mp 63-65°C; EI-HRMS m/e calcd for C₂₁H₂₆N₂O₅S₂ (M⁺) 450.1283, found 450.1294.
(d) From 3-cyclopentyl-2-(4-methanesulfonyl-phenyl)propionic acid and ethyl 2-amino-4-thiazoleacetate: {2-[3-Cyclopentyl-2-(4-methanesulfonyl-phenyl)-propionylamino]-thiazol-4-yl}-acetic acid ethyl ester as a light yellow solid: mp 61-63°C; EI-HRMS m/e calcd for C₂₂H₂₈N₂O₅S₂ (M⁺) 464.1440, found 464.1431.

### Example 4

### 2-(4-Amino-phenyl)-3-cyclopentyl-N-thiazol-2-yl-propionamide

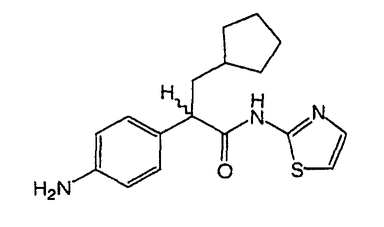

A solution of 3-cyclopentyl-2-(4-nitro-phenyl)-N-thiazol-2-yi-propionamide (prepared in Example **22**, 345 mg, 1.0 mmol) in ethyl acetate (100 mL) was treated with 10% palladium on activated carbon (34.5 mg). The reaction mixture was stirred under hydrogen gas at 60 psi at 25°C for 6 h. The catalyst was then filtered off through a pad of celite (ethyl acetate). The filtrate was concentrated *in vacuo* to give 2-(4-amino-phenyl)-3-cyclopentyl-N-thiazol-2-yl-propionamide (288.3 mg, 91.4%) as a yellow solid: mp 102-107°C; EI-HRMS m/e calcd for C₁₇H₂₁N₃OS (M⁺) 315.1405, found 315.1401.

### Example 5

### 2-(3-Amino-phenyl)-3-cyclopentyl-N-thiazol-2-yl-propionamide

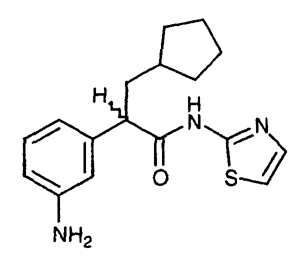

A solution of (3-nitro-phenyl)-acetic acid (5.0 g, 27.6 mmol) in methanol (50 mL) was treated with a catalytic amount of sulfuric acid. The reaction mixture was refluxed for 48 h. The reaction was then concentrated *in vacuo*. The residue was dissolved in methylene chloride (50 mL) and washed with a saturated aqueous sodium bicarbonate solution (2 x 25 mL), water (1 x 50 mL), and a saturated aqueous sodium chloride solution (1 x 50 mL). The combined organic layers were dried over sodium sulfate, filtered, and concentrated *in vacuo* to give (4-nitro-phenyl)-acetic acid methyl ester (5.27 g, 97.9%) as a pale yellow solid: mp 29-30°C; EI-HRMS m/e calcd for C₉H₉NO₄ (M⁺) 195.0531, found 195.0532.

A solution of freshly prepared lithium diisopropylamide (43.3 mL of a 0.3M stock solution, 12.99 mmol) cooled to -78°C was treated with (3-nitro-phenyl)-acetic acid methyl ester (2.45 g, 12.56 mmol) in tetrahydrofuran/1,3-dimethyl-3,4,5,6-tetrahydro-2(1H)-pyrimidinone (32 mL, 3:1). The resulting solution was stirred at -78°C for 45 min. Iodomethylcyclopentane (2.78 g, 13.23 mmol) was then added in 1,3-dimethyl-3,4,5,6-tetrahydro-2(1H)-pyrimidinone (2.78 mL), and the mixture was stirred at -78°C for 3 h. The reaction was warmed to 25°C and was stirred at 25°C for 16 h. The reaction mixture was then quenched by the dropwise addition of a saturated aqueous ammonium chloride solution (25 mL) and was concentrated *in vacuo*. The residue was diluted with water (50 mL) and extracted with ethyl acetate (3 x 50 mL). The organics were washed with a saturated aqueous lithium chloride solution (2 x 25 mL), dried over sodium sulfate, filtered, and concentrated *in vacuo*. Flash chromatography (Merck Silica gel 60, 230-400 mesh 80/20 hexanes/ethyl acetate) afforded 3-cyclopentyl-2-(3-nitro-phenyl)-propionic acid methyl ester (1.63 g, 46.8%) as pale yellow oil: EI-HRMS m/e calcd for C₁₅H₁₉NO₄ (M⁺) 277.1314, found 277.1317.

A solution of 3-cyclopentyl-2-(3-nitro-phenyl)-propionic acid methyl ester (0.55 g, 2.0 mmol) in tetrahydrofuran/water (10 mL, 3:1) was treated with lithium hydroxide (185 mg, 4.40 mmol). The reaction was stirred at 25°C for 48 h. The tetrahydrofuran was then removed *in vacuo*. The residue was diluted with water (25 mL) and extracted with ether (1 x 20 mL). The aqueous layer was acidified to pH = 2 with a 3N aqueous hydrochloric acid solution. The product was extracted into methylene chloride (3 x 25 mL), washed with a saturated aqueous sodium chloride solution (2 x 25 mL), dried over sodium sulfate, filtered, and concentrated *in vacuo* to give 3-cyclopentyl-2-(3-nitro-phenyl)-propionic acid (0.48 g, 91.9%) as a tan solid: mp 95-99°C; EI-HRMS m/e calcd for C₁₄H₁₇NO₄ (M⁺) 263.1157, found 263.1156.

A solution of 3-cyclopentyl-2-(3-nitro-phenyl)-propionic acid (432 mg, 1.64 mmol) in methylene chloride (16 mL) was cooled to 0°C and then treated with a 2.0M solution of oxalyl chloride in methylene chloride (0.90 mL, 1.80 mmol) and a few drops of *N*,*N*-dimethylformamide. The reaction mixture was stirred at 0°C for 15 min and at 25°C for 1.2 h. The reaction mixture was then treated with a solution of 2-aminothiazole (361.4 mg, 3.61 mmol) in tetrahydrofuran (16 mL) and *N*,*N*-diisopropylethylamine (0.70 mL, 3.93 mmol). The reaction mixture was stirred at 25°C for 6 h. At this time, the reaction was concentrated *in vacuo*. Flash chromatography (Merck Silica gel 60, 230-400 mesh 70/30 hexanes/ethyl acetate) afforded 3-cyclopentyl-2-(nitrophenyl)-N-thiazol-2-yl-propionamide (409.3 mg, 72.2%) as a tan solid: mp 171-174°C; EI-HRMS m/e,calcd for C₁₇H₁₉N₃O₃S (M⁺) 345.1147, found 345.1153.

A solution of 3-cyclopentyl-2-(nitrophenyl)-N-thiazol-2-yl-propionamide (327.8 mg, 0.95 mmol) in ethyl acetate (25 mL) was treated with 10% palladium on activated carbon. The reaction mixture was stirred under hydrogen gas at 60 psi at 25°C for 3 h. The catalyst was then filtered off through a pad of celite (ethyl acetate). The filtrate was concentrated *in vacuo* to give 2-(3-amino-phenyl)-3-cyclopentyl-N-thiazol-2-yl-propionamide (310 mg, 100%) as a white solid: mp 158-160°C; EI-HRMS m/e calcd for C₁₇H₂₁N₃OS (M⁺) 315.1405, found 315.1405.

### Example 6

### 2-(3-Chloro-phenyl)-3-cyclopentyl-N-thiazol-2-yl-propionamide

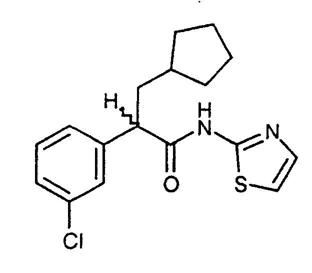

(3-Chloro-phenyl)-acetic acid (6.03 g, 0.03 mol) was dissolved in ethanol (37.7 mL) and treated with a catalytic amount of sulfuric acid. The reaction mixture was refluxed for 12 h. The reaction was concentrated *in vacuo*. Flash chromatography (Merck Silica gel 60, 230-400 mesh, 50/50 hexanes/ethyl acetate) afforded (3-chloro-phenyl)-acetic acid ethyl ester (6.10 g, 86.8%) as a clear oil: EI-HRMS m/e calcd for C₁₀H₁₁ClO₂ (M⁺) 198.0448, found 198.0442.

A solution of freshly prepared lithium diisopropylamide (23 mL of 0.31M stock solution, 7.13 mmol) cooled to -78°C was treated with (3-chloro-phenyl)-acetic acid ethyl ester (1.28 g, 6.48 mmol) in tetrahydrofuran/hexamethylphosphoramide (16.1 mL, 3:1). The resulting solution was stirred at -78°C for 45 min. At this time, the reaction was treated with a solution of iodomethylcyclopentane (1.50 g, 7.13 mmol) in hexamethylphosphoramide (1 mL). The reaction mixture was stirred at -78°C for 4 h. The reaction was warmed to 25°C and stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 2-(3-chloro-phenyl)-3-cyclopentyl-propionic acid ethyl ester (1.70 g, 93%) as a yellow oil: EI-HRMS m/e calcd for C₁₆H₂₁ClO₂ (M⁺) 280.1230, found 280.1238.

A mixture of 2-(3-chloro-phenyl)-3-cyclopentyl-propionic acid ethyl ester (1.70 g, 6.05 mmol) and methyl urea (673 mg, 9.08 mmol) in a solution of magnesium methoxide in methanol (7.4 wt%, 17.3 mL, 12.1 mmol) was refluxed at 100°C for 6 h. The reaction mixture was then concentrated *in vacuo*. Flash chromatography (Merck Silica gel 60, 230-400 mesh, 75/25 hexanes/ethyl acetate) afforded 1-[2-(3-chloro-phenyl)-3-cyclopentyl-propionyl]-3-methyl-urea (149.1 mg, 8 %) as a white solid: mp 52-55°C; EI-HRMS m/e calcd for C₁₆H₂₁ClN₂O₂ (M⁺) 308.1292, found 308.1287. The methyl ester of the starting material was recovered from the reaction mixture due to transesterification.

A mixture of 2-(3-chloro-phenyl)-3-cyclopentyl-propionic acid methyl ester (113 mg, 0.42 mmol) and 2-aminothiazole (84 mg, 0.84 mmol) in a solution of magnesium methoxide in methanol (7.4 wt%, 2.4 mL, 1.69 mmol) was refluxed at 100°C for 20 h. The reaction mixture was then concentrated *in vacuo*. Flash chromatography (Merck Silica gel 60, 230-400 mesh, 80/20 hexanes/ethyl acetate) afforded 2-(3-chloro-phenyl)-3-cyclopentyl-N-thiazol-2-yl-propionamide (87 mg, 53%) as a white solid: mp 138.8-141.2°C; EI-HRMS m/e calcd for C₁₇H₁₉ClN₂OS (M⁺) 334.0906, found 334.0907.

### Example 7

### 2-(4-Chloro-phenyl)-3-cyclopentyl-N-thiazol-2-yl-propionamide

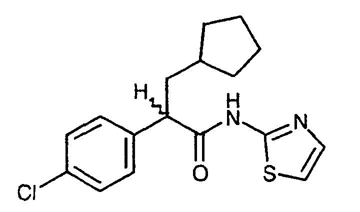

A solution of (4-chloro-phenyl)-acetic acid (6.29 g, 0.03 mol) in ethanol (38.4 mL) was treated with a catalytic amount of sulfuric acid. The reaction mixture was refluxed for 12 h. The reaction was then concentrated *in vacuo*. Flash chromatography (Merck Silica gel 60, 230-400 mesh, 50/50 hexanes/ethyl acetate) afforded (4-chloro-phenyl)-acetic acid ethyl ester (6.45 g, 88%) as a pale yellow solid: mp 39-41°C; EI-HRMS m/e calcd for C₁₀H₁₁ClO₂ (M⁺) 198.0448, found 198.0452.

A solution of freshly prepared lithium diisopropylamide (23.0 mL of 0.31M stock solution, 7.13 mmol) cooled to -78°C was treated with (4-chloro-phenyl)-acetic acid ethyl ester (1.28 g, 6.48 mmol) in tetrahydrofuran/hexamethylphosphoramide (16.1 mL, 3:1). The resulting solution was stirred at -78°C for 45 min. At this time, the reaction was treated with a solution of iodomethylcyclopentane (1.50 mg, 7.13 mmol) in hexamethylphosphoramide (1 mL). The reaction mixture was stirred at -78°C for 4 h. The reaction was warmed to 25°C and stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 2-(4-chloro-phenyl)-3-cyclopentyl-propionic acid ethyl ester (1.65 g, 90.9%) as a yellow oil: EI-HRMS m/e calcd for C₁₆H₂₁Cl₂O₂ (M⁺) 280.1230, found 280.1227.

A mixture of 2-(4-chloro-phenyl)-3-cyclopentyl-propionic acid ethyl ester (1.65 g, 5.89 mmol) and methyl urea (654 mg, 8.83 mmol) in a solution of magnesium methoxide in methanol (7.4 wt%, 16.9 mL, 11.78 mmol) was refluxed at 100°C for 6 h. The reaction mixture was then concentrated *in vacuo*. Flash chromatography (Merck Silica gel 60, 230-400 mesh, 75/25 hexanes/ethyl acetate) afforded 1-[2-(4-chloro-phenyl)-3-cyclopentyl-propionyl]-3-methyl-urea (105.3 mg, 5.8 %) as a white solid: mp 145-147°C; EI-HRMS m/e calcd for C₁₆H₂₁ClN₂O₂ (M⁺) 308.1292, found 308.1291. The methyl ester of the starting material was recovered from the reaction mixture due to transesterification.

A mixture of 2-(4-chloro-phenyl)-3-cyclopentyl-propionic acid methyl ester (648 mg, 2.43 mmol) and 2-aminothiazole (487 mg, 4.86 mmol) in a solution of magnesium methoxide in methanol (7.4 wt%, 14.0 mL, 9.72 mmol) was refluxed at 100°C for 20 h. The reaction mixture was then concentrated *in vacuo*. Flash chromatography (Merck Silica gel 60, 230-400 mesh, 80/20 hexanes/ethyl acetate) afforded 2-(4-chloro-phenyl)-3-cyclopentyl-N-thiazol-2-yl-propionamide (286 mg, 35%) as a white solid: mp 156.6-159.8°C; EI-HRMS m/e calcd for C₁₇H₁₉ClN₂OS (M⁺) 334.0906, found 334.0910.

### Example 8

### 3-Cyclopentyl-N-thiazol-2-yl-2-(4-trifluoromethyl-phenyl)-propionamide

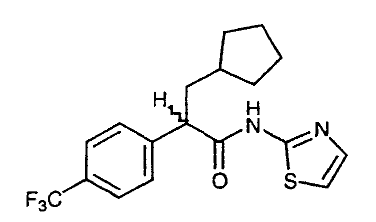

A solution of freshly prepared lithium diisopropylamide (23 mL of a 0.31M stock solution, 7.13 mmol) cooled to -78°C was treated with (4-trifluoromethyl-phenyl)-acetic acid (693 mg, 3.4 mmol) in tetrahydrofuran/hexamethylphosphoramide (8.5 mL, 3:1). The resulting solution was stirred at -78°C for 30 min. Iodomethylcyclopentane (784 mg, 3.7 mmol) was then added in hexamethylphosphoramide (1 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (10 mL). The excess solvent was removed *in vacuo*. The residue was acidified to pH = 1 with a 1N aqueous hydrochloric acid solution. The mixture was then poured into water (150 mL) and extracted with ethyl acetate (3 x 100 mL). The organics were dried over sodium sulfate, filtered, and concentrated *in vacuo*. Flash chromatography (Merck Silica gel 60, 230-400 mesh, 75/25 hexanes/ethyl acetate) afforded 3-cyclopentyl-2-(4-trifluoromethyl-phenyl)-propionic acid (634.9 mg, 65%) as a white solid: mp 94-95°C; EI-HRMS m/e calcd for C₁₅H₁₇F₃O₂ (M⁺) 309.1079, found 309.1072.

A solution of 3-cyclopentyl-2-(4-trifluoromethyl-phenyl)-propionic acid (185 mg, 0.64 mmol) in methylene chloride (6.5 mL) was cooled to 0°C and was treated with a 2.0M solution of oxalyl chloride in methylene chloride (0.35 mL, 0.71 mmol) and a few drops of *N,N*-dimethylformamide. The reaction mixture was stirred at 0°C for 10 min and at 25°C for 30 min. The reaction mixture was then treated with a solution of 2-aminothiazole (142 mg, 1.42 mmol) in tetrahydrofuran (3.23 mL) and *N,N*-diisopropylethylamine (0.27 mL, 1.55 mmol). The solution was stirred at 25°C for 5 h. At this time, the reaction was *concentrated in vacuo.* Flash chromatography (Merck Silica gel 60, 230-400 mesh, 80/20 hexanes/ethyl acetate) afforded 3-cyclopentyl-N-thiazol-2-yl-2-(4-trifluoromethyl-phenyl)-propionamide (127 mg, 53.3%) as a white solid: mp 210-212°C; EI-HRMS m/e calcd for C₁₈H₁₉F₃N₂OS (M⁺) 368.1175, found 368.1170.

### Example 9

### 3-Cyclopentyl-2-(4-methylsulfanyl-phenyl)-N-thiazol-2-yl-propionamide

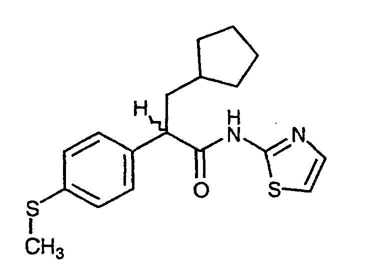

A solution of diisopropylamine (3.2 mL, 23.16 mmol) in dry tetrahydrofuran (10.3 mL) and 1,3-dimethyl-3,4,5,6-tetrahydro-2(1H)-pyrimidinone (3.4 mL) was cooled to -78°C under nitrogen and then treated with a 10M solution of n-butyllithium in hexanes (2.3 mL, 23.16 mmol). The resulting reaction mixture was stirred at -78°C for 30 min and then treated dropwise with a solution of 4-(methylthio)phenylacetic acid (2.01 g, 11.03 mmol) in dry tetrahydrofuran (10.3 mL) and 1,3-dimethyl-3,4,5,6-tetrahydro-2(1H)-pyrimidinone (3.4 mL). The reaction mixture was allowed to stir at -78°C for 1 h, at which time, a solution of iodomethylcyclopentane (2.55 g, 12.13 mmol) in a small amount of dry tetrahydrofuran was added dropwise. The reaction mixture was stirred at - 78°C for 30 min and then allowed to warm to 25°C where it was stirred for 24 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (200 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-methylsulfanyl-phenyl)propionic acid (1.01 g, 35%) as a cream solid: mp 91-93°C; EI-HRMS m/e calcd for C₁₅H₂₀O₂S (M⁺) 264.1184, found 264.1177.

A solution of 3-cyclopentyl-2-(4-methylsulfanyl-phenyl)propionic acid (200 mg, 0.76 mmol) and triphenylphosphine (198 mg, 0.76 mmol) in methylene chloride (2 mL) was cooled to 0°C and then treated with *N*-bromosuccinimide (150 mg, 0.84 mmol) in small portions. After the complete addition of *N*-bromosuccinimide, the reaction mixture was allowed to warm to 25°C over 30 min. The reaction mixture was then treated with 2-aminothiazole (160 mg, 1.60 mmol), and the resulting reaction mixture was stirred at 25°C for 15 h. The reaction mixture was then concentrated *in vacuo* to remove methylene chloride. The remaining residue was diluted with water and ethyl acetate. The organic layer was further washed with a 1N aqueous hydrochloric acid solution, washed with a saturated aqueous sodium bicarbonate solution, dried over magnesium sulfate, filtered, and concentrated *in vacuo*. Flash chromatography (Merck Silica gel 60, 230-400 mesh, 2/1 hexanes/ethyl acetate) afforded crude 3-cyclopentyl-2-(4-methylsulfanyl-phenyl)-N-thiazol-2-yl-propionamide as a yellow solid. Recrystallization from 2/1 hexanes/ethyl acetate afforded pure 3-cyclopentyl-2-(4-methylsulfanyl-phenyl)-N-thiazol-2-yl-propionamide (114 mg, 44%) as a white solid: mp 195-196°C; EI-HRMS m/e calcd for C₁₈H₂₂N₂OS₂ (M⁺) 346.1174, found 346.1171.

### Example 10

### 3-Cyclopentyl-N-thiazol-2-yl-2-(4-trifluoromethylsulfanyl-phenyl)-propionamide

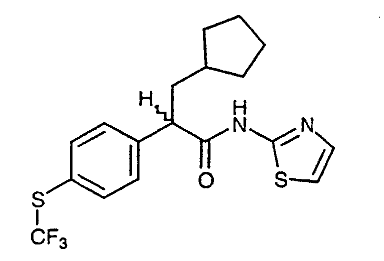

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (60 mg, 0.19 mmol) and triphenylphosphine (49.4 mg, 0.19 mmol) in methylene chloride (471 µL) was cooled to 0°C and then treated with *N*-bromosuccinimide (36.9 mg, 0.21 mmol) in small portions. After the complete addition of *N*-bromosuccinimide, the reaction mixture was allowed to warm to 25°C over 30 min. The bright orange reaction mixture was then treated with 2-aminothiazole (39.6 mg, 0.40 mmol). The resulting reaction mixture was stirred at 25°C for 18 h. The reaction mixture was then concentrated *in vacuo* to remove methylene chloride. The remaining residue was diluted with ethyl acetate (50 mL). The organic layer was washed with a 10% aqueous hydrochloric acid solution (1 x 50 mL), washed with a saturated aqueous sodium bicarbonate solution (1 x 50 mL), washed with water (1 x 50 mL), dried over sodium sulfate, filtered, and concentrated *in vacuo*. Flash chromatography (Merck Silica gel 60, 70-230 mesh, 9/1 hexanes/ethyl acetate) afforded 3-cyclopentyl-N-thiazol-2-yl-2-(4-trifluoromethylsulfanyl-phenyl)-propionamide (49.9 mg, 66%) as a white foam: mp 58-60°C; EI-HRMS m/e calcd for C₁₈H₁₉F₃N₂OS₂ (M⁺) 400.0890, found 400.0895.

### Example 11

### (A) 3-Cyclopentyl-N-thiazol-2-yl-2-(4-trifluoromethanesulfonyl-phenyl)-propionamide

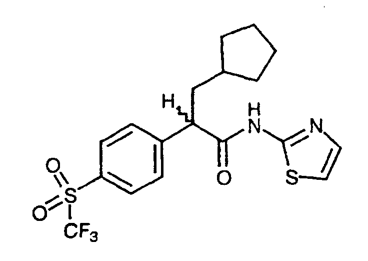

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanylphenyl)propionic acid methyl ester (1.37 g, 99%) as a light yellow oil: EI-HRMS m/e calcd for C₁₆H₁₉F₃O₂S (M⁺) 332.1058, found 332.1052.

A solution of 3-cyclopentyl-2-(4-trifluoromethylsulfanyl-phenyl)propionic acid methyl ester (1.14 g, 3.43 mmol) in methylene chloride (8.6 mL) was treated with 3-chloroperoxybenzoic acid (80-85% grade, 2.00 g based on 80%, 9.26 mmol). The reaction mixture was stirred at 25°C for 17 h, at which time, thin layer chromatography showed the presence of two new lower R_{f} products. An additional 2.00 g of 3-chloroperoxybenzoic acid was added to the reaction mixture to drive the conversion of the sulfoxide to the sulfone, and the resulting reaction mixture was stirred at 25°C for 3 d. The reaction mixture was concentrated *in vacuo* to remove methylene chloride. The resulting residue was diluted with ethyl acetate (300 mL). The organic phase was washed with a saturated aqueous sodium bicarbonate solution (3 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 19/1 hexanes/ethyl acetate) afforded 3-cyclopentyl-2-(4-trifluoromethanesulfonylphenyl)propionic acid methyl ester (1.19 g, 95%) as a light yellow oil: EI-HRMS m/e calcd for C₁₆H₁₉F₃O₄S (M⁺) 364.0956, found 364.0965.

A solution of 3-cyclopentyl-2-(4-trifluoromethanesulfonyl-phenyl)propionic acid methyl ester (708.2 mg, 1.94 mmol) in tetrahydrofuran (2.4 mL) was treated with a 0.8M aqueous lithium hydroxide solution (3.6 mL, 2.92 mmol). The reaction mixture was stirred at 25°C for 23 h and then concentrated *in vacuo* to remove tetrahydrofuran. The remaining aqueous layer was acidified to pH = 2 with a 10% aqueous hydrochloric acid solution and then extracted with ethyl acetate (2 x 100 mL). The combined organic layers were washed with a saturated aqueous sodium chloride solution (1 x 100 mL), dried over sodium sulfate, filtered, and concentrated *in vacuo* to afford a cream solid. This solid was purified by triturating with diethyl ether/petroleum ether to provide pure 3-cyclopentyl-2-(4-trifluoromethanesulfonyl-phenyl)propionic acid (527.0 mg, 77%) as a white solid: mp 143-145°C; EI-HRMS m/e calcd for C₁₅H₁₇F₃O₄S (M⁺) 350.0800, found 350.0816.

A solution of 3-cyclopentyl-2-(4-trifluoromethanesulfonyl-phenyl)propionic acid (164.0 mg, 0.47 mmol) and triphenylphosphine (184.2 mg, 0.70 mmol) in methylene chloride (1.2 mL) was cooled to 0°C and then treated with *N*-bromosuccinimide (141.6 mg, 0.80 mmol) in small portions. After the complete addition of *N*-bromosuccinimide, the reaction mixture was allowed to warm to 25°C where it was stirred for 1 h. The reaction mixture was then treated with 2-aminothiazole (140.6 mg, 1.40 mmol). The resulting reaction mixture was stirred at 25°C for 22 h. The reaction mixture was then concentrated *in vacuo*. Flash chromatography (Merck Silica gel 60, 230-400 mesh, 3/1 hexanes/ethyl acetate) afforded 3-cyclopentyl-N-thiazol-2-yl-2-(4-trifluoromethanesulfonyl-phenyl)-propionamide (47.9 mg, 24%) as a cream solid: mp 189-191°C; EI-HRMS m/e calcd for C₁₈H₁₉F₃N₂O₃S₂ (M⁺) 432.0789, found 432.0791.

### (B) In an analogous manner, there were obtained:

(a) From 3-cyclopentyl-2-(4-trifluoromethanesulfonyl-phenyl)propionic acid and 2-aminothiazole-4-carboxylic acid methyl ester: 2-[3-Cyclopentyl-2-(4-trifluoromethanesulfonyl-phenyl)-propionylamino)-thiazole-4-carboxylic acid methyl ester as a gray solid: mp 122-125°C; EI-HRMS m/e calcd for C₂₀H₂₁F₃N₂O₅S₂ (M⁺) 490.0844, found 490.0844.
(b) From 3-cyclopentyl-2-(4-trifluoromethanesulfonyl-phenyl)propionic acid and 2-aminothiazole-4-carboxylic acid ethyl ester: 2-[3-Cyclopentyl-2-(4-trifluoramethanesulfonyl-phenyl)-propionylamino]-thiazole-4-carboxylic acid ethyl ester as a white solid: mp 132-134°C; EI-HRMS m/e calcd for C₂₁H₂₃F₃N₂O₅S₂ (M⁺) 504.1000, found 504.0988.
(c) From 3-cyclopentyl-2-(4-trifluoromethanesulfonyl-phenyl)propionic acid and methyl 2-amino-4-thiazoleacetate: {2-[3-Cyclopentyl-2-(4-trifluoromethanesulfonyl-phenyl)-propionylamino]-thiazol-4-yl}-acetic acid methyl ester as a yellow foam: mp 48-52°C; EI-HRMS m/e calcd for C₂₁H₂₃F₃N₂O₅S₂ (M⁺) 504.1000, found 504.0998.

### Example 12

### 2-[3-Chloro-4-methanesulfonyl-phenyl]-3-cyclopentyl-N-thiazol-2-yl-propionamide

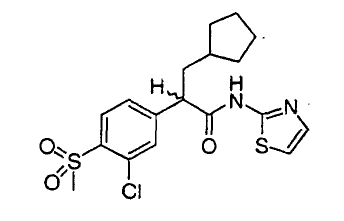

A solution of anhydrous aluminum chloride (5.00 g, 37.50 mmol) in chloroform (15 mL) was cooled to 0°C and stirred for 30 min under a nitrogen atmosphere. A solution of ethyl oxalyl chloride (3.91 g, 28.64 mmol) in chloroform (5 mL) was then added, and the resulting reaction mixture was stirred at 0°C for an additional 30 min. A solution of 2-chlorothioanisole (4.08 g, 25.58 mmol) in chloroform (20 mL) was then slowly added to the cooled reaction mixture. The solution became red in color and slowly became gum-like over a period of 30 min. The resulting reaction mixture was then stirred for an additional 3.5 h, and during this period, the reaction mixture was allowed to warm to 25°C. The reaction mixture was then quenched by the addition of water (25 mL). The aqueous layer was extracted with chloroform (3 x 25 mL). The combined organic layers were concentrated *in vacuo*. Flash chromatography (Merck Silica gel 60, 230-400 mesh, 4/1 hexanes/ethyl acetate) afforded (3-chloro-4-methylsulfanyl-phenyl)-oxo-acetic acid ethyl ester (4.32 g, 65.3%) as a yellow oil.

A solution of (3-chloro-4-methylsulfanyl-phenyl)-oxo-acetic acid ethyl ester (3.93 g, 15.19 mmol) in methanol (30 mL) was cooled to 0°C and then treated with sodium borohydride (530.9 mg, 14.03 mmol). The reaction mixture changed from yellow to colorless. The mixture was stirred for 15 min and then quenched with a 1N aqueous hydrochloric acid solution (10 mL). The resulting reaction mixture was then extracted with methylene chloride (2 x 30 mL). The combined organic layers were washed with a saturated aqueous sodium chloride solution (1 x 30 mL), dried over sodium sulfate, filtered, and concentrated *in vacuo.* Flash chromatography (Merck Silica gel 60, 230-400 mesh, 9/1 then 4/1 hexanes/ethyl acetate) afforded (3-chloro-4-methylsulfanyl-phenyl)-hydroxy-acetic acid ethyl ester (1.43 g, 38%) as a white solid: mp 56-57°C; EI-HRMS m/e calcd for C₁₁H₁₃ClO₃S (M⁺) 260.0273, found 260.0276.

A solution of (3-chloro-4-methylsulfanyl-phenyl)-hydroxy-acetic acid ethyl ester (1.43 g, 5.49 mmol) in pyridine (2 mL) was treated with acetic anhydride (2 mL) and 4-dimethylaminopyridine (50 mg, 0.41 mmol). The reaction mixture was stirred at 25°C for 16 h. The reaction mixture was then diluted with methylene chloride (100 mL). The organic layer was washed with a 1N aqueous hydrochloric acid solution (2 x 30 mL), washed with a saturated aqueous sodium chloride solution (1 x 30 mL), dried over sodium sulfate, filtered, and concentrated *in vacuo*. Flash chromatography (Merck Silica gel 60, 230-400 mesh, 4/1 hexanes/ethyl acetate) afforded acetoxy-(3-chloro-4-methylsulfanyl-phenyl)-acetic acid ethyl ester (1.51 g, 91%) as a light yellow oil: EI-HRMS m/e calcd for C₁₃H₁₅ClO₄S (M⁺) 302.0379, found 302.0387.

A solution of acetoxy-(3-chlaro-4-methylsulfanyl-phenyl)-acetic acid ethyl ester (1.47 g, 4.87 mmol) in hexamethylphosphoramide (7.2 mL) and methanol (20 µL) was treated with a 0.1M solution of samarium iodide in tetrahydrofuran (146 mL, 14.6 mmol). The reaction mixture was stirred at 25°C under nitrogen for 6 min. During this time period, the reaction mixture changed from purple to white. The reaction mixture was diluted with water (150 mL) and then extracted with methylene chloride (3 x 100 mL). The combined organic layers were dried over sodium sulfate, filtered, and concentrated *in vacuo*. Flash chromatography (Merck Silica gel 60, 230-400 mesh, 4/1 hexanes/ethyl acetate) afforded (3-chloro-4-methylsulfanyl-phenyl)-acetic acid ethyl ester (0.71 g, 60%) as a light yellow oil: EI-HRMS m/e calcd for C₁₁H₁₃ClO₂S (M⁺) 244.0324, found 244.0332.

A solution of diisopropylamine (457 µL, 3.26 mmol) in tetrahydrofuran (5 mL) was cooled to -78°C under a nitrogen atmosphere and then treated with a 2.5M solution of *n*-butyllithium in hexanes (1.3 mL, 3.26 mmol). The mixture was stirred at -78°C for 30 min, at which time, a solution of (3-chloro-4-methylsulfanyl-phenyl)-acetic acid ethyl ester (0.67 g, 2.75 mmol) in tetrahydrofuran (8 mL) was slowly added to the reaction mixture. The reaction mixture turned deep yellow in color. The reaction mixture was then further stirred at -78°C for 30 min, at which time, a solution of iodomethylcvclopentane (0.65 g, 3.09 mmol) in 1,3-dimethyl-3,4,5,6-tetrahydro-2(1H)-pyrimidone (1 mL) was added via syringe. The reaction mixture was then allowed to warm to 25°C, where it was stirred for 16 h. The reaction mixture turned red in color during this time period. The reaction mixture was quenched with a 6N aqueous hydrochloric acid solution (5 mL) and further diluted with water (20 mL). The reaction mixture was then extracted with methylene chloride (3 x 20 mL). The combined organic layers were washed with a saturated aqueous sodium chloride solution (1 x 25 mL), dried over sodium sulfate, filtered, and concentrated *in vacuo*. Flash chromatography (Merck Silica gel 60, 230-400 mesh, 4/1 hexanes/ethyl acetate) afforded 2-(3-chloro-4-methylsulfanyl-phenyl)-3-cyclopentyl-propionic acid ethyl ester (0.50 g, 56%) as a light yellow oil.

A solution of 2-(3-chloro-4-methylsulfanyl-phenyl)-3-cyclopentyl-propionic acid ethyl ester (0.45 g, 1.39 mmol) in ethanol (3 mL) was treated with a 10% aqueous potassium hydroxide solution (2 mL). The reaction mixture was stirred under nitrogen at 25°C for 16 h. The reaction mixture was then acidified with a 1N aqueous hydrochloric acid solution (5 mL). The reaction mixture was then extracted with methylene chloride (3 x 15 mL). The combined organic layers were dried over sodium sulfate, filtered, and concentrated *in vacuo* to afford 2-(3-chloro-4-methylsulfanyl-phenyl)-3-cyclopentylpropionic acid (0.29 g, 70%) as a white solid: EI-HRMS m/e calcd for C₁₅H₁₉ClO₂S (M⁺) 298.0794, found 298.0798.

A solution of benzotriazol-1-yloxytris(dimethylamino)phosphonium hexafluorophosphate (0.62 g, 1.41 mmol) and 2-(3-chloro-4-methylsulfanyl-phenyl)-3-cyclopentyl-propionic acid (0.29 g, 0.95 mmol) in methylene chloride (10 mL) was treated with *N*,*N*-diisopropylethylamine (500 µL, 2.87 mmol) and 2-aminothiazole (140 mg, 1.27mmol). The mixture was stirred under nitrogen at 25°C for 14 h. The reaction mixture was then washed with a 6N aqueous hydrochloric acid solution (1 x 15 mL) and washed with a saturated aqueous sodium chloride solution (1 x 25 mL). The organic layer was dried over sodium sulfate, filtered, and concentrated *in vacuo*. Flash chromatography (Merck Silica gel 60, 230-400 mesh, 4/1 hexanes/ethyl acetate) afforded 2-(3-chloro-4-methylsulfanyl-phenyl)-3-cyclopentyl-N-thiazol-2-yl-propionamide (0.26 g, 71%) as a white solid: EI-HRMS m/e calcd for C₁₈H₂₁ClN₂OS₂ (M⁺) 380.0783, found 380.0792.

A solution of 2-(3-chloro-4-methylsulfanyl-phenyl)-3-cyclopentyl-N-thiazol-2-yl-propionamide (187 mg, 0.49 mmol) in methylene chloride (10 mL) was cooled to 0°C under nitrogen and then treated with 3-chloroperoxybenzoic acid (456. 8 mg based on 50% purity). The reaction mixture was stirred for 3 h, and during this period, the temperature was allowed to warm to 25°C. The reaction mixture was then diluted with methylene chloride (50 mL). The organic layer was washed with a saturated aqueous sodium carbonate solution (1 x 20 mL), washed with a saturated aqueous sodium chloride solution (1 x 20 mL), dried over sodium sulfate, filtered, and concentrated *in vacuo*. Flash chromatography (Merck Silica gel 60, 230-400 mesh, 4/1 hexanes/ethyl acetate) afforded 2-(3-chloro-4-methanesulfonyl-phenyl)-3-cyclopentyl-N-thiazol-2-yl-propionamide (102 mg, 50%) as a white solid: EI-HRMS m/e calcd for C₁₈H₂₁ClN₂O₃S₂ (M⁺) 412.0682, found 412.0674.

### Example 13

### (2R)-3-Cyclopentyl-2-(4-methanesulfonylphenyl)-N-thiazol-2-yl-propionamide

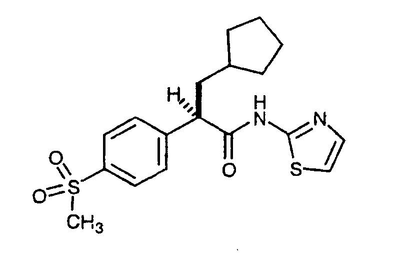

A solution of 4-(methanesulfonyl)phenyl acetic acid (43.63 g, 0.204 mol) in methanol (509 mL) was treated slowly with concentrated sulfuric acid (2 mL). The resulting reaction mixture was heated under reflux for 19 h. The reaction mixture was allowed to cool to 25°C and then concentrated *in vacuo* to remove methanol. The residue was diluted with ethyl acetate (800 mL). The organic phase was washed with a saturated aqueous sodium bicarbonate solution (1 x 200 mL), washed with a saturated aqueous sodium chloride solution (1 x 200 mL), dried over sodium sulfate, filtered, and concentrated *in vacuo*. Flash chromatography (Merck Silica gel 60, 70-230 mesh, 1/1 hexanes/ethyl acetate) afforded 4-(methanesulfonyl)phenyl acetic acid methyl ester (45.42 g, 98%) as a yellow oil which solidified to a cream colored solid upon sitting over time at 25°C: mp 78-80°C; EI-HRMS m/e calcd for C₁₀H₁₂O₄S (M⁺) 228.0456, found 228.0451.

A mechanical stirrer was used for this reaction. A solution of diisopropylamine (29.2 mL, 0.21 mol) in dry tetrahydrofuran (186 mL) and 1,3-dimethyl-3,4,5,6-tetrahydro-2(1H)-pyrimidinone (62 mL) was cooled to -78°C and then treated with a 2.5M solution of n-butyllithium in hexanes (83.4 mL, 0.21 mol). The yellow-orange reaction mixture was stirred at -78°C for 35 min and then slowly treated with a solution of 4-(methanesulfonyl)phenyl acetic acid methyl ester (45.35 g, 0.20 mol) in dry tetrahydrofuran (186 mL) and 1,3-dimethyl-3,4,5,6-tetrahydro-2(1H)-pyrimidinone (62 mL). The reaction mixture turned dark in color. The reaction mixture was then stirred at - 78°C for 50 min, at which time, a solution of iodomethylcyclopentane (50.08 g, 0.24 mol) in a small amount of dry tetrahydrofuran was added slowly. The reaction mixture was then stirred at -78°C for 50 min, and then allowed to warm to 25°C, where it was stirred for 36 h. The reaction mixture was quenched with water (100 mL), and the resulting reaction mixture was concentrated *in vacuo* to remove tetrahydrofuran. The remaining residue was diluted with ethyl acetate (1.5 L). The organic phase was washed with a saturated aqueous sodium chloride solution (1 x 500 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-methanesulfonylphenyl)propionic acid methyl ester (41.79 g, 68%) as a yellow viscous oil: EI-HRMS m/e calcd for C₁₆H₂₂O₄S (M⁺) 310.1239, found 310.1230.

A solution of 3-cyclopentyl-2-(4-methanesulfonylphenyl)propionic acid methyl ester (50.96 g, 0.16 mol) in methanol (410 mL) was treated with a 1N aqueous sodium hydroxide solution (345 mL, 0.35 mol). The reaction mixture was stirred at 25°C for 24 h. The reaction mixture was concentrated *in vacuo* to remove methanol. The resulting aqueous residue was acidified to pH = 2 with concentrated hydrochloric acid and then extracted with ethyl acetate (5 x 200 mL). The combined organic layers were dried over sodium sulfate, filtered, and concentrated *in vacuo* to afford pure 3-cyclopentyl-2-(4-methanesulfonylphenyl)propionic acid (43.61 g, 90%) as a white solid which was used without further purification: mp 152-154°C; EI-HRMS m/e calcd for C₁₅H₂₀O₄S (M⁺) 296.1082, found 296.1080.

Two separate reactions were setup in parallel: (1) A solution of (R)-(+)-4-benzyl-2-oxazolidinone (3.67 g, 20.73 mmol) in dry tetrahydrofuran (35 mL) was cooled to -78°C and then treated with a 2.5M solution of *n*-butyllithium in hexanes (7.9 mL, 19.86 mmol). The resulting reaction mixture was stirred at -78°C for 30 min and then allowed to warm to 25°C, where it was stirred for 1.5 h. (2) A solution of racemic 3-cyclopentyl-2-(4-methanesulfonylphenyl)propionic acid (5.12 g, 17.27 mmol) in dry tetrahydrofuran (35 mL) was cooled to 0°C and then treated with triethylamine (2.8 mL, 19.86 mmol). The reaction mixture was stirred at 0°C for 10 min and then treated dropwise with trimethylacetyl chloride (2.6 mL, 20.73 mmol). The resulting reaction mixture was stirred at 0°C for 2 h and then cooled to -78°C for the addition of the freshly prepared chiral oxazolidinone. The reaction mixture containing the oxazolidinone was then added to the cooled (-78°C) mixed anhydride solution. The resulting reaction mixture was stirred as -78°C for 1 h and allowed to gradually warm to 25°C. The reaction mixture was then stirred at 25°C for 3 d. The resulting reaction mixture was quenched with water (100 mL) and then concentrated *in vacuo* to remove tetrahydrofuran. The resulting aqueous residue was diluted with ethyl acetate (600 mL). The organic layer was washed with a saturated aqueous sodium chloride solution (1 x 300 mL), dried over sodium sulfate, filtered, and concentrated *in vacuo*. Thin layer chromatography using 13/7 hexanes/ethyl acetate as the developing solvent indicated the presence of two products. The higher moving product had a R_{f} =0.32 and the lower moving product had a R_{f}= 0.19. Flash chromatography (Merck Silica gel 60, 230-400 mesh, 9/1 then 13/7 hexanes/ethyl acetate) afforded two products: (1) The higher R_{f} product (4R, 2'S)-4-benzyl-3-[3-cyclopentyl-2-(4-methanesulfonylphenyl)propionyl]-oxazolidin-2-one (2.12 g, 54%) as a white foam: mp 62-64°C; [α]²³₅₈₉ = +6.3° (c=0.24, chloroform); EI-HRMS m/e calcd for C₂₅H₂₉NO₅S (M⁺) 455.1766, found 455.1757. (2) The lower R_{f} product (4R, 2R)-4-benzyl-3-[3-cyclopentyl-2-(4-methanesulfonylphenyl)propionyl]-oxazolidin-2-one (3.88 g, 99%) as a white foam: mp 59-61°C; [α]²³₅₈₉ = -98.3° (c=0.35, chloroform); EI-HRMS m/e calcd for C₂₅H₂₉NO₅S (M⁺) 455.1766, found 455.1753. The combined mass recovery from the two products was 6.00 g, providing a 76% conversion yield for the reaction.

An aqueous solution of lithium hydroperoxide was freshly prepared from mixing a solution of anhydrous lithium hydroxide powder (707.3 mg, 16.86 mmol) in 5.27 mL of water with a 30% aqueous hydrogen peroxide solution (3.44 mL, 33.71 mmol). This freshly prepared aqueous lithium hydroperoxide solution was cooled to 0°C and then slowly added to a cooled (0°C) solution of (4R, 2'R)-4-benzyl-3-[3-cyclopentyl-2-(4-methanesulfonylphenyl)propionyl]-oxazolidin-2-one (3.84 g, 8.43 mmol) in tetrahydrofuran (33 mL) and water (11 mL). The reaction mixture was stirred 0°C for 1.5 h. The reaction mixture was then quenched with a 1.5N aqueous sodium sulfite solution (25 mL). The reaction mixture was further diluted with water (300 mL). The resulting aqueous layer was continuously extracted with diethyl ether until thin layer chromatography indicated the absence of the recovered chiral oxazolidinone in the aqueous layer. The aqueous layer was then acidified to pH = 2 with a 10% aqueous hydrochloric acid solution and extracted with ethyl acetate (300 mL). The organic extract was dried over sodium sulfate, filtered, and concentrated *in vacuo* to afford (2R)-3-cyclopentyl-2-(4-methanesulfonylphenyl)propionic acid as a white solid (2.23 g, 89%) which was used without further purification. Flash chromatography (Merck Silica gel 60, 70-230 mesh, 30/1 methylene chloride/methanol then 10/1 methylene chloride/methanol) was used to obtain a purified sample for analytical data and afforded pure (2R)-3-cyclopentyl-2-(4-methanesulfonylphenyl)propionic acid as a white foam: mp 62-64°C (foam to gel); [α]²³₅₈₉ = -50.0° (c=0.02, chloroform); EI-HRMS m/e calcd for C₁₅H₂₀O₄S (M⁺) 296.1082, found 296.1080.

A solution of triphenylphosphine (3.35 g, 12.79 mmol) in methylene chloride (19 mL) was cooled to 0°C and then slowly treated with *N*-bromosuccinimide (2.28 g, 12.79 mmol) in small portions. The reaction mixture was stirred at 0°C for 30 min, and during this time period, the color of the reaction mixture changed from light yellow to a darker yellow then to a purple color. The cooled purple reaction mixture was then treated with the (2R)-3-cyclopentyl-2-(4-methanesulfonylphenyl)propionic acid (2.23 g, 7.52 mmol). The resulting reaction mixture was then allowed to warm to 25°C over 45 min, at which time, the reaction mixture was then treated with 2-aminothiazole (1.88 g, 18.81 mmol). The resulting reaction mixture was stirred at 25°C for 12 h. The reaction mixture was then concentrated *in vacuo* to remove methylene chloride. The remaining black residue was diluted with ethyl acetate (300 mL) and then washed well with a 10% aqueous hydrochloric acid solution (2 x 100 mL), a 5% aqueous sodium bicarbonate solution (3 x 100 mL), and a saturated aqueous sodium chloride solution (1 x 200 mL). The organic layer was then dried over sodium sulfate, filtered, and concentrated *in vacuo*. Flash chromatography (Merck Silica gel 60, 70-230 mesh, 9/1, 3/1, and then 11/9 hexanes/ethyl acetate) afforded (2R)-3-cyclopentyl-2-(4-methanesulfonylphenyl)-N-thiazol-2-yl-propionamide (2.10 g, 74%) as a white foam: mp 78-80°C (foam to gel); [α]²³₅₈₉ = -70.4° (c=0.027, chloroform); EI-HRMS m/e calcd for C₁₈H₂₂N₂O₃S₂ (M⁺) 378.1072, found 378.1081.

### Example 14

### 3-Cyclopentyl-2-(4-methanesulfonyl-3-nitrophenyl)-N-thiazol-2-yl-propionamide

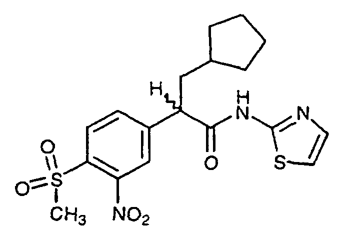

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo.* Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A solution of triphenylphosphine (138 mg, 0.53 mmol) in methylene chloride (2 mL) was cooled to 0°C and then slowly treated with *N*-bromosuccinimide (94 mg, 0.53 mmol) in small portions. The reaction mixture was stirred at 0°C for 10 min and then treated with 3-cyclopentyl-2-(4-methanesulfonyl-3-nitrophenyl)-propionic acid (150 mg, 0.44 mmol). The resulting reaction mixture was stirred at 0°C for 5 min and then allowed to warm to 25°C, where it was stirred for 25 min. The reaction mixture was then treated with 2-aminothiazole (97 mg, 0.97 mmol). The resulting reaction mixture was stirred at 25°C for 15 h. The crude reaction mixture was directly purified by flash chromatography, (Merck Silica gel 60, 230-400 mesh, 1/1 hexanes/ethyl acetate), to afford 3-cyclopentyl-2-(4-methanesulfonyl-3-nitrophenyl)-N-thiazol-2-yl-propionamide (96 mg, 52%) as a pale yellow solid: mp 121-124°C; FAB-HRMS m/e calcd for C₁₈H₂₁N₃O₅S₂ (M+H)⁺ 424.1001, found 424.1000.

### Example 15

### 3-Cyclopentyl-2-(3,4-dichlorophenyl)-N-(5-hydroxymethyl-thiazol-2-yl)-propionamide

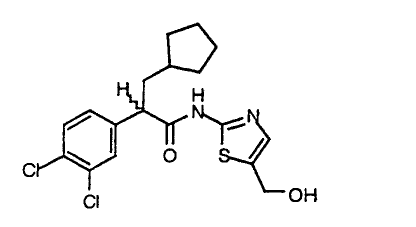

A solution of 2-[3-cyclopentyl-2-(3,4-dichlorophenyl)-propionylamino]-thiazole-5-carboxylic acid ethyl ester (prepared in Example **1** (B)(g), 110 mg, 0.25 mmol) in diethyl ether (2 mL) at 0°C was slowly treated with lithium aluminum hydride (12 mg, 0.31 mmol). The resulting reaction mixture continued to stir at 0°C and was allowed to gradually warm to 25°C. The reaction mixture was then stirred at 25°C over a period of 14 h. The reaction mixture was slowly quenched by the dropwise addition of water (5 mL). The resulting reaction mixture was partitioned between water and ethyl acetate. A saturated aqueous sodium chloride solution was added to break up the emulsions. The organic layer was dried over magnesium sulfate, filtered, and concentrated *in vacuo*. Flash chromatography (Merck Silica gel 60, 230-400 mesh, 1/3 hexanes/ethyl acetate) afforded 3-cyclopentyl-2-(3,4-dichlorophenyl)-N-(5-hydroxymethyl-thiazol-2-yl)-propionamide (52.9 mg, 53%) as a light yellow solid: mp 128-130°C; EI-HRMS m/e calcd for C₁₈H₂₀Cl₂N₂O₂S (M⁺) 398.0623, found 398.0623.

### Example 16

### 3-Cyclopentyl-2-(3,4-dichlorophenyl)-N-[4-(2-hydroxyethyl)-thiazol-2-yl]-propionamide

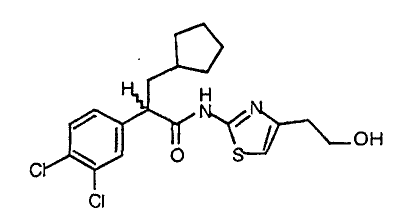

A solution of {2-[3-cyclopentyl-2-(3,4-dichlorophenyl)-propionylamino]-thiazol-4-yl}-acetic acid ethyl ester (prepared in Example **1** (B)(c), 129 mg, 0.28 mmol) in tetrahydrofuran (1.4 mL) at 25°C was slowly treated with sodium borohydride (22.5 mg, 0.59 mmol). The resulting reaction mixture was stirred at 25°C for 10 h. After 10 h at 25°C, a substantial amount of starting material still remained. An additional amount of sodium borohydride powder (21.4 mg, 0.57 mmol) was added to the reaction mixture, and the reaction mixture was heated under reflux for 14 h. The reaction mixture was allowed to cool to 25°C, and then slowly quenched by the dropwise addition of water. The resulting reaction mixture was concentrated *in vacuo* to remove tetrahydrofuran. The resulting residue was diluted with ethyl acetate (100 mL) and washed with a saturated aqueous sodium chloride solution (1 x 50 mL). The organic layer was dried over sodium sulfate, filtered, and concentrated *in vacuo*. Flash chromatography (Merck Silica gel 60, 230-400 mesh, 3/1 then 1/1 hexanes/ethyl acetate) afforded 3-cyclopentyl-2-(3,4-dichlorophenyl)-N-[4-(2-hydroxyethyl)-thiazol-2-yl]-propionamide (68.1 mg, 58%) as a white foam: mp 85-86°C; FAB-HRMS m/e calcd for C₁₉H₂₂Cl₂N₂O₂S (M+H)⁺ 413.0858, found 413.0838.

### Example 17

### 3-Cyclopentyl-2-(3,4-dichlorophenyl)-N-(4-hydroxymethyl-thiazol-2-yl)-propionamide

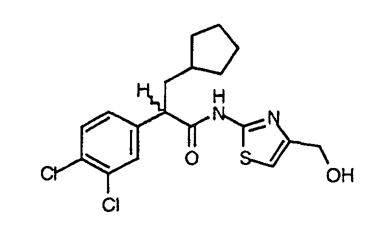

A solution of 2-[3-cyclopentyl-2-(3,4-dichlorophenyl)-propionylamino]-thiazole-4-carboxylic acid ethyl ester (prepared in Example **1**(B)(f), 200 mg, 0.45 mmol) in tetrahydrofuran (3 mL) at 25°C was slowly treated with sodium borohydride (26.0 mg, 0.68 mmol). The reaction mixture was heated under reflux for 48 h. The reaction mixture was allowed to cool to 25°C and then slowly quenched by the dropwise addition of water. The resulting reaction mixture was partitioned between water and ethyl acetate. The organic layer was dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(3,4-dichlorophenyl)-N-(4-hydroxymethyl-thiazol-2-yl)-propionamide (44.9 mg, 25%) as a white solid: mp 88-90°C; EI-HRMS m/e calcd for C₁₈H₂₀Cl₂N₂O₂S (M⁺) 398.0623, found 398.0631.

### Example 18

### {2-[3-Cyclopentyl-2-(3,4-dichlorophenyl)-propionylamino]-thiazol-4-yl}-acetic acid

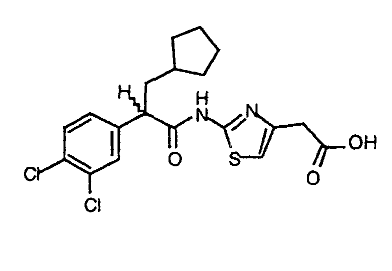

A solution of {2-[3-cyclopentyl-2-(3,4-dichlorophenyl)-propionylamino]-thiazol-4-yl}-acetic acid ethyl ester (prepared in Example **1**(B)(c), 198.1 mg, 0.44 mmol) in absolute ethanol (2.2 mL) was treated with a 1N aqueous sodium hydroxide solution (910 µL, 0.91 mmol). The reaction mixture was heated under reflux for 2 h. The reaction mixture was allowed to cool to 25°C and then concentrated *in vacuo* to remove absolute ethanol. The resulting residue was acidified to pH = 2 with a 10% aqueous hydrochloric acid solution and extracted with ethyl acetate (1 x 150 mL). The organic layer was washed with a saturated aqueous sodium chloride solution (1 x 100 mL), dried over sodium sulfate, filtered, and concentrated *in vacuo*. The resulting white residue was washed well with cold water and dried to afford {2-[3-cyclopentyl-2-(3,4-dichlorophenyl)-propionylamino]-thiazot-4-yl}-acetic acid (150 mg, 81%) as a white solid: mp 100-102°C; FAB-HRMS m/e calcd for C₁₉H₂₀Cl₂N₂O₃S (M+H)⁺ 427.0650, found 427.0633.

### Example 19

### 2-[3-Cyclopentyl-2-(3,4-dichlorophenyl)-propionylamino]-thiazole-5-carboxylic acid

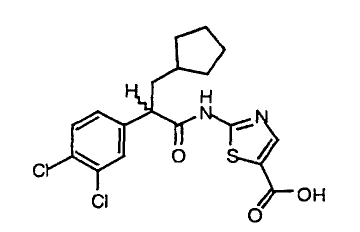

A solution of 2-[3-cyclopentyl-2-(3,4-dichlorophenyl)-propionylamino]-thiazole-5-carboxylic acid ethyl ester (prepared in Example **1**(B)(g), 1.0 g, 2.27 mmol) in absolute ethanol (10 mL) was treated with a 1N aqueous sodium hydroxide solution (4.77 mL, 4.77 mmol). The reaction mixture was heated under reflux for 15 h. The reaction mixture was allowed to cool to 25°C and then concentrated *in vacuo* to remove absolute ethanol. The resulting yellow residue was acidified to pH = 2 with concentrated hydrochloric acid and extracted with ethyl acetate (2 x 75 mL). The combined organic layers were dried over magnesium sulfate, filtered, and concentrated *in vacuo*. Recrystallization from ethyl acetate afforded 2-[3-cyclopentyl-2-(3,4-dichlorophenyl)-propionylamino]-thiazole-5-carboxylic acid (210 mg, 22%) as a white solid: mp 269-270°C; FAB-HRMS m/e calcd for C₁₈H₁₈Cl₂N₂O₃S (M+H)⁺ 413.0493, found 413.0483.

### Example 20

### 2-[3-Cyclopentyl-2-(3,4-dichlorophenyl)-propionylamino]-thiazole-4-carboxylic acid

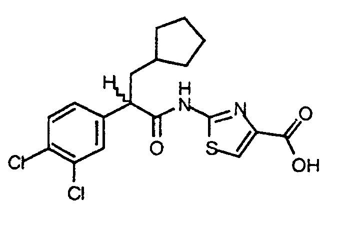

A solution of 2-[3-cyclopentyl-2-(3,4-dichlorophenyl)-propionylamino]-thiazole-4-carboxylic acid ethyl ester (prepared in Example **1**(B)(f), 600 mg, 1.36 mmol) in absolute ethanol (6 mL) was treated with a 1N aqueous sodium hydroxide solution (2.85 mL, 2.85 mmol). The reaction mixture was heated under reflux for 15 h. The reaction mixture was allowed to cool to 25°C and then concentrated *in vacuo* to remove absolute ethanol. The resulting yellow residue was acidified to pH = 2 with concentrated hydrochloric acid and extracted with ethyl acetate (2 x 100 mL). The combined organic layers were dried over magnesium sulfate, filtered, and concentrated *in vacuo*. Precipitation from 1/1 hexanes/ethyl acetate afforded 2-[3-cyclopentyl-2-(3,4-dichlorophenyl)-propionylamino]-thiazole-4-carboxylic acid (399 mg, 71%) as a white solid: mp 285-287°C; FAB-HRMS m/e calcd for C₁₈H₁₈Cl₂N₂O₃S (M+H)⁺ 413.0493, found 413.0481.

### Example 21

### (A) {2-[3-Cyclopentyl-2-(3,4-dichlorophenyl)-propionylamino]-thiazol-4-yl}-acetic acid methyl ester

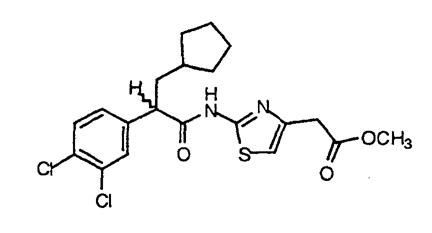

A solution of {2-[3-cyclopentyl-2-(3,4-dichlorophenyl)-propionylamino]-thiazol-4-yl}-acetic acid (prepared in Example **18,** 95.4 mg, 0.223 mmol) in methanol (1.1 mL) was treated with 1 drop of concentrated sulfuric acid. The reaction mixture was heated under reflux for 15 h. The reaction mixture was allowed to cool to 25°C and then concentrated *in vacuo* to remove methanol. The resulting residue was diluted with ethyl acetate (100 mL). The organic phase was washed with a saturated aqueous sodium chloride solution (1 x 100 mL), dried over sodium sulfate, filtered, and concentrated *in vacuo*. Flash chromatography (Merck Silica gel 60, 230-400 mesh, 3/1 hexanes/ethyl acetate) afforded {2'-[3-cyclopentyl-2-(3,4-dichlorophenyl)-propionylaminol-thiazol-4-yl}-acetic acid methyl ester (77.2 mg, 78%) as a yellow viscous oil: FAB-HRMS m/e calcd for C₂₀H₂₂Cl₂N₂O₃S (M+H)⁺ 441.0807, found 441.0804.

### (B) In an analogous manner, there were obtained:

(a) From 2-[3-cyclopentyl-2-(3,4-dichlorophenyl)-propionylamino]-thiazole-4-carboxylic acid (prepared in Example **20**): 2-[3-Cyclopentyl-2-(3,4-dichlorophenyl)-propionylamino]-thiazole-4-carboxylic acid methyl ester as a white solid: mp 153-155°C; FAB-HRMS m/e calcd for C₁₉H₂₀Cl₂N₂O₃S (M+H)⁺ 427.0650, found 427.0659.
(b) From 2-[3-cyclopentyl-2-(3,4-dichlorophenyl)-propionylamino]-thiazole-5-carboxylic acid (prepared in Example **19**): 2-[3-Cyclopentyl-2-(3,4-dichlorophenyl)-propionylamino]-thiazole-5-carboxylic acid methyl ester as a white solid: mp 150-151°C; FAB-HRMS m/e calcd for C₁₉H₂₀Cl₂N₂O₃S (M+H)⁺ 427.0650, found 427.0650.

### Example 22

### (A) 3-Cyclopentyl-2-(4-nitro-phenyl)-N-thiazol-2-yl-propionamide

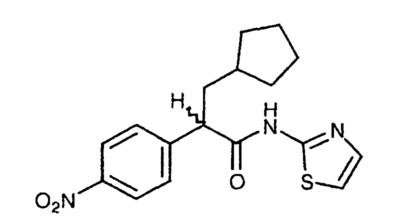

A solution of freshly prepared lithium diisopropylamide (430.55 mL of a 0.3M stock solution, 129.16 mmol) cooled to -78°C was treated with (4-nitro-phenyl)-acetic acid ethyl ester (26.32 g, 125.83 mmol) in tetrahydrofuran/hexamethylphosphoramide (312.5 mL, 3:1). The resulting solution was stirred at -78°C for 45 min. lodomethylcyclopentane (27.75 g, 132.1 mmol) was then added in hexamethylphosphoramide (27.75 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (250 mL). This mixture was concentrated, diluted with water (250 mL), and extracted with ethyl acetate (3 x 300 mL). The organics were washed with a saturated aqueous lithium chloride solution (2 x 250 mL), dried over magnesium sulfate, filtered, and concentrated *in vacuo.* Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as a yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (14.1 g, 48.06 mmol) in tetrahydrofuran/water (300 mL, 3:1) was treated with lithium hydroxide (4.35 g, 103.67 mmol). The reaction was stirred at 25°C for 21 h. The tetrahydrofuran was then removed *in vacuo*. The residue was diluted with water (75 mL) and extracted with ether (3 x 75 mL). The aqueous layer was acidified to pH = 1 with a 3N aqueous hydrochloric acid solution. The product was extracted into methylene chloride (3 x 75 mL), washed with a saturated aqueous sodium chloride solution (2 x 100 mL), dried over magnesium sulfate, filtered, and concentrated *in vacuo* to give 3-cyclopentyl-2-(4-nitro-phenyl)-propionic acid (11.97 g, 93.6%) as a yellow solid: mp 119-125°C; EI-HRMS m/e calcd for C₁₄H₁₇NO₄ (M⁺) 263.1157, found 263.1162.

A solution of 3-cyclopentyl-2-(4-nitro-phenyl)-propionic acid (131 mg, 0.5 mmol) in methylene chloride (5.0 mL) was cooled to 0°C and then treated with a 2.0M solution of oxalyl chloride in methylene chloride (1.0 mL, 2.0 mmol) and a few drops of *N,N*-dimethylformamide. The reaction mixture was stirred at 0°C for 15 min and at 25°C for 30 min. The reaction mixture was then treated with a solution of 2-aminothiazole (110 mg, 1.0 mmol) in tetrahydrofuran (5 mL) and *N,N*-diisopropylethylamine (0.28 mL, 0.55 mmol). The solution was stirred at 25°C for 24 h. At this time, the reaction was concentrated *in vacuo*. Flash chromatography (Merck Silica gel 60, 230-400 mesh, 50/50 hexanes/ethyl acetate) afforded 3-cyclopentyl-2-(4-nitro-phenyl)-N-thiazol-2-yl-propionamide (38 mg, 22.4%) as a yellow solid: mp 186-187°C; EI-HRMS m/e calcd for C₁₇H₁₉N₃O₃S (M⁺) 345.1147, found 345.1148.

### (B) In an analogous manner, there was obtained:

(a) From ethyl 2-amino-4-thiazole glyoxylate and 3-cyclopentyl-2-(4-nitro-phenyl)-propionic acid: {2-[3-Cyclopentyl-2-(4-nitro-phenyl)-propionylamino]-thiazol-4-yl}-oxo-acetic acid ethyl ester (57.5%) as a white solid: mp 134-136°C; FAB-HRMS m/e calcd for C₂₁H₂₃N₃O₆S (M+H)⁺ 446.1400, found 446.1386.

### Example 23

### {2-[3-Cyclopentyl-2-(4-nitro-phenyl)-propionylamino]-thiazol-4-yl}-acetic acid ethyl ester

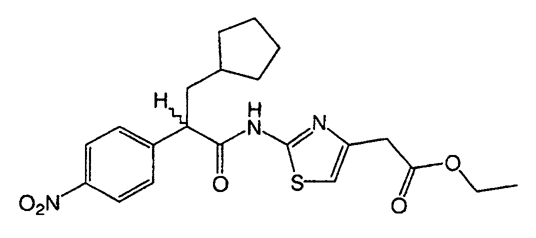

A solution of 3-cyclopentyl-2-(4-nitro-phenyl)-propionic acid (prepared in Example **22A**, 263.0 mg, 1.0 mmol) in *N*,*N*-dimethylformamide (10 mL) was treated with *O*-benzotriazol-1-yl-*N*,*N*,*N*',*N*'-tetramethyluronium hexafluorophosphate (379 mg, 1.0 mmol), (2-amino-thiazol-4-yl)-acetic acid ethyl ester (279 mg, 1.5 mmol) and *N*,*N*-diisopropylethylamine (0.34 mL, 2.0 mmol). The reaction mixture was stirred at 25°C for 5 h. The reaction mixture was then poured into a 2N aqueous hydrochloric acid solution (25 mL) and extracted with ethyl acetate (3 x 25 mL). The organic layers were combined and washed with water (1 x 75 mL.), a saturated aqueous sodium bicarbonate solution (1 x 75 mL), a saturated aqueous sodium chloride solution (3 x 75 mL), dried over sodium sulfate, filtered and concentrated *in vacuo*. Flash chromatography (Merck Silica gel 60, 230-400 mesh, 70/30 hexanes/ethyl acetate) afforded {2-[3-cyclopentyl-2-(4-nitrophenyl)-propionylamino]-thiazol-4-yl}-acetic acid ethyl ester (70.0 mg, 39.4%) as a pale yellow oil: FAB-HRMS m/e calcd for C₂₁H₂₅N₃O₅S (M+H)⁺ 432.1593, found 432.1595.

### Example 24

### {2-[3-Cyclopentyl-2-(4-nitro-phenyl)-propionylamino]-thiazol-4-yl}-acetic acid methyl ester

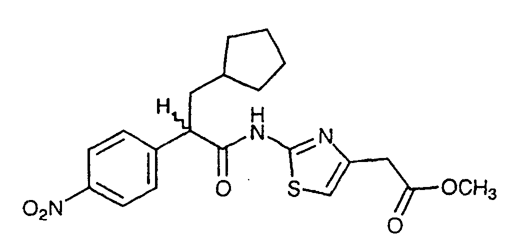

A solution of {2-[3-cyclopentyl-2-(4-nitro-phenyl)-propionylamino]-thiazol-4-yl}-acetic acid ethyl ester (prepared in Example **23**, 160 mg, 0.37 mmol) in methanol (10 mL) was treated with a catalytic amount of sulfuric acid. The reaction mixture was refluxed for 68 h. The reaction was concentrated *in vacuo*. Flash chromatography (Merck Silica gel 60, 230-400 mesh, 75/25 hexanes/ethyl acetate) afforded {2-[3-cyclopentyl-2-(4-nitrophenyl)-propionylamino]-thiazol-4-yl}-acetic acid methyl ester (82.3 mg, 53.3%) as a pale yellow oil: FAB-HRMS m/e calcd for C₂₀H₂₃N₃O₅S (M+H)⁺ 418.1436, found 418.1424.

### Example 25

### {2-[2-(4-Amino-phenyl)-3-cyclopentyl-propionylamino]-thiazol-4-yl}-acetic acid methyl ester

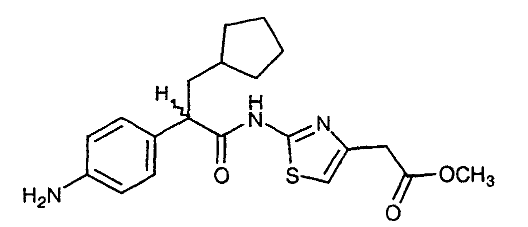

A solution of {2-[3-cyclopentyl-2-(4-nitro-phenyl)-propionylamino]-thiazol-4-yl}-acetic acid methyl ester (prepared in Example **24**, 75.3 mg, 0.18 mmol) in ethyl acetate (25 mL) was treated with 10% palladium on activated carbon. The reaction mixture was stirred under hydrogen gas at 60 psi at 25°C for 4 h. The catalyst was then filtered off through a pad of celite (ethyl acetate). The filtrate was concentrated *in vacuo* to give {2-[2-(4-amino-phenyl)-3-cyclopentyl-propionylamino]-thiazol-4-yl}-acetic acid methyl ester (64.5 mg, 93.3%) as a tan oil: EI-HRMS m/e calcd for C₂₀H₂₅N₃O₃S (M⁺) 387.1616, found 387.1612.

### Example 26

### 2-[3-Cyclopentyl-2-(4-nitro-phenyl)-propionylamino]-thiazole-4-carboxylic acid methyl ester

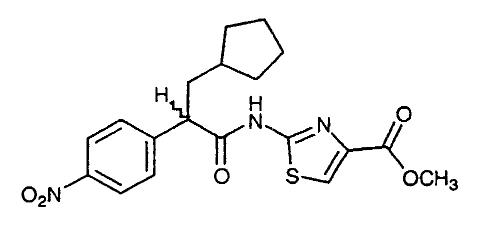

A solution of 2-[3-cyclopentyl-2-(4-nitro-phenyl)-propionylamino]-thiazole-4-carboxylic acid ethyl ester (prepared in Example **39**(B)(b), 135 mg, 0.32 mmol) in methanol (10 mL) was treated with a catalytic amount of sulfuric acid. The reaction mixture was refluxed for 68 h. The reaction was concentrated *in vacuo.* Flash chromatography (Merck Silica gel 60, 230-400 mesh, 50/50 hexanes/ethyl acetate) afforded 2-[3-cyclopentyl-2-(4-nitrophenyl)-propionylamino]-thiazole-4-carboxylic acid methyl ester (71.4 mg, 54.8%) as a pale yellow solid: EI-HRMS m/e calcd for C₁₉H₂₁N₃O₅S (M⁺) 403.1201, found 403.1188.

### Example 27

### 2-[2-(4-Amino-phenyl)-3-cyclopentyl-propionylamino]-thiazole-4-carboxylic acid methyl ester

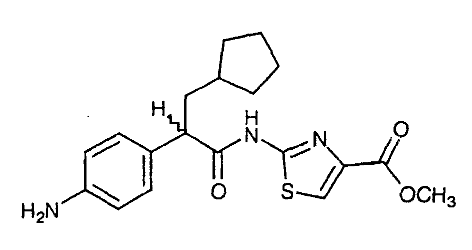

A solution of 2-[3-cyclopentyl-2-(4-nitro-phenyl)-propionylamino]-thiazole-4-carboxylic acid methyl ester (prepared in Example **26**, 60.0 mg, 0.14 mmol) in ethyl acetate (25 mL) was treated with 10% palladium on activated carbon. The reaction mixture was stirred under hydrogen gas at 60 psi at 25°C for 4.5 h. The catalyst was then filtered off through a pad of celite (ethyl acetate). The filtrate was concentrated *in vacuo* to give 2-[2-(4-amino-phenyl)-3-cyclopentyl-propionylamino]-thiazole-4-carboxylic acid methyl ester (61.3 mg, 100%) as a pale yellow oil: EI-HRMS m/e calcd for C₁₉H₂₃N₃O₃S (M⁺) 373.1460, found 373.1454.

### Example 28

### (A) {2-[2-(3-Chloro-phenyl)-3-cyclopentyl-propionylamino]-thiazol-4-yl}-acetic acid ethyl ester

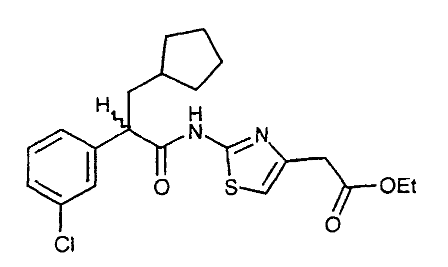

A solution of freshly prepared lithium diisopropylamide (141.3 mL of a 0.32M stock solution, 45.0 mmol) cooled to -78°C was treated with (3-chloro-phenyl)-acetic acid (3.41 g, 20.0 mmol) in tetrahydrofuran/1,3-dimethyl-3,4,5,6-tetrahydro-2(1H)-pyrimidinone (49.7 mL, 3:1). The resulting reaction solution was stirred at -78°C for 1 h. Iodomethylcyclopentane (4.64 g, 22.08 mmol) was then added in 1,3-dimethyl-3,4,5,6-tetrahydro-2(1H)-pyrimidinone (4.64 mL). The reaction mixture was stirred at -78°C for 4 h. The reaction was then warmed to 25°C and was stirred at 25°C for 48 h. The solution was then quenched by the slow addition of the reaction mixture to a 2N aqueous hydrochloric acid solution (50 mL). The product was extracted into ethyl acetate (1 x 150 mL), dried over sodium sulfate, filtered, and concentrated *in vacuo*. Flash chromatography (Merck Silica gel 60, 230-400 mesh, 85/15 hexanes/ethyl acetate) afforded 2-(3-chloro-phenyl)-3-cyclopentyl-propionic acid (3.68 g, 72.9%) as a yellow solid: mp 70-72°C; EI-HRMS m/e calcd for C₁₄H₁₇ClO₂ (M⁺) 252.0917, found 252.0915.

A solution of 2-(3-chloro-phenyl)-3-cyclopentyl-propionic acid (252 mg, 1.0 mmol) in methylene chloride (10 mL) was cooled to 0°C and then treated with a 2.0M solution oxalyl chloride in methylene chloride (0.6 mL, 1.2 mmol) and a few drops of *N,N*-dimethylformamide. The reaction mixture was stirred at 0°C for 15 min and at 25°C for 2 h. The reaction mixture was then treated with (2-amino-thiazol-4-yl)-acetic acid ethyl ester (409 mg, 2.2 mmol) and *N*,*N*-diisopropylethylamine (0.5 mL, 2.4 mmol). This solution was stirred at 25°C for 48 h. At this time, the reaction was concentrated *in vacuo*. Flash chromatography (Merck Silica gel 60, 230-400 mesh, 80/20 hexanes/ethyl acetate) afforded {2-[2-(3-chloro-phenyl)-3-cyclopentyl-propionylamino]-thiazol-4-yl}-acetic acid ethyl ester (254 mg, 60.3%) as a white solid: mp 121-125°C; EI-HRMS m/e calcd for C₂₁H₂₅ClN₂O₃S (M⁺) 420.1274, found 420.1268.

### (B) In an analogous manner, there were obtained:

(a) From 2-amino-thiazole-4-carboxylic acid ethyl ester and 2-(3-chloro-phenyl)-3-cyclopentyl-propionic acid: 2-[2-(3-Chloro-phenyl)-3-cyclopentyl-propionylamino]-thiazole-4-carboxylic acid ethyl ester as a white solid: mp 167-168°C; EI-HRMS m/e calcd for C₂₀H₂₃ClN₂O₃S (M⁺) 406.1117, found 406.1103.
(b) From 2-amino-pyridine and 2-(3-chloro-phenyl)-3-cyclopentyl-propionic acid: 2-(3-Chloro-phenyl)-3-cyclopentyl-N-pyridin-2-yl-propionamide as a clear oil: EI-HRMS m/e calcd for C₁₉H₂₁ClN₂O (M⁺) 328.1342, found 328.1333.
(c) From 6-amino-nicotinic acid methyl ester and 2-(3-chloro-phenyl)-3-cyclopentylpropionic acid: 6-[2-(3-Chloro-phenyl)-3-cyclopentyl-propionylamino]-nicotinic acid methyl ester as a colorless oil: EI-HRMS m/e calcd for C₂₁H₂₃ClN₂O₃ (M⁺) 386.1397, found 386.1398.

### Example 29

### {2-[2-(3-Chloro-phenyl)-3-cyclopentyl-propionylamino]-thiazol-4-yl}-acetic acid methyl ester

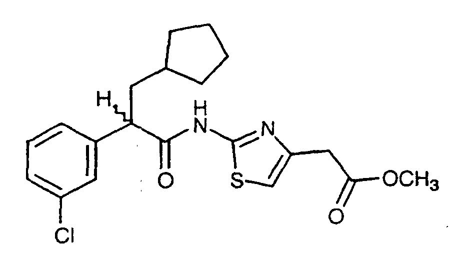

A solution of {2-[2-(3-chloro-phenyl)-3-cyclopentyl-propionylamino]-thiazol-4-yl}-acetic acid ethyl ester (prepared in Example **28**, 177.2 mg, 0.42 mmol) in methanol (15 mL) was treated with a catalytic amount of sulfuric acid. The reaction mixture was refluxed for 40 h. The reaction was then concentrated *in vacuo*. Flash chromatography (Merck Silica gel 60, 230-400 mesh, 70/30 hexanes/ethyl acetate) afforded {2-[2-(3-chloro-phenyl)-3-cyclopentyl-propionylamino]-thiazol-4-yl}-acetic acid methyl ester (104.4 mg, 60.9%) as a clear oil: EI-HRMS m/e calcd for C₂₀H₂₃ClN₂O₃S (M⁺) 406.1117, found 406.1118.

### Example 30

### 2-[2-(3-Chloro-phenyl)-3-cyclopentyl-propionylamino]-thiazole-4-carboxylic acid methyl ester

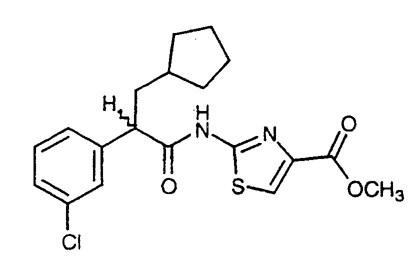

A solution of 2-[2-(3-chloro-phenyl)-3-cyclopentyl-propionylamino]-thiazole-4-carboxylic acid ethyl ester (prepared in Example **28**(B)(a), 94.5 mg, 0.23 mmol) in methanol (15 mL) was treated with a catalytic amount of sulfuric acid. The reaction mixture was refluxed for 40 h. The reaction was then concentrated *in vacuo.* Flash chromatography (Merck Silica gel 60, 230-400 mesh, 75/25 hexanes/ethyl acetate) afforded 2-[2-(3-chloro-phenyl)-3-cyclopentyl-propionylamino]-thiazole-4-carboxylic acid methyl ester (36.8 mg, 40.3%) as a white solid: mp 95-98°C; EI-HRMS m/e calcd for C₁₉H₂₁ClN₂O₃S (M⁺) 392.0961, found 392.0989.

### Example 31

### (A) {2-[2-(4-Chloro-phenyl)-3-cyclopentyl-propionylamino]-thiazol-4-yl}-acetic acid ethyl ester

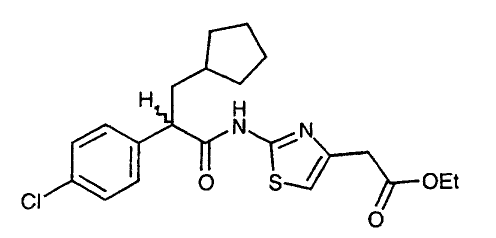

A solution of freshly prepared lithium diisopropylamide (78.0 mL of a 0.91M stock solution, 70.98 mmol) cooled to -78°C was treated with (4-chloro-phenyl)-acetic acid (5.76 g, 33.8 mmol) in tetrahydrofuran/1,3-dimethyl-3,4,5,6-tetrahydro-2(1H)-pyrimidinone (84 mL, 3:1). The resulting solution was stirred at -78°C for 1 h. Iodomethylcyclopentane (7.45 g, 35.49 mmol) was then added in 1,3-dimethyl-3,4,5,6-tetrahydro-2(1H)-pyrimidinone (2 mL). This solution was stirred at -78°C for 4 h. The reaction was then warmed to 25°C and was stirred at 25°C for 16 h. The reaction mixture was then quenched by the dropwise addition of a saturated aqueous ammonium chloride solution (20 mL). The excess solvent was removed *in vacuo*. The residue was acidified to pH = I with a 1N aqueous hydrochloric acid solution. The mixture was then poured into water (150 mL) and extracted with ethyl acetate (3 x 50 mL). The organics were dried over sodium sulfate, filtered, and concentrated *in vacuo*. Flash chromatography (Merck Silica gel 60, 230-400 mesh, 80/20 hexanes/ethyl acetate) afforded 2-(4-chlorophenyl)-3-cyclopentyl-propionic acid (6.76 g, 79.1%) as a yellow solid: mp 82-84°C: EI-HRMS m/e calcd for C₁₄H₁₇ClO₂ (M⁺) 252.0917, found 252.0906.

A solution of 2-(4-chloro-phenyl)-3-cyclopentyl-propionic acid (252 mg, 1.0 mmol) in methylene chloride (10 mL) was cooled to 0°C and then treated with 2.0M solution of oxalyl chloride in methylene chloride (0.55 mL, 1.1 mmol) and a few drops of *N,N*-dimethylformamide. The reaction mixture was stirred at 0°C for 15 min and then at 25°C for 1.5 h. The reaction mixture was then treated with (2-amino-thiazol-4-yl)-acetic acid ethyl ester (409 mg, 2.2 mmol) and *N,N*-diisopropylethylamine (0.5 mL, 2.4 mmol). This solution was stirred at 25°C for 24 h. At this time, the reaction was concentrated in *vacuo*. Flash chromatography (Merck Silica gel 60, 230-400 mesh, 85/15 hexanes/ethyl acetate) afforded {2-[2-(4-chloro-phenyl)-3-cyclopentyl-propionylamino]-thiazol-4-yl]-acetic acid ethyl ester (183.3 mg, 43.5%) as a pale yellow oil: EI-HRMS m/e calcd for C₂₁H₂₅ClN₂O₃S (M⁺) 420.1274, found 420.1272.

### (B) In an analogous manner, there were obtained:

(a) From 2-amino-thiazole-4-carboxylic acid ethyl ester and 2-(4-chloro-phenyl)-3-cyclopentyl-propionic acid: 2-[2-(4-Chloro-phenyl)-3-cyclopentyl-propionylamino]-thiazole-4-carboxylic acid ethyl ester as a white solid: mp 114-116°C; EI-HRMS m/e calcd for C₂₀H₂₃ClN₂O₃S (M⁺) 406.1117, found 406.1119.
(b) From 2-amino-pyridine and 2-(4-chloro-phenyl)-3-cyclopentyl-propionic acid: 2-(4-Chloro-phenyl)-3-cyclopentyl-N-pyridin-2-yl-propionamide as a clear oil: EI-HRMS m/e calcd for C₁₉H₂₁ClN₂O (M⁺) 328.1342, found 328.1355.
(c) From 6-amino-nicotinic acid methyl ester and 2-(4-chloro-phenyl)-3-cyclopentylpropionic acid: 6-[2-(4-Chloro-phenyl)-3-cyclopentyl-propionylamino]-nicotinic acid methyl ester as a white foam: EI-HRMS m/e calcd for C₂₁H₂₃ClN₂O₃ (M⁺) 386.1397, found 386.1384.

### Example 32

### 2-[2-(4-Chloro-phenyl)-3-cyclopentyl-propionylamino]-thiazole-4-carboxylic acid methyl ester:

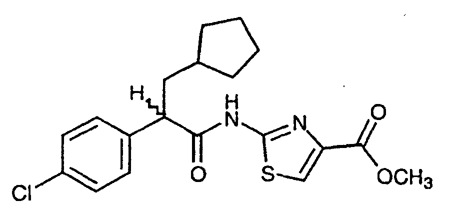

A solution of 2-[2-(4-chloro-phenyl)-3-cyclopentyl-propionylamino]-thiazole-4-carboxylic acid ethyl ester (prepared in Example **31**(B)(a), 105 mg, 0.25 mmol) in methanol (10 mL) was treated with a catalytic amount of sulfuric acid. The reaction mixture was refluxed for 68 h. The reaction was concentrated *in vacuo*. High pressure liquid chromatography (Chromegasphere SI-60, 10 µm, 60 Å, 25 cm X 23 cm ID, 75/25 heptane/ethyl acetate) afforded 2-[2-(4-chloro-phenyl)-3-cyclopentyl-propionylamino]-thiazole-4-carboxylic acid methyl ester (41.3 mg, 40.7%) as a white solid: mp 156-157°C; EI-HRMS m/e calcd for C₁₉H₂₁ClN₂O₃S (M⁺) 392.0961, found 392.0956.

### Example 33

### {2-(2-(4-Chloro-phenyl)-3-cyclopentyl-propionylamino]-thiazol-4-yl}-acetic acid methyl ester

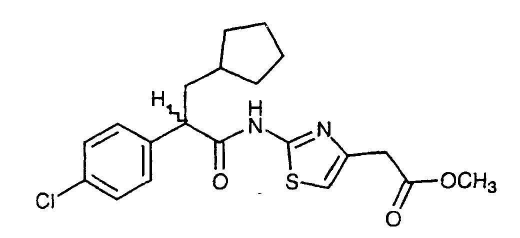

A solution of {2-[2-(4-chloro-phenyl)-3-cyclopentyl-propionylamino]-thiazol-4-yl}-acetic acid ethyl ester (prepared in Example **31**A, 76.1 mg, 0.18 mmol) in methanol (5 mL) was treated with a catalytic amount of sulfuric acid. The reaction mixture was refluxed for 72 h. The reaction was concentrated *in vacuo*. High pressure liquid chromatography (Chromegasphere SI-60, 10 µM, 60Å, 25cm x 23cm ID, 75/25 heptane/ethyl acetate) afforded {2-[3-cyclopentyl-2-(4-nitro-phenyl)-propionylamino]-thiazol-4-yl}-acetic acid methyl ester (21.5 mg, 29.2%) as a colorless oil: EI-HRMS m/e calcd for C₂₀H₂₃ClN₂O₃S (M⁺) 406.1117, found 406.1114.

### Example 34

### 2-(4-Chloro-phenyl)-3-cyclopentyl-N-(5-hydroxymethyl-thiazol-2-yl)-propionamide

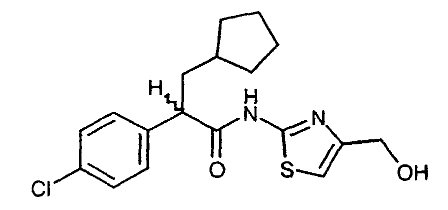

A solution of 2-[2-(4-chloro-phenyl)-3-cyclopentyl-propionylamino]-thiazole-4-carboxylic acid methyl ester (prepared in Example **32**, 127.7 mg, 0.31 mmol) in tetrahydrofuran (0.4 mL) was added to a slurry of lithium aluminum hydride (15.0 mg, 0.39 mmol) in tetrahydrofuran (2.24 mL) at 0°C. The reaction mixture was stirred at 0°C for 2 h. The reaction was then quenched by the dropwise addition of water. The reaction was then diluted with more water (25 mL) and extracted with ethyl acetate (3 x 25 mL). The organics were dried over sodium sulfate, filtered and concentrated *in vacuo*. Flash chromatography (Merck Silica gel 60, 230-400 mesh, 75/25 hexanes/ethyl acetate) afforded 2-(4-chloro-phenyl)-3-cyclopentyl-N-(5-hydroxymethyl-thiazol-2-yl)-propionamide (63.4 mg, 55.4%) as a white solid: mp 115-117°C; EI-HRMS m/e calcd for C₁₈H₂₁ClN₂O₂S (M⁺) 364.1012, found 364.1004.

### Example 35

### 3-Cyclopentyl-N-(4-hydroxymethyl-thiazol-2-yl)-2-(4-methanesulfonyl-phenyl)-propionamide

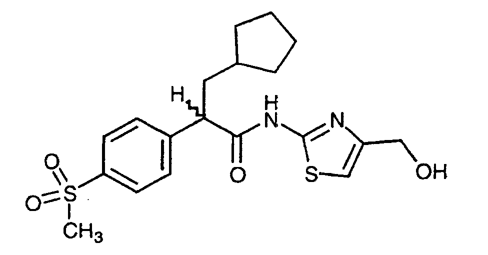

A solution of 2-[3-cyclopentyl-2-(4-methanesulfonyl-phenyl)-propionylamino]-thiazole-4-carboxylic acid ethyl ester (prepared in Example 3(B)(b), 130 mg, 0.29 mmol) in diethyl ether (2 mL) was cooled to 0°C and then slowly treated with lithium aluminum hydride (17 mg, 0.44 mmol). The reaction mixture was allowed to warm to 25°C where it was stirred for 4 h. After 4 h at 25°C, thin layer chromatography indicated the presence of starting material. An additional amount of lithium aluminum hydride (11 mg, 0.29 mmol) was added to the reaction mixture, and the reaction mixture was allowed to stir at 25°C for 15 h. The reaction mixture was then slowly quenched by the dropwise addition of water. The resulting mixture was partitioned between water and ethyl acetate. The organic layer was dried over magnesium sulfate, filtered, and concentrated *in vacuo*. Flash chromatography (Merck Silica gel 60, 230-400 mesh, ethyl acetate) afforded 3-cyclopentyl-N-(4-hydroxymethyl-thiazol-2-yl)-2-(4-methanesulfonyl-phenyl)-propionamide (55 mg, 46%) as a white solid: mp 124-126°C; EI-HRMS m/e calcd for C₁₉H₂₄N₂O₄S₂ (M⁺) 408.11-78, found 408.1164.

### Example 36

### 3-Cyclopentyl-N-[4-(2-hydroxyethyl)-thiazol-2-yl]-2-(4-methanesulfonyl-phenyl)-propionamide

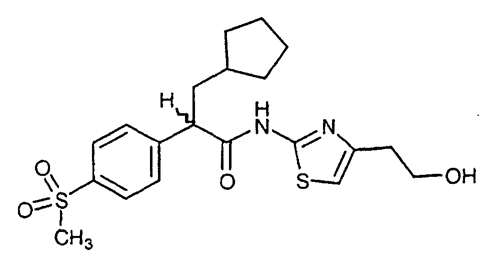

A solution of {2-[3-cyclopentyl-2-(4-methanesulfonyl-phenyl)-propionylamino]-thiazo]-4-yl}-acetic acid ethyl ester (prepared in Example **3**(B)(d), 120 mg, 0.26 mmol) in diethyl ether (500 µL) was cooled to 0°C and then slowly treated with lithium aluminum hydride (15 mg, 0.39 mmol). The reaction mixture was allowed to warm to 25°C where it was stirred for 1 h. After 1 h at 25°C, thin layer chromatography still indicated the presence of the {2-[3-cyclopentyl-2-(4-methanesulfonyl-phenyl)-propionylamino]-thiazol-4-yl}-acetic acid ethyl ester. An additional amount of lithium aluminum hydride (10 mg, 0.26 mmol) was added to the reaction mixture, and the reaction mixture was allowed to stir at 25°C for 1 h. The reaction mixture was then slowly quenched by the dropwise addition of water (10 mL). The resulting mixture was partitioned between water and ethyl acetate. The organic layer was dried over magnesium sulfate, filtered, and concentrated *in vacuo*. Flash chromatography (Merck Silica gel 60, 230-400 mesh, 1/3 hexanes/ethyl acetate) afforded 3-cyclopentyl-N-[4-(2-hydroxyethyl)-thiazol-2-yl]-2-(4-methanesulfonylphenyl)-propionamide (20 mg, 18%) as a yellow foam: mp 84-87°C; EI-HRMS m/e calcd for C₂₀H₂₆N₂O₄S₂ (M⁺) 422.1334, found 422.1335.

### Example 37

### (2R)-2-[3-Cyclopentyl-2-(3,4-dichloro-phenyl)-propionylamino]-thiazole-4-carboxylic acid methyl ester

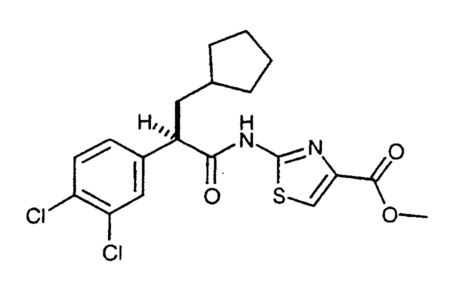

A solution triphenylphosphine (164 mg, 0.63 mmol) in methylene chloride (3 mL) was cooled to 0°C and then treated with *N*-bromosuccinimide (112 mg, 0.63 mmol) in small portions. The resulting orange reaction mixture was stirred at 0°C for 20 min and then treated with (2R)-3-cyclopentyl-2-(3,4-dichloro-phenyl)-propionic acid (prepared in Example **54**, 150 mg, 0.52 mmol). The reaction mixture was stirred at 0°C for an additional 15 min and then allowed to warm to 25°C. The reaction mixture was then treated with 2-aminothiazole-4-carboxylic acid methyl ester (181 mg, 1.15 mmol). The resulting reaction mixture was stirred at 25°C for 15 h. The crude reaction mixture was directly purified by flash chromatography (Merck Silica gel 60, 230-400 mesh, 3/1 hexanes/ethyl acetate) to afford impure (2R)-2-[3-cyclopentyl-2-(3,4-dichloro-phenyl)-propionylamino]-thiazole-4-carboxylic acid methyl ester. The impure product was diluted with ethyl acetate and then washed with a saturated aqueous sodium bicarbonate solution. The organic layer was dried over magnesium sulfate, filtered, and concentrated *in vacuo* to provide pure (2R)-2-[3-cyclopentyl-2-(3,4-dichloro-phenyl)-propionylamino]-thiazole-4-carboxylic acid methyl ester (92 mg, 41%) as a white solid: mp 143-144°C; [α]²³₅₈₉ = -10.2° (c=0.98, chloroform); EI-HRMS m/e calcd for C₁₉H₂₀Cl₂N₂O₃S (M⁺) 426.0572, found 426.0562.

### Example 38

### (A) 3-Cyclopentyl-2-(3,4-dichloro-phenyl)-N-pyridin-2-yl-propionamide:

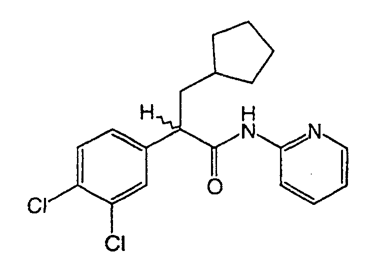

A solution of triphenylphosphine (28.80 g, 109.8 mmol) and imidazole (14.9 g, 219.6 mmol) in methylene chloride (160 mL) was cooled to 0°C and then slowly treated with iodine (27.87 g, 109.8 mmol). The reaction mixture was then treated dropwise with a solution of cyclopentylmethanol (10.0 g, 99.8 mmol) in methylene chloride (10 mL). The resulting reaction mixture was allowed to warm to 25°C, where it was stirred for 4 h. The reaction mixture was then diluted with water (50 mL), and the reaction mixture was further extracted with methylene chloride (3 x 20 mL). The combined organic layers were dried over sodium sulfate, filtered and concentrated *in vacuo* at 25°C. The resulting solid was washed with pentane (4 x 50 mL) and filtered through a silica gel plug. The filtrate was concentrated *in vacuo* at 25°C to afford iodomethylcyclopentane (18.48 g, 88%) as a clear colorless liquid: EI-HRMS m/e calcd for C₆H₁₁I₁ (M⁺) 209.9906, found 209.9911.

A solution of diisopropylamine (13.36 mL, 101.89 mmol) in tetrahydrofuran (250 mL) was cooled to -78°C under a nitrogen atmosphere and then treated with a 2.0M solution of n-butyllithium in hexanes (51 mL, 101.89 mmol). The reaction mixture was stirred at - 78°C for 15 min, at which time, a solution of 3,4-dichlorophenyl acetic acid (9.08 g, 44.3 mmol) in tetrahydrofuran (60 mL) and hexamethylphosphoramide (20 mL) was slowly added via a cannula. The bright yellow solution was allowed to stir at -78°C for 1 h, at which time, a solution of iodomethylcyclopentane (11.17 g, 53.2 mmol) in hexamethylphosphoramide (10 mL) was added via a cannula. The reaction mixture was stirred at -78°C for 1 h. The reaction mixture was then allowed to warm to 25°C, where it was stirred for 14 h. The reaction mixture was then acidified to pH = 2 by the dropwise addition of a 1N aqueous hydrochloric acid solution and extracted with ethyl acetate (3 x 50 mL). The combined organic layers were dried over sodium sulfate, filtered, and concentrated *in vacuo*. Flash chromatography (Merck Silica gel 60, 230-400 mesh, chloroform then 99/1 chloroform/methanol) afforded 3-cyclopentyl-2-(3,4-dichlorophenyl)-propionic acid (10.28 g, 81%) as a white solid: mp 74.5-76.9°C; EI-HRMS m/e calcd for C₁₄H₁₆Cl₂O₂ (M⁺) 286.0527, found 286.0534.

A solution of 3-cyclopentyl-2-(3,4-dichlorophenyl)-propionic acid (114 mg, 0.39 mmol) in methylene chloride (10 mL) was treated with 1 drop of *N,N*-dimethylformamide and then cooled to 0°C. The reaction mixture was then treated with a 2.0M solution of oxalyl chloride in methylene chloride (0.22 mL, 0.44 mmol). The reaction mixture was stirred at 0°C for 30 min and then treated with a solution of 2-aminopyridine (78 mg, 0.83 mmol) and *N,N*-diisopropylethylamine (0.16 mL, 0.95 mmol) in tetrahydrofuran (2 mL). The resulting reaction mixture was stirred at 25°C for 14 h. The reaction mixture was then diluted with water (10 mL) and extracted with methylene chloride (2 x 15 mL). The combined organic layers were dried over sodium sulfate, filtered, and concentrated *in vacuo*. Flash chromatography (Merck Silica gel 60, 230-400 mesh, hexanes then 19/1 to 4/1 hexanes/ethyl acetate) afforded 3-cyclopentyl-2-(3,4-dichloro-phenyl)-N-pyridin-2-yl-propionamide (58 mg, 50%) as a white foam: EI-HRMS m/e calcd for C₁₉H₂₀Cl₂N₂O (M⁺) 362.0953, found 362.0955.

### (B) In an analogous manner, there were obtained:

(a) From 2-amino-5-nitropyridine and 3-cyclopentyl-2-(3,4-dichlorophenyl)-propionic acid: 3-Cyclopentyl-2-(3,4-dichloro-phenyl)-N-(5-nitropyridin)-2-yl-propionamide as a yellow-orange foam: EI-HRMS m/e calcd for C₁₉H₁₉Cl₂N₃O₃ (M⁺) 407.0803, found 407.0799.
(b) From 2-amino-5-carboxymethylpyridine and 3-cyclopentyl-2-(3,4-dichlorophenyl)-propionic acid: 3-Cyclopentyl-2-(3,4-dichloro-phenyl)-N-(5-carboxymethylpyridin)-2-yl-propionamide as a white foam: EI-HRMS m/e calcd for C₂₁H₂₂Cl₂N₂O₃ (M⁺) 420.1007, found 420.0994.
(c) From 4-aminopyrimidine and 3-cyclopentyl-2-(3,4-dichlorophenyl)-propionic acid: 3-Cyclopentyl-2-(3,4-dichloro-phenyl)-N-pyrimidine-6-yl-propionamide as a white foam: EI-HRMS m/e calcd for C₁₈H₁₉Cl₂N₃O (M⁺) 363.0905, found 363.0910.
(d) From 2-amino-5-methylpyridine and 3-cyclopentyl-2-(3,4-dichlorophenyl)-propionic acid: 3-Cyclopentyl-2-(3,4-dichloro-phenyl)-N-(5-methylpyridin)-2-yl-propionamide as a white solid: EI-HRMS m/e calcd for C₂₀H₂₂Cl₂N₂O (M⁺) 376.1109, found 376.1119.
(e) From 2-amino-4-methylpyridine and 3-cyclopentyl-2-(3,4-dichlorophenyl)-propionic acid: 3-Cyclopentyl-2-(3,4-dichloro-phenyl)-N-(4-methylpyridin)-2-yl-propionamide as a white solid: EI-HRMS m/e calcd for C₂₀H₂₂Cl₂N₂O (M⁺) 376.1109, found 376.1106.
(f) From 2-amino-6-methylpyridine and 3-cyclopentyl-2-(3,4-dichlorophenyl)-propionic acid: 3-Cyclopentyl-2-(3,4-dichloro-phenyl)-N-(6-methylpyridin)-2-yl-propionamide as a light - yellow solid: EI-HRMS m/e calcd for C₂₀H₂₂Cl₂N₂O (M⁺) 376.1109, found 376.1107.
(g) From 2-amino-5-chloropyridine and 3-cyclopentyl-2-(3,4-dichlorophenyl)-propionic acid: 3-Cyclopentyl-2-(3,4-dichloro-phenyl)-N-(5-chloropyridin)-2-yl-propionamide as a white foam: EI-HRMS m/e calcd for C₁₉H₁₉Cl₃N₂O (M⁺) 396.0563, found 396.0564.
(h) From 2-amino-5-bromopyridine and 3-cyclopentyl-2-(3,4-dichlorophenyl)-propionic acid: 3-Cyclopentyl-2-(3,4-dichloro-phenyl)-N-(5-bromopyridin)-2-yl-propionarnide as a white solid: EI-HRMS m/e calcd for C₁₉H₁₉BrCl₂N₂O (M⁺) 440.0058, found 440.0066.

### Example 39

### (A) 3-Cyclopentyl-2-(4-nitro-phenyl)-N-pyridin-2-yl-propionamide

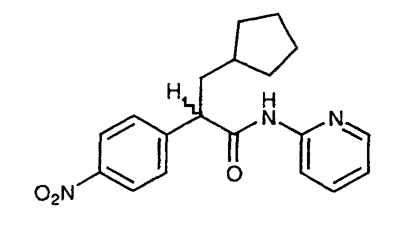

A solution of 3-cyclopentyl-2-(4-nitro-phenyl)-propionic acid (prepared in Example 22, 263 mg, 1.0 mmol) in methylene chloride (5 mL) was cooled to 0°C and then treated with a 2.0M solution of oxalyl chloride in methylene chloride (0.6 mL, 1.2 mmol) and a few drops of *N*,*N*-dimethylformamide. The reaction mixture was stirred at 0°C for 15 min and then at 25°C for 1 h. The reaction mixture was then treated with a solution of 2-aminopyridine (207 mg, 2.2 mmol) in tetrahydrofuran (5 mL) and *N*,*N*-diisopropylethylamine (0.42 mL, 2.5 mmol). The reaction mixture was stirred at 25°C for 24 h. At this time, the reaction was concentrated *in vacuo.* Flash chromatography (Merck Silica gel 60, 230-400 mesh, 80/20 hexanes/ethyl acetate) afforded 3-cyclopentyl-2-(4-nitro-phenyl)-N-pyridin-2-yl-propionamide (110.2 mg, 32.5%) as a white solid: mp 152-154°C; EI-HRMS m/e calcd for C₁₉H₂₁N₃O₃ (M⁺) 339.1582, found 339.1581.

### (B) In an analogous manner, there were obtained:

(a) From 4-aminopyrimidine and 3-cyclopentyl-2-(4-nitro-phenyl)-propionic acid: 3-Cyclopentyl-2-(4-nitro-phenyl)-N-pyrimidin-4-yl-propionamide as a white solid: mp 152-153°C; EI-HRMS m/e calcd for C₁₈H₂₀N₄O₃ (M⁺) 340.1535, found 340.1533.
(b) From 2-amino-thiazole-4-carboxylic acid ethyl ester and 3-cyclopentyl-2-(4-nitrophenyl)-propionic acid: 2-[3-Cyclopentyl-2-(4-nitro-phenyl)-propionylamino]-thiazole-4-carboxylic acid ethyl ester as a pale yellow solid: mp 110-115°C; EI-HRMS m/e calcd for C₂₀H₂₃N₃O₅S (M⁺) 417.1358, found 417.1346.

### Example 40

### 3-Cyclopentyl-2-(4-methylsulfanyl-phenyl)-N-pyridin-2-yl-propionamide

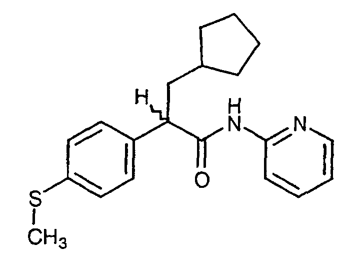

A solution of diisopropylamine (3.2 mL, 23.16 mmol) in dry tetrahydrofuran (10.3 mL) and 1,3-dimethyl-3,4,5,6-tetrahydro-2(1H)-pyrimidinone (3.4 mL) was cooled to -78°C under nitrogen and then treated with a 10M solution of *n*-butyllithium in hexanes (2.3 mL, 23.16 mmol). The resulting reaction mixture was stirred at -78°C for 30 min and then treated dropwise with a solution of 4-(methylthio)phenylacetic acid (2.01 g, 11.03 mmol) in dry tetrahydrofuran (10.3 mL) and 1,3-dimethyl-3,4,5,6-tetrahydro-2(1H)-pyrimidinone (3.4 mL). The reaction mixture was allowed to stir at -78°C for 1 h, at which time, a solution of iodomethylcyclopentane (2.55 g, 12.13 mmol) in a small amount of dry tetrahydrofuran was added dropwise. The reaction mixture was stirred at - 78°C for 30 min and then allowed to warm to 25°C where it was stirred for 24 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 200 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-methylsulfanyl-phenyl)propionic acid (1.01 g, 35%) as a cream solid: mp 91-93°C; EI-HRMS m/e calcd for C₁₅H₂₀O₂S (M⁺) 264.1184, found 264.1177.

A solution of 3-cyclopentyl-2-(4-methylsulfanyl-phenyl)propionic acid (200 mg, 0.76 mmol) and triphenylphosphine (198 mg, 0.76 mmol) in methylene chloride (2 mL) was cooled to 0°C and then treated with *N*-bromosuccinimide (150 mg, 0.84 mmol) in small portions. After the complete addition of *N*-bromosuccinimide, the reaction mixture was allowed to warm to 25°C over 30 min. The orange reaction mixture was then treated with 2-aminopyridine (151 mg, 1.60 mmol), and the resulting reaction mixture was stirred at 25°C for 15 h. The reaction mixture was then concentrated *in vacuo* to remove methylene chloride. The remaining residue was partitioned between water and ethyl acetate. The organic layer was washed with a 1N aqueous hydrochloric acid solution, washed with a saturated aqueous sodium bicarbonate solution, dried over magnesium sulfate, filtered, and concentrated *in vacuo*. Flash chromatography (Merck Silica gel 60, 230-400 mesh, 3/1 hexanes/ethyl acetate) afforded 3-cyclopentyl-2-(4-methylsulfanyl-phenyl)-N-pyridin-2-yl-propionamide (83 mg, 32%) as a white solid: mp 127-128°C; EI-HRMS m/e calcd for C₂₀H₂₄N₂OS (M⁺) 340.1609, found 340.1611.

### Example 41

### 3-Cyclopentyl-N-pyridin-2-yl-2-(4-trifluoromethylsulfanyl-phenyl)-propionamide

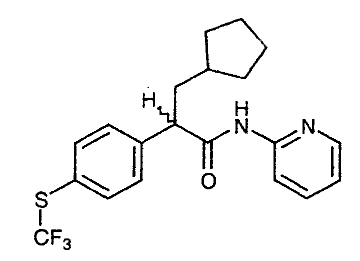

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (59.6 mg, 0.187 mmol) and triphenylphosphine (49.1 mg, 0.187 mmol) in methylene chloride (468 µL) was cooled to 0°C and then treated with *N*-bromosuccinimide (36.7 mg, 0.206 mmol) in small portions. After the complete addition of *N*-bromosuccinimide, the reaction mixture was allowed to warm to 25°C over 30 min. The orange reaction mixture was then treated with 2-aminopyridine (35.2 mg, 0.374 mmol). The resulting reaction mixture was stirred at 25°C for 16 h. The reaction mixture was then concentrated *in vacuo* to remove methylene chloride. The remaining residue was diluted with ethyl acetate (50 mL). The organic layer was washed with a 10% aqueous hydrochloric acid solution (1 x 50 mL), washed with a saturated aqueous sodium bicarbonate solution (1 x 50 mL), washed with water (1 x 50 mL), dried over sodium sulfate, filtered, and concentrated *in vacuo*. Flash chromatography (Merck Silica gel 60, 70-230 mesh, 9/1 hexanes/ethyl acetate) afforded 3-cyclopentyl-N-pyridin-2-yl-2-(4-trifluoromethylsulfanyl-phenyl)-propionamide (25.0 mg, 34%) as a cream solid: mp 101-102°C; EI-HRMS m/e calcd for C₂₀H₂₁F₃N₂OS (M⁺) 394.1327, found 394.1321.

### Example 42

### 3-Cyclopentyl-2-(4-methanesulfonyl-phenyl)-N-pyridin-2-yl-propionamide

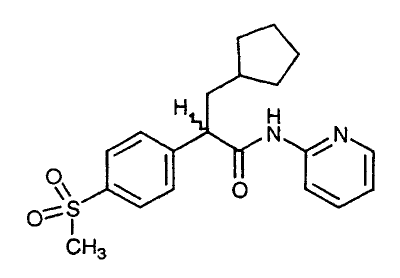

A solution of 2-aminopyridine (95 mg, 1.01 mmol) in acetonitrile (2 mL) was treated with 3-cyclopentyl-2-(4-methanesulfonyl-phenyl)propionic acid (prepared in Example 3(A), 250 mg, 0.84 mmol), triphenylphosphine (243 mg, 0.93 mmol), triethylamine (350 µL, 2.53 mmol), and carbon tetrachloride (1 mL). The resulting reaction mixture was stirred at 25°C for 15 h. The cloudy reaction mixture was diluted with water and then extracted with methylene chloride. The organic layer was dried over magnesium sulfate, filtered, and concentrated *in vacuo*. Flash chromatography (Merck Silica gel 60, 230-400 mesh, 1/3 hexanes/ethyl acetate) afforded impure 3-cyclopentyl-2-(4-methanesulfonyl-phenyl)-N-pyridin-2-yl-propionamide. Recrystallization from hexanes/methylene chloride provided pure 3-cyclopentyl-2-(4-methanesulfonyl-phenyl)-N-pyridin-2-yl-propionamide (170 mg, 54%) as a white solid: mp 172-173°C; EI-HRMS m/e calcd for C₂₀H₂₄N₂O₃S (M⁺) 372.1508, found 372.1498.

### Example 43

### (A) 3-Cyclopentyl-N-pyridin-2-yl-2-(4-trifluoromethanesulfonyl-phenyl)-propionamide

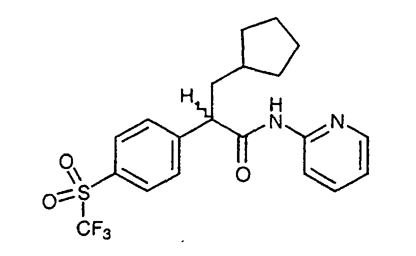

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanylphenyl)propionic acid methyl ester (1.37 g, 99%) as a light yellow oil: EI-HRMS m/e calcd for C₁₆H₁₉F₃O₂S (M⁺) 332.1058, found 332.1052.

A solution of 3-cyclopentyl-2-(4-trifluoromethylsulfanyl-phenyl)propionic acid methyl ester (1.14 g, 3.43 mmol) in methylene chloride (8.6 mL) was treated with 3-chloroperoxybenzoic acid (80-85% grade, 2.00 g based on 80%, 9.26 mmol). The reaction mixture was stirred at 25°C for 17 h, at which time, thin layer chromatography showed the presence of two new lower R_{f} products. An additional 2.00 g of 3-chloroperoxybenzoic acid was added to the reaction mixture to drive the conversion of the sulfoxide to the sulfone, and the resulting reaction mixture was stirred at 25°C for 3 d. The reaction mixture was concentrated *in vacuo* to remove methylene chloride. The resulting residue was diluted with ethyl acetate (300 mL). The organic phase was washed with a saturated aqueous sodium bicarbonate solution (3 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 19/1 hexanes/ethyl acetate) afforded 3-cyclopentyl-2-(4-trifluoromethanesulfonylphenyl)propionic acid methyl ester (1.19 g, 95%) as a light yellow oil: EI-HRMS m/e calcd for C₁₆H₁₉F₃O₄S (M⁺) 364.0956, found 364.0965.

A solution of 3-cyclopentyl-2-(4-trifluoromethanesulfonyl-phenyl)propionic acid methyl ester (708.2 mg, 1.94 mmol) in tetrahydrofuran (2.4 mL) was treated with a 0.8M aqueous lithium hydroxide solution (3.6 mL, 2.92 mmol). The reaction mixture was stirred at 25°C for 23 h and then concentrated *in vacuo* to remove tetrahydrofuran. The remaining aqueous layer was acidified to pH = 2 with a 10% aqueous hydrochloric acid solution and then extracted with ethyl acetate (2 x 100 mL). The combined organic layers were washed with a saturated aqueous sodium chloride solution (1 x 100 mL), dried over sodium sulfate, filtered, and concentrated *in vacuo* to afford a cream solid. This solid was purified by triturating with diethyl ether/petroleum ether to provide pure 3-cyclopentyl-2-(4-trifluoromethanesulfonyl-phenyl)propionic acid (527.0 mg, 77%) as a white solid: mp 143-145°C; EI-HRMS m/e calcd for C₁₅H₁₇F₃O₄S (M⁺) 350.0800, found 350.0816.

A solution of 3-cyclopentyl-2-(4-trifluoromethanesulfonyl-phenyl)propionic acid (118.9 mg, 0.34 mmol) and triphenylphosphine (133.5 mg, 0.51 mmol) in methylene chloride (848 µL) was cooled to 0°C and then treated with *N*-bromosuccinimide (102.7 mg, 0.58 mmol) in small portions. After the complete addition of *N*-bromosuccinimide, the reaction mixture was allowed to warm to 25°C where it was stirred for 45 min. The reaction mixture was then treated with 2-aminopyridine (95.8 mg, 1.02 mmol). The resulting reaction mixture was stirred at 25°C for 22 h. The reaction mixture was then concentrated *in vacuo*. Flash chromatography (Merck Silica gel 60, 230-400 mesh, 5/1 hexanes/ethyl acetate) afforded 3-cyclopentyl-N-pyridin-2-yl-2-(4-trifluoromethanesulfonyl-phenyl)-propionamide (37.1 mg, 26%) as a light yellow solid: mp 151-153°C; EI-HRMS m/e calcd for C₂₀H₂₁F₃N₂O₃S (M⁺) 426.1225, found 426.1220.

### (B) In an analogous manner, there were obtained:

(a) From 3-cyclopentyl-2-(4-trifluoromethanesulfonyl-phenyl)propionic acid and 2-amino-5-chloropyridine: N-(5-Chloro-pyridin-2-yl)-3-cyclopentyl-2-(4-trifluoromethanesulfonyl-phenyl)-propionamide as a cream solid: mp 146-148°C; EI-HRMS m/e calcd for C₂₀H₂₀ClF₃N₂O₃S (M⁺) 460.0835, found 460.0846.
(b) From 3-cyclopentyl-2-(4-trifluoromethanesulfonyl-phenyl)propionic acid and 2-amino-5-methyl pyridine: 3-Cyclopentyl-N-(5-methyl-pyridin-2-yl)-2-(4-trifluoromethanesulfonyl-phenyl)-propionamide as a pale yellow solid: mp 155-157°C; EI-HRMS m/e calcd for C₂₁H₂₃F₃N₂O₃S (M⁺) 440.1381, found 440.1376.
(c) From 3-cyclopentyl-2-(4-trifluoromethanesulfonyl-phenyl)propionic acid and 6-aminonicotinic acid methyl ester: 6-[3-Cyclopentyl-2-(4-trifluoromethanesulfonyl-phenyl)-propionylamino]-nicotinic acid methyl ester as a yellow foam: mp 58-62°C; EI-HRMS m/e calcd for C₂₂H₂₃F₃N₂O₅S (M⁺) 484.1280, found 484.1274.

### Example 44

### 3-Cyclopentyl-2-(4-methanesulfonyl-3-nitrophenyl)-N-pyridin-2-yl-propionamide

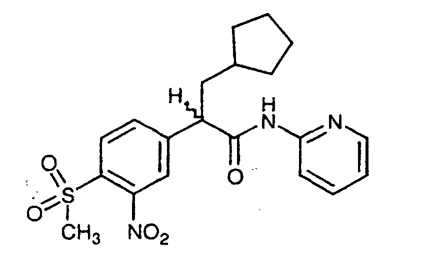

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst@ 15 ion exchange resin. The filtrate was concentrated *in vacuo*. Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A solution of triphenylphosphine (138 mg, 0.53 mmol) in methylene chloride (2 mL) was cooled to 0°C and then slowly treated with *N*-bromosuccinimide (94 mg, 0.53 mmol) in small portions. The reaction mixture was stirred at 0°C for 10 min and then treated with 3-cyclopentyl-2-(4-methanesulfonyl-3-nitrophenyl)-propionic acid (150 mg, 0.44 mmol). The resulting reaction mixture was stirred at 0°C for 5 min and then allowed to warm to 25°C, where it was stirred for 25 min. The reaction mixture was then treated with 2-aminopyridine (91 mg, 0.97 mmol). The resulting reaction mixture was stirred at 25°C for 15 h. The crude reaction mixture was directly purified by flash chromatography (Merck Silica gel 60, 230-400 mesh, 1/1 hexanes/ethyl acetate) to afford 3-cyclopentyl-2-(4-methanesulfonyl-3-nitrophenyl)-N-pyridin-2-yl-propionamide (106 mg, 58%) as a white foam: mp 92-95°C (foam to gel); FAB-HRMS m/e calcd for C₂₀H₂₃N₃O₅S (M+H)⁺ 418.1436, found 418.1430.

### Example 45

### 6-[3-Cyclopentyl-2(R)-(3,4-dichloro-phenyl)-propionylamino]-nicotinic acid methyl ester

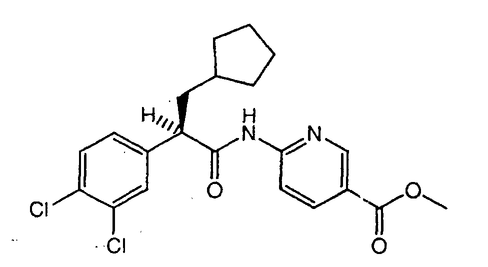

A mixture of 6-aminonicotinic acid (4.0 g, 28.9 mmol), methanol (75 mL), and concentrated hydrochloric acid (4 mL) was heated under reflux for 16 h. The reaction mixture was allowed to cool to 25°C and then concentrated *in vacuo* to remove methanol. The resulting solid was treated with water (20 mL) and enough sodium bicarbonate to adjust the pH = 8. The solution was then extracted with ethyl acetate (3 x 25 mL). The combined organic layers were dried over sodium sulfate, filtered, and concentrated in *vacuo* to afford 6-aminonicotinic acid methyl ester (3.12 g, 71 %) as white foam: EI-HRMS m/e calcd for C₇H₈N₂O₂ (M⁺) 152.0586, found 152.0586.

A solution of triphenylphosphine (1.23 g, 4.69 mmol) in methylene chloride (15 mL) was cooled to 0°C and then treated with *N*-bromosuccinimide (947 mg, 5.32 mmol). The resulting brown-purple solution was stirred at 0°C for 5 min and then treated with 3-cyclopentyl-2(R)-(3,4-dichloro-phenyl)-propionic acid (prepared in Example **54**, 900 mg, 3.13 mmol). The reaction mixture was stirred at 0°C and then allowed to warm to 25°C over 45 min. The reaction mixture was then treated with 6-aminonicotinic acid methyl ester (620 mg, 4.07 mmol) and pyridine (0.38 mL, 4.7 mmol), and the reaction mixture was allowed to stir at 25°C for 20 h. The resulting reaction mixture was diluted with water (15 mL) and then extracted with methylene chloride (3 x 15 mL). The combined organic layers were dried over sodium sulfate, filtered, and concentrated *in vacuo*. Flash chromatography (Merck Silica gel 60, 230-400 mesh, 9/1 hexanes/ethyl acetate) afforded 6-[3-cyclopentyl-2(R)-(3,4-dichloro-phenyl)-propionylamino]-nicotinic acid methyl ester (1.10 g, 84%) as a white foam: [α]²³₅₈₉ = -68.0° (c=0.128, chloroform); FAB-HRMS m/e calcd for C₂₁H₂₂Cl₂N₂O₃ (M+H)⁺ 421.1086, found 421.1079.

### Example 46

### 6-[3-Cyclopentyl-2-(3,4-dichloro-phenyl)-propionylamino]-nicotinic acid

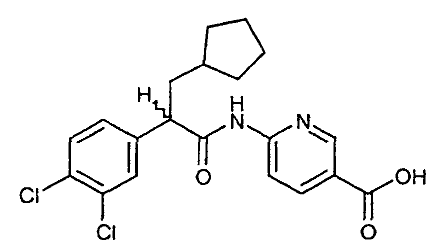

A solution of 3-cyclopentyl-2-(3,4-dichloro-phenyl)-N-(5-carboxymethylpyridin)-2-yl-propionamide (prepared in Example **38**(B)(b), 50 mg, 0.12 mmol) in ethanol (10 mL) at 25°C was treated with a solution of potassium hydroxide (20 mg, 0.36 mmol) in water (2 mL). The reaction was stirred at 25°C for 2 h. At this time, the reaction was diluted with water (5 mL). The ethanol was removed *in vacuo*. The aqueous layer was then acidified to pH = 2 with a 1N aqueous hydrochloric acid solution. This solution was extracted with methylene chloride (3 x 10 mL). The organic layers were dried over sodium sulfate, filtered and concentrated *in vacuo*. Flash chromatography (Merck Silica gel 60, 230-400 mesh, 80/20 hexanes/ethyl acetate with acetic acid) afforded 6-[3-cyclopentyl-2-(3,4-dichloro-phenyl)-propionylamino]-nicotinic acid (34 mg, 71%) as white foam: EI-HRMS m/e calcd for C₂₀H₂₀Cl₂N₂O₃ (M⁺) 406.0851, found 406.0852.

### Example 47

### 6-[2-(4-Chloro-phenyl)-3-cyclopentyl-propionylamino)-nicotinic acid

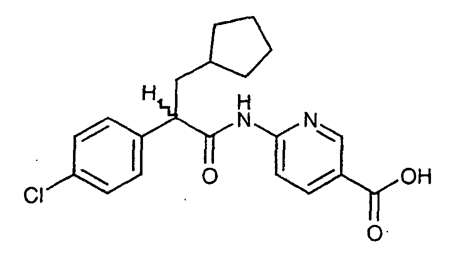

A solution of 6-[2-(4-chloro-phenyl)-3-cyclopentyl-propionylamino]-nicotinic acid methyl ester (prepared in Example **31**(B)(c), 62.6 mg, 0.16 mmol) in tetrahydrofuran/water/methanol (0.40 mL, 3:1:1) was treated with a 2N aqueous sodium hydroxide solution (0.16 mL, 0.32 mmol). The reaction was stirred at 25°C for 24 h. The reaction mixture was then poured into water and extracted with chloroform (2 x 30 mL). The aqueous layer was then acidified to pH = 1 with a 1N aqueous hydrochloric acid solution. The product was extracted into chloroform/methanol (9:1, 3 x 25 mL), dried over sodium sulfate, filtered, and concentrated *in vacuo*. Flash chromatography (Merck Silica gel 60, 230-400 mesh, 75/25 hexanes/ethyl acetate w/acetic acid) afforded 6-[2-(4-chloro-phenyl)-3-cyclopentyl-propionylamino]-nicotinic acid (17.0 mg, 31.5%) as a white solid: mp 206-208°C; EI-HRMS m/e calcd for C₂₀H₂₁ClN₂O₂ (M⁺) 372.1240, found 372.1244.

### Example 48

### 6-[3-Cyclopentyl-2-(4-methanesulfonyl-phenyl)-propionylamino]-nicotinic acid

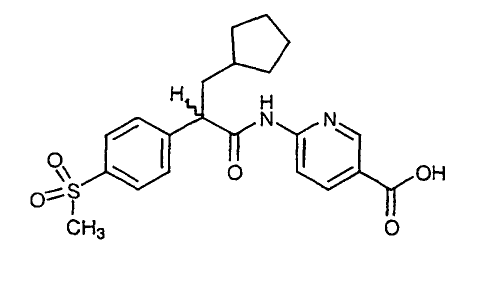

A solution of 6-[3-cyclopentyl-2-(4-methanesulfonyl-phenyl)-propionylamino]-nicotinic acid methyl ester (prepared in Example **53**(B)(a), 100 mg, 0.23 mmol) in tetrahydrofuran (500 µL) was treated with a 0.8M aqueous lithium hydroxide solution (300 µL, 0.23 mmol). The solution was stirred at 25°C for 4 h. The reaction mixture was then directly purified by column chromatography. Flash chromatography (Merck Silica gel 60, 230-400 mesh, 1/3 methanol/ethyl acetate) afforded 6-[3-cyclopentyl-2-(4-methanesulfonyl-phenyl)-propionylamino]-nicotinic acid (65 mg, 70%) as a white solid: mp 191-193°C; FAB-HRMS m/e calcd for C₂₁H₂₄N₂O₅S (M+H)⁺ 417.1484, found 417.1484.

### Example 49

### 3-Cyclopentyl-2(3,4-dichloro-phenyl)-N-(5-hydroxymethyl-pyridin-2-yl)-propionamide

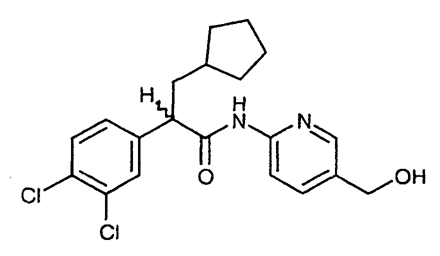

A solution of 3-cyclopentyl-2-(3,4-dichloro-phenyl)-N-(5-carboxymethylpyridin)-2-yl-propionamide (prepared in Example **38**(B)(b), 398 mg, 0.95 mmol) in diethyl ether (30 mL) cooled to 0°C was treated with lithium aluminum hydride (54 mg, 1.4 mmol). This slurry was allowed to slowly warm to 25°C. The reaction was stirred at 25°C for 16 h. At this time, the reaction was quenched with water (10 mL) and extracted with ethyl acetate (3 x 15 mL). The organics were dried over sodium sulfate, filtered, and concentrated *in vacuo*. Flash chromatography (Merck Silica gel 60, 230-400 mesh, 50/50 hexanes/ethyl acetate) afforded 3-cyclopentyl-2-(3,4-dichloro-phenyl)-N-(5-hydroxymethyl-pyridin-2-yl)-propionamide (131 mg, 35%) as a white foam: EI-HRMS m/e calcd for C₂₀H₂₂Cl₂N₂O₂ (M⁺) 392.1058, found 392.1062.

### Example 50

### 2-(4-Chloro-phenyl)-3-cyclopentyl-N-(5-hydroxymethyl-pyridin-2-yl)-propionamide

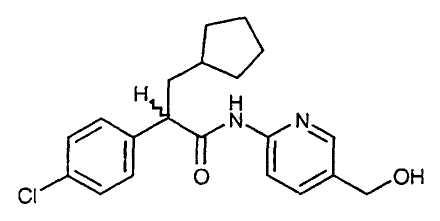

A solution of 6-[2-(4-chloro-phenyl)-3-cyclopentyl-propionylamino]-nicotinic acid methyl ester (prepared in Example **31**(B)(c), 83.3 mg, 0.21 mmol) in tetrahydrofuran (2.1 mL) was added to a cooled (0°C) slurry of lithium aluminum hydride (12.0 mg, 0.32 mmol) in tetrahydrofuran (1.54 mL). The reaction mixture was stirred at 0°C for 2.5 h. The reaction was then quenched by the dropwise addition of water (25 mL). The reaction was further diluted with water and was then extracted with ethyl acetate (3 x 35 mL). The organics were dried over sodium sulfate, filtered, and concentrated *in vacuo*. Flash chromatography (Merck Silica gel 60, 230-400 mesh, 70/30 hexanes/ethyl acetate) afforded 2-(4-chloro-phenyl)-3-cyclopentyl-N-(5-hydroxymethyl-pyridin-2-yl)-propionamide (12.5 mg, 16.1%) as a white solid: mp 60-62°C; EI-HRMS m/e calcd for C₂₀H₂₃ClN₂O₂ (M⁺) 358.1448, found 358.1443.

### Example 51

### 3-Cyclopentyl-2-(3,4-dichloro-phenyl)-N-(5-hydroxy-pyridin-2-yl)-propionamide

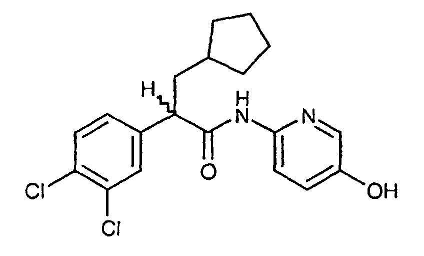

A solution of 3-cyclopentyl-2-(3,4-dichloro-phenyl)-propionic acid (prepared in Example **38**, 183 mg, 0.63 mmol) in methylene chloride (6.37 mL) was cooled to 0°C and then treated with a 2.0M solution of oxalyl chloride in methylene chloride (0.35 mL, 0.7 mmol) and a few drops of *N*,*N*-dimethylformamide. The reaction mixture was stirred at 0°C for 10 min and then at 25°C for 30 min. The reaction mixture was then treated with 5-benzyloxy-pyridin-2-ylamine (281 mg, 1.4 mmol) and *N*,*N*-diisopropylethylamine (0.26 mL, 1.5 mmol). The reaction mixture was stirred at 25°C for 16 h. At this time, the reaction was concentrated *in vacuo*. Flash chromatography (Merck Silica gel 60, 230-400 mesh, 70/30 hexanes/ethyl acetate) afforded N-(5-benzyloxy-pyridin-2-yl)-3-cyclopentyl-2-(3,4-dichloro-phenyl)-propionamide (150 mg, 50.0%) as a yellow solid: mp 47-49°C; EI-HRMS m/e calcd for C₂₆H₂₆Cl₂N₂O₂ (M⁺) 469.1449, found 469.1455.

A solution of N-(5-benzyloxy-pyridin-2-yl)-3-cyclopentyl-2-(3,4-dichloro-phenyl)-propionamide (145.3 mg, 0.3 mmol) in methanol (5.1 mL) was treated with 10% palladium on activated carbon. The reaction mixture was stirred under hydrogen gas at 25°C for 16 h. The catalyst was then filtered off through a pad of celite (ethyl acetate). The filtrate was concentrated *in vacuo* to give 3-cyclopentyl-2-(3,4-dichloro-phenyl)-N-(5-hydroxy-pyridin-2-yl)-propionamide (92.2 mg, 78.5%) as a tan solid: mp 79-81°C; EI-HRMS m/e calcd for C₁₉H₂₀Cl₂N₂O₂ (M⁺) 378.0896, found 378.0890.

### Example 52

### 3-Cyclopentyl-N-(5-hydroxymethyl-pyridin-2-yl)-2-(4-methanesulfonyl-phenyl)-propionamide

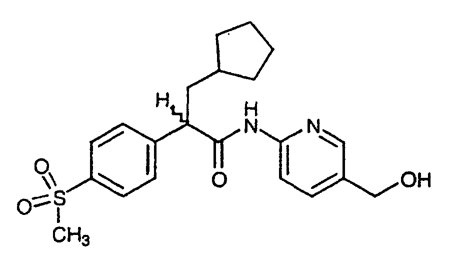

A solution of 6-[3-cyclopentyl-2-(4-methanesulfonyl-phenyl)-propionylamino)-nicotinic acid methyl ester (prepared in example **53**(B)(a), 110 mg, 0.26 mmol) in diethyl ether (500 µL) was cooled to 0°C and then slowly treated with lithium aluminum hydride (15 mg, 0.38 mmol). The reaction mixture was stirred at 0°C for 30 min then allowed to warm to 25°C. After 1 h at 25°C, thin layer chromatography still indicated the presence of the starting material. An additional amount of lithium aluminum hydride (10 mg, 0.26 mmol) was added to the reaction mixture, and the reaction mixture was allowed to stir at 25°C for 1 h. The reaction mixture was then slowly quenched by the dropwise addition of water (10 mL). The resulting mixture was partitioned between water and ethyl acetate. The organic layer was dried over magnesium sulfate, filtered, and concentrated *in vacuo*. Flash chromatography (Merck Silica gel 60, 230-400 mesh, 1/3 hexanes/ethyl acetate) afforded the 3-cyclopentyl-N-(5-hydroxymethyl-pyridin-2-yl)-2-(4-methanesulfonyl-phenyl)-propionamide (60 mg, 57%) as a yellow foam: mp 74-77°C; EI-HRMS m/e calcd for C₂₁H₂₆N₂O₄S (M⁺) 402.1613, found 402.1617.

### Example 53

### (A) 3-Cyclopentyl-2-(4-methanesulfonyl-phenyl)-N-(5-methyl-pyridin-2-yl)-propionamide

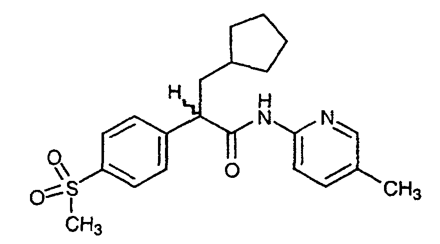

A solution triphenylphosphine (177 mg, 0.68 mmol) in methylene chloride (3 mL) was cooled to 0°C and then treated with *N*-bromosuccinimide (132 mg, 0.74 mmol) in small portions. The reaction mixture was allowed to warm to 25°C over 30 min and then was treated with 3-cyclopentyl-2-(4-methanesulfonyl-phenyl)propionic acid (prepared in Example **3**(A), 200 mg, 0.68 mmol). The reaction mixture was stirred at 25°C for 30 min and then treated with 2-amino-5-methylpyridine (154 mg, 1.42 mmol). The resulting reaction mixture was stirred at 25°C for 1 h. The crude reaction mixture was directly purified by flash chromatography (Merck Silica gel 60, 230-400 mesh, 1/1 hexanes/ethyl acetate) to afford impure 3-cyclopentyl-2-(4-methanesulfonyl-phenyl)-N-(5-methyl-pyridin-2-yl)-propionamide as a red solid. The impure product was further purified by precipitation from 1/1 hexanes/ethyl acetate to afford pure 3-cyclopentyl-2-(4-methanesulfonyl-phenyl)-N-(5-methyl-pyridin-2-yl)-propionamide (80 mg, 31%) as an off-white solid: mp 184-185°C; EI-HRMS m/e calcd for C₂₁H₂₆N₂O₃S (M⁺) 386.1664, found 386.1664.

### (B) In an analogous manner, there was obtained:

(a) From 3-cyclopentyl-2-(4-methanesulfonyl-phenyl)propionic acid and 6-aminonicotinic acid methyl ester: 6-[3-Cyclopentyl-2-(4-methanesulfonyl-phenyl)-propionylamino]-nicotinic acid methyl ester as a yellow foam: mp 82-85°C; EI-HRMS m/e calcd for C₂₂H₂₆N₂O₅S (M⁺) 430.1562, found 430.1571.

### Example 54

### (A) N-(5-Chloro-pyridin-2-yl)-3-cyclopentyl-2(R)-(3,4-dichloro-phenyl)-propionamide

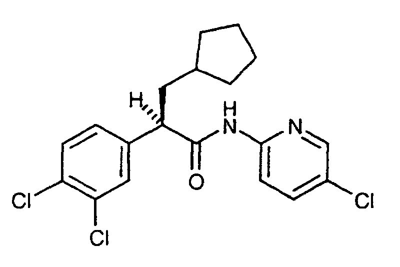

A solution of 3-cyclopentyl-2-(3,4-dichloro-phenyl)-propionic acid (prepared in Example 38, 5.00 g, 17.4 mmol) in tetrahydrofuran (150 mL) cooled to -78°C was treated with triethylamine (2.77 mL, 19.9 mmol) followed by trimethylacetyl chloride (2.24 mL, 18.2 mmol). The resulting white slurry was stirred at -78°C for 15 min and then at 0°C for 45 min. In a separate flask, a solution of (S)-4-isopropyl-2-oxazolidinone (2.14 g, 16.57 mmol) in tetrahydrofuran (80 mL) cooled to -78°C was treated with a 2.0M solution of *n*-butyllithium in hexanes (8.7 mL, 17.4 mmol). The solution was stirred at -78°C for 10 min and then allowed to warm to 25°C where it was stirred for an additional 10 min. At this time, the first reaction mixture was recooled to -78°C. The second reaction mixture was added to the first reaction mixture over a period of 5 min via cannula. The combined reaction was then stirred at -78°C for 15 min and then allowed to warm to 25°C where it was stirred for an additional 1.5 h. At this time, the reaction was quenched by the addition of a saturated aqueous sodium bisulfite solution (50 mL) and extracted with ethyl acetate (3 x 40 mL). The combined organic layers were washed with a saturated aqueous sodium bicarbonate solution (1 x 20 mL) and a saturated aqueous sodium chloride solution (1 x 20 mL), dried over sodium sulfate, filtered, and concentrated *in vacuo.* Flash chromatography (Merck Silica gel 60, 230-400 mesh, 85/15 hexanes/ethyl acetate) afforded (1) 3-[3-cyclopentyl-2(S)-(3,4-dichloro-phenyl)-propionyl)-4(S)-isopropyl-oxazolidin-2-one (2.15 g, 33%) as a clear oil: [α]²³₅₈₉ = +87.5° (c=0.160, chloroform); EI-HRMS m/e calcd for C₂₀H₂₅Cl₂NO₃ (M⁺) 397.1211, found 397.1215 and (2) 3-[3-cyclopentyl-2(R)-(3,4-dichloro-phenyl)-propionyl]-4(S)-isopropyl-oxazolidin-2-one (1.88 g, 28%) as a white solid: mp 71.9-74.6°C, [α]²³₅₈₉ = -27.6° (c=0.188, chloroform); EI-HRMS m/e calcd for C₂₀H₂₅Cl₂NO₃ (M⁺) 397.1211, found 397.1212.

A solution of 3-[3-cyclopentyl-2(R)-(3,4-dichloro-phenyl)-propionyl]-4(S)-isopropyl-oxazolidin-2-one (1.88 g, 4.72 mmol) in tetrahydrofuran (73 mL) and water (22 mL) cooled to 0°C was treated with a 30% aqueous hydrogen peroxide solution (2.1 mL) and lithium hydroxide (394 mg, 9.4 mmol). The reaction was stirred at 0°C for 1 h. At this time, the reaction was quenched with a saturated aqueous sodium sulfite solution (16 mL) followed by the addition of a 0.5N aqueous sodium bicarbonate solution (50 mL). The tetrahydrofuran was then removed *in vacuo*. The residue was diluted with water (40 mL) and extracted with methylene chloride (3 x 20 mL). The aqueous layer was then acidified to pH = 2 with 5N aqueous hydrochloric acid solution and extracted with ethyl acetate (4 x 25 mL). The combined organic layers were then dried over sodium sulfate, filtered, and concentrated *in vacuo* to afforded of 3-cyclopentyl-2(R)-(3,4-dichlorophenyl)-propionic acid (928 mg, 70%) as a white solid: mp 75.1-78.3°C; [α]²³₅₈₉ = -50.3° (c=0.100, chloroform); EI-HRMS m/e calcd for C₁₄H₁₆Cl₂O₂ (M⁺) 286.0527, found 286.0535.

A solution of triphenylphosphine (344 mg, 1.31 mmol) in methylene chloride (10 mL) cooled to 0°C was treated with *N*-bromosuccinimide (263 mg, 1.48 mmol). The reaction solution was stirred at 0°C for 5 min. At this time, 3-cyclopentyl-2-(R)-(3,4-dichlorophenyl) propionic acid (250 mg, 0.87 mmol) was added. The reaction was allowed to slowly warm to 25°C over 45 min. At this time, 5-chloro-2-aminopyridine (145 mg, 1.13 mmol) and pyridine (0.11 mL, 1.31 mmol) were added to the reaction mixture. The reaction was stirred at 25°C for 20 h. At this time, the reaction was diluted with water (10 mL) and extracted with methylene chloride (3 x 10 mL). The organics were dried over sodium sulfate, filtered, and concentrated *in vacuo*. Flash chromatography (Merck Silica gel 60, 230-400 mesh, 90/10 hexanes/ethyl acetate) afforded N-(5-chloro-pyridin-2-yl)3-cyclopentyl-2-(R)-(3,4-dichloro-phenyl)-propionamide (289 mg, 84%) as a white solid: mp 125-128°C; [α]²³₅₈₉ = -65.6° (c=0.16, chloroform); EI-HRMS m/e calcd for C₁₉H₁₉Cl₃N₂O (M⁺) 396.0563, found 396.0565.

### (B) In an analogous manner, there were obtained:

(a) From 2-amino pyridine and 3-cyclopentyl-2-(R)-(3,4-dichloro-phenyl) propionic acid : 3-Cyclopentyl-2(R)-(3,4-dichloro-phenyl)-N-pyridin-2-yl-propionamide as a white foam: [α]²³₅₈₉ = -56.2° (c=0.153, chloroform); EI-HRMS m/e calcd for C₁₉H₂₀Cl₂N₂O (M⁺) 362.0953, found 362.0952.
(b) From 2-aminothiazole and 3-cyclopentyl-2-(R)-(3,4-dichloro-phenyl) propionic acid: 3-Cyclopentyl-2(R)-(3,4-dichloro-phenyl)-N-thiazol-2-yl-propionamide as a white solid: mp 133.4-136.5°C; [α]²³₅₈₉ = -66.0° (c=0.106, chloroform); EI-HRMS m/e calcd for C₁₇H₁₈Cl₂N₂OS (M⁺) 368.0517, found 368.0519.
(c) From 2-(amino-thiazol-5-yl)-oxo-acetic acid ethyl ester and 3-cyclopentyl-2-(R)-3-cyclopentyl-2-(3,4-dichlorophenyl)-propionic acid: (2R)-{2-[3-Cyclopentyl-2-(3,4-dichlorophenyl)-propionylamino]-thiazol-5-yl}-oxo-acetic acid ethyl ester as a light yellow foam: mp 117-120° C; FAB-HRMS m/e calcd for C₂₁H₂₂Cl₂N₂O₄S (M+H)⁺ 469.0755, found 469.0753.
(d) From ethyl 2-amino-4-thiazole glyoxylate and 3-cyclopentyl-2-(R)-3-cyclopentyl-2-(3,4-dichlorophenyl)-propionic acid: (2R)-{2-[3-Cyclopentyl-2-(3,4-dichloro-phenyl)-propionylamino]-thiazol-4-yl}-oxo-acetic acid ethyl ester as a white solid: EI-HRMS m/e calcd for C₂₁H₂₂Cl₂N₂O₄S (M⁺) 468.0677, found 468.0677.

### Example 55

### 3-Cyclopentyl-2-(3,4-dichlorophenyl)-N-(1H-imidazol-2-yl)-propionamide

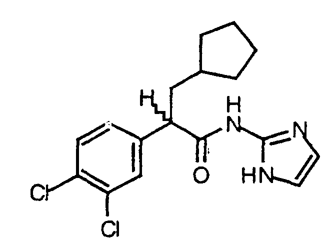

A solution of 3-cyclopentyl-2-(3,4-dichlorophenyl)-propionic acid (prepared in Example **38**, 200 mg, 0.70 mmol), benzotriazol-1-yloxy-tris(dimethylamino)phosphonium hexafluorophosphate (310 mg, 0.70 mmol), *N*,*N*-diisopropylethylamine (244 µL, 1.40 mmol), and 2-aminoimidazole sulfate (140 mg, 1.05 mmol) in dry *N*,*N*-dimethylformamide (5 mL) was stirred at 25°C under nitrogen for 15 h. The reaction mixture was partitioned between water and ethyl acetate. The organic layer was washed with a 1N aqueous hydrochloric acid solution, washed with water, and washed with a saturated aqueous sodium chloride solution. The organic layer was dried over magnesium sulfate, filtered, and concentrated *in vacuo*. Flash chromatography (Merck Silica gel 60, 230-400 mesh, 1/3 hexanes/ethyl acetate) afforded 3-cyclopentyl-2-(3,4-dichlorophenyl)-N-(1H-imidazol-2-yl)-propionamide (81.4 mg, 33%) as a white solid: mp 58-60°C; EI-HRMS m/e calcd for C₁₇H₁₉Cl₂N₃O (M⁺) 351.0905, found 351.0901.

### Example 56

### 3-Cyclopentyl-2-(3,4-dichlorophenyl)-N-(5-methyl-isoxazol-3-yl)-propionamide

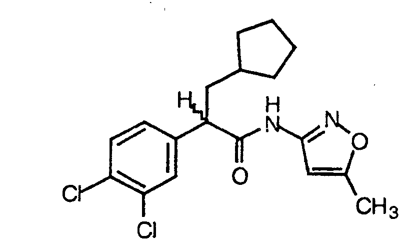

A solution of 3-cyclopentyl-2-(3,4-dichlorophenyl)-propionic acid (prepared in Example **38**, 70.7 mg, 0.25 mmol) in oxalyl chloride (215 µL, 2.46 mmol) was cooled to 0°C and then treated with 1 drop of dry *N*,*N*-dimethylformamide. The reaction mixture was stirred at 0°C for 30 min and then stirred at 25°C for 3 h. The reaction mixture was concentrated *in vacuo* to afford a yellow oil. This yellow oil was dissolved in a small amount of methylene chloride and then slowly added to a solution of 3-amino-5-methylisoxazole (48.3 mg, 0.49 mmol) and triethylamine (68 mL, 0.49 mmol) in methylene chloride (1.2 mL). The resulting reaction mixture was stirred at 25°C for 14 h. The reaction mixture was concentrated *in vacuo* to remove methylene chloride. The resulting residue was diluted with ethyl acetate (100 mL) and then washed with a 10% aqueous hydrochloric acid solution. The organic layer was dried over sodium sulfate, filtered, and concentrated *in vacuo*. Flash chromatography (Merck Silica gel 60, 230-400 mesh, 3/1 hexanes/ethyl acetate) afforded 3-cyclopentyl-2-(3,4-dichlorophenyl)-N-(5-methyl-isoxazol-3-yl)-propionamide (78.3 mg, 87%) as a yellow glass: mp 84-86°C; FAB-HRMS m/e calcd for C₁₈H₂₀Cl₂N₂O₂ (M+H)⁺ 367.0981, found 367.0982.

### Example 57

### 3-Cyclopentyl-2-(3,4-dichlorophenyl)-N-oxazol-2-yl-propionamide

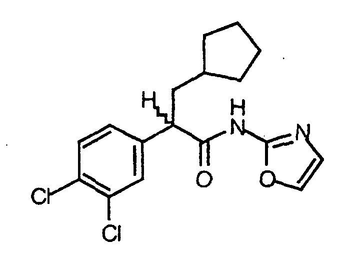

A solution of benzotriazol-1-yloxy-tris(dimethylamino)phosphonium hexafluorophosphate (102 mg, 0.23 mmol), 3-cyclopentyl-2-(3,4-dichlorophenyl)-propionic acid (prepared in Example **38,** 60 mg, 0.21 mmol), *N*,*N*-diisopropylethylamine (73 µL, 0.42 mmol), and 2-aminooxazole (27 mg, 0.31 mmol) in dry *N*,*N*-dimethylformamide (1 mL) was stirred at 25°C under nitrogen for 15 h. The reaction mixture was partitioned between water and ethyl acetate. The organic layer was washed with a 1N aqueous hydrochloric acid solution, washed with water, and washed with a saturated aqueous sodium chloride solution. The organic layer was dried over magnesium sulfate, filtered, and concentrated *in vacuo*. Flash chromatography (Marck Silica gel 60, 230-400 mesh, 1/1 hexanes/ethyl acetate) afforded 3-cyclopentyl-2-(3,4-dichlorophenyl)-N-oxazol-2-yl-propionamide (34.9 mg, 47%) as a white solid: mp 134-136°C; EI-HRMS m/e calcd for C₁₇H₁₈Cl₂N₂O₂ (M⁺) 352.0745, found 352.0750.

### Example 58

### 3-Cyclopentyl-2-(3,4-dichlorophenyl)-N-pyridazin-3-yl-propionamide

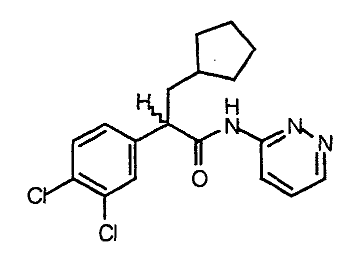

A solution of 3-cyclopentyl-2-(3,4-dichlorophenyl)-propionic acid (prepared in Example **38**, 625.2 mg, 2.18 mmol), *O*-benzotriazol-1-yl-*N,N,N',N*'-tetramethyluronium hexafluorophosphate (908.3 mg, 2.39 mmol), *N,N*-diisopropylethylamine (1.1 mL, 6.53 mmol), and 3-aminopyridazine (310.6 mg, 3.27 mmol) in dry *N,N*-dimethylformamide (11 mL) was stirred at 25°C under nitrogen for 72 h. The reaction mixture was concentrated *in vacuo* to remove *N,N*-dimethylformamide. The resulting residue was diluted with ethyl acetate (200 mL). The organic layer was washed with a 10% aqueous hydrochloric acid solution and washed with a saturated aqueous sodium chloride solution. The organic layer was dried over sodium sulfate, filtered, and concentrated *in vacuo*. Flash chromatography (Merck Silica gel 60, 230-400 mesh, 1/1 hexanes/ethyl acetate) afforded 3-cyclopentyl-2-(3,4-dichlorophenyl)-N-pyridazin-3-yl-propionamide (493.8 mg, 62%) as a white foam: mp 70-71°C; EI-HRMS m/e calcd for C₁₈H₁₉Cl₂N₃O (M⁺) 363.0905, found 363.0908.

### Example 59

### 3-Cyclopentyl-2-(3,4-dichlorophenyl)-N-pyrimidin-2-yl-propionamide

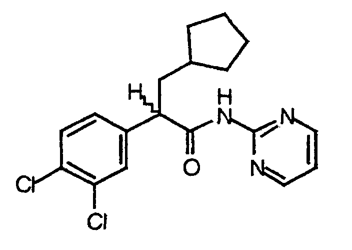

A solution of 3-cyclopentyl-2-(3,4-dichlorophenyl)-propionic acid (prepared in Example **38,** 100 mg, 0.35 mmol) in methylene chloride (1 mL) was treated with 2 drops of dry *N*,*N*-dimethylformamide. The reaction mixture was cooled to 0°C and then treated dropwise with oxalyl chloride (34 mL, 0.39 mmol). The reaction mixture was stirred at 0°C for 10 min and then stirred at 25°C for 2 h. The reaction mixture was concentrated *in vacuo*. The resulting residue was dissolved in a small amount of methylene chloride and was slowly added to a cooled (0°C) solution of 2-aminopyrimidine (67 mg, 0.70 mmol) in methylene chloride (1 mL). The resulting reaction mixture was stirred at 0°C for 30 min and then stirred at 25°C for 2 h. The reaction mixture was concentrated *in vacuo* to remove methylene chloride. The resulting residue was diluted with water and extracted with ethyl acetate (3 x 50 mL). The combined organic extracts were washed with a saturated aqueous sodium chloride solution. The organic layer was dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(3,4-dichlorophenyl)-N-pyrimidin-2-yl-propionamide (85.4 mg, 67%) as a white solid: mp 103-105°C; EI-HRMS m/e calcd for C₁₈H₁₉Cl₂N₃O (M⁺) 363.0905, found 363.0915.

### Example 60

### 3-Cyclopentyl-2(R)-(3,4-dichloro-phenyl)-N-pyrimidine-6-yl-propionamide

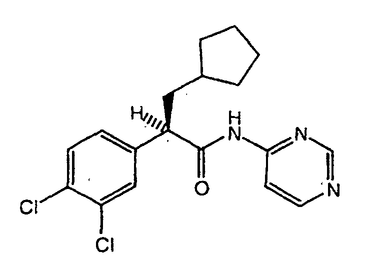

A solution of 3-cyclopentyl-2(R)-(3,4-dichlorophenyl)-propionic acid (prepared in Example **54**(A), 200 mg, 0.69 mmol) in methylene chloride (5 mL) was treated with 1 drop of *N,N*-dimethylformamide and then cooled to 0°C. The reaction mixture was then treated with a 2.0M solution of oxalyl chloride in methylene chloride (0.52 mL, 1.04 mmol). The reaction mixture was stirred at 0°C for 30 min and then treated with a solution of 4-aminopyrimidine (131 mg, 1.38 mmol) in tetrahydrofuran (10 mL) and pyridine (0.28 mL, 3.45 mmol). The resulting reaction mixture was stirred at 23°C for 14 h. The reaction mixture was then diluted with water (10 mL) and extracted with methylene chloride (3 x 15 mL). The combined organic layers were dried over sodium sulfate, filtered, and concentrated *in vacuo*. Flash chromatography (Merck Silica gel 60, 230-400 mesh, 3/2 hexanes/ethyl acetate) afforded 3-cyclopentyl-2(R)-(3,4-dichlorophenyl)-N-pyrimidin-4-yl-propionamide (147 mg, 60%) as a white solid: mp 166.5-169.3°C; EI-HRMS m/e calcd for C₁₈H₁₉Cl₂N₃O (M⁺) 363.0905, found 363.0909.

### Example 61

### 3-Cyclopentyl-2-(4-methanesulfinyl-phenyl)-N-thiazol-2-yl-propionamide

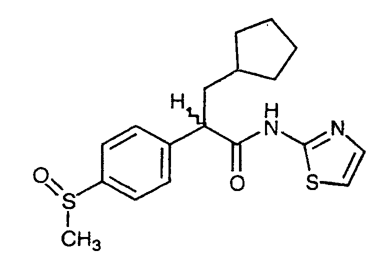

A solution of diisopropylamine (3.2 mL, 23.16 mmol) in dry tetrahydrofuran (10.3 mL) and 1,3-dimethyl-3,4,5,6-tetrahydro-2(1H)-pyrimidinone (3.4 mL) was cooled to -78°C under nitrogen and then treated with a 10M solution of *n*-butyllithium in hexanes (2.3 mL, 23.16 mmol). The resulting reaction mixture was stirred at -78°C for 30 min and then treated dropwise with a solution of 4-(methylthio)phenylacetic acid (2.01 g, 11.03 mmol) in dry tetrahydrofuran (10.3 mL) and 1,3-dimethyl-3,4,5,6-tetrahydro-2(1H)-pyrimidinone (3.4 mL). The reaction mixture was allowed to stir at -78°C for 1 h, at which time, a solution of iodomethylcyclopentane (2.55 g, 12.13 mmol) in a small amount of dry tetrahydrofuran was added dropwise. The reaction mixture was stirred at - 78°C for 30 min and then allowed to warm to 25°C where it was stirred for 24 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 200 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-methylsulfanyl-phenyl)propionic acid (1.01 g, 35%) as a cream solid: mp 91-93°C; EI-HRMS m/e calcd for C₁₅H₂₀O₂S (M⁺) 264.1184, found 264.1177.

A solution of 3-cyclopentyl-2-(4-methylsulfanyl-phenyl)propionic acid (200 mg, 0.76 mmol) and triphenylphosphine (198 mg, 0.76 mmol) in methylene chloride (2 mL) was cooled to 0°C and then slowly treated with N-bromosuccinimide (150 mg, 0.84 mmol). After the complete addition of *N*-bromosuccinimide, the reaction mixture was allowed to warm to 25°C over 30 min. The reaction mixture was then treated with 2-aminothiazole (160 mg, 1.60 mmol), and the resulting reaction mixture was stirred at 25°C for 15 h. The reaction mixture was then concentrated *in vacuo* to remove methylene chloride. The remaining residue was diluted with water and ethyl acetate. The organic layer was washed with a 1N aqueous hydrochloric acid solution, washed with a saturated aqueous sodium bicarbonate solution, dried over magnesium sulfate, filtered, and concentrated *in vacuo*. Flash chromatography (Merck Silica gel 60, 230-400 mesh, 2/1 hexanes/ethyl acetate) afforded crude 3-cyclopentyl-2-(4-methylsulfanyl-phenyl)-N-thiazol-2-yl-propionamide as a yellow solid. Recrystallization from 3/1 hexanes/ethyl acetate afforded pure 3-cyclopentyl-2-(4-methylsulfanyl-phenyl)-N-thiazol-2-yl-propionamide (114 mg, 44%) as a white solid: mp 195-196°C; EI-HRMS m/e calcd for C₁₈H₂₂N₂OS₂ (M⁺) 346.1174, found 346.1171.

A solution 3-cyclopentyl-2-(4-methylsulfanyl-phenyl)-N-thiazol-2-yl-propionamide (75 mg, 0.216 mmol) in methylene chloride (1 mL) was treated with 3-chloroperoxybenzoic acid (75% grade, 50 mg, 0.216 mmol). The reaction mixture was immediately monitored by thin layer chromatography and the results indicated the immediate absence of starting material. The reaction mixture was partitioned between water and methylene chloride and then washed with a saturated aqueous sodium bicarbonate solution. The organic layer was further washed with water and then dried over magnesium sulfate, filtered, and concentrated *in vacuo*. Recrystallization from 1/1 hexanes/ethyl acetate afforded 3-cyclopentyl-2-(4-methanesulfinyl-phenyl)-N-thiazol-2-yl-propionamide (25 mg, 32%) as a white solid: mp 170-173°C; EI-HRMS m/e calcd for C₁₈H₂₂N₂O₂S₂ (M⁺) 362.1123, found 362.1121.

### Example 62

### {2-[3-Cyclopentyl-2-(4-trifluoromethanesulfonyl-phenyl)-propionylamino]-thiazol-4-yl}-acetic acid ethyl ester

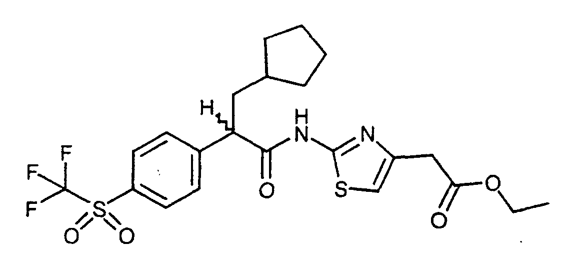

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanylphenyl)propionic acid methyl ester (1.37 g, 99%) as a light yellow oil: EI-HRMS m/e calcd for C₁₆H₁₉F₃O₂S (M⁺) 332.1058, found 332.1052.

A solution of 3-cyclopentyl-2-(4-trifluoromethylsulfanyl-phenyl)propionic acid methyl ester (1.14 g, 3.43 mmol) in methylene chloride (8.6 mL) was treated with 3-chloroperoxybenzoic acid (80-85% grade, 2.00 g based on 80%, 9.26 mmol). The reaction mixture was stirred at 25°C for 17 h, at which time, thin layer chromatography showed the presence of two new lower R_{f} products. An additional 2.00 g of 3-chloroperoxybenzoic acid was added to the reaction mixture to drive the conversion of the sulfoxide to the sulfone, and the resulting reaction mixture was stirred at 25°C for 3 d. The reaction mixture was concentrated *in vacuo* to remove methylene chloride. The resulting residue was diluted with ethyl acetate (300 mL). The organic phase was washed with a saturated aqueous sodium bicarbonate solution (3 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 19/1 hexanes/ethyl acetate) afforded 3-cyclopentyl-2-(4-trifluoromethanesulfonylphenyl)propionic acid methyl ester (1.19 g, 95%) as a light yellow oil: EI-HRMS m/e calcd for C₁₆H₁₉F₃O₄S (M⁺) 364.0956, found 364.0965.

A solution of 3-cyclopentyl-2-(4-trifluoromethanesulfonyl-phenyl)propionic acid methyl ester (708.2 mg, 1.94 mmol) in tetrahydrofuran (2.4 mL) was treated with a 0.8M aqueous lithium hydroxide solution (3.6 mL, 2.92 mmol). The reaction mixture was stirred at 25°C for 23 h and then concentrated *in vacuo* to remove tetrahydrofuran. The remaining aqueous layer was acidified to pH = 2 with a 10% aqueous hydrochloric acid solution and then extracted with ethyl acetate (2 x 100 mL). The combined organic layers were washed with a saturated aqueous sodium chloride solution (1 x 100 mL), dried over sodium sulfate, filtered, and concentrated *in vacuo* to afford a cream solid. This solid was purified by triturating with diethyl ether/petroleum ether to provide pure 3-cyclopentyl-2-(4-trifluoromethanesulfonyl-phenyl)propionic acid (527.0 mg, 77%) as a white solid: mp 143-145°C; EI-HRMS m/e calcd for C₁₅H₁₇F₃O₄S (M⁺) 350.0800, found 350.0816.

A solution of triphenylphosphine (97 mg, 0.371 mmol) in methylene chloride (1.5 mL) was cooled to 0°C and then slowly treated with *N*-bromosuccinimide (66 mg, 0.371 mmol). The reaction mixture was stirred at 0°C for 20 min and then treated with 3-cyclopentyl-2-(4-trifluoromethanesulfonyl-phenyl)propionic acid (100 mg, 0.285 mmol). The resulting reaction mixture was stirred at 0°C for 10 min, allowed to warm to 25°C, and then treated with ethyl 2-amino-4-thiazoleacetate (123 mg, 0.657 mmol). The resulting reaction mixture was stirred at 25°C for 3 d. The reaction mixture was then concentrated *in vacuo*. Flash chromatography (Merck Silica gel 60, 230-400 mesh, 3/1 hexanes/ethyl acetate) afforded {2-[3-cyclopentyl-2-(4-trifluoromethanesulfonyl-phenyl)-propionyl-amino]-thiazol-4-yl}-acetic acid ethyl ester (107 mg, 72%) as a yellow foam: mp 48-51°C; EI-HRMS m/e calcd for C₂₂H₂₅F₃N₂O₅S₂ (M⁺) 518.1157, found 518.1157.

### Example 63

### N-(5-Bromo-pyridin-2-yl)-3-cyclopentyl-2-(4-trifluoromethanesulfonyl-phenyl)-propionamide

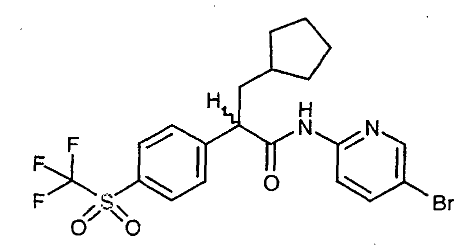

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of n-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL) The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanylphenyl)propionic acid (1.47 g, 58%) as a cream solid: mp 69-71 °C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanylphenyl)propionic acid methyl ester (1.37 g, 99%) as a light yellow oil: EI-HRMS m/e calcd for C₁₆H₁₉F₃O₂S (M⁺) 332.1058, found 332.1052.

A solution of 3-cyclopentyl-2-(4-trifluoromethylsulfanyl-phenyl)propionic acid methyl ester (1.14 g, 3.43 mmol) in methylene chloride (8.6 mL) was treated with 3-chloroperoxybenzoic acid (80-85% grade, 2.00 g based on 80%, 9.26 mmol). The reaction mixture was stirred at 25°C for 17 h, at which time, thin layer chromatography showed the presence of two new lower R_{f} products. An additional 2.00 g of 3-chloroperoxybenzoic acid was added to the reaction mixture to drive the conversion of the sulfoxide to the sulfone, and the resulting reaction mixture was stirred at 25°C for 3 d. The reaction mixture was concentrated *in vacuo* to remove methylene chloride. The resulting residue was diluted with ethyl acetate (300 mL). The organic phase was washed with a saturated aqueous sodium bicarbonate solution (3 x 100 mL), washed with a saturated aqueous sodium chloride solution (1 x 100 mL), dried over sodium sulfate, filtered, and concentrated *in vacuo.* Flash chromatography (Merck Silica gel 60, 70-230 mesh, 19/1 hexanes/ethyl acetate) afforded 3-cyclopentyl-2-(4-trifluoromethanesulfonylphenyl)propionic acid methyl ester (1.19 g, 95%) as a light yellow oil: EI-HRMS m/e calcd for C₁₆H₁₉F₃O₄S (M⁺) 364.0956, found 364.0965.

A solution of 3-cyclopentyl-2-(4-trifluoromethanesulfonyl-phenyl)propionic acid methyl ester (708.2 mg, 1.94 mmol) in tetrahydrofuran (2.4 mL) was treated with a 0.8M aqueous lithium hydroxide solution (3.6 mL, 2.92 mmol). The reaction mixture was stirred at 25°C for 23 h and then concentrated *in vacuo* to remove tetrahydrofuran. The remaining aqueous layer was acidified to pH = 2 with a 10% aqueous hydrochloric acid solution and then extracted with ethyl acetate (2 x 100 mL). The combined organic layers were washed with a saturated aqueous sodium chloride solution (1 x 100 mL), dried over sodium sulfate, filtered, and concentrated *in vacuo* to afford a cream solid. This solid was purified by triturating with diethyl ether/petroleum ether to provide pure 3-cyclopentyl-2-(4-trifluoromethanesulfonyl-phenyl)propionic acid (527.0 mg, 77%) as a white solid: mp 143-145°C; EI-HRMS m/e calcd for C₁₅H₁₇F₃O₄S (M⁺) 350.0800, found 350.0816.

A solution of triphenylphosphine (206 mg, 0.785 mmol) in methylene chloride (4 mL) was cooled to 0°C and then slowly treated with N-bromosuccinimide (140 mg, 0.785 mmol). The reaction mixture was stirred at 0°C for 10 min and then treated with 3-cyclopentyl-2-(4-trifluoromethanesulfonyl-phenyl)propionic acid (250 mg, 0.710 mmol). The resulting reaction mixture was stirred at 0°C for 10 min and then allowed to warm to 25°C where it was stirred for 30 min. The reaction mixture was then treated with 2-amino-5-bromopyridine (271 mg, 1.57 mmol). The resulting reaction mixture was stirred at 25°C for 15 h. The reaction mixture was then concentrated *in vacuo*. Flash chromatography (Merck Silica gel 60, 230-400 mesh, 2/1 hexanes/ethyl acetate) afforded the pure N-(5-bromo-pyridin-2-yl)-3-cyclopentyl-2-(4-trifluoromethanesulfonyl-phenyl)-propionamide (226 mg, 63%) as a yellow foam: mp 130-132°C; EI-HRMS m/e calcd for C₂₀H₂₀BrF₃N₂O₃S (M⁺) 504.0330, found 504.0325.

### Example 64

### 2-(4-Chloro-3-nitro-phenyl)-3-cyclopentyl-N-thiazol-2-yl-propionamide

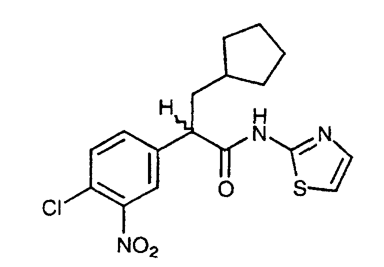

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitro-phenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of n-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitro-phenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitro-phenyl)-3-cyclopentyl-propionic acid methyl ester (260 mg, 0.834 mmol) in tetrahydrofuran (3 mL) was treated with a 0.8M aqueous lithium hydroxide solution (1.25 mL, 1.00 mmol). The reaction mixture was stirred at 25°C for 15 h. The resulting reaction mixture was partitioned between water (50 mL) and ethyl acetate (50 mL) and then treated with a 1N aqueous hydrochloric acid solution (10 mL). The layers were shaken and separated. The aqueous layer was further extracted with ethyl acetate (50 mL). The combined organic layers were dried over magnesium sulfate, filtered, and concentrated *in vacuo* to afford 2-(4-chloro-3-nitro-phenyl)-3-cyclopentylpropionic acid (243 mg, 98%) as a yellow solid which was used without further purification: mp 112-115°C; FAB-HRMS m/e calcd for C₁₄H₁₆ClNO₄ (M+H)⁺ 298.0847, found 298.0851.

A solution of triphenylphosphine (105 mg, 0.403 mmol) in methylene chloride (1 mL) was cooled to 0°C and then slowly treated with *N*-bromosuccinimide (72 mg, 0.403 mmol). The reaction mixture was stirred at 0°C for 20 min and then treated with 2-(4-chloro-3-nitro-phenyl)-3-cyclopentyl-propionic acid (100 mg, 0.336 mmol). The resulting reaction mixture was stirred at 0°C for 10 min and then allowed to warm to 25°C, where it was stirred for 20 min. The reaction mixture was then treated with 2-aminothiazole (74 mg, 0.739 mmol). The resulting reaction mixture was stirred at 25°C for 15 h. The crude reaction mixture was treated with a solution of hexanes/ethyl acetate (2 mL, 3:1) and then directly purified by flash chromatography (Merck Silica gel 60, 230-400 mesh, 3/1 hexanes/ethyl acetate). The pure 2-(4-chloro-3-nitro-phenyl)-3-cyclopentyl-N-thiazol-2-yl-propionamide (93 mg, 73%) was obtained as a pale yellow foam: mp 68-72°C (foam to gel); EI-HRMS m/e calcd for C₁₇H₁₈ClN₃O₃S (M⁺) 379.0757, found 379.0760.

### Example 65

### 2-(4-Chloro-3-nitro-phenyl)-3-cyclopentyl-N-pyridin-2-yl-propionamide

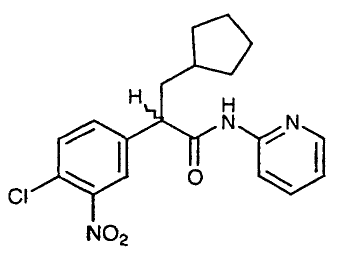

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitro-phenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitro-phenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitro-phenyl)-3-cyclopentyl-propionic acid methyl ester (260 mg, 0.834 mmol) in tetrahydrofuran (3 mL) was treated with a 0.8M aqueous lithium hydroxide solution (1.25 mL, 1.00 mmol). The reaction mixture was stirred at 25°C for 15 h. The resulting reaction mixture was partitioned between water (50 mL) and ethyl acetate (50 mL) and then treated with a 1N aqueous hydrochloric acid solution (10 mL). The layers were shaken and separated. The aqueous layer was further extracted with ethyl acetate (50 mL). The combined organic layers were dried over magnesium sulfate, filtered, and concentrated *in vacuo* to afford 2-(4-chloro-3-nitro-phenyl)-3-cyclopentylpropionic acid (243 mg, 98%) as a yellow solid which was used without further purification: mp 112-115°C; FAB-HRMS m/e calcd for C₁₄H₁₆ClNO₄ (M+H)⁺ 298.0847, found 298.0851.

A solution of triphenylphosphine (105 mg, 0.403 mmol) in methylene chloride (1 mL) was cooled to 0°C and then slowly treated with *N*-bromosuccinimide (72 mg, 0.403 mmol). The reaction mixture was stirred at 0°C for 20 min and then treated with 2-(4-chloro-3-nitro-phenyl)-3-cyclopentyl-propionic acid (100 mg, 0.336 mmol). The resulting reaction mixture was stirred at 0°C for 10 min and then allowed to warm to 25°C, where it was stirred for 20 min. The reaction mixture was then treated with 2-aminopyridine (70 mg, 0.739 mmol). The resulting reaction mixture was stirred at 25°C for 15 h. The crude reaction mixture was treated with a solution of hexanes/ethyl acetate (2 mL, 3:1) and then directly purified by flash chromatography (Merck Silica gel 60, 230-400 mesh, 3/1 hexanes/ethyl acetate). The pure 2-(4-chloro-3-nitro-phenyl)-3-cyclopentyl-N-pyridin-2-yl-propionamide (60 mg, 48%) was obtained as a pale yellow foam: mp 48-52°C (foam to gel); EI-HRMS m/e calcd for C₁₉H₂₀ClN₃O₃ (M⁺) 373.1193, found 373.1185.

### Example 66

### N-(5-Bromo-pyridin-2-yl)-3-cyclopentyl-2-(4-methanesulfonyl-3-nitro-phenyl)-propionamide

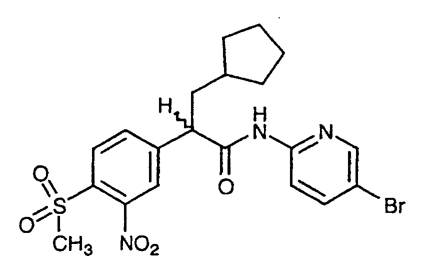

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of n-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo.* Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A solution of triphenylphosphine (212 mg, 0.81 mmol) in methylene chloride (3 mL) was cooled to 0°C and then slowly treated with *N*-bromosuccinimide (144 mg, 0.81 mmol). The reaction mixture was stirred at 0°C for 10 min and then treated with 3-cyclopentyl-2-(4-methanesulfonyl-3-nitrophenyl)-propionic acid (250 mg, 0.73 mmol). The resulting reaction mixture was stirred at 0°C for 5 min and then allowed to warm to 25°C, where it was stirred for 30 min. The reaction mixture was then treated with 2-amino-5-bromopyridine (279 mg, 1.61 mmol). The resulting reaction mixture was stirred at 25°C for 15 h. The crude reaction mixture was directly purified by flash chromatography, (Merck Silica gel 60, 230-400 mesh, 3/1 hexanes/ethyl acetate), to afford N-(5-bromo-pyridin-2-yl)-3-cyclopentyl-2-(4-methanesulfonyl-3-nitro-phenyl)-propionamide (121 mg, 33%) as a white foam: mp 80-83°C (foam to gel); FAB-HRMS m/e calcd for C₂₀H₂₂BrN₃O₅S (M+H)⁺ 496.0542, found 496.0543.

### Example 67

### 3-Cyclopentyl-2-(3-hydroxyamino-4-methanesulfonyl-phenyl)-N-thiazol-2-yl-propionamide

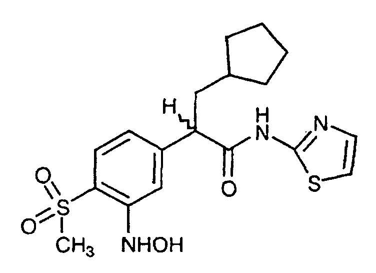

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of n-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A solution of triphenylphosphine (138 mg, 0.53 mmol) in methylene chloride (2 mL) was cooled to 0°C and then slowly treated with *N*-bromosuccinimide (94 mg, 0.53 mmol). The reaction mixture was stirred at 0°C for 10 min and then treated with 3-cyclopentyl-2-(4-methanesulfonyl-3-nitrophenyl)-propionic acid (150 mg, 0.44 mmol). The resulting reaction mixture was stirred at 0°C for 5 min and then allowed to warm to 25°C, where it was stirred for 25 min. The reaction mixture was then treated with 2-aminothiazole (97 mg, 0.97 mmol). The resulting reaction mixture was stirred at 25°C for 15 h. The crude reaction mixture was directly purified by flash chromatography, (Merck Silica gel 60, 230-400 mesh, 1/1 hexanes/ethyl acetate), to afford 3-cyclopentyl-2-(4-methanesulfonyl-3-nitrophenyl)-N-thiazol-2-yl-propionamide (96 mg, 52%) as a pale yellow solid: mp 121-124°C; FAB-HRMS m/e calcd for C₁₈H₂₁N₃O₅S₂ (M+H)⁺ 424.1001, found 424.1000.

A solution of 3-cyclopentyl-2-(4-methanesulfonyl-3-nitrophenyl)-N-thiazol-2-yl-propionamide (150 mg, 0.354 mmol) in methanol (3 mL) was treated with 10% palladium on activated carbon (50 mg). The reaction mixture was stirred under a positive pressure of hydrogen gas (balloon) at 25°C and atmospheric pressure for 3 h. The catalyst was then filtered off through a pad of celite, and the celite pad was washed well with ethyl acetate. The filtrate was concentrated *in vacuo*. Flash chromatography (Merck Silica gel 60, 230-400 mesh, 20/1 methylene chloride/methanol) afforded 3-cyclopentyl-2-(3-hydroxyamino-4-methanesulfonyl-phenyl)-N-thiazol-2-yl-propionamide (85 mg, 59%) as a white solid: mp 124-126°C; EI-HRMS m/e calcd for C₁₈H₂₃N₃O₄S₂ (M⁺) 409.1130, found 409.1131.

### Example 68

### 2-(3-Amino-4-methanesulfonyl-phenyl)-3-cyclopentyl-N-thiazol-2-yl-propionamide

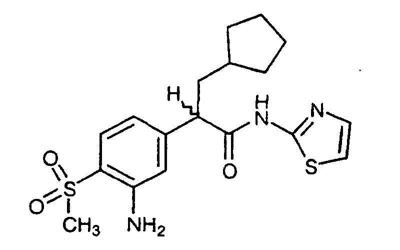

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentylpropionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A solution of triphenylphosphine (138 mg, 0.53 mmol) in methylene chloride (2 mL) was cooled to 0°C and then slowly treated with *N*-bromosuccinimide (94 mg, 0.53 mmol). The reaction mixture was stirred at 0°C for 10 min and then treated with 3-cyclopentyl-2-(4-methanesulfonyl-3-nitrophenyl)-propionic acid (150 mg, 0.44 mmol). The resulting reaction mixture was stirred at 0°C for 5 min and then allowed to warm to 25°C, where it was stirred for 25 min. The reaction mixture was then treated with 2-aminothiazole (97 mg, 0.97 mmol). The resulting reaction mixture was stirred at 25°C for 15 h. The crude reaction mixture was directly purified by flash chromatography, (Merck Silica gel 60, 230-400 mesh, 1/1 hexanes/ethyl acetate), to afford 3-cyclopentyl-2-(4-methanesulfonyl-3-nitrophenyl)-N-thiazol-2-yl-propionamide (96 mg, 52%) as a pale yellow solid: mp 121-124°C; FAB-HRMS m/e calcd for C₁₈H₂₁N₃O₅S₂ (M+H)⁺ 424.1001, found 424.1000.

A solution of 3-cyclopentyl-2-(4-methanesulfonyl-3-nitrophenyl)-N-thiazol-2-yl-propionamide (100 mg, 0.236 mmol) in methanol (2 mL) was treated with a solution of ammonium chloride (27 mg, 0.500 mmol) in water (200 µL). The reaction mixture was then treated with zinc dust (151 mg, 2.31 mmol). The reaction mixture was heated under reflux for 2 h. The reaction mixture was allowed to cool to 25°C and then filtered through a pad of celite. The celite pad was washed well with methanol. The filtrate was concentrated *in vacuo*. Flash chromatography (Merck Silica gel 60, 230-400 mesh, 1/1 hexanes/ethyl acetate) afforded 2-(3-amino-4-methanesulfonyl-phenyl)-3-cyclopentyl-N-thiazol-2-yl-propionamide (40 mg, 43%) as a white solid: mp 207-209°C; EI-HRMS m/e calcd for C₁₈H₂₃N₃O₃S₂ (M⁺) 393.1181, found 393.1180.

### Example 69

### 3-Cyclopentyl-N-thiazol-2-yl-2-(3-trifluoromethanesulfonyl-phenyl)-propionamide

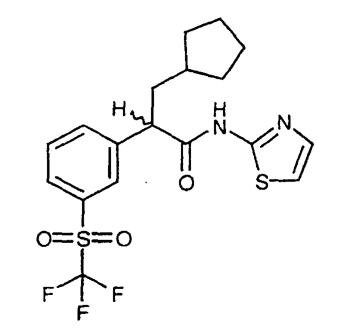

A solution of 3-(trifluoromethylthio)phenylacetic acid (5.00 g, 21.17 mmol) in methanol (50 mL) was treated slowly with 10 drops of concentrated sulfuric acid. The resulting reaction mixture was heated under reflux for 18 h. The reaction mixture was allowed to cool to 25°C and then *concentrated in vacuo* to remove methanol. The residue was diluted with ethyl acetate (100 mL). The organic phase was washed with a saturated aqueous sodium bicarbonate solution (1 x 100 mL), dried over magnesium sulfate, and filtered. The filtrate was concentrated *in vacuo* to afford (3-trifluoromethylsulfanylphenyl)-acetic acid methyl ester (5.28 g, 99%) as a pale yellow oil which was used without further purification: EI-HRMS m/e calcd for C₁₀H₉F₃O₂S (M⁺) 250.0275, found 250.0274.

A solution of diisopropylamine (1.5 mL, 10.5 mmol) in dry tetrahydrofuran (27 mL) and 1,3-dimethyl-3,4,5,6-tetrahydro-2(1H)-pyrimidinone (8 mL) was cooled to -78°C under nitrogen and then treated with a 2.5M solution of n-butyllithium in hexanes (4.2 mL, 10.5 mmol). The resulting reaction mixture was stirred at -78°C for 30 min and then treated dropwise with a solution of (3-trifluoromethylsulfanyl-phenyl)-acetic acid methyl ester (2.50 g, 10.0 mmol) in a small amount of tetrahydrofuran. The reaction mixture was allowed to stir at -78°C for 1 h, at which time, a solution of iodomethylcyclopentane (2.10 g, 10.0 mmol) in a small amount of dry tetrahydrofuran was added dropwise. The reaction mixture was allowed to warm to 25°C where it was stirred for 15 h. The reaction mixture was quenched with water (50 mL) and then partitioned between water (75 mL) and ethyl acetate (75 mL). The layers were shaken and separated. The organic layer was dried over magnesium sulfate, filtered, and concentrated *in vacuo*. Flash chromatography (Merck Silica gel 60, 230-400 mesh, 8/1 hexanes/ethyl acetate) afforded 3-cyclopentyl-2-(3-trifluoromethylsulfanyl-phenyl)-propionic acid methyl ester (2.95 g, 89%) as a colorless oil: EI-HRMS m/e calcd for C₁₆H₁₉F₃O₂S (M⁺) 332.1058, found 332.1047.

A solution of 3-cyclopentyl-2-(3-trifluoromethylsulfanyl-phenyl)-propionic acid methyl ester (2.75 g, 8.27 mmol) in methylene chloride (30 mL) was treated with 3-chloroperoxybenzoic acid (80-85% grade, 4.28 g based on 80%, 20.67 mmol). The reaction mixture was stirred at 25°C for 6 h, at which time, thin layer chromatography showed the presence of two new lower R_{f} products. An additional 4.00 g of 3-chloroperoxybenzoic acid was added to the reaction mixture to drive the conversion of the sulfoxide to the sulfone, and the resulting reaction mixture was stirred at 40°C for 3 d. The reaction mixture was allowed to cool to 25°C and then partitioned between water (100 mL) and methylene chloride (100 mL). The layers were shaken and separated. The organic phase was washed twice with a saturated aqueous sodium bicarbonate solution, washed with water, dried over magnesium sulfate, filtered, and concentrated *in vacuo*. Flash chromatography (Merck Silica gel 60, 230-400 mesh, 1/1 hexanes/methylene chloride) afforded 3-cyclopentyl-2-(3-trifluoromethanesulfonyl-phenyl)-propionic acid methyl ester (2.07 g, 69%) as a colorless oil: EI-HRMS m/e calcd for C₁₆H₁₉F₃O₄S (M⁺) 364.0956, found 364.0947.

A solution of 3-cyclopentyl-2-(3-trifluoromethanesulfonyl-phenyl)-propionic acid methyl ester (1.28 g, 3.52 mmol) in tetrahydrofuran (12 mL) was treated with a 0.8M aqueous lithium hydroxide solution (4.9 mL, 3.88 mmol). The reaction mixture was stirred at 25°C for 24 h and then concentrated *in vacuo* to remove tetrahydrofuran. The resulting yellow oil was partitioned between water (50 mL) and ethyl acetate (50 mL) and then treated with a 1N aqueous hydrochloric acid solution. The organic layer was dried over magnesium sulfate, filtered, and concentrated *in vacuo.* Flash chromatography (Merck Silica gel 60, 230-400 mesh, 1/1 hexanes/ethyl acetate) afforded 3-cyclopentyl-2-(3-trifluoromethanesulfonyl-phenyl)-propionic acid (1.09 g, 99%) as a viscous yellow oil that solidified upon sitting to a white solid: mp 86-88°C; EI-HRMS m/e calcd for C₁₅H₁₇F₃O₄S (M⁺) 350.0800, found 350.0792.

A solution of triphenylphosphine (194 mg, 0.74 mmol) in methylene chloride (3 mL) was cooled to 0°C and then slowly treated with *N*-bromosuccinimide (132 mg, 0.74 mmol). The reaction mixture was stirred at 0°C for 15 min and then treated with 3-cyclopentyl-2-(3-trifluoromethanesulfonyl-phenyl)-propionic acid (200 mg, 0.57 mmol). The resulting reaction mixture was stirred at 0°C for 5 min and then allowed to warm to 25°C over 30 min. The reaction mixture was then treated with 2-aminothiazole (143 mg, 1.43 mmol). The resulting reaction mixture was stirred at 25°C for 15 h. The crude reaction mixture was then directly purified by flash chromatography (Merck Silica gel 60, 230-400 mesh, 3/1 hexanes/ethyl acetate) to afford pure 3-cyclopentyl-N-thiazol-2-yl-2-(3-trifluoromethanesulfonyl-phenyl)-propionamide (178 mg, 72%) as a light yellow foam: mp 61-64°C (foam to gel); EI-HRMS m/e calcd for C₁₈H₁₉F₃N₂O₃S₂ (M⁺) 432.0789, found 432.0790.

### Example 70

### 3-Cyclopentyl-2-(3-fluoro-4-trifluoromethyl-phenyl)-N-thiazol-2-yl-propionamide

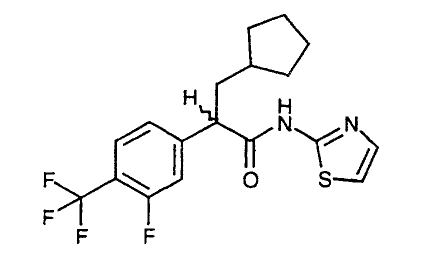

A solution of 3-fluoro-4-(trifluoromethyl)phenylacetic acid (2.50 g, 11.25 mmol) in methanol (25 mL) was treated slowly with 4 drops of concentrated sulfuric acid. The resulting reaction mixture was heated under reflux for 15 h. The reaction mixture was allowed to cool to 25°C and then concentrated *in vacuo* to remove methanol. Flash chromatography (Merck Silica gel 60, 230-400 mesh, 1/1 hexanes/ethyl acetate) afforded (3-fluoro-4-trifluoromethyl-phenyl)-acetic acid methyl ester (2.58 g, 97%) as a colorless oil: EI-HRMS m/e calcd for C₁₀H₈F₄O₂ (M⁺) 236.0460, found 236.0457.

A solution of diisopropylamine (1.5 mL, 10.67 mmol) in dry tetrahydrofuran (24 mL) and 1,3-dimethyl-3,4,5,6-tetrahydro-2(1H)-pyrimidinone (8 mL) was cooled to -78°C under nitrogen and then treated with a 2.5M solution of n-butyllithium in hexanes (4.3 mL, 10.67 mmol). The resulting reaction mixture was stirred at -78°C for 45 min and then treated dropwise with a solution of (3-fluoro-4-trifluoromethyl-phenyl)-acetic acid methyl ester (2.40 g, 10.16 mmol) in a small amount of tetrahydrofuran. The reaction mixture was allowed to stir at -78°C for 1 h, at which time, a solution of iodomethylcyclopentane (2.24 g, 10.67 mmol) in a small amount of dry tetrahydrofuran was added dropwise. The reaction mixture was allowed to warm to 25°C where it was stirred for 15 h. The reaction mixture was quenched with a saturated aqueous ammonium chloride solution (10 mL) and then partitioned between water (75 mL) and ethyl acetate (75 mL). The layers were shaken and separated. The aqueous layer was further extracted with ethyl acetate (1 x 75 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 5/1 hexanes/ethyl acetate) afforded 3-cyclopentyl-2-(3-fluoro-4-trifluoromethyl-phenyl)-propionic acid methyl ester (2.69 g, 83%) as a colorless oil: EI-HRMS m/e calcd for C₁₆H₁₈F₄O₂ (M⁺) 318.1243, found 318.1250.

A solution of 3-cyclopentyl-2-(3-fluoro-4-trifluoromethyl-phenyl)-propionic acid methyl ester (1.80 g, 5.69 mmol) in tetrahydrofuran (15 mL) was treated with a 0.8M aqueous lithium hydroxide solution (7.1 mL, 5.69 mmol). The reaction mixture was stirred at 25°C for 15 h. The reaction mixture was concentrated *in vacuo*. The resulting residue was diluted with ethyl acetate (100 mL) and then washed with a 5% aqueous hydrochloric acid solution and a saturated aqueous sodium chloride solution. The organic layer was dried over magnesium sulfate, filtered, and concentrated *in vacuo*. Flash chromatography (Merck Silica gel 60, 230-400 mesh, 2/1 hexanes/ethyl acetate) afforded 3-cyclopentyl-2-(3-fluoro-4-trifluoromethyl-phenyl)-propionic acid (1.11 g, 64%) as a white solid: mp 93-95°C; FAB-HRMS m/e calcd for C₁₅H₁₆F₄O₂ (M+H)⁺ 305.1165, found 305.1175.

A solution of triphenylphosphine (312 mg, 1.19 mmol) in methylene chloride (5 mL) was cooled to 0°C and then slowly treated with *N*-bromosuccinimide (212 mg, 1.19 mmol). The reaction mixture was stirred at 0°C for 30 min and then treated with 3-cyclopentyl-2-(3-fluoro-4-trifluoromethyl-phenyl)-propionic acid (300 mg, 0.99 mmol). The resulting reaction mixture was stirred at 0°C for 15 min and then allowed to warm to 25°C where it was stirred for 30 min. The reaction mixture was then treated with 2-aminothiazole (218 mg, 2.18 mmol). The resulting reaction mixture was stirred at 25°C for 3 d. The crude reaction mixture was then directly purified by flash chromatography (Merck Silica gel 60, 230-400 mesh, 2/1 hexanes/ethyl acetate) to afford 3-cyclopentyl-2-(3-fluoro-4-trifluoromethyl-phenyl)-N-thiazol-2-yl-propionamide (243 mg, 64%) as a white solid: mp 194-195°C; EI-HRMS m/e calcd for C₁₈H₁₈F₄N₂OS (M⁺) 386.1076, found 386.1076.

### Example 71

### 3-Cyclopentyl-2-(3-fluoro-4-trifluoromethyl-phenyl)-N-pyridin-2-yl-propionamide

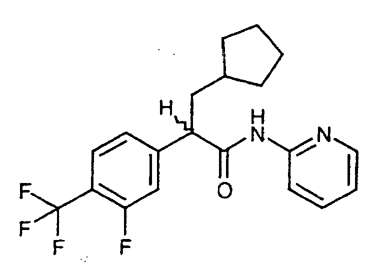

A solution of 3-fluoro-4-(trifluoromethyl)phenylacetic acid (2.50 g, 11.25 mmol) in methanol (25 mL) was treated slowly with 4 drops of concentrated sulfuric acid. The resulting reaction mixture was heated under reflux for 15 h. The reaction mixture was allowed to cool to 25°C and then concentrated *in vacuo* to remove methanol. Flash chromatography (Merck Silica gel 60, 230-400 mesh, 1/1 hexanes/ethyl acetate) afforded (3-fluoro-4-trifluoromethyl-phenyl)-acetic acid methyl ester (2.58 g, 97%) as a colorless oil: EI-HRMS m/e calcd for C₁₀H₈F₄O₂ (M⁺) 236.0460, found 236.0457.

A solution of diisopropylamine (1.5 mL, 10.67 mmol) in dry tetrahydrofuran (24 mL) and 1,3-dimethyl-3,4,5,6-tetrahydro-2(1H)-pyrimidinone (8 mL) was cooled to -78°C under nitrogen and then treated with a 2.5M solution of *n*-butyllithium in hexanes (4.3 mL, 10.67 mmol). The resulting reaction mixture was stirred at -78°C for 45 min and then treated dropwise with a solution of (3-fluoro-4-trifluoromethyl-phenyl)-acetic acid methyl ester (2.40 g, 10.16 mmol) in a small amount of tetrahydrofuran. The reaction mixture was allowed to stir at -78°C for 1 h, at which time, a solution of iodomethylcyclopentane (2.24 g, 10.67 mmol) in a small amount of dry tetrahydrofuran was added dropwise. The reaction mixture was allowed to warm to 25°C where it was stirred for 15 h. The reaction mixture was quenched with a saturated aqueous ammonium chloride solution (10 mL) and then partitioned between water (75 mL) and ethyl acetate (75 mL). The layers were shaken and separated. The aqueous layer was further extracted with ethyl acetate (75 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 5/1 hexanes/ethyl acetate) afforded 3-cyclopentyl-2-(3-fluoro-4-trifluoromethyl-phenyl)-propionic acid methyl ester (2.69 g, 83%) as a colorless oil: EI-HRMS m/e calcd for C₁₆H₁₈F₄O₂ (M⁺) 318.1243, found 318.1250.

A solution of 3-cyclopentyl-2-(3-fluoro-4-trifluoromethyl-phenyl)-propionic acid methyl ester (1.80 g, 5.69 mmol) in tetrahydrofuran (15 mL) was treated with a 0.8M aqueous lithium hydroxide solution (7.1 mL, 5.69 mmol). The reaction mixture was stirred at 25°C for 15 h. The reaction mixture was concentrated *in vacuo*. The resulting residue was diluted with ethyl acetate (100 mL) and then washed with a 5% aqueous hydrochloric acid solution and a saturated aqueous sodium chloride solution. The organic layer was dried over magnesium sulfate, filtered, and concentrated *in vacuo*. Flash chromatography (Merck Silica gel 60, 230-400 mesh, 2/1 hexanes/ethyl acetate) afforded 3-cyclopentyl-2-(3-fluoro-4-trifluoromethyl-phenyl)-propionic acid (1.11 g, 64%) as a white solid: mp 93-95°C; FAB-HRMS m/e calcd for C₁₅H₁₆F₄O₂ (M+H)⁺, 305.1165, found 305.1175.

A solution of triphenylphosphine (312 mg, 1.19 mmol) in methylene chloride (5 mL) was cooled to 0°C and then slowly treated with *N*-bromosuccinimide (212 mg, 1.19 mmol). The reaction mixture was stirred at 0°C for 30 min and then treated with 3-cyclopentyl-2-(3-fluoro-4-trifluoromethyl-phenyl)-propionic acid (300 mg, 0.99 mmol). The resulting reaction mixture was stirred at 0°C for 15 min and then allowed to warm to 25°C where it was stirred for 30 min. The reaction mixture was then treated with 2-aminopyridine (205 mg, 2.18 mmol). The resulting reaction mixture was stirred at 25°C for 3 d. The crude reaction mixture was then directly purified by flash chromatography (Merck Silica gel 60, 230-400 mesh, 2/1 hexanes/ethyl acetate) to afford 3-cyclopentyl-2-(3-fluoro-4-trifluoromethyl-phenyl)-N-pyridin-2-yl-propionamide (171 mg, 45%) as a pale yellow foam: mp 40-44°C (foam to gel); EI-HRMS m/e calcd for C₂₀H₂₀F₄N₂O (M⁺) 380.1512, found 380.1519.

### Example 72

### 2-(3-Bromo-4-methanesulfonyl-phenyl)-3-cyclopentyl-N-thiazol-2-yl-propionamide

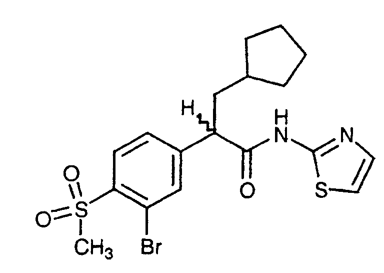

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was slowly treated with bromine (1.74 mL, 33.84 mmol). The reaction mixture was stirred at 25°C for 4 h, at which time, thin layer chromatography still indicated the presence of a substantial amount of starting material. The reaction mixture was treated with more bromine (1.74 mL, 33.84 mmol). The reaction mixture was stirred an additional 4 h at 25°C and then quenched with a 10% aqueous sodium bisulfite solution (150 mL). The reaction mixture was concentrated *in vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) was cooled to -78°C under nitrogen and then treated with a 2.5M solution of *n*-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h, at which time, a solution of iodomethylcyclopentane (5.59 g, 26.60 mmol) in a small amount of dry tetrahydrofuran was added dropwise. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate). afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A solution of 2-(3-bromo-4-methanesulfonyl-phenyl)-3-cyclopentyl-propionic acid methyl ester (1.62 g, 4.16 mol) in methanol (10 mL) was treated with a 1N aqueous sodium hydroxide solution (8.7 mL, 8.74 mol). The reaction mixture was stirred at 25°C for 27 h. The reaction mixture was concentrated *in vacuo* to remove methanol. The resulting aqueous residue was acidified to pH = 2 with a 10% aqueous hydrochloric acid solution and then extracted with ethyl acetate (1 x 400 mL). The organic layer was washed with water (1 x 300 mL) and a saturated aqueous sodium chloride solution (1 x 300 mL). The organic layer was then dried over sodium sulfate, filtered, and concentrated *in vacuo* to afford 2-(3-bromo-4-methanesulfonyl-phenyl)-3-cyclopentylpropionic acid (1.39 g, 89%) as a white solid which was used without further purification: mp 149-150°C; FAB-HRMS m/e calcd for C₁₅H₁₉BrO₄S (M+H)⁺ 375.0266, found 375.0274.

A solution of triphenylphosphine (168 mg, 0.64 mmol) in methylene chloride (3 mL) was cooled to 0°C and then slowly treated with *N*-bromosuccinimide (114 mg, 0.64 mmol). The reaction mixture was stirred at 0°C for 10 min and then treated with 2-(3-bromo-4-methanesulfonyl-phenyl)-3-cyclopentyl-propionic acid (200 mg, 0.53 mmol). The resulting reaction mixture was stirred at 0°C for 5 min and then allowed to warm to 25°C where it was stirred for 25 min. The reaction mixture was then treated with 2-aminothiazole (117 mg, 1.17 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3-bromo-4-methanesulfonyl-phenyl)-3-cyclopentyl-N-thiazol-2-yl-propionamide (214 mg, 88%) as a yellow solid: mp 106-107°C; EI-HRMS m/e calcd for C₁₈H₂₁BrN₂O₃S₂ (M⁺) 456.0177, found 456.0175.

### Example 73

### 2-(3-Bromo-4-methanesulfonyl-phenyl)-3-cyclopentyl-N-pyridin-2-yl-propionamide

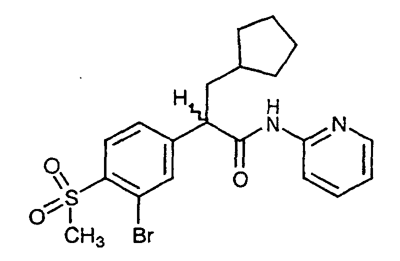

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was slowly treated with bromine (1.74 mL, 33.84 mmol). The reaction mixture was stirred at 25°C for 4 h, at which time, thin layer chromatography still indicated the presence of a substantial amount of starting material. The reaction mixture was treated with more bromine (1.74 mL, 33.84 mmol). The reaction mixture was stirred an additional 4 h at 25°C and then quenched with a 10% aqueous sodium bisulfite solution (150 mL). The reaction mixture was concentrated *in vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) was cooled to -78°C under nitrogen and then treated with a 2.5M solution of *n*-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h, at which time, a solution of iodomethylcyclopentane (5.59 g, 26.60 mmol) in a small amount of dry tetrahydrofuran was added dropwise. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated in *vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A solution of 2-(3-bromo-4-methanesulfonyl-phenyl)-3-cyclopentyl-propionic acid methyl ester (1.62 g, 4.16 mol) in methanol (10 mL) was treated with a 1N aqueous sodium hydroxide solution (8.7 mL, 8.74 mol). The reaction mixture was stirred at 25°C for 27 h. The reaction mixture was concentrated *in vacuo* to remove methanol. The resulting aqueous residue was acidified to pH = 2 with a 10% aqueous hydrochloric acid solution and then extracted with ethyl acetate (1 x 400 mL). The organic layer was washed with water (1 x 300 mL) and a saturated aqueous sodium chloride solution (1 x 300 mL). The organic layer was then dried over sodium sulfate, filtered, and concentrated *in vacuo* to afford 2-(3-bromo-4-methanesulfonyl-phenyl)-3-cyclopentylpropionic acid (1.39 g, 89%) as a white solid which was used without further purification: mp 149-150°C; FAB-HRMS m/e calcd for C₁₅H₁₉BrO₄S (M+H)⁺ 375.0266, found 375.0274.

A solution of triphenylphosphine (168 mg, 0.64 mmol) in methylene chloride (3 mL) was cooled to 0°C and then slowly treated with *N*-bromosuccinimide (114 mg, 0.64 mmol). The reaction mixture was stirred at 0°C for 10 min and then treated with 2-(3-bromo-4-methanesulfonyl-phenyl)-3-cyclopentyl-propionic acid (200 mg, 0.53 mmol). The resulting reaction mixture was stirred at 0°C for 5 min and then allowed to warm to 25°C where it was stirred for 25 min. The reaction mixture was then treated with 2-aminopyridine (110 mg, 1.17 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3-bromo-4-methanesulfonyl-phenyl)-3-cyclopentyl-N-pyridin-2-yl-propionamide (175 mg, 73%) as a white foam: mp 99-101°C; FAB-HRMS m/e calcd for C₂₀H₂₃BrN₂O₃S (M+H)⁺ 451.0692, found 451.0689.

### Example 74

### 2-(3-Bromo-4-methanesulfonyl-phenyl)-N-(5-bromo-pyridin-2-yl)-3-cyclopentylpropionamide

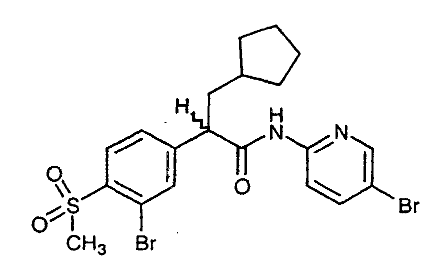

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was slowly treated with bromine (1.74 mL, 33.84 mmol). The reaction mixture was stirred at 25°C for 4 h, at which time, thin layer chromatography still indicated the presence of a substantial amount of starting material. The reaction mixture was treated with more bromine (1.74 mL, 33.84 mmol). The reaction mixture was stirred an additional 4 h at 25°C and then quenched with a 10% aqueous sodium bisulfite solution (150 mL). The reaction mixture was concentrated in *vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) was cooled to -78°C under nitrogen and then treated with a 2.5M solution of *n*-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h, at which time, a solution of iodomethylcyclopentane (5.59 g, 26.60 mmol) in a small amount of dry tetrahydrofuran was added dropwise. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A solution of 2-(3-bromo-4-methanesulfonyl-phenyl)-3-cyclopentyl-propionic acid methyl ester (1.62 g, 4.16 mol) in methanol (10 mL) was treated with a 1N aqueous sodium hydroxide solution (8.7 mL, 8.74 mol). The reaction mixture was stirred at 25°C for 27 h. The reaction mixture was concentrated *in vacuo* to remove methanol. The resulting aqueous residue was acidified to pH = 2 with a 10% aqueous hydrochloric acid solution and then extracted with ethyl acetate (1 x 400 mL). The organic layer was washed with water (1 x 300 mL) and a saturated aqueous sodium chloride solution (1 x 300 mL). The organic layer was then dried over sodium sulfate, filtered, and concentrated *in vacuo* to afford 2-(3-bromo-4-methanesulfonyl-phenyl)-3-cyclopentylpropionic acid (1.39 g, 89%) as a white solid which was used without further purification: mp 149-150°C; FAB-HRMS m/e calcd for C₁₅H₁₉BrO₄S (M+H)⁺ 375.0266, found 375.0274.

A solution of triphenylphosphine (154 mg, 0.59 mmol) in methylene chloride (3 mL) was cooled to 0°C and then slowly treated with *N*-bromosuccinimide (104 mg, 0.59 mmol). The reaction mixture was stirred at 0°C for 10 min and then treated with 2-(3-bromo-4-methanesulfonyl-phenyl)-3-cyclopentyl-propionic acid (200 mg, 0.53 mmol). The resulting reaction mixture was stirred at 0°C for 5 min and then allowed to warm to 25°C where it was stirred for 30 min. The reaction mixture was then treated with 2-amino-5-bromopyridine (203 mg, 1.17 mmol). The resulting reaction mixture was stirred at 25°C for 15 h. The crude reaction mixture was then directly purified by flash chromatography (Merck Silica gel 60, 230-400 mesh, 3/1 hexanes/ethyl acetate) to afford the 2-(3-bromo-4-methanesulfonyl-phenyl)-N-(5-bromo-pyridin-2-yl)-3-cyclopentyl-propionamide (164 mg, 58%) as a white foam: mp 83-86°C (foam to gel); FAB-HRMS m/e calcd for C₂₀H₂₂Br₂N₂O₃S (M+H)⁺ 528.9796, found 528.9783.

### Example 75

### 2-(3-Cyano-4-methanesulfonyl-phenyl)-3-cyclopentyl-N-thiazol-2-yl-propionamide

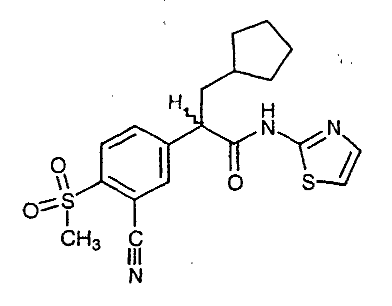

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was slowly treated with bromine (1.74 mL, 33.84 mmol). The reaction mixture was stirred at 25°C for 4 h, at which time, thin layer chromatography still indicated the presence of a substantial amount of starting material. The reaction mixture was treated with more bromine (1.74 mL, 33.84 mmol). The reaction mixture was stirred an additional 4 h at 25°C and then quenched with a 10% aqueous sodium bisulfite solution (150 mL). The reaction mixture was concentrated in *vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) was cooled to -78°C under nitrogen and then treated with a 2.5M solution of *n*-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h, at which time, a solution of iodomethylcyclopentane (5.59 g, 26.60 mmol) in a small amount of dry tetrahydrofuran was added dropwise. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate , solution (3 x 200 mL) and a saturated aqueous sodium chloride solution (1 x 100 mL), . dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A mixture of 2-(3-bromo-4-methanesulfonyl-phenyl)-3-cyclopentyl-propionic acid methyl ester (990.0 mg, 2.54 mmol) and copper(I) cyanide (273.3 mg, 3.05 mmol) in dry *N*,*N*-dimethylformamide (2.5 mL) was heated under reflux for 4 h. The reaction was allowed to cool to 25°C, and the crude reaction mixture was directly purified without further chemical work-up. Flash chromatography (Merck Silica gel 60, 70-230 mesh, 100% hexanes then 3/1 hexanes/ethyl acetate) afforded 2-(3-cyano-4-methanesulfonyl-phenyl)-3-cyclopentyl-propionic acid methyl ester (646.5 mg, 76%) as a very light yellow oil: EI-HRMS m/e calcd for C₁₇H₂₁NO₄S (M⁺) 335.1191, found 335.1185.

A solution of 2-(3-cyano-4-methanesulfonyl-phenyl)-3-cyclopentyl-propionic acid methyl ester (4.84 g, 14.4 mol) in tetrahydrofuran (25 mL) was treated with a 0.8M aqueous lithium hydroxide solution (27 mL, 21.6 mmol). The reaction mixture was stirred at 25°C for 2.5 h. The reaction mixture was partitioned between water and ethyl acetate and then acidified to pH = 2 with a 10% aqueous hydrochloric acid solution. The layers were shaken and separated. The resulting organic layer was washed with a saturated aqueous sodium chloride solution, dried over magnesium sulfate, filtered, and concentrated *in vacuo* to afford crude 2-(3-cyano-4-methanesulfonyl-phenyl)-3-cyclopentyl-propionic acid (3.80 g, 82%) as a pale yellow oil that solidified to a pale yellow solid. An analytical sample was obtained by recrystallization from ethyl acetate to afford 2-(3-cyano-4-methanesulfonyl-phenyl)-3-cyclopentyl-propionic acid as a white solid: mp 180-181°C; EI-HRMS m/e calcd for C₁₆H₁₉NO₄S (M⁺) 321.1034, found 321.1039.

A solution of triphenylphosphine (98 mg, 0.37 mmol) in methylene chloride (1 mL) was cooled to 0°C and then slowly treated with *N*-bromosuccinimide (67 mg, 0.37 mmol). The reaction mixture was stirred at 0°C for 15 min and then treated with 2-(3-cyano-4-methanesulfonyl-phenyl)-3-cyclopentyl-propionic acid (100 mg, 0.31 mmol). The resulting reaction mixture was stirred at 0°C for 5 min and then allowed to warm to 25°C where it was stirred for 30 min. The reaction mixture was then treated with 2-aminothiazole (68 mg, 0.68 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3-cyano-4-methanesulfonyl-phenyl)-3-cyclopentyl-N-thiazol-2-yl-propionamide (117 mg, 93%) as a white solid: mp 145-148°C; EI-HRMS m/e calcd for C₁₉H₂₁N₃O₃S₂ (M⁺) 403.1024, found 403.1023.

### Example 76

### 2-(3-Cyano-4-methanesulfonyl-phenyl)-3-cyclopentyl-N-pyridin-2-yl-propionamide

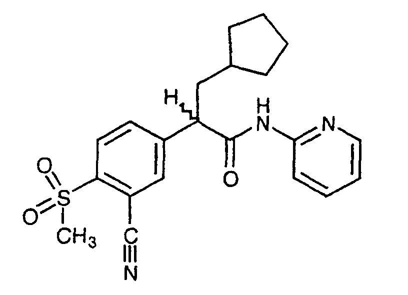

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfinyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was slowly treated with bromine (1.74 mL, 33.84 mmol). The resulting reaction mixture was stirred at 25°C for 4 h, at which time, thin layer chromatography still indicated the presence of a substantial amount of starting material. The reaction mixture was treated with more bromine (1.74 mL, 33.84 mmol). The reaction mixture was stirred an additional 4 h at 25°C and then quenched with a 10% aqueous sodium bisulfite solution (150 mL). The reaction mixture was concentrated *in vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) was cooled to -78°C under nitrogen and then treated with a 2.5M solution of *n*-butyllithium in hexanes (9.8 mL, 24.38 mmol). The reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for I h, at which time, a solution of iodomethylcyclopentane (5.59 g, 26.60 mmol) in a small amount of dry tetrahydrofuran was added dropwise. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A mixture of 2-(3-bromo-4-methanesulfonyl-phenyl)-3-cyclopentyl-propionic acid methyl ester (990.0 mg, 2.54 mmol) and copper(I) cyanide (273.3 mg, 3.05 mmol) in dry *N*,*N*-dimethylformamide (2.5 mL) was heated under reflux for 4 h. The reaction was allowed to cool to 25°C, and the crude reaction mixture was directly purified without further chemical work-up. Flash chromatography (Merck Silica gel 60, 70-230 mesh, 100% hexanes then 3/1 hexanes/ethyl acetate) afforded 2-(3-cyano-4-methanesulfonyl-phenyl)-3-cyclopentyl-propionic acid methyl ester (646.5 mg, 76%) as a very light yellow oil: EI-HRMS m/e calcd for C₁₇H₂₁NO₄S (M⁺) 335.1191, found 335.1185.

A solution of 2-(3-cyano-4-methanesulfonyl-phenyl)-3-cyclopentyl-propionic acid methyl ester (4.84 g, 14.4 mol) in tetrahydrofuran (25 mL) was treated with a 0.8M aqueous lithium hydroxide solution (27 mL, 21.6 mmol). The reaction mixture was stirred at 25°C for 2.5 h. The reaction mixture was partitioned between water and ethyl acetate and then acidified to pH = 2 with a 10% aqueous hydrochloric acid solution. The layers were shaken and separated. The resulting organic layer was washed with a saturated aqueous sodium chloride solution, dried over magnesium sulfate, filtered, and concentrated *in vacuo* to afford crude 2-(3-cyano-4-methanesulfonyl-phenyl)-3-cyclopentyl-propionic acid (3.80 g, 82%) as a pale yellow oil that solidified to a pale yellow solid. An analytical sample was obtained by recrystallization from ethyl acetate to afford 2-(3-cyano-4-methanesulfonyl-phenyl)-3-cyclopentyl-propionic acid as a white solid: mp 180-181°C; EI-HRMS m/e calcd for C₁₆H₁₉NO₄S (M⁺) 321.1034, found 321.1039.

A solution of triphenylphosphine (98 mg, 0.37 mmol) in methylene chloride (1 mL) was cooled to 0°C and then slowly treated with *N*-bromosuccinimide (67 mg, 0.37 mmol). The reaction mixture was stirred at 0°C for 15 min and then treated with 2-(3-cyano-4-methanesulfonyl-phenyl)-3-cyclopentyl-propionic acid (100 mg, 0.31 mmol). The resulting reaction mixture was stirred at 0°C for 5 min and then allowed to warm to 25°C where it was stirred for 30 min. The reaction mixture was then treated with 2-aminopyridine (64 mg, 0.68 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3-cyano-4-methanesulfonyl-phenyl)-3-cyclopentyl-N-pyridin-2-yl-propionamide (94.5 mg, 76%) as a yellow foam: mp 87-90°C (foam to gel); EI-HRMS m/e calcd for C₂₁H₂₃N₃O₃S (M⁺) 397.1460, found 397.1460.

### Example 77

### 3-Cyclopentyl-2-(4-ethanesulfonyl-phenyl)-N-thiazol-2-yl-propionamide

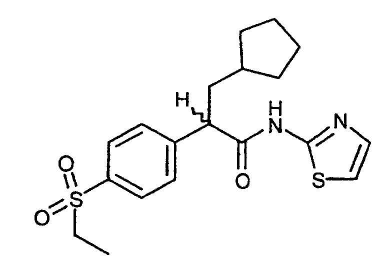

A mixture of aluminum chloride (72.35 g, 0.54 mol) in chloroform (181 mL) was cooled to 0°C and stirred until the solid material dissolved. The reaction mixture was then slowly treated with ethyl oxalyl chloride (61 mL, 0.54 mol), and the resulting reaction mixture was stirred at 0°C for 30 min. The reaction mixture was then slowly treated with ethyl phenyl sulfide (25.00 g, 0.18 mol). The solution turned to a wine color and slowly became gum-like. The resulting reaction mixture was then stirred at 0°C for 2 h. The reaction mixture was slowly poured into a large amount of ice/water. The resulting aqueous layer was extracted with chloroform (3 x 200 mL). The combined organic layers were dried over sodium sulfate, filtered, and concentrated *in vacuo*. Flash chromatography (Merck Silica gel 60, 70-230 mesh, 9/1 hexanes/ethyl acetate) afforded (4-ethylsulfanyl-phenyl)-oxo-acetic acid ethyl ester (23.64 g, 55%) as a yellow oil. The material was used without further purification and characterization in subsequent reactions.

A solution of iodomethylcyclopentane (4.60 g, 21.89 mmol) and triphenylphosphine (5.74 g, 21.89 mmol) in acetonitrile (22 mL) was heated under reflux for 2 weeks. The reaction mixture was allowed to cool to 25°C and then concentrated *in vacuo* to provide an orange solid. The orange solid was triturated with diethyl ether and then filtered. The solid was washed well with diethyl ether until the washings showed the absence of iodomethylcyclopentane and triphenylphosphine by thin layer chromatography. The solid was allowed to air dry to afford cyclopentylmethyl triphenylphosphonium iodide (8.92 g, 86%) as a light orange solid: mp 195-198°C; FAB-HRMS m/e calcd for C₂₄H₂₆P (M+H)⁺ 345.1772, found 345.1784.

A suspension of cyclopentylmethyl triphenylphosphonium iodide (24.48 g, 51.82 mmol) in dry tetrahydrofuran (100 mL) was cooled to 0°C and then treated dropwise with a 1.0M solution of sodium bis(trimethylsilyl)amide in tetrahydrofuran (52 mL, 51.82 mmol). The bright orange reaction mixture was stirred at 0°C for 1 h. The reaction mixture was then treated with (4-ethylsulfanyl-phenyl)-oxo-acetic acid ethyl ester (9.50 g, 39.87 mmol). The resulting reaction mixture was allowed to warm to 25°C where it was stirred for 20 h. The reaction mixture was concentrated *in vacuo* to remove tetrahydrofuran and then diluted with water (300 mL). The aqueous layer was extracted with ethyl acetate (3 x 200 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded the 3-cyclopentyl-2-(4-ethylsulfanyl-phenyl)-acrylic acid ethyl ester (6.08 g, 50%) as a yellow oil containing a 1.82:1 mixture of (E):(Z) isomers: FAB-LRMS m/e calcd for C₁₈H₂₄O₂S (M+H)⁺ integer mass 304, found 305. The isomeric mixture was used without further separation in subsequent reactions.

A solution of 3-cyclopentyl-2-(4-ethylsulfanyl-phenyl)-acrylic acid ethyl ester [5.76 g, 18.92 mmol, (E):(Z) = 1.82:1] in methylene chloride (47 mL) was slowly treated with 3-chloroperoxybenzoic acid (57-86% grade, 11.45 g based on 57%, 37.83 mmol). The reaction mixture was stirred at 25°C for 1 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 200 mL), dried over sodium sulfate, filtered, and concentrated *in vacuo*. Flash chromatography (Merck Silica gel 60, 230-400 mesh, 3/1 hexanes/ethyl acetate) afforded 3-cyclopentyl-2-(4-ethanesulfonyl-phenyl)-acrylic acid ethyl ester (4.89 g, 77%) as a colorless oil. The product was a 3:1 mixture of (E):(Z) isomers that was used without further purification and characterization.

A solution of 3-cyclopentyl-2-(4-ethanesulfonyl-phenyl)-acrylic acid ethyl ester [4.89 g, 14.53 mmol, (E):(Z) = 3:1] in ethanol (36 mL) was slowly treated with 10% palladium on activated carbon (244.5 mg). The reaction mixture was stirred under a positive pressure of hydrogen gas (balloon) at 25°C and atmospheric pressure for 44 h. The catalyst was then filtered off through a pad of celite, and the celite pad was washed well with ethyl acetate. The filtrate was concentrated *in vacuo*. Flash chromatography (Merck Silica gel 60, 70-230 mesh, 3/1 hexanes/ethyl acetate) afforded 3-cyclopentyl-2-(4-ethanesulfonyl-phenyl)-propionic acid ethyl ester (3.50 g, 71%) as a colorless viscous oil: FAB-LRMS m/e calcd for C₁₈H₂₆O₄S (M+H)⁺, integer mass 338, found 339.

A solution of 3-cyclopentyl-2-(4-ethanesulfonyl-phenyl)-propionic acid ethyl ester (2.50 g, 7.39 mmol) in tetrahydrofuran (30 mL) was treated with a 0.8M aqueous lithium hydroxide solution (11.1 mL, 8.86 mmol). The reaction mixture was stirred at 25°C for 23 h. The resulting reaction mixture was partitioned between water (75 mL) and ethyl acetate (75 mL) and then treated with a 1N aqueous hydrochloric acid solution (15 mL). The layers were shaken and separated. The organic layer was dried over magnesium sulfate, filtered, and concentrated *in vacuo* to afford 3-cyclopentyl-2-(4-ethanesulfonyl-phenyl)-propionic acid (2.20 g, 96%) as a white solid which was used without further purification: mp 137-138°C; FAB-HRMS m/e calcd for C₁₆H₂₂O₄S (M+H)⁺ 311.1317, found 311.1321.

A solution of triphenylphosphine (279 mg, 1.06 mmol) in methylene chloride (5 mL) was cooled to 0°C and then slowly treated with *N*-bromosuccinimide (189 mg, 1.06 mmol). The reaction mixture was stirred at 0°C for 20 min and then treated with 3-cyclopentyl-2-(4-ethanesulfonyl-phenyl)-propionic acid (300 mg, 0.97 mmol). The resulting reaction mixture was stirred at 0°C for 10 min and then allowed to warm to 25°C where it was stirred for 30 min. The reaction mixture was then treated with 2-aminothiazole (213 mg, 2.13 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 3-cyclopentyl-2-(4-ethanesulfonyl-phenyl)-N-thiazol-2-yl-propionamide (330 mg, 87%) as a pale yellow solid: mp 178-179°C; EI-HRMS m/e calcd for C₁₉H₂₄N₂O₃S₂ (M⁺) 392.1228, found 392.1230.

### Example 78

### 3-Cyclopentyl-2-(4-ethanesulfonyl-phenyl)-N-pyridin-2-yl-propionanude

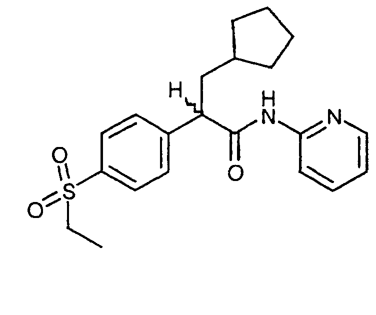

A mixture of aluminum chloride (72.35 g, 0.54 mol) in chloroform (181 mL) was cooled to 0°C and stirred until the solid material dissolved. The reaction mixture was then slowly treated with ethyl oxalyl chloride (61 mL, 0.54 mol), and the resulting reaction mixture was stirred at 0°C for 30 min. The reaction mixture was then slowly treated with ethyl phenyl sulfide (25.00 g, 0.18 mol). The solution turned to a wine color and slowly became gum-like. The resulting reaction mixture was then stirred at 0°C for 2 h. The reaction mixture was slowly poured into a large amount of ice/water. The resulting aqueous layer was extracted with chloroform (3 x 200 mL). The combined organic layers were dried over sodium sulfate, filtered, and concentrated *in vacuo*. Flash chromatography (Merck Silica gel 60, 70-230 mesh, 9/1 hexanes/ethyl acetate) afforded (4-ethylsulfanyl-phenyl)-oxo-acetic acid ethyl ester (23.64 g, 55%) as a yellow oil. The material was used without further purification and characterization in subsequent reactions.

A solution of iodomethylcyclopentane (4.60 g, 21.89 mmol) and triphenylphosphine (5.74 g, 21.89 mmol) in acetonitrile (22 mL) was heated under reflux for 2 weeks. The reaction mixture was allowed to cool to 25°C and then concentrated *in vacuo* to provide an orange solid. The orange solid was triturated with diethyl ether and then filtered. The solid was washed well with diethyl ether until the washings showed the absence of iodomethylcyclopentane and triphenylphosphine by thin layer chromatography. The solid was allowed to air dry to afford cyclopentylmethyl triphenylphosphonium iodide (8.92 g, 86%) as a light orange solid: mp 195-198°C; FAB-HRMS m/e calcd for C₂₄H₂₆P (M+H)⁺ 345.1772, found 345,1784.

A suspension of cyclopentylmethyl triphenylphosphonium iodide (24.48 g, 51.82 mmol) in dry tetrahydrofuran (100 mL) was cooled to 0°C and then treated dropwise with a 1.0M solution of sodium bis(trimethylsilyl)amide in tetrahydrofuran (52 mL, 51.82 mmol). The bright orange reaction mixture was stirred at 0°C for 1 h. The reaction mixture was then treated with (4-ethylsulfanyl-phenyl)-oxo-acetic acid ethyl ester (9.50 g, 39.87 mmol).

The resulting reaction mixture was allowed to warm to 25°C where it was stirred for 20 h. The reaction mixture was concentrated *in vacuo* to remove tetrahydrofuran and then diluted with water (300 mL). The aqueous layer was extracted with ethyl acetate (3 x 200 mL). The combined organic layers were washed with a saturated aqueous sodium chloride solution (1 x 200 mL), dried over sodium sulfate, filtered, and concentrated in *vacuo*. Flash chromatography (Merck Silica gel 60, 70-230 mesh, 19/1 hexanes/ethyl acetate) afforded the 3-cyclopentyl-2-(4-ethylsulfanyl-phenyl)-acrylic acid ethyl ester (6.08 g, 50%) as a yellow oil containing a 1.82:1 mixture of (E):(Z) isomers: FAB-LRMS m/e calcd for C₁₈H₂₄O₂S (M+H)⁺ integer mass 304, found 305.

A solution of 3-cyclopentyl-2-(4-ethylsulfanyl-phenyl)-acrylic acid ethyl ester [5.76 g, 18.92 mmol, (E):(Z) = 1.82:1] in methylene chloride (47 mL) was slowly treated with 3-chloroperoxybenzoic acid (57-86% grade, 11.45 g based on 57%, 37.83 mmol). The reaction mixture was stirred at 25°C for 1 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 200 mL), dried over sodium sulfate, filtered, and concentrated *in vacuo*. Flash chromatography (Merck Silica gel 60, 230-400 mesh, 3/1 hexanes/ethyl acetate) afforded 3-cyclopentyl-2-(4-ethanesulfonyl-phenyl)-acrylic acid ethyl ester (4.89 g, 77%) as a colorless oil. The product was a 3:1 mixture of (E):(Z) isomers that was used without further purification and characterization.

A solution of 3-cyclopentyl-2-(4-ethanesulfonyl-phenyl)-acrylic acid ethyl ester [4.89 g, 14.53 mmol, (E):(Z) = 3:1] in ethanol (36 mL) was slowly treated with 10% palladium on activated carbon (244.5 mg). The reaction mixture was stirred under a positive pressure of hydrogen gas (balloon) at 25°C and atmospheric pressure for 44 h. The catalyst was then filtered off through a pad of celite, and the celite pad was washed well with ethyl acetate. The filtrate was concentrated *in vacuo*. Flash chromatography (Merck Silica gel 60, 70-230 mesh, 3/1 hexanes/ethyl acetate) afforded 3-cyclopentyl-2-(4-ethanesulfonyl-phenyl)-propionic acid ethyl ester (3.50 g, 71%) as a colorless viscous oil: FAB-LRMS m/e calcd for C₁₈H₂₆O₄S (M+H)⁺ integer mass 338, found 339.

A solution of 3-cyclopentyl-2-(4-ethanesulfonyl-phenyl)-propionic acid ethyl ester (2.50 g, 7.39 mmol) in tetrahydrofuran (30 mL) was treated with a 0.8M aqueous lithium hydroxide solution (11.1 mL, 8.86 mmol). The reaction mixture was stirred at 25°C for 23 h. The resulting reaction mixture was partitioned between water (75 mL) and ethyl acetate (75 mL) and then treated with a 1N aqueous hydrochloric acid solution (15 mL). The layers were shaken and separated. The organic layer was dried over magnesium sulfate, filtered, and concentrated *in vacuo* to afford 3-cyclopentyl-2-(4-ethanesulfonyl-phenyl)-propionic acid (2.20 g, 96%) as a white solid which was used without further purification: mp 137-138°C; FAB-HRMS m/e calcd for C₁₆H₂₂O₄S (M+H)⁺ 311.1317, found 311.1321.

A solution of triphenylphosphine (279 mg, 1.06 mmol) in methylene chloride (5 mL) was cooled to 0°C and then slowly treated with *N*-bromosuccinimide (189 mg, 1.06 mmol). The reaction mixture was stirred at 0°C for 20 min and then treated with 3-cyclopentyl-2-(4-ethanesulfonyl-phenyl)-propionic acid (300 mg, 0.97 mmol). The resulting reaction mixture was stirred at 0°C for 10 min and then allowed to warm to 25°C where it was stirred for 30 min. The reaction mixture was then treated with 2-aminopyridine (200 mg, 2.13 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 3-cyclopentyl-2-(4-ethanesulfonyl-phenyl)-N-pyridin-2-yl-propionamide (185 mg, 50%) as a pale orange solid: mp 144-145°C; EI-HRMS m/e calcd for C₂₁H₂₆N₂O₃S (M⁺) 386.1664, found 386.1660.

### Example 79

### 2-(3,4-Bis-methanesulfonyl-phenyl)-3-cyclopentyl-N-thiazol-2-yl-propionamide

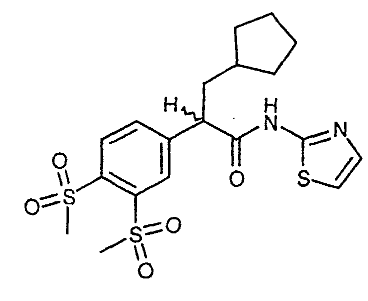

A solution of 3,4-difluorophenylacetic acid (5.00 g, 29.05 mmol) in methanol (73 mL) was slowly treated with concentrated sulfuric acid (4 mL). The resulting reaction mixture was heated under reflux for 65 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford (3,4-difluoro-phenyl)-acetic acid methyl ester (5.38 g, 99%), as a yellow oil which was used without further purification.

A solution of sodium thiomethoxide (6.39 g, 86.69 mmol) in dimethyl sulfoxide (72 mL) was treated with (3,4-difluoro-phenyl)-acetic acid methyl ester (5.38 g, 28.89 mmol). The reaction mixture was stirred at 25°C for 2 h then heated at 70°C for 15 min, at which time, thin layer chromatography indicated the absence of starting material and the presence of a very polar new product. The reaction indicated that the ester hydrolyzed to the acid upon heating. The resulting reaction mixture was allowed to cool to 25°C. The reaction mixture was then treated with a 10% aqueous hydrochloric acid solution (200 mL) and then extracted with chloroform (3 x 200 mL). The combined organic layers were dried over magnesium sulfate, filtered, and concentrated *in vacuo* to afford a yellow oil. This yellow oil was dissolved in methanol (100 mL) and then slowly treated with concentrated sulfuric acid (5 mL). The resulting reaction mixture was heated under reflux for 3 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford an inseparable, isomeric mixture of (3-fluoro-4-methylsulfanyl-phenyl)-acetic acid methyl ester and (4-fluoro-3-methylsulfanyl-phenyl)-acetic acid methyl ester as a yellow oil (4.65 g, 75%) which was used without further purification and characterization.

A solution of the inseparable, isomeric mixture of (3-fluoro-4-methylsulfanyl-phenyl)-acetic acid methyl ester and (4-fluoro-3-methylsulfanyl-phenyl)-acetic acid methyl ester (4.44 g, 20.72 mmol) in methylene chloride (103 mL) was slowly treated with 3-chloroperoxybenzoic acid (57-86% grade, 13.80 g based on 57%, 45.59 mmol). The reaction mixture was stirred at 25°C for 4 h. The reaction mixture was concentrated *in vacuo* to remove methylene chloride. The resulting residue was diluted with ethyl acetate (300 mL). The organic phase was washed with a saturated aqueous sodium bicarbonate solution (1 x 200 mL) and a saturated aqueous sodium chloride solution (1 x 200 mL), dried over magnesium sulfate, filtered, and concentrated *in vacuo*. Flash chromatography (Merck Silica gel 60, 70-230 mesh, 20/1 methylene chloride/ethyl acetate) afforded an inseparable, isomeric mixture of (3-fluoro-4-methanesulfonyl-phenyl)-acetic acid methyl ester and (4-fluoro-3-methanesulfonyl-phenyl)-acetic acid methyl ester as a colorless liquid (3.31 g, 65%) which was used without further purification and characterization.

A solution of the inseparable, isomeric mixture of (3-fluoro-4-methanesulfonyl-phenyl)-acetic acid methyl ester and (4-fluoro-3-methanesulfonyl-phenyl)-acetic acid methyl ester (2.28 g, 9.26 mmol) in dimethyl sulfoxide (23 mL) was treated with sodium thiomethoxide (1.37 g, 18.52 mmol). The reaction mixture was stirred at 25°C for 4 h and then quenched with a 10% aqueous hydrochloric acid solution. The aqueous layer was extracted with chloroform (1 x 400 mL), dried over magnesium sulfate, filtered, and concentrated *in vacuo*. Flash chromatography (Merck Silica gel 60, 70-230 mesh, 3/2 hexanes/ethyl acetate) afforded an inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester as a yellow liquid (2.19 g, 86%) which was used without further purification and characterization.

A solution of the inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester (2.19 g, 7.98 mmol) in methylene chloride (20 mL) was slowly treated with 3-chloroperoxybenzoic acid (57-86% grade, 6.41 g based on 57%, 31.93 mmol). The reaction mixture was stirred at 25°C for 5 h and then slowly quenched with a 1.5N aqueous sodium sulfite solution. The resulting reaction mixture was extracted with methylene chloride (300 mL). The organic phase was dried over magnesium sulfate, filtered, and concentrated *in vacuo*. Flash chromatography (Merck Silica gel 60, 70-230 mesh, 10/1 methylene chloride/ethyl acetate) afforded (3,4-bis-methanesulfonyl-phenyl)-acetic acid methyl ester (1.89 g, 77%) as a white solid: mp 157-158°C; EI-HRMS m/e calcd for C₁₁H₁₄O₆S₂ (M⁺) 306.0232, found 306.0234.

A solution of diisopropylamine (951 µL, 6.79 mmol) in dry tetrahydrofuran (6 mL) and 1,3-dimethyl-3,4,5,6-tetrahydro-2(1H)-pyrimidinone (2 mL) was cooled to -78°C under nitrogen and then treated with a 2.5M solution of *n*-butyllithium in hexanes (2.5 mL, 6.79 mmol). The resulting reaction mixture was stirred at -78°C for 30 min and then treated dropwise with a solution of (3,4-bis-methanesulfonyl-phenyl)-acetic acid methyl ester (1.89 g, 6.17 mmol) in dry tetrahydrofuran (12 mL) and 1,3-dimethyl-3,4,5,6-tetrahydro-2(1H)-pyrimidinone (4 mL). The resulting reaction mixture was allowed to stir at -78°C for 1 h, at which time, a solution of iodomethylcyclopentane (1.56 g, 7.40 mmol) in a small amount of dry tetrahydrofuran was added dropwise. The reaction mixture was allowed to warm to 25°C where it was stirred for 64 h. The reaction mixture was quenched with water (150 mL) and then concentrated *in vacuo* to remove tetrahydrofuran.

The remaining residue was further diluted with water (100 mL) and then extracted with ethyl acetate (1 x 250 mL). The organic layer was washed with a saturated aqueous sodium chloride solution (1 x 100 mL), dried over magnesium sulfate, filtered, and concentrated *in vacuo*. Flash chromatography (Merck Silica gel 60, 70-230 mesh, 3/1 hexanes/ethyl acetate) afforded 2-(3,4-bis-methanesulfonyl-phenyl)-3-cyclopentyl-propionic acid methyl ester (1.61 g, 67%) as a yellow oil: EI-HRMS m/e calcd for C₁₇H₂₄O₆S₂ (M⁺) 388.1014, found 388.1014.

A solution of 2-(3,4-bis-methanesulfonyl-phenyl)-3-eyclopentyl-propionic acid methyl ester (1.17 g, 3.01 mmol) in tetrahydrofuran (12 mL) was treated with a 0.8M aqueous lithium hydroxide solution (5.6 mL, 4.52 mmol). The reaction mixture was stirred at 25°C for 3 h. The resulting reaction mixture was partitioned between water (75 mL) and ethyl acetate (75 mL) and then treated with a 1N aqueous hydrochloric acid solution (10 mL). The layers were shaken and separated. The organic layer was dried over magnesium sulfate, filtered, and concentrated *in vacuo* to afford 2-(3,4-bis-methanesulfonyl-phenyl)-3-cyclopentyl-propionic acid (1.10 g, 98%) as a white foam which was used without further purification: mp 64-68°C (foam to gel); FAB-HRMS m/e calcd for C₁₆H₂₂O₆S₂ (M+H)⁺ 375.0936, found 375.0932.

A solution of triphenylphosphine (154 mg, 0.59 mmol) in methylene chloride (2 mL) was cooled to 0°C and then slowly treated with *N*-bromosuccinimide (105 mg, 0.59 mmol). The reaction mixture was stirred at 0°C for 10 min and then treated with 2-(3,4-bis-methanesulfonyl-phenyl)-3-cyclopentyl-propionic acid (200 mg, 0.53 mmol). The resulting reaction mixture was stirred at 0°C for 5 min and then allowed to warm to 25°C where it was stirred for 30 min. The reaction mixture was then treated with 2-aminothiazole (118 mg, 1.18 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3,4-bis-methanesulfonyl-phenyl)-3-cyclopentyl-N-thiazol-2-yl-propionamide (150 mg, 61%) as a pale yellow foam: mp 104-107°C; EI-HRMS m/e calcd for C₁₉H₂₄N₂O₅S₃ (M⁺) 456.0847, found 456.0846.

### Example 80

### 2-(3,4-Bis-methanesulfonyl-phenyl)-3-cyclopentyl-N-pyridin-2-yl-propionamide

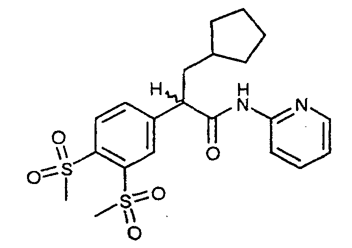

A solution of 3,4-difluorophenylacetic acid (5.00 g, 29.05 mmol) in methanol (73 mL) was slowly treated with concentrated sulfuric acid (4 mL). The resulting reaction mixture was heated under reflux for 65 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford (3,4-difluoro-phenyl)-acetic acid methyl ester (5.38 g, 99%) as a yellow oil which was used without further purification. A solution of sodium thiomethoxide (6.39 g, 86.69 mmol) in dimethyl sulfoxide (72 mL) was treated with (3,4-difluoro-phenyl)-acetic acid methyl ester (5.38 g, 28.89 mmol). The reaction mixture was stirred at 25°C for 2 h then heated at 70°C for 15 min, at which time, thin layer chromatography indicated the absence of starting material and the presence of a very polar new product. The reaction indicated that the ester hydrolyzed to the acid upon heating. The resulting reaction mixture was allowed to cool to 25°C. The reaction mixture was then treated with a 10% aqueous hydrochloric acid solution (200 mL) and then extracted with chloroform (3 x 200 mL). The combined organic layers were dried over magnesium sulfate, filtered, and concentrated *in vacuo* to afford a yellow oil. This yellow oil was dissolved in methanol (100 mL) and then slowly treated with concentrated sulfuric acid (5 mL). The resulting reaction mixture was heated under reflux for 3 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford an inseparable, isomeric mixture of (3-fluoro-4-methylsulfanyl-phenyl)-acetic acid methyl ester and (4-fluoro-3-methylsulfanyl-phenyl)-acetic acid methyl ester as a yellow oil (4.65 g, 75%) which was used without further purification and characterization.

A solution of the inseparable, isomeric mixture of (3-fluoro-4-methylsulfanyl-phenyl)-acetic acid methyl ester and (4-fluoro-3-methylsulfanyl-phenyl)-acetic acid methyl ester (4.44 g, 20.72 mmol) in methylene chloride (103 mL) was slowly treated with 3-chloroperoxybenzoic acid (57-86% grade, 13.80 g based on 57%, 45.59 mmol). The reaction mixture was stirred at 25°C for 4 h. The reaction mixture was concentrated *in vacuo* to remove methylene chloride. The resulting residue was diluted with ethyl acetate (300 mL). The organic phase was washed with a saturated aqueous sodium bicarbonate solution (1 x 200 mL) and a saturated aqueous sodium chloride solution (1 x 200 mL), dried over magnesium sulfate, filtered, and concentrated *in vacuo*. Flash chromatography (Merck Silica gel 60, 70-230 mesh, 20/1 methylene chloride/ethyl acetate) afforded an inseparable, isomeric mixture of (3-fluoro-4-methanesulfonyl-phenyl)-acetic acid methyl ester and (4-fluoro-3-methanesulfonyl-phenyl)-acetic acid methyl ester as a colorless liquid (3.31 g, 65%) which was used without further purification and characterization.

A solution of the inseparable, isomeric mixture of (3-fluoro-4-methanesulfonyl-phenyl)-acetic acid methyl ester and (4-fluoro-3-methanesulfonyl-phenyl)-acetic acid methyl ester (2.28 g, 9.26 mmol) in dimethyl sulfoxide (23 mL) was treated with sodium thiomethoxide (1.37 g, 18.52 mmol). The resulting reaction mixture was stirred at 25°C for 4 h and then quenched with a 10% aqueous hydrochloric acid solution. The aqueous layer was extracted with chloroform (1 x 400 mL), dried over magnesium sulfate, filtered, and concentrated *in vacuo*. Flash chromatography (Merck Silica gel 60, 70-230 mesh, 3/2 hexanes/ethyl acetate) afforded an inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester as a yellow liquid (2.19 g, 86%) which was used without further purification and characterization.

A solution of the inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester (2.19 g, 7.98 mmol) in methylene chloride (20 mL) was slowly treated with 3-chloroperoxybenzoic acid (57-86% grade, 6.41 g based on 57%, 31.93 mmol). The reaction mixture was stirred at 25°C for 5 h and then slowly quenched with a 1.5N aqueous sodium sulfite solution. The resulting reaction mixture was extracted with methylene chloride (300 mL). The organic phase was dried over magnesium sulfate, filtered, and concentrated *in vacuo*. Flash chromatography (Merck Silica gel 60, 70-230 mesh, 10/1 methylene chloride/ethyl acetate) afforded (3,4-bis-methanesulfonyl-phenyl)-acetic acid methyl ester (1.89 g, 77%) as a white solid: mp 157-158°C; EI-HRMS m/e calcd for C₁₁H₁₄O₆S₂ (M⁺) 306.0232, found 306.0234.

A solution of diisopropylamine (951 µL, 6.79 mmol) in dry tetrahydrofuran (6 mL) and 1,3-dimethyl-3,4,5,6-tetrahydro-2(1H)-pyrimidinone (2 mL) was cooled to -78°C under nitrogen and then treated with a 2.5M solution of *n*-butyllithium in hexanes (2.5 mL, 6.79 mmol). The resulting reaction mixture was stirred at -78°C for 30 min and then treated dropwise with a solution of (3,4-bis-methanesulfonyl-phenyl)-acetic acid methyl ester (1.89 g, 6.17 mmol) in dry tetrahydrofuran (12 mL) and 1,3-dimethyl-3,4,5,6-tetrahydro-2(1H)-pyrimidinone (4 mL). The resulting reaction mixture was allowed to stir at -78°C for 1 h, at which time, a solution of iodomethylcyclopentane (1.56 g, 7.40 mmol) in a small amount of dry tetrahydrofuran was added dropwise. The reaction mixture was allowed to warm to 25°C where it was stirred for 64 h. The reaction mixture was quenched with water (150 mL) and then concentrated *in vacuo* to remove tetrahydrofuran. The remaining residue was further diluted with water (100 mL) and then extracted with ethyl acetate (1 x 250 mL). The organic layer was washed with a saturated aqueous sodium chloride solution (1 x 100 mL), dried over magnesium sulfate, filtered, and concentrated *in vacuo.* Flash chromatography (Merck Silica gel 60, 70-230 mesh, 3/1 hexanes/ethyl acetate) afforded 2-(3,4-bis-methanesulfonyl-phenyl)-3-cyclopentyl-propionic acid methyl ester (1.61 g, 67%) as a yellow oil: EI-HRMS m/e calcd for C₁₇H₂₄O₆S₂ (M⁺) 388.1014, found 388.1014.

A solution of 2-(3,4-bis-methanesulfonyl-phenyl)-3-cyclopentyl-propionic acid methyl ester (1.17 g, 3.01 mmol) in tetrahydrofuran (12 mL) was treated with a 0.8M aqueous lithium hydroxide solution (5.6 mL, 4.52 mmol). The reaction mixture was stirred at 25°C for 3 h. The resulting reaction mixture was partitioned between water (75 mL) and ethyl acetate (75 mL) and then treated with a 1N aqueous hydrochloric acid solution (10 mL). The layers were shaken and separated. The organic layer was dried over magnesium sulfate, filtered; and concentrated *in vacuo* to afford 2-(3,4-bis-methanesulfonyl-phenyl)-3-cyclopentyl-propionic acid (1.10 g, 98%) as a white foam which was used without further purification: mp 64-68°C (foam to gel); FAB-HRMS m/e calcd for C₁₆H₂₂O₆S₂ (M+H)⁺ 375.0936, found 375.0932.

A solution of triphenylphosphine (154 mg, 0.59 mmol) in methylene chloride (2 mL) was cooled to 0°C and then slowly treated with *N*-bromosuccinimide (105 mg, 0.59 mmol). The reaction mixture was stirred at 0°C for 10 min and then treated with 2-(3,4-bis-methanesulfonyl-phenyl)-3-cyclopentyl-propionic acid (200 mg, 0.53 mmol). The resulting reaction mixture was stirred at 0°C for 5 min and then allowed to warm to 25°C where it was stirred for 30 min. The reaction mixture was then treated with 2-aminopyridine (110 mg, 1.18 mmol). The resulting reaction mixture was stirred at 25°C for 15 h. The crude reaction mixture was then directly purified by flash chromatography (Merck Silica gel 60, 230-400 mesh, 1/1 hexanes/ethyl acetate) to afford 2-(3,4-bis-methanesulfonyl-phenyl)-3-cyclopentyl-N-pyridin-2-yl-propionamide (117 mg, 49%) as a pale yellow foam: mp 107-110°C; EI-HRMS m/e calcd for C₂₁H₂₆N₂O₅S₂ (M⁺) 450.1283, found 450.1282.

### Example 81

### 3-Cyclopentyl-2-(3,4-dichlorophenyl)-N-[1,2,4]triazin-3-yl-propionamide

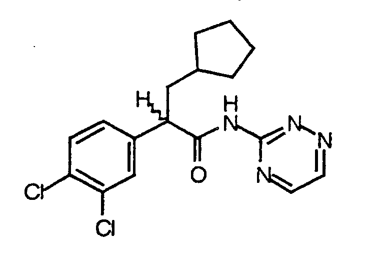

A solution of 3-cyclopentyl-2-(3,4-dichlorophenyl)-propionic acid (prepared in Example 38, 400 mg, 1.40 mmol) in dry pyridine (5 mL) was treated with 1,3-dicyclohexylcarbodiimide (316 mg, 1.53 mmol). The reaction mixture was stirred at 25°C for 3.5 h and then treated with 3-amino-1,2,4-triazine (296 mg, 3.08 mmol) and an additional amount of pyridine (1 mL). The reaction mixture was warmed at 100°C for 20 h. The reaction mixture was concentrated *in vacuo* to remove pyridine. The resulting residue was diluted with ethyl acetate then filtered. The filtrate was washed with a 1N aqueous hydrochloric acid solution and washed with water. The organic layer was dried over magnesium sulfate, filtered, and concentrated *in vacuo*. Flash chromatography (Merck Silica gel 60, 230-400 mesh, 1/1 ethyl acetate/hexanes) afforded 3-cyclopentyl-2-(3,4-dichlorophenyl)-N-[1,2,4]triazin-3-yl-propionamide (40.9 mg, 8%) as a yellow-orange solid: mp 81-83°C; EI-HRMS m/e calcd for C₁₇H₁₈Cl₂N₄O (M⁺) 364.0858, found 364.0857.

### Example 82

### 3-Cyclopentyl-2-(4-sulfamoyl-phenyl)-N-thiazol-2-yl-propionamide

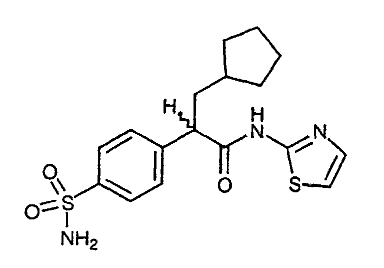

A solution of diisopropylamine (3.3 mL, 23.5 mmol) in dry tetrahydrofuran (50 mL) and 1,3-dimethyl-3,4,5,6-tetrahydro-2(1H)-pyrimidinone (10 mL) was cooled to -78°C under nitrogen and then treated with a 10M solution of n-butyllithium in hexanes (2.35 mL, 23.5 mmol). The yellow reaction mixture was stirred at -78°C for 30 min and then treated dropwise with a solution of 4-methylsulfonylphenylacetic acid (2.40 g, 11.2 mmol) in a small amount of dry tetrahydrofuran. After approximately one-half of the 4-methylsulfonylphenylacetic acid in dry tetrahydrofuran was added, a precipitate formed. Upon further addition of the remaining 4-methylsulfonylphenylacetic acid in dry tetrahydrofuran, the reaction mixture became thick in nature. After complete addition of the 4-methylsulfonylphenylacetic acid in dry tetrahydrofuran, the reaction mixture was very thick and became difficult to stir. An additional amount of dry tetrahydrofuran (20 mL) was added to the thick reaction mixture, and the reaction mixture was stirred at - 78°C for 45 min, at which time, a solution of iodomethylcyclopentane (2.35 g, 11.2 mmol) in a small amount of dry tetrahydrofuran was added dropwise. The reaction mixture was allowed to warm to 25°C where it was stirred for 15 h. The reaction mixture was quenched with water (100 mL), and the resulting yellow reaction mixture was concentrated *in vacuo* to remove tetrahydrofuran. The aqueous residue was acidified to pH = 2 using concentrated hydrochloric acid. The aqueous layer was extracted with ethyl acetate. The organic phase was dried over magnesium sulfate, filtered, and concentrated *in vacuo*. Flash chromatography (Merck Silica gel 60, 230-400 mesh, 1/3 hexanes/ ethyl acetate) afforded 3-cyclopentyl-2-(4-methanesulfonyl-phenyl)propionic acid (1.80 g, 52%) as a white solid: mp 152-154°C; EI-HRMS m/e calcd for C₁₅H₂₀O₄S (M⁺) 296.1082, found 296.1080.

A solution of 3-cyclopentyl-2-(4-methanesulfonyl-phenyl)propionic acid (4.91 g, 16.56 mmol) and triphenylphosphine (6.52 g, 24.85 mmol) in methylene chloride (41 mL) was cooled to 0°C and then treated with *N*-bromosuccinimide (5.01 g, 28.16 mmol) in small portions. The reaction mixture color changed from light yellow to a darker yellow then to brown. After the complete addition of *N*-bromosuccinimide, the reaction mixture was allowed to warm to 25°C over 30 min. The brown reaction mixture was then treated with 2-aminothiazole (4.98 g, 49.69 mmol). The resulting reaction mixture was stirred at 25°C for 19 h. The reaction mixture was then concentrated *in vacuo* to remove methylene chloride. The remaining black residue was diluted with a 10% aqueous hydrochloric acid solution (400 mL) and then extracted with ethyl acetate (3 x 200 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 1/3 hexanes/ethyl acetate then 1/1 hexanes/ethyl acetate) afforded 3-cyclopentyl-2-(4-methanesulfonyl-phenyl)-N-thiazol-2-yl-propionamide (4.49 g, 72%) as a white solid: mp 216-217°C; EI-HRMS m/e calcd for C₁₈H₂₂N₂O₃S₂ (M⁺) 378.1072, found 378.1071.

A solution of diisopropylamine (559 µL, 3.99 mmol) in dry tetiahydrofuran (1.2 mL) was cooled to -78°C under nitrogen and then treated with a 2.5M solution of n-butyllithium in hexanes (1.6 mL, 3.99 mmol). The resulting reaction mixture was allowed to warm to 0°C and then was treated with 3-cyclopentyl-2-(4-methanesulfonyl-phenyl)-N-thiazol-2-yl-propionamide (463.1 mg, 1.22 mmol) in small portions. The reaction mixture turned orange in color. The reaction mixture was then allowed to warm to 25°C where it was stirred for 30 min. After 30 min at 25°C, the reaction mixture was cooled back down to 0°C and then treated with a 1M solution of tributylborane in tetrahydrofuran (1.8 mL, 1.84 mmol). The resulting reaction mixture was stirred at 0°C for 10 min then allowed to warm to 25°C. The reaction mixture was stirred at 25°C for 30 min then heated under reflux for 20 h. The reaction mixture was cooled to 0°C and then treated with water (3 mL) followed by sodium acetate (702.5 mg, 8.56 mmol) and then finally hydroxyamine-*O*-sulfonic acid (484.2 mg, 4.28 mmol). The resulting reaction mixture was stirred at 0°C for 30 min then allowed to warm to 25°C where it was stirred for 44 h. The reaction mixture was concentrated *in vacuo* to remove tetrahydrofuran. The resulting aqueous residue was diluted with ethyl acetate (150 mL). The organic layer was washed with a saturated aqueous sodium bicarbonate solution (1 x 100 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/2 hexanes/ethyl acetate) afforded 3-cyclopentyl-2-(4-sulfamoyl-phenyl)-N-thiazol-2-yl-propionamide (191.8 mg, 72%) as a white solid: mp 179-181°C; EI-HRMS m/e calcd for C₁₇H₂₁N₃O₂S₂ (M⁺) 379.1024, found 379.1029.

### Example 85

### 3-Cyelopentyl-2-(3,4-dichiorophenyl)-N-[1,3,4]thiadiazol-2-yl-propianamide

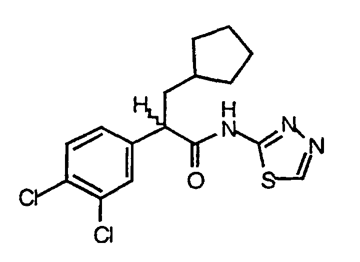

A solution of 3-cyclopentyl-2-(3,4-dichlorophenyl)-propionic acid (prepared from Example **38**, 200.0 mg, 0.70 mmol), O-benzotriazol-1-yl-N,N,N',N'-tetramethyluronium hexafluorophosphate (316.9 mg, 0.84 mmol), *N,N*-diisopropylethylamine (365 mL, 2.09 mmol), and 2-amino-1,3,4-thiadiazole (140.8 mg, 1.39 mmol) in dry *N,N*-dimethylformamide (2 mL) was stirred at 25°C under nitrogen for 20 h. The reaction mixture was concentrated *in vacuo* to remove *N,N*-dimethylformamide. The resulting residue was diluted with ethyl acetate (100 mL). The organic layer was washed with a saturated aqueous sodium bicarbonate solution (1 x 50 mL), a 10% aqueous hydrochloric acid solution (1 x 100 mL), and a saturated aqueous sodium chloride solution (1 x 100 mL). The organic layer was dried over sodium sulfate, filtered, and concentrated *in vacuo*. Flash chromatography (Merck Silica gel 60, 230-400 mesh, 2/1 hexanes/ethyl acetate) afforded 3-cyclopentyl-2-(3,4-dichlorophenyl)-N-[1,3,4]thiadiazol-2-yl-propionamide (197.3 mg, 77%) as a white foam: mp 90-91°C; EI-HRMS m/e calcd for C₁₆H₁₇Cl₂N₃OS (M⁺) 369.0469, found 369.0476.

### Example 86

### (A) 2-(4-Cyano-phenyl)-3-cyclopentyl-N-thiazol-2-yl-propionamide

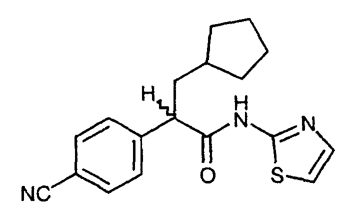

A solution of freshly prepared lithium diisopropylamide (23 mL of a 0.32M stock solution, 7.13 mmol) cooled to -78°C was treated with (4-bromo-phenyl)-acetic acid methyl ester (1.48 g, 6.48 mmol) in tetrahydrofuran/1,3-dimethyl-3,4,5,6-tetrahydro-2(1H)-pyrimidinone (16.2 mL, 3:1). The resulting solution was stirred at -78°C for 45 min. Iodomethylcyclopentane (1.49 g, 7.13 mmol) was then added in 1,3-dimethyl-3,4,5,6-tetrahydro-2(1H)-pyrimidinone (2 mL). The reaction mixture was stirred at -78°C for 4 h. The reaction was then warmed to 25°C and was stirred at 25°C for 18 h. The reaction mixture was then quenched by the dropwise addition of a saturated aqueous ammonium chloride solution (10 mL). This mixture was poured into water (100 mL) and extracted with ethyl acetate (3 x 50 mL). The organics were washed with a saturated aqueous lithium chloride solution (1 x 50 mL), dried over sodium sulfate, filtered, and *concentrated in vacuo*. Flash chromatography (Merck Silica gel 60, 230-400 mesh, 95/5 hexanes/ethyl acetate) afforded 2-(4-bromo-phenyl)-3-cyclopentyl-propionic acid methyl ester (1.60 g, 79.3%) as a clear oil: EI-HRMS m/e calcd for C₁₅H₁₉O₂Br (M⁺) 310.0568 found 310.0564.

A solution of 2-(4-bromo-phenyl)-3-cyclopentyl-propionic acid methyl ester (500 mg, 1.60 mmol) in *N,N*-dimethylformamide (4.01 mL) was treated with copper(I) cyanide (144 mg, 1.60 mmol). The mixture was heated at 170°C for 1 h. At this time, the reaction was cooled to 25°C and poured into aqueous ammonium hydroxide (5 mL). The solution was diluted with water (25 mL) and extracted with ethyl acetate (3 x 35 mL). The organics were dried over sodium sulfate, filtered, and concentrated *in vacuo*. Flash chromatography (Merck Silica gel 60, 230-400 mesh, 90/10 hexanes/ethyl acetate) afforded 2-(4-cyano-phenyl)-3-cyclopentyl-propionic acid methyl ester (65.6 g, 15.8%) as a clear oil: EI-HRMS m/e calcd for C₁₆H₁₉NO₂ (M⁺) 257.1415 found 257.1406.

A solution of 2-(4-cyano-phenyl)-3-cyclopentyl-propionic acid methyl ester (65.0 mg, 0.25 mmol) in tetrahydrofuran/water/methanol (2.5 mL, 3:1:1) was treated with a 1N aqueous lithium hydroxide solution (0.27 mL; 0.27 mmol). The reaction was stirred at 25°C for 6 h. At this time, the reaction was acidified to pH = 1 with a 1N aqueous hydrochloric acid solution and extracted with chloroform/methanol (9:1, 3 x 25 mL). The organics were dried over sodium sulfate, filtered, and concentrated *in vacuo*. Flash chromatography (Merck Silica gel 60, 230-400 mesh, 80/20 hexanes/ethyl acetate) afforded 2-(4-cyano-phenyl)-3-cyclopentyl-propionic acid (36.0 mg, 58.6%) as a white solid: mp 126-128°C; EI-HRMS m/e calcd for C₁₅H₁₇NO₂ (M⁺) 243.1259 found 243.1268.

A solution of 2-(4-cyano-phenyl)-3-cyclopentyl-propionic acid (33.0 mg, 0.13 mmol) in methylene chloride (1.36 mL) was cooled to 0°C and then treated with a 2.0M solution of oxalyl chloride in methylene chloride (0.07 mL, 0.14 mmol) and a few drops of *N,N*-dimethylformamide. The reaction mixture was stirred at 0°C for 10 min and at 25°C for 30 min. The reaction mixture was then treated with a solution of 2-aminothiazole (30.0 mg, 0.29 mmol) and *N,N*-diisopropylethylamine (0.05 mL, 0.32 mmol) in tetrahydrofuran (0.67 mL). This solution was stirred at 25°C for 3 h. At this time, the reaction was concentrated *in vacuo*. Flash chromatography (Merck Silica gel 60, 230-400 mesh, 50/50 hexanes/ethyl acetate) afforded 2-(4-cyano-phenyl)-3-cyclopentyl-N-thiazol-2-yl-propionamide (44.1 mg, 100%) as a white solid: mp 64-66°C; EI-HRMS m/e calcd for C₁₈H₁₉N₃OS (M⁺) 325.1248 found 325.1247.

### (B) In an analogous manner, there was obtained:

(a) From 2-aminopyridine and 2-(4-cyano-phenyl)-3-cyclopentyl-propionic acid: 2-(4-Cyano-phenyl)-3-cyclopentyl-N-pyridin-2-yl-propionamide as a white solid: mp 61-63°C; EI-HRMS m/e calcd for C₂₀H₂₁N₃O (M⁺) 319.1684, found 319.1697.
(b) From 2-(4-cyano-phenyl)-3-cyclopentyl-propionic acid and 6-amino-nicotinic acid methyl ester: 6-[2-(4-Cyano-phenyl)-3-cyclopentyl-propionylamino]-nicotinic acid methyl ester as a white solid: mp 62-64°C; EI-HRMS m/e calcd for C₂₂H₂₃N₃O₃ (M⁺) 377.1739, found 377.1736.

### Example 87

### (A) 3-Cyclopentyl-N-pyridin-2-yl-2-(4-trifluoromethyl-phenyl)-propionamide

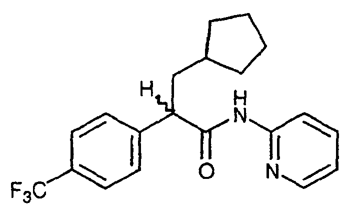

A solution of freshly prepared lithium diisopropylamide (23 mL of a 0.31M stock solution, 7.13 mmol) cooled to -78°C was treated with (4-trifluoromethyl)phenylacetic acid (693 mg, 3.4 mmol) in tetrahydrofuran/ hexamethylphosphoramide (8.5 mL, 3:1). The resulting solution was stirred at -78°C for 30 min. Iodomethylcyclopentane (784 mg, 3.7 mmol) was then added in hexamethylphosphoramide (1 mL). The reaction mixture was stirred at -78°C for 4 h. The reaction was then warmed to 25°C and was stirred at 25°C for 16 h. The reaction mixture was then quenched by the dropwise addition of saturated aqueous ammonium chloride solution (10 mL). The excess solvent was removed *in vacuo*. The residue was acidified to pH = 1 with a 1N aqueous hydrochloric acid solution. The mixture was poured into water (150 mL) and extracted with ethyl acetate (3 x 100 mL). The organics were dried over sodium sulfate, filtered, and concentrated *in vacuo*. Flash chromatography (Merck Silica gel 60, 230-400 mesh, 95/5 hexanes/ethyl acetate) afforded 3-cyclopentyl-2-(4-trifluoromethyl-phenyl)-propionic acid (634.9 mg, 65%) as a white solid: mp 94-95°C; FAB-HRMS m/e calcd for C₁₅H₁₇F₃O₂ (M+Na)⁺ 309.1079, found 309.1072.

A solution of benzotriazol-1-yloxy-tris(dimethylamino)phosphonium hexafluorophosphate (170 mg, 0.38 mmol), 3-cyclopentyl-2-(4-trifluoromethyl-phenyl)-propionic acid (100 mg, 0.34 mmol), and 2-aminopyridine (36 mg, 0.38 mmol) in *N*,*N*-dimethylformamide (1.75 mL) was treated with *N*,*N*-diisopropylethylamine (0.12 mL, 0.73 mmol). The reaction mixture was stirred at 25°C for 18 h. At this time, the reaction was poured into water (50 mL) and extracted with ethyl acetate (3 x 50 mL). The combined organic layers were washed with a 1N aqueous hydrochloric acid solution (1 x 50 mL), dried over sodium sulfate, filtered, and concentrated *in vacuo*. Flash chromatography (Merck Silica gel 60, 230-400 mesh, 90/10 hexanes/ethyl acetate) afforded 3-cyclopentyl-N-pyridin-2-yl-2-(4-trifluoromethyl-phenyl)-propionamide (127 mg, 53.3%) as a white gum: EI-HRMS m/e calcd for C₂₀H₂₁F₃N₂O (M⁺) 362.1605, found 362.1592.

### (B) In an analogous manner, there was obtained:

(a) From 6-amino-nicotinic acid methyl ester and 3-cyclopentyl-2-(4-trifluoromethyl-phenyl)-propionic: 6-[3-Cyclopentyl-2-(4-trifluoromethyl-phenyl)-propionylamino]-nicotinic acid methyl ester as a white gum: EI-HRMS m/e calcd for C₂₂H₂₃F₃N₂O₃ (M⁺) 420.1660, found 420.1661.

### Example 88

### (A) 2-[4-(Butane-1-sulfonyl)-phenyl]-3-cyclopentyl-N-thiazol-2-yl-propionamide

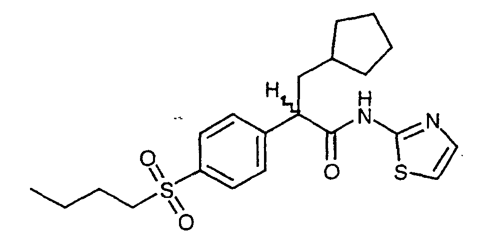

A solution of freshly prepared lithium diisopropylamide (430.55 mL of a 0.3M stock solution, 129.16 mmol) cooled to -78°C was treated with (4-nitro-phenyl)-acetic acid ethyl ester (prepared in Example **22**, 26.32 g, 125.83 mmol) in tetrahydrofuran/hexamethylphosphoramide (312.5 mL, 3:1). The resuming solution was stirred at -78°C for 45 min. Iodomethylcyclopentane (27.75 g, 132.1 mmol) was then added in hexamethylphosphoramide (27.75 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (250 mL). This mixture was concentrated *in vacuo*. The residue was diluted with water (250 mL) and extracted with ethyl acetate (3 x 300 mL). The organics were washed with a saturated aqueous lithium chloride solution (2 x 250 mL), dried over magnesium sulfate, filtered, and concentrated *in vacuo*. Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as a yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (7.37 g, 25.3 mmol) in ethyl acetate (316 mL) was treated with 10% palladium on activated carbon.

The reaction mixture was stirred under hydrogen gas at 60 psi at 25°C for 18 h. The catalyst was then filtered off through a pad of celite (ethyl acetate). The filtrate was concentrated *in vacuo*. Flash chromatography (Merck Silica gel 60, 230-400 mesh, 50/50 hexanes/ethyl acetate) afforded 2-(4-amino-phenyl)-3-cyclopentyl-propionic acid ethyl ester (3.52 mg, 53.3%) as a yellow oil: EI-HRMS m/e calcd for C₁₆H₂₃NO₂ (M⁺) 261.1729 found 261.1727.

A mixture of concentrated hydrochloric acid (0.38 mL) and ice (380 mg) cooled to 0°C was treated with 2-(4-amino-phenyl)-3-cyclopentyl-propionic acid ethyl ester (497 mg, 1.90 mmol). After 5 min, a solution of sodium nitrite (139 mg, 2.01 mmol) in water (0.31 mL) was added to the reaction mixture. The resulting solution was stirred at 0°C for 5 min. At this time, the solution was added to a solution of n-butyl mercaptan (0.23 mL, 2.20 mmol) in water (0.41 mL) warmed to 45°C. The reaction was stirred at 45°C for 3 h. At this time, the reaction was diluted with water (50 mL) and extracted with chloroform (3 x 50 mL). The organics were dried over sodium sulfate, filtered, and concentrated *in vacuo*. The crude brown oil (588 mg) in methylene chloride (8.8 mL) was cooled to 0°C and treated with 3-chloroperoxybenzoic acid (80-85% grade, 1.5 g, 8.78 mmol). The reaction mixture was stirred at 25°C for 18 h. At this time, the reaction was diluted with water (75 mL) and extracted with chloroform (2 x 30 mL). The organics were dried over sodium sulfate, filtered, and concentrated *in vacuo*. Flash chromatography (Merck Silica gel 60, 230-400 mesh, 80/20 hexanes/ethyl acetate) afforded 2-[4-(butane-1-sulfonyl)-phenyl]-3-cyclopentyl-propionic acid ethyl ester (144.3 mg, 20.7%) as a yellow oil: EI-HRMS m/e calcd for C₂₀H₃₀O₄S (M⁺) 366.1865 found 366.1858.

A solution of 2-[4-(butane-1-sulfonyl)-phenyl]-3-cyclopentyl-propionic acid ethyl ester (140 mg, 0.38 mmol in tetrahydrofuran/water/methanol (0.95 mL, 3:1:1) was treated with a 1N aqueous lithium hydroxide solution (0.76 mL, 0.76 mmol). The reaction was stirred at 25°C for 8 h. At this time, the reaction was acidified to pH = 1 with a 1N aqueous hydrochloric acid solution and extracted with chloroform (3 x 50 mL). The organics were dried over sodium sulfate, filtered, and concentrated *in vacuo*. Flash chromatography (Merck Silica gel 60, 230-400 mesh, 90/10 chloroform/methanol) afforded 2-[4-(butane-1-sulfonyl)-phenyl]-3-cyclopentyl-propionic acid (88.3 mg, 68.4%) as a clear oil: FAB-HRMS m/e calcd for C₁₈H₂₆O₄S (M+H)⁺ 339.1631 found 339.1638.

A solution of triphenylphosphine (99 mg, 0.37 mmol) and *N*-bromosuccinimide (76 mg, 0.42 mmol) in methylene chloride (1.26 mL) cooled to 0°C was treated with 2-[4-(butane-1-sulfonyl)-phenyl]-3-cyclopentyl-propionic acid (85 mg, 0.25 mmol) in methylene chloride. The reaction mixture was stirred at 25°C for 45 min. At this time, the reaction was treated with 2-aminothiazole (33 mg, 0.32 mmol) and pyridine (0.03 mL, 0.37 mmol). The reaction was stirred at 25°C for 18 h. The reaction mixture was then concentrated *in vacuo* to remove methylene chloride. At this time, the reaction was diluted with water (50 mL) and extracted with chloroform (3 x 50 mL). The organics were dried over sodium sulfate, filtered, and concentrated *in vacuo*. Flash chromatography (Merck Silica gel 60, 70-230 mesh, 50/50 hexanes/ethyl acetate) afforded 2-[4-(butane-1-sulfonyl)-phenyl]-3-cyclopentyl-N-thiazol-2-yl-propionamide (69.3 mg, 65.6%) as an off-white solid: mp 163-165°C; EI-HRMS m/e calcd for C₂₁H₂₈N₂O₃S₂ (M⁺) 420.1541 found 420.1535.

### (B) In an analogous manner, there was obtained:

(a) From 2-aminothiazole and 3-cyclopentyl-2-[4-(propane-1-sulfonyl)-phenyl]-propionic acid: 3-Cyclopentyl-2-[4-(propane-1-sulfonyl)-phenyl]-N-thiazol-2-yl-propionamide as a yellow oil: EI-HRMS m/e calcd for C₂₀H₂₆N₂O₃S₂ (M⁺) 406.1385 found 406.1389.

### Example 89

### (A) 3-Cyclopentyl-2-(4-fluoro-3-trifluoromethyl-phenyl)-N-thiazol-2-yl-propionamide

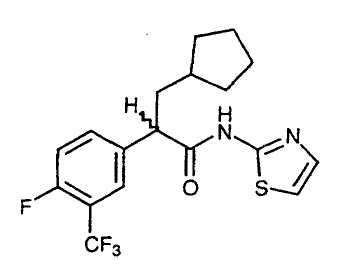

A solution of freshly prepared lithium diisopropylamide (35.32 mL of a 0.31M stock solution, 10.95 mmol) cooled to -78°C was treated with (4-fluoro-3-trifluoromethyl-phenyl)-acetic acid (1.11 g, 5.0 mmol) in tetrahydrofuran/1,3-dimethyl-3,4,5,6-tetrahydro-2(1H)-pyrimidinone (12.42 mL, 3:1). The resulting solution was stirred at -78°C for 1 h. Iodomethylcyclopentane (1.16 g, 5.52 mmol) was then added in 1,3-dimethyl-3,4,5,6-tetrahydro-2(1H)-pyrimidinone (1.2 mL). The reaction mixture was stirred at -78°C for 4 h. The reaction was then warmed to 25°C and was stirred at 25°C for 24 h. This solution was then quenched by the slow addition of the reaction mixture to a 2N aqueous hydrochloric acid solution (50 mL). The product was extracted into ethyl acetate (1 x 300 mL) and diethyl ether (1 x 50 mL). The organics were dried over sodium sulfate, filtered, and *concentrated in vacuo*. Flash chromatography (Merck Silica gel 60, 230-400 mesh, 50/50 hexanes/ethyl acetate) afforded 3-cyclopentyl-2-(4-fluoro-3-trifluoromethyl-phenyl)-propionic acid (1.28 g, 84.3%) as a white solid: mp 65-68°C; EI-HRMS m/e calcd for C₁₅H₁₆F₄O₂ (M⁺) 305.1165, found 305.1174.

A solution of 3-cyclopentyl-2-(4-fluoro-3-trifluoromethyl-phenyl)-propionic acid (304 mg, 1.0 mmol) in methylene chloride (10 mL) was cooled to 0°C and then treated with a 2.0M solution of oxalyl chloride in methylene chloride (0.6 mL, 1.2 mmol) and a few drops of *N,N*-dimethylformamide. The reaction mixture was stirred at 0°C for 15 min and at 25°C for 24 h. The reaction mixture was then treated with 2-amino-thiazole (175 mg, 1.75 mmol) and *N,N*-diisopropylethylamine (0.4 mL, 2.41 mmol). This solution was stirred at 25°C for 48 h. At this time, the reaction was concentrated *in vacuo*. High pressure liquid chromatography (Chromegasphere SI-60, 10 µM, 60Å, 25cm x 23cm ID, 60/40 heptane/ethyl acetate) afforded 3-cyclopentyl-2-(4-fluoro-3-trifluoromethyl-phenyl)-N-thiazol-2-yl-propionamide (326 mg, 84.5%) as a light yellow solid: mp 125-127°C; EI-HRMS m/e calcd for C₁₈H₁₈F₄N₂OS (M⁺) 386.1076, found 386.1086.

### (B) In an analogous manner, there was obtained:

(a) From ethyl 2-amino-4-thiazole glyoxylate and 3-cyclopentyl-2-(4-fluoro-3-trifluoromethyl-phenyl)-propionic acid: {2-[3-Cyclopentyl-2-(4-fluoro-3-trifluoromethyl-phenyl)-propionylamino]-thiazol-4-yl}-oxo-acetic acid ethyl ester as a light yellow solid: mp 155-158°C; FAB-HRMS m/e calcd for C₂₂H₂₂F₄N₂O₄S (M+H)⁺ 487.1314, found 487.1319.
(b) From 5-methyl-2-aminopyridine and 3-cyclopentyl-2-(4-fluoro-3-trifluoromethyl-phenyl)-propionic acid: 3-Cyclopentyl-2-(4-fluoro-3-trifluoromethyl-phenyl)-N-(5-methyl-pyridin-2-yl)-propionamide as a white solid: mp 132-133°C; EI-HRMS m/e calcd for C₂₁H₂₂F₄N₂O (M⁺) 392.1668, found 392.1669.
(c) From 2-aminopyridine and 3-cyclopentyl-2-(4-fluoro-3-trifluoromethyl-phenyl)-propionic acid: 3-Cyclopentyl-2-(4-fluoro-3-trifluoromethyl-phenyl)-N-pyridin-2-yl-propionamide as a light yellow oil: EI-HRMS m/e calcd for C₂₀H₂₀F₄N₂O (M⁺) 380.1511, found 380.1521.

### Example 90

### 3-Cyclopentyl-N-thiazol-2-yl-2-(3-trifluoromethyl-phenyl)-propionamide

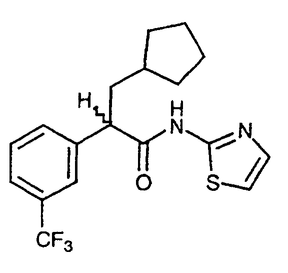

A solution of freshly prepared lithium diisopropylamide (35.32 mL of a 0.31M stock solution, 10.9 mmol) cooled to -78°C was treated with (3-trifluoromethyl-phenyl)-acetic acid (1.02 g, 5.0 mmol) in tetrahydrofuran/1,3-dimethyl-3,4,5,6-tetrahydro-2(1H)-pyrimidinone (12.4 mL, 3:1). The resulting solution was stirred at -78°C for 3 h. Iodomethylcyclopentane (1.16 g, 5.52 mmol) was then added in 1,3-dimethyl-3,4,5,6-tetrahydro-2(1H)-pyrimidinone (1.16 mL). The reaction mixture was stirred at -78°C for 4 h. The reaction was then warmed to 25°C and was stirred at 25°C for 48 h. This solution was then quenched by the slow addition of the reaction mixture to a 2N aqueous hydrochloric acid solution (50 mL). The product was extracted into ethyl acetate (3 x 100 mL) and diethyl ether (1 x 50 mL). The organics were washed with a saturated aqueous lithium chloride solution (2 x 100 mL) and a saturated aqueous sodium chloride solution (1 x 150 mL), dried over magnesium sulfate and sodium sulfate, filtered, and concentrated *in vacuo*. Flash chromatography (Merck Silica gel 60, 230-400 mesh, 50/50 hexanes/ethyl acetate with acetic acid) afforded 3-cyclopentyl-2-(3-trifluoromethyl-phenyl)-propionic acid (1.16 g, 80.5%) as an off-white solid: mp 64-65°C; EI-HRMS m/e calcd for C₁₅H₁₇F₃O₂ (M + Na⁺) 309.1079, found 309.1084.

A solution of 3-cyclopentyl-2-(3-trifluoromethyl-phenyl)-propionic acid (286 mg, 1.0 mmol) in methylene chloride (10 mL) was cooled to 0°C and then treated with a 2.0M solution of oxalyl chloride in methylene chloride (0.6 mL, 1.2 mmol) and a few drops of *N*,*N*-dimethylformamide. The reaction mixture was stirred at 0°C for 15 min and at 25°C for 1.25 h. The reaction mixture was then treated with a solution of 2-aminothiazole (175 mg, 1.75 mmol) and *N*,*N-*diisopropylethylamine (0.42 mL, 2.41mmol) in tetrahydrofuran (10 mL). This solution was stirred at 25°C for 24 h. At this time, the reaction was concentrated *in vacuo*. High pressure liquid chromatography (Chromegasphere SI-60, 10 µM, 60Å, 25cm x 23cm ID, 60/40 heptane/ethyl acetate) afforded 3-cyclopentyl-N-thiazol-2-yl-2-(3-trifluoromethyl-phenyl)-propionamide (299.2 mg, 81.4%) as a light yellow solid: mp 134-136°C; EI-HRMS m/e calcd for C₁₈H₁₉F₃N₂OS (M⁺) 368.1170, found 368.1165.

### Example 91

### (A) 3-Cyclopentyl-2-(4-methanesulfonyl-3-trifluoromethyl-phenyl)-N-thiazol-2-yl-propionamide

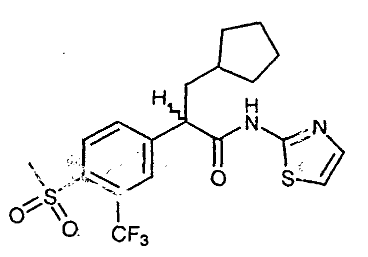

A solution of freshly prepared lithium diisopropylamide (35.3 mL of a 0.31M stock solution, 10.9 mmol) cooled to -78°C was treated with (4-fluoro-3-trifluoromethyl-phenyl)-acetic acid (1.11 g, 5.0 mmol) in tetrahydrofuran/1,3-dimethyl-3,4,5,6-tetrahydro-2(1H)-pyrimidinone (12.4 mL, 3:1). The resulting solution was stirred at -78°C for 1 h. At this time, the reaction was treated with a solution of iodomethylcyclopentane (1.16 g, 5.52 mmol) in 1,3-dimethyl-3,4,5,6-tetrahydro-2(1H)-pyrimidinone (1.2 mL). The reaction mixture was stirred at -78°C for 4 h. The reaction was then warmed to 25°C and was stirred at 25°C for 48 h. This solution was then quenched by the slow addition of the reaction mixture to a 2N aqueous hydrochloric acid solution (50 mL). The product was extracted into ethyl acetate (3 x 100 mL) and diethyl ether (1 x 50 mL). The organics were dried over magnesium sulfate and sodium sulfate, filtered, and concentrated *in* *vacuo*. Flash chromatography (Merck Silica gel 60, 230-400 mesh, 50/50 hexanes/ethyl acetate with acetic acid) afforded 3-cyclopentyl-2-(4-fluoro-3-trifluoromethyl-phenyl)-propionic acid (1.28 g, 84.3%) as a white solid: mp 66-68°C; EI-HRMS m/e calcd for C₁₅H₁₆F₄O₂ (M⁺) 305.1165, found 305.1174.

A solution of 3-cyclopentyl-2-(4-fluoro-3-trifluoromethyl-phenyl)-propionic acid (7.77 g, 25.3 mmol) in methanol (50 mL) was treated slowly with concentrated sulfuric acid (0.01 mL). The resulting reaction mixture was heated under reflux for 24 h. The reaction mixture was allowed to cool to 25°C and then concentrated *in vacuo*. The residue was dissolved in ethyl acetate (75 mL) and washed with a saturated aqueous sodium bicarbonate solution (1 x 50 mL), water (1 x 50 mL), and a saturated aqueous sodium chloride solution (4 x 50 mL). The organics were dried over magnesium sulfate and sodium sulfate, filtered, and concentrated *in vacuo* to afford 3-cyclopentyl-2-(4-fluoro-3-trifluoromethyl-phenyl)-propionic acid methyl ester (8.48 g, 87.5%) as yellow oil: EI-HRMS m/e calcd for C₁₆H₁₈F₄O₂ (M⁺) 318.1243, found 318.1240.

A solution of 3-cyclopentyl-2-(4-fluoro-3-trifluoromethyl-phenyl)-propionic acid methyl ester (7.0 g, 21.9 mmol) in *N,N*-dimethylformamide (50 mL) was treated with sodium methanethiolate (2.61 g, 33.0 mmol). The reaction mixture was then heated at 100-110°C for 24 h. At this time, the reaction was poured onto a mixture of ice and a 2N aqueous hydrochloric acid solution (100 mL). This mixture was extracted into ethyl acetate (3 x 75 mL) and diethyl ether (1 x 50 mL). The organics were then washed with water (1 x 75 mL) and a saturated aqueous sodium chloride solution (3 x 100 mL). The organics were dried over magnesium sulfate and sodium sulfate, filtered, and concentrated *in vacuo*. Flash chromatography (Merck Silica gel 60, 230-400 mesh, 85/15 hexanes/ethyl acetate) afforded 3-cyclopentyl-2-(4-methylsulfanyl-3-trifluoromethyl-phenyl)-propionic acid methyl ester (2.48g, 35.5%) as a pale yellow oil: EI-HRMS m/e calcd for C₁₇H₂₁F₃O₂S (M⁺) 346.1214 , found 346.1212.

A solution of 3-cyclopentyl-2-(4-methylsulfanyl-3-trifluoromethyl-phenyl)-propionic acid methyl ester (2.36 g, 6.81 mmol) in methylene chloride (75 mL) at 25°C was treated with 3-chloroperoxybenzoic acid (80-85% grade, 9.69 g, 40.1 mmol). The reaction mixture was stirred at 25°C for 16 h. At this time, the reaction was diluted with methylene chloride (75 mL). The solution was washed with a saturated aqueous sodium bisulfite solution (2 x 50 mL), water (1 x 50 mL), a saturated aqueous sodium chloride solution (3 x 75 mL), a saturated aqueous sodium bicarbonate solution (1 x 75 mL), and a saturated aqueous sodium chloride solution (3 x 75 mL). The organics were dried over magnesium sulfate and sodium sulfate, filtered, and concentrated *in vacuo* to afford 3-cyclopentyl-2-(4-methanesulfonyl-3-trifluoromethyl-phenyl)-propionic acid methyl ester (2.88 g) as a clear oil: EI-HRMS m/e calcd for C₁₇H₂₁F₃O₄S (M⁺) 378.1112 found 3-78.1116.

A solution of 3-cyclopentyl-2-(4-methanesulfonyl-3-trifluoromethyl-phenyl)-propionic acid methyl ester (395mg, 1.04 mmol) and 2-aminothiazole (209 mg, 1.38 mmol) in a solution of magnesium methoxide in methanol (7.4 wt%, 2.09 mL, 1.38 mmol) was heated at 110°C for 24 h. The reaction mixture was then concentrated *in vacuo*. Flash chromagaphy (Merck Silica gel 60, 230-400 mesh, 50/50 hexanes/ethyl acetate) afforded the 3-cyclopentyl-2-(4-methanesulfonyl-3-trifluoromethyl-phenyl)-N-thiazol-2-yl-propionamide (256.7 mg, 55.1%) as a white solid: mp 95-100°C; EI-HRMS m/e calcd for C₁₉H₂₁F₃N₂O₃S₂ (M⁺)446.0946, found 446.0944.

### (B) In an analogous manner, there was obtained:

(a) From (2-amino-thiazol-4-yl)-acetic acid methyl ester and 3-cyclopentyl-2-(4-methanesulfonyl-3-trifluoromethyl-phenyl)-propionic acid: {2-[3-Cyclopentyl-2-(4-methanesulfonyl-3-trifluoromethyl-phenyl)-propionylamino]-thiazol-4-yl}-acetic acid methyl ester as a white solid: mp 81-86°C; FAB-HRMS m/e calcd for C₂₂H₂₅F₃N₂O₅S₂ (M+H)⁺ 518.1157, found 518.1161.
(b) From 2-amino-thiazole-4-carboxylic acid methyl ester and 3-cyclopentyl-2-(4-methanesulfonyl-3-trifluoromethyl-phenyl)-propionic acid: 2-[3-Cyclopentyl-2-(4-methanesulfonyl-3-trifluoromethyl-phenyl)-propionylamino]-thiazole-4-carboxylic acid methyl ester as a white solid: mp 117-121°C; FAB-HRMS m/e calcd for C₂₁H₂₃F₃N₂O₅S₂ (M+H)⁺ 504.1000, found 504.1000.

### Example 92

### 3-Cyclopentyl-2-(4-methanesulfonyl-3-trifluoromethyl-phenyl)-N-pyridin-2-yl-propionamide

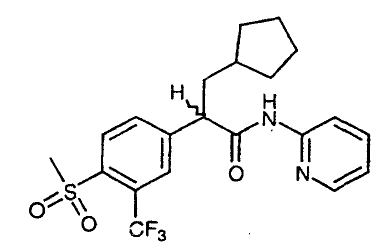

A solution of freshly prepared lithium diisopropylamide (141.28 mL of a 0.31M stock solution, 43.8 mmol) cooled to -78°C was treated with (4-fluoro-3-trifluoromethyl-phenyl)-acetic acid (4.44 g, 20.0 mmol) in tetrahydrofuran/1,3-dimethyl-3,4,5,6-tetrahydro-2(1H)-pyrimidinone (49.68 mL, 3:1). The resulting solution was stirred at - 78°C for 1 h. At this time, the reaction was treated with a solution of iodomethylcyclopentane (4.64 g, 22.09 mmol) in 1,3-dimethyl-3,4,5,6-tetrahydro-2(1H)-pyrimidinone (4.6 mL). The reaction mixture was stirred at -78°C for 4 h. The reaction was then warmed to 25°C and was stirred at 25°C for 48 h. This solution was then quenched by the slow addition of the reaction mixture to a 2N aqueous hydrochloric acid solution. The product was extracted into ethyl acetate (3 x 400 mL) and diethyl ether (1 x 200 mL). The organics were dried over magnesium sulfate and sodium sulfate, filtered, and concentrated *in vacuo*. Flash chromatography (Merck Silica gel 60, 230-400 mesh, 50/50 hexanes/ethyl acetate with acetic acid) afforded 3-cyclopentyl-2-(4-fluoro-3-trifluoromethyl-phenyl)-propionic acid (3.37 g, 55.4%) as a white solid: mp 66-68°C; EI-HRMS m/e calcd for C₁₅H₁₆F₄O₂ (M⁺) 305.1165, found 305.1174.

A solution of 3-cyclopentyl-2-(4-fluoro-3-trifluoromethyl-phenyl)-propionic acid (1.52 g, 5.0 mmol) in *N,N*-dimethylformamide (10mL) was treated with sodium methanethiolate (0.59 g, 7.5 mmol). The reaction mixture was then heated to 100-110°C for 14 h. At this time, the reaction was poured onto a mixture of ice and a 2N aqueous hydrochloric acid solution (25 mL). This mixture was extracted into ethyl acetate (3 x 35 mL) and diethyl ether (1 x 25 mL). The organics were then washed with water (1 x 50 mL) and a saturated aqueous sodium chloride solution (3 x 75 mL). The organics were dried over magnesium sulfate and sodium sulfate, filtered, and concentrated *in vacuo*. Flash chromatography (Merck Silica gel 60, 230-400 mesh, 80/20 hexanes/ethyl acetate w/acetic acid) afforded 3-cyclopentyl-2-(4-methylsulfanyl-3-trifluoromethyl-phenyl)-propionic acid (1.36 g, 83.4%) as a pale yellow oil: EI-HRMS m/e calcd for C₁₆H₁₉F₃O₂S (M⁺) 332.1058, found 332.1057.

A solution of 3-cyclopentyl-2-(4-methylsulfanyl-3-trifluoromethyl-phenyl)-propionic acid (1.29 g, 3.89 mmol) in ethanol (25 mL) was treated slowly with concentrated sulfuric acid (0.01 mL). The resulting reaction mixture was heated under reflux for 48 h. The reaction mixture was allowed to cool to 25°C and then concentrated *in vacuo*. The residue was dissolved in ethyl acetate (35 mL) and washed with a saturated aqueous sodium bicarbonate solution (1 x 15 mL), water (1 x 15 mL), and a saturated aqueous sodium chloride solution (3 x 20 mL). The organics were dried over magnesium sulfate and sodium sulfate, filtered, and concentrated *in vacuo* to afford 3-cyclopentyl-2-(4-methylsulfanyl-3-trifluoromethyl-phenyl)-propionic acid ethyl ester (1.39 g, 94.8%) as yellow oil: EI-HRMS m/e calcd for C₁₈H₂₃F₃O₂S (M⁺) 360.1370, found 360.1370.

A solution of 3-cyclopentyl-2-(4-methylsulfanyl-3-trifluoromethyl-phenyl)-propionic acid ethyl ester (1.32 g, 3.69 mmol) in methylene chloride (50 mL) at 25°C was treated with 3-chloroperoxybenzoic acid (80-85% grade, 4.8 g, 19.8 mmol). The reaction mixture was stirred at 25°C for 4 d. At this time, the reaction was diluted with methylene chloride (25 mL). This solution was washed with a saturated aqueous sodium bisulfite solution (1 x 50 mL), water (1 x 50 mL), a saturated aqueous sodium bicarbonate solution (1 x 50 mL), water (1 x 50 mL), and a saturated aqueous sodium chloride solution (3 x 50mL). The organics were dried over magnesium sulfate and sodium sulfate, filtered, and concentrated *in vacuo*. Flash chromatography (Merck Silica gel 60, 230-400 mesh, 70/30 hexanes/ethyl acetate with acetic acid) afforded 3-cyclopentyl-2-(4-methanesulfonyl-3-trifluoromethyl-phenyl)-propionic acid ethyl ester (1.28 g, 89.0%) as a clear oil: EI-HRMS m/e calcd for C₁₈H₂₃F₃O₄S (M⁺) 392.1269 found 392.1268.

A solution of 3-cyclopentyl-2-(4-methanesulfonyl-3-trifluoromethyl-phenyl)-propionic acid ethyl ester (707mg, 1.80 mmol in tetrahydrofuran/water (24 mL, 3:1) was treated with lithium hydroxide (166 mg, 3.97 mmol). The reaction was stirred at 25°C for 24 h. At this time, the reaction concentrated *in vacuo*. The residue was diluted with water (25 mL) and extracted with diethyl ether (1 x 15 mL). The aqueous layer was acidified to pH = I with a 2N aqueous hydrochloric acid solution, and extracted with chloroform (3 x 25 mL). The organics were washed with water (1 x 25 mL), a saturated aqueous sodium chloride solution (3 x 25 mL), dried over magnesium sulfate, filtered, and concentrated *in vacuo* to afford 3-cyclopentyl-2-(4-methanesulfonyl-3-trifluoromethyl-phenyl)-propionic acid (426.7 mg, 65%) as a white solid: mp 122-123°C; EI-HRMS m/e calcd for C₁₆H₁₉F₃O₄S (M⁺) 364.0956 found 364.0956.

A solution of and 3-cyclopentyl-2-(4-methanesulfonyl-3-trifluoromethyl-phenyl)-propionic acid (73 mg, 0.2 mmol) and triphenylphosphine (79 mg, 0.3 mmol) in methylene chloride (5.0 mL) was cooled to 0°C and then treated with N-bromosuccinimide (60.5 mg, 0.34 mmol). After the complete addition of N-bromosuccinimide, the reaction mixture was allowed to warm to 25°C over 30 min. The reaction mixture was then treated with 2-aminopyridine (28.2 mg, 0.3mmol) and pyridine (1 drop). The resulting reaction mixture was stirred at 25°C for 48 h. The reaction mixture was then diluted with methylene chloride (50 mL). The organic layer was washed with water (1 x 50 mL) and a saturated aqueous sodium chloride solution (2 x 25 mL), dried over magnesium sulfate and sodium sulfate, filtered, and concentrated *in vacuo*. High pressure liquid chromatography (Chromegasphere SI-60, 10 µM, 60Å, 25cm x 23cm ID, 50/50 heptane/ethyl acetate) afforded 3-cyclopentyl-2-(4-methanesulfonyl-3-trifluoromethyl-phenyl)-N-pyridin-2-yl-propionamide (54.2 mg, 61.5%) as a white solid: mp 86-89°C; EI-HRMS m/e calcd for C₂₁H₂₃F₃N₂O₃S (M⁺) 440.1383, found 440.1381.

### Example 93

### 3-Cyclopentyl-2-(4-methylsulfanyl-3-trifluoromethyl-phenyl)-N-thiazol-2-yl-propionamide

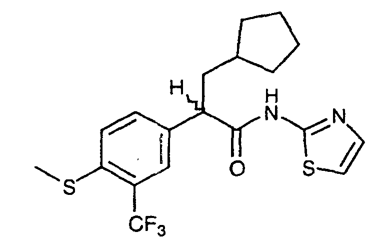

A solution of 3-cyclopentyl-2-(4-fluoro-3-trifluoromethyl-phenyl)-propionic acid (prepared in Example **89,** 1.52 g, 5.0 mmol) in *N,N*-dimethylformamide (10 mL) was treated with sodium methanethiolate (593 mg, 7.5 mmol). The reaction mixture was then heated to 100-110°C for 14 h. At this time, the reaction was cooled to 25°C and poured onto a 1N aqueous hydrochloric acid solution (25 mL) and extracted into ethyl acetate (3 x 25 mL) and diethyl ether (1 x 25 mL). The organics were then washed with water (1 x 50 mL) and a saturated aqueous sodium chloride solution (3 x 75 mL), dried over magnesium sulfate and sodium sulfate, filtered, and concentrated *in vacuo*. Flash chromatography (Merck Silica gel 60, 230-400 mesh, 80/20 hexanes/ethyl acetate) afforded 3-cyclopentyl-2-(4-methylsulfanyl-3-trifluoromethyl-phenyl)-propionic acid (1.37 g, 82.4%) as a pale yellow oil: EI-HRMS m/e calcd for C₁₆H₁₉F₃O₂S (M⁺) 332.1058, found 332.1057.

A solution of benzotriazol-1-yloxy-tris(dimethylamino)phosphonium hexafluorophosphate (188 mg, 0.42 mmol) and 3-cyclopentyl-2-(4-methylsulfanyl-3-trifluoromethyl-phenyl)-propionic acid (94 mg, 0.28 mmol) in *N,N*-dimethylformamide (5 mL) was treated with *N,N*-diisopropylethylamine (150 µL, 0.85 mmol) and 2-aminothiazole (42.5 mg, 0.42 mmol). The mixture was stirred at 25°C for 48 h. At this time, the reaction mixture was poured into cold water (25 mL) containing a 1N aqueous hydrochloric acid solution (50 mL) and extracted into ethyl acetate (2 x 75 mL) and diethyl ether (1 x 25 mL). The organics were then washed with water (2 x 75 mL) and a saturated aqueous sodium chloride solution (3 x 75 mL), dried over magnesium sulfate and sodium sulfate, filtered, and concentrated *in vacuo*. Flash chromatography (Merck Silica gel 60, 230-400 mesh, 50/50 hexanes/ethyl acetate) afforded 3-cyclopentyl-2-(4-methylsulfanyl-3-trifluoromethyl-phenyl)-N-thiazol-2-yl-propionamide (50.5 mg, 43.1%) as a clear oil: FAB-HRMS m/e calcd for C₁₉H₂₁F₃N₂OS₂ (M+H)⁺ 415.1125, found 415.1123.

### Example 94

### 2-(3-Chloro-4-methanesulfonyl-phenyl)-3-cyclopentyl-N-pyridin-2-yl-propionamide

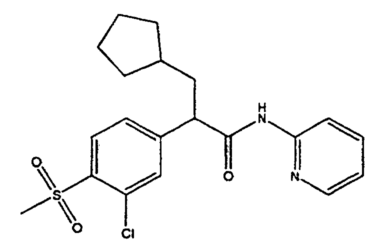

A solution of aluminum trichloride (34.8 g, 261.4 mmol) in chloroform (120 mL) under argon and cooled to 0°C and was then treated dropwise with a solution of ethyl chlorooxoacetate (18.7 mL, 167.5 mmol) in chloroform (120 mL). The mixture was then stirred at 0°C for 30 min. After this time a solution of 2-chlorothioanisole (25.0 g, 156.5 mmol) in chloroform (120 mL) was added dropwise to the above mixture at 0°C and it turned red in color. It was allowed to warm to 25°C and stirred for an additional 3.5 h. The reaction was quenched by slowly adding water (500 mL). The solution turned yellow in color and was then transferred to a separatory funnel and extracted with chloroform (3 x 50 mL). The organic phase was dried over sodium sulfate, filtered and concentrated *in vacuo*. Flash chromatography (Merck Silica gel 60, 230-400 mesh, 80/20 hexanes/ethyl acetate) afforded (3-chloro-4-methylsulfanyl-phenyl)-oxo-acetic acid ethyl ester (31.37 g, 77%) as a yellow oil.

A solution of cylcopenytlmethyl triphenylphosphine iodide (725 mg, 1.53 mmol) in tetrahydrofuran (10 mL) was cooled to 0°C. To this cooled solution was added sodium bis(trimethylsilyl)amide (1.0 M in THF, 2.14 mL, 2.14 mmol) and the reaction turned red in color. It was stirred at 0°C for 45 minutes and then a solution of (3-chloro-4-methylsulfanyl-phenyl)-oxo-acetic acid ethyl ester (355 mg, 1.37 mmol) in tetrahydrofuran (5 mL) was added slowly. The reaction was warmed to 25°C and stirred for 20 h. The reaction was diluted with water (50 mL) and transferred to a separatory funnel and extracted with diethyl ether ( 3 x 25 mL). The organic layers were dried over sodium sulfate, filtered and concentrated *in vacuo*. Chromatography (Biotage Flash 12M column, 80/20 hexanes/ethyl acetate) afforded 2-(3-chloro-4-methylsulfanyl-phenyl)-3-cyclopentyl-acrylic acid ethyl ester (267 mg, 60%, mixture of E and Z isomers (2:1)) as a yellow oil and taken on without characterization.

A solution of E and Z 2-(3-chloro-4-methylsulfanyl-phenyl)-3-cyclopentyl-acrylic acid ethyl ester (100 mg, 0.31 mmol) dissolved in methylene chloride (5 mL) cooled to 0°C was treated with 3-chloroperoxybenzoic acid (80%, 157 mg, 0.729 mmol) and stirred for 3.5 h. The reaction mixture was diluted with methylene chloride (25 mL), transferred to a separatory funnel and washed with saturated aqueous sodium carbonate solution (2 x 10 mL) and brine (2 x 10mL). The organic layers were dried over sodium sulfate, filtered and concentrated *in vacuo*. Chromatography (Biotage Flash 12M column, 80/20 hexanes/ethyl acetate) afforded 2-(3-chloro-4-methanesulfonyl-phenyl)-3-cyclopentyl-acrylic acid ethyl ester (95 mg, 86%, mixture of E and Z isomers (2:1)) as a colorless oil and taken on without characterization.

The mixture of E and Z isomers of 2-(3-chloro-4-methanesulfonyl-phenyl)-3-cyclopentyl-acrylic acid ethyl ester (1.04 g, 2.91 mmol), nickel chloride hexahydrate (69 mg, 0.29 mmol) and methanol (25 mL) were placed in a flask under argon. To this green solution was then added sodium borohydride (221 mg, 5.83 mmol) slowly in small portions using an ice bath if necessary to keep the temperature at 20°C. The solution turned black and a fine precipitate formed after addition of the sodium borohydride. This was then stirred at 25°C for 1.5 h. After such time the reaction was filtered through celite and washed with methanol. The filtrate and washings were combined and concentrated *in vacuo* to reduce the volume. The residual solution was then diluted with water (15 mL) and extracted with ethyl acetate (3 x 15 mL) dried over sodium sulfate, filtered and concentrated in *vacuo*. Flash chromatography (Merck Silica gel 60, 230-400 mesh, 75/25 hexanes/ethyl acetate) afforded a mixture of 2-(3-chloro-4-methanesulfonyl-phenyl)-3-cyclopentyl-propionic acid methyl ester and 2-(3-chloro-4-methanesulfonyl-phenyl)-3-cyclopentyl-propionic acid ethyl ester (transesterification occurred under the reaction conditions) (937 mg) as a clear colorless oil. (It was carried on without characterization because it was a mixture of esters)

The mixture of 2-(3-chloro-4-methanesulfonyl-phenyl)-3-cyclopentyl-propionic acid methyl ester and 2-(3-chloro-4-methanesulfonyl-phenyl)-3-cyclopentyl-propionic acid ethyl ester, from above, (937 mg) was dissolved in ethanol (30 mL) and allowed to dissolve. To this solution was then added a solution of potassium hydroxide (733 mg, 13.1 mmol) in water (7 mL). The yellow solution was then stirred for 3 h at 25°C. It was concentrated *in vacuo* to remove the ethanol and then 1N hydrochloric acid was added until the pH = 2. This was then extracted with methylene chloride (3 X 15 mL). The organic layers were then dried over sodium sulfate, filtered and concentrated *in vacuo*. Flash chromatography (Merck Silica gel 60, 230-400 mesh, 75/25 hexanes/ethyl acetate plus 1% acetic acid) afforded 2-(3-chloro-4-methanesulfonyl-phenyl)-3-cyclopentyl-propionic acid (787 mg, 82% over two steps) as a white solid: mp: 123.9-126.2°C; FAB-HRMS m/e calcd for C₁₅H₁₉O₄SCl (M+H)⁺ 331.0771, found 331.0776.

Triphenylphosphine (238 mg, 0.91 mmol) was dissolved in methylene chloride (10 mL) and cooled to 0°C. To this solution was added N-bromosuccinimide (183 mg, 1.03 mmol) and was stirred at 0°C until it was completely dissolved and became light purple in color. The 2-(3-chloro-4-methanesulfonyl-phenyl)-3-cyclopentyl-propionic acid (200 mg, 0.61 mmol) was then added and it was stirred at 0°C for 20 min and then warmed to 25°C and stirred for 30 min. After such time 2-aminopyridine (85 mg, 0.91 mmol) and pyridine (0.088 mL, 1.09 mmol) were added and it was stirred at 25°C for 16 h. The reaction was then diluted with water (10 mL) and then extracted with methylene chloride (3 x 15 mL). The organic layers were then combined and dried over sodium sulfate, filtered and concentrated *in vacuo*. Flash chromatography (Merck Silica gel 60, 230-400 mesh, 60/40 hexanes/ethyl acetate) afforded 2-(3-chloro-4-methanesulfonyl-phenyl)-3-cyclopentyl-N-pyridin-2-yl-propionamide (210 mg, 85%) as a colorless oil: EI-HRMS m/e calcd for C₂₀H₂₃N₂O₃SCl (M⁺) 406.1118, found 406.1120.

### Example 95

### N-(5-Bromo-pyridin-2-yl)-2-(3-chloro-4-methanesulfonyl-phenyl)-3-cyclopentyl-propionamide

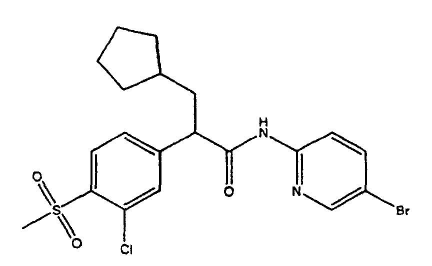

A solution of triphenylphosphine (238 mg, 0.91 mmol) in methylene chloride (10 mL) was cooled to 0°C and then treated with *N*-bromosuccinimide (183 mg, 1.03 mmol). The reaction mixture was stirred at 0°C until it was completely dissolved and became light purple in color. The reaction mixture was then treated with 2-(3-chloro-4-methanesulfonyl-phenyl)-3-cyclopentyl-propionic acid (prepared in Example **94**, 200 mg, 0.61 mmol) and stirred at 0°C for 20 min and then warmed to 25°C where it was stirred for 30 min. After such time, the reaction mixture was treated with 2-amino-5-bromopyridine (157 mg, 0.91 mmol) and pyridine (0.088 mL, 1.09 mmol), and reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Flash chromatography (Merck Silica gel 60, 230-400 mesh, 70/30 hexanes/ethyl acetate) afforded 2-(3-chloro-4-methanesulfonyl-phenyl)-3-cyclopentyl-N-(5-bromo-pyridin-2-yl)-propionamide (245 mg, 83%) as a white foam: EI-HRMS m/e calcd for C₂₀H₂₂Br ClN₂O₃S (M⁺) 484.0223, found 484.0222.

### Example 96

### N-(5-Chloro-pyridin-2-yl)-2-(3-chloro-4-methanesulfonyl-phenyl)-3-cyclopentyl-propionamide

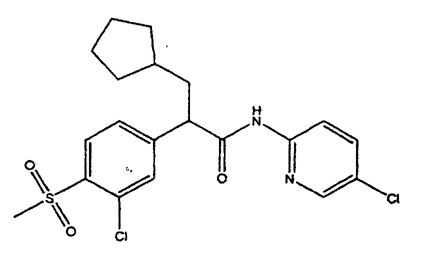

A solution of triphenylphosphine (238 mg, 0.91 mmol) in methylene chloride (10 mL) was cooled to 0°C and then treated with *N*-bromosuccinimide (183 mg, 1.03 mmol). The reaction mixture was stirred at 0°C until it was completely dissolved and became light purple in color. The reaction mixture was then treated with 2-(3-chloro-4-methanesulfonyl-phenyl)-3-cyclopentyl-propionic acid (prepared in Example **94,** 200 mg, 0.61 mmol) and stirred at 0°C for 20 min and then warmed to 25°C where it was stirred for 30 min. After such time, the reaction mixture was treated with 2-amino-5-chloropyridine (117 mg, 0.91 mmol) and pyridine (0.088 mL, 1.09 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Flash chromatography (Merck Silica gel 60, 230-400 mesh, 80/20 hexanes/ethyl acetate) afforded 2-(3-chloro-4-methanesulfonyl-phenyl)-3-cyclopentyl-N-(5-chloro-pyridin-2-yl)-propionamide (110 mg, 41%) as a yellow foam: EI-HRMS m/e calcd for C₂₀H₂₂ Cl₂N₂O₃S (M⁺) 440.0728, found 440.0728.

### Example 97

### 2-(3-Chloro-4-methanesulfonyl-phenyl)-3-cyclopentyl-N-(5-trifluoromethyl-pyridin-2-yl)-propionamide

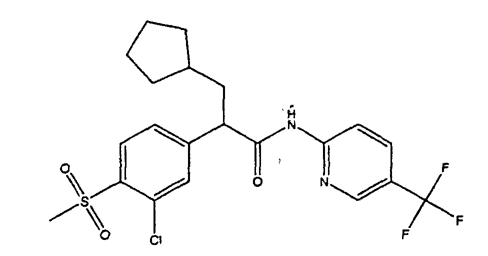

A solution of triphenylphosphine (238 mg, 0.91 mmol) in methylene chloride (10 mL) was cooled to 0°C and then treated with *N*-bromosuccinimide (183 mg, 1.03 mmol). The reaction mixture was stirred at 0°C until it was completely dissolved and became light purple in color. The reaction mixture was then treated with 2-(3-chloro-4-methanesulfonyl-phenyl)-3-cyclopentyl-propionic acid (prepared in Example **94,** 200 mg, 0.61 mmol) and stirred at 0°C for 20 min and then warmed to 25°C where it was stirred for 30 min. After such time, the reaction mixture was treated with 2-amino-5-trifluoromethyl-pyridine (147 mg, 0.91 mmol) and pyridine (0.088 mL, 1.09 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Flash chromatography (Merck Silica gel 60, 230-400 mesh, 60/40 hexanes/ethyl acetate) afforded 2-(3-chloro-4-methanesulfonyl-phenyl)-3-cyclopentyl-N-(5- trifluoromethyl - pyridin-2-yl)-propionamide (122 mg, 43%) as a white foam: EI-HRMS m/e calcd for C₂₀H₂₂ ClF₃N₂O₃S (M⁺) 474.0992, found 474.0990.

### Example 98

### {2-[2-(3-Chloro-4-methanesulfonyl-phenyl)-3-cyclopentyl-propionylamino]-thiazol-4-yl}-oxo-acetic acid ethyl ester

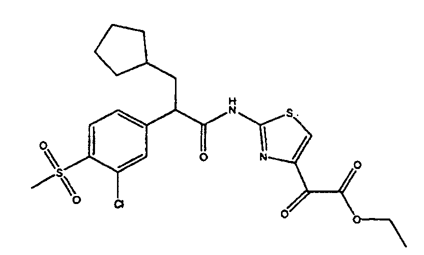

A solution of triphenylphosphine (238 mg, 0.91 mmol) in methylene chloride (10 mL) was cooled to 0°C and then treated with *N*-bromosuccinimide (183 mg, 1.03 mmol). The reaction mixture was stirred at 0°C until it was completely dissolved and became light purple in color. The reaction mixture was then treated with 2-(3-chloro-4-methanesulfonyl-phenyl)-3-cyclopentyl-propionic acid (prepared in Example **94**, 200 mg, 0.61 mmol) and stirred at 0°C for 20 min and then warmed to 25°C where it was stirred for 30 min. After such time, the reaction mixture was treated with 2-(amino-thiazol-4-yl)-oxo-acetic acid ethyl ester (182 mg, 0.91 mmol) and pyridine (0.088 mL, 1.09 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Flash chromatography (Merck Silica gel 60, 230-400 mesh, 75/25 hexanes/ethyl acetate) afforded {2-[2-(3-chloro-4-methanesulfonyl-phenyl)-3-cyclopentyl-propionylamino]-thiazol-4-yl}-oxo-acetic acid ethyl ester (208 mg, 67%) as a clear colorless oil: EI-HRMS m/e calcd for C₂₂H₂₅ClN₂O₆S₂ (M⁺) 513.0921, found 513.0919.

### Example 99

### 2(R)-(3-Chloro-4-methanesulfonyl-phenyl)-3-cyclopentyl-N-thiazol-2-yl-propionamide

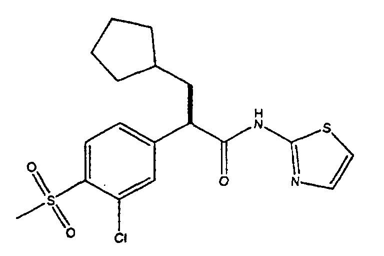

A mixture of 2-(3-chloro-4-methanesulfonyl-phenyl)-3-cyclopentyl-propionic acid (prepared in Example **94**, 6.07 g, 18.35 mmol), (R)-(+)-4-benzyl-2-oxazolidinone (2.83 g, 15.96 mmol), and triethylamine (6.68 mL, 47.71 mmol) in toluene (50 mL) was heated at 80°C under argon until a homogeneous solution was obtained. The reaction mixture was then treated with trimethylacetyl chloride (3.55 mL, 28.81 mmol) in toluene (10 mL), and the reaction became yellow in color and a precipitate formed. The reaction mixture was then heated at 80°C for 36 h. The reaction was cooled to 25°C and then the toluene was removed *in vacuo*. The residue was diluted with ethyl acetate (150 mL). The organic layer was washed with a 1N aqueous hydrochloric solution (1 x 100 mL), a 10% aqueous sodium carbonate solution (1 x 100 mL), and a saturated aqueous sodium chloride solution (1 x 100 mL). The organic layer was then dried over magnesium sulfate, filtered, and concentrated *in vacuo*. Flash chromatography (Merck Silica gel 60, 230-400 mesh, 90/5/5 methylene chloride/hexanes/ethyl acetate) afforded (1) 4(R)-benzyl-3-[2(S)-(3-chloro-4-methanesulfonyl-phenyl)-3-cyclopentyl-propionyl]-oxazolidin-2-one (2.08 g, 23%) as a white foam: [α]²³₅₈₉ = +10.4° (c=0.144, chloroform); FAB-HRMS m/e calcd for C₂₅H₂₈ClNO₅S (M+H)⁺ 490.1455, found 490.1457 and (2) 4(R)-benzyl-3-[2(R)-(3-chloro-4-methanesulfonyl-phenyl)-3-cyclopentyl-propionyl]-oxazolidin-2-one (2.20 g, 25%) as a white foam: [α]²³₅₈₉ = -93.9° (c=0.165, chloroform); FAB-HRMS m/e calcd for C₂₅H₂₈ClNO₅S (M+H)⁺ 490.1455, found 490.1443.

A solution of lithium hydroxide (215 mg, 9.0 mmol) in water (2.8 mL) was treated with a 30% aqueous hydrogen peroxide solution (2.0 mL, 18 mmol). This freshly prepared lithium hydroperoxide solution was then cooled to 0°C and then slowly added to a cooled (0°C) solution of 4(R)-benzyl-3-[2(R)-(3-chloro-4-methanesulfonyl-phenyl)-3-cyclopentyl-propionyl]-oxazolidin-2-one (2.20 g, 4.5 mmol) in tetrahydrofuran (18 mL) and water (5.8 mL). After 1.5 h at 0°C, the reaction was quenched with a 1.5N aqueous sodium sulfite solution (25 mL) and was diluted with water (150 mL). The aqueous layer was extracted with diethyl ether (3 x 50 mL). The aqueous layer was then acidified with a 1N aqueous hydrochloric acid solution to pH = 2 and extracted with ethyl acetate (3 x 50 mL). The combined organic layers were dried over sodium sulfate, filtered, and concentrated *in vacuo*. Flash chromatography (Merck Silica gel 60, 230-400 mesh, 75/25 hexanes/ethyl acetate with 1% acetic acid) afforded 2(R)-(3-chloro-4-methanesulfonyl-phenyl)-3-cyclopentyl-propionic acid (1.26 g, 85%) as a white solid: mp 106.1-108.8°C; [α]²³₅₈₉ = -43.0° (c=0.172, chloroform); EI-HRMS m/e calcd for C₁₅H₁₉ClO₄S (M⁺) 330.0692, found 330.0690.

A solution of triphenylphosphine (248 mg, 0.94 mmol) in methylene chloride (9 mL) was cooled to 0°C and then treated with *N*-bromosuccinimide (190 mg, 1.07 mmol). The reaction mixture was stirred at 0°C until it was completely dissolved and became light purple in color. The reaction mixture was then treated with 2(R)-(3-chloro-4-methanesulfonyl-phenyl)-3-cyclopentyl-propionic acid (208 mg, 0.63 mmol). The reaction mixture was stirred at 0°C for 20 min and then warmed to 25°C where it was stirred for 30 min. After such time, the reaction mixture was treated with 2-aminothiazole (95 mg, 0.94 mmol) and pyridine (0.092 mL, 1.13 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo.* Biotage chromatography (FLASH 40S, Silica, 65/35 hexanes/ethyl acetate) afforded 2(R)-(3-chloro-4-methanesulfonyl-phenyl)-3-cyclopentyl-N-thiazol-2-yl-propionamide (210 mg, 81%) as a white foam: [α]²³₅₈₉ = -54.3° (c=0.081, chloroform); EI-HRMS m/e calcd for C₁₈H₂₁ClN₂O₃S₂ (M⁺) 412.0682, found 412.0679.

### Example 100

### 2(R)-(3-Chloro-4-methanesulfonyl-phenyl)-3-cyclopentyl-N-pyridin-2-yl-propionamide

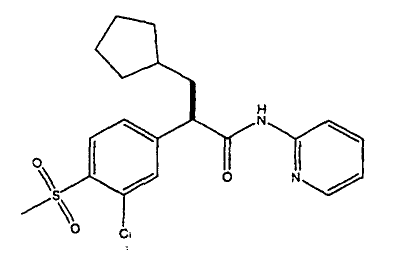

A solution of triphenylphosphine (238 mg, 0.91 mmol) in methylene chloride (10 mL) was cooled to 0°C. and then treated with *N*-bromosuccinimide (183 mg, 1.03 mmol). The reaction mixture was stirred at 0°C until it was completely dissolved and became light purple in color. The reaction mixture was then treated with 2(R)-(3-chloro-4-methanesulfonyl-phenyl)-3-cyclopentyl-propionic acid (prepared in Example **99,** 200 mg, 0.61 mmol). The reaction mixture was stirred at 0°C for 20 min and then warmed to 25°C where it was stirred for 30 min. After such time, the reaction mixture was treated with 2-aminopyridine (85 mg, 0.91 mmol) and pyridine (0.088 mL, 1.09 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Biotage chromatography (FLASH 40S, Silica, 60/40 hexanes/ethyl acetate) afforded 2(R)-(3-chloro-4-methanesulfonyl-phenyl)-3-cyclopentyl-N-pyridin-2-yl-propionamide (202 mg, 81.5%) as a white foam: [α]²³₅₈₉ = -41.8° (c=0.098, chloroform); EI-HRMS m/e calcd for C₂₀H₂₃ClN₂O₃S (M⁺) 406.1118, found 406.1119.

### Example 101

### N-(5-Bromo-pyridin-2-yl)-2(R)-(3-chloro-4-methanesulfonyl-phenyl)-3-cyclopentyl-propionamide

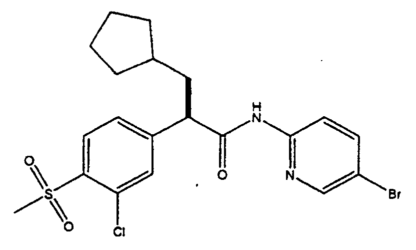

A solution of triphenylphosphine (238 mg, 0.91 mmol) in methylene chloride (10 mL) was cooled to 0°C and then treated with N-bromosuccinimide (183 mg, 1.03 mmol). The reaction mixture was stirred at 0°C until it was completely dissolved and became light purple in color. The reaction mixture was then treated with 2(R)-(3-chloro-4-methanesulfonyl-phenyl)-3-cyclopentyl-propionic acid (prepared in Example **99,** 200 mg, 0.61 mmol). The reaction mixture was stirred at 0°C for 20 min and then warmed to 25°C where it was stirred for 30 min. After such time, the reaction mixture was treated with 2-aminopyridine (85 mg, 0.91 mmol) and pyridine (0.088 mL, 1.09 mmol), and the reaction mixture was stirred at 25°C for 16 h. The reaction was then diluted with water (10 mL) and then extracted with methylene chloride (3 x 15 mL). The combined organic layers were dried over sodium sulfate, filtered, and concentrated *in vacuo*. Biotage chromatography (FLASH 40S, Silica, 60/40 hexanes/ethyl acetate) afforded N-(5-bromo-pyridin-2-yl)-2(R)-(3-chloro-4-methanesulfonyl-phenyl)-3-cyclopentyl-propionamide (222 mg, 76%) as an off-white foam: [α]²³₅₈₉ = -48.6° (c=0.105, chloroform); EI-HRMS m/e calcd for C₂₀H₂₂BrClN₂O₃S (M⁺) 484.0223, found 484.0223.

### Example 102

### N-(5-Cyano-pyridin-2-yl)-3-cyclopentyl-2-(3,4-dichloro-phenyl)-propionamide

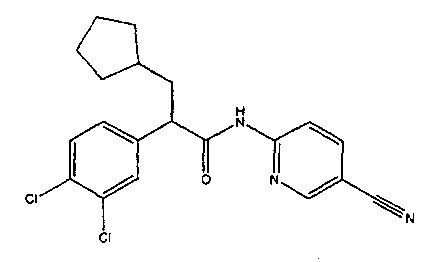

A solution of nickel(II) bromide (253 mg, 1.16 mmol), triphenylphosphine (1.15g, 4.39 mmol), and zinc powder (113 mg, 1.73 mmol) in acetonitrile (11 mL) was stirred under argon at 60°C for 1 h. The reaction turned dark brown in color. After such time, the reaction mixture was treated with sodium cyanide (578 mg, 11.8 mmol) and 2-amino-5-bromopyridine (2.00 g, 11.6 mmol), and the reaction mixture was stirred at 60°C for 16 h. The reaction mixture was then cooled to 25°C, diluted with ethyl acetate (50 mL), and then filtered through celite. The filtrate was concentrated *in vacuo*. Flash chromatography (Merck Silica gel 60, 230-400 mesh, 100% ethyl acetate) afforded 6-amino-nicotinonitrile (577 mg, 42%) as a white solid: mp 156.8-158.5°C, EI-HRMS m/e calcd for C₆H₅N₃ (M⁺) 119.0483, found 119.0480.

A solution of triphenylphosphine (1.23 g, 4.70 mmol) in methylene chloride (26 mL) was cooled to 0°C and then treated with *N*-bromosuccinimide (948 mg, 5.33 mmol). The reaction mixture was stirred at 0°C until it was completely dissolved and became light purple in color. The reaction mixture was then treated with 3-cyclopentyl-2-(3,4-dichloro-phenyl)-propionic acid (prepared in Example **38**, 900 mg, 3.13 mmol). The reaction mixture was stirred at 0°C for 20 min and then warmed to 25°C where it stirred for 30 min. After such time, the reaction mixture was treated with 6-amino-nicotinonitrile (560 mg, 4.70 mmol) and pyridine (0.46 mL, 5.64 mmol), and the reaction mixture was stirred at 25°C for 16 h. The reaction was then diluted with water (25 mL) and then extracted with methylene chloride (3 x 25 mL). The combined organic layers were dried over sodium sulfate, filtered, and concentrated *in vacuo*. Flash chromatography (Merck Silica gel 60, 230-400 mesh, 85/15 hexanes/ethyl acetate) afforded N-(5-cyano-pyridin-2-yl)-3-cyclopentyl-2-(3,4-dichloro-phenyl)-propionamide (882 mg, 73%) as a pink foam: EI-HRMS m/e calcd for C₂₀H₁₉Cl₂N₃O (M⁺) 387.0905, found 387.0905.

### Example 103

### 3-Cyclopentyl-2(R)-(3,4-dichloro-phenyl)-N-(5-trifluoromethyl-pyridin-2-yl)-propionamide

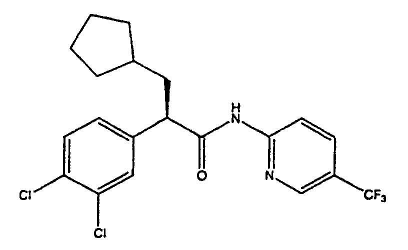

A solution of 3-cyclopentyl-2(R)-(3,4-dichloro-phenyl)-propionic acid (prepared in Example **54,** 200 mg, 0.69 mmol) in methylene chloride (10 mL) and one drop of *N,N*-dimethylformamide was cooled to 0°C and then treated with a 2.0M solution of oxalyl chloride in methylene chloride (0.42 mL, 0.84 mmol). Gas evolution began immediately. The reaction mixture was allowed to warm slowly to 25°C where it was stirred for 30 min. After this time, the reaction mixture was treated with a solution of *N,N*-diisopropylethylamine (0.24 mL, 1.39 mmol) and 5-trifluoromethyl-2-aminopyridine (150 mg, 0.905 mmol) in tetrahydrofuran (4 mL) in one portion. The resulting reaction mixture was stirred for 16 h at 25°C. After such time, the reaction was diluted with water (15 mL) and was extracted with methylene chloride (3 x 15 mL). The combined organic layers were dried over sodium sulfate, filtered, and concentrated *in vacuo*. Flash chromatography (Merck Silica gel 60, 230-400 mesh, 90/10 hexanes/ethyl acetate) afforded the 3-cyclopentyl-2(R)-(3,4-dichloro-phenyl)-N-(5-trifluoromethyl-pyridin-2-yl)-propionamide (77 mg, 26%) as a white solid: mp 113.8-117.5°C; EI-HRMS m/e calcd for C₂₀H₁₉Cl₂F₃N₂O (M⁺) 430.0826, found 430.0835.

### Example 104

### 6-[3-Cyclopentyl-2(R)-(3,4-dichloro-phenyl)-propionylamino]-nicotinic acid

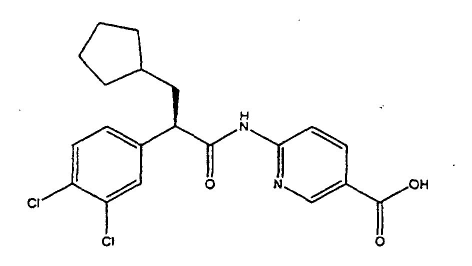

A solution of 6-[3-cyclopentyl-2(R)-(3,4-dichloro-phenyl)-propionylamino]-nicotinic acid methyl ester (prepared in Example **45**, 188 mg, 0.45 mmol) in tetrahydrofuran (3 mL) was treated with a 3N aqueous hydrochloric acid solution (3 mL). The resulting reaction mixture was heated under reflux at 60°C for 4 h. After such time, the reaction was cooled to 25°C, diluted with water (5 mL), and then extracted with ethyl acetate (3 x 20 mL). The combined organic layers were dried over sodium sulfate, filtered, and concentrated in *vacuo*. Flash chromatography (Merck Silica gel 60, 230-400 mesh, 90/10 hexanes/ethyl acetate with 1% acetic acid) afforded 6-[3-cyclopentyl-2(R)-(3,4-dichloro-phenyl)-propionylamino]-nicotinic acid (8 mg, 4%) as a white solid: [α]²³₅₈₉ = -41.4° (c=0.099, chloroform); FAB-HRMS m/e calcd for C₂₀H₂₀Cl₂N₂O₃ (M+H)⁺ 407.0930, found 407.0928.

### Example 105

### 6-[3-Cyclopentyl-2-(3,4-dichloro-phenyl)-propionylamino]-N-methyl-nicotinamide

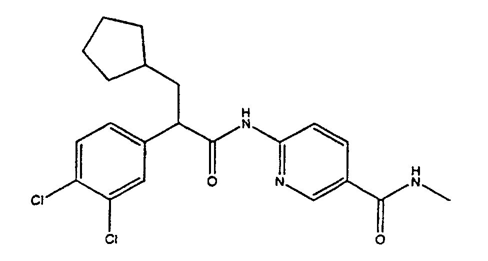

A solution of 6-[3-cyclopentyl-2-(3,4-dichloro-phenyl)-propionylamino]-nicotinic acid (prepared in Example **46**, 125 mg, 0.31 mmol), *N,N*-diisopropylethylamine (0.10 mL, 0.61 mmol), and benzotriazol-1-yloxy-tris(dimethylamino)phosphonium hexafluorophosphate (142 mg, 0.32 mmol) in *N,N*-dimethylformamide (15 mL) at 25°C was treated dropwise with a 2.0M solution of methylamine in tetrahydrofuran (0.16 mL, 0.32 mmol). The resulting reaction mixture was stirred at 25°C for 16 h. The reaction mixture was then diluted with water (10 mL) and extracted with ethyl acetate (3 x 10 mL). The combined organic layers were dried over sodium sulfate, filtered, and concentrated *in vacuo*. Flash chromatography (Merck Silica gel 60, 230-400 mesh, 50/50 hexanes/ethyl acetate) afforded 6-[3-cyclopentyl-2-(3,4-dichloro-phenyl)-propionylamino]-N-methyl-nicotinamide (83 mg, 64%) as white solid: mp 229.1-231.7°C; FAB-HRMS m/e calcd for C₂₁H₂₃Cl₂N₃O₂ (M+H)⁺ 420.1245, found 420.1247.

### Example 106

### 3-Cyclopentyl-2-(3,4-dichloro-phenyl)-N-pyrazin-2-yl-propionamide

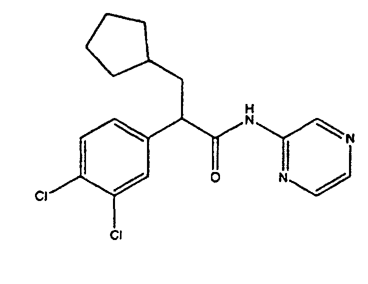

A solution of 3-cyclopentyl-2-(3,4-dichloro-phenyl)-propionic acid (prepared in Example **38**, 100 mg, 0.35 mmol) in methylene chloride (5 mL) and one drop of *N,N*-dimethylformamide was cooled to 0°C and then treated with a 2.0M solution of oxalyl chloride in methylene chloride (0.20 mL, 0.39 mmol). Gas evolution began immediately. The reaction mixture was stirred for 30 min at 0°C. After this time, the reaction mixture was treated with a solution of *N,N*-diisopropylethylamine (0.15 mL, 0.84 mmol) and aminopyrazine (69 mg, 0.73 mmol) in tetrahydrofuran (4 mL) in one portion. The resulting reaction mixture was stirred for 16 h at 25°C. The reaction mixture was then diluted with water (10 mL) and extracted with methylene chloride (3 x 15 mL). The combined organic layers were dried over sodium sulfate, filtered, and concentrated *in vacuo*. Flash chromatography (Merck Silica gel 60, 230-400 mesh, 90/10 hexanes/ethyl acetate) afforded 3-cyclopentyl-2-(3,4-dichloro-phenyl)-N-pyrazin-2-yl-propionamide (38 mg, 30%) as a yellow solid: mp 46.5-51.3°C; EI-HRMS m/e calcd for C₁₈H₁₉Cl₂N₃O (M⁺) 363.0905, found 363.0907.

### Example 107

### N-(5-Bromo-pyridin-2-yl)-3-cyclopentyl-2(R)-(3,4-dichloro-phenyl)-propionamide

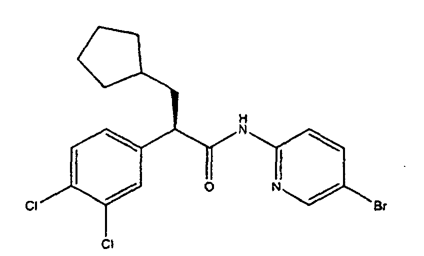

A solution of triphenylphosphine (411 mg, 1.57 mmol) in methylene chloride (15 mL) was cooled to 0°C and then treated with *N*-bromosuccinimide (316 mg, 1.78 mmol). The reaction mixture was stirred at 0°C until it was completely dissolved and became light purple in color. The reaction mixture was then treated with 3-cyclopentyl-2(R)-(3,4-dichloro-phenyl)-propionic acid (prepared in Example **54,** 300 mg, 1.05 mmol). The reaction mixture was stirred at 0°C for 20 min and then warmed to 25°C where it was stirred for 30 min. After such time, the reaction mixture was treated with 2-amino-5-bromopyridine (271 mg, 1.57 mmol) and pyridine (0.15 mL, 1.88 mmol). The resulting reaction mixture was stirred at 25°C for 16 h. The reaction was then diluted with water (10 mL) and extracted with methylene chloride (3 x 15 mL). The combined organic layers were dried over sodium sulfate, filtered, and concentrated *in vacuo*. Flash chromatography (Merck Silica gel 60, 230-400 mesh, 90/10 hexanes/ethyl acetate) afforded N-(5-bromo-pyridin-2-yl)-3-cyclopentyl-2(R)-(3,4-dichloro-phenyl)-propionamide (448 mg, 97%) as a white solid: mp 107.3-109.9°C; [α]²³₅₈₉ = -66.7° (c=0.084, chloroform); EI-HRMS m/e calcd for C₁₉H₁₉BrCl₂N₂O (M⁺) 440.0058, found 440.0056.

### Example 108

### 3-Cyclopentyl-2(R)-(3,4-dichloro-phenyl)-N-(5-hydroxymethyl-pyridin-2-yl) propionamide

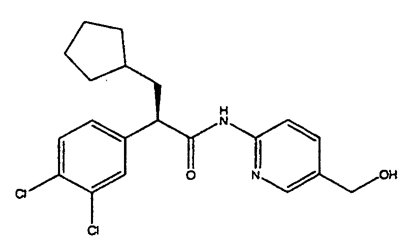

A solution of 6-[3-cyclopentyl-2(R)-(3,4-dichloro-phenyl)-propionylamino]-nicotinic acid methyl ester (prepared in Example **45,** 398 mg, 0.95 mmol) in diethyl ether (30 mL) was cooled to 0°C and then treated with lithium aluminum hydride (54 mg, 1.4 mmol) in one portion. There was immediate gas evolution. The reaction mixture was allowed to slowly warm to 25°C and was stirred at 25°C 16 h. After such time, the reaction mixture was diluted with water (10 mL) and then extracted with ethyl acetate (3 x 15 mL). The combined organic layers were dried over sodium sulfate, filtered, and concentrated *in vacuo*. Flash chromatography (Merck Silica gel 60, 230-400 mesh, 90/10 hexanes/ethyl acetate) afforded 3-cyclopentyl-2-(3,4-dichloro-phenyl)-N-(5-hydroxymethyl-pyridin-2-yl) propionamide (131 mg, 35%) as a white foam: FAB-HRMS m/e calcd for C₂₀H₂₂Cl₂N₂O₂ (M+H)⁺ 392.1058, found 392.1062.

### Example 109

### 3-Cycloheptyl-2-(4-methanesulfonyl-phenyl)-N-thiazol-2-yl-propionamide

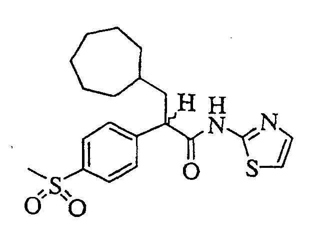

A mixture of magnesium metal (4.81 g, 200 mmol) and dry tetrahydrofuran (10 mL) under argon was treated with a solution of 1,2-dibromoethane (0.94 g, 5 mmol) in dry tetrahydrofuran (5 mL). The resulting reaction mixture was stirred for 10 min to activate the magnesium metal. The reaction mixture was then treated dropwise with a solution of cycloheptyl bromide (17.7 g, 100 mmol) in dry tetrahydrofuran (30 mL), one-fifth portion over a period of 5 min. The resulting reaction mixture was stirred for 5-10 min to initiate the exothermic reaction. The remaining portion of the cycloheptyl bromide solution was then added dropwise while controlling the inside temperature below 50°C. After complete addition, the solution was stirred for 1 h and then diluted with dry tetrahydrofuran (80 mL). In a separate reaction flask, a mixture of lithium chloride (8.48 g, 200 mmol, predried at 130°C under high vacuum for 3 h) and copper(I) cyanide (8.96 g, 100 mmol) in dry tetrahydrofuran (110 mL) was stirred at 25°C under argon for 10 min to obtain a clear solution. The reaction mixture was cooled to -70°C and then slowly treated with the freshly prepared cycloheptylmagnesium bromide. After the addition, the reaction mixture was allowed to warm to -10°C where it was stirred for 5 min. The resulting reaction mixture was again cooled back to -70°C and then treated with methyl propiolate (7.57 g, 90 mmol). The reaction mixture was stirred for 15 h at -70°C to -50°C and then slowly treated with a solution of iodine (34.3 g, 135 mmol) in dry tetrahydrofuran (30 mL), with the temperature kept at -70°C to -60°C. After addition of the iodine solution, the cooling bath was removed, and the reaction mixture was allowed to warm to 25°C where it was stirred for 2 h. The reaction mixture was then poured into a solution consisting of a saturated aqueous ammonium chloride solution (400 mL) and ammonium hydroxide (100 mL), and the organic compound was extracted into ethyl acetate (3 x 200 mL). The combined organic extracts were successively washed with a saturated aqueous sodium thiosulfate solution (1 x 400 mL) and a saturated aqueous sodium chloride solution (1 x 400 mL). The organic layer was then dried over anhydrous magnesium sulfate, filtered, and concentrated *in vacuo*. Flash chromatography (Merck Silica gel 60, 230-400 mesh, 20/1 to 10/1 hexanes/diethyl ether) afforded (E)-3-cycloheptyl-2-iodo-acrylic acid methyl ester (17.86 g, 64%) as a colorless oil: EI-HRMS m/e calcd for C₁₁H₁₇IO₂ (M⁺) 308.0273, found 308.0273.

A mixture of zinc dust (2.6 g, 40 mmol, Aldrich, -325 mesh) and dry tetrahydrofuran (3 mL) under argon was treated with 1,2-dibromoethane (0.38 g, 2 mmol). The zinc suspension was then heated with a heat gun to ebullition, allowed to cool, and heated again. This process was repeated three times to make sure the zinc dust was activated. The activated zinc dust suspension was then treated with trimethylsilyl chloride (220 mg, 2 mmol), and the suspension was stirred for 15 min at 25°C. The reaction mixture was then treated dropwise with a solution of (E)-3-cycloheptyl-2-iodo-acrylic acid methyl ester (6.16 g, 20 mmol) in dry tetrahydrofuran (5 mL) over 10 min. The reaction mixture was then stirred at 40-45°C for 1 h and then stirred overnight at 25°C. The reaction mixture was then diluted with dry tetrahydrofuran (10 mL), and the stirring was stopped to allow the excess zinc dust to settle down (∼2 h). In a separate reaction flask, bis(dibenzylideneacetone)palladium(0) (270 mg, 0.5 mmol) and triphenylphosphine (520 mg, 2 mmol) in dry tetrahydrofuran (25 mL) was stirred at 25°C under argon for 10 min and then treated with 4-bromophenyl methyl sulfone (4.23 g, 18 mmol) and the freshly prepared zinc compound in tetrahydrofuran. The resulting brick red solution was heated at 50°C for 24 h. The reaction mixture was cooled to 25°C and then poured into a saturated aqueous ammonium chloride solution (150 mL), and the organic compound was extracted into ethyl acetate (3 x 150 mL). The combined organic extracts were washed with a saturated aqueous sodium chloride solution (1 x 300 mL), dried over anhydrous magnesium sulfate, filtered, and concentrated *in vacuo*. Flash chromatography (Merck Silica gel 60, 230-400 mesh, 4/1 to 1/1 hexanes/ethyl acetate) afforded (E)-3-cycloheptyl-2-(4-methanesulfonyl-phenyl)-acrylic acid methyl ester (6.01 g, 99%) as a viscous yellow oil: EI-HRMS m/e calcd for C₁₈H₂₄O₄S (M⁺) 336.1395, found 336.1395.

A solution of nickel(II) chloride hexahydrate (7.8 mg, 0.033 mmol) and (E)-3-cycloheptyl-2-(4-methanesulfonyl-phenyl)-acrylic acid methyl ester (111 mg, 0.33 mmol) in methanol (3 mL) was cooled to 0°C and then treated with sodium borohydride (25 mg, 0.66 mmol) in two portions. After the addition, the black reaction mixture was stirred for 15 min at 0°C and then allowed to warm to 25°C where it was stirred for 15 h. The black solid was filtered using filter paper and washed with methanol. The combined solvents were concentrated *in vacuo*, and the residue was diluted with water (25 mL) and ethyl acetate (25 mL). The two layers were separated, and the aqueous layer was extracted with ethyl acetate (1 x 15 mL). The combined organic extracts were washed with a saturated aqueous sodium chloride solution (1 x 50 mL), dried over anhydrous magnesium sulfate, filtered, and concentrated *in vacuo* to afford racemic 3-cycloheptyl-2-(4-methanesulfonyl-phenyl)-propionic acid methyl ester (101 mg, 91%) as a colorless oil: EI-HRMS m/e calcd for C₁₈H₂₆O₄S (M⁺) 338.1552, found 338.1555.

A solution of 3-cycloheptyl-2-(4-methanesulfonyl-phenyl)-propionic acid methyl ester (95 mg, 0.28 mmol) in ethanol (2 mL) was treated with a 1N aqueous sodium hydroxide solution (1.5 mL). The solution was heated at 45-50°C for 15 h, at which time, thin layer chromatography analysis of the reaction mixture indicated the absence of starting material. The reaction mixture was concentrated *in vacuo* to remove ethanol. The residue was diluted with water (10 mL) and extracted with diethyl ether (1 x 20 mL) to remove any neutral impurities. The aqueous layer was then acidified with a 1N aqueous hydrochloric acid solution, and the resulting acid was extracted into ethyl acetate (2 x 15 mL). The combined organic layers were washed with a saturated aqueous sodium chloride solution (1 x 50 mL), dried over anhydrous magnesium sulfate, filtered, and concentrated *in vacuo* to afford 3-cycloheptyl-2-(4-methanesulfonyl-phenyl)-propionic acid (78 mg, 86%) as a white solid: EI-HRMS m/e calcd for C₁₇H₂₄O₄S (M+H)⁺ 325.1474, found 325.1478.

A solution of triphenylphosphine (116 mg, 0.44 mmol) in methylene chloride (2 mL) was cooled to 0°C and then treated with *N*-bromosuccinimide (78 mg, 0.44 mmol). The reaction mixture was stirred at 0°C for 30 min and then treated with a solution of 3-cycloheptyl-2-(4-methanesulfonyl-phenyl)-propionic acid (72 mg, 0.22 mmol) in methylene chloride (2 mL). The clear solution was stirred for 10 min at 0°C and then allowed to warm to 25°C where it was stirred for 1.5 h. The reaction mixture was then treated with 2-aminothiazole (66 mg, 0.66 mmol), and the resulting suspension was stirred for 20 h at 25°C. The reaction mixture was then concentrated *in vacuo* to remove methylene chloride, and the residue was diluted with ethyl acetate (30 mL) and a 1N aqueous hydrochloric acid solution (30 mL). The two layers were separated, and the aqueous layer was extracted with ethyl acetate (1 x 10 mL). The combined organic extracts were successively washed with a saturated aqueous sodium bicarbonate solution (1 x 20 mL) and a saturated aqueous sodium chloride solution (1 x 30 mL), dried over anhydrous magnesium sulfate, filtered, and concentrated *in vacuo*. Biotage chromatography (FLASH 40S, Silica, 4/1 to 1/1 hexanes/ethyl acetate) afforded 3-cycloheptyl-2-(4-methanesulfonyl-phenyl)-N-thiazol-2-yl-propionamide (68 mg, 76%) as an amorphous solid: EI-HRMS m/e calcd for C₂₀H₂₆N₂O₃S₂ (M⁺) 406.1426, found 406.1424.

### Example 110

### 3-Cyclohexyl-2-(4-methanesulfonyl-phenyl)-N-thiazol-2-yl-propionamide

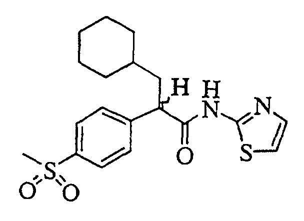

A mixture of zinc dust (16.34 g, 250 mmol, Aldrich, -325 mesh) and dry tetrahydrofuran (6 mL) under argon was treated with 1,2-dibromoethane (0.94 g, 5 mmol). The zinc suspension was then heated with a heat gun to ebullition, allowed to cool, and heated again. This process was repeated three times to make sure the zinc dust was activated. The activated zinc dust suspension was then treated with trimethylsilyl chloride (0.54 g, 5 mmol), and the suspension was stirred for 15 min at 25°C. The reaction mixture was then treated dropwise with a solution of cyclohexyl iodide (21 g, 100 mmol) in dry tetrahydrofuran (30 mL) over 15 min. During the addition, the temperature rose to 60°C. The reaction mixture was then stirred for 3 h at 40-45°C. The reaction mixture was then cooled to 25°C and diluted with dry tetrahydrofuran (60 mL). The stirring was stopped to allow the excess zinc dust to settle down (-3 h). In a separate reaction flask, a mixture of lithium chloride (8.48 g, 200 mmol, predried at 130°C under high vacuum for 3 h) and copper(I) cyanide (8.95 g, 100 mmol) in dry tetrahydrofuran (110 mL) was stirred for 10 min at 25°C to obtain a clear solution. The reaction mixture was cooled to -70°C and then slowly treated with the freshly prepared zinc solution using a syringe. After the addition, the reaction mixture was allowed to warm to 0°C where it was stirred for 5 min. The reaction mixture was again cooled back to -70°C and then slowly treated with methyl propiolate (7.56 g, 90 mmol). The resulting reaction mixture was stirred for 15 h at -70°C to -50°C and then slowly treated with a solution of iodine (34.26 g, 135 mmol) in dry tetrahydrofuran (30 mL), with the temperature kept at -70°C to -60°C. After addition of the iodine solution, the cooling bath was removed, and the reaction mixture was allowed to warm to 25°C where it was stirred for 2 h. The reaction mixture was then poured into a solution consisting of a saturated aqueous ammonium chloride solution (400 mL) and ammonium hydroxide (100 mL), and the organic compound was extracted into ethyl acetate (3 x 250 mL). The combined organic extracts were successively washed with a saturated aqueous sodium thiosulfate solution (1 x 500 mL) and a saturated aqueous sodium chloride solution (1 x 500 mL), dried over anhydrous magnesium sulfate, filtered, and concentrated *in vacuo*. Flash chromatography (Merck Silica gel 60, 230-400 mesh, 9/1 hexanes/diethyl ether) afforded (E)-3-cyclohexyl-2-iodo-acrylic acid methyl ester (26.3 g, 99%) as a light pink oil: EI-HRMS m/e calcd for C₁₀H₁₅IO₂ (M⁺) 294.0117, found 294.0114.

A mixture of zinc dust (2.6 g, 40 mmol, Aldrich, -325 mesh) and dry tetrahydrofuran (3 mL) under argon was treated with 1,2-dibromoethane (0.37 g, 2 mmol). The zinc suspension was then heated with a heat gun to ebullition, allowed to cool, and heated again. This process was repeated three times to make sure the zinc dust was activated. The activated zinc dust suspension was then treated with trimethylsilyl chloride (217 mg, 2 mmol), and the suspension was stirred for 15 min at 25°C. The reaction mixture was then treated dropwise with a solution of (E)-3-cyclohexyl-2-iodo-acrylic acid methyl ester (5.88 g, 20 mmol) in dry tetrahydrofuran (5 mL) over 5 min. During the addition, the temperature rose to 50°C. The reaction mixture was then stirred at 40-45°C for 1 h and then stirred overnight at 25°C. The reaction mixture was then diluted with dry tetrahydrofuran (10 mL), and the stirring was stopped to allow the excess zinc dust to settle down (-2 h). In a separate reaction flask, bis(dibenzylideneacetone)palladium(0) (270 mg, 0.5 mmol) and triphenylphosphine (520 mg, 2 mmol) in dry tetrahydrofuran (25 mL) was stirred at 25°C under argon for 10 min and then treated with 4-bromophenyl methyl sulfone (4.23 g, 18 mmol) and the freshly prepared zinc compound in tetrahydrofuran. The resulting brick red solution was heated at 50°C for 24 h, at which time, thin layer chromatography analysis of the reaction mixture indicated the absence of starting material. The reaction mixture was cooled to 25°C and then poured into a saturated aqueous ammonium chloride solution (150 mL), and the organic compound was extracted into ethyl acetate (3 x 100 mL). The combined organic extracts were washed with a saturated aqueous sodium chloride solution (1 x 200 mL), dried over anhydrous magnesium sulfate, filtered, and concentrated *in vacuo*. Flash chromatography (Merck Silica gel 60, 230-400 mesh, 3/2 hexanes/ethyl acetate) afforded (E)-3-cyclohexyl-2-(4-methanesulfonyl-phenyl)-acrylic acid methyl ester (5.79 g, 99%) as a low melting white solid: EI-HRMS m/e calcd for C₁₇H₂₂O₄S (M⁺) 322.1238, found 322.1236.

A solution of nickel(II) chloride hexahydrate (157 mg, 0.66 mmol) and (E)-3-cyclohexyl-2-(4-methanesulfonyl-phenyl)-acrylic acid methyl ester (1.07 g, 3.31 mmol) in methanol (30 mL) was cooled to 0°C and then treated with sodium borohydride (380 mg, 10 mmol) in four portions. After the addition, the black reaction mixture was stirred for 15 min at 0°C and then allowed to warm to 25°C where it was stirred for 15 h. The black solid was filtered using filter paper and washed with methanol. The combined solvents were concentrated *in vacuo*, and the residue was diluted with water (50 mL) and ethyl acetate (50 mL). The two layers were separated, and the aqueous layer was extracted with ethyl acetate (1 x 25 mL). The combined organic extracts were washed with a saturated aqueous sodium chloride solution (1 x 50 mL), dried over anhydrous magnesium sulfate, filtered, and concentrated *in vacuo* to afford racemic 3-cyclohexyl-2-(4-methanesulfonyl-phenyl)-propionic acid methyl ester (1.04 g, 97%) as an amorphous white solid: EI-HRMS m/e calcd for C₁₇H₂₄O₄S (M⁺) 324.1395, found 324.1395.

A solution of 3-cyclohexyl-2-(4-methanesulfonyl-phenyl)-propionic acid methyl ester (1.00 g, 3.08 mmol) in ethanol (15 mL) was treated with a 1N aqueous sodium hydroxide solution (6 mL). The solution was heated at 45-50°C for 15 h, at which time, thin layer chromatography analysis of the mixture indicated the absence of starting material. The reaction mixture was then concentrated in *vacuo* to remove ethanol, and the residue was diluted with water (20 mL) and extracted with diethyl ether (1 x 40 mL) to remove any neutral impurities. The aqueous layer was acidified with a 1N aqueous hydrochloric acid solution. The resulting acid was extracted into ethyl acetate (2 x 50 mL). The combined organic layers were washed with a saturated aqueous sodium chloride solution (1 x 50 mL), dried over anhydrous magnesium sulfate, filtered, and concentrated *in vacuo* to afford 3-cyclohexyl-2-(4-methanesulfonyl-phenyl)-propionic acid (570 mg, 60%) as a white solid: mp 139-143°C; EI-HRMS m/e calcd for C₁₆H₂₂O₄S (M⁺) 310.1239, found 310.1241.

A solution of triphenylphosphine (416 mg, 1.58 mmol) in methylene chloride (8 mL) was cooled to 0°C and then treated with *N*-bromosuccinimide (281 mg, 1.58 mmol). The reaction mixture was stirred at 0°C for 30 min and then treated with a solution of 3-cyclohexyl-2-(4-methanesulfonyl-phenyl)-propionic acid (290 mg, 0.93 mmol) in methylene chloride (5 mL). The clear solution was stirred for 15 min at 0°C and then allowed to warm to 25°C where it was stirred for 1.5 h. The reaction mixture was then treated with 2-aminothiazole (233 mg, 2.32 mmol), and the resulting suspension was stirred for 20 h at 25°C. The reaction mixture was concentrated *in vacuo* to remove methylene chloride, and the residue was diluted with ethyl acetate (50 mL) and a 1N aqueous hydrochloric acid solution (50 mL). The two layers were separated, and the aqueous layer was extracted with ethyl acetate (1 x 30 mL). The combined organic extracts were successively washed with a saturated aqueous sodium bicarbonate solution (1 x 50 mL) and a saturated aqueous sodium chloride solution (1 x 50 mL), dried over anhydrous magnesium sulfate, filtered, and, concentrated *in vacuo*. Biotage chromatography (FLASH 40S, Silica, 4/1 to 1/1 hexanes/ethyl acetate) afforded 3-cyclohexyl-2-(4-methanesulfonyl-phenyl)-N-thiazol-2-yl-propionamide (337 mg, 92%) as an amorphous solid: EI-HRMS m/e calcd for C₁₉H₂₄N₂O₃S₂ (M⁺) 392.1228, found 392.1230.

### Example 111

### 3-Cyclopentyl-2-(3-nitrophenyl)-N-thiazol-2-yl-propionamide

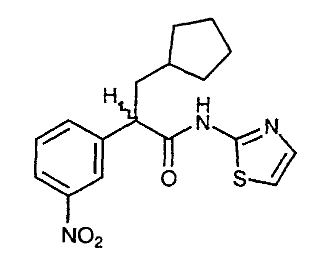

A solution of (3-nitro-phenyl)-acetic acid (5.0 g, 27.6 mmol) in methanol (50 mL) was treated with a catalytic amount of sulfuric acid. The reaction mixture was refluxed for 48 h. The reaction was then concentrated *in vacuo*. The residue was dissolved in methylene chloride (50 mL) and washed with a saturated aqueous sodium bicarbonate solution (2 x 25 mL), water (1 x 50 mL) and a saturated aqueous sodium chloride solution (1 x 50 mL). The combined organic layers were dried over sodium sulfate, filtered, and concentrated *in vacuo* to give (4-nitro-phenyl)-acetic acid methyl ester (5.27 g, 97.9%) as a pale yellow solid: mp 29-30°C; EI-HRMS m/e calcd for C₉H₉NO₄ (M⁺) 195.0531, found 195.0532.

A solution of freshly prepared lithium diisopropylamide (43.3 mL of a 0.3M stock solution, 12.99 mmol) cooled to -78°C was treated with (3-nitro-phenyl)-acetic acid methyl ester (2.45 g, 12.56 mmol) in tetrahydrofuran/1,3-dimethyl-3,4,5,6-tetrahydro-2(1H)-pyrimidinone (32 mL, 3:1). The resulting solution was stirred at -78°C for 45 min. At this time, the reaction was treated with a solution of iodomethylcyclopentane (2.78 g, 13.23 mmol) in 1,3-dimethyl-3,4,5,6-tetrahydro-2(1H)-pyrimidinone (2.78 mL) and the mixture was stirred at -78°C for 3 h. The reaction was warmed to 25°C and was stirred at 25°C for 16 h. The reaction mixture was then quenched by the dropwise addition of a saturated aqueous ammonium chloride solution (25 mL) and was concentrated *in vacuo*. The residue was diluted with water (50 mL) and extracted with ethyl acetate (3 x 50 mL). The organics were washed with a saturated aqueous lithium chloride solution (2 x 25 mL), dried over sodium sulfate, filtered, and concentrated *in vacuo.* Flash chromatography (Merck Silica gel 60, 230-400 mesh 80/20 hexanes/ ethyl acetate) afforded 3-cyclopentyl-2-(3-nitro-phenyl)-propionic acid methyl ester (1.63 g, 46.8%) as pale yellow oil: EI-HRMS m/e calcd for C₁₅H₁₉NO₄ (M⁺) 277.1314, found 277.1317.

A solution of 3-cyclopentyl-2-(3-nitro-phenyl)-propionic acid methyl ester (0.55 g, 2.0 mmol) in tetrahydrofuran/water (12 mL, 3:1) was treated with lithium hydroxide (185 mg, 4.40 mmol). The reaction was stirred at 25°C for 48 h. The tetrahydrofuran was then removed *in vacuo*. The residue was diluted with water (25 mL) and extracted with ether (1 x 20 mL). The aqueous layer was acidified to pH = 2 with a 3N aqueous hydrochloric acid solution. The solution was extracted with methylene chloride (3 x 25 mL). The organics were washed with a saturated aqueous sodium chloride solution (2 x 25 mL), dried over sodium sulfate, filtered, and concentrated *in vacuo* to give 3-cyclopentyl-2-(3-nitro-phenyl)-propionic acid (0.48 g, 91.9%) as a tan solid: mp 95-99°C; EI-HRMS m/e calcd for C₁₄H₁₇NO₄ (M⁺) 263.1157, found 263.1156.

A solution of 3-cyclopentyl-2-(3-nitro-phenyl)-propionic acid (432 mg, 1.64 mmol) in methylene chloride (16 mL) was cooled to 0°C and then treated with a 2.0M solution of oxalyl chloride in methylene chloride (0.90 mL, 1.80 mmol) and a few drops of *N*,*N*-dimethylformamide. The reaction mixture was stirred at 0°C for 15 min and at 25°C for 1.2 h. The reaction mixture was then treated with a solution of 2-aminothiazole (361.4 mg, 3.61 mmol) and *N,N*-diisopropylethylamine (0.70 mL, 3.93 mmol) in tetrahydrofuran (16 mL). The reaction mixture was stirred at 25°C for 6 h. At this time, the reaction was concentrated *in vacuo*. Flash chromatography (Merck Silica gel 60, 230-400 mesh 70/30 hexanes/ethyl acetate) afforded 3-cyclopentyl-2-(nitrophenyl)-N-thiazol-2-yl-propionamide (409.3 mg, 72.2%) as a tan solid: mp 171-174°C; EI-HRMS m/e calcd for C₁₇H₁₉N₃O₃S (M⁺) 345.1147, found 345.1153.

### Example 112

### 3-Cyclopentyl-2-(3-methoxy-phenyl)-N-thiazol-2-yl-propionamide

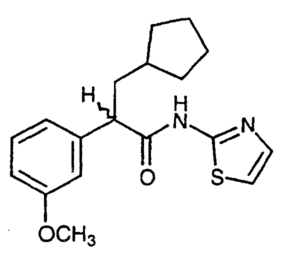

A solution of freshly prepared lithium diisopropylamide (23 mL of a 0.31M stock solution, 7.13 mmol) cooled to -78°C was treated with (3-methoxy-phenyl)-acetic acid methyl ester (1.07 g, 5.94 mmol) in tetrahydrofuran/1,3-dimethyl-3,4,5,6-tetrahydro-2(1H)-pyrimidinone (14.8 mL, 3:1). The resulting solution was stirred at -78°C for 45 min. At this time, the reaction was treated with a solution of iodomethylcyclopentane (1.37 g, 6.53 mmol) in 1,3-dimethyl-3,4,5,6-tetrahydro-2(1H)-pyrimidinone (1.16 mL). The reaction mixture was stirred at -78°C for 3 h. The reaction was then warmed to 25°C and was stirred at 25°C for 16 h. At this time, the reaction was quenched by the dropwise addition of a saturated aqueous ammonium chloride solution. This solution was diluted with water (100 mL) and extracted into ethyl acetate (3 x 50 mL). The organics were washed with a saturated aqueous lithium chloride solution (1 x 75 mL), dried over sodium sulfate, filtered, and concentrated *in vacuo*. Flash chromatography (Merck Silica gel 60, 230-400 mesh, 95/5 hexanes/ethyl acetate) afforded 3-cyclopentyl-2-(3-methoxy-phenyl)-propionic acid methyl ester (1.39 g, 89.1%) as a clear oil: EI-HRMS m/e calcd for C₁₆H₂₂O₃ (M⁺) 262.1568, found 262.1561.

A solution of 3-cyclopentyl-2-(3-methoxy-phenyl)-propionic acid methyl ester (1.39 g, 5.29 mmol) in tetrahydrofuran/water/methanol (13.2 mL, 3:1:1) at 25°C was treated with a 2N aqueous sodium hydroxide solution (3.97 mL, 7.94 mmol). The reaction was stirred at 25°C for 48 h. At this time, the reaction mixture was poured into water (50 mL) and extracted with chloroform (3 x 25 mL). The aqueous layer was acidified to pH = 1 with a 1N aqueous hydrochloric acid solution. The aqueous layer was extracted with a solution of chloroform/methanol (9:1). The organics were dried over sodium sulfate, filtered, and concentrated in vacuo. Flash chromatography (Merck Silica gel 60, 230-400 mesh, 70/30 hexanes/ethyl acetate with glacial acetic acid) afforded 3-cyclopentyl-2-(3-methoxy-phenyl)-propionic acid (1.05 g, 79.8%) as a clear wax: EI-HRMS m/e calcd for C₁₅H₂₀O₃ (M⁺) 248.1412, found 248.1409.

A solution of 3-cyclopentyl-2-(3-methoxy-phenyl)-propionic acid (500 mg, 2.0 mmol) in methylene chloride (20 mL) cooled to 0°C was treated with a 2.0M solution of oxalyl chloride in methylene chloride (1.1 mL, 2.20 mmol) and a few drops of *N*,*N*-dimethylformamide. The reaction mixture was stirred at 0°C for 10 min then at 25°C for 30 min. The reaction mixture was then treated with 2-aminothiazole (444 mg, 4.42 mmol) and *N*,*N-*diisopropylethylamine (0.84 mL, 4.83 mmol) in tetrahydrofuran (10.1 mL). This solution was stirred at 25°C for 18 h. At this time, the reaction was concentrated *in vacuo*. Flash chromatography (Merck Silica gel 60, 230-400 mesh, 80/20 hexanes/ethyl acetate) afforded 3-cyclopentyl-2-(3-methoxy-phenyl)-N-thiazol-2-yl-propionamide (549mg, 82.6%) as a white solid: mp 44-45°C; EI-HRMS m/e calcd for C₁₈H₂₂N₂O₂S (M⁺) 330.1402 found 330.1398.

### Example 113

### 3-Cyclopentyl-2-(3-hydroxy-phenyl)-N-thiazol-2-yl-propionamide

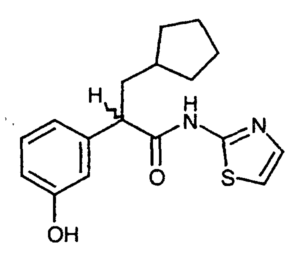

A 1.0M solution of boron tribromide in methylene chloride (3.53 mL, 3.53 mmol) at 25°C was treated with a solution of 3-cyclopentyl-2-(3-methoxy-phenyl)-N-thiazol-2-yl-propionamide (prepared in Example **112,** 0.11 g, 0.35 mmol) in methylene chloride (3.5 mL). This solution was stirred at 25°C for 1 h. At this time, the reaction was cooled to 0°C and treated with a dilute aqueous ammonium hydroxide solution. This mixture was stirred at 0°C for 15 min. At this time, the aqueous layer was separated from the organic layer. The aqueous layer was extracted with chloroform (3 x 50 mL). The organics were dried over sodium sulfate, filtered, and concentrated *in vacuo*. Flash chromatography (Merck Silica gel 60, 230-400 mesh, 50/50 hexanes/ethyl acetate) afforded 3-cyclopentyl-2-(3-hydroxy-phenyl)-N-thiazol-2-yl-propionamide (50 mg, 44.7%) as a white solid: mp 177-179°C; EI-HRMS m/e calcd for C₁₇H₂₀N₂O₂S (M⁺) 316.1245 found 316.1244.

### Example 114

### 3-Cyclopentyl-N-thiazol-2-yl-2-(4-trifluoromethoxy-phenyl)-propionamide

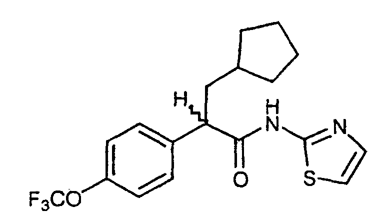

A solution of freshly prepared lithium diisopropylamide (23 mL of a 0.31M stock solution, 7.13 mmol) cooled to -78°C was treated with (4-trifluoromethoxy-phenyl)-acetic acid (0.74 g, 3.39 mmol) in tetrahydrofuran/ 1,3-dimethyl-3,4,5,6-tetrahydro-2(1H)-pyrimidinone (8.5 mL, 3:1). The resulting solution was stirred at -78°C for 45 min. At this time, the reaction was treated with a solution of iodomethylcyclopentane (0.78 g, 3.73 mmol) in 1,3-dimethyl-3,4,5,6-tetrahydro-2(1H)-pyrimidinone (1 mL). The reaction mixture was stirred at -78°C for 4 h. The reaction was then warmed to 25°C and was stirred at 25°C for 18 h. The reaction mixture was then quenched by the dropwise addition of saturated aqueous ammonium chloride solution (10 mL). The resulting mixture was concentrated *in vacuo* to remove the excess solvent. The residue was diluted with water (100 mL) and acidified to pH = 1 with a 1N aqueous hydrochloric acid solution. This solution was extracted with ethyl acetate (3 x 50 mL). The organics were washed with a saturated aqueous lithium chloride solution (1 x 100 mL), dried over sodium sulfate, filtered, and concentrated *in vacuo*. Flash chromatography (Merck Silica gel 60, 230-400 mesh, 50/50 hexanes/ethyl acetate) afforded 3-cyclopentyl-2-(4-trifluoromethoxy-phenyl)-propionic acid (0.31 g, 30.6%) as a tan solid: mp 62-64°C; EI-HRMS m/e calcd for C₁₅H₁₇F₃O₃ (M⁺) 302.1129 found 302.1131.

A solution of 3-cyclopentyl-2-(4-trifluoromethoxy-phenyl)-propionic acid (0.16 g, 0.52 mmol) in methylene chloride (5.3 mL) cooled to 0°C was treated with a 2.0M solution of oxalyl chloride in methylene chloride (0.29 mL, 0.58 mmol) and a few drops of *N*,*N*-dimethylformamide. The reaction mixture was stirred at 0°C for 10 min and at 25°C for 30 min. The reaction mixture was then treated with a solution of 2-aminothiazole (0.11 g, 1.16 mmol) and *N*,*N*-diisopropylethylamine (0.22 mL, 1.27 mmol) in tetrahydrofuran (2.65 mL). The reaction mixture was stirred at 25°C for 18 h. At this time, the reaction was concentrated *in vacuo*. Flash chromatography (Merck Silica gel 60, 230-400 mesh 80/20 hexanes/ethyl acetate) afforded 3-cyclopentyl-N-thiazol-2-yl-2-(4-trifluoromethoxy-phenyl)-propionamide (203.8 mg, 100%) as a white solid: mp 168-170°C; EI-HRMS m/e calcd for C₁₈H₁₉F₃N₂O₂S (M⁺) 384.1119, found 384.1118.

### Example 115

### 3-Cyclopentyl-2-(3,4-dimethoxy-phenyl)-N-thiazol-2-yl-propionamide

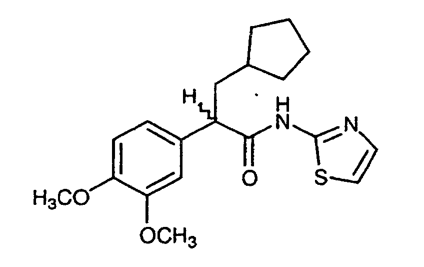

A solution of freshly prepared lithium diisopropylamide (58.5 mL of a 0.91M stock solution, 53.2 mmol) cooled to -78°C was treated with (3,4-dimethoxy-phenyl)-acetic acid (4.97 g, 25.3 mmol) in tetrahydrofuran/1,3-dimethyl-3,4,5,6-tetrahydro-2(1H)-pyrimidinone (25.3 mL, 3:1). The resulting solution was stirred at -78°C for 45 min and at 25°C for 15 min. At this time, the reaction was cooled to 0°C and was treated with a solution of iodomethylcyclopentane (5.87 g, 27.8 mmol) in 1,3-dimethyl-3,4,5,6-tetrahydro-2(1H)-pyrimidinone (1 mL). The reaction mixture was stirred at 0°C for 30 min. The reaction was then warmed to 25°C and was stirred at 25°C for 18 h. The reaction mixture was then quenched by the dropwise addition of saturated aqueous ammonium chloride solution (10 mL). The resulting mixture was concentrated *in vacuo*. The residue was diluted with water (100 mL) and acidified to pH = 1 with a 1N aqueous hydrochloric acid solution. This solution was extracted with ethyl acetate (3 x 50 mL). The organics were washed with a saturated aqueous lithium chloride solution (1 x 100 mL), dried over sodium sulfate, filtered, and concentrated *in vacuo.* Flash chromatography (Merck Silica gel 60, 230-400 mesh, 70/30 hexanes/ethyl acetate) afforded 3-cyclopentyl-2-(3,4-dimethoxy-phenyl)-propionic acid (4.5 g, 63.8%) as a yellow solid: mp 111-112°C; EI-HRMS m/e calcd for C₁₆H₂₂O₄ (M⁺) 278.1518 found 278.1517.

A solution of 3-cyclopentyl-2-(3,4-dimethoxy-phenyl)-propionic acid (0.50 g, 1.79 mmol) in methylene chloride (17.9 mL) cooled to 0°C was treated with a 2.0M solution of oxalyl chloride in methylene chloride (1.0 mL, 1.97 mmol) and a few drops of *N*,*N*-dimethylformamide. The reaction mixture was stirred at 0°C for 10 min and at 25°C for 30 min. The reaction mixture was then treated with a solution of 2-aminothiazole (0.39 g, 3.95 mmol) and *N*,*N*-diisopropylethylamine (0.76 mL, 4.3 mmol) in tetrahydrofuran (8.98 mL). The reaction mixture was stirred at 25°C for 18 h. At this time, the reaction was concentrated *in vacuo.* Flash chromatography (Merck Silica gel 60, 230-400 mesh 80/20 hexanes/ethyl acetate) afforded 3-cyclopentyl-2-(3,4-dimethoxy-phenyl)-N-thiazol-2-yl-propionamide (665 mg, 100%) as a pale yellow solid: mp 50-52°C; EI-HRMS m/e calcd for C₁₉H₂₄N₂O₃S (M⁺) 360.1507, found 360.1516.

### Example 116

### 3-Cyclopentyl-2-(3,4-dihydroxy-phenyl)-N-thiazol-2-yl-propionamide

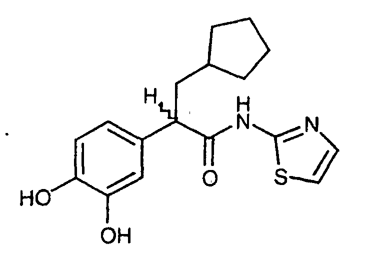

A 1.0M solution of boron tribromide in methylene chloride (7.43 mL, 7.43 mmol) at 25°C was treated with a solution of 3-cyclopentyl-2-(3,4-dimethoxy-phenyl)-N-thiazol-2-yl-propionamide (prepared in Example **115**, 0.27 g, 0.74 mmol) in methylene chloride (7.43 mL). This solution was stirred at 25°C for 1 h. At this time, the reaction was cooled to 0°C and treated with a dilute aqueous ammonium hydroxide solution. This mixture was stirred at 0°C for 20 min. At this time, the reaction was poured into water and was extracted with chloroform (3 x 50 mL). The organics were dried over sodium sulfate, filtered, and concentrated *in vacuo*. Flash chromatography (Merck Silica gel 60, 230-400 mesh, 70/30 hexanes/ethyl acetate) afforded 3-cyclopentyl-2-(3,4-dihydroxy-phenyl)-N-thiazol-2-yl-propionamide (38.8 mg, 15.7%) as a white solid: mp 170-173°C; EI-HRMS m/e calcd for C₁₇H₂₀N₂O₃S (M⁺) 332.1194 found 332.1192.

### Example 117

### 3-Cyclopentyl-2-(4-methoxy-phenyl)-N-thiazol-2-yl-propionamide

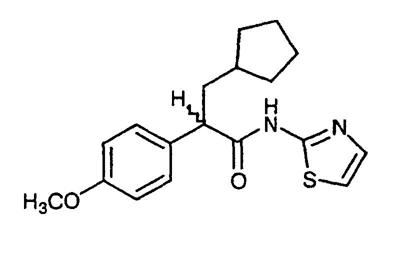

A solution of freshly prepared lithium diisopropylamide (58.5 mL of a 0.93M stock solution, 53.2 mmol) cooled to -78°C was treated with (4-methoxy-phenyl)-acetic acid (4.21 g, 25.35 mmol) in tetrahydrofuran/ 1,3-dimethyl-3,4,5,6-tetrahydro-2(1H)-pyrimidinone (25.3 mL, 3: 1). The resulting solution was stirred at -78°C for 45 min. At this time, the reaction was treated with a solution of iodomethylcyclopentane (5.85 g, 27.8 mmol) in 1,3-dimethyl-3,4,5,6-tetrahydro-2(1H)-pyrimidinone (1 mL). The reaction mixture was stirred at -78°C for 45 min and at 0°C for 1 h. The reaction was then warmed to 25°C and was stirred at 25°C for 16 h. The reaction mixture was then quenched by the dropwise addition of saturated aqueous ammonium chloride solution (10 mL). The excess solvent was removed *in vacuo*. The residue was acidified to pH = 1 with a 1N aqueous hydrochloric acid solution. This mixture was poured into water (50 mL) and extracted with ethyl acetate (3 x 50mL). The organics were washed with a saturated aqueous lithium chloride solution (1 x 100 mL), dried over sodium sulfate, filtered, and concentrated *in vacuo*. Flash chromatography (Merck Silica gel 60, 230-400 mesh, 70/30 hexanes/ethyl acetate) afforded 3-cyclopentyl-2-(4-methoxy-phenyl)-propionic acid (2.76 g, 43.8%) as a yellow solid: mp 119-121°C; EI-HRMS m/e calcd for C₁₅H₂₀O₃ (M⁺) 248.1412 found 248.1415.

A solution of 3-cyclopentyl-2-(4-methoxy-phenyl)-propionic acid (500 mg, 2.0 mmol) in methylene chloride (20.1 mL) cooled to 0°C was treated with a 2.0M solution of oxalyl chloride in methylene chloride (1.1 mL, 2.21 mmol) and a few drops of *N*,*N*-dimethylformamide. The reaction mixture was stirred at 0°C for 10 min then at 25°C for 30 min. The reaction mixture was then treated with a solution of 2-aminothiazole (444 mg, 4.42 mmol) and *N*,*N*-diisopropylethylamine (0.84 mL, 4.83 mmol) in tetrahydrofuran (10.1 mL). This solution was stirred at 25°C for 18 h. At this time, the reaction was concentrated *in vacuo*. Flash chromatography (Merck Silica gel 60, 230-400 mesh, 50/50 hexanes/ethyl acetate) afforded 3-cyclopentyl-2-(4-methoxy-phenyl)-N-thiazol-2-yl-propionamide (638 mg, 95.8%) as a pale yellow solid: mp 166-167°C; EI-HRMS m/e calcd for C₁₈H₂₂N₂O₂S (M⁺) 330.1402 found 330.1398.

### Example 118

### 3-Cyclopentyl-2-(4-hydroxy-phenyl)-N-thiazol-2-yl-propionamide

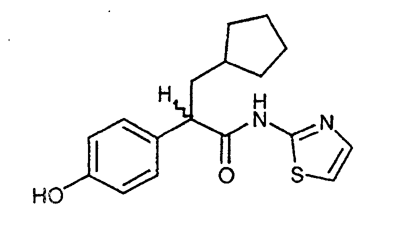

A solution 3-cyclopentyl-2-(4-methoxy-phenyl)-N-thiazol-2-yl-propionamide (1.03 g, 3.12 mmol) in methylene chloride (31.26 mL) at 25°C was treated with a 1.0M solution of boron tribromide in methylene chloride (31.26 mL, 31.26 mmol). This solution was stirred at 25°C for 4 h. At this time, the reaction was cooled to 0°C and was then quenched by the dropwise addition of a dilute aqueous ammonium hydroxide solution. The resulting solution was stirred at 0°C for 15 min. This mixture was then poured into water (50 mL) and extracted with ethyl acetate (3 x 30 mL). The organics were dried over sodium sulfate, filtered, and concentrated *in vacuo*. Flash chromatography (Merck Silica gel 60, 230-400 mesh, 70/30 hexanes/ethyl acetate) afforded 3-cyclopentyl-2-(4-hydroxy-phenyl)-N-thiazol-2-yl-propionamide (626.8 mg, 63.4%) as a off-white solid: mp 198-200°C; EI-HRMS m/e calcd for C₁₇H₂₀N₂O₂S (M⁺) 316.1245 found 316.1256.

### Example 119

### 4-[2-Cyclopentyl-1-(thiazol-2-ylcarbamoyl)-ethyl]-benzoic acid methyl ester

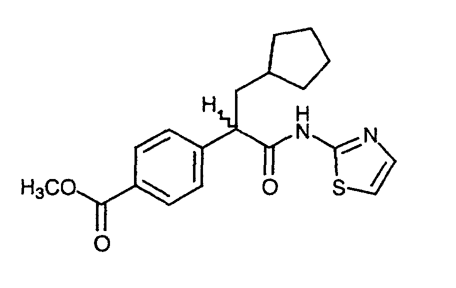

A solution of 4-methyl-benzoic acid (10 g, 73.4 mmol) in benzene (133 mL) was treated with benzoyl peroxide (72 mg, 0.29 mmol). This mixture was heated to reflux until it became homogeneous. At this time, the reaction was treated with *N*-bromosuccinimide (13 g, 73.4 mmol) and additional benzoyl peroxide (72 mg, 0.29 mmol). This mixture was heated at reflux for 2.5 h. At this time, the reaction was cooled to 25°C. The resulting precipitate was collected by filtration and washed with hot water (50 mL). The solid was taken up in water (150 mL). This slurry was heated at 80°C and then filtered while hot. The solid that was collected was dried *in vacuo* to afford 4-bromomethyl-benzoic acid (12,3, 77.9%) as a white solid: mp 224-226°C; EI-HRMS m/e calcd for C₈H₇BrO₂ (M⁺) 213.9629, found 213.9628.

A solution of 4-bromomethyl-benzoic acid (4.0 g, 18.6 mmol) in acetonitrile (186 mL) was treated with a solution of sodium cyanide (1.0 g, 20.4 mmol) and sodium hydroxide (0.74 g, 18.6 mmol) in water (24 mL). The reaction mixture was heated at reflux for 2 h. At this time, the reaction was cooled to 25°C and concentrated *in vacuo*. The resulting solution was washed with chloroform (1 x 50 mL). The aqueous layer was acidified to pH = 3 with a 1N aqueous hydrochloric acid solution. The aqueous layer was extracted with a solution of chloroform/methanol (9:1, 3 x 100 mL). The organics were dried over sodium sulfate, filtered, and concentrated *in vacuo* to afford 4-cyanomethyl-benzoic acid (0.79 g, 26.3%) as a white solid: mp 193-195°C; EI-HRMS m/e calcd for C₉H₇NO₂ (M⁺) 161.0476, found 161.0483.

A solution of 4-cyanomethyl-benzoic acid (0.53 g, 3.31 mmol) in a 50% aqueous hydrochloric acid solution (42.8 mL) was heated at 80°C for 16 h. At this time, the reaction was cooled to 25°C and brought to pH = 3 by the dropwise addition of a 50% aqueous sodium hydroxide solution. The resulting mixture was diluted with water and extracted with butanol (2 x 50 mL). The organics were then extracted with water (5 x 50 mL, pH = 6-7). The aqueous extracts were brought to pH = 3 with a 3M aqueous hydrochloric acid solution and concentrated *in vacuo* to afford 4-carboxymethyl-benzoic acid (70 mg, 11.7%) as a white solid: mp 235-237°C; EI-HRMS m/e calcd for C₉H₈O₄ (M⁺) 180.0422, found 180.

A mixture of 4-carboxymethyl-benzoic acid (0.20 g, 1.11 mmol) and nickel(II) chloride hexahydrate (27 mg, 0.11 mol) in methanol (1.11 mL) was heated at 120°C for 24 h. At this time, the reaction mixture was cooled to 25°C and concentrated *in vacuo*. Flash chromatography (Merck Silica gel 60, 230-400 mesh, 70/30 hexanes/ethyl acetate) afforded 4-methoxycarbonylmethyl-benzoic acid methyl ester (66.7 mg, 28.8%) as a clear oil: EI-HRMS m/e calcd for C₁₁H₁₂O₄ (M⁺) 208.0735, found 208.0733.

A solution of freshly prepared lithium diisopropylamide (2.3 mL of a 0.31M stock solution, 0.71mmol) cooled to -78°C was treated with a solution of 4-methoxycarbonylmethyl-benzoic acid methyl ester (66 mg, 0.31 mmol) in tetrahydrofuran/1,3-dimethyl-3,4,5,6-tetrahydro-2(1H)-pyrimidinone (0.85 mL, 3:1). The resulting solution was stirred at -78°C for 45 min. At this time, the reaction was treated with a solution of iodomethylcyclopentane (86 mg, 0.40 mmol) in 1,3-dimethyl-3,4,5,6-tetrahydro-2(1H)-pyrimidinone (1 mL). The reaction mixture was stirred at - 78°C for 4 h. The reaction was then warmed to 25°C and stirred at 25°C for 18 h. At this time, the reaction mixture was quenched by the slow addition of a saturated aqueous ammonium chloride solution (10 mL). The reaction mixture was then poured into water (50 mL). This solution was extracted into ethyl acetate (3 x 25 mL). The organics were dried over sodium sulfate, filtered, and concentrated *in vacuo*. Flash chromatography (Merck Silica gel 60, 230-400 mesh, 80/20 hexanes/ethyl acetate) afforded 4-(2-cyclopentyl-1-methoxycarbonyl-ethyl)-benzoic acid methyl ester (60.5 mg, 65.7%) as a clear oil: EI-HRMS m/e calcd for C₁₇H₂₂O₄ (M⁺) 290.1518, found 290.1518.

A solution of 4-(2-cyclopentyl-1-methoxycarbonyl-ethyl)-benzoic acid methyl ester (0.40 g, 1.37 mmol) in tetrahydrofuran/water/methanol (13.7 mL, 3:1:1) was treated with a 1N aqueous lithium hydroxide solution. The reaction mixture was stirred at 25°C for 1 h. At this time, the reaction was poured into water. The aqueous layer was acidified to pH = 1 with a 1N aqueous hydrochloric acid solution and extracted with a solution of chloroform/methanol (9:1, 4 x 25 mL). The organics were dried over sodium sulfate, filtered, and concentrated *in vacuo*. Flash chromatography (Merck Silica gel 60, 230-400 mesh, 50/50 hexanes/ethyl acetate) afforded a mixture of 4-(1-carboxy-2-cyclopentyl-ethyl)-benzoic acid methyl ester and 4-(1-carboxy-2-cyclopentyl-ethyl)-benzoic acid methyl ester (161.8 mg, 42.5%) as a clear oil: EI-HRMS m/e calcd for C₁₆H₂₀O₄ (M⁺) 276.1361, found 276.1364.

A solution of the mixture of 4-(1-carboxy-2-cyclopentyl-ethyl)-benzoic acid methyl ester and 4-(1-carboxy-2-cyclopentyl-ethyl)-benzoic acid methyl ester (24.2 mg, 0.08 mmol) in methylene chloride (0.87 mL) cooled to 0°C was treated with a 2.0M solution of oxalyl chloride in methylene chloride (0.05 mL, 0.10 mmol) and a few drops of *N*,*N*-dimethylformamide. The reaction mixture was stirred at 0°C for 10 min and at 25°C for 30 min. The reaction mixture was then treated with a solution of 2-aminothiazole (19.3 mg, 0.19 mmol) and *N*,*N*-diisopropylethylamine (0.04 mL, 0.21 mmol) in tetrahydrofuran (0.44 mL). The reaction mixture was stirred at 25°C for 4 h. At this time, the reaction was concentrated *in vacuo*. Flash chromatography (Merck Silica gel 60, 230-400 mesh, 50/50 hexanes/ethyl acetate) afforded 4-[2-cyclopentyl-1-(thiazol-2-ylcarbamoyl)-ethyl]-benzoic acid methyl ester (18.1 mg, 57.6%) as an off-white solid: mp 54-56°C; EI-HRMS m/e calcd for C₁₉H₂₂N₂O₃S (M⁺) 358.1351, found 358.1346.

### Example 120

### 3-Cyclopentyl-2-(3-fluoro-4-methoxy-phenyl)-N-thiazol-2-yl-propionamide

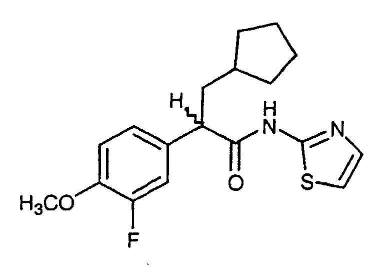

A solution of (3-fluoro-4-hydroxy-phenyl)-acetic acid (1.0 g, 5.87 mmol) in methanol (20 mL) was treated with a catalytic amount of sulfuric acid. The reaction was heated at 120°C for 6 h. At this time, the reaction was concentrated *in vacuo*. Flash chromatography (Merck Silica gel 60, 230-400 mesh, 50/50 hexanes/ethyl acetate) afforded (3-fluoro-4-hydroxy-phenyl)-acetic acid methyl ester (1.05 g, 97.6%) as a white solid: mp 34-36°C: EI-HRMS m/e calcd for C₉H₉FO₃ (M⁺) 184.0535, found 184.0533.

A mixture of 3-fluoro-4-hydroxy-phenyl)-acetic acid methyl ester (1.0 g, 5.43 mmol), potassium carbonate (1.87 g, 13.57 mmol), and methyl iodide (1.12 g, 8.14 mmol) in acetone (27.1 mL) was heated at 90°C for 4 h. At this time, the potassium carbonate was removed by filtration. The filtrate was concentrated *in vacuo*. Flash chromatography (Merck Silica gel 60, 230-400 mesh, 80/20 hexanes/ethyl acetate) afforded (3-fluoro-4-methoxy-phenyl)-acetic acid methyl ester (1.01 g, 94.3%) as a clear oil: EI-HRMS m/e calcd for C₁₀H₁₁FO₃(M⁺) 198.0692, found 198.0693.

A solution of freshly prepared lithium diisopropylamide (21.6 mL of 0.31M stock solution, 6.69 mmol) cooled to -78°C was treated with a solution of (3-fluoro-4-methoxy-phenyl)-acetic acid methyl ester (1.26 g, 6.38 mmol) in tetrahydrofuran/1,3-dimethyl-3,4,5,6-tetrahydro-2(1H)-pyrimidinone (16 mL, 3:1). The resulting solution was stirred at -78°C for 45 min. At this time, the reaction was treated with a solution of iodomethylcyclopentane (1.47 g, 7.02 mmol) in 1,3-dimethyl-3,4,5,6-tetrahydro-2(1H)-pyrimidinone (2 mL). The reaction mixture was stirred at -78°C for 4 h. The reaction was warmed to 25°C and stirred at 25°C for 48 h. The reaction mixture was then quenched by the slow addition of a saturated aqueous ammonium chloride solution (10 mL). The reaction mixture was then poured into water (100 mL) and extracted with ethyl acetate (3 x 50 mL). The organics were washed with a saturated aqueous lithium chloride solution (1 x 50 mL), dried over sodium sulfate, filtered, and concentrated *in vacuo*. Flash chromatography (Merck Silica gel 60, 230-400 mesh, 90/10 hexanes/ethyl acetate) afforded 3-cyclopentyl-2-(3-fluoro-4-methoxy-phenyl)-propionic acid methyl ester (1.50 g, 83.8%) as a clear oil: EI-HRMS m/e calcd for C₁₆H₂₁FO₃(M⁺) 280.1477 found 280.1474.

A solution of 3-cyclopentyl-2-(3-fluoro-4-methoxy-phenyl)-propionic acid methyl ester (1.04 g, 3.73 mmol) in tetrahydrofuran/water/methanol (9.3 mL, 3:1:1) was treated with a 1N aqueous lithium hydroxide solution (3.73 mL, 3.73 mmol). The reaction was stirred at 25°C for 18 h. At this time, the reaction was acidified to pH = 1 with a 1N aqueous hydrochloric acid solution and extracted with ethyl acetate (3 x 50 mL). The organics were dried over sodium sulfate, filtered, and concentrated *in vacuo*. Flash chromatography (Merck Silica gel 60, 230-400 mesh, 50/50 hexanes/ethyl acetate) afforded 3-cyclopentyl-2-(3-fluoro-4-methoxy-phenyl)-propionic acid (707.8 mg, 71.3%) as a white solid: mp 149-151°C; EI-HRMS m/e calcd for C₁₅H₁₉F O₃ (M⁺) 266.1318 found 266.1317.

A solution of 3-cyclopentyl-2-(3-fluoro-4-methoxy-phenyl)-propionic acid (400.0 mg, 1.50 mmol) in methylene chloride (5.0 mL) cooled to 0°C was treated with a 2.0M solution of oxalyl chloride in methylene chloride (0.82 mL, 1.65 mmol) and a few drops of *N*,*N*-dimethylformamide. The reaction mixture was stirred at 0°C for 10 min and at 25°C for 30 min. The reaction mixture was then treated with a solution of 2-aminothiazole (331 mg, 3.30 mmol) and *N*,*N*-diisopropylethylamine (0.62 mL, 3.60 mmol) in tetrahydrofuran (7.5 mL). This solution was stirred at 25°C for 18 h. At this time, the reaction was concentrated *in vacuo*. Flash chromatography (Merck Silica gel 60, 230-400 mesh, 80/20 hexanes/ethyl acetate) afforded 3-cyclopentyl-2-(3-fluoro-4-methoxy-phenyl)-N-thiazol-2-yl-propionamide (538.4 mg, 100%) as a white solid: mp 51-53°C: EI-HRMS m/e calcd for C₁₈H₂₁FN₂O₂S (M⁺) 348.1307 found 348.1312.

### Example 121

### 3-Cyclopentyl-2-(3-fluoro-4-hydroxy-phenyl)-N-thiazol-2-yl-propionamide

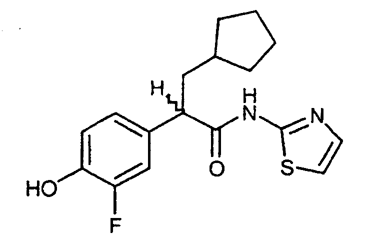

A solution of 3-cyclopentyl-2-(3-fluoro-4-methoxy-phenyl)-N-thiazol-2-yl-propionamide (prepared in Example **120,** 305.4 mg, 0.87 mmol) in methylene chloride (8.7 mL) at 25°C was treated with a 1.0M solution of boron tribromide in methylene chloride (8.75 mL, 8.75 mmol). This solution was stirred at 25°C for 5 h. At this time, the reaction was cooled to 0°C and quenched by the dropwise addition of a dilute aqueous ammonium hydroxide solution. The resulting solution was stirred at 0°C for 15 min. This mixture was then poured into water (50 mL) and extracted with ethyl acetate (3 x 30 mL). The organics were dried over sodium sulfate, filtered, and concentrated *in vacuo*. Flash chromatography (Merck Silica gel 60, 230-400 mesh, 50/50 hexanes/ethyl acetate) afforded 3-cyclopentyl-2-(3-fluoro-4-hydroxy-phenyl)-N-thiazol-2-yl-propionamide (212.7 mg, 72.5%) as a white solid: mp 199-201°C: EI-HRMS m/e calcd for C₁₇H₁₉FN₂O₂S (M⁺) 334.1151 found 334.1152.

### Example 122

### 6-[2-(3-Chloro-phenyl)-3-cyclopentyl-propionylamino]-nicotinic acid

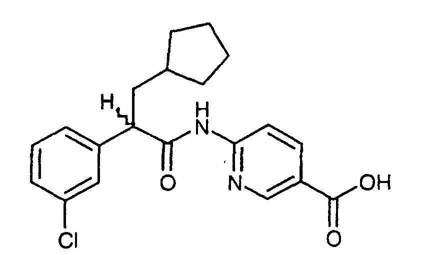

A solution of freshly prepared lithium diisopropylamide (141.3 mL of a 0.32M stock solution, 45.0 mmol) cooled to -78°C was treated with (3-chloro-phenyl)-acetic acid (3.41 g, 20.0 mmol) in tetrahydrofuran/1,3-dimethyl-3,4,5,6-tetrahydro-2(1H)-pyrimidinone (49.7 mL, 3: 1). The resulting solution was stirred at -78°C for 1 h. At this time, the reaction was treated with a solution of iodomethylcyclopentane (4.64 g, 22.08 mmol) in 1,3-dimethyl-3,4,5,6-tetrahydro-2(1H)-pyrimidinone (4.64 mL). The reaction mixture was stirred at -78°C for 4 h. The reaction was then warmed to 25°C and was stirred at 25°C for 48 h. This solution was then quenched by the slow addition of the reaction mixture to a 2N aqueous hydrochloric acid solution (50 mL). The product was extracted into ethyl acetate (1 x 150 mL). The organic layer was dried over sodium sulfate, filtered, and concentrated *in vacuo*. Flash chromatography (Merck Silica gel 60, 230-400 mesh, 85/15 hexanes/ethyl acetate) afforded 2-(3-chloro-phenyl)-3-cyclopentyl-propionic acid (3.68 g, 72.9%) as a yellow solid: mp 70-72°C; EI-HRMS m/e calcd for C₁₄H₁₇ClO₂ (M⁺) 252.0917, found 252.0915.

A solution of 2-(3-chloro-phenyl)-3-cyclopentyl-propionic acid (504 mg, 2.0 mmol) in methylene chloride (20 mL) cooled to 0°C was treated with a 2.0M solution of oxalyl chloride in methylene chloride (1.1 mL, 2.2 mmol) and a few drops of *N,N*-dimethylformamide. The reaction mixture was stirred at 0°C for 15 min and at 25°C for 2 h. The reaction mixture was then treated with 6-amino-nicotinic acid methyl ester (532 mg, 3.5 mmol) and *N,N*-diisopropylethylamine (0.84 mL, 4.8 mmol). This solution was stirred at 25°C for 18 h. At this time, the reaction was concentrated *in vacuo.* Flash chromatography (Merck Silica gel 60, 230-400 mesh, 50/50 hexanes/ethyl acetate) afforded 6-[2-(3-chloro-phenyl)-3-cyclopentyl-propionylamino]-nicotinic acid methyl ester (151.9 mg, 19.7%) as a colorless oil: EI-HRMS m/e calcd for C₂₁H₂₃ClN₂O₃ (M⁺) 386.1397, found 386.1398.

A solution of 6-[2-(3-chloro-phenyl)-3-cyclopentyl-propionylamino]-nicotinic acid methyl ester (146.9 mg, 0.38 mmol) in tetrahydrofuran/water/methanol (10 mL, 3:1:1) at 25°C was treated with a 2N aqueous sodium hydroxide solution (0.4 mL, 0.80 mmol). The reaction mixture was stirred at 25°C for 4 d. At this time, the reaction was concentrated *in vacuo*. The residue was diluted with water (50 mL) and extracted with diethyl ether (1 x 50 mL). The aqueous layer was acidified to pH = 1 by the dropwise addition of a 3N aqueous hydrochloric acid solution. This solution was extracted with a solution of methylene chloride/methanol (3:1, 3 x 75 mL). The organics were dried over magnesium sulfate, filtered, and concentrated *in vacuo*. The resulting solid was triturated with diethyl ether/hexanes (2:1) to afford 6-[2-(3-chloro-phenyl)-3-cyclopentyl-propionylamino]-nicotinic acid (63.6 mg, 44.4%) as a white solid: mp 251-255°C; EI-HRMS m/e calcd for C₂₀H₂₁ClN₂O₃ (M⁺) 372.1240, found 372.1250.

### Example 123

### 6-[3-Cyclopentyl-2-(4-nitro-phenyl)-propionylamino]-nicotinic acid methyl ester

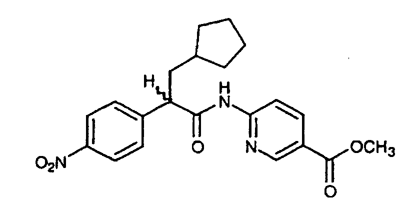

A solution of freshly prepared lithium diisopropylamide (430.55 mL of a 0.3M stock solution, 129.16 mmol) cooled to -78°C was treated with (4-nitro-phenyl)-acetic acid ethyl ester (26.32 g, 125.83 mmol) in tetrahydrofuran/hexamethylphosphoramide (312.5 mL, 3: 1). The resulting solution was stirred at -78°C for 45 min. At this time, the reaction was treated with a solution of iodomethylcyclopentane (27.75 g, 132.1 mmol) in hexamethylphosphoramide (27.75 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (250 mL). This mixture was concentrated *in vacuo*. The resulting residue was diluted with water (250 mL) and extracted with ethyl acetate (3 x 300 mL). The organics were washed with a saturated aqueous lithium chloride solution (2 x 250 mL), dried over magnesium sulfate, filtered, and concentrated *in vacuo*. Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as an yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (14.1 g, 48.06 mmol) in tetrahydrofuran/water (300 mL, 3:1) was treated with lithium hydroxide (4.35 g, 103.67 mmol). The reaction was stirred at 25°C for 21 h. The tetrahydrofuran was then removed *in vacuo*. The residue was diluted with water (75 mL) and extracted with ether (3 x 75 mL). The aqueous layer was acidified to pH = 1 with a 3N aqueous hydrochloric acid solution and extracted into methylene chloride (3 x 75 mL). The organics were washed with a saturated aqueous sodium chloride solution (2 x 100 mL), dried over magnesium sulfate, filtered, and concentrated *in vacuo* to afford 3-cyclopentyl-2-(4-nitro-phenyl)-propionic acid (11.97 g, 93.6%) as a yellow solid: mp 119-125°C; EI-HRMS m/e calcd for C₁₄H₁₇NO₄ (M⁺) 263.1157, found 263.1162.

A solution of 3-cyclopentyl-2-(4-nitro-phenyl)-propionic acid (526 mg, 2.0 mmol) in methylene chloride (20 mL) cooled to 0°C was treated with a 2.0M solution of oxalyl chloride in methylene chloride (1.2 mL, 2.4 mmol) and a few drops of *N*,*N*-dimethylformamide. The reaction mixture was stirred at 0°C for 15 min and at 25°C for 30 min. The reaction mixture was then treated with a solution of 6-amino-nicotinic acid methyl ester (532 mg, 3.5 mmol) in tetrahydrofuran (10 mL) and *N,N*-diisopropylethylamine (0.84 mL, 4.8 mmol). This solution was stirred at 25°C for 48 h. At this time, the reaction was concentrated *in vacuo*. Flash chromatography (Merck Silica gel 60, 230-400 mesh, 80/20 hexanes/ethyl acetate) afforded 6-[3-cyclopentyl-2-(4-nitro-phenyl)-propionylamino]-nicotinic acid methyl ester (353.9 mg, 44.6%) as a pale orange glass: EI-HRMS m/e calcd for C₂₁H₂₃N₃O₅ (M⁺) 397.1637, found 397.1631.

### Example 124

### 2-(4-Amino-phenyl)-3-cyclopentyl-N-pyridin-2-yl-propionamide

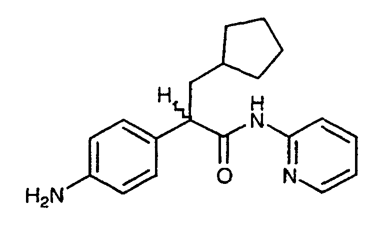

A solution of 3-cyclopentyl-2-(4-nitro-phenyl)-propionic acid (prepared in Example **22**, 263 mg, 1.0 mmol) in methylene chloride (10 mL) cooled to 0°C was treated with a 2.0M solution of oxalyl chloride in methylene chloride (0.6 mL, 1.2 mmol) and a few drops of *N,N*-dimethylformamide. The reaction mixture was stirred at 0°C for 15 min and at 25°C for 30 min. The reaction mixture was then treated with a solution of 2-aminopyridine (200.6 mg, 2.14 mmol) in tetrahydrofuran (5 mL) and *N,N*-diisopropylethylamine (0.42 mL, 2.4 mmol). This solution was stirred at 25°C for 48 h. At this time, the reaction was concentrated *in vacuo*. Flash chromatography (Merck Silica gel 60, 230-400 mesh, 80/20 hexanes/ethyl acetate) afforded 3-cyclopentyl-2-(4-nitro-phenyl)-N-pyridin-2-yl-propionamide (138.6 mg, 40.9%) as a pale yellow glass: EI-HRMS m/e calcd for C₁₉H₂₁N₃O₃ (M⁺) 339.1581, found 339.1582.

A mixture of 3-cyclopentyl-2-(4-nitro-phenyl)-N-pyridin-2-yl-propionamide (130 mg, 0.38 mmol) in ethyl acetate (50 mL) and methanol (5 mL) was treated with a catalytic amount of 10% palladium on activated carbon (50 mg). The resulting mixture was shaken at 25°C under 60 psi of hydrogen gas in a Parr apparatus for 24 h. At this time, the catalyst was removed by filtration through a plug of celite. The filtrate was concentrated *in vacuo* to afford 2-(4-amino-phenyl)-3-cyclopentyl-N-pyridin-2-yl-propionamide (99.9 mg, 84.3%) as a tan oil: EI-HRMS m/e calcd for C₁₉H₂₃N₃O (M⁺) 309.1834, found 309.1849.

### Example 125

### 6-[2-(4-Amino-phenyl)-3-cyclopentyl-propionylamino]-nicotinic acid methyl ester

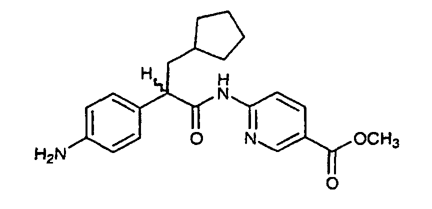

A solution of 3-cyclopentyl-2-(4-nitro-phenyl)-propionic acid (prepared in Example **22,** 526 mg, 2.0 mmol) in methylene chloride (20 mL) cooled to 0°C was treated with a 2.0M solution of oxalyl chloride in methylene chloride (1.2 mL, 2.4 mmol) and a few drops of *N,N*-dimethylformamide. The reaction mixture was stirred at 0°C for 15 min and at 25°C for 30 min. The reaction mixture was then treated with a solution of 6-amino-nicotinic acid methyl ester (532 mg, 3.5 mmol) in tetrahydrofuran (10 mL) and *N,N*-diisopropylethylamine (0.84 mL, 4.8 mmol). This solution was stirred at 25°C for 48 h. At this time, the reaction was concentrated *in vacuo*. Flash chromatography (Merck Silica gel 60, 230-400 mesh, 80/20 hexanes/ethyl acetate) afforded 6-[3-cyclopentyl-2-(4-nitro-phenyl)-propionylamino]-nicotinic acid methyl ester (353.9 mg, 44.6%) as a pale orange glass: EI-HRMS m/e calcd for C₂₁H₂₃N₃O₅ (M⁺) 397.1637, found 397.1631.

A mixture of 6-[3-cyclopentyl-2-(4-nitro-phenyl)-propionylamino]-nicotinic acid methyl ester (300 mg, 0.75 mmol) in ethyl acetate (30 mL) was treated with a catalytic amount of 10% palladium on activated carbon (30 mg). The resulting mixture was shaken at 25°C under 60 psi of hydrogen gas in a Parr apparatus for 24 h. At this time, the catalyst was removed by filtration through a plug of celite. The filtrate was concentrated *in vacuo* to afford 6-[2-(4-amino-phenyl)-3-cyclopentyl-propionylamino]-nicotinic acid methyl ester (262.8 mg, 94.7%) as a pale yellow glass: EI-HRMS m/e calcd for C₂₁H₂₅N₃O₃ (M⁺) 367.1895, found 367.1899.

### Example 126

### 3-Cyclohexyl-2-(4-methanesulfonyl-3-trifluoromethyl-phenyl)-N-thiazol-2-yl-propionamide

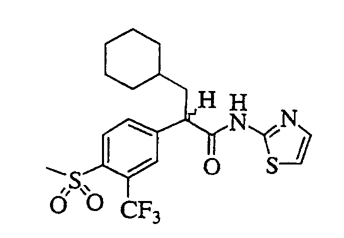

A solution of isoamyl nitrite (4.02 mL, 30 mmol) in dimethyl disulfide (19.8 mL, 220 mmol) at 25°C was slowly treated with 4-bromo-2-(trifluoromethyl)aniline (4.8 g, 20 mmol). The reaction was exothermic with gas evolution. The resulting brown reaction mixture was heated to 80-90°C for 2 h, at which time, thin layer chromatography analysis of the reaction mixture indicated the absence of starting material. The reaction mixture was cooled to 25°C and then concentrated *in vacuo*. The resulting residue was dissolved in ethyl acetate (200 mL). The organic layer was washed successively with a 1N aqueous hydrochloric acid solution (1 x 200 mL) and a saturated aqueous sodium chloride solution (1 x 200 mL), dried over anhydrous magnesium sulfate, filtered, and concentrated *in vacuo*. Biotage chromatography (FLASH 40M, Silica, 8/1 hexanes/ethyl acetate) afforded 4-bromo-1-methylsulfanyl-2-trifluoromethyl-benzene (4.73 g, 87%) as a brown oil: EI-HRMS m/e calcd for C₈H₆BrF₃S (M⁺) 269.9326, found 269.9327.

A solution of 4-bromo-1-methylsulfanyl-2-trifluoromethyl-benzene (4.71 g, 17.4 mmol) in methylene chloride (100 mL) was cooled to -10°C and then treated with 3-chloroperoxybenzoic acid (86% grade, 9.0 g, 52.2 mmol). The reaction mixture was stirred at -10°C for 10 min and then allowed to warm to 25°C where it was stirred overnight. At this time, thin layer chromatography analysis of the reaction mixture indicated the absence of starting material. The reaction mixture was then filtered, and the solids were washed with methylene chloride (1 x 50 mL). The filtrate was concentrated *in vacuo*. The resulting residue was dissolved in ethyl acetate (100 mL). The organic layer was washed successively with a saturated aqueous sodium bicarbonate solution (2 x 100 mL) and a saturated aqueous sodium chloride solution (1 x 100 mL), dried over anhydrous magnesium sulfate, filtered, and concentrated *in vacuo* to afford a yellow solid. Recrystallization from methylene chloride (20 mL), diethyl ether (10 mL), and hexanes afforded 4-bromo-1-methanesulfonyl-2-trifluoromethyl-benzene (3.46 g, 57%) as a white solid: mp 110-112 °C; EI-HRMS m/e calcd for C₈H₆BrF₃O₂S (M⁺) 301.9224, found 301.9223.

A mixture of zinc dust (1.3 g, 20 mmol, Aldrich, 325 mesh) and dry tetrahydrofuran (2 mL) under argon was treated with 1,2-dibromoethane (187 mg, 1 mmol). The zinc suspension was then heated with a heat gun to ebullition, allowed to cool, and heated again. This process was repeated three times to make sure the zinc dust was activated. The activated zinc dust suspension was then treated with trimethylsilyl chloride (110 mg, 1 mmol), and the suspension was stirred for 15 min at 25°C. The reaction mixture was then treated dropwise with a solution of (E)-3-cyclohexyl-2-iodo-acrylic acid methyl ester (prepared in Example **110**, 2.5 g, 8.5 mmol) in dry tetrahydrofuran (3 mL) over 5 min. After the addition, the reaction mixture was stirred for 1 h at 40-45°C and then stirred overnight at 25°C. The reaction mixture was then diluted with dry tetrahydrofuran (4 mL), and the stirring was stopped to allow the excess zinc dust to settle down (-2 h). In a separate reaction flask, bis(dibenzylideneacetone)palladium(0) (108 mg, 0.2 mmol) and triphenylphosphine (209 mg, 0.8 mmol) in dry tetrahydrofuran (10 mL) was stirred at 25°C under argon for 10 min and then treated with 4-bromo-1-methanesulfonyl-2-trifluoromethyl-benzene (2.12 g, 7 mmol) and the freshly prepared zinc compound in tetrahydrofuran. The resulting brick red solution was heated at 40-45°C for 2 d. The reaction mixture was cooled to 25°C and then poured into a saturated aqueous ammonium chloride solution (100 mL), and the organic compound was extracted into ethyl acetate (3 x 75 mL). The combined organic extracts were washed with a saturated aqueous sodium chloride solution (1 x 100 mL), dried over anhydrous magnesium sulfate, filtered, and concentrated *in vacuo*. Biotage chromatography (FLASH 40M, Silica, 9/1 to 3/1 hexanes/ethyl acetate) afforded (E)-3-cyclohexyl-2-(4-methanesulfonyl-3-trifluoromethyl-phenyl)-acrylic acid methyl ester (2.7 g, 99%) as a viscous oil: EI-HRMS m/e calcd for C₁₈H₂₁F₃O₄S (M⁺) 391.1191, found 391.1200.

A solution of nickel(II) chloride hexahydrate (36.6 mg, 0.154 mmol) and (E)-3-cyclohexyl-2-(4-methanesulfonyl-3-trifluoromethyl-phenyl)-acrylic acid methyl ester (302 mg, 0.77 mmol) in methanol (8 mL) was cooled to 0°C and then treated with sodium borohydride (87 mg, 2.29 mmol) in four portions. After the addition, the black reaction mixture was stirred for 15 min at 0°C and then allowed to warm to 25°C where it was stirred for 15 h. The black solid was filtered using filter paper and washed with methanol. The combined solvents were concentrated *in vacuo*, and the residue was diluted with ethyl acetate (50 mL). The organic layer was washed successively with a 3N aqueous hydrochloric acid solution (1 x 50 mL), a saturated aqueous sodium bicarbonate solution (1 x 50 mL) and a saturated aqueous sodium chloride solution (1 x 50 mL), dried over anhydrous magnesium sulfate, filtered, and concentrated *in vacuo* to afford racemic 3-cyclohexyl-2-(4-methanesulfonyl-3-trifluoromethyl-phenyl)-propionic acid methyl ester (280 mg, 93%) as a viscous oil: EI-HRMS m/e calcd for C₁₈H₂₃F₃O₄S (M⁺) 392.1269, found 392.1276.

A solution of 3-cyclohexyl-2-(4-methanesulfonyl-3-trifluoromethyl-phenyl)-propionic acid methyl ester (265 mg, 0.67 mmol) in ethanol (5 mL) was treated with a 1N aqueous sodium hydroxide solution (1.5 mL). The solution was heated at 45-50°C for 5 h, at which time, thin layer chromatography analysis of the mixture indicated the absence of starting material. The reaction mixture was then concentrated *in vacuo* to remove ethanol, and the residue was diluted with water (20 mL) and extracted with diethyl ether (1 x 40 mL) to remove any neutral impurities. The aqueous layer was acidified with a 1N aqueous hydrochloric acid solution. The resulting acid was extracted into ethyl acetate (2 x 50 mL). The combined organic layers were washed with a saturated aqueous sodium chloride solution (1 x 50 mL), dried over anhydrous magnesium sulfate, filtered, and concentrated *in vacuo* to afford 3-cyclohexyl-2-(4-methanesulfonyl-3-trifluoromethyl-phenyl)-propionic acid (249 mg, 97%) as a viscous oil: EI-HRMS m/e calcd for C₁₇H₂₁F₃O₄S (M⁺) 378.1113, found 378.1121.

A solution of triphenylphosphine (279 mg, 1.06 mmol) in methylene chloride (5 mL) was cooled to 0°C and then treated with *N*-bromosuccinimide (188.7 mg, 1.06 mmol). The reaction mixture was stirred at 0°C for 30 min and then treated with a solution of 3-cyclohexyl-2-(4-methanesulfonyl-3-trifluoromethyl-phenyl)-propionic acid (237 mg, 0.626 mmol) in methylene chloride (4 mL). The clear solution was stirred for 15 min at 0°C and then allowed to warm to 25°C where it was stirred for 2 h. The reaction mixture was then treated with 2-aminothiazole (188 mg, 1.88 mmol), and the resulting suspension was stirred for 15 h at 25°C. The reaction mixture was concentrated *in vacuo* to remove methylene chloride, and the residue was diluted with ethyl acetate (50 mL) and a 1N aqueous hydrochloric acid solution (50 mL). The two layers were separated, and the aqueous layer was extracted with ethyl acetate (1 x 30 mL). The combined organic extracts were successively washed with a saturated aqueous sodium bicarbonate solution (1 x 50 mL) and a saturated aqueous sodium chloride solution (1 x 50 mL), dried over anhydrous magnesium sulfate, filtered, and concentrated *in vacuo*. Biotage chromatography (FLASH 40S, Silica, 4/1 to 2/1 hexanes/ethyl acetate) afforded 3-cyclohexyl-2-(4-methanesulfonyl-3-trifluoromethyl-phenyl)-N-thiazol-2-yl-propionamide (83 mg, 29%) as an amorphous solid: EI-HRMS m/e calcd for C₂₀H₂₃F₃N₂O₃S₂ (M⁺) 460.1102, found 460.1100.

### Example 127

### (A) 3-Cyclohexyl-2-(3,4-dichloro-phenyl)-propionyl]-urea

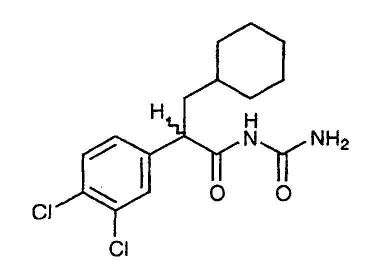

A solution of (3,4-dichloro-phenyl)-acetic acid (14.0 g, 0.068 mol) in methanol (71mL) was treated with a catalytic amount of sulfuric acid. The reaction mixture was refluxed for 12 h. The reaction was concentrated *in vacuo*. Flash chromatography (Merck Silica gel 60, 230-400 mesh, 50/50 hexanes/ethyl acetate) afforded (3,4-dichloro-phenyl)-acetic acid methyl ester (15.0 g, quant.) as a white solid: mp 30-32°C; EI-HRMS m/e calcd for C₉H₈Cl₂O₂ (M⁺) 217.9901, found 217.9907.

A solution of freshly prepared lithium diisopropylamide (16.3 mL of a 0.31M stock solution, 5.04 mmol) cooled to -78°C was treated with (3,4-dichloro-phenyl)-acetic acid methyl ester (1.0 g, 4.58 mmol) in tetrahydrofuran/hexamethylphosphoramide (8.6 mL, 3:1). The resulting solution was stirred at -78°C for 45 min. At this time, the reaction was treated with a solution of bromomethylcyclohexane (1.92 mL, 13.76 mmol) in hexamethylphosphoramide (1 mL). The reaction mixture was stirred at -78°C for 3 h. The reaction was warmed to 25°C and stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 3-cyclohexyl-2-(3,4-dichloro-phenyl)-propionic acid methyl ester (1.5 g, quant.) as a clear oil: EI-HRMS m/e calcd for C₁₆H₂₀Cl₂O₂ (M⁺) 314.0840, found 314.0836.

A mixture of 3-cyclphexyl-2-(3,4-dichloro-phenyl)-propionic acid methyl ester (582 mg, 1.84 mmol) and urea (222 mg, 3.69 mmol) in a solution of magnesium methoxide in methanol (7.4 wt%, 3.96 mL, 2.76 mmol) was refluxed at 120°C for 12 h. The reaction mixture was then concentrated *in vacuo*. Flash chromatography (Merck Silica gel 60, 230-400 mesh, 50/50 hexanes/ethyl acetate) afforded 3-cyclohexyl-2-(3,4-dichloro-phenyl)-propionyl]-urea (52.9 mg, 8.3%) as a white solid: mp 76-79°C; EI-HRMS m/e calcd for C₁₆H₂₀Cl₂N₂O₂ (M⁺) 342.0902, found 342.0904.

### (B) In an analogous manner, there were obtained:

(a) From 3-cyclopropyl-2-(3,4-dichloro-phenyl)-propionic acid methyl ester and urea: [3-Cyclopropyl-2-(3,4-dichloro-phenyl)-propionyl]-urea as a white solid: mp 117-119°C; EI-HRMS m/e calcd for C₁₃H₁₄Cl₂N₂O₂ (M⁺) 300.0432, found 300.0431.
(b) From 3-cyclopentyl-2-(3,4-dichloro-phenyl)-propionic acid methyl ester and urea: [3-Cyclopentyl-2-(3,4-dichloro-phenyl)-propionyl]-urea as a white solid: mp 103-105°C; EI-HRMS m/e calcd for C₁₅H₁₈Cl₂N₂O₂ (M⁺) 328.0745, found 328.0750.
(c) From 3-cyclobutyl-2-(3,4-dichloro-phenyl)-propionic acid methyl ester and urea: [3-Cyclobutyl-2-(3,4-dichloro-phenyl)-propionyl]-urea as a white solid: mp 65-67°C; EI-HRMS m/e calcd for C₁₄H₁₆Cl₂N₂O₂ (M⁺) 314.0589, found 314.0597.
(d) From 3-cycloheptyl-2-(3,4-dichloro-phenyl)-propionic acid methyl ester and urea: [3-Cycloheptyl-2-(3,4-dichloro-phenyl)-propionyl]-urea as a white solid: mp 69-71 °C; EI-HRMS m/e calcd for C₁₇H₂₂Cl₂N₂O₂ (M⁺) 356.1058, found 356.1054.
(e) From 1-[3-cyclopentyl-2-(3,4-dichloro-phenyl)-propionyl]-3-methyl ester and methyl urea: 1-[3-Cyclopentyl-2-(3,4-dichloro-phenyl)-propionyl]-3-methyl-urea as a white solid: mp 120-125°C; EI-HRMS m/e calcd for C₁₆H₂₀Cl₂N₂O₂ (M⁺) 342.0902, found 342.0903.
(f) From 3-cyclohexyl-2-(3,4-dichloro-phenyl)-propionic acid methyl ester and methyl urea: [3-Cyclohexyl-2-(3,4-dichloro-phenyl)-propionyl]-3-methyl-urea as a white solid: mp 69-73°C; EI-HRMS m/e calcd for C₁₇H₂₂Cl₂N₂O₂ (M⁺) 356.1058, found 356.1046.
(g) From 1-[2-(3,4-dichloro-phenyl)-4-methyl-pentanoyl]-3-methyl ester and methyl-urea: 1-[2-(3,4-Dichloro-phenyl)-4-methyl-pentanoyl]-3-methyl-urea as a white solid: mp 123-125°C; EI-HRMS m/e calcd for C₁₄H₁₈Cl₂N₂O₂ (M⁺) 316.0745, found 316.0740.
(h) From 2-(3,4-dichloro-phenyl)-hexanoic acid methyl ester and methyl-urea: 1-[2-(3,4-Dichloro-phenyl)-hexanoyl]-3-methyl-urea as a clear oil: EI-HRMS m/e calcd for C₁₄H₁₈Cl₂N₂O₂ (M⁺) 316.0743, found 316.0745.

### Example 128

### 1-[2-(3-Chloro-phenyl)-3-cyclopentyl-propionyl]-3-methyl-urea

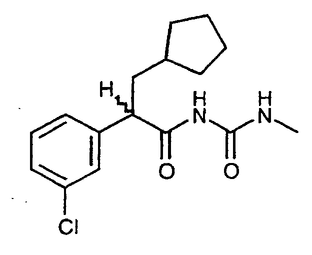

(3-Chloro-phenyl)-acetic acid (6.03 g, 0.03 mol) was dissolved in ethanol (37.7 mL) and treated with a catalytic amount of sulfuric acid. The reaction mixture was refluxed for 12 h. The reaction was concentrated *in vacuo*. Flash chromatography (Merck Silica gel 60, 230-400 mesh, 50/50 hexanes/ethyl acetate) afforded (3-chloro-phenyl)-acetic acid ethyl ester (6.10 g, 86.8%) as a clear oil: EI-HRMS m/e calcd for C₁₀H₁₁ClO₂ (M⁺) 198.0448, found 198.0442.

A solution of freshly prepared lithium diisopropylamide (23 mL of 0.31M stock solution, 7.13 mmol) cooled to -78°C was treated with (3-chloro-phenyl)-acetic acid ethyl ester (1.28 g, 6.48 mmol) in tetrahydrofuran/hexamethylphosphoramide (16.1 mL, 3:1). The resulting solution was stirred at -78°C for 45 min. At this time, the reaction was treated with a solution of iodomethylcyclopentane (1.50 g, 7.13 mmol) in hexamethylphosphoramide (1 mL). The reaction mixture was stirred at -78°C for 4 h. The reaction was warmed to 25°C and stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 2-(3-chloro-phenyl)-3-cyclopentyl-propionic acid ethyl ester (1.70 g, 93%) as a yellow oil: EI-HRMS m/e calcd for C₁₆H₂₁ClO₂ (M⁺) 280.1230, found 280.1238.

A mixture of 2-(3-chloro-phenyl)-3-cyclopentyl-propionic acid ethyl ester (1.70 g, 6.05 mmol) and methyl urea (673 mg, 9.08 mmol) in a solution of magnesium methoxide in methanol (7.4 wt%, 17.3 mL, 12.1 mmol) was refluxed at 100°C for 6 h. The reaction mixture was then concentrated *in vacuo*. Flash chromatcgraphy (Merck Silica gel 60, 230-400 mesh, 75/25 hexanes/ethyl acetate) afforded 1-[2-(3-chloro-phenyl)-3-cyclopentyl-propionyl]-3-methyl-urea (149.1 mg, 8 %) as a white solid: mp 52-55°C; EI-HRMS m/e calcd for C₁₆H₂₁ClN₂O₂ (M⁺) 308.1292, found 308.1287.

### Example 129

### 1-[3-Cyclopentyl-2-(3,4-difluoro-phenyl)-propionyl]-3-methyl-urea

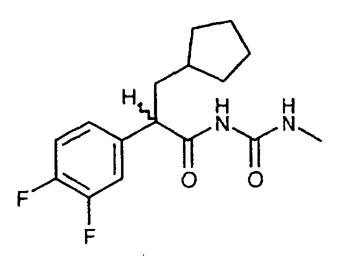

A solution of (3,4-difluoro-phenyl)-acetic acid (5.0 g, 0.029 mol) in methanol (30.0 mL) was treated with a catalytic amount of sulfuric acid. The reaction mixture was refluxed for 4 h. The reaction was concentrated *in vacuo*. Flash chromatography (Merck Silica gel 60, 230-400 mesh, 50/50 hexanes/ethyl acetate) afforded (3,4-difluoro-phenyl)-acetic acid methyl ester (5.15 g, 95.2%) as a clear oil: EI-HRMS m/e calcd for C₉H₈F₂O₂ (M⁺) 186.0493, found 186.0492.

A solution of freshly prepared lithium diisopropylamide (23.0 mL of a 0.31M stock solution, 7.13 mmol) cooled to -78°C was treated with (3,4-difluoro-phenyl)-acetic acid methyl ester (1.20 g, 6.48 mmol) in tetrahydrofuran/hexamethylphosphoramide (16.1 mL, 3: 1). The resulting solution was stirred at -78°C for 45 min. At this time, the reaction was treated with a solution of iodomethylcyclopentane (1.50 g, 7.13 mmol) in hexamethylphosphoramide (1 mL). The reaction mixture was stirred at -78°C for 4 h. The reaction was then warmed to 25°C and was stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 3-cyctopentyl-2-(3,4-difluoro-phenyl)-propionic acid methyl ester (1.79 g, quant.) as a clear oil: EI-HRMS m/e calcd for C₁₅H₁₈F₂O₂ (M⁺) 268.1275, found 268.1278.

A mixture of 3-cyclopentyl-2-(3,4-difluoro-phenyl)-propionic acid methyl ester (1.65 g, 6.14 mmol) and methyl urea (683 mg, 9.22 mmol) in a solution of magnesium methoxide in methanol (7.4 wt%, 16.6 mL, 12.3 mmol) was refluxed at 100°C for 8 h. The reaction mixture was then concentrated *in vacuo*. Flash chromatography (Merck Silica gel 60, 230-400 mesh, 75/25 hexanes/ethyl acetate) afforded 1-[3-cyclopentyl-2-(3,4-difluoro-phenyl)-propionyl]-3-methyl-urea (180 mg, 9.4%) as a white solid: mp 111-113°C; EI-HRMS m/e calcd for C₁₆H₂₀F₂N₂O₂ (M⁺) 310.1493, found 310.1499.

### Example 130

### 1-[2-(4-Chloro-phenyl)-3-cyclopentyl-propionyl]-3-methyl-urea

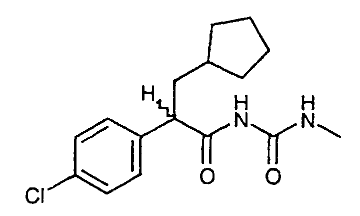

A solution of (4-chloro-phenyl)-acetic acid (6.29 g, 0.03 mol) in ethanol (38.4 mL) was treated with a catalytic amount of sulfuric acid. The reaction mixture was refluxed for 12 h. The reaction was then concentrated *in vacuo*. Flash chromatography (Merck Silica gel 60, 230-400 mesh, 50/50 hexanes/ethyl acetate) afforded (4-chloro-phenyl)-acetic acid ethyl ester (6.45 g, 88%) as a pale yellow solid: mp 39-41°C; EI-HRMS m/e calcd for C₁₀H₁₁ClO₂ (M⁺) 198.0448, found 198.0452.

A solution of freshly prepared lithium diisopropylamide (23.0 mL of 0.31M stock solution, 7.13 mmol) cooled to -78°C was treated with (4-chloro-phenyl)-acetic acid ethyl ester (1.28 g, 6.48 mmol) in tetrahydrofuran/hexamethylphosphoramide (16.1 mL, 3:1). The resulting solution was stirred at -78°C for 45 min. At this time, the reaction was treated with a solution of iodomethylcyclopentane (1.50 mg, 7.13 mmol) in hexamethylphosphoramide (1 mL). The reaction mixture was stirred at -78°C for 4 h. The reaction was warmed to 25°C and stirred at 25°C for 16 h. The reaction mixture was then quenched by the dropwise addition of a saturated aqueous ammonium chloride solution (20 mL). This mixture was poured into water (100 mL) and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate) afforded 2-(4-chloro-phenyl)-3-cyclopentyl-propionic acid ethyl ester (1.65 g, 90.9%) as a yellow oil: EI-HRMS m/e calcd for C₁₆H₂₁Cl₂O₂ (M⁺) 280.1230, found 280.1227.

A mixture of 2-(4-chloro-phenyl)-3-cyclopentyl-propionic acid ethyl ester (1.65 g, 5.89 mmol) and methyl urea (654 mg, 8.83 mmol) in a solution of magnesium methoxide in methanol (7.4 wt%, 16.9 mL, 11.78 mmol) was refluxed at 100°C for 6 h. The reaction mixture was then concentrated *in vacuo*. Flash chromatography (Merck Silica gel 60, 230-400 mesh, 75/25 hexanes/ethyl acetate) afforded 1-[2-(4-chloro-phenyl)-3-cyclopentyl-propionyl)-3-methyl-urea (105.3 mg, 5.8 %) as a white solid: mp 145-147°C; EI-HRMS m/e calcd for C₁₆H₂₁ClN₂O₂ (M⁺) 308.1292, found 308.1291.

### Example 131

### 1-[3-Cyclopentyl-2-(4-nitro-phenyl)-propionyl]-3-methyl-urea

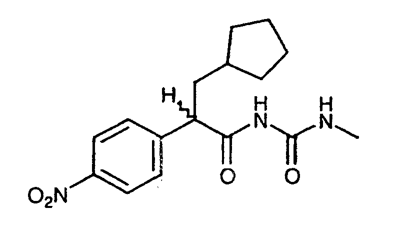

A solution of freshly prepared lithium diisopropylamide (430.55 mL of a 0.3M stock solution, 129.16 mmol) cooled to -78°C was treated with (4-nitro-phenyl)-acetic acid ethyl ester (26.32 g, 125.83 mmol) in tetrahydrofuran/hexamethylphosphoramide (312.5 mL, 3:1). The resulting solution was stirred at -78°C for 45 min. At this time, the reaction was treated with a solution of iodomethylcyclopentane (27.75 g, 132.1 mmol) in hexamethylphosphoramide (27.8 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (250 mL). The reaction mixture was concentrated *in vacuo*. The residue was diluted with water (250 mL) and extracted with ethyl acetate (3 x 300 mL). The organics were washed with a saturated aqueous lithium chloride solution (2 x 250 mL), dried over magnesium sulfate, filtered, and concentrated *in vacuo*. Flash chromatography (Merck Silica gel 60, 230-400 mesh, 95/5 hexanes/ethyl acetate) afforded 3-cyclopentyl-2-(4-nitro-phenyl)-propionic acid ethyl ester (28.30 g, 77.2%) as a yellow oil: EI-HRMS m/e calcd for C₁₆H₂₁NO₄ (M⁺) 291.1470, found 291.1470.

A mixture of 3-cyclopentyl-2-(4-nitro-phenyl)-propionic acid ethyl ester (1.27 g, 4.36 mmol) and methyl urea (647 mg, 8.73 mmol) in a solution of magnesium methoxide in methanol (7.4 wt%, 9.36 mL, 6.54 mmol) was refluxed at 100°C for 8.5 h. The reaction mixture was then concentrated *in vacuo.* Flash chromatography (Merck Silica gel 60, 230-400 mesh, 50/50 hexanes/ethyl acetate) afforded 1-[3-cyclopentyl-2-(4-nitro-phenyl)-propionyl]-3-methyl-urea (183.6 mg, 13.2 %) as a white solid: mp 179-183°C; EI-HRMS m/e calcd for C₁₆H₂₁N₃O₄ (M⁺) 319.1532, found 319.1527.

### Example 132

### 1-(3-Cyclopentyl-2-phenyl-propionyl)-3-methyl-urea

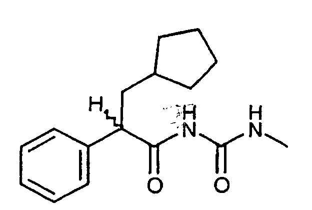

A solution of freshly prepared lithium diisopropylamide (23 mL of a 0.31M stock solution, 7.13 mmol) cooled to -78°C was treated with phenyl-acetic acid ethyl ester (1.06 g, 6.48 mmol) in tetrahydrofuran/hexamethylphosphoramide (16.1 mL, 3:1). The resulting solution was stirred at -78°C for 45 min. At this time, the reaction was treated with a solution of iodomethylcyclopentane (1.50 g, 7.14 mmol) in hexamethylphosphoramide (1.5 mL). The mixture was stirred at -78°C for 4 h. The reaction was then warmed to 25°C and was stirred at 25°C for 48 h. The reaction mixture was then quenched by the dropwise addition of a saturated aqueous ammonium chloride solution (5 mL). The reaction mixture was poured into water (100 mL) and extracted with ethyl acetate (2 x 100 mL). The organics were washed with a saturated aqueous lithium chloride solution (2 x 50 mL), dried over magnesium sulfate, filtered, and concentrated *in vacuo*. Flash chromatography (Merck Silica gel 60, 230-400 mesh, 95/5 hexanes/ethyl acetate) afforded 3-cyclopentyl-2-phenyl-propionic acid ethyl ester (1.70 g, quant.) as a pale yellow oil: EI-HRMS m/e calcd for C₁₆H₂₂O₂ (M⁺) 247.1698, found 247.1704.

A mixture of 3-cyclopentyl-2-phenyl-propionic acid ethyl ester (1.70 g, 7.06 mmol) and methyl urea (1.04 mg, 14.13 mmol) in a solution of magnesium methoxide in methanol (7.4 wt%, 130.3 mL, 21.18 mmol) was refluxed at 100°C for 24 h. The reaction mixture was then concentrated *in vacuo*. Flash chromatography (Merck Silica gel 60, 230-400 mesh, 50/50 hexanes/ethyl acetate) afforded 1-(3-cyclopentyl-2-phenyl-propionyl)-3-methyl-urea (1.21 mg, 62.4 %) as a white solid: mp 145-147°C; EI-HRMS m/e calcd for C₁₆H₂₂N₂O₂ (M⁺) 274.1681, found 274.1682.

### Example 133

### 1-[2-(4-Bromo-phenyl)-3-cyclopentyl-propionyl]-3-methyl urea

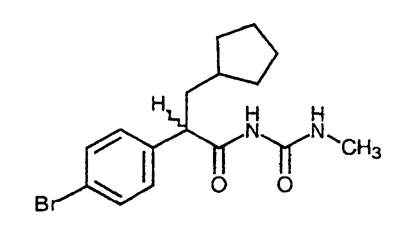

A solution of diisopropylamine (7.7 mL, 54.88 mmol) in dry tetrahydrofuran (23 mL) and 1,3-dimethyl-3,4,5,6-tetrahydro-2(1H)-pyrimidinone (10 mL) was cooled to -78°C under nitrogen and then treated with a 2.5M solution of *n*-butyllithium in hexanes (22.0 mL, 54.88 mmol). The reaction mixture was stirred at -78°C for 30 min and then treated dropwise with a solution of 4-bromophenylacetic acid (5.62 g, 26.13 mmol) in dry tetrahydrofuran (23 mL) and 1,3-dimethyl-3,4,5,6-tetrahydro-2(1H)-pyrimidinone (10 mL). The reaction mixture turned dark in color and was allowed to stir at -78°C for 1 h, at which time, a solution of iodomethylcyclopentane (5.76 g, 27.44 mmol) in a small amount of dry tetrahydrofuran was added dropwise. The reaction mixture was allowed to warm to 25°C where it was stirred for 24 h. The reaction mixture was quenched with water and then concentrated *in vacuo* to remove tetrahydrofuran. The aqueous residue was acidified using a 10% aqueous hydrochloric acid solution. The resulting aqueous layer was extracted with ethyl acetate (2 x 100 mL). The combined organic extracts were dried over sodium sulfate, filtered, and concentrated *in vacuo*. Flash chromatography (Merck Silica gel 60, 230-400 mesh, 3/1 hexanes/ethyl acetate) afforded 2-(4-bromo-phenyl)-3-cyclopentyl-propionic acid (3.88 g, 50%) as a light yellow solid: mp 91-93°C; EI-HRMS m/e calcd for C₁₄H₁₇BrO₂ (M⁺) 296.0412, found 296.0417.

A solution of 2-(4-bromo-phenyl)-3-cyclopentyl-propionic acid (1.37 g, 4.61 mmol) in methanol (23 mL) was treated slowly with 5 drops of concentrated sulfuric acid. The resulting reaction mixture was heated under reflux for 42 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aquecus sodium chloride solution (1 x 100 mL), dried over sodium sulfate, filtered, and concentrated *in vacuo*. Flash chromatography (Merck Silica gel 60, 70-230 mesh, 19/1 hexanes/ethyl acetate) afforded 2-(4-bromo-phenyl)-3-cyclopentyl-propionic acid methyl ester (1.40 g, 97%) as a light yellow oil: EI-HRMS m/e calcd for C₁₅H₁₉BrO₂ (M⁺) 310.0568, found 310.0569.

2-(4-Bromo-phenyl)-3-cyclopentyl-propionic acid methyl ester (420.0 mg, 1.35 mmol) and methyl urea (299.9 mg, 4.05 mmol) were treated with a solution of magnesium methoxide in methanol (7.4 wt%, 7.7 mL, 5.40 mmol). The resulting reaction mixture was then heated under reflux for 48 h. The reaction mixture was allowed to cool to 25°C and then filtered through celite. The celite was thoroughly washed with ethyl acetate, and the filtrate was concentrated *in vacuo.* Flash chromatography (Merck Silica gel 60, 70-230 mesh, 3/1 hexanes/ethyl acetate) afforded 1-[2-(4-bromo-phenyl)-3-cyclopentyl-propionyl]-3-methyl urea (58.7 mg, 12%) as a white solid: mp 184-186°C; EI-HRMS m/e calcd for C₁₆H₂₁BrN₂O₂ (M⁺) 352.0786, found 352.0791.

### Example 134

### 1-[3-Cyclopentyl-2-(4-trifluoromethylsulfanyl-phenyl)-propionyl]-3-methyl urea

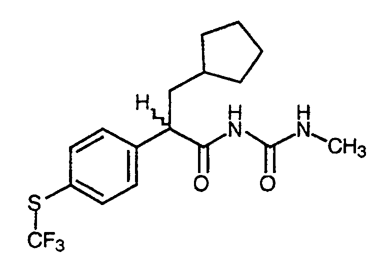

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of n-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanyl-phenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanyl-phenyl)propionic acid methyl ester (1.37 g, 99%) as a light yellow oil: EI-HRMS m/e calcd for C₁₆H₁₉F₃O₂S (M⁺) 332.1058, found 332.1052.

3-Cyclopentyl-2-(4-trifluoromethylsulfanyl-phenyl)propionic acid methyl ester (210.1 mg, 0.63 mmol) and methyl urea (140.5 mg, 1.90 mmol) were treated with a solution of magnesium methoxide in methanol (7.4 wt%, 3.6 mL, 2.53 mmol). The resulting reaction mixture was then heated under reflux for 64 h. The reaction mixture was allowed to cool to 25°C and then filtered through celite. The celite was thoroughly washed with ethyl acetate until the solvent passing through the celite showed absence of desired product by thin layer chromatography. The filtrate was concentrated *in vacuo*. Flash chromatography (Merck Silica gel 60, 70-230 mesh, 3/1 hexanes/ethyl acetate) afforded 1-[3-cyclopentyl-2-(4-trifluoromethylsulfanyl-phenyl)-propionyl]-3-methyl urea (42.7 mg, 18%) as a white solid: mp 144-145°C; EI-HRMS m/e calcd for C₁₇H₂₁F₃N₂O₂S (M⁺) 374.1276, found 374.1270.

### Example 135

### 1-[3-Cyclopentyl-2-(4-trifluoromethanesulfonyl-phenyl)-propionyl]-3-methyl urea

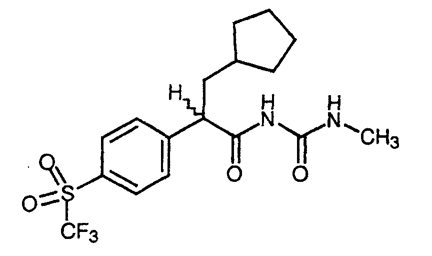

A solution of diisopropylamine (2.4 mL, 16.80 mmol) in dry tetrahydrofuran (7.5 mL) and 1,3-dimethyl-3,4,5,6-tetrahydro-2(1H)-pyrimidinone (2.5 mL) was cooled to -78°C under nitrogen and then treated with a 2.5M solution of *n*-butyllithium in hexanes (6.7 mL, 16.80 mmol). The resulting reaction mixture was stirred at -78°C for 30 min and then treated dropwise with a solution of 4-(trifluoromethylthio)phenylacetic acid (1.89 g, 8.00 mmol) in dry tetrahydrofuran (7.5 mL) and 1,3-dimethyl-3,4,5,6-tetrahydro-2(1H)-pyrimidinone (2.5 mL). The reaction mixture was allowed to stir at -78°C for 55 min, at which time, a solution of iodomethylcyclopentane (1.85 g, 8.80 mmol) in a small amount of dry tetrahydrofuran was added dropwise. The reaction mixture was allowed to warm to 25°C where it was stirred for 41 h. The reaction mixture was quenched with water and then concentrated *in vacuo* to remove tetrahydrofuran. The remaining aqueous phase was acidified to pH = 2 with a 10% aqueous hydrochloric acid solution and then extracted with ethyl acetate (1 x 300 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-trifluoromethylsulfanyl-phenyl)propionic acid (1.47 g, 58%) as a cream solid: mp 69-71°C; EI-HRMS m/e calcd for C₁₅H₁₇F₃O₂S (M⁺) 318.0901, found 318.0912.

A solution of 3-cyclopentyl-2-(4-trifluoromethylsulfanyl-phenyl)propionic acid (1.33 g, 4.18 mmol) in methanol (10 mL) was treated slowly with 4 drops of concentrated sulfuric acid. The resulting reaction mixture was heated under reflux for 36 h. The reaction mixture was allowed to cool to 25°C and then concentrated *in vacuo* to remove methanol. The residue was diluted with ethyl acetate (200 mL). The organic phase was washed with a saturated aqueous sodium bicarbonate solution (1 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 97/3 hexanes/ethyl acetate) afforded 3-cyclopentyl-2-(4-trifluoromethylsulfanyl-phenyl)propionic acid methyl ester (1.37 g, 99%) as a light yellow oil: EI-HRMS m/e calcd for C₁₆H₁₉F₃O₂S (M⁺) 332.1058, found 332.1052.

A solution of 3-cyclopentyl-2-(4-trifluoromethylsulfanyl-phenyl)propionic acid methyl ester (1.14 g, 3.43 mmol) in methylene chloride (8.6 mL) was treated with 3-chloroperoxybenzoic acid (80-85% grade, 2.00 g based on 80%, 9.26 mmol). The reaction mixture was stirred at 25°C for 17 h, at which time, thin layer chromatography showed the presence of two new lower R_{f} products. An additional 2.00 g of 3-chloroperoxybenzoic acid was added to the reaction mixture to drive the conversion of the sulfoxide to the sulfone, and the resulting reaction mixture was stirred at 25°C for 3 d. The reaction mixture was concentrated *in vacuo* to remove methylene chloride. The resulting residue was diluted with ethyl acetate (300 mL). The organic phase was washed with a saturated aqueous sodium bicarbonate solution (3 x 100 mL), washed with a saturated aqueous sodium chloride solution (1 x 100 mL), dried over sodium sulfate, filtered, and concentrated *in vacuo*. Flash chromatography (Merck Silica gel 60, 70-230 mesh, 19/1 hexanes/ethyl acetate) afforded 3-cyclopentyl-2-(4-trifluoromethanesulfonyl-phenyl)propionic acid methyl ester (1.19 g, 95%) as a light yellow oil: EI-HRMS m/e calcd for C₁₆H₁₉F₃O₄S (M⁺) 364.0956, found 364.0965.

3-Cyclopentyl-2-(4-trifluoromethanesulfonyl-phenyl)propionic acid methyl ester (383.8 mg, 1.05 mmol) and methyl urea (234.1 mg, 3.16 mmol) were treated with a solution of magnesium methoxide in methanol (7.4 wt%, 6.0 mL, 4.21 mmol). The resulting reaction mixture was then heated under reflux for 2 d. The reaction mixture was allowed to cool to 25°C and then filtered through celite. The celite was thoroughly washed with ethyl acetate until the solvent passing through the celite showed absence of desired product by thin layer chromatography. The filtrate was concentrated *in vacuo*. Flash chromatography (Merck Silica gel 60, 70-230 mesh, 3/1 hexanes/ethyl acetate) afforded 1-[3-cyclopentyl-2-(4-trifluoromethanesulfonyl-phenyl)-propionyl]-3-methyl urea (119.3 mg, 28%) as a white solid: mp 191-192°C; FAB-HRMS m/e calcd for C₁₇H₂₁F₃N₂O₄S (M+H)⁺ 407.1252, found 407.1247.

### Example 136

### 1-[3-Cyclopentyl-2-(4-methylsulfanyl-phenyl)-propionyl]-3-methyl urea

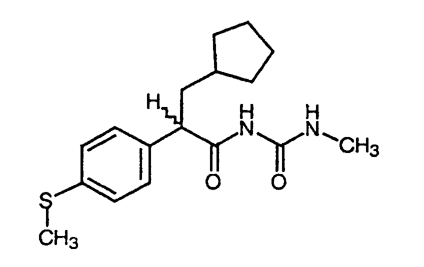

A solution of diisopropylamine (3.2 mL, 23.16 mmol) in dry tetrahydrofuran (10.3 mL) and 1,3-dimethyl-3,4,5,6-tetrahydro-2(1H)-pyrimidinone (3.4 mL) was cooled to -78°C under nitrogen and then treated with a 10M solution of n-butyllithium in hexanes (2.3 mL, 23.16 mmol). The resulting reaction mixture was stirred at -78°C for 30 min and then treated dropwise with a solution of 4-(methylthio)phenylacetic acid (2.01 g, 11.03 mmol) in dry tetrahydrofuran (10.3 mL) and 1,3-dimethyl-3,4,5,6-tetrahydro-2(1H)-pyrimidinone (3.4 mL). The reaction mixture was allowed to stir at -78°C for 1 h, at which time, a solution of iodomethylcyclopentane (2.55 g, 12.13 mmol) in a small amount of dry tetrahydrofuran was added dropwise. The reaction mixture was stirred at - 78°C for 30 min and then allowed to warm to 25°C where it was stirred for 24 h. The reaction mixture was quenched with water and then concentrated in *vacuo* to remove tetrahydrofuran. The remaining aqueous phase was acidified to pH = 2 with a 10% aqueous hydrochloric acid solution and then extracted with ethyl acetate (200 mL). The organic layer was washed with a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 3-cyclopentyl-2-(4-methylsuifanyl-phenyl)propionic acid (1.01 g, 35%) as a cream solid: mp 91-93°C; EI-HRMS m/e calcd for C₁₅H₂₀O₂S (M⁺) 264.1184, found 264.1177.

A solution of 3-cyclopentyl-2-(4-methylsulfanyl-phenyl)propionic acid (500 mg, 1.89 mmol) in methanol (8 mL) was treated slowly with 2 drops of concentrated sulfuric acid. The resulting reaction mixture was heated under reflux for 15 h. The reaction mixture was allowed to cool to 25°C and then concentrated *in vacuo*. The orange residue was partitioned between water and ethyl acetate. The organic layer was dried over magnesium sulfate, filtered, and concentrated *in vacuo* to provide pure 3-cyclopentyl-2-(4-methylsulfanyl-phenyl)propionic acid methyl ester (481 mg, 91%) as a yellow-orange oil which was used without further purification: EI-HRMS m/e calcd for C₁₆H₂₂O₂S (M⁺) 278.1341, found 278.1347. 3-Cyclopentyl-2-(4-methylsulfanyl-phenyl)propionic acid methyl ester (400 mg, 1.44 mmol) and methyl urea (267 mg, 3.60 mmol) were treated with a solution of magnesium methoxide in methanol (7.4 wt%, 5.6 mL, 3.89 mmol). The reaction mixture was then concentrated *in vacuo* to approximately one-half the volume of methanol. The resulting reaction mixture was then heated under reflux for 15 h. The reaction mixture was allowed to cool to 25°C, filtered through celite, and the celite was thoroughly washed with ethyl acetate. The ethyl acetate filtrate was washed with water. The organic layer was dried over magnesium sulfate, filtered, and concentrated *in vacuo*. Flash chromatography (Merck Silica gel 60, 230-400 mesh, 2/1 hexanes/ethyl acetate) afforded 1-[3-cyclopentyl-2-(4-methylsulfanyl-phenyl)-propionyl]-3-methyl urea (141 mg, 31%) as a white solid: mp 185-186°C; EI-HRMS m/e calcd for C₁₇H₂₄N₂O₂S (M⁺) 320.1559, found 320.1559.

### Example 137

### 1-[3-Cyclopentyl-2-(4-methanesulfonyl-phenyl)-propionyl]-3-methyl urea

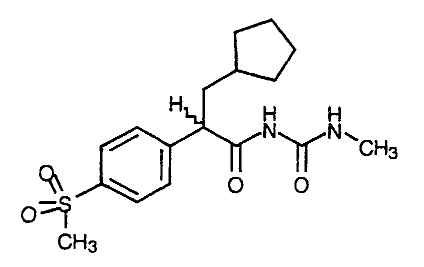

A solution of diisopropylamine (3.3 mL, 23.5 mmol) in dry tetrahydrofuran (50 mL) and 1,3-dimethyl-3,4,5,6-tetrahydro-2(1H)-pyrimidinone (10 mL) was cooled to -78°C under nitrogen and then treated with a 10M solution of *n*-butyllithium in hexanes (2.35 mL, 23.5 mmol). The yellow reaction mixture was stirred at -78°C for 30 min and then treated dropwise with a solution of 4-methylsulfonylphenylacetic acid (2.40 g, 11.2 mmol) in a small amount of dry tetrahydrofuran. After approximately one-half of the 4-methylsulfonylphenylacetic acid in dry tetrahydrofuran was added, a precipitate formed. Upon further addition of the remaining 4-methylsulfonylphenylacetic acid in dry tetrahydrofuran, the reaction mixture became thick in nature. After complete addition of the 4-methylsulfonylphenylacetic acid in dry tetrahydrofuran, the reaction mixture was very thick and became difficult to stir. An additional amount of dry tetrahydrofuran (20 mL) was added to the thick reaction mixture, and the reaction mixture was stirred at - 78°C for 45 min, at which time, a solution of iodomethylcyclopentane (2.35 g, 11.2 mmol) in a small amount of dry tetrahydrofuran was added dropwise. The reaction mixture was allowed to warm to 25°C where it was stirred for 15 h. The reaction mixture was quenched with water (100 mL), and the resulting yellow reaction mixture was concentrated *in vacuo* to remove tetrahydrofuran. The aqueous residue was acidified to pH = 2 using concentrated hydrochloric acid. The aqueous layer was extracted with ethyl acetate. The organic phase was dried over magnesium sulfate, filtered, and concentrated *in vacuo*. Flash chromatography (Merck Silica gel 60, 230-400 mesh, 1/3 hexanes/ethyl acetate) afforded 3-cyclopentyl-2-(4-methanesulfonyl-phenyl)propionic acid (1.80 g, 52%) as a white solid: mp 152-154°C; EI-HRMS m/e calcd for C₁₅H₂₀O₄S (M⁺) 296.1082, found 296.1080.

A solution of 3-cyclopentyl-2-(4-methanesulfonyl-phenyl)propionic acid (500 mg, 1.89 mmol) in methanol (15 mL) was treated slowly with concentrated sulfuric acid (3 drops). The resulting reaction mixture was heated under reflux for 15 h. The reaction mixture was allowed to cool to 25°C and then concentrated *in vacuo*. Flash chromatography (Merck Silica gel 60, 230-400 mesh, 1/3 hexanes/ethyl acetate) afforded 3-cyclopentyl-2-(4-methanesulfonyl-phenyl)propionic acid methyl ester (377 mg, 72%) as a white solid: mp 63-66°C; EI-HRMS m/e calcd for C₁₆H₂₂O₄S (M⁺) 310.1239, found 310.1230.

3-Cyclopentyl-2-(4-methanesulfonyl-phenyl)propionic acid methyl ester (350 mg, 1.13 mmol) and methyl urea (184 mg, 2.48 mmol) were treated with a solution of magnesium methoxide in methanol (7.4 wt%, 6.0 mL, 4.18 mmol). The reaction mixture was then concentrated *in vacuo* to approximately one-half the volume of methanol. The resulting reaction mixture was then heated under reflux for 15 h. The reaction mixture was allowed to cool to 25°C, filtered through celite, and the celite was thoroughly washed with ethyl acetate. The ethyl acetate filtrate was washed with water. The organic layer was dried over magnesium sulfate, filtered, and concentrated *in vacuo*. Flash chromatography (Merck Silica gel 60, 230-400 mesh, 1/3 hexanes/ethyl acetate) afforded 1-[3-cyclopentyl-2-(4-methanesulfonyl-phenyl)-propionyl]-3-methyl urea (124 mg, 31%) as a white solid: mp 205-206°C; EI-HRMS m/e calcd for C₁₇H₂₄N₂O₄S (M⁺) 352.1457, found 352.1445.

### Example 138

### (A) 1-[3-Cyclopentyl-2-(3,4-dichloro-phenyl)-propionyl]-3-ethyl-urea

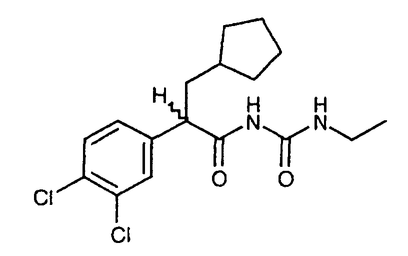

A solution of triphenylphosphine (28.80 g, 109.8 mmol) and imidazole (14.9 g, 219.6 mmol) in methylene chloride (160 mL) was cooled to 0°C and then slowly treated with iodine (27.87 g, 109.8 mmol). The reaction mixture was then treated dropwise with a solution of cyclopentylmethanol (10.0 g, 99.8 mmol) in methylene chloride (10 mL). The resulting reaction mixture was allowed to warm to 25°C, where it was stirred for 4 h. The reaction mixture was then diluted with water (50 mL), and the reaction mixture was further extracted with methylene chloride (3 x 20 mL). The combined organic layers were dried over sodium sulfate, filtered and concentrated *in vacuo* at 25°C. The resulting solid was washed with pentane (4 x 50 mL) and filtered through a silica gel plug. The filtrate was concentrated *in vacuo* at 25°C to afford iodomethylcyclopentane (18.48 g, 88%) as a clear colorless liquid: EI-HRMS m/e calcd for C₆H₁₁I (M⁺) 209.9906, found 209.9911.

A solution of diisopropylamine (13.36 mL, 101.89 mmol) in tetrahydrofuran (250 mL) was cooled to -78°C under a nitrogen atmosphere and then treated with a 2.0M solution of *n*-butyllithium in hexanes (51 mL, 101.89 mmol). The reaction mixture was stirred at - 78°C for 15 min, at which time, a solution of 3,4-dichlorophenyl acetic acid (9.08 g, 44.3 mmol) in tetrahydrofuran (60 mL) and hexamethylphosphoramide (20 mL) was slowly added via a cannula. The bright yellow solution was allowed to stir at -78°C for 1 h, at which time, a solution of iodomethylcyclopentane (11.17 g, 53.2 mmol) in hexamethylphosphoramide (10 mL) was added via a cannula. The reaction mixture was stirred at -78°C for 1 h. The reaction mixture was then allowed to warm to 25°C, where it was stirred for 14 h. The reaction mixture was then acidified to pH = 2 by the dropwise addition of a 1N aqueous hydrochloric acid solution and extracted with ethyl acetate (3 x 50 mL). The combined organic layers were dried with sodium sulfate, filtered, and concentrated *in vacuo*. Flash chromatography (Merck Silica gel 60, 230-400 mesh, chloroform then 99/1 chloroform/methanol) afforded 3-cyclopentyl-2-(3,4-dichlorophenyl)-propionic acid (10.28 g, 81%) as a white solid: mp 74.5-76.9°C; EI-HRMS m/e calcd for C₁₄H₁₆Cl₂O₂ (M⁺) 286.0527, found 286.0534.

A solution of 3-cyclopentyl-2-(3,4-dichlorophenyl)-propionic acid (366 mg, 1.27 mmol) in methylene chloride (10 mL) and 1 drop of *N*,*N*-dimethylformamide was cooled to 0°C and then treated with a 2.0M solution of oxalyl chloride in methylene chloride (0.76 mL, 1.53 mmol). The reaction was stirred for 30 min at 0°C, at which time, 1,1,1,3,3,3-hexamethyldisilazane (0.81 mL, 3.81 mmol) was added to the reaction mixture. The reaction was allowed to slowly warm to 25°C and then stirred at 25°C for 16 h. The reaction mixture was then treated with methanol (5 mL). The resulting reaction mixture was washed with a 5% aqueous sulfuric acid solution (2 x 10 mL). The combined aqueous layers were extracted with methylene chloride (3 x 10 mL). The combined organic layers were then washed with a saturated aqueous sodium chloride solution (1 x 10 mL), dried over magnesium sulfate, filtered and concentrated *in vacuo*. Flash chromatography (Merck Silica gel 60, 230-400 mesh, 70/30 hexanes/ethyl acetate) afforded 3-cyclopentyl-2-(3,4-dichloro-phenyl)-propionamide (229 mg, 63%) as a white solid: mp 98.6-100.1°C; EI-HRMS m/e calcd for C₁₄H₁₇Cl₂NO (M⁺) 285.0687, found 285.0688.

A solution of 3-cyclopentyl-2-(3,4-dichloro-phenyl)-propionamide (98 mg, 0.33 mmol) in toluene (10 mL) was treated with ethyl isocyanate (0.03 mL, 0.42 mmol). The resulting solution was heated under reflux for 24 h. At this time, the reaction was concentrated *in vacuo*. Flash chromatography (Merck Silica gel 60, 230-400 mesh, 90/10 hexanes/ethyl afforded 1-[3-cyclopentyl-2-(3,4-dichloro-phenyl)-propionyl]-3-ethyl-urea (29 mg, 26%) as a white foam: EI-HRMS m/e calcd for C₁₇H₂₂Cl₂N₂O₂ (M⁺) 356.1058, found 356.1066.

### (B) In an analogous manner, there were obtained:

(a) From 3-cyclopentyl-2-(3,4-dichloro-phenyl)-propionamide and isopropyl isocyanate: 1-[3-Cyclopentyl-2-(3,4-dichloro-phenyl)-propionyl]-3-isopropyl-urea as a white solid: mp 134.6-138.3°C; EI-HRMS m/e calcd for C₁₈H₂₄Cl₂N₂O₂ (M⁺) 370.1215, found 370.1232.
(b) From 3-cyclopentyl-2-(3,4-dichloro-phenyl)-propionamide and propyl isocyanate: 1-[3-Cyclopentyl-2-(3,4-dichloro-phenyl)-propionyl]-3-propyl-urea as a white solid: mp 117.8-120°C; EI-HRMS m/e calcd for C₁₈H₂₄Cl₂N₂O₂ (M⁺) 370.1215, found 370.1209.
(c) From 3-cyclopentyl-2-(3,4-dichloro-phenyl)-propionamide and ethyl 3-isocyanatopropionate: 3-{3-[3-Cyclopentyl-2-(3,4-dichloro-phenyl)-propionyl]-ureido}-propionic acid ethyl ester as a light yellow oil: EI-HRMS m/e calcd for C₂₀H₂₆Cl₂N₂O₄ (M⁺) 428.1270, found 428.1265.
(d) From 3-cyclopentyl-2-(3,4-dichloro-phenyl)-propionamide and ethyl isocyanatoacetate: {3-[3-Cyclopentyl-2-(3,4-dichloro-phenyl)-propionyl}-ureido}-acetic acid ethyl ester as a light yellow oil: EI-HRMS m/e calcd for C₁₉H₂₄Cl₂N₂O₄ (M⁺) 414.1113, found 414.1108.
(e) From 3-cyclopentyl-2-(3,4-dichloro-phenyl)-propionamide and allyl isocyanate: 1-Allyl-3-[3-cyclopentyl-2-(3,4-dichloro-phenyl)-propionyl]-urea as a clear colorless oil: EI-HRMS m/e calcd for C₁₈H₂₂Cl₂N₂O₂ (M⁺) 368.1058, found 368.1064.

### Example 139

### 1-[3-Cyclopentyl-2(R)-(3,4-dichloro-phenyl)-propionyl]-3-methyl-urea

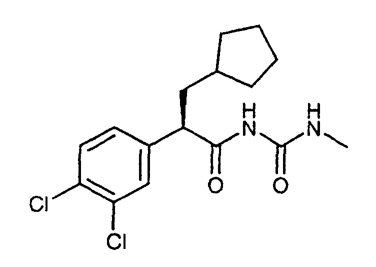

A solution of 3-cyclopentyl-2-(3,4-dichloro-phenyl)-propionic acid (prepared in Example 12, 5.00 g, 17.4 mmol) in tetrahydrofuran (150 mL) cooled to -78°C was treated with triethylamine (2.77 mL, 19.9 mmol) followed by trimethylacetyl chloride (2.24 mL, 18.2 mmol). The resulting white slurry was stirred at -78°C for 15 min and then at 0°C for 45 min. In a separate flask, a solution of (S)-4-isopropyl-2-oxazolidinone (2.14 g, 16.57 mmol) in tetrahydrofuran (80 mL) cooled to -78°C was treated with a 2.0M solution of n-butyllithium in hexanes (8.7 mL, 17.4 mmol). This solution was stirred at -78°C for 10 min. It was then warmed 25°C and stirred for an additional 10 min. At this time, the first reaction mixture was re-cooled to -78°C. The second reaction mixture was added to the first reaction mixture over a period of 5 min via cannula. The combined reaction was then stirred at -78°C for 15 min. It was then warmed to 25°C and was stirred for an additional 1.5 h. At this time, the reaction was quenched by the addition of a saturated aqueous sodium bisulfite solution (50 mL) and extracted with ethyl acetate (3 x 40 mL). The organic layers were combined, washed with a saturated aqueous sodium bicarbonate solution (1 x 20 mL), a saturated aqueous sodium chloride solution (1 x 20 mL), dried over sodium sulfate, filtered, and concentrated *in vacuo*. Flash chromatography (Merck Silica gel 60, 230-400 mesh, 85/15 hexanes/ethyl acetate) afforded (1) 3-[3-cyclopentyl-2(S)-(3,4-dichloro-phenyl)-propionyl]-4(S)-isopropyl-oxazolidin-2-one (2.15 g, 33%) as a clear oil: [α]²³₅₈₉ = +87.5° (c=0.160, chloroform); EI-HRMS m/e calcd for C₂₀H₂₅Cl₂NO₃ (M⁺) 397.1211, found 397.1215 and (2) 3-[3-cyclopentyl-2(R)-(3,4-dichloro-phenyl)-propionyl]-4(S)-isopropyl-oxazolidin-2-one (1.88 g, 28%) as a white solid: mp 71.9-74.6°C; [α]²³₅₈₉ = -27.6° (c=0.188, chloroform); EI-HRMS m/e calcd for C₂₀H₂₅Cl₂NO₃ (M⁺) 397.1211, found 397.1212.

A solution of 3-[3-cyclopentyl-2(R)-(3,4-dichloro-phenyl)-propionyl]-4(S)-isopropyloxazolidin-2-one (1.88 g, 4.72 mmol) in tetrahydrofuran (73 mL) and water (22 mL) cooled to 0°C was treated with a 30% aqueous hydrogen peroxide solution (2.1 mL) and lithium hydroxide (394 mg, 9.4 mmol). The reaction was stirred at 0°C for 1 h. At this time, the reaction was quenched with a saturated aqueous sodium sulfite solution (16 mL) followed by the addition of an aqueous solution of 0.5N sodium bicarbonate (50 mL). The tetrahydrofuran was then removed *in vacuo*. The residue was diluted with water (40 mL) and extracted with methylene chloride (3 x 20 mL). The aqueous layer was then acidified to pH = 2 with a 5N aqueous hydrochloric acid solution and extracted with ethyl acetate (4 x 25 mL). The ethyl acetate layers were then dried over sodium sulfate, filtered, and concentrated *in vacuo* to afforded of 3-cyclopentyl-2(R)-(3,4-dichloro-phenyl)-propionic acid (928 mg, 70%) as a white solid: mp 75.1-78.3°C; [α]²³₅₈₉= -50.3° (c=0.100, chloroform); EI-HRMS m/e calcd for C₁₄H₁₆Cl₂O₂ (M⁺) 286.0527, found 286.0535.

A solution of 3-cyclopentyl-2(R)-(3,4-dichlorophenyl)-propionic acid (105 mg, 0.37 mmol) in methylene chloride (10 mL) and 1 drop of *N*,*N*-dimethylformamide was cooled to 0°C and then treated with a 2.0M solution of oxalyl chloride in methylene chloride (0.18 mL, 0.37 mmol). The reaction was stirred for 30 min at 0°C, at which time, 1,1,1,3,3,3-hexamethyldisilazane (0.25 mL, 1.17 mmol) was added to the reaction mixture. The reaction was then allowed to slowly warm to 25°C and stirred at 25°C for 16h. The reaction mixture was then washed with a 5% aqueous sulfuric acid solution (2 x 10 mL). The combined aqueous layers were extracted with methylene chloride (3 x 10 mL). The combined organic layers were washed with a saturated aqueous sodium chloride solution (1 x 10 mL), dried over magnesium sulfate, filtered and concentrated *in vacuo*. Flash chromatography (Merck Silica gel 60, 230-400 mesh, 60/40 hexanes/ethyl acetate) afforded 3-cyclopentyl-2(R)-(3,4-dichloro-phenyl)-propionamide (60 mg, 58%) as a white solid: [α]²³₅₈₉ = -67.6° (c=0.106, chloroform); EI-HRMS m/e calcd for C₁₄H₁₇Cl₂N₁O₁ (M⁺) 285.0687, found 285.0685.

A solution of 3-cyclopentyl-2(R)-(3,4-dichloro-phenyl)-propionamide (54 mg, 0.19 mmol) in toluene (5 mL) was treated with methyl isocyanate (0.03 mL, 0.47 mmol). The resulting solution was heated under reflux for 24 h. At this time, the reaction was concentrated *in vacuo*. Flash chromatography (Merck Silica gel 60, 230-400 mesh 70/30 hexanes/ethyl acetate) afforded 1-[3-cyclopentyl-2(R)-(3,4-dichloro-phenyl)-propionyl]-3-methyl-urea (40 mg, 63%) as a white solid: mp 124.8-127.5 °C; [α]²³₅₈₉ = -21.2° (c=0.099, chloroform); EI-HRMS m/e calcd for C₁₆H₂₀Cl₂N₂O₂ (M⁺) 342.0902, found 342.0902.

### Example 140

### 1-[3-Cyclopentyl-2-(4-methanesulfonyl-3-nitrophenyl)-propionyl]-3-methyl-urea

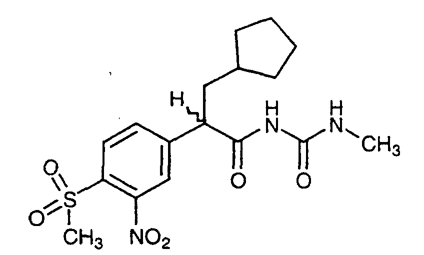

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo.* Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) was cooled to -78°C and then treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h, at which time, a solution of iodomethylcyclopentane (4.58 g, 21.8 mol) in a small amount of dry tetrahydrofuran was added dropwise. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentyl-propionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then diluted with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (865 mg, 2.43 mmol) in tetrahydrofuran (6 mL) was treated with a 0.8M aqueous lithium hydroxide solution (4.6 mL, 3.65 mmol). The reaction mixture was stirred at 25°C for 3 h. The reaction mixture was concentrated *in vacuo* to remove tetrahydrofuran. The resulting aqueous residue was diluted with water (25 mL) and then treated with a 1N aqueous hydrochloric acid solution (10 mL). The resulting aqueous layer was then extracted with ethyl acetate (2 x 50 mL). The combined organic layers were dried over magnesium sulfate, filtered, and concentrated *in vacuo*. Flash chromatography (Merck Silica gel 60, 230-400 mesh, 1/4 hexanes/ethyl acetate) afforded 3-cyclopentyl-2-(4-methanesulfonyl-3-nitrophenyl)-propionic acid (723 mg, 87%) as a white foam. Analytical data indicated the presence of a small impurity; however, the 3-cyclopentyl-2-(4-methanesulfonyl-3-nitrophenyl)-propionic acid was used without further purification in subsequent reactions.

A mixture of 3-cyclopentyl-2-(4-methanesulfonyl-3-nitrophenyl)-propionic acid (300 mg, 0.88 mmol) and 1 drop of *N*,*N*-dimethylformamide in methylene chloride (2 mL) was cooled to 0°C and then slowly treated with oxalyl chloride (84 µL, 0.97 mmol). The reaction mixture was stirred at 0°C for 10 min and then stirred at 25°C for 1 h. The resulting reaction mixture was then treated dropwise with 1,1,1,3,3,3-hexamethyldisilazane (560 µL, 2.64 mmol) and subsequently stirred at 25°C for 15 h. The resulting reaction mixture was diluted with methylene chloride (20 mL) and methanol (15 mL) and then washed with a 5% aqueous sulfuric acid solution (20 mL). The organic layer was dried over sodium sulfate, filtered, and concentrated *in vacuo*.

Flash chromatography (Merck Silica gel 60, 230-400 mesh, 1/3 hexanes/ethyl acetate) afforded 3-cyclopentyl-2-(4-methanesulfonyl-3-nitrophenyl)-propionamide (140 mg, 47%) as a yellow foam: mp 72-76°C (foam to gel); FAB-HRMS m/e calcd for C₁₅H₂₀N₂O₅S (M+H)⁺ 341.1172, found 341.1181.

A solution of 3-cyclopentyl-2-(4-methanesulfonyl-3-nitrophenyl)-propionamide (126 mg, 0.37 mmol) and methyl isocyanate (211 mg, 3.70 mmol) in toluene (2 mL) was heated under reflux (120°C) for 15 h. The reaction mixture was allowed to cool to 25°C and then concentrated *in vacuo*. The resulting yellow oil was treated with a small amount of a 1/1 mixture of hexanes/ethyl acetate, and a precipitate started to form. The material was further cooled in the freezer for 2 h to facilitate additional precipitation. The solid was collected by filtration and then dried *in vacuo* to afford 1-[3-cyclopentyl-2-(4-methanesulfonyl-3-nitrophenyl)-propionyl]-3-methyl-urea (50 mg, 35%) as a pale yellow solid: mp 241-242°C; FAB-HRMS m/e calcd for C₁₇H₂₃N₃O₆S (M+H)⁺ 398.1386, found 398.1399.

### Example 141

### 1-[3-Cyclopentyl-2(R)-(3,4-dichloro-phenyl)-propionyl]-3-ethyl-urea

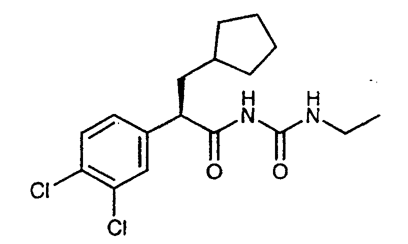

A solution of 3-cyclopentyl-2(R)-(3,4-dichloro-phenyl)-propionamide (prepared in Example **139**, 103 mg, 0.36 mmol) in toluene (10 mL) was treated with ethyl isocyanate (40 µL, 0.54 mmol). The resulting reaction mixture was heated under reflux for 24 h. The reaction mixture was then concentrated *in vacuo.* Flash chromatography (Merck Silica gel 60, 230-400 mesh, 4/1 hexanes/ethyl acetate) afforded 1-[3-cyclopentyl-2(R)-(3,4-dichloro-phenyl)-propionyl]-3-ethyl-urea (54 mg, 42%) as a white foam: [α]²³₅₈₉ = - 41.9° (c=0.031, chloroform); FAB-HRMS m/e calcd for C₁₇H₂₂Cl₂N₂O₂ (M+H)⁺ 357.1136, found 357.1137.

### Example 142

### [2-(4-Chloro-phenyl)-4-methyl-pentanoyl]-urea

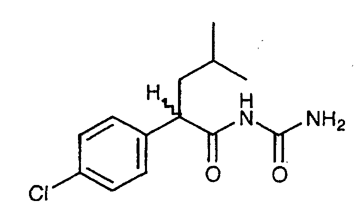

A solution of (4-chloro-phenyl)-acetic acid (6.29 g, 0.03 mol) in ethanol (38.4 mL) was treated with a catalytic amount of sulfuric acid. The reaction mixture was refluxed for 12 h. The reaction was then concentrated *in vacuo*. Flash chromatography (Merck Silica gel 60, 230-400 mesh, 50/50 hexanes/ethyl acetate) afforded (4-chloro-phenyl)-acetic acid ethyl ester (6.45 g, 88%) as a pale yellow solid: mp 39-41°C; EI-HRMS m/e calcd for C₁₀H₁₁ClO₂ (M⁺) 198.0448, found 198.0452.

A solution of freshly prepared lithium diisopropylamide (21.2 mL of a 0.31M stock solution, 6.14 mmol) cooled to -78°C was treated with (4-chloro-phenyl)-acetic acid ethyl ester (1.11 g, 5.58 mmol) in tetrahydrofuran/hexamethylphosphoramide (13.9 mL, 3:1). The resulting solution was stirred at -78°C for 45 min. At this time, the reaction was treated with a solution of 1-bromo-2-methyl-propane (1.81 mL, 16.7 mmol) in hexamethylphosphoramide (1 mL). The mixture was stirred at -78°C for 3 h. The reaction was then warmed to 25°C and was stirred at 25°C for 16 h. The reaction mixture was then quenched by the dropwise addition of a saturated aqueous ammonium chloride solution (20 mL). The reaction mixture was poured into water (100 mL) and extracted with ethyl acetate (3 x 50 mL). The organics layers dried over sodium sulfate, filtered, and concentrated *in vacuo*. Flash chromatography (Merck Silica gel 60, 230-400 mesh, 50/50 hexanes/ethyl acetate) afforded 2-(4-chloro-phenyl)-4-methyl-pentanoic acid ethyl ester (1.24 g, 87.1%) as a white solid: mp 34-35°C; EI-HRMS m/e calcd for C₁₄H₁₉ClO₂ (M⁺) 254.1074, found 254.1069.

A mixture of 2-(4-chloro-phenyl)-4-methyl-pentanoic acid ethyl ester (508 mg, 1.99 mmol) and urea (239 mg, 3.99 mmol) in a solution of magnesium methoxide in methanol (7.4 wt%, 4.28 mL, 2.99 mmol) was heated to reflux for 24 h. The reaction mixture was then concentrated *in vacuo*. Flash chromatography (Merck Silica gel 60, 230-400 mesh, 50/50 hexanes/ethyl acetate) afforded [2-(4-chloro-phenyl)-4-methyl-pentanoyl]-urea (28.1 mg, 5.2%) as a white solid: mp 164-165°C; EI-HRMS m/e calcd for C₁₃H₁₇ClN₂O₂ (M⁺) 268.0979, found 268.0972.

### Example 143

### R-[2-(3,4-Dichloro-phenyl)-4-methyl-pentanoyl]-urea

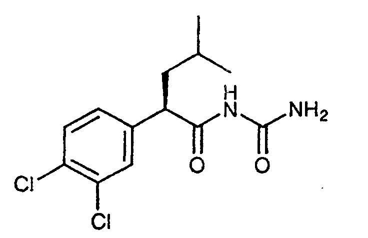

A solution of (3,4-dichloro-phenyl)-acetic acid (10.0 g, 0.048 mol) in ethanol (50 mL) was treated with a catalytic amount of sulfuric acid. The reaction mixture was refluxed for 7 h. The reaction was concentrated *in vacuo*, diluted with diethyl ether, and poured into water. The ether layer was washed with a saturated aqueous sodium bicarbonate solution and water. The organics were then dried over sodium sulfate, filtered, and concentrated *in vacuo*. Vacuum distillation (bath temperature: 175°C; head temperature: 125°C) afforded (3,4-dichloro-phenyl)-acetic acid ethyl ester (9.38 g, 82.5%) as a clear oil: EI-HRMS m/e calcd for C₁₀H₁₀Cl₂O₂ (M⁺) 232.0058, found 232.0066.

A solution of freshly prepared lithium diisopropylamide (4.88 mL of 0.29M stock solution, 1.41 mmol) cooled to -78°C was treated with (3,4-dichloro-phenyl)-acetic acid ethyl ester (300 mg, 1.28 mmol) in tetrahydrofuran/hexamethylphosphoramide (3.2 mL, 3:1). The resulting solution was stirred at -78°C for 45 min. At this time, the reaction was treated with a solution of 1-bromo-2-methyl-propane (1.53 mL, 1.41 mmol) in hexamethylphosphoramide (1 mL). The reaction mixture was stirred at -78°C for 6 h. The reaction was then warmed to 25°C and stirred at 25°C for 16 h. The reaction mixture was then quenched by the dropwise addition of saturated aqueous ammonium chloride solution (1 mL). This mixture was poured into water (50 mL) and extracted with ethyl acetate (3 x 50 mL). The organics were dried over sodium sulfate, filtered, and concentrated *in vacuo*. Flash chromatography (Merck Silica gel 60, 230-400 mesh, 50/50 hexanes/ethyl acetate) afforded 2-(3,4-dichloro-phenyl)-4-methyl-pentanoic acid ethyl ester (356.4 mg, 95.8%) as a clear oil: EI-HRMS m/e calcd for C₁₄H₁₈Cl₂O₂ (M⁺) 288.0683, found 288.0677.

A mixture of 2-(3,4-dichloro-phenyl)-4-methyl-pentanoic acid ethyl ester (197 mg, 0.68 mmol) and urea (82 mg, 1.36 mmol) in a solution of magnesium methoxide in methanol (7.4 wt%, 1.46 mL, 1.02 mmol) was heated to reflux for 3 h. The reaction mixture was then concentrated *in vacuo*. Flash chromatography (Merck Silica gel 60, 230-400 mesh, 75/25 hexanes/ethyl acetate) followed by high pressure liquid chromatography [Chirobiotic T, 5µM, 25cm x 4.6mm ID, 60/40 buffer (0.1% triethylamine in water titrated to pH 5 with glacial acetic acid)/ethanol] afforded R-[2-(3,4-dichloro-phenyl)-4-inethyl-pentanoyl]-urea (120.0 mg, 58.1%) as a white solid: mp 138-140°C; EI-HRMS m/e calcd for C₁₃H₁₆Cl₂N₂O₂ (M⁺) 302.0589, found 302.0595.

### Example 144

### 1-[3-Cyclopentyl-2-(3,4-dichloro-phenyl)-propionyl]-3-(2-hydroxy-ethyl)-urea

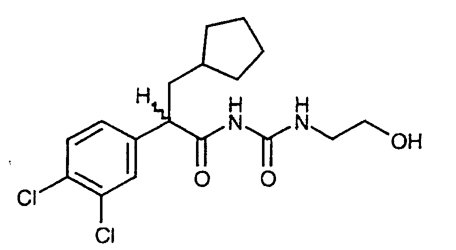

A solution of 1-allyl-3-[3-cyclopentyl-2-(3,4-dichloro-phenyl)-propionyl]-urea (prepared in Example 12B-e, 75 mg, 0.20 mmol) in dry methylene chloride (10 mL) and methanol (2 drops, needed to solubilize the compound) was cooled to -78°C and deoxygenated by bubbling argon through the reaction mixture. Ozone was then generated and bubbled through the reaction until a blue color appeared and then the reaction was stirred for five min.

At this time, argon was bubbled through the solution again until the blue color disappeared. Triphenylphosphine (54 mg, 0.20 mmol) was then added and the reaction warmed to 25°C and stirred for 16 h. At this time, the reaction was concentrated *in vacuo* and then dissolved in dry methanol (10 mL). The reaction was cooled to 0°C and then slowly treated with sodium borohydride (31 mg, 0.81 mmol). The reaction was then warmed to 25°C and stirred for 1 h. It was then quenched with water (10 mL) and extracted with ethyl acetate (3 x 15 mL). The organics were combined and washed with water (1 x 15 mL), a saturated aqueous sodium chloride solution (1 x 15 mL), dried over sodium sulfate, filtered, and concentrated *in vacuo*. Flash chromatography (Merck Silica gel 60, 230-400 mesh, 1/1 hexanes/ethyl acetate) afforded 1-[3-cyclopentyl-2-(3,4-dichloro-phenyl)-propionyl]-3-(2-hydroxy-ethyl)-urea (48 mg, 64%) as a hygroscopic white solid: EI-HRMS m/e calcd for C₁₇H₂₂Cl₂N₂O₃ (M⁺) 370.1215, found 370.1209.

### Example 145

### 1-[3-Cyclopentyl-2-(3,4-dichloro-phenyl)-propionyl]-3-(2-hydroxy-propyl)-urea

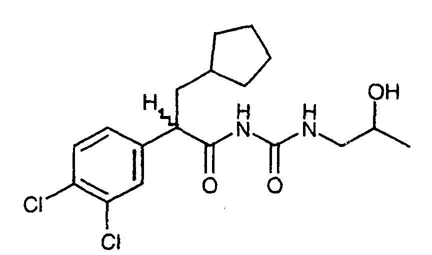

A solution of 1-allyl-3-[3-cyclopentyl-2-(3,4-dichloro-phenyl)-propionyl]-urea (prepared in Example 12B-e, 132 mg, 0.36 mmol) in tetrahydrofuran (10 mL) cooled to 0°C was treated with a 1M solution of borane-tetrahydrofuran (0.7 mL, 0.72 mmol). The reaction mixture was allowed to warm from 0°C to 25°C over 1 h. At this time, the solution was re-cooled to 0°C and treated with ethanol (2 mL) followed by the slow addition of a mixture of a saturated aqueous sodium bicarbonate solution (6 mL) and 30% hydrogen peroxide (2 mL). The resulting mixture was allowed to slowly warm to 25°C over 1 h. At this time, the reaction was re-cooled to 0°C and was slowly quenched with a saturated aqueous sodium sulfite solution (20 mL). This mixture was extracted with ethyl acetate (3 x 20 mL). The organics were washed with a saturated aqueous sodium chloride solution (1 x 15 mL), dried over sodium sulfate, and concentrated *in vacuo*. Flash chromatography (Merck Silica gel 60, 230-400 mesh, 80/20 hexanes/ethyl acetate) effected the separation of two spots, the first of the two product spots to elute off the column afforded 1-[3-cyclopentyl-2-(3,4-dichloro-phenyl)-propionyl]-3-(2-hydroxy-propyl)-urea (36 mg, 26%) as a white solid: mp 116.7-119.9°C; EI-HRMS m/e calcd for C₁₈H₂₄Cl₂N₂O₃ (M⁺) 386.1164, found 386.1173.

### Example 146

### 1-[3-Cyclopentyl-2-(3,4-dichloro-phenyl)-propionyl]-3-(3-hydroxy-propyl)-urea

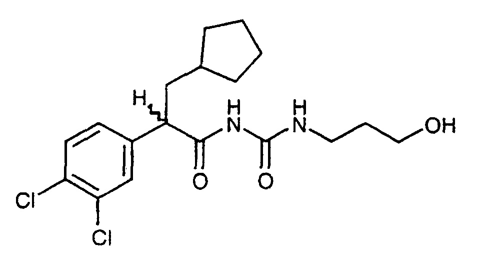

A solution of 1-allyl-3-[3-cyclopentyl-2-(3,4-dichloro-phenyl)-propionyl]-urea (prepared in Example 12B-e, 132 mg, 0.36 mmol) in tetrahydrofuran (10 mL) cooled to 0°C was treated with a 1M solution of borane-tetrahydrofuran (0.7 mL, 0.72 mmol). The reaction mixture was allowed to slowly warm from 0°C to 25°C over 1 h.. At this time, the solution was re-cooled to 0°C and ethanol (2 mL) followed by a mixture of a saturated aqueous sodium bicarbonate solution (6 mL) and 30% hydrogen peroxide (2 mL) was added slowly. This mixture was allowed to slowly warm to 25°C while stirring for 1 h. At this time, the reaction was re-cooled to 0°C and slowly quenched with a saturated aqueous sodium sulfite solution (20 mL). This solution was extracted with ethyl acetate (3 x 20 mL). The organics were washed with a saturated aqueous sodium chloride solution (1 x 15 mL), dried over sodium sulfate, and concentrated *in vacuo*. Flash chromatography (Merck Silica gel 60, 230-400 mesh, 80/20 hexanes/ethyl acetate) effected the separation of two spots, the second of the two product spots to elute off the column afforded 1-[3-cyclopentyl-2-(3,4-dichloro-phenyl)-propionyl)-3-(3-hydroxy-propyl)-urea (73 mg, 53%) as a white hygroscopic solid: EI-HRMS m/e calcd for C₁₈H₂₄Cl₂N₂O₃ (M⁺) 386.1164, found 386.1172.

### Example 147

### {3-[3-Cyclopentyl-2-(3,4-dichloro-phenyl)-propionyl]-ureido}-acetic acid methyl ester

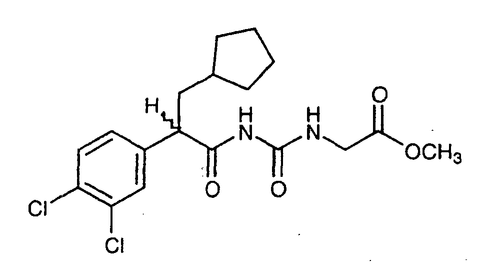

A solution of {3-[3-cyclopentyl-2-(3,4-dichloro-phenyl)-propionyl)-ureido}-acetic acid ethyl ester (prepared in Example 12B-d, 77 mg, 0.19 mmol) in ethanol (5 mL) at 25°C was treated with a solution of potassium hydroxide (36 mg, 0.65 mmol) in water (1 mL). The reaction mixture was stirred at 25°C for 2 h. At this time, the reaction was diluted with water (5 mL) and the ethanol was removed *in vacuo*. The aqueous layer was then acidified to pH = 2 with a 1N aqueous hydrochloric acid solution and extracted with methylene chloride (3 x 15 mL). The organics were then dried over sodium sulfate, filtered, and concentrated *in vacuo*. Flash chromatography (Merck Silica gel 60, 230-400 mesh, 88/12 chloroform/methanol plus 1% acetic acid) afforded {3-[3-cyclopentyl-2-(3,4-dichloro-phenyl)-propionyl]-ureido}-acetic acid (43 mg, 60%) as a white solid: mp 204.2-206.8°C; EI-HRMS m/e calcd for C₁₇H₂₀Cl₂N₂O₄ (M⁺) 386.0800, found 386.0795.

A solution of {3-[3-cyclopentyl-2-(3,4-dichloro-phenyl)-propionyl]-ureido}-acetic acid (30 mg, 0.08 mmol) in methanol (5 mL) was treated with concentrated sulfuric acid (4 drops). The reaction was heated to 80°C for 8 h. At this time, the reaction was cooled to 25°C and diluted with water (10mL). This solution was extracted with ethyl acetate (3 x 20 mL). The organics were washed with a saturated aqueous sodium bicarbonate solution (1 x 20 mL), a saturated aqueous sodium chloride solution (1 x 20 mL) and water (1 x 10 mL), dried over sodium sulfate, filtered, and concentrated *in vacuo*. Flash chromatography (Merck Silica gel 60, 230-400 mesh, 80/20 hexanes/ethyl acetate) afforded {3-[3-cyclopentyl-2-(3,4-dichloro-phenyl)-propionyl]-ureido}-acetic acid methyl ester (21 mg, 68%) as a white solid: mp 134.5-136.6°C; EI-HRMS m/e calcd for C₁₈H₂₂Cl₂N₂O₄ (M⁺) 400.0957, found 400.0970.

### Example 148

### 3-{3-[3-Cyclopentyl-2-(3,4-dichloro-phenyl)-propionyl]-ureido}-propionic acid methyl ester

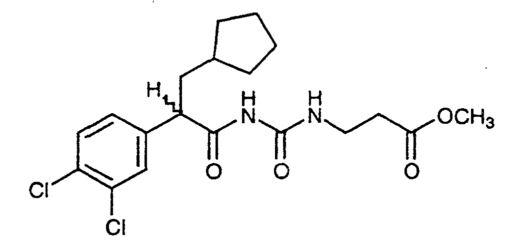

A solution of 3-{3-[3-cyclopentyl-2-(3,4-dichloro-phenyl)-propionyl]-ureido}-propionic acid ethyl ester (prepared in Example 12B-c, 94 mg, 0.22 mmol) in ethanol (5 mL) at 25°C was treated with a solution of potassium hydroxide (43 mg, 0.77 mmol) in water (1 mL). This solution was stirred at 25°C for 2 h. At this time, the reaction was diluted with water (5 mL) and the ethanol was removed *in vacuo*. The aqueous layer was acidified to pH = 2 with a 1N aqueous hydrochloric acid solution and extracted with methylene chloride (3 x 15 mL). The organic layers were then dried over sodium sulfate, filtered, and concentrated *in vacuo*. Flash chromatography (Merck Silica gel 60, 230-400 mesh, 50/50 hexanes/ethyl acetate plus 1% acetic acid) afforded 3-{3-[3-cyclopentyl-2-(3,4-dichloro-phenyl)-propionyl]-ureido}-propionic acid (30 mg, 35%) as a white foam: FAB-HRMS m/e calcd for C₁₈H₂₂Cl₂N₂O₄ (M+H)⁺ 401.1035, found 401.1022.

A solution of 3-{3-[3-cyclopentyl-2-(3,4-dichloro-phenyl)-propionyl)-ureido}-propionic acid (20 mg, 0.05 mmol) in methanol (5 mL) was treated with concentrated sulfuric acid (4 drops). This solution was heated to 80°C for 8 h. At this time, the reaction was cooled to 25°C and diluted with water (10mL). This solution extracted with ethyl acetate (3 x 20 mL). The organics were washed with a saturated aqueous sodium bicarbonate solution (1 x 20 mL), a saturated aqueous sodium chloride solution (1 x 20 mL) and water (1 x 10 mL), dried over sodium sulfate, filtered, and concentrated *in vacuo*. Flash chromatography (Merck Silica gel 60, 230-400 mesh, 80/20 hexanes/ethyl acetate) afforded 3-{3-[3-cyclopentyl-2-(3,4-dichloro-phenyl)-propionyl]-ureido}-propionic acid methyl ester (18 mg, 86%) as a white solid: 93.6-95.8°C: EI-HRMS m/e calcd for C₁₉H₂₄Cl₂N₂O₄ (M⁺) 414.1113, found 414.1114.

### Example 149

### {3-[3-Cyclopentyl-2(R)-(3,4-dichloro-phenyl)-propionyl]-ureido}-acetic acid ethyl ester

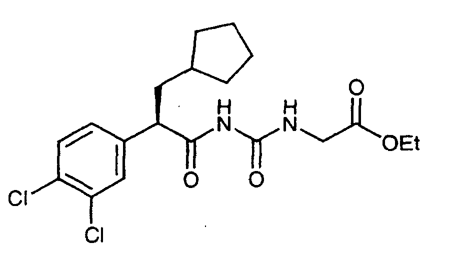

A solution of 3-cyclopentyl-2(R)-(3,4-dichloro-phenyl)-propionamide (prepared in Example 13, 600 mg, 2.10 mmol) in toluene (15 mL) was treated with ethyl isocyantoacetate (0.35 mL, 3.14 mmol). This solution was heated under reflux for 16 h. At this time, the reaction was cooled to 25°C and concentrated *in vacuo*. Flash chromatography (Merck Silica gel 60, 230-400 mesh, 90/10 hexanes/ethyl acetate) afforded {3-[3-cyclopentyl-2(R)-(3,4-dichloro-phenyl)-propionyl]-ureido}-acetic acid ethyl ester (525 mg, 60%) as a colorless oil; [α]²³₅₈₉ = -27.4° (c=0.113, chloroform); EI-HRMS m/e calcd for C₁₉H₂₄Cl₂N₂O₄ (M⁺) 414.1113, found 414.1123.

### Example 150

### 1-Allyl-3-[3-cyclopentyl-2(R)-(3,4-dichloro-phenyl)-propionyl]-urea

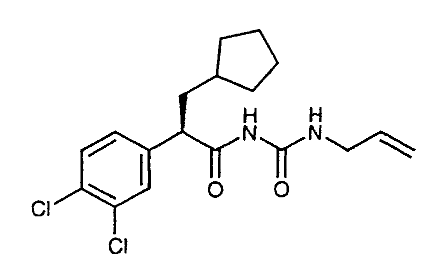

A solution of 3-cyclopentyl-2(R)-(3,4-dichloro-phenyl)-propionamide (prepared in Example 13, 1.02 g, 3.55 mmol) in toluene (30 mL) was treated with allyl isocyanate (0.47 mL, 5.33 mmol). This solution was heated to reflux for 16 h. At this time, the reaction was cooled to 25°C and concentrated *in vacuo*. Flash chromatography (Merck Silica gel 60, 230-400 mesh, 90/10 hexanes/ethyl acetate) afforded 1-allyl-3-[3-cyclopentyl-2(R)-(3,4-dichloro-phenyl)-propionyl]-urea (1.06 g, 81%) as a colorless oil: [α]²³₅₈₉ = -25.2° (c=0.151, chloroform); EI-HRMS m/e calcd for C₁₈H₂₂Cl₂N₂O₂ (M⁺) 368.1058, found 368.1054.

### Example 151

### 1-[3-Cyclopentyl-2(R)-(3,4-dichloro-phenyl)-propionyl]-3-(2-hydroxy-propyl)-urea

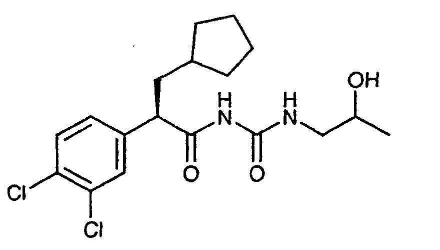

A solution of 1-allyl-3-[3-cyclopentyl-2(R)-(3,4-dichloro-phenyl)-propionyl]-urea (prepared in Example 24, 765 mg, 2.07 mmol) in tetrahydrofuran (50 mL) cooled to 0°C was treated with a 1.0M borane solution in tetrahydrofuran (4.14 mL, 4.14 mmol). The reaction was allowed to warm from 0°C to 25°C over 1 h. At this time, the reaction was re-cooled to 0°C and treated with ethanol (15 mL) followed by a mixture of a saturated aqueous sodium bicarbonate solution (45 mL) and hydrogen peroxide (15 mL). The resulting mixture was allowed to warm from 0°C to 25°C over 1 h. The reaction was then slowly quenched with a saturated aqueous sodium sulfite solution and then extracted with ethyl acetate (3 x 30 mL). The organics were washed with a saturated aqueous sodium chloride solution (1 x 20 mL), dried over sodium sulfate, filtered, and concentrated in *vacuo*. Flash chromatography (Merck Silica gel 60, 230-400 mesh, 80/20 hexanes/ethyl acetate) afforded 1-[3-cyclopentyl-2(R)-(3,4-dichloro-phenyl)-propionyl]-3-(2-hydroxy-propyl)-urea (103 mg, 11%) as a white foam: [α]²³₅₈₉ = -33.0° (c=0.094, chloroform); EI-HRMS m/e calcd for C₁₈H₂₄Cl₂N₂O₃ (M⁺) 386.1164, found 386.1151.

### Example 152

### 1-[3-Cyclopentyl-2(R)-(3,4-dichloro-phenyl)-propionyl]-3-(3-hydroxy-propyl)-urea

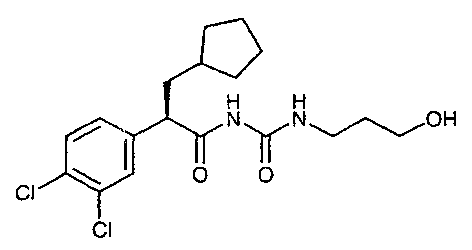

A solution of 1-allyl-3-[3-cyclopentyl-2(R)-(3,4-dichloro-phenyl)-propionyl]-urea (prepared in Example 24, 765 mg, 2.07 mmol) in tetrahydrofuran (50 mL) cooled to 0°C was treated with a 1.0M borane solution in tetrahydrofuran (4.14 mL, 4.14 mmol). The reaction was allowed to warm from 0°C to 25°C over 1 h. At this time, the reaction was re-cooled to 0°C and treated with ethanol (15 mL) followed by a mixture of a saturated aqueous sodium bicarbonate solution (45 mL) and hydrogen peroxide (15 mL). The resulting mixture was allowed to warm from 0°C to 25°C over 1 h. The reaction was then slowly quenched with a saturated aqueous sodium sulfite solution and then extracted with ethyl acetate (3 x 30 mL). The organics were washed with a saturated aqueous solution of sodium chloride (1 x 20 mL), dried over sodium sulfate, filtered, and concentrated *in* *vacuo.* Flash chromatography (Merck Silica gel 60, 230-400 mesh, 80/20 hexanes/ethyl acetate) afforded 1-[3-cyclopentyl-2-(3,4-dichloro-phenyl)-propionyl]-3-(3-hydroxy-propyl)-urea (173 mg, 22%) as a white foam: [α]²³₅₈₉ = -37.3° (c=0.075, chloroform); EI-HRMS m/e calcd for C₁₈H₂₄Cl₂N₂O₃ (M⁺) 386.1164, found 386.1154.

### Example 153

### 1-[2-(3-Chloro-4-methanesulfonyl-phenyl)-3-cyclopentyl-propionyl]-3-methyl-urea

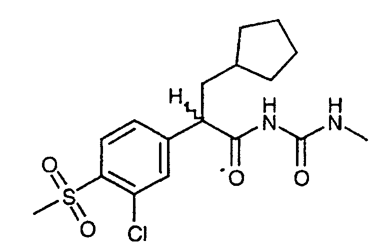

A solution of aluminum trichloride (34.8 g, 261.4 mmol) in chloroform (120 mL) cooled to 0°C was treated with a solution of ethyl chlorooxoacetate (18.7 mL, 167.5 mmol) in chloroform (120 mL). The mixture was stirred at 0°C for 30 min. At this time, a solution of 2-chlorothioanisole (25.0 g, 156.5 mmol) in chloroform (120 mL) was added dropwise to the reaction mixture. It was then allowed to warm to 25°C and stirred for an additional 3.5 h at 25°C. At this time, the reaction was quenched by the slow addition of water (500 mL) and extracted with chloroform (3 x 50 mL). The organics were dried over sodium sulfate, filtered, and concentrated *in vacuo*. Flash chromatography (Merck Silica gel 60, 230-400 mesh, 80/20 hexanes/ethyl acetate) afforded (3-chloro-4-methylsulfanyl-phenyl)-oxo-acetic acid ethyl ester (31.37 g, 77%) as a yellow oil: EI-HRMS m/e calcd for C₁₈H₂₄Cl₂N₂O₃ (M⁺) 386.1164, found 386.1154.

A solution of cylcopentylmethyl triphenylphosphonium iodide (prepared in Example 33, 725 mg, 1.53 mmol) in tetrahydrofuran (10 mL) cooled to 0°C was treated with a 1.0M solution of sodium bis(trimethylsilyl)amide in tetrahydrofuran (2.14 mL, 2.14 mmol).

The reaction was stirred at 0°C for 45 min. At this time, the reaction was treated with a solution of (3-chloro-4-methylsulfanyl-phenyl)-oxo-acetic acid ethyl ester (355 mg, 1.37 mmol) in tetrahydrofuran (5 mL). The reaction was then warmed to 25°C and stirred at 25°C for 20 h. The reaction was then diluted with water (50 mL) and extracted with diethyl ether (3 x 25 mL). The organics were dried over sodium sulfate, filtered, and concentrated *in vacuo*. Biotage chromatography (FLASH 12M, Silica, 80/20 hexanes/ethyl acetate) afforded 2-(3-chloro-4-methylsulfanyl-phenyl)-3-cyclopentyl-acrylic acid ethyl ester (267 mg, 60%, 2:1 mixture of E:Z isomers) as a yellow oil and taken on without characterization.

A solution of E:Z isomers of 2-(3-chloro-4-methylsulfanyl-phenyl)-3-cyclopentyl-acrylic acid ethyl ester (100 mg, 0.31 mmol) in methylene chloride (5 mL) cooled to 0°C was treated with 3-chloroperoxybenzoic acid (80%, 157 mg, 0.73 mmol) and stirred for 3.5 h. The reaction mixture was then diluted with methylene chloride (25 mL). This solution was washed with a saturated aqueous sodium carbonate solution (2 x 10 mL) and a saturated aqueous sodium chloride solution (2 x 10mL). The organics were dried over sodium sulfate, filtered, and concentrated *in vacuo*. Biotage chromatography (FLASH 12M, Silica, 80/20 hexanes/ethyl acetate) afforded 2-(3-chloro-4-methanesulfonyl-phenyl)-3-cyclopentyl-acrylic acid ethyl ester (95 mg, 86%, 2:1 mixture of E:Z isomers) as a colorless oil and taken on without characterization.

A solution of E:Z isomers of 2-(3-chloro-4-methanesulfonyl-phenyl)-3-cyclopentyl-acrylic acid ethyl ester (1.04 g, 2.91 mmol), nickel chloride hexahydrate (69 mg, 0.29 mmol) in methanol (25 mL) cooled to 0°C was treated with sodium borohydride (221 mg, 5.83 mmol) at a rate to maintain the reaction temperature below 20°C. After complete addition of the sodium borohydride, the reaction was stirred at 25°C for 1.5 h. At this time, the reaction was filtered through celite and washed with methanol. The filtrate was concentrated *in vacuo*. The residue was diluted with water (15 mL) and extracted with ethyl acetate (3 x 15 mL). The organics were dried over sodium sulfate, filtered and concentrated *in vacuo*. Flash chromatography (Merck Silica gel 60, 230-400 mesh, 75/25 hexanes/ethyl acetate) afforded a mixture of 2-(3-chloro-4-methanesulfonyl-phenyl)-3-cyclopentyl-propionic acid methyl ester and 2-(3-chloro-4-methanesulfonyl-phenyl)-3-cyclopentyl-propionic acid ethyl ester (transesterification occurred under the reaction conditions) (937 mg) as a clear colorless oil. (Transesterification occurred under the reaction conditions and the mixture of esters was carried on without characterization)

A solution of 2-(3-chloro-4-methanesulfonyl-phenyl)-3-cyclopentyl-propionic acid methyl ester and 2-(3-chloro-4-methanesulfonyl-phenyl)-3-cyclopentyl-propionic acid ethyl ester (268 mg) and methyl urea (110 mg, 1.5 mmol) in magnesium methoxide in methanol (7.4 wt%, 1.6 mL, 1.1 mmol) was heated to 100°C for 8 h. At this time, the reaction was concentrated *in vacuo*. The residue was then dissolved in ethyl acetate (50 mL), filtered through a plug of silica gel, and washed with ethyl acetate (100 mL). The filtrate was concentrated *in vacuo*. Flash chromatography (Merck Silica gel 60, 230-400 mesh, 60/40 hexanes/ethyl acetate) afforded 1-[2-(3-chloro-4-methanesulfonyl-phenyl)-3-cyclopentyl-propionyl]-3-methyl-urea (55 mg, 19% yield) as a white foam: FAB-HRMS m/e calcd for C₁₇H₂₃ClN₂O₄S (M+H)⁺ 387.1145, found 387.1156.

### Example 154

### 1-[2-(3-Chloro-4-methanesulfonyl-phenyl)-3-cyclopentyl-propionyl]-3-ethyl-urea

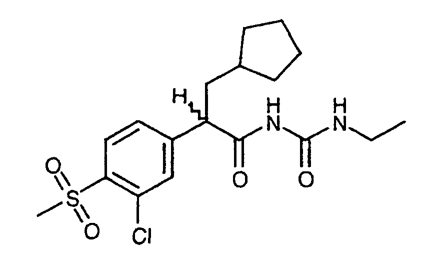

A solution of 2-(3-chloro-4-methanesulfonyl-phenyl)-3-cyclopentyl-propionic acid methyl ester and 2-(3-chloro-4-methanesulfonyl-phenyl)-3-cyclopentyl-propionic acid ethyl ester (prepared in Example 27, 937 mg) in ethanol (30 mL) at 25°C was treated with a solution of potassium hydroxide (733 mg, 13.1 mmol) in water (7 mL). This solution was stirred at 25°C for 3 h. At this time, the reaction mixture was concentrated *in vacuo*. The residue was acidified to pH = 2 by treatment with a 1N aqueous hydrochloric acid solution. This solution was then extracted with methylene chloride (3 x 15 mL). The organics were dried over sodium sulfate, filtered, and concentrated *in vacuo*. Flash chromatography (Merck Silica gel 60, 230-400 mesh, 75/25 hexanes/ethyl acetate plus 1% acetic acid) afforded 2-(3-chloro-4-methanesulfonyl-phenyl)-3-cyclopentyl-propionic acid (787 mg, 82% over two steps) as a white solid: mp 123.9-126.2°C; FAB-HRMS m/e calcd for C₁₅H₁₉ClO₄S (M+H)⁺ 331.0771, found 331.0776.

A solution of 2-(3-chloro-4-methanesulfonyl-phenyl)-3-cyclopentyl-propionic acid (147 mg, 0.44 mmol) in toluene (5 mL) with *N,N*-dimethylformamide (2 drops) at 25°C was treated with oxalyl chloride (0.05 niL, 0.53 mmol). The reaction was stirred at 25°C for 30 min. At this time, the reaction was cooled to -60°C and treated with ammonium hydroxide (0.50 mL, 3.8 mmol). The resulting suspension was allowed to warm to 25°C and stirred at 25°C for 1 h. At this time, the reaction was quenched by the addition of a 2N aqueous hydrochloric acid solution (1 mL) and then extracted with diethyl ether (3 x 25 mL). The organics were dried over magnesium sulfate, filtered, and concentrated *in vacuo*. Flash chromatography (Merck Silica gel 60, 230-400 mesh, 65/35 hexanes/ethyl acetate) afforded 2-(3-chloro-4-methanesulfonyl-phenyl)-3-cyclopentyl-propionamide (130 mg, 89%) as a white foam: EI-HRMS m/e calcd for C₁₅H₂₀ClNO₃S (M⁺) 329.0852, found 329.0852.

### Example 155

### 1-[2(R)-(3-Chloro-4-methanesulfonyl-phenyl)-3-cyclopentyl-propionyl]-3-methyl urea

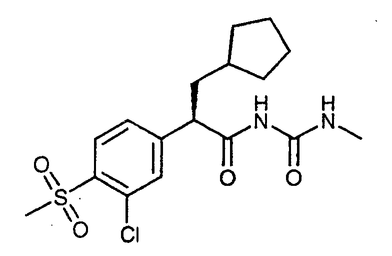

A solution of 2-(3-chloro-4-methanesulfonyl-phenyl)-3-cyclopentyl-propionic acid (prepared in Example 28, 6.1 g, 18.3 mmol), (R)-(+)-4-benzyl-2-oxazolidinone (2.83g, 15.9 mmol), triethylamine (6.68 mL, 47.7 mmol), in toluene (50 mL) heated to 80°C was treated with pivaloyl chloride (3.55 mL, 28.8 mmol) in toluene (10 mL). The resulting slurry was heated at 80°C for 36 h. At this time, the reaction was cooled to 20°C and concentrated *in vacuo*. The residue was diluted with ethyl acetate (150 mL) washed with a 1N aqueous hydrochloric acid solution (100 mL), a 10% aqueous sodium carbonate solution (100 mL), and a saturated aqueous sodium chloride solution (100 mL). The organics were dried over magnesium sulfate, filtered, and concentrated *in vacuo*. Flash chromatography (Merck Silica gel 60, 230-400 mesh, 90/5/5 methylene chloride/hexanes/ethyl acetate) afforded 4(R)-benzyl-3-[2(S)-(3-chloro-4-methanesulfonyl-phenyl)-3-cyclopentyl-propionyl]-oxazolidin-2-one (2.08 g, 23%) as a white foam: [α]²³₅₈₉ = +10.4° (c=0.144, chloroform); FAB-HRMS m/e calcd for C₂₅H₂₈ClNO₅S (M+H)⁺ 490.1455, found 490.1457 and 4(R)-benzyl-3-[2(R)-(3-chloro-4-methanesulfonyl-phenyl)-3-cyclopentyl-propionyl]-oxazolidin-2-one (2.20 g, 25%) as a white foam: [α]²³₅₈₉ = -93.9° (c=0.165, chloroform); FAB-HRMS m/e calcd for C₂₅H₂₈ClNO₅S (M+H)⁺ 490.1455, found 490.1443.

A solution of lithium hydroxide (215 mg, 9.0 mmol) in water (2.8 mL) was treated with a 30% aqueous solution of hydrogen peroxide (2.0 mL, 18 mmol). This lithium hydroperoxide solution was cooled to 0°C and was then slowly added to a solution of 4(R)-benzyl-3-[2(R)-(3-chloro-4-methanesulfonyl-phenyl)-3-cyclopentyl-propionyl]-oxazolidin-2-one (2.20 g, 4.5 mmol) in tetrahydrofuran (18 mL) and water (5.8 mL) cooled to 0°C. The reaction was stirred at 0°C for 1.5 h. At this time, the reaction was quenched with a 1.5N aqueous sodium sulfite solution (25 mL) and was further diluted with water (150 mL). This solution was extracted with diethyl ether (3 x 50 mL). The aqueous layer was then acidified to pH = 2 with a 1N aqueous hydrochloric acid solution and extracted with ethyl acetate (3 x 50 mL). The organics were dried over sodium sulfate, filtered, and concentrated *in vacuo*. Flash chromatography (Merck Silica gel 60, 230-400 mesh, 75/25 hexanes/ethyl acetate plus 1% acetic acid) afforded 2(R)-(3-chloro-4-methanesulfonyl-phenyl)-3-cyclopentyl-propionic acid (1.26 g, 85%) as a white solid: mp 106.1-108.8°C; [α]²³₅₈₉ = -43.0° (c=0.172, chloroform); EI-HRMS m/e calcd for C₁₅H₁₉ClO₄S (M⁺) 330.0692, found 330.0690.

A solution of 2(R)-(3-chloro-4-methanesulfonyl-phenyl)-3-cyclopentyl-propionic acid (200 mg, 0.61 mmol) in toluene (4.8 mL) and *N,N*-dimethylformamide (12 µL) at 25°C was treated with a 2.0 M solution of oxalyl chloride in methylene chloride (0.36 mL, 0.73 mmol). This solution was stirred at 25°C for 30 min. At this time, the reaction was cooled to -60°C and treated dropwise with a 30% aqueous ammonium hydroxide solution (0.59 mL, 5.24 mmol). The resulting suspension was allowed to gradually warm to 25°C and stirred at 25°C for 1 h. The reaction mixture was then extracted with ethyl acetate (3 x 25 mL). The organics were dried over magnesium sulfate, filtered and concentrated *in vacuo*. Flash chromatography (Biotage Flash 40S column, ethyl acetate) afforded 2(R)-(3-chloro-4-methanesulfonyl-phenyl)-3-cyclopentyl-propionamide (175 mg, 88%) as a colorless oil: [α]²³₅₈₉ = -45.8° (c=0.096, chloroform); EI-HRMS m/e calcd for C₁₅H₂₀ClNO₃S (M⁺) 329.0852, found 329.0851.

A solution of 2(R)-(3-chloro-4-methanesulfonyl-phenyl)-3-cyclopentyl-propionamide (160 mg, 0.49 mmol) in toluene (5 mL) was treated with methyl isocyanate (0.12 mL, 1.94 mmol). The reaction mixture was then heated at 100°C for 16 h. At this time, the reaction was concentrated *in vacuo*. Biotage chromatography (FLASH 40S, Silica, 60/40 hexanes/ethyl acetate) afforded 1-[2(R)-(3-chloro-4-methanesulfonyl-phenyl)-3-cyclopentyl-propionyl]-3-methyl-urea (79 mg, 42%) as a white foam: [α]²³₅₈₉ = - 8.9° (c=0.09, chloroform); FAB-HRMS m/e calcd for C₁₇H₂₃ClN₂O₄S (M+H)⁺ 387.1145, found 387.1142.

### Example 156

### 1-(2-Chloro-ethyl)-3-[3-cyclopentyl-2-(3,4-dichlorophenyl)-propionyl]-urea

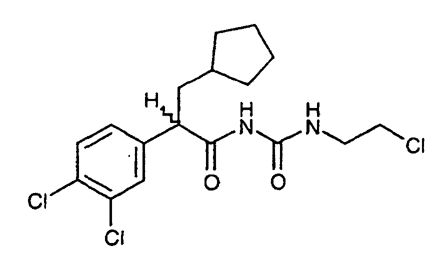

A solution of 3-cyclopentyl-2-(3,4-dichloro-phenyl)-propionamide (prepared in Example 12, 182 mg, 0.64 mmol) in toluene (10 mL) was treated with 2-chloroethyl isocyanate (0.08 mL, 0.95 mmol). The reaction was heated at reflux for 16 h. At this time, the reaction was cooled to 25°C and concentrated *in vacuo.* Flash chromatography (Merck Silica gel 60, 230-400 mesh, 90/10 hexanes/ethyl acetate) afforded 1-(2-chloro-ethyl)-3-[3-cyclopentyl-2-(3,4-dichlorophenyl)-propionyl]-urea (189 mg, 76%) as a colorless oil: EI-HRMS m/e calcd for C₁₇H₂₁Cl₃N₂O₂ (M⁺) 390.0669, found 390.0659.

### Example 157

### 1-[3-Cyclopentyl-2-(3-trifluoromethanesulfonyl-phenyl)-propionyl]-3-methyl-urea

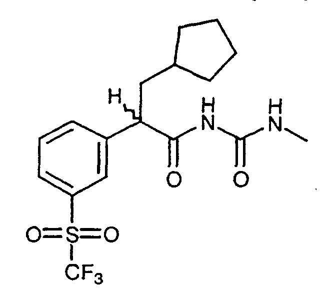

A solution of 3-(trifluoromethylthio)phenylacetic acid (5.00 g, 21.17 mmol) in methanol (50 mL) was treated slowly with concentrated sulfuric acid (10 drops). The resulting reaction mixture was heated to reflux for 18 h. The reaction mixture was allowed to cool to 25°C and then concentrated *in vacuo* to remove methanol. The residue was diluted with ethyl acetate (100 mL). The organic phase was washed with a saturated aqueous sodium bicarbonate solution (1 x 100 mL), dried over magnesium sulfate, and filtered. The filtrate was concentrated *in vacuo* to afford crude (3-trifluoromethylsulfanyl-phenyl)-acetic acid methyl, ester (5.28 g, 99%) as a pale yellow oil which was used without further purification: EI-HRMS m/e calcd for C₁₀H₉F₃O₂S (M⁺) 250.0275, found 250.0274.

A solution of diisopropylamine (1.5 mL, 10.5 mmol) in dry tetrahydrofuran (27 mL) and 1,3-dimethyl-3,4,5,6-tetrahydro-2(1H)-pyrimidinone (8 mL) cooled to -78°C was treated with a 2.5M solution of *n*-butyllithium in hexanes (4.2 mL, 10.5 mmol). The resulting reaction mixture was stirred at -78°C for 30 min and then treated dropwise with a solution of (3-trifluoromethylsulfanyl-phenyl)-acetic acid methyl ester (2.50 g, 10.0 mmol) in a small amount of tetrahydrofuran. The reaction mixture was allowed to stir at -78°C for 1 h. At this time, the reaction was treated with a solution of iodomethylcyclopentane (2.10 g, 10.0 mmol) in a small amount of dry tetrahydrofuran. The reaction mixture was allowed to warm to 25°C where it was stirred for 15 h. The reaction mixture was quenched with water (50 mL) and then partitioned between water (75 mL) and ethyl acetate (75 mL). The organic layer was dried over magnesium sulfate, filtered, and concentrated *in vacuo*. Flash chromatography (Merck Silica gel 60, 230-400 mesh, 8/1 hexanes/ethyl acetate) afforded 3-cyclopentyl-2-(3-trifluoromethylsulfanyl-phenyl)-propionic acid methyl ester (2.95 g, 89%) as a colorless oil: EI-HRMS m/e calcd for C₁₆H₁₉F₃O₂S (M⁺) 332.1058, found 332.1047.

A solution of 3-cyclopentyl-2-(3-trifluoromethylsulfanyl-phenyl)-propionic acid methyl ester (2.75 g, 8.27 mmol) in methylene chloride (30 mL) was treated with 3-chloroperoxybenzoic acid (80-85% grade, 4.28 g based on 80%, 20.67 mmol). The reaction mixture was stirred at 25°C for 6 h. At this time, thin layer chromatography showed the presence of two new lower R_{f} products. An additional 4.00 g of 3-chloroperoxybenzoic acid was added to the reaction mixture to drive the conversion of the sulfoxide to the sulfone, and the resulting reaction mixture was stirred at 40°C for 3 d. The reaction mixture was cooled to 25°C and then partitioned between water (100 mL) and methylene chloride (100 mL). The layers were shaken and separated. The organic phase was washed twice with a saturated aqueous sodium bicarbonate solution, washed with water, dried over magnesium sulfate, filtered, and concentrated *in vacuo*. Flash chromatography (Merck Silica gel 60, 230-400 mesh, 1/1 hexanes/methylene chloride) afforded 3-cyclopentyl-2-(3-trifluoromethanesulfonyl-phenyl)-propionic acid methyl ester (2.07 g, 69%) as a colorless oil: EI-HRMS m/e calcd for C₁₆H₁₉F₃O₄S (M⁺) 364.0956, found 364.0947.

3-Cyclopentyl-2-(3-trifluoromethanesulfonyl-phenyl)-propionic acid methyl ester (500 mg, 1.37 mmol) and methyl urea (305 mg, 4.12 mmol) were treated with a solution of magnesium methoxide in methanol (7.4 wt%, 5.9 mL, 4.12 mmol). The reaction mixture was then concentrated *in vacuo* to approximately one-half the volume of methanol. The resulting reaction mixture was then heated under reflux for 15 h. The reaction mixture was allowed to cool to 25°C, diluted with ethyl acetate (10 mL), and then filtered through celite. The celite was thoroughly washed with ethyl acetate. The filtrate was concentrated *in vacuo*. Flash chromatography (Merck Silica gel 60, 230-400 mesh, 3/1 hexanes/ethyl acetate) afforded 1-[3-cyclopentyl-2-(3-trifluoromethanesulfonyl-phenyl)-propionyl]-3-methyl-urea (253 mg, 45%) as a white foam: mp 59-62°C (foam to gel); EI-HRMS m/e calcd for C₁₇H₂₁F₃N₂O₄S (M⁺) 406.1174, found 406.1178.

### Example 158

### 1-[3-Cyclopentyl-2-(3-fluoro-4-trifluoromethyl-phenyl)-propionyl]-3-methyl-urea

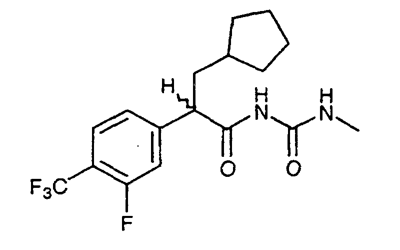

A solution of 3-fluoro-4-(trifluoromethyl)phenylacetic acid (2.50 g, 11.25 mmol) in methanol (25 mL) was treated with concentrated sulfuric acid (4 drops). The resulting reaction mixture was heated under reflux for 15 h. The reaction mixture was allowed to cool to 25°C and then concentrated *in vacuo* to remove methanol. Flash chromatography (Merck Silica gel 60, 230-400 mesh, 1/1 hexanes/ethyl acetate) afforded (3-fluoro-4-trifluoromethyl-phenyl)-acetic acid methyl ester (2.58 g, 97%) as a colorless oil: EI-HRMS m/e calcd for C₁₀H₈F₄O₂ (M⁺) 236.0460, found 236.0457.

A solution of diisopropylamine (1.5 mL, 10.67 mmol) in dry tetrahydrofuran (24 mL) and 1,3-dimethyl-3,4,5,6-tetrahydro-2(1H)-pyrimidinone (8 mL) cooled to -78°C was treated with a 2.5M solution of n-butyllithium in hexanes (4.3 mL, 10.67 mmol). The resulting reaction mixture was stirred at -78°C for 45 min and then treated dropwise with a solution of (3-fluoro-4-trifluoromethyl-phenyl)-acetic acid methyl ester (2.40 g, 10.16 mmol) in a small amount of tetrahydrofuran. The reaction mixture was allowed to stir at -78°C for 1 h. At this time, the reaction was treated with a solution of iodomethylcyclopentane (2.24 g, 10.67 mmol) in a small amount of dry tetrahydrofuran. The reaction mixture was allowed to warm to 25°C where it was stirred for 15 h. The reaction mixture was quenched with a saturated aqueous ammonium chloride solution (10 mL) and then partitioned between water (75 mL) and ethyl acetate (75 mL). The layers were shaken and separated. The aqueous layer was further extracted with ethyl acetate (75 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 5/1 hexanes/ethyl acetate) afforded 3-cyclopentyl-2-(3-fluoro-4-trifluoromethyl-phenyl)-propionic acid methyl ester (2.69 g, 83%) as a colorless oil: EI-HRMS m/e calcd for C₁₆H₁₈F₄O₂ (M⁺) 318.1243, found 318.1250.

3-Cyclopentyl-2-(3-fluoro-4-trifluoromethyl-phenyl)-propionic acid methyl ester (750 mg, 2.36 mmol) and methyl urea (437 mg, 5.90 mmol) were treated with a solution of magnesium methoxide in methanol (7.4 wt%, 14.5 mL, 7.08 mmol). The reaction mixture was then concentrated *in vacuo* to approximately one-half the volume of methanol. The resulting reaction mixture was then heated under reflux for 15 h. The reaction mixture was allowed to cool to 25°C and then partitioned between water (75 mL) and ethyl acetate (75 mL). An emulsion formed, and a saturated aqueous sodium chloride solution was added to break down the emulsion. The aqueous layer was further extracted with ethyl acetate (2 x 75 mL). The combined organic layers were dried over magnesium sulfate, filtered, and concentrated *in vacuo* to afford a white semi-solid. The semi-solid was treated with a solution of 2/1 hexanes/ethyl acetate, and a white solid formed. The solid was filtered, washed well with hexanes, and dried to afford 1-[3-cyclopentyl-2-(3-fluoro-4-trifluoromethyl-phenyl)-propionyl]-3-methyl-urea (322 mg, 38%) as a white solid: mp 187-189°C; FAB-HRMS m/e calcd for C₁₇H₂₀F₄N₂O₂ (M+H)⁺ 361.1539, found 361.1549.

### Example 159

### 1-[3-Cyclopentyl-2-(4-ethanesulfonyl-phenyl)-propionyl]-3-methyl-urea

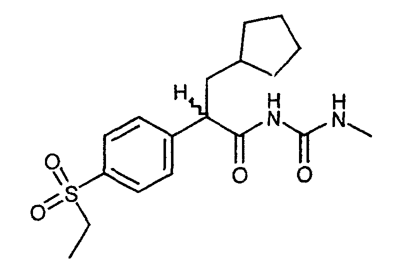

A mixture of aluminum chloride (72.35 g, 0.54 mol) in chloroform (181 mL) cooled to 0°C was stirred until homogeneous. The reaction mixture was then treated slowly with ethyl oxalyl chloride (61 mL, 0.54 mol). The resulting reaction mixture was stirred at 0°C for 30 min. It was then slowly treated with ethyl phenyl sulfide (25.0 g, 0.18 mol). The solution turned to a wine color and slowly became gum-like. The resulting reaction mixture was then stirred at 0°C for 2 h. The reaction mixture was slowly poured into a large amount of ice/water. The resulting aqueous layer was extracted with chloroform (3 x 200 mL). The combined organic layers were dried over sodium sulfate, filtered, and concentrated *in vacuo*. Flash chromatography (Merck Silica gel 60, 70-230 mesh, 9/1 hexanes/ethyl acetate) afforded (4-ethylsulfanyl-phenyl)-oxo-acetic acid ethyl ester (23.64 g, 55%) as a yellow oil. The material was used without further purification and characterization in subsequent reactions.

A solution of iodomethylcyclopentane (4.60 g, 21.89 mmol) and triphenylphosphine (5.74 g, 21.89 mmol) in acetonitrile (22 mL) was heated under reflux for 2 weeks. The reaction mixture was allowed to cool to 25°C and then concentrated *in vacuo* to provide an orange solid. The orange solid was triturated with diethyl ether and then filtered. The solid was washed well with diethyl ether until the washings showed the absence of iodomethylcyclopentane and triphenylphosphine by thin layer chromatography. The solid was allowed to air dry to afford cyclopentylmethyl triphenylphosphonium iodide (8.92 g, 86%) as a light orange solid: mp 195-198°C; FAB-HRMS m/e calcd for C₂₄H₂₆P (M+H)⁺ 345.1772, found 345.1784.

A suspension of cyclopentylmethyl triphenylphosphonium iodide (24.48 g, 51.82 mmol) in tetrahydrofuran (100 mL) cooled to 0°C was treated dropwise with a 1.0M solution of sodium bis(trimethylsilyl)amide in tetrahydrofuran (52 mL, 51.82 mmol). The bright orange reaction mixture was stirred at 0°C for 1 h. The reaction mixture was then treated with (4-ethylsulfanyl-phenyl)-oxo-acetic acid ethyl ester (9.50 g, 39.87 mmol). The resulting reaction mixture was allowed to warm to 25°C where it was stirred for 20 h. The reaction mixture was concentrated *in vacuo* to remove tetrahydrofuran and then diluted with water (300 mL). The aqueous layer was extracted with ethyl acetate (3 x 200 mL). The combined organic layers were washed with a saturated aqueous sodium chloride solution (1 x 200 mL), dried over sodium sulfate, filtered, and concentrated in *vacuo*. Flash chromatography (Merck Silica gel 60, 70-230 mesh, 19/1 hexanes/ethyl acetate) afforded the 3-cyclopentyl-2-(4-ethylsulfanyl-phenyl)-acrylic acid ethyl ester (6.08 g, 50%) as a yellow oil containing a 1.82:1 mixture of E:Z isomers: FAB-LRMS m/e calcd for C₁₈H₂₄O₂S (M+H)⁺ integer mass 304, found 305.

A solution of 3-cyclopentyl-2-(4-ethylsulfanyl-phenyl)-acrylic acid ethyl ester [5.76 g, 18.92 mmol, E:Z = 1.82:1] in methylene chloride (47 mL) was slowly treated with 3-chloroperoxybenzoic acid (57-86% grade, 11.45 g based on 57%, 37.83 mmol). The reaction mixture was stirred at 25°C for 1 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL), washed with a saturated aqueous sodium chloride solution (1 x 200 mL), dried over sodium sulfate, filtered, and concentrated *in vacuo.* Flash chromatography (Merck Silica gel 60, 230-400 mesh, 3/1 hexanes/ethyl acetate) afforded 3-cyclopentyl-2-(4-ethanesulfonyl-phenyl)-acrylic acid ethyl ester (4.89 g, 77%) as a colorless oil. The product was a 3:1 mixture of (E):(Z) isomers that was used without further purification and characterization.

A solution of 3-cyclopentyl-2-(4-ethanesulfonyl-phenyl)-acrylic acid ethyl ester [4.89 g, 14.53 mmol, (E):(Z) = 3:1] in ethanol (36 mL) was slowly treated with 10% palladium on activated carbon (244.5 mg). The reaction mixture was stirred under a positive pressure of hydrogen gas (balloon) at 25°C and atmospheric pressure for 44 h. The catalyst was then filtered off through a pad of celite, and the celite pad was washed well with ethyl acetate. The filtrate was concentrated *in vacuo*. Flash chromatography (Merck Silica gel 60, 70-230 mesh, 3/1 hexanes/ethyl acetate) afforded 3-cyclopentyl-2-(4-ethanesulfonyl-phenyl)-propionic acid ethyl ester (3.50 g, 71 %) as a colorless viscous oil: FAB-LRMS m/e calcd for C₁₈H₂₆O₄S (M+H)⁺ integer mass 338, found 339. 3-Cyclopentyl-2-(4-ethanesulfonyl-phenyl)-propionic acid ethyl ester (500 mg, 1.48 mmol) and methyl urea (274 mg, 3.70 mmol) were treated with a solution of magnesium methoxide in methanol (7.4 wt%, 6.5 mL, 4.43 mmol). The reaction mixture was then concentrated *in vacuo* to approximately one-half the volume of methanol. The resulting reaction mixture was then heated under reflux for 2 d. The reaction mixture was allowed to cool to 25°C and then diluted with ethyl acetate (25 mL). The mixture was filtered through a pad of celite, and the celite pad was washed with ethyl acetate (50 mL). The resulting filtrate was washed with water (40 mL) then the water layer was further extracted with ethyl acetate (40 mL). The combined organic layers were dried over magnesium sulfate, filtered, and concentrated *in vacuo* to afford a colorless oil. The oil was treated with a solution of 2/1 hexanes/ethyl acetate (10 mL), and a white solid began to precipitate. The suspension was placed in the freezer to enhance crystallization. The solid was filtered to afford 1-[3-cyclopentyl-2-(4-ethanesulfonyl-phenyl)-propionyl]-3-methyl-urea (178 mg, 33%) as a white solid: mp 200-201°C; FAB-HRMS m/e calcd for C₁₈H₂₆N₂O₄S (M+H)⁺ 367.1691, found 367.1697.

### Example 160

### 1-[2-(4-Chloro-3-nitro-phenyl)-3-cyclopentyl-propionyl]-3-methyl-urea

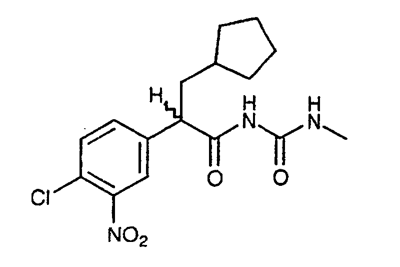

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitro-phenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) cooled to -78°C was treated dropwise with a 2.5M solution of n-butyllithium in hexanes (9.56 mL, 23.9 mmol) over a 10 min period. The pale yellow reaction mixture was stirred at -78°C for 20 min. At this time, the reaction was treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h. At this time, the reaction was treated with a solution of iodomethylcyclopentane (4.58 g, 21.8 mol) in a small amount of dry tetrahydrofuran. The reaction mixture was then stirred at -78°C and then allowed to warm to 25°C, where it was stirred for 48 h. The reaction mixture was then quenched with a saturated aqueous ammonium chloride solution (50 mL), and the resulting reaction mixture was concentrated *in vacuo* to remove tetrahydrofuran. The residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitro-phenyl)-3-cyclopentyl-propionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitro-phenyl)-3-cyclopentyl-propionic acid methyl ester (260 mg, 0.834 mmol) in tetrahydrofuran (3 mL) at 25°C was treated with a 0.8M aqueous lithium hydroxide solution (1.25 mL, 1.00 mmol). The reaction mixture was stirred at 25°C for 15 h. The resulting reaction mixture was partitioned between water (50 mL) and ethyl acetate (50 mL) and then treated with a 1N aqueous hydrochloric acid solution (10 mL). The layers were shaken and separated. The aqueous layer was further extracted with ethyl acetate (50 mL). The combined organic layers were dried over magnesium sulfate, filtered, and concentrated *in vacuo* to afford 2-(4-chloro-3-nitro-phenyl)-3-cyclopentyl-propionic acid (243 mg, 98%) as a yellow solid which was used without further purification: mp 112-115°C; FAB-HRMS m/e calcd for C₁₄H₁₆ClNO₄ (M+H)⁺ 298.0847, found 298.0851.

A mixture of 2-(4-chloro-3-nitro-phenyl)-3-cyclopentyl-propionic acid (450 mg, 1.51 mmol) in methylene chloride (4 mL) was treated with *N*,*N*-dimethylformamide (1 drop) and then cooled to 0°C. The reaction mixture was then slowly treated with oxalyl chloride (145 µL, 1.66 mmol). The reaction mixture was stirred at 0°C for 10 min and then stirred at 25°C for 1 h. The resulting reaction mixture was then treated dropwise with 1,1,1,3,3,3-hexamethyldisilazane (960 µL, 4.53 mmol) and subsequently stirred at 25°C for 15 h. The resulting reaction mixture was diluted with methylene chloride (10 mL) and methanol (10 mL). The organic layer was washed with a 5% aqueous sulfuric acid solution and a saturated aqueous sodium chloride solution. The organic layer was dried over sodium sulfate, filtered, and concentrated *in vacuo* to afford 2-(4-chloro-3-nitro-phenyl)-3-cyclopentyl-propionamide (295 mg, 67%) as a yellow oil which solidified upon sitting to a yellow solid. The yellow solid was used without further purification: mp 112-114°C; EI-HRMS m/e calcd for C₁₄H₁₇ClN₂O₃ (M⁺) 296.0927, found 296.0931.

A solution of 2-(4-chloro-3-nitro-phenyl)-3-cyclopentyl-propionamide (200 mg, 0.67 mmol) and methyl isocyanate (382 mg, 6.70 mmol) in toluene (3 mL) was heated under reflux (120°C) for 15 h. The reaction mixture was allowed to cool to 25°C and then concentrated *in vacuo*. Flash chromatography (Merck Silica gel 60, 230-400 mesh, 2/1 hexanes/ethyl acetate) afforded 1-[2-(4-chloro-3-nitro-phenyl)-3-cyclopentyl-propionyl]-3-methyl-urea (139 g, 60%) as a white foam: mp 61-64°C (foam to gel); FAB-HRMS m/e calcd for C₁₆H₂₀ClN₃O₄ (M+H)⁺ 354.1220, found 354.1232.

### Example 161

### 1-[2-(3-Bromo-4-methanesulfonyl-phenyl)-3-cyclopentyl-propionyl]-3-methyl-urea

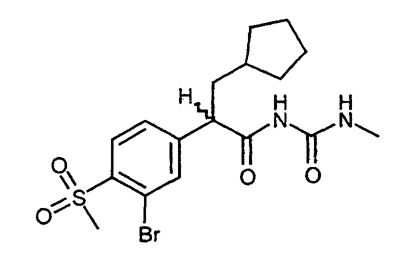

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was treated slowly with bromine (1.74 mL, 33.84 mmol). The reaction mixture was stirred at 25°C for 4 h, at which time, thin layer chromatography still indicated the presence of a substantial amount of starting material. The reaction mixture was further treated with more bromine (1.74 mL, 33.84 mmol). The reaction mixture was stirred an additional 4 h at 25°C and then quenched with a 10% aqueous sodium bisulfite solution (150 mL). The reaction mixture was concentrated *in vacuo* to remove carbon tetrachloride. The resulting aqueous layer was extracted with ethyl acetate (3 × 150 mL). The combined organic layers were dried over sodium sulfate, filtered and concentrated in *vacuo.* Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) cooled to -78°C was treated with a 2.5M solution of n-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h. At this time, the reaction was treated with a solution of iodomethylcyclopentane (5.59 g, 26.60 mmol) in a small amount of dry tetrahydrofuran. The resulting reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A solution of 2-(3-bromo-4-methanesulfonyl-phenyl)-3-cyclopentyl-propionic acid methyl ester (1.62 g, 4.16 mol) in methanol (10 mL) was treated with a 1N aqueous sodium hydroxide solution (8.7 mL, 8.74 mol). The reaction mixture was stirred at 25°C for 27 h. The reaction mixture was concentrated *in vacuo* to remove methanol. The resulting aqueous residue was acidified to pH = 2 with a 10% aqueous hydrochloric acid solution and then extracted with ethyl acetate (1 x 400 mL). The organic layer was washed with water (1 x 300 mL) and washed with a saturated aqueous sodium chloride solution (1 x 300 mL). The organic layer was then dried over sodium sulfate, filtered, and concentrated *in vacuo* to afford 2-(3-bromo-4-methanesulfonyl-phenyl)-3-cyclopentyl-propionic acid (1.39 g, 89%) as a white solid which was used without further purification: mp 149-150°C; FAB-HRMS m/e calcd for C₁₅H₁₉BrO₄S (M+H)⁺ 375.0266, found 375.0274.

A mixture of 2-(3-bromo-4-methanesulfonyl-phenyl)-3-cyclopentyl-propionic acid (400 mg, 1.07 mmol) in methylene chloride (4 mL) cooled to 0°C was treated with *N,N*-dimethylformamide (2 drops) followed by oxalyl chloride (100 µL, 1.18 mmol). The reaction mixture was stirred at 0°C for 10 min and then stirred at 25°C for 1 h. The resulting reaction mixture was then treated dropwise with 1,1,1,3,3,3-hexamethyldisilazane (680 µL, 3.21 mmol) and subsequently stirred at 25°C for 15 h. The reaction mixture was then diluted with methylene chloride (20 mL) and methanol (20 mL). The organic layer was washed with a 5% aqueous sulfuric acid solution (1 x 40 mL) and a saturated aqueous sodium chloride solution. The organic layer was dried over magnesium sulfate, filtered, and concentrated *in vacuo* to afford 2-(3-bromo-4-methanesulfonyl-phenyl)-3-cyclopentyl-propionamide (271 mg, 68%) as a white foam. The white foam was used without further purification: mp 60-63°C; EI-HRMS m/e calcd for C₁₅H₂₀BrNO₃S (M⁺) 373.0347, found 373.0348.

A solution of 2-(3-bromo-4-methanesulfonyl-phenyl)-3-cyclopentyl-propionamide (200 mg, 0.53 mmol) and methyl isocyanate (61 mg, 1.07 mmol) in toluene (1 mL) was heated under reflux for 24 h. The reaction mixture turned very cloudy with the forming of a white precipitate. The reaction mixture was allowed to cool to 25°C and then treated with hexanes. The reaction mixture was placed in the freezer for 1 h then filtered. The white solid was washed with cold hexanes to afford 1-[2-(3-bromo-4-methanesulfonyl-phenyl)-3-cyclopentyl-propionyl]-3-methyl-urea (100 mg, 44%) as a white solid: mp 259-260°C; FAB-HRMS m/e calcd for C₁₇H₂₃BrN₂O₄S (M+H)⁺ 431.0641, found 431.0646.

### Example 162

### 1-[2-(3-Cyano-4-methanesulfonyl-phenyl)-3-cyclopentyl-propionyl]-3-methyl-urea

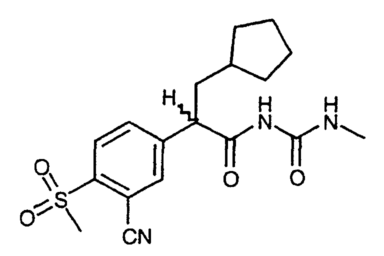

A solution of 4-(methylthio)phenylacetic acid (21.21 g, 116.38 mmol) in methanol (291 mL) was treated slowly with concentrated sulfuric acid (3 mL). The resulting reaction mixture was heated under reflux for 3 d. The reaction mixture was allowed to cool to 25°C and then concentrated *in vacuo* to remove methanol. The resulting residue was diluted with diethyl ether (600 mL). The organic layer was washed with a saturated aqueous sodium bicarbonate solution (3 x 300 mL) and a saturated aqueous sodium chloride solution (1 x 300 mL). The organic layer was dried over sodium sulfate, filtered, and concentrated *in vacuo* to afford (4-methylsulfanyl-phenyl)-acetic acid methyl ester (20.95 g, 92%) as a yellow liquid which was used without further purification: EI-HRMS m/e calcd for C₁₀H₁₂O₂S (M⁺) 196.0558, found 196.0559.

A solution of (4-methylsulfanyl-phenyl)-acetic acid methyl ester (5.11 g, 26.03 mmol) in carbon tetrachloride (130 mL) was treated slowly with bromine (1.74 mL, 33.84 mmol). The reaction mixture was stirred at 25°C for 4 h, at which time, thin layer chromatography still indicated the presence of a substantial amount of starting material. The reaction mixture was further treated with more bromine (1.74 mL, 33.84 mmol). The reaction mixture was stirred an additional 4 h at 25°C and then quenched with a 10% aqueous sodium bisulfite solution (150 mL). The reaction mixture was concentrated *in vacuo* to remove carbon tetrachloride. The resulting aqueous layer was extracted with ethyl acetate (3 x 150 mL). The combined organic layers were dried over sodium sulfate, filtered and concentrated *in vacuo*. Flash chromatography (Merck Silica gel 60, 70-230 mesh, 9/1 hexanes/ethyl acetate) afforded (3-bromo-4-methylsulfanyl-phenyl)-acetic acid methyl ester (6.10 g, 85%) as a light yellow oil: EI-HRMS m/e calcd for C₁₀H₁₁BrO₂S (M⁺) 273.9663, found 273.9661.

A solution of diisopropylamine (3.4 mL, 24.38 mmol) in dry tetrahydrofuran (21 mL) and 1,3-dimethyl-3,4,5,6-tetrahydro-2(1H)-pyrimidinone (7 mL) cooled to -78°C was treated with a 2.5M solution of *n*-butyllithium in hexanes (9.8 mL, 24.38 mmol). The resulting reaction mixture was stirred at -78°C for 30 min and then treated dropwise with a solution of (3-bromo-4-methylsulfanyl-phenyl)-acetic acid methyl ester (6.10 g, 22.17 mmol) in dry tetrahydrofuran (21 mL) and 1,3-dimethyl-3,4,5,6-tetrahydro-2(1H)-pyrimidinone (7 mL). The resulting reaction mixture was allowed to stir at -78°C for 1 h. At this time, the reaction was treated with a solution of iodomethylcyclopentane (5.59 g, 26.60 mmol) in a small amount of dry tetrahydrofuran. The reaction mixture was allowed to warm to 25°C where it was stirred for 15 h. The reaction mixture was quenched with water (300 mL) and then concentrated *in vacuo* to remove tetrahydrofuran. The remaining aqueous phase was extracted with ethyl acetate (3 x 150 mL). The combined organic layers were washed with a saturated aqueous sodium chloride solution (1 x 200 mL), dried over sodium sulfate, filtered, and concentrated *in vacuo*. Flash chromatography (Merck Silica gel 60, 70-230 mesh, 19/1 hexanes/ethyl acetate) afforded 2-(3-bromo-4-methylsulfanyl-phenyl)-3-cyclopentyl-propionic acid methyl ester (4.52 g, 57%) as a light yellow oil: EI-HRMS m/e calcd for C₁₆H₂₁BrO₂S (M⁺) 356.0446, found 356.0435.

A solution of 2-(3-bromo-4-methylsulfanyl-phenyl)-3-cyclopentyl-propionic acid methyl ester (1.07 g, 2.99 mmol) in methylene chloride (15 mL) was treated with 3-chloroperoxybenzoic acid (57-86% grade, 1.81 g based on 57%, 5.99 mmol). The reaction mixture was stirred at 25°C for 3 h. The reaction mixture was concentrated *in vacuo* to remove methylene chloride. The resulting residue was diluted with diethyl ether (300 mL). The organic phase was washed with a saturated aqueous sodium bicarbonate solution (3 x 200 mL) and a saturated aqueous sodium chloride solution (1 x 100 mL), dried over sodium sulfate, filtered, and concentrated *in vacuo*. Flash chromatography (Merck Silica gel 60, 70-230 mesh, 3/1 hexanes/ethyl acetate) afforded 2-(3-bromo-4-methanesulfonyl-phenyl)-3-cyclopentyl-propionic acid methyl ester (1.09 g, 94%) as a colorless oil: EI-HRMS m/e calcd for C₁₆H₁₉BrO₄S (M⁺) 388.0344, found 388.0343.

A mixture of 2-(3-bromo-4-methanesulfonyl-phenyl)-3-cyclopentyl-propionic acid methyl ester (990.0 mg, 2.54 mmol) and copper(I) cyanide (273.3 mg, 3.05 mmol) in dry *N*,*N*-dimethylformamide (2.5 mL) was heated under reflux for 4 h. The reaction was allowed to cool to 25°C, and the crude reaction mixture was directly purified without further chemical work-up. Flash chromatography (Merck Silica gel 60, 70-230 mesh, 100% hexanes then 3/1 hexanes/ethyl acetate) afforded 2-(3-cyano-4-methanesulfonyl-phenyl)-3-cyclopentyl-propionic acid methyl ester (646.5 mg, 76%) as a pale yellow oil: EI-HRMS m/e calcd for C₁₇H₂₁NO₄S (M⁺) 335.1191, found 335.1185.

A solution of 2-(3-cyano-4-methanesulfonyl-phenyl)-3-cyclopentyl-propionic acid methyl ester (4.84 g, 14.4 mol) in tetrahydrofuran (25 mL) was treated with a 0.8M aqueous lithium hydroxide solution (27 mL, 21.6 mmol). The reaction mixture was stirred at 25°C for 2.5 h. The reaction mixture was partitioned between water and ethyl acetate and then acidified to pH = 2 with a 10% aqueous hydrochloric acid solution. The layers were shaken and separated. The resulting organic layer was washed with a saturated aqueous sodium chloride solution, dried over magnesium sulfate, filtered, and concentrated in *vacuo* to afford crude 2-(3-cyano-4-methanesulfonyl-phenyl)-3-cyclopentyl-propionic acid (3.80 g, 82%) as a pale yellow oil that solidified to a pale yellow solid. An analytical sample was obtained by recrystallization from ethyl acetate to afford 2-(3-cyano-4-methanesulfonyl-phenyl)-3-cyclopentyl-propionic acid as a white solid: mp 180-181°C; EI-HRMS m/e calcd for C₁₆H₁₉NO₄S (M⁺) 321.1034, found 321.1039.

A mixture of 2-(3-cyano-4-methanesulfonyl-phenyl)-3-cyclopentyl-propionic acid (200 mg, 0.62 mmol) in methylene chloride (2 mL) cooled to 0°C was treated with *N*,*N*-dimethylformamide (2 drops). The reaction mixture was then slowly treated with oxalyl chloride (60 µL, 0.69 mmol). The reaction mixture was stirred at 0°C for 10 min and then stirred at 25°C for 1.5 h. The resulting reaction mixture was then treated dropwise with 1,1,1,3,3,3-hexamethyldisilazane (395 µL, 1.87 mmol) and subsequently stirred at 25°C for 15 h. The reaction mixture was then partitioned between methylene chloride (20 mL), methanol (15 mL), and a 5% aqueous sulfuric acid solution (25 mL). The organic layer was washed with a saturated aqueous sodium chloride solution, dried over magnesium sulfate, filtered, and concentrated *in vacuo*. Flash chromatography (Merck Silica gel 60, 230-400 mesh, 1/3 hexanes/ethyl acetate) afforded 2-(3-cyano-4-methanesulfonyl-phenyl)-3-cyclopentyl-propionamide (141 mg, 70%) as a white foam: mp 67-70°C (foam to gel); EI-HRMS m/e calcd for C₁₆H₂₀N₂O₃S (M⁺) 320.1195, found 320.1195.

A solution of 2-(3-cyano-4-methanesulfonyl-phenyl)-3-cyclopentyl-propionamide (135 mg, 0.42 mmol) and methyl isocyanate (72 mg, 1.26 mmol) in toluene (1 mL) was heated under reflux for 15 h. The reaction mixture was allowed to cool to 25°C and then concentrated *in vacuo* to afford a yellow semi-solid. The semi-solid was treated with a solution of 2/1 hexanes/ethyl acetate (3 mL) and a white precipitate formed. The suspension was placed in the freezer for 1 h and then filtered to afford 1-[2-(3-cyano-4-methanesulfonyl-phenyl)-3-cyclopentyl-propionyl]-3-methyl-urea (55 mg, 35%) as a white solid: mp 237-239°C; FAB-HRMS m/e calcd for C₁₈H₂₃N₃O₄S (M+H)⁺ 378.1487, found 378.1483.

### Example 163

### 1-[2-(3,4-Bis-methanesulfonyl-phenyl)-3-cyctopentyl-propionyl]-3-methyl-urea

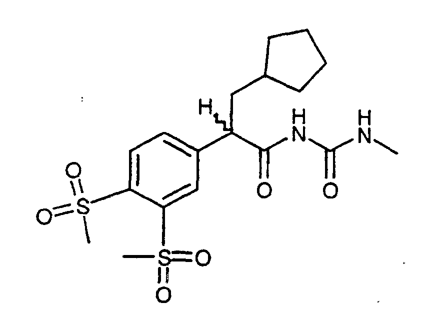

A solution of 3,4-difluorophenylacetic acid (5.00 g, 29.05 mmol) in methanol (73 mL) was slowly treated with concentrated sulfuric acid (4 mL). The resulting reaction mixture was heated under reflux for 65 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford (3,4-difluoro-phenyl)-acetic acid methyl ester (5.38 g, 99%) as a yellow oil which was used without further purification.

A solution of sodium thiomethoxide (6.39 g, 86.69 mmol) in dimethyl sulfoxide (72 mL) was treated with (3,4-difluoro-phenyl)-acetic acid methyl ester (5.38 g, 28.89 mmol). The reaction mixture was stirred at 25°C for 2 h and then at 70°C for 15 min. At this time, thin layer chromatography indicated the absence of starting material and the presence of a very polar new product. The reaction indicated that the ester hydrolyzed to the acid upon heating. The resulting reaction mixture was allowed to cool to 25°C. The reaction mixture was then treated with a 10% aqueous hydrochloric acid solution (200 mL) and then extracted with chloroform (3 x 200 mL). The combined organic layers were dried over magnesium sulfate, filtered, and concentrated *in vacuo* to afford a yellow oil. This yellow oil was dissolved in methanol (100 mL) and then slowly treated with concentrated sulfuric acid (5 mL). The resulting reaction mixture was heated under reflux for 3 h. The reaction mixture was allowed to cool to 25°C and then concentrated *in vacuo* to remove methanol. The resulting residue was slowly diluted with a saturated aqueous sodium bicarbonate solution (300 mL) and then extracted with ethyl acetate (1 x 300 mL). The organic layer was dried over magnesium sulfate, filtered, and concentrated *in vacuo* to afford an inseparable, isomeric mixture of (3-fluoro-4-methylsulfanyl-phenyl)-acetic acid methyl ester and (4-fluoro-3-methylsulfanyl-phenyl)-acetic acid methyl ester as a yellow oil (4.65 g, 75%) which was used without further purification and characterization.

A solution of the inseparable, isomeric mixture of (3-fluoro-4-methylsulfanyl-phenyl)-acetic acid methyl ester and (4-fluoro-3-methylsulfanyl-phenyl)-acetic acid methyl ester (4.44 g, 20.72 mmol) in methylene chloride (103 mL) was slowly treated with 3-chloroperoxybenzoic acid (57-86% grade, 13.80 g based on 57%, 45.59 mmol). The reaction mixture was stirred at 25°C for 4 h. The reaction mixture was concentrated *in vacuo* to remove methylene chloride. The resulting residue was diluted with ethyl acetate (300 mL). The organic phase was washed with a saturated aqueous sodium bicarbonate solution (1 x 200 mL) and a saturated aqueous sodium chloride solution (1 x 200 mL), dried over magnesium sulfate, filtered, and concentrated in *vacuo*. Flash chromatography (Merck Silica gel 60, 70-230 mesh, 20/1 methylene chloride/ethyl acetate) afforded an inseparable, isomeric mixture of (3-fluoro-4-methanesulfonyl-phenyl)-acetic acid methyl ester and (4-fluoro-3-methanesulfonyl-phenyl)-acetic acid methyl ester as a colorless liquid (3.31 g, 65%) which was used without further purification and characterization.

A solution of the inseparable, isomeric mixture of (3-fluoro-4-methanesulfonyl-phenyl)-acetic acid methyl ester and (4-fluoro-3-methanesulfonyl-phenyl)-acetic acid methyl ester (2.28 g, 9.26 mmol) in dimethyl sulfoxide (23 mL) was treated with sodium thiomethoxide (1.37 g, 18.52 mmol). The reaction mixture was stirred at 25°C for 4 h and then quenched with a 10% aqueous hydrochloric acid solution. The aqueous layer was extracted with chloroform (1 x 400 mL), dried over magnesium sulfate, filtered, and concentrated *in vacuo*. Flash chromatography (Merck Silica gel 60, 70-230 mesh, 3/2 hexanes/ethyl acetate) afforded an inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester as a yellow liquid (2.19 g, 86%) which was used without further purification and characterization.

A solution of the inseparable, isomeric mixture of (3-methanesulfonyl-4-methylsulfanyl-phenyl)-acetic acid methyl ester and (4-methanesulfonyl-3-methylsulfanyl-phenyl)-acetic acid methyl ester (2.19 g, 7.98 mmol) in methylene chloride (20 mL) was slowly treated with 3-chloroperoxybenzoic acid (57-86% grade, 6.41 g based on 57%, 31.93 mmol). The reaction mixture was stirred at 25°C for 5 h and then slowly quenched with a 1.5N aqueous sodium sulfite solution. The resulting reaction mixture was extracted with methylene chloride (300 mL). The organic phase was dried over magnesium sulfate, filtered, and concentrated *in vacuo*. Flash chromatography (Merck Silica gel 60, 70-230 mesh, 10/1 methylene chloride/ethyl acetate) afforded (3,4-bis-methanesulfonyl-phenyl)-acetic acid methyl ester (1.89 g, 77%) as a white solid: mp 157-158°C; EI-HRMS m/e calcd for C₁₁H₁₄O₆S₂ (M⁺) 306.0232, found 306.0234.

A solution of diisopropylamine (951 µL, 6.79 mmol) in dry tetrahydrofuran (6 mL) and 1,3-dimethyl-3,4,5,6-tetrahydro-2(1H)-pyrimidinone (2 mL) was cooled to -78°C under nitrogen and then treated with a 2.5M solution of *n*-butyllithium in hexanes (2.5 mL, 6.79 mmol). The resulting reaction mixture was stirred at -78°C for 30 min and then treated dropwise with a solution of (3,4-bis-methanesulfonyl-phenyl)-acetic acid methyl ester (1.89 g, 6.17 mmol) in dry tetrahydrofuran (12 mL) and 1,3-dimethyl-3,4,5,6-tetrahydro-2(1H)-pyrimidinone (4 mL). The resulting reaction mixture was allowed to stir at -78°C for 1 h. At this time, the reaction was treated with a solution of iodomethylcyclopentane (1.56 g, 7.40 mmol) in a small amount of dry tetrahydrofuran. The reaction mixture was allowed to warm to 25°C where it was stirred for 64 h. The reaction mixture was quenched with water (150 mL) and then concentrated *in vacuo* to remove tetrahydrofuran. The remaining residue was further diluted with water (100 mL) and then extracted with ethyl acetate (1 x 250 mL). The organic layer was washed with a saturated aqueous sodium chloride solution (1 x 100 mL), dried over magnesium sulfate, filtered, and concentrated *in vacuo*. Flash chromatography (Merck Silica gel 60, 70-230 mesh, 3/1 hexanes/ethyl acetate) afforded 2-(3,4-bis-methanesulfonyl-phenyl)-3-cyclopentyl-propionic acid methyl ester (1.61 g, 67%) as a yellow oil: EI-HRMS m/e calcd for C₁₇H₂₄O₆S₂ (M⁺) 388.1014, found 388.1014. 2-(3,4-Bis-methanesulfonyl-phenyl)-3-cyclopentyl-propionic acid methyl ester (375 mg, 0.97 mmol) and methyl urea (214 mg, 2.90 mmol) were treated with a solution of magnesium methoxide in methanol (7.4 wt%, 4.2 mL, 2.90 mmol). The reaction mixture was then concentrated *in vacuo* to approximately one-half the volume of methanol. The resulting reaction mixture was then heated under reflux for 48 h. The reaction mixture was allowed to cool to 25°C, diluted with ethyl acetate (5 mL), and then filtered through celite. The celite was thoroughly washed with ethyl acetate. The filtrate was concentrated *in vacuo.* Flash chromatography (Merck Silica gel 60, 230-400 mesh, 1/2 hexanes/ethyl acetate) afforded 1-[2-(3,4-bis-methanesulfonyl-phenyl)-3-cyclopentyl-propionyl]-β-methyl-urea (65 mg, 16%) as a white solid: mp 220-222°C; EI-HRMS m/e calcd for C₁₈H₂₆N₂O₆S₂ (M⁺) 430.1232, found 430.1231.

### Example 164

### 1-[3-Cyclopentyl-2-(4-fluoro-3-trifluoromethyl-phenyl)-propionyl]-3-methyl-urea

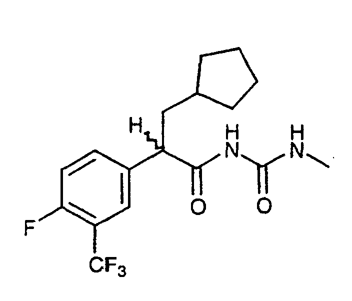

A solution of freshly prepared lithium diisopropylamide (35.3 mL of a 0.31M stock solution, 10.9 mmol) cooled to -78°C was treated with (4-fluoro-3-trifluoromethyl-phenyl)-acetic acid (1.11 g, 5.0 mmol) in tetrahydrofuran/1,3-dimethyl-3,4,5,6-tetrahydro-2(1H)-pyrimidinone (12.4 mL, 3:1). The resulting solution was stirred at -78°C for 1 h. At this time, the reaction was treated with a solution of iodomethylcyclopentane (1.16 g, 5.52 mmol) in 1.3-dimethyl-3,4,5,6-tetrahydro-2(1H)-pyrimidinone (1.2 mL). The reaction mixture was stirred at -78°C for 4 h. The reaction was then warmed to 25°C and was stirred at 25°C for 48 h. This solution was then quenched by the slow addition of the reaction mixture to an aqueous solution of 2N hydrochloric acid (50 mL). The product was extracted into ethyl acetate (3 x 100 mL) and diethyl ether (1 x 50 mL). The organic layers were dried over magnesium sulfate and sodium sulfate, filtered, and concentrated *in vacuo*. Flash chromatography (Merck Silica gel 60, 230-400 mesh, 50/50 hexanes/ethyl acetate with acetic acid) afforded 3-cyclopentyl-2-(4-fluoro-3-trifluoromethyl-phenyl)-propionic acid (1.28 g, 84.3%) as a white solid: mp 66-68°C: EI-HRMS m/e calcd for C₁₅H₁₆F₄O₂(M⁺) 305.1165, found 305.1174.

A solution 3-cyclopentyl-2-(4-fluoro-3-trifluoromethyl-phenyl)-propionic acid (304 mg, 1.0 mmol) in methylene chloride (10 mL) and *N,N*-dimethylformamide (1 drop) was cooled to 0°C and then treated with a 2.0M solution of oxalyl chloride in methylene chloride (1.5 mL, 3.0 mmol). The reaction was stirred for 30 min at 0°C. At this time, 1,1,1,3,3,3-hexamethyldisilazane (2.0 mL, 9.5 mmol) was added to the reaction mixture. The reaction was allowed to slowly warm to 25°C and then stirred at 25°C for 16 h. The reaction mixture was then treated with methanol (3 mL) and diluted with methylene chloride (35 mL). The resulting mixture was washed with a 5% aqueous sulfuric acid solution (2 x 10 mL), water (1 x 25 mL), and a saturated aqueous sodium chloride solution (3 x 25 mL). The organic layers were dried over sodium sulfate, filtered, and concentrated *in vacuo* to afford 3-cyclopentyl-2-(4-fluoro-3-trifluoromethyl-phenyl)-propionamide (333 mg, 92.5%) as a pale yellow oil: EI-HRMS m/e calcd for C₁₅H₁₇F₄NO (M⁺) 303.1246, found 303.1252.

A solution of 3-cyclopentyl-2-(4-fluoro-3-trifluoromethyl-phenyl)-propionamide (303 mg, 1.0 mmol) in toluene (5 mL) was treated with methyl isocyanate (0.59 mL, 10 mmol). The resulting solution was heated to reflux for 24 h. At this time, the reaction was concentrated *in vacuo*. Flash chromatography (Merck Silica gel 60, 230-400 mesh, 50/50 hexanes/ethyl afforded 1-[3-cyclopentyl-2-(4-fluoro-3-trifluoromethyl-phenyl)-propionyl]-3-methyl-urea (360 mg, 49.2%) as a pale yellow oil: FAB-HRMS m/e calcd for C₁₇H₂₀F₄N₂O₂ (M+H)⁺ 361.1539, found 361.1534.

### Example 165

### 1-{2-[4-(Butane-1-sulfonyl)-phenyl]-3-cyclopentyl-propionyl}-3-methyl-urea

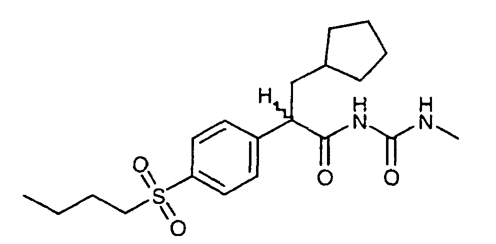

A solution of freshly prepared lithium diisopropylamide (430.55 mL of a 0.3M stock solution, 129.16 mmol) cooled to -78°C was treated with (4-nitro-phenyl)-acetic acid ethyl ester (26.32 g, 125.83 mmol) in tetrahydrofuran/hexamethylphosphoramide (312.5 mL, 3:1). The resulting solution was stirred at -78°C for 45 min. At this time, the reaction was treated with a solution of iodomethylcyclopentane (27.75 g, 132.1 mmol) in hexamethylphosphoramide (27.75 mL). The mixture was stirred at -78°C for 4 h. The reaction was then warmed to 25°C and was stirred at 25°C for 16 h. The reaction mixture was then quenched by the dropwise addition of a saturated aqueous ammonium chloride solution (250 mL). This mixture was concentrated *in vacuo*, diluted with water (250 mL), and extracted with ethyl acetate (3 x 300 mL). The organics were washed with a saturated aqueous lithium chloride solution (2 x 250 mL), dried over magnesium sulfate, filtered, and concentrated *in vacuo*. Flash chromatography (Merck Silica gel 60, 230-400 mesh, 98/2 hexanes/ethyl acetate) afforded 3-cyclopentyl-2-(4-nitro-phenyl)-propionic acid ethyl ester (28.30 g, 77.2%) as an yellow oil: EI-HRMS m/e calcd for C₁₆H₂₁NO₄ (M⁺) 291.1470, found 291.1470.

A solution of 3-cyclopentyl-2-(4-nitro-phenyl)-propionic acid ethyl ester (7.37 g, 25.3 mmol) in ethyl acetate (316 mL) was treated with 10% palladium on activated carbon (500 mg). The reaction mixture was stirred under hydrogen gas at 60 psi at 25°C for 18 h. The catalyst was then filtered off through a pad of celite (ethyl acetate). The filtrate was concentrated *in vacuo* to give 2-(4-amino-phenyl)-3-cyclopentyl-propionic acid ethyl ester (3.52 g, 53.3%) as a yellow oil: EI-HRMS m/e calcd for C₁₆H₂₃NO₂ (M⁺) 261.1727, found 261.1727.

A mixture of concentrated hydrochloric acid (0.38 mL) and ice (380 mg) cooled to 0°C was treated with 2-(4-amino-phenyl)-3-cyclopentyl-propionic acid ethyl ester (497 mg, 1.90 mmol). After 5 min, a solution of sodium nitrite (139 mg, 2.0 mmol) in water (0.31 mL) was added to the reaction mixture. The resulting solution was stirred at 0°C for 5 min. At this time, this solution was added to a solution of *n*-butyl mercaptan (0.23 mL, 2.20 mmol) in water (0.4 mL) warmed to 45°C. The reaction was stirred at 45°C for 3 h. At this time, the reaction was diluted with water (50 mL) and extracted with methylene chloride (3 x 50 mL). The organics were dried over sodium sulfate, filtered, and concentrated *in vacuo*. The crude brown oil (588 mg) in methylene chloride (16.5 mL) was cooled to 0°C and treated with 3-chloroperoxybenzoic acid (80-85% grade, 1.5 g, 8.78 mmol). The reaction mixture was stirred at 25°C for 48 h. At this time, the reaction was diluted with methylene chloride (100 mL). This solution was washed with a saturated aqueous sodium bisulfite solution (1 x 100 mL), a saturated aqueous sodium chloride solution (1 x 100 mL), a saturated aqueous sodium bicarbonate solution (1 x 100 mL), and a saturated aqueous sodium chloride solution (1 x 100 mL). The organics were dried over sodium sulfate, filtered, and concentrated *in vacuo*. Flash chromatography (Merck Silica gel 60, 230-400 mesh, 80/20 hexanes/ethyl acetate) afforded 2-[4-(butane-1-sulfonyl)-phenyl]-3-cyclopentyl-propionic acid ethyl ester (144.3 mg, 20.7%) as a yellow oil: EI-HRMS m/e calcd for C₂₀H₃₀O₄S (M⁺) 366.1865 found 366.1858.

A solution of 2-[4-(butane-1-sulfonyl)-phenyl]-3-cyclopentyl-propionic acid ethyl ester (125.3 mg, 0.34mmol) in magnesium methoxide in methanol (7.4 wt%, 0.98 mL, 0.68 mmol) was treated with methyl urea (38 mg, 0.51 mmol). This mixture was refluxed at 110°C for 12 h. The reaction mixture was then concentrated *in vacuo*. Flash chromatography (Merck Silica gel 60, 230-400 mesh, 50/50 hexanes/ethyl acetate) afforded 1-{2-[4-(butane-1-sulfonyl)-phenyl]-3-cyclopentyl-propionyl}-3-methyl-urea (61.8 mg, 45.8%) as a white solid: mp 189-191°C; EI-HRMS m/e calcd for C₂₀H₃₀N₂O₄S (M⁺) 395.2005, found 395.2008.

### Example 166

### 1-[3-Cyclopentyl-2-(4-methanesulfonyl-3-trifluoromethyl-phenyl)-propionyl]-3-methyl-urea

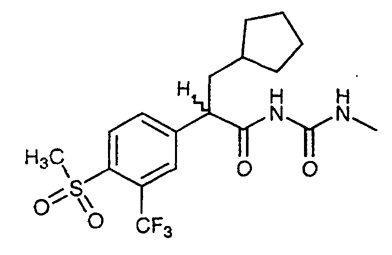

A solution of freshly prepared lithium diisopropylamide (35.3 mL of a 0.31M stock solution, 10.9 mmol) cooled to -78°C was treated with (4-fluoro-3-trifluoromethyl-phenyl)-acetic acid (1.11 g, 5.0 mmol) in tetrahydrofuran/1,3-dimethyl-3,4,5,6-tetrahydro-2(1H)-pyrimidinone (12.4 mL, 3:1). The resulting solution was stirred at -78°C for 1 h. At this time, the reaction was treated with a solution of iodomethylcyclopentane (1.16 g, 5.52 mmol) in 1,3-dimethyl-3,4,5,6-tetrahydro-2(1H)-pyrimidinone (1.2 mL). The reaction mixture was stirred at -78°C for 4 h. The reaction was then warmed to 25°C and was stirred at 25°C for 48 h. This solution was then quenched by the slow addition of the reaction mixture to an aqueous solution of 2N hydrochloric acid (50 mL). The product was extracted into ethyl acetate (3 x 100 mL) and diethyl ether (1 x 50 mL). The organics were dried over magnesium sulfate and sodium sulfate, filtered, and concentrated in *vacuo*. Flash chromatography (Merck Silica gel 60, 230-400 mesh, 50/50 hexanes/ethyl acetate with acetic acid) afforded 3-cyclopentyl-2-(4-fluoro-3-trifluoromethyl-phenyl)-propionic acid (1.28 g, 84.3%) as a white solid: mp 66-68°C; EI-HRMS m/e calcd for C₁₅H₁₆F₄O₂(M⁺) 305.1165, found 305.1174.

A solution of 3-cyclopentyl-2-(4-fluoro-3-trifluoromethyl-phenyl)-propionic acid (7.77 g, 25.3 mmol) in methanol (50 mL) was treated slowly with concentrated sulfuric acid (0.01 mL). The resulting reaction mixture was heated under reflux for 24 h. The reaction mixture was allowed to cool to 25°C and then concentrated *in vacuo*. The residue was dissolved in ethyl acetate (75 mL) and washed with a saturated aqueous sodium bicarbonate solution (1 x 50 mL), water (1 x 50 mL), and a saturated aqueous sodium chloride solution (4 x 50 mL). The organics were dried over magnesium sulfate and sodium sulfate, filtered, and concentrated *in vacuo* to afford 3-cyclopentyl-2-(4-fluoro-3-trifluoromethyl-phenyl)-propionic acid methyl ester (8.48 g, 87.5%) as yellow oil: EI-HRMS m/e calcd for C₁₆H₁₈F₄O₂ (M⁺) 318.1243, found 318.1240.

A solution of 3-cyclopentyl-2-(4-fluoro-3-trifluoromethyl-phenyl)-propionic acid methyl ester (7.0 g, 21.9 mmol) in *N,N*-dimethylformamide (50 mL) was treated with sodium methanethiolate (2.61 g, 33.0 mmol). The reaction mixture was then heated to 100-110°C for 24 h. At this time, the reaction was poured onto a mixture of ice and an aqueous solution of 2N hydrochloric acid (100 mL). This mixture was extracted into ethyl acetate (3 x 75 mL) and diethyl ether (1 x 50 mL). The organics were then washed with water (1 x 75 mL) and a saturated aqueous sodium chloride solution (3 x 100 mL). The organics were dried over magnesium sulfate and sodium sulfate, filtered, and concentrated *in vacuo*. Flash chromatography (Merck Silica gel 60, 230-400 mesh, 85/15 hexanes/ethyl acetate) afforded 3-cyclopentyl-2-(4-methylsulfanyl-3-trifluoromethyl-phenyl)-propionic acid methyl ester (2.48g. 35.5%) as a pale yellow oil: EI-HRMS m/e calcd for C₁₇H₂₁F₃O₂S (M⁺) 346.1214 , found 346.1212.

A solution of 3-cyclopentyl-2-(4-methylsulfanyl-3-trifluoromethyl-phenyl)-propionic acid methyl ester (2.36 g, 6.81 mmol) in methylene chloride (75 mL) at 25°C was treated with 3-chloroperoxybenzoic acid (80-85% grade, 9.69 g, 40.1 mmol). The reaction mixture was stirred at 25°C for 16 h. At this time, the reaction was diluted with methylene chloride (75 mL). This solution was washed with a saturated aqueous sodium bisulfite solution (2 x 50 mL), water (1 x 50 mL), a saturated aqueous sodium chloride solution (3 x 75 mL), a saturated aqueous sodium bicarbonate solution (1 x 75 mL), and a saturated aqueous sodium chloride solution (3 x 75 mL). The organics were dried over magnesium sulfate and sodium sulfate, filtered, and concentrated *in vacuo* to afford 3-cyclopentyl-2-(4-methanesulfonyl-3-trifluoromethyl-phenyl)-propionic acid methyl ester (2.88 g) as a clear oil: EI-HRMS m/e calcd for C₁₇H₂₁F₃O₂S (M⁺) 378.1112, found 378.1116.

A solution of 3-cyclopentyl-2-(4-methylsulfanyl-3-trifluoromethyl-phenyl)-propionic acid methyl ester (378 mg, 1.0 mmol) in magnesium methoxide in methanol (7.4 wt%, 2.0 mL, 1.40 mmol) was treated with methyl urea (148 mg, 2.0 mmol). This mixture was refluxed at 110°C for 12 h. The reaction mixture was then concentrated *in vacuo*. Flash chromatography (Merck Silica gel 60, 230-400 mesh, 40/60 hexanes/ethyl acetate) afforded 1-[3-cyclopentyl-2-(4-methanesulfonyl-3-trifluoromethyl-phenyl)-propionyl]-3-methyl-urea (61.8 mg, 45.8%) as a white solid: mp 268-270°C; EI-HRMS m/e calcd for C₁₈H₂₃F₃N₂O₄S (M⁺) 420.1331, found 420.1345.

### Example 167

### 3-{3-[3-Cyclopentyl-2-(3,4-dichloro-phenyl)-propionyl]-ureido}-3-oxo-propionic acid ethyl ester

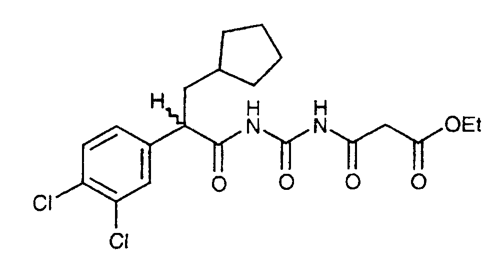

A solution of [3-cyclopentyl-2-(3,4-dichloro-phenyl)-propionyl]-urea (prepared in Example 1B-d, 402 mg, 1.22 mmol) and pyridine (0.15 mL, 1.83 mmol) in toluene (15 mL) was treated with ethyl malonyl chloride (0.19 mL, 1.5 mmol). The resulting reaction mixture was heated at reflux for 2 h. At this time, additional pyridine (0.15 mL, 1.83 mmol) and ethyl malonyl chloride (0.19 mL, 1.5 mmol) were added. The reaction was then heated at reflux for 90 min. The reaction was then cooled to 25°C, diluted with ethyl acetate (50 mL), washed with water (2 x 25 mL), and dried over magnesium sulfate. The solution was concentrated *in vacuo*. Flash column chromatography (Merck Silica gel 60, 230-400 mesh, 4/1 hexane/ethyl acetate) afforded 3-{3-[3-cyclopentyl-2-(3,4-dichloro-phenyl)-propionyl]-ureido}-3-oxo-propionic acid ethyl ester (172 mg, 32%) as a colorless gum: EI-HRMS m/e calcd for C₂₀H₂₄Cl₂N₂O₅ (M⁺) 443.1140, found 443.1128.

### Example 168

### 1-[3-Cyctopentyl-2-(3-fluoro-4-methanesulfonyl-phenyl)-propionyl]-3-methyl-urea

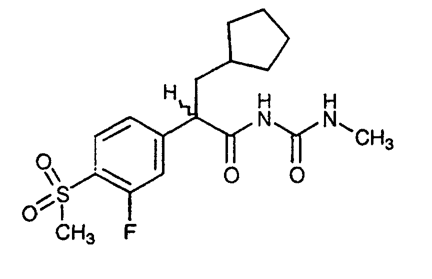

A solution of 4-chloro-3-nitrophenylacetamide (2.00 g, 9.32 mmol) in methanol (40 mL) was treated with Amberlyst® 15 ion exchange resin (15.00 g). The resulting reaction mixture was heated under reflux for 64 h. The reaction mixture was allowed to cool to 25°C and then filtered to remove the Amberlyst® 15 ion exchange resin. The filtrate was concentrated *in vacuo*. Flash chromatography (Merck Silica gel 60, 230-400 mesh, 3/1 hexanes/ethyl acetate) afforded 4-chloro-3-nitrophenylacetic acid methyl ester (1.91 g, 89%) as a yellow oil: EI-HRMS m/e calcd for C₉H₈ClNO₄ (M⁺) 229.0142, found 229.0146.

A solution of diisopropylamine (3.35 mL, 23.9 mmol) in dry tetrahydrofuran (45 mL) and 1,3-dimethyl-3,4,5,6-tetrahydro-2(1H)-pyrimidinone (15 mL) cooled to -78°C was treated dropwise with a 2.5M solution of *n*-butyllithium in hexanes (9.56 mL, 23.9 mmol) over a 10 min period. The pale yellow reaction mixture was stirred at -78°C for 20 min and then slowly treated with a solution of 4-chloro-3-nitrophenylacetic acid methyl ester (5.00 g, 21.8 mmol) in a small amount of tetrahydrofuran over a 15 min period. The reaction mixture turned deep purple (almost black) in color. The reaction mixture was then stirred at -78°C for 1 h. At this time, the reaction was treated with a solution of iodomethylcyclopentane (4.58 g, 21.8 mol) in a small amount of dry tetrahydrofuran. The reaction mixture was then stirred at -78°C and then allowed to warm to 25°C, where it was stirred for 48 h. The reaction mixture was quenched with a saturated aqueous ammonium chloride solution (50 mL), and the resulting reaction mixture was concentrated *in vacuo* to remove tetrahydrofuran. The remaining residue was diluted with ethyl acetate (150 mL) and water (50 mL). The organic phase was washed with a saturated aqueous sodium chloride solution, dried over magnesium sulfate, filtered, and concentrated *in vacuo*. Flash chromatography (Merck Silica gel 60, 230-400 mesh, 4/1 hexanes/ethyl acetate) afforded 2-(4-chloro-3-nitrophenyl)-3-cyclopentyl-propionic acid methyl ester (2.17 g, 32%) as a yellow oil: EI-HRMS m/e calcd for C₁₅H₁₈ClNO₄ (M⁺) 311.0924, found 311.0927.

A solution of 2-(4-chloro-3-nitrophenyl)-3-cyclopentyl-propionic acid methyl ester (1.00 g, 3.21 mmol) and sodium methanesulfinate (0.36 g, 3.53 mmol) in dimethyl sulfoxide (3 mL) was heated at 130°C for 5 h. The black reaction mixture was then poured over ice (20 g), resulting in the formation of a brown sticky substance. The resulting mixture was then treated with ethyl acetate (50 mL) and water (50 mL), and the layers were separated. The aqueous layer was further extracted with ethyl acetate (2 x 50 mL). The combined organic layers were washed with a saturated aqueous sodium chloride solution, dried over magnesium sulfate, filtered, and concentrated *in vacuo*. Flash chromatography (Merck Silica gel 60, 230-400 mesh, 1/1 hexanes/ethyl acetate) afforded 3-cyclopentyl-2-(4-methanesulfonyl-3-nitrophenyl)-propionic acid methyl ester (0.95 g, 84%) as a yellow gel: FAB-HRMS m/e calcd for C₁₆H₂₁NO₆S (M+H)⁺ 356.1169, found 356.1175.

A solution of 3-cyclopentyl-2-(4-methanesulfonyl-3-nitrophenyl)-propionic acid methyl ester (1.50 g, 4.22 mmol) in methanol (30 mL) was treated with a solution of ammonium chloride (474 mg, 8.86 mmol) in water (3 mL). The reaction mixture was stirred at 25°C for 5 min and then treated with zinc dust (2.70 g, 41.36 mmol). The reaction mixture was heated under reflux for 2 h. The reaction mixture was allowed to cool to 25°C and then filtered through a pad of celite. The filtrate was concentrated *in vacuo*. The resulting orange oil was dissolved in ethyl acetate, dried over magnesium sulfate, filtrated, and concentrated *in vacuo*. Flash chromatography (Merck Silica gel 60, 230-400 mesh, 2/1 hexanes/ethyl acetate) afforded 2-(3-amino-4-methanesulfonyl-phenyl)-3-cyclopentyl-propionic acid methyl ester (1.49 g, 98%) as a white solid: mp 98-100°C; EI-HRMS m/e calcd for C₁₆H₂₃NO₄S (M⁺) 325.1348, found 325.1358.

A slurry of nitrosonium tetrafluoroborate (215 mg, 1.84 mmol) in methylene chloride (6 mL) cooled to 0°C was treated dropwise with a solution of 2-(3-amino-4-methanesulfonyl-phenyl)-3-cyclopentyl-propionic acid methyl ester (500 mg, 1.54 mmol) in a small amount of methylene chloride. The resulting reaction mixture was stirred at 0°C for 1 h. The reaction mixture was then allowed to warm to 25°C and then treated with 1,2-dichlorobenzene (6 mL). The resulting reaction mixture was heated at 100°C for I h, during which time, the methylene chloride was distilled off. After 1 h at 100°C, the reaction mixture was allowed to cool to 25°C. The crude reaction mixture was directly purified by flash chromatography, (Merck Silica gel 60, 230-400 mesh, 9/1 hexanes/ethyl acetate to elute 1,2-dichlorobenzene then 2/1 hexanes/ethyl acetate), to afford impure 3-cyclopentyl-2-(3-fluoro-4-methanesulfonyl-phenyl)-propionic acid methyl ester as a yellow oil. Repurification by flash chromatography (Merck Silica gel 60, 230-400 mesh, 3/1 hexanes/ethyl acetate) afforded pure 3-cyclopentyl-2-(3-fluoro-4-methanesulfonyl-phenyl)-propionic acid methyl ester (214 mg, 42%) as a pale yellow oil which solidified upon sitting at 25°C to a pale yellow solid: mp 66-68°C; EI-HRMS m/e calcd for C₁₆H₂₁FO₄S (M⁺) 328.1144, found 328.1148. 3-Cyclopentyl-2-(3-fluoro-4-methanesulfonyl-phenyl)-propionic acid methyl ester (90 mg, 0.274 mmol) and methyl urea (61 mg, 0.822 mmol) were treated with a solution of magnesium methoxide in methanol (7.4 wt%, 1.0 mL, 0.685 mmol). The reaction mixture was then concentrated *in vacuo* to approximately one-half the volume of methanol. The resulting reaction mixture was then heated under reflux for 24a. The resulting cloudy, white reaction mixture was allowed to cool to 25°C and then filtered through celite. The celite was thoroughly washed with ethyl acetate. The filtrate was concentrated *in vacuo*. Flash chromatography (Merck Silica gel 60, 230-400 mesh, 1/1 hexanes/ethyl acetate) afforded 1-[3-cyclopentyl-2-(3-fluoro-4-methanesulfonyl-phenyl)-propionyl]-3-methyl-urea (23.3 mg, 23%) as a white solid: mp 199-200°C; EI-HRMS m/e calcd for C₁₇H₂₃FN₂O₄S (M⁺) 370.1362, found 370.1370.

### Example 169

### 1-[2-(4-Cyano-phenyl)-3-cyclopentyl-propionyl]-3-methyl-urea

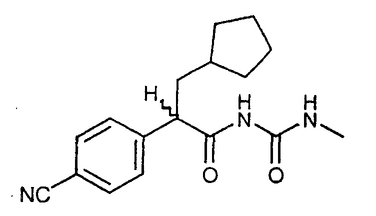

A solution of (4-bromo-phenyl)-acetic acid (6.77 g, 31.48 mmol) in methanol (32 mL) was treated with a catalytic amount of concentrated sulfuric acid (3 drops). The reaction mixture was then heated to reflux for 24 h. At this time, the reaction was concentrated *in vacuo*. The residue was treated with an aqueous solution of sodium bicarbonate (100 mL). This solution was extracted with ethyl acetate (3 x 100 mL). The organic layers were washed with a saturated aqueous solution of sodium chloride, dried over sodium sulfate, filtered and concentrated *in vacuo* to afford (4-bromo-phenyl)-acetic acid methyl ester (6.75 g, 94%) as a yellow oil. The product was used without further purification.

A solution of freshly prepared lithium diisopropylamide (50.5 mL of 0.3 M, 15.15 mmol) cooled to -78°C was treated with (4-bromo-phenyl)-acetic acid methyl ester (3.36 g, 14.67 mmol) in tetrahydrofuran/1,3-dimethyl-3,4,5,6-tetrahydro-2(1H)-pyrimidinone (37 mL, 3:1). The resulting solution was stirred at -78°C for 45 min. At this time, the reaction was treated with a solution of iodomethylcyclopentane (3.24 g, 15.45 mmol) in 1,3-dimethyl-3,4,5,6-tetrahydro-2(1H)-pyrimidinone (3.24 mL). The reaction mixture was stirred at - 78°C for 4 h. The reaction was warmed to 25°C and stirred at 25°C for 20 h. The reaction mixture was then quenched by the slow addition of a saturated ammonium chloride solution (40 mL). The reaction mixture was then poured into water (100 mL) and the product was extracted into ethyl acetate (3 x 75 mL). The organics were washed with a saturated aqueous sodium chloride solution (3 x 100 mL) and a saturated aqueous solution of lithium chloride (3 x 100 mL), dried over sodium sulfate and magnesium sulfate, filtered, and concentrated *in vacuo*. Flash chromatography (Merck Silica gel 60, 230-400 mesh, 80/20 hexanes/ethyl acetate) afforded 2-(4-bromo-phenyl)-3-cyclopentyl-propionic acid methyl ester (3.86 g, 84.6%) as a clear oil: EI-HRMS m/e calcd for C₁₅H₁₉BrO₂ (M⁺) 310.0568 found 310.0564.

A solution of 2-(4-bromo-phenyl)-3-cyclopentyl-propionic acid methyl ester (1.55 g, 5.0 mmol) in *N*,*N*-dimethylformamide (12.5 mL) was treated with copper cyanide (672 mg, 7.5 mmol). This mixture was heated at reflux for 21 h. The reaction mixture was cooled to 25°C and poured into aqueous ammonium hydroxide (25 mL). The resulting solution was diluted with water (25 mL). This solution was extracted with ethyl acetate (3 x 50 mL). The organics were washed with a saturated aqueous sodium chloride solution (3 x 75 mL), dried over sodium sulfate and magnesium sulfate, filtered, and concentrated *in vacuo*. Flash chromatography (Merck Silica gel 60, 230-400 mesh, 90/10 hexanes/ethyl acetate) afforded 2-(4-cyano-phenyl)-3-cyclopentyl-propionic acid methyl ester (1.17 g, 91.3%) as a clear oil; EI-HRMS m/e calcd for C₁₆H₁₉NO₂ (M⁺) 257.1415 found 257.1406.

A mixture of 2-(4-cyano-phenyl)-3-cyclopentyl-propionic acid methyl ester (257 mg, 1.0 mmol) and methyl urea (148 mg, 2.0 mmol) in a solution of magnesium methoxide in methanol (7.4 wt%, 2.0 mL, 1.40 mmol) was heated to reflux for 48 h. The reaction mixture was then concentrated in vacuo. Flash chromatography (Merck Silica gel 60, 230-400 mesh, 70/30 ethyl acetate/hexanes) afforded 1-[2-(4-cyano-phenyl)-3-cyclopentyl-propionyl]-3-methyl-urea (48.8 mg, 16.3%) as a white solid: mp 61-65°C; FAB-HRMS m/e calcd for C₁₇H₂₁N₃O₂ (M+H)⁺ 300.1712 found 300.1722.

### Biological Activity Examples

### Example A: In Vitro Glucokinase Activity

*Glucokinase Assay*: Glucokinase (GK) was assayed by coupling the production of glucose-6-phosphate to the generation of NADH with glucose-6-phosphate dehydrogenase (G6PDH, 0.75-1 kunits/mg; Boehringer Mannheim, Indianapolis, IN) from *Leuconostoc mesenteroides* as the coupling enzyme (Scheme 2). 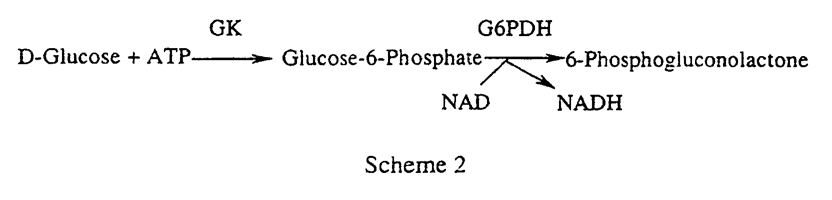 Recombinant Human liver GK1 was expressed in *E. coli* as a glutathione S-transferase fusion protein (GST-GK) [Liang et al, 1995] and was purified by chromatography over a glutathione-Sepharose 4B affinity column using the procedure provided by the manufacturer (Amersham Pharmacia Biotech, Piscataway, NJ). Previous studies have demonstrated that the enzymatic properties of native GK and GST-GK are essentially identical (Liang et al, 1995; Neet et al., 1990).

The assay was conducted at 25° C in a flat bottom 96-well tissue culture plate from Costar (Cambridge, MA) with a final incubation volume of 120 µL. The incubation mixture contained: 25 mM Hepes buffer (pH, 7.1), 25 mM KCI, 5 mM D-glucose, 1 mM ATP, 1.8 mM NAD, 2 mM MgCl₂, 1 µM sorbitol-6-phosphate, 1 mM dithiothreitol, test drug or 10% DMSO, 1.8 unit/ml G6PDH, and GK (see below). All organic reagents were > 98 % pure and were from Boehringer Mannheim with the exceptions of D-glucose and Hepes that were from Sigma Chemical Co, St Louis, MO. Test compounds were dissolved in DMSO and were added to the incubation mixture minus GST-GK in a volume of 12 µl to yield a final DMSO concentration of 10%. This mix was preincubated in the temperature controlled chamber of a SPECTRAmax 250 microplate spectrophotometer (Molecular Devices Corporation, Sunnyvale, CA) for 10 minutes to allow temperature equilibrium and then the reaction was started by the addition of 20 µl GST-GK.

After addition of enzyme, the increase in optical density (OD) at 340 nm was monitored over a 10 minute incubation period as a measure of GK activity. Sufficient GST-GK was added to produce an increase in OD₃₄₀ of 0.08 to 0.1 units over the 10 minute incubation period in wells containing 10% DMSO, but no test compound. Preliminary experiments established that the GK reaction was linear over this period of time even in the presence of activators that produced a 5-fold increase in GK activity. The GK activity in control wells was compared with the activity in wells containing test GK activators, and the concentration of activator that produced a 50% increase in the activity of GK, i.e., the SC_{1.5}, was calculated. All of the compounds of formula I described in the Synthesis Examples had an SC_{1.5} less than or equal to 30 µM.

Liang, Y., Kesavan, P., Wang, L., Niswender, K., Tanizawa, Y., Permut, M. A., Magnuson, M., and Matschinsky, F. M. Variable effects of maturity-onset-diabetes-of-youth (MODY)-associated glucokinase mutations on the substrate interactions and stability of the enzyme. *Biochem. J.* 309: 167-173, 1995.

Neet, K., Keenan, R. P., and Tippett, P.S. Observation of a kinetic slow transition in monomeric glucokinase. *Biochemistry* 29;770-777, 1990.

### Example B: In Vivo Glucokinase Activity

### Glucokinase Activator in vivo Screen Protocol

C57BL/6J mice are orally dosed via gavage with Glucokinase (GK) activator at 50 mg/kg body weight following a two hour fasting period. Blood glucose determinations are made five times during the six hour post-dose study period.

Mice (n=6) are weighed and fasted for a two hour period prior to oral treatment. GK activators are formulated at 6.76 mg/ml in Gelucire vehicle (Ethanol:Gelucire44/14:PEG400q.s. 4:66:30 v/w/v. Mice are dosed orally with 7.5µL formulation per gram of body weight to equal a 50 mg/kg dose. Immediately prior to dosing, a pre dose (time zero) blood glucose reading is acquired by snipping off a small portion of the animals tail (~1 mm) and collecting 15 µL blood into a heparinized capillary tube for analysis. Following GK activator administration, additional blood glucose readings are taken at 1, 2, 4, and 6 hours post dose from the same tail wound. Results are interpreted by comparing the mean blood glucose values of six vehicle treated mice with six GK activator treated mice over the six hour study duration. Compounds are considered active when they exhibit a statistically significant (p ≤ 0.05) decrease in blood glucose compared to vehicle for two consecutive assay time points.

## Claims

1. A compound comprising an amide of the formula I: 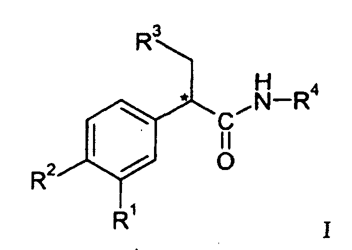 wherein
the * indicates an asymmetric carbon atom;
R¹ and R² are independently hydrogen, halo, amino, hydroxyamino, nitro, cyano, sulfonamido, lower alkyl,-OR⁵, -C(O)OR⁵, perfluoro-lower alkyl, lower alkyl thio, perfluoro-lower alkyl thio, lower alkyl sulfonyl, perfluoro-lower alkyl sulfonyl or lower alkyl sulfinyl;
R³ is cycloalkyl having from 3 to 7 carbon atoms;
R⁴ is -C(O)NHR⁴⁰; or an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, -(CH₂)ₙ-C(O)OR⁷, -(CH₂)ₙ-C(O)NHR⁶, - C(O)-C(O)OR⁸, -(CH₂)ₙ-NHR⁶;
R⁴⁰ is hydrogen, lower alkyl, lower alkenyl, hydroxy lower alkyl, halo lower alkyl, - (CH₂)ₙ-C(O)OR⁵ or -C(O)-(CH₂)ₙ-C(O)OR⁶;
R⁵ is hydrogen, lower alkyl or perfluoro-lower alkyl;
R⁶, R⁷ and R⁸ are independently hydrogen or lower alkyl; and
n is 0, 1, 2, 3 or 4;
"lower alkyl" and "lower alkoxy" indicate groups with 1 to 7 carbon atoms; and
"lower alkenyl"indicates groups with 2 to 6 carbon atoms;
or a pharmaceutically acceptable salt thereof.

2. A compound according to claim 1, wherein
R¹ and R² are independently hydrogen, halo, amino, hydroxyamino, cyano, nitro, lower alkyl, -OR⁵, -C(O)OR⁵, perfluoro-lower alkyl, lower alkyl thio, perfluoro-lower alkyl thio, lower alkyl sulfonyl, perfluoro-lower alkyl sulfonyl, lower alkyl sulfinyl, or sulfonamido;
R⁴ is an unsubstituted or mono-substituted five- or six-membered heteroaromatic ring connected by a ring carbon atom to the amine group shown, which five- or six-membered heteroaromatic ring contains from I to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, -(CH₂)ₙ-C(O)OR⁷, -(CH₂)ₙ-C(O)NHR⁶, -C(O)-C(O)OR⁸ or -(CH₂)ₙ-NHR⁶;
n is 0, 1, 2, 3 or 4;
R⁵ is hydrogen, lower alkyl, or perfluoro-lower alkyl; and
R⁶, R⁷ and R⁸ are independently hydrogen or lower alkyl;
or a pharmaceutically acceptable salt thereof.

3. A compound according to any of claims 1 to 2, wherein R⁴ is an unsubstituted or mono-substituted five- or six-membered heteroaromatic ring selected from the group consisting of thiazolyl, pyridinyl, imidazolyl, isoxazolyl, oxazolyl, pyridazinyl, pyrimidinyl or thiadiazolyl.

4. A compound according to any of claims 1 to 3, wherein R⁴ is unsubstituted thiazolyl, unsubstituted pyridinyl or pyridinyl substituted by halogen, lower alkyl, hydroxy lower alkyl or -C(O)OR⁵, wherein R⁵ is lower alkyl.

5. A compound according to claim 1, wherein
R¹ and R² are independently hydrogen, halo, amino, nitro, cyano, sulfonamido, lower alkyl, perfluoro-lower alkyl, lower alkyl thio, perfluoro-lower alkyl thio, lower alkyl sulfonyl, or perfluoro-lower alkyl sulfonyl;
R⁴ is -C(O)NHR⁴⁰;
R⁴⁰ is hydrogen, lower alkyl, lower alkenyl, hydroxy lower alkyl, halo lower alkyl, -(CH₂)ₙ-C(O)OR⁵ or -C(O)-(CH₂)ₙ-C(O)OR⁶;
R⁵ and R⁶ are hydrogen or lower alkyl; and
n is 0, 1, 2, 3 or 4;
or a pharmaceutically acceptable salt thereof.

6. A compound according to any of claims 1 or 5, wherein R⁴ is -C(O)NHR⁴⁰, and R⁴⁰ is lower alkyl or lower alkenyl.

7. A compound according to any of claims 1 to 6, wherein R¹ is hydrogen, halo, nitro or cyano.

8. A compound according to any of claims 1 to 7, wherein R¹ is hydrogen or halo.

9. A compound according to any of claims 1 to 8, wherein R² is hydrogen, lower alkyl sulfonyl, perfluoro-lower alkyl, perfluoro-lower alkyl sulfonyl, halo or -OR⁵ wherein R⁵ is perfluoro-lower alkyl.

10. A compound according to any of claims 1 to 9, wherein R² is halo or lower alkyl sulfonyl.

11. A compound according to any of claims 1 to 10, wherein the amide is in the R configuration at the asymmetric carbon shown.

12. A compound according to any of claims 1 to 11, wherein R³ is cyclopentyl, cyclohexyl or cycloheptyl.

13. A compound according to any of claims 1 to 12, wherein R³ is cyclopentyl.

14. A compound according to any of claims 1 to 13, selected from the group consisting of:
2-(3-chloro-phenyl)-3-cyclopentyl-N-thiazol-2-yl-propionamide,
2-(4-bromo-phenyl)-3-cyclopentyl-N-thiazol-2-yl-propionamide,
2-(4-chloro-phenyl)-3-cyclopentyl-N-thiazol-2-yl-propionamide,
3-cyclopentyl-2-(3,4-dichlorophenyl)-N-thiazol-2-yl-propionamide,
3-cyclopentyl-N-thiazol-2-yl-2-(4-trifluoromethyl-phenyl)-propionamide,
3-cyclopentyl-2-(3-fluoro-4-trifluoromethyl-phenyl)-N-thiazol-2-yl-propionamide,
3-cyclopentyl-2-(4-fluoro-3-trifluoromethyl-phenyl)-N-thiazol-2-yl-propionamide,
3-cyclopentyl-N-thiazol-2-yl-2-(3-trifluoromethyl-phenyl)-propionamide,
3-cyclopentyl-2(R)-(3,4-dichloro-phenyl)-N-thiazol-2-yl-propionamide,
3-cyclopentyl-2-(4-nitrophenyl)-N-thiazol-2-yl-propionamide,
2-(4-amino-phenyl)-3-cyclopentyl-N-thiazol-2-yl-propionamide,
2-(3-amino-phenyl)-3-cyclopentyl-N-thiazol-2-yl-propionamide,
2-(4-chloro-3-nitro-phenyl)-3-cyclopentyl-N-thiazol-2-yl-propionamide,
2-(4-cyano-phenyl)-3-cyclopentyl-N-thiazol-2-yl-propionamide,
3-cyclopentyl-N-thiazol-2-yl-2-(4-trifluoromethylsulfanyl-phenyl)-propionamide,
3-cyclopentyl-2-(4-methylsulfanyl-phenyl)-N-thiazol-2-yl-propionamide,
3-cyclopentyl-2-(4-methylsulfanyl-3-trifluoromethyl-phenyl)-N-thiazol-2-yl-propionamide,
3-cyclopentyl-2-(4-methanesulfinyl-phenyl)-N-thiazol-2-yl-propionamide,
3-cyclopentyl-2-(4-methanesulfonyl-phenyl)-N-thiazol-2-yl-propionamide,
3-cyclopentyl-2-(4-methanesulfonyl-3-nitrophenyl)-N-thiazol-2-yl-propionamide,
2-(3-amino-4-methanesulfonyl-phenyl)-3-cyclopentyl-N-thiazol-2-yl-propionamide,
3-cyclopentyl-2-(3-hydroxyamino-4-methanesulfonyl-phenyl)-N-thiazol-2-yl-propionamide,
2-(3-cyano-4-methanesulfonyl-phenyl)-3-cyclopentyl-N-thiazol-2-yl-propionamide,
3-cyclopentyl-2-(4-ethanesulfonyl-phenyl)-N-thiazol-2-yl-propionainide,
2-(3,4-bis-methanesulfonyl-phenyl)-3-cyclopentyl-N-thiazol-2-yl-propionamide,
3-cyclopentyl-2-(4-sulfamoyl-phenyl)-N-thiazol-2-yl-propionamide,
2-[4-(butane-1-sulfonyl)-phenyl]-3-cyclopentyl-N-thiazol-2-yl-propionamide,
3-cyclopentyl-2-[4-(propane-1-sulfonyl)-phenyl]-N-thiazol-2-yl-propionamide,
3-cyclopentyl-2-(4-methanesulfonyl-3-trifluoromethyl-phenyl)-N-thiazol-2-yl-propionamide,
2(R)-(3-chloro-4-methanesulfonyl-phenyl)-3-cyclopentyl-N-thiazol-2-yl-propionarnide,
2-[3-chloro-4-methanesulfonyl-phenyl]-3-cyclopentyl-N-thiazol-2-yl-propionamide,
2-(3-bromo-4-methanesulfonyl-phenyl)-3-cyclopentyl-N-thiazol-2-yl-propionamide,
3-cyclopentyl-N-thiazol-2-yl-2-(4-trifluoromethanesulfonyl-phenyl)-propionamide,
3-cyclopentyl-N-thiazol-2-yl-2-(3-trifluoromethanesulfonyl-phenyl)-propionamide,
3-cyclopentyl-N-thiazol-2-yl-2-(4-trifluoromethoxy-phenyl)-propionamide,
3-cyclopentyl-2-(3-methoxy-phenyl)-N-thiazol-2-yl-propionamide,
3-cyclopentyl-2-(3-hydroxy-phenyl)-N-thiazol-2-yl-propionarnide,
3-cyclopentyl-2-(3,4-dimethoxy-phenyl)-N-thiazol-2-yl-propionamide,
3-cyclopentyl-2-(3,4-dihydroxy-phenyl)-N-thiazol-2-yl-propionamide,
3-cyclopentyl-2-(4-methoxy-phenyl)-N-thiazol-2-yl-propionamide,
3-cyclopentyl-2-(4-hydroxy-phenyl)-N-thiazol-2-yl-propionamide,
4-[2-cyclopentyl-1-(thiazol-2-ylcarbamoyl)-ethyl]-benzoic acid methyl ester,
3-cyclopentyl-2-(3-fluoro-4-methoxy-phenyl)-N-thiazol-2-yl-propionamide,
3-cyclopentyl-2-(3-fluoro-4-hydroxy-phenyl)-N-thiazol-2-yl-propionamide,
3-cyclopentyl-2-(3,4-dichlorophenyl)-N-(5-hydroxymethyl-thiazol-2-yl)-propionamide,
3-cyclopentyl-2-(3,4-dichlorophenyl)-N-[4-(2-hydroxyethyl)-thiazol-2-yl]-propionamide,
2-(4-chloro-phenyl)-3-cyclopentyl-N-(5-hydroxymethyl-thiazol-2-yl)-propionamide,
3-cyclopentyl-2-(3,4-dichlorophenyl)-N-(4-hydroxymethyl-thiazol-2-yl)-propionamide,
3-cyclopentyl-N-(4-hydroxymethyl-thiazol-2-yl)-2-(4-methanesulfonyl-phenyl)-propionamide,
3-cyclopentyl-N-[4-(2-hydroxyethyl)-thiazol-2-yl]-2-(4-methanesulfonyl-phenyl)-propionamide,
3-cyclopentyl-2-(3,4-dichlorophenyl)-N-(4-methyl-thiazol-2-yl)-propionamide,
3-cyclopentyl-2-(3,4-dichlorophenyl)-N-(5-methyl-thiazol-2-yl)-propionamide,
{2-[2-(3-chloro-phenyl)-3-cyclopentyl-propionylamino]-thiazol-4-yl}-acetic acid ethyl ester,
{2-[2-(3-chloro-phenyl)-3-cyclopentyl-propionylamino]-thiazol-4-yl}-acetic acid methyl ester,
2-[2-(3-chloro-phenyl)-3-cyclopentyl-propionylamino]-thiazole-4-carboxylic acid methyl ester,
2-[2-(3-chloro-phenyl)-3-cyclopentyl-propionylamino]-thiazole-4-carboxylic acid ethyl ester,
{2-[2-(4-chloro-phenyl)-3-cyclopentyl-propionylamino]-thiazol-4-yl}-acetic acid ethyl ester,
2-[2-(4-chloro-phenyl)-3-cyclopentyl-propionylamino]-thiazole-4-carboxylic acid methyl ester,
2-[2-(4-chloro-phenyl)-3-cyclopentyl-propionylamino]-thiazole-4-carboxylic acid ethyl ester,
{2-[2-(4-chloro-phenyl)-3-cyclopentyl-propionylamino]-thiazol-4-yl}-acetic acid methyl ester,
{2-[3-cyclopentyl-2-(3,4-dichlorophenyl)-propionylamino]-thiazol-4-yl}-acetic acid,
{2-[3-cyclopentyl-2-(3,4-dichlorophenyl)-propionylamino]-thiazol-4-yl}-acetic acid ethyl ester,
2-[3-cyclopentyl-2-(3,4-dichlorophenyl)-propionylamino]-thiazole-5-carboxylic acid,
2-[3-cyclopentyl-2-(3,4-dichlorophenyl)-propionylamino]-thiazole-4-carboxylic acid,
{2-[3-cyclopentyl-2-(3,4-dichlorophenyl)-propionylamino]-thiazol-4-yl}-acetic acid methyl ester,
(2R)-2-[3-cyclopentyl-2-(3,4-dichloro-phenyl)-propionylamino]-thiazole-4-carboxylic acid methyl ester,
2-[3-cyclopentyl-2-(3,4-dichlorophenyl)-propionylamino]-thiazole-5-carboxylic acid methyl ester,
2-[3-cyclopentyl-2-(3,4-dichlorophenyl)-propionylamino]-thiazole-5-carboxylic acid ethyl ester,
{2-[3-cyclopentyl-2-(4-nitro-phenyl)-propionylamino]-thiazol-4-yl}-acetic acid ethyl ester.
{2-[3-cyclopentyl-2-(4-nitro-phenyl)-propionylamino]-thiazol-4-yl}-acetic acid methyl ester,
2-[3-cyclopentyl-2-(4-nitro-phenyl)-propionylamino]-thiazole-4-carboxylic acid methyl ester,
2-[3-cyclopentyl-2-(4-nitro-phenyl)-propionylamino]-thiazole-4-carboxylic acid ethyl ester,
{2-[2-(4-amino-phenyl)-3-cyclopentyl-propionylamino]-thiazol-4-yl}-acetic acid methyl ester,
2-[2-(4-amino-phenyl)-3-cyclopentyl-propionylamino]-thiazole-4-carboxylic acid methyl ester,
{2-[3-cyclopentyl-2-(4-methanesulfonyl-phenyl)-propionylamino]-thiazole-4-carboxylic acid ethyl ester,
{2-[3-cyclopentyl-2-(4-methanesulfonyl-phenyl)-propionylamino]-thiazol-4-yl}-4-acetic acid methyl ester,
{2-[3-cyclopentyl-2-(4-methanesulfonyl-3-trifluoromethyl-phenyl)-propionylamino]-thiazol-4-yl}-acetic acid methyl ester,
2-[3-cyclopentyl-2-(4-methanesulfonyl-3-trifluoromethyl-phenyl)-propionylamino]-thiazole-4-carboxylic acid methyl ester,
{2-[3-cyclopentyl-2-(4-nitro-phenyl)-propionylamino]-thiazol-4-yl}-oxo-acetic acid ethyl ester,
{2-[3-cyclopentyl-2-(3,4-dichlorophenyl)-propionylamino]thiazol-5-yl}-oxo-acetic ethyl ester,
{2-[3-cyclopentyl-2-(4-fluoro-3-trifluoromethyl-phenyl)-propionylamino]-thiazol-4-yl}-oxo-acetic acid ethyl ester,
{2-[2-(3-chloro-4-methanesulfonyl-phenyl)-3-cyclopentyl-propionylamino]-thiazol-4-yl}-oxo-acetic acid ethyl ester,
3-cyclopentyl-2-(3,4-dichlorophenyl)-N-(5-nitro-thiazol-2-yl)-propionamide,
3-cyclopentyl-2-(3-fluoro-4-trifluoromethyl-phenyl)-N-pyridin-2-yl-propionamide,
3-cyclopentyl-2-(3,4-dichloro-phenyl)-N-pyridin-2-yl-propionamide,
3-cyclopentyl-N-pyridin-2-yl-2-(4-trifluoromethyl-phenyl)-2-propionamide,
3-cyclopentyl-N-thiazol-2-yl-2-(3-trifluoromethyl-phenyl)-propionamide,
2-(3-chloro-phenyl)-3-cyclopentyl-N-pyridin-2-yl-propionamide,
2-(4-amino-phenyl)-3-cyclopentyl-N-pyridin-2-yl-propionamide,
2-(4- cyano-phenyl)-3-cyclopentyl-N-pyridin-2-yl-propionamide,
2-(4-chloro-phenyl)-3-cyclopentyl-N-pyridin-2-yl-propionamide,
2-(4-chloro-3-nitro-phenyl)-3-cyclopentyl-N-pyridin-2-yl-propionamide,
3-cyclopentyl-2-(4-nitro-phenyl)-N-pyridin-2-yl-propionamide,
2-(4-cyano-phenyl)-3-cyclopentyl-N-pyridin-2-yl-propionamide,
3-cyclopentyl-2-(4-methylsulfanyl-phenyl)-N-pyridin-2-yl-propionamide,
3-cyclopentyl-N-pyridin-2-yl-2-(4-trifluoromethylsulfanyl-phenyl)-propionamide,
3-cyclopentyl-2-(4-methanesulfonyl-3-nitrophenyl)-N-pyridin-2-yl-propionamide,
3-cyclopentyl-2-(4-methanesulfonyl-phenyl)-N-pyridin-2-yl-propionamide,
2-(3-bromo-4-methanesulfonyl-phenyl)-3-cyclopentyl-N-pyridin-2-yl-propionamide,
2-(3-cyano-4-methanesulfonyl-phenyl)-3-cyclopentyl-N-pyridin-2-yl-propionamide,
3-cyclopentyl-2-(4-ethanesulfonyl-phenyl)-N-pyridin-2-yl-propionamide,
2-(3,4-bis-methanesulfonyl-phenyl)-3-cyclopentyl-N-pyridin-2-yl-propionamide,
2-(3-chloro-4-methanesulfonyl-phenyl)-3-cyclopentyl-N-pyridin-2-yl-propionamide,
3-cyclopentyl-2-(4-methanesulfonyl-3-trifluoromethyl-phenyl)-N-pyridin-2-yl-propionamide,
3-cyclopentyl-N-pyridin-2-yl-2-(4-trifluoromethanesulfonyl-phenyl)-propionamide,
3-cyclopentyl-2-(3,4-dichloro-phenyl)-N-(5-carboxymethylpyridin)-2-yl-propionamide,
6-[3-cyclopentyl-2(R)-(3,4-dichloro-phenyl)-propionylamino]-nicotinic acid methyl ester,
6-[2-(4-chloro-phenyl)-3-cyclopentyl-propionylamino]-nicotinic acid,
6-[2-(3-chloro-phenyl)-3-cyclopentyl-propionylamino]-nicotinic acid methyl ester,
6-[2-(4-chloro-phenyl)-3-cyclopentyl-propionylamino]-nicotinic acid methyl ester,
6-[3-cyclopentyl-2-(4-nitro-phenyl)-propionylamino]-nicotinic acid methyl ester,
6-[2-(4-amino-phenyl)-3-cyclopentyl-propionylamino]-nicotinic acid methyl ester,
6-[2-(3-chloro-phenyl)-3-cyclopentyl-propionylamino]-nicotinic acid,
6-[2-(4-cyano-phenyl)-3-cyclopentyl-propionylamino]-nicotinic acid,
6-[3-cyclopentyl-2-(4-trifluoromethanesulfonyl-phenyl)-propionylamino]-nicotinic acid methyl ester,
6-[3-cyclopentyl-2-(4-trifluoromethanesulfonyl-phenyl)-propionylamino]-nicotinic acid,
6-[3-cyclopentyl-2-(4-methanesulfonyl-phenyl)-propionylamino]-nicotinic acid methyl ester,
3-cyclopentyl-2(R)-(3,4-dichloro-phenyl)-N-(5-hydroxymethyl-pyridin-2-yl)-propionamide,
3-cyclopentyl-2-(3,4-dichloro-phenyl)-N-(5-hydroxy-pyridin-2-yl)-propionamide,
2-(4-chloro-phenyl)-3-cyclopentyl-N-(5-hydroxymethyl-pyridin-2-yl)-propionamide,
3-cyclopentyl-N-(5-hydroxymethyl-pyridin-2-yl)-2-(4-methanesulfonyl-phenyl)-propionamide,
N-(5-chloro-pyridin-2-yl)-3-cyclopentyl-2-(3,4-dichloro-phenyl)-propionamide,
3-cyclopentyl-2-(3,4-dichloro-phenyl)-N-(5-brompyridin)-2-yl-propionamide,
3-cyclopentyl-2(R)-(3,4-dichloro-phenyl)-N-(5-trifluoromethyl-pyridin-2-yl)-propionamide,
N-(5-bromo-pyridin-2-yl)-3-cyclopentyl-2(R)-(3,4-dichloro-phenyl)-propionamide,
N-(5-chloro-pyridin-2-yl)-3-cyclopentyl-2-(4-trifluoromethanesulfonyl-phenyl)-propionamide,
N-(5-bromo-pyridin-2-yl)-3-cyclopentyl-2-(4-trifluoromethanesulfonyl-phenyl)-propionamide,
N-(5-bromo-pyridin-2-yl)-3-cyclopentyl-2-(4-methanesulfonyl-3-nitro-phenyl)-propionamide,
2-(3-bromo-4-methanesulfonyl-phenyl)-N-(5-bromo-pyridin-2-yl)-3-cyclopentyl-propionamide,
N-(5- bromo-pyridin-2-yl)-2-(3-chloro-4-methanesulfonyl-phenyl)-3-cyclopentyl-propionamide,
2-(3-chloro-4-methanesulfonyl-phenyl)-3-cyclopentyl-N-(5-trifluoromethyl-pyridin-2-yl)-propionamide,
N-(5-chloro-pyridin-2-yl)-2-(3-chloro-4-methanesulfonyl-phenyl)-3-cyclopentyl-propionamide,
3-cyclopentyl-2-(3,4-dichloro-phenyl)-N-(5-nitropyridin)-2-yl-propionamide,
3-cyclopentyl-2-(3,4-dichloro-phenyl)-N-(5-methylpyridin)-2-yl-propionamide,
3-cyclopentyl-2-(3,4-dichioro-phenyl)-N-{4-methylpyridin)-2-yl-propionamide,
3-cyclopentyl-2-(3,4-dichloro-phenyl)-N-(6-methylpyridin)-2-yl-propionamide,
3-cyclopentyl-N-(5-methyl-pyridin-2-yl)-2-(4-trifluoromethanesulfonyl-phenyl)-propionamide,
3-cyclopentyl-2-(4-fluoro-3-trifluoromethyl-phenyl)-N-(5-methyl-pyridin-2-yl)-propionamide,
6-[3-cyclopentyl-2-(3,4-dichloro-phenyl)-propionylamino]-N-methyl-nicotinamide,
3-cyclopentyl-2-(3,4-dichlorophenyl)-N-(1H-imidazol-2-yl)-propionamide,
3-cyclopentyl-2-(3,4-dichlorophenyl)-N-(5-methyl-isoxazol-3-yl)-propionamide,
3-cyclopentyl-2-(3,4-dichlorophenyl)-N-oxazol-2-yl-propionamide,
3-cyclopentyl-2-(3,4-dichlorophenyl)-N-pyridazin-3-yl-propionamide,
3-cyclopentyl-2-(3,4-dichlorophenyl)-N-pyrimidin-2-yl-propionamide,
3-cyclopentyl-2-(3,4-dichlorophenyl)-N-pyrimidin-6-yl-propionamide,
3-cyclopentyl-2-(4-nitro-phenyl)-N-pyrimidin-4-yl-propionamide,
3-cyclopentyl-2-(3,4-dichlorophenyl)-N-[ 1,3,4]thiadiazol-2-yl-propionamide,
2-[4-methanesulfonyl phenyl]-3-cyclohexyl N-thiazol-2-yl-propionamide, and
2-[4-methanesulfonyl phenyl]-3-cycloheptyl N-thiazol-2-yl-propionamide.

15. A compound according to any of claims 1 to 13, selected from the group consisting of:
1-(3-cyclopentyl-2-phenyl-propionyl)-3-methyl-urea,
1-[2-(3-chloro-phenyl)-3 cyclopentyl-propionyl]-3-methyl-urea,
1-[2-(4-chloro-phenyl)-3-cyclopentyl-propionyl]-3-methyl-urea,
1-[2-(4-cyano-phenyl)-3-cyclopentyl-propionyl]-3-methyl-urea,
1-[2-(4-bromo-phenyl)-3-cyclopentyl-propionyl]-3-methyl urea,
[3-cyclopentyl-2-(3,4-dichloro-phenyl)-propionyl]-urea,
1-[3-cyclopentyl-2-(3,4-dichloro-phenyl)-propionyl]-3-methyl-urea,
1-[3-cyclopentyl-2(R)-(3,4-dichloro-phenyl)-propionyl]-3-ethyl-urea,
1-allyl-3-[3-cyclopentyl-2-(3,4-dichloro-phenyl)-proprionyl]-urea,
1-allyl-3-[3-cyclopentyl-2(R)-(3,4-dichloro-phenyl)-proprionyl]-urea,
1-[3-cyclopentyl-2-(3,4-dichloro-phenyl)-propionyl]-3-ethyl-urea,
1-[3-cyclopentyl-2(R)-(3,4-dichloro-phenyl)-propionyl]-3-methyl-urea,
1-[3-cyclopentyl-2-(3,4-dichloro-phenyl)-propionyl]-3-isopropyl-urea,
1-[3-cyclopentyl-2-(3,4-dichloro-phenyl)-propionyl]-3-propyl-urea,
1-[3-cyclopentyl-2-(3,4-difluoro-phenyl)-propionyl]-3-methyl-urea,
1-[2-(4-chloro-3-nitro-phenyl)-3-cyclopentyl-proprionyl]-3-methyl-urea,
1-[3-cyclopentyl-2-(4-nitro-phenyl)-propionyl]-3-methyl-urea,
1-[3-cyclopentyl-2-(4-trifluoromethylsulfanyl-phenyl)-propionyl]-3-methyl urea,
1-[3-cyclopentyl-2-(4-methylsulfanyl-phenyl)-propionyl]-3-methyl urea,
1-[3-cyclopentyl-2-(4-trifluoromethanesulfonyl-phenyl)-propionyl]-3-methyl urea,
1-[3-cyclopentyl-2-(3-trifluoromethanesulfonyl-phenyl)-propionyl]-3-methyl urea,
1-[3-cyclopentyl-2-(4-methanesulfonyl-phenyl)-propionyl]-3-methyl urea,
1-{2-[4-(butane-1-sulfonyl)-phenyl]-3-cyclopentyl-proprionyl}-3-methyl-urea,
1-[3-cyclopentyl-2-(4-ethanesulfonyl-phenyl)-proprionyl]-3-methyl-urea,
1-[2-(3,4-bis-inethanesulfonyl-phenyl)-3-cyclopentyl-proprionyl]-3-metliyl-urea,
1-[2-(3-bromo-4-methanesulfonyl-phenyl)-3-cyclopentyl-proprionyl]-3-methyl-urea,
1-[3-cyctopentyl-2-(3-fluoro-4-methanesulfonyl-phenyl)-proprionyl]-3-methyl-urea,
1-[2-(3-chloro-4-methanesulfonyl-phenyl)-3-cyclopentyl-proprionyl]-3-methyl-urea,
1-[2(R)-(3-chloro-4-methanesulfonyl-phenyl)-3-cyclopentyl-proprionyl]-3-methyl-urea,
1-[2-(3-chloro-4-methanesulfonyl-phenyl)-3-cyclopentyl-proprionyl]-3-ethyl-urea,
1-[2-(3-cyano-4-methanesulfonyl-phenyl)-3-cyclopentyl-proprionyl]-3-methyl-urea,
1-[3-cyclopentyl-2-(4-methanesulfonyl-3-trifluoromethyl-phenyl)-proprionyl]-3-methyl-urea,
1-[3-cyclopentyl-2-(4-fluoro-3-trifluoromethyl-phenyl)-propionyl]-3-methyl urea,
1-[3-cyclopentyl-2-(3-fluoro-4-trifluoromethyl-phenyl)-propionyl]-3-methyl urea,
1-[3-cyclopentyl-2-(4-methanesulfonyl-3-nitrophenyl)-propionyl]-3-methyl-urea,
[3-cyclopropyl-2-(3,4-dichloro-phenyl)-propionyl]-urea,
[3-cyclobutyl-2-(3,4-dichloro-phenyl)-propionyl]-urea,
3-[cyclohexyl-2-(3,4-dichloro-phenyl)-propionyl]-urea,
[3-cyclohexyl-2-(3,4-dichloro-phenyl)-propionyl]-3-methyl-urea,
[3-cycloheptyl-2-(3,4-dichloro-phenyl)-propionyl]-urea,
3-{3-[3-cyclopentyl-2-(3,4-dichloro-phenyl)-propionyl]-ureido}-propionic acid ethyl ester,
{3-[3-cyclopentyl-2-(3,4-dichloro-phenyl)-propionyl]-ureido)-acetic acid ethyl ester,
{3-[3-cyclopentyl-2-(3,4-dichloro-phenyl)-propionyl]-ureido}-acetic acid methyl ester,
3-{3-[3-cyclopentyl-2-(3,4-dichloro-phenyl)-propionyl]-ureido}-propionic acid methyl ester,
{3-[3-cyclopentyl-2(R)-(3,4-dichloro-phenyl)-propionyl]-ureido}-acetic acid ethyl ester,
3-{3-[3-cyclopentyl-2-(3,4-dichloro-phenyl)-propionyl]-ureido}-3-oxo-propionic acid ethyl ester,
1-[3-cyclopentyl-2-(3,4-dichloro-phenyl)-proprionyl]-3-(2-hydroxy-ethyl)-urea,
1-[3-cyclopentyl-2-(3,4-dichloro-phenyl)-proprionyl]-3-(2-hydroxy-propyl)-urea,
1-[3-cyclopentyl-2-(3,4-dichloro-phenyl)-proprionyl]-3-(3-hydroxy-propyl)-urea,
1-[3-cyclopentyl-2(R)-(3,4-dichloro-phenyl)-proprionyl]-3-(2-hydroxy-propyl)-urea,
1-(2-chloro-ethyl)-3-[3-cyclopentyl-2-(3,4-dichloro-phenyl)-proprionyl]-urea, and
1-[3-cyclopentyl-2(R)-(3,4-dichloro-phenyl)-proprionyl]-3-(3-hydroxy-propyl)-urea.

16. A compound according to any of claims 1 to 14, selected from the group consisting of:
3-Cyclopentyl-2-(4-methanesulfonyl-phenyl)-N-thiazol-2-yl-propionamide,
3-Cyclopentyl-N-thiazol-2-yl-2-(4-trifluoromethoxy-phenyl)-propionamide,
3-Cyclopentyl-N-thiazol-2-yl-2-(4-trifluoromethanesulfonyl-phenyl)-propionamide,
3-Cyclopentyl-2(R)-(3,4-dichloro-phenyl)-N-pyridin-2-yl-propionamide,
6-[3-Cyclopentyl-2(R)-(3,4-dichloro-phenyl)-propionylamino]-nicotinic acid methyl ester,
N-(5-Chloro-pyridin-2-yl)-3-cyclopentyl-2(R)-(3,4-dichloro-phenyl)-propionamide,
3-Cyclopentyl-N-pyridin-2-yl-2-(4-trifluoromethanesulfonyl-phenyl)-propionamide,
3-Cyclopentyl-N-(5-methyl-pyridin-2-yl)-2-(4-trifluoromethanesulfonyl-phenyl)-propionamide,
3-Cyclopentyl-2(R)-(3,4-dichloro-phenyl)-N-(5-hydroxymethyl-pyridin-2-yl) propionamide,
6-[3-Cyclopentyl-2-(4-trifluoromethanesulfonyl-phenyl)-propionylamino]-nicotinic acid methyl ester,
3-Cyclopentyl-2-(3-fluoro-4-trifluoromethyl-phenyl)-N-pyridin-2-yl-propionamide,
3-Cyclopentyl-2-(4-methanesulfonyl-3-nitrophenyl)-N-pyridin-2-yl-propionamide,
2-(3-Bromo-4-methanesulfonyl-phenyl)-3-cyclopentyl-N-pyridin-2-yl-propionamide,
2-(3-Cyano-4-methanesulfonyl-phenyl)-3-cyclopentyl-N-pyridin-2-yl-propionamide,
2-(4-Chloro-3-nitro-phenyl)-3-cyclopentyl-N-pyridin-2-yl-propionamide,
2-(3-Chloro-4-methanesulfonyl-phenyl)-3-cyclopentyl-N-pyridin-2-yl-propionamide,
N-(5-Bromo-pyridin-2-yl)-2-(3-chloro-4-methanesulfonyl-phenyl)-3-cyclopentyl-propionamide,
2-[3-Chloro-4-methanesulfonyl-phenyl]-3-cyclopentyl-N-thiazol-2-yl-propionamide,
(2R)-3-Cyclopentyl-2-(4-methanesulfonylphenyl)-N-thiazol-2-yl-propionamide,
2-(3-Bromo-4-methanesulfonyl-phenyl)-3-cyclopentyl-N-thiazol-2-yl-propionamide,
2-(3-Cyano-4-methanesulfonyl-phenyl)-3-cyclopentyl-N-thiazol-2-yl-propionamide,
3-Cyclopentyl-2-(4-ethanesulfonyl-phenyl)-N-thiazol-2-yl-propionamide,
3-Cyclopentyl-2-(4-methanesulfonyl-3-trifluoromethyl-phenyl)-N-thiazol-2-yl-propionamide, and
N-(5-Bromo-pyridin-2-yl)-2(R)-(3-chloro-4-methanesulfonyl-phenyl)-3-cyclopentyl-propionamide.

17. A compound according to any of claims 1 to 13 and 15, selected from the group consisting of:
1-[3-cyclopentyl-2(R)-(3,4-dichloro-phenyl)-propionyl]-3-ethyl-urea,
1-[3-cyclopentyl-2(R)-(3,4-dichloro-phenyl)-propionyl]-3-methyl-urea,
1-[3-cyclopentyl-2-(3,4-dichloro-phenyl)-propionyl]-3-methyl urea,
1-[3-cyclopentyl-2-(4-methanesulfonyl-phenyl)-propionyl)-3-methyl urea,
1-Allyl-3-[3-cyclopentyl-2(R)-(3,4-dichloro-phenyl)-propionyl]-urea,
1-[2-(3-Chloro-4-methanesulfonyl-phenyl)-3-cyclopentyl-propionyl]-3-methyl-urea,
1-[2(R)-(3-Chloro-4-methanesulfonyl-phenyl)-3-cyclopentyl-propionyl]-3-methyl-urea, and
1-[2-(3-Bromo-4-methanesulfonyl-phenyl)-3-cyclopentyl-propionyl]-3-methyl-urea.

18. A pharmaceutical composition comprising a compound of any of claims 1 to 17 and a pharmaceutically acceptable carrier and/or adjuvant.

19. The use of a compound according to any of claims 1 to 17 for the preparation of medicaments containing a compound according to any of claims 1 to 17 for the treatment or prophylaxis of type II diabetes.

20. A process for the preparation of the compounds of formula I according to any of claims 1 to 17, 21 and 22, which process comprises:
(a) The condensation of a compound of formula XI: 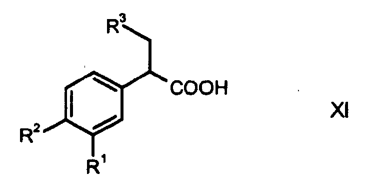 wherein R¹, R² and R³ are as defined in claim 1;
with a compound of the formula VIII:
R⁴²-NH₂ VIII
wherein R⁴² is an unsubstituted or mono-substituted five- or six-membered heteroaromatic ring connected by a ring carbon atom to the amine group shown, which five- or six-membered heteroaromatic ring contains from 1 to 3 heteroatoms selected from sulfur, oxygen or nitrogen, with one heteroatom being nitrogen which is adjacent to the connecting ring carbon atom; said mono-substituted heteroaromatic ring being monosubstituted at a position on a ring carbon atom other than adjacent to said connecting carbon atom with a substituent selected from the group consisting of lower alkyl, halo, nitro, cyano, -(CH₂)ₙ-OR⁶, -(CH₂)ₙ-C(O)OR⁷, -(CH₂)ₙ-C(O)NHR⁶, - C(O)-C(O)OR⁸ or -(CH₂)ₙ-NHR⁶; R⁶, R⁷, R⁸ and n are as defined in claim 1;
via peptide coupling to produce a compound of formula I-d: 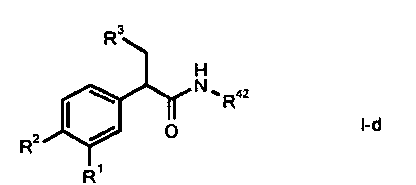 wherein R¹, R², R³ and R⁴² are as defined above;
optionally followed by the conversion of one or both of the substituents R¹ and/or R² into another substituent R¹ and/or R² as defined in claim 1.
(b) The reaction of a compound of formula IX: 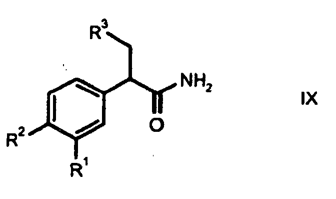 wherein R¹, R³ and R³ are as defined in claim 1;
with a compound of the formula X:
R⁴¹-N=C=O X
wherein R⁴¹ is lower alkyl, lower alkenyl, hydroxy lower alkyl, halo lower alkyl or - (CH₂)ₙ-C(O)OR⁵, wherein R⁵ is hydrogen or lower alkyl and n is as defined above;
to produce a compound of formula I-a: 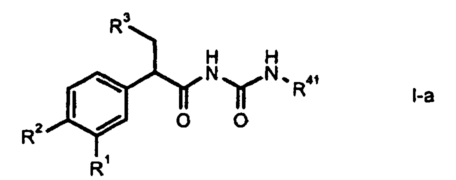 wherein R¹, R², R³ and R⁴¹ are as defined above;
optionally followed by the conversion of one or both of the substituents R¹ and/or R² into another substituent R¹ and/or R² as defined in claim 1.
(c) The reaction of a compound of the formula XI: 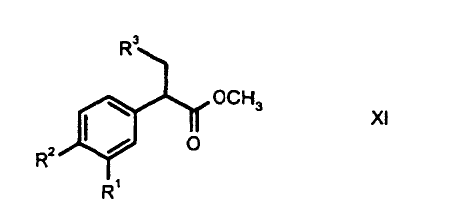 wherein R¹, R² and R³ are as defined in claim 1;
with a compound of the formula:
H₂NCONH₂
to produce a compound of the formula I-b: 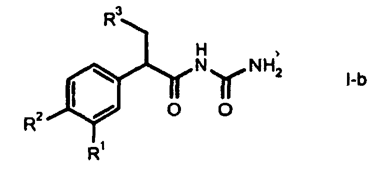 wherein R¹, R² and R³ are as defined above;
optionally followed by the conversion of one or both of the substituents R¹ and/or R² into another substituent R¹ and/or R² as defined in claim 1.
(d) The reaction of a compound of the formula I-b 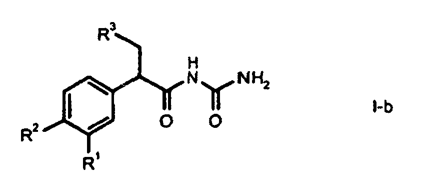 wherein R¹, R² and R³ are as defined in claim 1;
with a compound of the formula XIII:
ClC(O)-(CH₂)ₙ-C(O)OR⁶ XIII
wherein R⁶ is hydrogen or lower alkyl and n is as defined in claim 1; to produce a compound of the formula I-c: 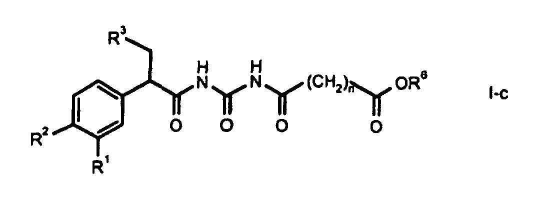
wherein R¹, R², R³, R⁶ and n are as defined above;
optionally followed by the conversion of one or both of the substituents R¹ and/or R² into another substituent R¹ and/or R² as defined in claim 1.

21. 3-Cyclopentyl-2-(4-methanesulfonyl-phenyl)-N-thiazol-2-yl-propionamide.

22. (2R)-3-Cyclopentyl-2-(4-methanesulfonyl-phenyl)-N-thiazol-2-yl-propionamide.

## Patentansprüche

1. Verbindung umfassend ein Amid der Formel I: 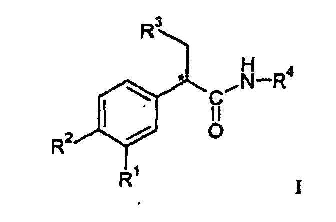 wobei
der * ein asymmetrisches Kohlenstoffatom anzeigt;
R¹ und R² unabhängig voneinander Wasserstoff, Halogen, Amino, Hydroxyamino, Nitro, Cyano, Sulfonamido, Niederalkyl, -OR⁵, -C(O)OR⁵, Perfluorniederalkyl, Niederalkylthio, Perfluorniederalkylthio, Niederalkylsulfonyl, Perfluorniederalkylsulfonyl oder Niederalkylsulfinyl sind;
R³ ist Cycloalkyl mit 3 bis 7 Kohlenstoffatomen;
R⁴ ist -C(O)NHR⁴⁰; oder ein unsubstituierter oder monosubstituierter fünf- oder sechsgliedriger heteroaromatischer Ring, der mit einem Ringkohlenstoffatom an die gezeigte Amingruppe gebunden ist, wobei der fünf- oder sechsgliedrige heteroaromatische Ring 1 bis 3 Heteroatome enthält, die ausgewählt sind aus Schwefel, Sauerstoff oder Stickstoff, wobei ein Heteroatom Stickstoff ist, das zu dem verbindenden Ringkohlenstoffatom benachbart ist; und wobei der mono-substituierte heteroaromatische Ring in einer Position an einem Ringkohlenstoffatom mono-substituiert ist, das nicht dasjenige ist, das zu dem verbindenden Kohlenstoffatom benachbart ist, mit einem Substituenten ausgewählt aus der Gruppe bestehend aus Niederalkyl, Halo, Nitro, Cyano, -(CH₂)ₙ-OR⁶, -(CH₂)ₙ-C(O)OR⁷, -(CH₂)ₙ-C(O)NHR⁶, -C(O)-C(O)OR⁸, -(CH₂)ₙ-NHR⁶;
R⁴⁰ ist Wasserstoff, Niederalkyl, Niederalkenyl, Hydroxyniederalkyl, Haloniederalkyl, - (CH₂)ₙ-C(O)OR⁵ oder -C(O)-(CH₂)ₙ-C(O)OR⁶;
R⁵ ist Wasserstoff, Niederalkyl oder Perfluorniederalkyl;
R⁶, R⁷ und R⁸ sind unabhängig voneinander Wasserstoff oder Niederalkyl; und
n ist 0, 1, 2, 3 oder 4;
"Niederalkyl" und "Niederalkoxy" bedeuten Gruppen mit 1 bis 7 Kohlenstoffatomen; und
"Niederalkenyl" bedeutet Gruppen mit 2 bis 6 Kohlenstoffatomen;
oder ein pharmazeutisch verträgliches Salz davon.

2. Verbindung gemäß Anspruch 1, wobei
R¹ und R² unabhängig voneinander Wasserstoff, Halo, Amino, Hydroxyamino, Cyano, Nitro, Niederalkyl, -OR⁵, -C(O)OR⁵, Perfluorniederalkyl, Niederalkylthio, Perfluorniederalkylthio, Niederalkylsulfonyl, Perfluorniederalkylsulfonyl, Niederalkylsulfinyl oder Sulfonamido sind;
R⁴ ist ein unsubstituierter oder monosubstituierter fünf- oder sechsgliedriger heteroaromatischer Ring, der mit einem Ringkohlenstoffatom an die gezeigte Amingruppe gebunden ist, wobei dieser fünf- oder sechsgliedrige heteroaromatische Ring 1 bis 3 Heteroatome enthält, die ausgewählt sind aus Schwefel, Sauerstoff oder Stickstoff, wobei ein Heteroatom Stickstoff ist, das zu dem verbindenden Ringkohlenstoffatom benachbart ist; und wobei der mono-substituierte heteroaromatische Ring in einer Position an einem Ringkohlenstoffatom mono-substituiert ist, das nicht dasjenige ist, das zu dem verbindenden Kohlenstoffatom benachbart ist, mit einem Substituenten ausgewählt aus der Gruppe bestehend aus Niederalkyl, Halo, Nitro, Cyano, -(CH₂)ₙ-OR⁶, -(CH₂)ₙ-C(O)OR⁷, -(CH₂)ₙ-C(O)NHR⁶, -C(O)-C(O)OR⁸, -(CH₂)ₙ-NHR⁶;
n ist 0, 1, 2, 3 oder 4;
R⁵ ist Wasserstoff, Niederalkyl oder Perfluomiederalkyl; und
R⁶, R⁷ und R⁸ sind unabhängig voneinander Wasserstoff oder Niederalkyl;
oder ein pharmazeutisch verträgliches Salz davon.

3. Verbindung gemäß einem der Ansprüche 1 bis 2, wobei R⁴ ein unsubstituierter oder monosubstituierter fünf- oder sechsgliedriger heteroaromatischer Ring ist, ausgewählt aus der Gruppe bestehend aus Thiazolyl, Pyridinyl, Imidazolyl, Isoxazolyl, Oxazolyl, Pyridazinyl, Pyrimidinyl oder Thiadiazolyl.

4. Verbindung nach einem der Ansprüche 1 bis 3, wobei R⁴ unsubstituiertes Thiazolyl, unsubstituiertes Pyridinyl oder Pyridinyl substituiert mit Halogen, Niederalkyl, Hydroxyniederalkyl oder -C(O)OR⁵ ist, wobei R⁵ Niederalkyl ist.

5. Verbindung gemäß Anspruch 1, wobei
R¹ und R² unabhängig voneinander Wasserstoff, Halo, Amino, Nitro, Cyano, Sulfonamido, Niederalkyl, Perfluorniederalkyl, Niederalkylthio, Perfluorniederalkylthio, Niederalkylsulfonyl oder Perfluorniederalkylsulfonyl sind;
R⁴ ist -C(O)NHR⁴⁰;
R⁴⁰ ist Wasserstoff, Niederalkyl, Niederalkenyl, Hydroxyniederalkyl, Haloniederalkyl, - (CH₂)ₙ-C(O)OR⁵ oder -C(O)-(CH₂)ₙ-C(O)OR⁶;
R⁵ und R⁶ sind Wasserstoff oder Niederalkyl; und
n ist 0, 1, 2, 3 oder 4;
oder ein pharmazeutisches verträgliches Salz davon.

6. Verbindung gemäß einem der Ansprüche 1 oder 5, wobei R⁴ -C(O)NHR⁴⁰ ist und R⁴⁰ Niederalkyl oder Niederalkenyl ist.

7. Verbindung gemäß einem der Ansprüche 1 bis 6, wobei R¹ Wasserstoff, Halo, Nitro oder Cyano ist.

8. Verbindung gemäß einem der Ansprüche 1 bis 7, wobei R¹ Wasserstoff oder Halo ist.

9. Verbindung gemäß einem der Ansprüche 1 bis 8, wobei R² Wasserstoff, Niederalkylsulfonyl, Perfluorniederalkyl, Perfluorniederalkylsulfonyl, Halo oder -OR⁵ ist, wobei R⁵ Perfluorniederalkyl ist.

10. Verbindung gemäß einem der Ansprüche 1 bis 9, wobei R² Halo oder Niederalkylsulfonyl ist.

11. Verbindung nach einem der Ansprüche 1 bis 10, wobei das Amid an dem gezeigten asymmetrischen Kohlenstoffatom in der R-Konfiguration ist.

12. Verbindung gemäß einem der Ansprüche 1 bis 11, wobei R³ Cyclopentyl, Cyclohexyl oder Cycloheptyl ist.

13. Verbindung gemäß einem der Ansprüche 1 bis 12, wobei R³ Cyclopentyl ist.

14. Verbindung gemäß einem der Ansprüche 1 bis 13, ausgewählt aus der Gruppe bestehend aus:
2-(3-Chlorphenyl)-3-cyclopentyl-N-thiazol-2-ylpropionamid,
2-(4-Bromphenyl)-3-cyclopentyl-N-thiazol-2-ylpropionamid,
2-(4-Chlorphenyl)-3-cyclopentyl-N-thiazol-2-ylpropionamid,
3-Cyclopentyl-2-(3,4-dichlorphenyl)-N-thiazol-2-ylpropionamid,
3-Cyclopentyl-N-thiazol-2-yl-2-(4-trifluormethylphenyl)propionamid,
3-Cyclopentyl-2-(3-fluor-4-trifluormethylphenyl)-N-thiazol-2-ylpropionamid,
3-Cyclopentyl-2-(4-fluor-3-trifluormethylphenyl)-N-thiazol-2-ylpropionamid,
3-Cyclopentyl-N-thiazol-2-yl-2-(3-trifluormethylphenyl)propionamid,
3-Cyclopentyl-2(R)-(3,4-dichlorphenyl)-N-thiazol-2-ylpropionamid,
3-Cyclopentyl-2-(4-nitrophenyl)-N-thiazol-2-ylpropionamid,
2-(4-Aminophenyl)-3-cyclopentyl-N-thiazol-2-ylpropionamid,
2-(3-Aminophenyl)-3-cyclopentyl-N-thiazol-2-ylpropionamid,
2-(4-Chlor-3-nitrophenyl)-3-cyclopentyl-N-thiazol-2-ylpropionamid,
2-(4-Cyanophenyl)-3-cyclopentyl-N-thiazol-2-ylpropionamid,
3-Cyclopentyl-N-thiazol-2-yl-2-(4-trifluormethylsulfanylphenyl)propionamid,
3-Cyclopentyl-2-(4-methylsulfanylphenyl)-N-thiazol-2-ylpropionamid,
3-Cyclopentyl-2-(4-methylsulfanyl-3-trifluormethylphenyl)-N-thiazol-2-ylpropionamid,
3-Cyclopentyl-2-(4-methansulfinylphenyl)-N-thiazol-2-ylpropionamid,
3-Cyclopentyl-2-(4-methansulfonylphenyl)-N-thiazol-2-ylpropionamid,
3-Cyclopentyl-2-(4-methansulfonyl-3-nitrophenyl)-N-thiazol-2-ylpropionamid,
2-(3-Amino-4-methansulfonylphenyl)-3-cyclopentyl-N-thiazol-2-ylpropionamid,
3-Cyclopentyl-2-(3-hydroxyamino-4-methansulfonylphenyl)-N-thiazol-2-ylpropionamid,
2-(3-Cyano-4-methansulfonylphenyl)-3-cyclopentyl-N-thiazol-2-ylpropionamid,
3-Cyclopentyl-2-(4-ethansulfonylphenyl)-N-thiazol-2-ylpropionamid,
2-(3,4-Bismethansulfonylphenyl)-3-cyclopentyl-N-thiazol-2-ylpropionamid,
3-Cyclopentyl-2-(4-sulfamoylphenyl)-N-thiazol-2-ylpropionamid,
2-[4-(Butan-1-sulfonyl)phenyl]-3-cyclopentyl-N-thiazol-2-ylpropionamid,
3-Cyclopentyl-2-[4-(propan-1-sulfonyl)phenyl]-N-thiazol-2-ylpropionamid,
3-Cyclopentyl-2-(4-methansulfonyl-3-trifluormethylphenyl)-N-thiazol-2-ylpropionamid,
2(R)-(3-Chlor-4-methansulfonylphenyl)-3-cyclopentyl-N-thiazol-2-ylpropionamid,
2-[3-Chlor-4-methansulfonylphenyl]-3-cyclopentyl-N-thiazol-2-ylpropionamid,
2-(3-Brom-4-methansulfonylphenyl)-3-cyclopentyl-N-thiazol-2-ylpropionamid,
3-Cyclopentyl-N-thiazol-2-yl-2-(4-trifluormethansulfonylphenyl)propionamid,
3-Cyclopentyl-N-thiazol-2-yl-2-(3-trifluormethansulfonylphenyl)propionamid,
3-Cyclopentyl-N-thiazol-2-yl-2-(4-trifluormethoxyphenyl)propionamid,
3-Cyclopentyl-2-(3-methoxyphenyl)-N-thiazol-2-ylpropionamid,
3-Cyclopentyl-2-(3-hydroxyphenyl)-N-thiazol-2-ylpropionamid,
3-Cyclopentyl-2-(3,4-dimethoxyphenyl)-N-thiazol-2-ylpropionamid,
3-Cyclopentyl-2-(3,4-dihydroxyphenyl)-N-thiazol-2-ylpropionamid,
3-Cyclopentyl-2-(4-methoxyphenyl)-N-thiazol-2-ylpropionamid,
3-Cyclopentyl-2-(4-hydroxyphenyl)-N-thiazol-2-ylpropionamid,
4-[2-Cyclopentyl-1-(thiazol-2-ylcarbamoyl)ethyl]benzoesäuremethylester,
3-Cyclopentyl-2-(3-fluor-4-methoxyphenyl)-N-thiazol-2-ylpropionamid,
3-Cyclopentyl-2-(3-fluor-4-hydroxyphenyl)-N-thiazol-2-ylpropionamid,
3-Cyclopentyl-2-(3,4-dichlorphenyl)-N-(5-hydroxymethylthiazol-2-yl)propionamid,
3-Cyclopentyl-2-(3,4-dichlorphenyl)-N-[4-(2-hydroxyethyl)thiazol-2-yl]propionamid,
2-(4-Chlorphenyl)-3-cyclopentyl-N-(5-hydroxymethylthiazol-2-yl)propionamid,
3-Cyclopentyl-2-(3,4-dichlorphenyl)-N-(4-hydroxymethylthiazol-2-yl)propionamid,
3-Cyclopentyl-N-(4-hydroxymethylthiazol-2-yl)-2-(4-methansulfonylphenyl)propionamid,
3-Cyclopentyl-N-[4-(2-hydroxyethyl)thiazol-2-yl]-2-(4-methansulfonylphenyl)propionamid,
3-Cyclopentyl-2-(3,4-dichlorphenyl)-N-(4-methylthiazol-2-yl)propionamid,
3-Cyclopentyl-2-(3,4-dichlorphenyl)-N-(5-methylthiazol-2-yl)propionamid,
{2-[2-(3-Chlorphenyl)-3-cyclopentylpropionylamino]thiazol-4-yl}essigsäureethylester,
{2-[2-(3-Chlorphenyl)-3-cyclopentylpropionylamino]thiazol-4-yl}essigsäuremethylester,
2-[2-(3-Chlorphenyl)-3-cyclopentylpropionylamino]thiazol-4-carbonsäuremethylester,
2-[2-(3-Chlorphenyl)-3-cyclopentylpropionylamino]thiazol-4-carbonsäureethylester,
{2-[2-(4-Chlorphenyl)-3-cyclopentylpropionylamino]thiazol-4-yl}essigsäureethylester,
2-[2-(4-Chlorphenyl)-3-cyclopentylpropionylamino]thiazol-4-carbonsäuremethylester,
2-[2-(4-Chlorphenyl)-3-cyclopentylpropionylamino]thiazol-4-carbonsäureethylester,
{2-[2-(4-Chlorphenyl)-3-cyclopentylpropionylamino]thiazol-4-yl}essigsäuremethylester,
{2-[3-Cyclopentyl-2-(3,4-dichlorphenyl)propionylamino]thiazol-4-yl}essigsäure,
{2-[3-Cyclopentyl-2-(3,4-dichlorphenyl)propionylamino]thiazol-4-yl}essigsäureethylester,
2-[3-Cyclopentyl-2-(3,4-dichlorphenyl)propionylamino]thiazol-5-carbonsäure,
2-[3-Cyclopentyl-2-(3,4-dichlorphenyl)propionylamino]thiazol-4-carbonsäure,
{2-[3-Cyclopentyl-2-(3,4-dichlorphenyl)propionylamino]thiazol-4-yl}essigsäuremethylester,
(2R)-2-[3-Cyclopentyl-2-(3,4-dichlorphenyl)propionylamino]thiazol-4-carbonsäuremethylester,
2-[3-Cyclopentyl-2-(3,4-dichlorphenyl)propionylamino]thiazol-5-carbonsäuremethylester,
2-[3-Cyclopentyl-2-(3,4-dichlorphenyl)propionylamino]thiazol-5-carbonsäureethylester,
{2-[3-Cyclopentyl-2-(4-nitrophenyl)propionylamino]thiazol-4-yl}essigsäureethylester.
{2-[3-Cyclopentyl-2-(4-nitrophenyl)propionylamino]thiazol-4-yl}essigsäuremethylester,
2-[3-Cyclopentyl-2-(4-nitrophenyl)propionylamino]thiazol-4-carbonsäuremethylester,
2-[3-Cyclopentyl-2-(4-nitrophenyl)propionylamino]thiazol-4-carbonsäureethylester,
{2-[2-(4-Aminophenyl)-3-cyclopentylpropionylamino]thiazol-4-yl}essigsäuremethylester,
2-[2-(4-Aminophenyl)-3-cyclopentylpropionylamino]thiazol-4-carbonsäuremethylester,
{2-[3-Cyclopentyl-2-(4-methansulfonylphenyl)propionylamino]thiazol-4-carbonsäureethylester,
{2-[3-Cyclopentyl-2-(4-methansulfonylphenyl)propionylamino]thiazol-4-yl}-4-essigsäuremethylester,
{2-[3-Cyclopentyl-2-(4-methansulfonyl-3-trifluormethylphenyl)propionylamino]thiazol-4-yl}-4-essigsäuremethylester,
2-[3-Cyclopentyl-2-(4-methansulfonyl-3-trifluormethylphenyl)propionylamino]thiazol-4-carbonsäuremethylester,
{2-[3-Cyclopentyl-2-(4-nitrophenyl)propionylamino]thiazol-4-yl}oxoessigsäureethylester, {2-[3-Cyclopentyl-2-(3,4-dichlorphenyl)propionylamino]thiazol-5-yl}oxoessigsäureethylester,
{2-[3-Cyclopentyl-2-(4-fluor-3-trifluormethylphenyl)propionylamino]thiazol-4-yl}oxoessigsäureethylester,
{2-[2-(3-Chlor-4-methansulfonylphenyl)-3-cyclopentylpropionylamino]thiazol-4-yl}oxoessigsäureethylester,
3-Cyclopentyl-2-(3,4-dichlorphenyl)-N-(5-nitrothiazol-2-yl)propionamid,
3-Cyclopentyl-2-(3-fluor-4-trifluormethylphenyl)-N-pyridin-2-ylpropionamid,
3-Cyclopentyl-2-(3,4-dichlorphenyl)-N-pyridin-2-ylpropionamid,
3-Cyclopentyl-N-pyridin-2-yl-2-(4-trifluormethylphenyl)-2-propionamid,
3-Cyclopentyl-N-thiazol-2-yl-2-(3-trifluormethylphenyl)propionamid,
2-(3-Chlorphenyl)-3-cyclopentyl-N-pyridin-2-ylpropionamid,
2-(4-Aminophenyl)-3-cyclopentyl-N-pyridin-2-ylpropionamid,
2-(4-Cyanophenyl)-3-cyclopentyl-N-pyridin-2-ylpropionamid,
2-(4-Chlorphenyl)-3-cyclopentyl-N-pyridin-2-ylpropionamid,
2-(4-Chlor-3-nitrophenyl)-3-cyclopentyl-N-pyridin-2-ylpropionamid,
3-Cyclopentyl-2-(4-nitrophenyl)-N-pyridin-2-ylpropionamid,
2-(4-Cyanophenyl)-3-cyclopentyl-N-pyridin-2-ylpropionamid,
3-Cyclopentyl-2-(4-methylsulfanylphenyl)-N-pyridin-2-ylpropionamid,
3-Cyclopentyl-N-pyridin-2-yl-2-(4-trifluormethylsulfanylphenyl)propionamid,
3-Cyclopentyl-2-(4-methansulfonyl-3-nitrophenyl)-N-pyridin-2-ylpropionamid,
3-Cyclopentyl-2-(4-methansulfonylphenyl)-N-pyridin-2-ylpropionamid,
2-(3-Brom-4-methansulfonylphenyl)-3-cyclopentyl-N-pyridin-2-ylpropionamid,
2-(3-Cyano-4-methansulfonylphenyl)-3-cyclopentyl-N-pyridin-2-ylpropionamid,
3-Cyclopentyl-2-(4-ethansulfonylphenyl)-N-pyridin-2-ylpropionamid,
2-(3,4-Bismethansulfonylphenyl)-3-cyclopentyl-N-pyridin-2-ylpropionamid,
2-(3-Chlor-4-methansulfonylphenyl)-3-cyclopentyl-N-pyridin-2-ylpropionamid,
3-Cyclopentyl-2-(4-methansulfonyl-3-trifluormethylphenyl)N-pyridin-2-ylpropionamid,
3-Cyclopentyl-N-pyridin-2-yl-2-(4-trifluormethansulfonylphenyl)propionamid,
3-Cyclopentyl-2-(3,4-dichlorphenyl)-N-(5-carboxymethylpyridin)-2-ylpropionamid,
6-[3-Cyclopentyl-2(R)-(3,4-dichlorphenyl)propionylamino]nicotinsäuremethylester,
6-[2-(4-Chlorphenyl)-3-cyclopentylpropionylamino]nicotinsäure,
6-[2-(3-Chlorphenyl)-3-cyclopentylpropionylamino]nicotinsäuremethylester,
6-[2-(4-Chlorphenyl)-3-cyclopentylpropionylamino]nicotinsäuremethylester,
6-[3-Cyclopentyl-2-(4-nitrophenyl)propionylamino]nicotinsäuremethylester,
6-[2-(4-Aminophenyl)-3-cyclopentylpropionylamino]nicotinsäuremethylester,
6-[2-(3-Chlorphenyl)-3-cyclopentylpropionylamino]nicotinsäure,
6-[2-(4-Cyanophenyl)-3-cyclopentylpropionylamino]nicotinsäure,
6-[3-Cyclopentyl-2-(4-trifluormethansulfonylphenyl)propionylamino]nicotinsäuremethylester,
6-[3-Cyclopentyl-2-(4-trifluormethansulfonylphenyl)propionylamino]nicotinsäure,
6-[3-Cyclopentyl-2-(4-methansulfonylphenyl)propionylamino]nicotinsäuremethylester,
3-Cyclopentyl-2(R)-(3,4-dichlorphenyl)-N-(5-hydroxymethylpyridin-2-yl)propionamid,
3-Cyclopentyl-2-(3,4-dichlorphenyl)-N-(5-hydroxypyridin-2-yl)propionamid,
2-(4-Chlorphenyl)-3-cyclopentyl-N-(5-hydroxymethylpyridin-2-yl)propionamid,
3-Cyclopentyl-N-(5-hydroxymethylpyridin-2-yl)-2-(4-methansulfonylphenyl)propionamid,
N-(5-Chlorpyridin-2-yl)-3-cyclopentyl-2-(3,4-dichlorphenyl)propionamid,
3-Cyclopentyl-2-(3,4-dichlorphenyl)-N-(5-brompyridin)-2-ylpropionamid,
3-Cyclopentyl-2-(R)-(3,4-dichlorphenyl)-N-(5-trifluormethylpyridin-2-yl)propionamid,
N-(5-Brompyridin-2-yl)-3-cyclopentyl-2-(R)-(3,4-dichlorphenyl)propionamid,
N-(5-Chlorpyridin-2-yl)-3-cyclopentyl-2-(4-trifluormethansulfonylphenyl)propionamid,
N-(5-Brompyridin-2-yl)-3-cyclopentyl-2-(4-trifluormethansulfonylphenyl)propionamid,
N-(5-Brompyridin-2-yl)-3-cyclopentyl-2-(4-methansulfonyl-3-nitrophenyl)propionamid,
2-(3-Brom-4-methansulfonylphenyl)-N-(5-brompyridin-2-yl)-3-cyclopentylpropionamid,
N-(5-Brompyridin-2-yl)-2-(3-chlor-4-methansulfonylphenyl)-3-cyclopentylpropionamid,
2-(3-Chlor-4-methansulfonylphenyl)-3-cyclopentyl-N-(5-trifluormethylpyridin-2yl)propionamid,
N-(5-Chlorpyridin-2-yl)-2-(3-chlor-4-methansulfonylphenyl)-3-cyclopentylpropionamid,
3-Cyclopentyl-2-(3,4-dichlorphenyl)-N-(5-nitropyridin)-2-ylpropionamid,
3-Cyclopentyl-2-(3,4-dichlorphenyl)-N-(5-methylpyridin)-2-ylpropionamid,
3-Cyclopentyl-2-(3,4-dichlorphenyl)-N-(4-methylpyridin)-2-ylpropionamid,
3-Cyclopentyl-2-(3,4-dichlorphenyl)-N-(6-methylpyridin)-2-ylpropionamid,
3-Cyclopentyl-N-(5-methylpyridin-2-yl)-2-(4-trifluormethansulfonylphenyl)propionamid,
3-Cyclopentyl-2-(4-fluor-3-trifluormethylphenyl)-N-(5-methylpyridin-2-yl)propionamid,
6-[3-Cyclopentyl-2-(3,4-dichlorphenyl)propionylamino]-N-methylnicotinamid,
3-Cyclopentyl-2-(3,4-dichlorphenyl)-N-(1 H-imidazol-2-yl)propionamid,
3-Cyclopentyl-2-(3,4-dichlorphenyl)-N-(5-methylisoxazol-3-yl)propionamid,
3-Cyclopentyl-2-(3,4-dichlorphenyl)-N-oxazol-2-ylpropionamid,
3-Cyclopentyl-2-(3,4-dichlorphenyl)-N-pyridazin-3-ylpropionamid,
3-Cyclopentyl-2-(3,4-dichlorphenyl)-N-pyrimidin-2-ylpropionamid,
3-Cyclopentyl-2-(3,4-dichlorphenyl)-N-pyrimidin-6-ylpropionamid,
3-Cyclopentyl-2-(4-nitrophenyl)-N-pyrimidin-4-ylpropionamid,
3-Cyclopentyl-2-(3,4-dichlorphenyl)-N-[1,3,4]thiadiazol-2-ylpropionamid,
2-[4-Methansulfonylphenyl]-3-cyclohexyl-N-thiazol-2-ylpropionamid und
2-[4-Methansulfonylphenyl]-3-cycloheptyl-N-thiazol-2-ylpropionamid.

15. Verbindung gemäß einem der Ansprüche 1 bis 13, ausgewählt aus der Gruppe bestehend aus:
1-(3-Cyclopentyl-2-phenylpropionyl)-3-methylharnstoff,
1-[2-(3-Chlorphenyl)-3-cyclopentylpropionyl]-3-methylharnstoff,
1-[2-(4-Chlorphenyl)-3-cyclopentylpropionyl]-3-methylharnstoff,
1-[2-(4-Cyanophenyl)-3-cyclopentylpropionyl]-3-methylharnstoff,
1-[2-(4-Bromphenyl)-3-cyclopentylpropionyl]-3-methylharnstoff,
[3-Cyclopentyl-2-(3,4-dichlorphenyl)propionyl]harnstoff,
1-[3-Cyclopentyl-2-(3,4-dichlorphenyl)propionyl]-3-methylharnstoff,
1-[3-Cyclopentyl-2(R)-(3,4-dichlorphenyl)propionyl]-3-ethylharnstoff,
1-Allyl-3-[3-cyclopentyl-2-(3,4-dichlorphenyl)propionyl]harnstoff,
1-Allyl-3-[3-cyclopentyl-2(R)-(3,4-dichlorphenyl)propionyl]harnstoff,
1-[3-Cyclopentyl-2-(3,4-dichlorphenyl)propionyl]-3-ethylharnstoff,
1-[3-Cyclopentyl-2(R)-(3,4-dichlorphenyl)propionyl]-3-methylharnstoff,
1-[3-Cyclopentyl-2-(3,4-dichlorphenyl)propionyl]-3-isopropylharnstoff,
1-[3-Cyclopentyl-2-(3,4-dichlorphenyl)propionyl]-3-propylharnstoff,
1-[3-Cyclopentyl-2-(3,4-difluorphenyl)propionyl]-3-methylharnstoff,
1-[2-(4-Chlor-3-nitrophenyl)-3-cyclopentylpropionyl]-3-methylharnstoff,
1-[3-Cyclopentyl-2-(4-nitrophenyl)propionyl]-3-methylharnstoff,
1-[3-Cyclopentyl-2-(4-trifluormethylsulfanylphenyl)propionyl]-3-methylharnstoff,
1-[3-Cyclopentyl-2-(4-methylsulfanylphenyl)propionyl]-3-methylharnstoff,
1-[3-Cyclopentyl-2-(4-trifluormethansulfonylphenyl)propionyl]-3-methylharnstoff,
1-[3-Cyclopentyl-2-(3-trifluormethansulfonylphenyl)propionyl]-3-methylharnstoff,
1-[3-Cyclopentyl-2-(4-methansulfonylphenyl)propionyl]-3-methylhamstoff,
1-{2-[4-(Butan-1-sulfonyl)phenyl]-3-cyctopentylpropionyl}-3-methylharnstoff,
1-[3-Cyclopentyl-2-(4-ethansulfonylphenyl)propionyl)-3-methylharnstoff,
1-[2-(3,4-Bismethansulfonylphenyl)-3-cyclopentylpropionyl]-3-methylharnstoff,
1-[2-(3-Brom-4-methansulfonylphenyl)-3-cyclopentylpropionyl]-3-methylhamstoff,
1-[3-Cyclopentyl-2-(3-fluor-4-methansulfonylphenyl)propionyl]-3-methylharnstoff,
1-[2-(3-Chlor-4-methansulfonylphenyl)-3-cyclopentylpropionyl]-3-methylharnstoff,
1-[2(R)-(3-Chlor-4-methansulfonylphenyl)-3-cyclopentylpropionyl]-3-methylhamstoff,
1-[2-(3-Chlor-4-methansulfonylphenyl)-3-cyclopentylpropionyl]-3-ethylharnstoff,
1-[2-(3-Cyano-4-methansulfonylphenyl)-3-cyclopentylpropionyl]-3-methylharnstoff,
1-[3-Cyclopentyl-2-(4-methansulfonyl-3-trifluormethylphenyl)propionyl]-3-methylharnstoff,
1-[3-Cyclopentyl-2-(4-fluor-3-trifluormethylphenyl)propionyl]-3-methylharnstoff,
1-[3-Cyclopentyl-2-(3-fluor-4-trifluormethylphenyl)propionyl]-3-methylharnstoff,
1-[3-Cyclopentyl-2-(4-methansulfonyl-3-nitrophenyl)propionyl)-3-methylharnstoff,
[3-Cyclopropyl-2-(3,4-dichlorphenyl)propionyl]harnstoff,
[3-Cyclobutyl-2-(3,4-dichlorphenyl)propionyl]harnstoff,
[3-Cyclohexyl-2-(3,4-dichlorphenyl)propionyl]harnstoff,
[3-Cyclohexyl-2-(3,4-dichlorphenyl)propionyl]-3-methylharnstoff,
[3-Cycloheptyl-2-(3,4-dichlorphenyl)propionyl]harnstoff,
3- {3-[3-Cyclopentyl-2-(3,4-dichlorphenyl)propionyl]ureido}propionsäureethylester,
{3-[3-Cyclopentyl-2-(3,4-dichlorphenyl)propionyl]ureido}essigsäureethylester,
{3-[3-Cyclopentyl-2-(3,4-dichlorphenyl)propionyl]ureido}essigsäuremethylester,
3-{3-[3-Cyclopentyl-2-(3,4-dichlorphenyl)propionyl]ureido}propionsäuremethylester,
{3-[3-Cyclopentyl-2(R)-(3,4-dichlorphenyl)propionyl]ureido}essigsäureethylester,
3-{3-[3-Cyclopentyl-2-(3,4-dichlorphenyl)propionyl]ureido}-3-oxopropionsäureethylester,
1-[3-Cyclopentyl-2-{3,4-dichlorphenyl)propionyl]-3-(2-hydroxyethyl)harnstoff,
1-[3-Cyclopentyl-2-(3,4-dichlorphenyl)propionyl]-3-(2-hydroxypropyl)harnstoff,
1-[3-Cyclopentyl-2-(3,4-dichlorphenyl)propionyl]-3-(3-hydroxypropyl)harnstoff,
1-[3-Cyclopentyl-2(R)-(3,4-dichlorphenyl)propionyl]-3-(2-hydroxypropyl)harnstoff,
1-(2-Chlorethyl)-3-[3-cyclopentyl-2-(3,4-dichlorphenyl)propionyl]harnstoff und
1-[3-Cyclopentyl-2(R)-(3,4-dichlorphenyl)propionyl]-3-(3-hydroxypropyl)harnstoff.

16. Verbindung gemäß einem der Ansprüche 1 bis 14, ausgewählt aus der Gruppe bestehend aus:
3-Cyclopentyl-2-(4-methansulfonylphenyl)-N-thiazol-2-ylpropionamid,
3-Cyclopentyl-N-thiazol-2-yl-2-(4-trifluormethoxyphenyl)propionamid,
3-Cyclopentyl-N-thiazol-2-yl-2-(4-trifluormethansulfonylphenyl)propionamid,
3-Cyclopentyl-2(R)-(3,4-dichlorphenyl)-N-pyridin-2-ylpropionamid,
6-[3-Cyclopentyl-2(R)-(3,4-dichlorphenyl)propionylamino]nicotinsäuremethylester,
N-(5-Chlorpyridin-2-yl)-3-cyclopentyl-2(R)-(3,4-dichlorphenyl)propionamid,
3-Cyclopentyl-N-pyridin-2-yl-2-(4-trifluormethansulfonylphenyl)propionamid,
3-Cyclopentyl-N-(5-methylpyridin-2-yl)-2-(4-trifluormethansulfonylphenyl)propionamid,
3-Cyclopentyl-2(R)-(3,4-dichlorphenyl)-N-(5-hydroxymethylpyridin-2-yl)propionamid,
6-[3-Cyclopentyl-2-(4-trifluormethansulfonylphenyl)propionylamino]nicotinsäuremethylester,
3-Cyclopentyl-2-(3-fluor-4-trifluormethylphenyl)-N-pyridin-2-ylpropionamid,
3-Cyclopentyl-2-(4-methansulfonyl-3-nitrophenyl)-N-pyridin-2-ylpropionamid,
2-(3-Brom-4-methansulfonylphenyl)-3-cyclopentyl-N-pyridin-2-ylpropionamid,
2-(3-Cyano-4-methansulfonylphenyl)-3-cyclopentyl-N-pyridin-2-ylpropionamid,
2-(4-Chlor-3-nitrophenyl)-3-cyclopentyl-N-pyridin-2-ylpropionamid,
2-(3-Chlor-4-methansulfonylphenyl)-3-cyclopentyl-N-pyridin-2-ylpropionamid,
N-(5-Brompyridin-2-yl)-2-(3-chlor-4-methansulfonylphenyl)-3-cyclopentylpropionamid,
2-[3-Chlor-4-methansulfonylphenyl]-3-cyclopentyl-N-thiazol-2-ylproplonamid,
2(R)-3-Cyclopentyl-2-(4-methansulfonylphenyl)-N-thiazol-2-ylpropionamid,
2-(3-Brom-4-methansulfonylphenyl)-3-cyclopentyl-N-thiazol-2-ylpropionamid,
2-(3-Cyano-4-methansulfonylphenyl)-3-cyclopentyl-N-thiazol-2-ylpropionamid,
3-cyclopentyl-2-(4-ethansulfonylphenyl)-N-thiazol-2-ylpropionamid,
3-Cyclopentyl-2-(4-methansulfonyl-3-trifluormethylphenyl)-N-thiazol-2-ylpropionamid und
N-(5-Brompyridin-2-yl)-2(R)-(3-chlor-4-methansulfonylphenyl)-3-cyclopentylpropionamid.

17. Verbindung gemäß einem der Ansprüche 1 bis 13 und 15, ausgewählt aus der Gruppe bestehend aus:
1-[3-Cyclopentyl-2(R)-(3,4-dichlorphenyl)propionyl]-3-ethylharnstoff,
1-[3-Cyclopentyl-2(R)-(3,4-dichlorphenyl)propionyl]-3-methylharnstoff,
1-[3-Cyclopentyl-2-(3,4-dichlorphenyl)propionyl]-3-methylharnstoff,
1-[3-Cyclopentyl-2-(4-methansulfonylphenyl)propionyl]-3-methylharnstoff,
1-Allyl-3-[3-cyclopentyl-2(R)-(3,4-dichlorphenyl)propionyl]harnstoff,
1-[2-(3-Chlor-4-methansulfonylphenyl)-3-cyclopentylpropionyl]-3-methylharnstoff,
1-[2(R)-(3-Chlor-4-methansulfonylphenyl)-3-cyclopentylpropionyl]-3-methylharnstoff, und
1-[2-(3-Brom-4-methansulfonylphenyl)-3-cyclopentylpropionyl]-3-methylharnstoff,

18. Arzneimittel umfassend eine Verbindung gemäß einem der Ansprüche 1 bis 17 und einen pharmazeutisch verträglichen Träger und/oder Hilfsstoff.

19. Die Verwendung einer Verbindung gemäß einem der Ansprüche 1 bis 17 zur Herstellung von Medikamenten, die eine Verbindung gemäß einer der Ansprüche 1 bis 17 enthalten, zur Behandlung oder Prophylaxe von Typ II Diabetis.

20. Verfahren zur Herstellung von Verbindungen der Formel I gemäß einem der Ansprüche 1 bis 17, 21 und 22, wobei das Verfahren umfasst:
(a) Die Kondensation einer Verbindung der Formel XI: 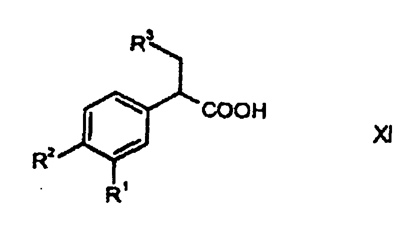 wobei R¹, R² und R³ wie in Anspruch 1 definiert sind;
mit einer Verbindung der Formel VIII:
R⁴²-NH₂ VIII
wobei R⁴² ein unsubstituierter oder monosubstituierter fünf- oder sechsgliedriger heteroaromatischer Ring ist, der mit einem Ringkohlenstoffatom an die gezeigte Amingruppe gebunden ist, wobei der fünf- oder sechsgliedrige heteroaromatische Ring 1 bis 3 Heteroatome enthält, die ausgewählt sind aus Schwefel, Sauerstoff oder Stickstoff, wobei ein Heteroatom Stickstoff ist, das zu dem verbindenden Ringkohlenstoffatom benachbart ist; und wobei der mono-substituierte heteroaromatische Ring in einer Position an einem Ringkohlenstoffatom mono-substituiert ist, das nicht dasjenige ist, das zu dem verbindenden Kohlenstoffatom benachbart ist, mit einem Substituenten ausgewählt aus der Gruppe bestehend aus Niederalkyl, Halo, Nitro, Cyano, -(CH₂)ₙ-OR⁶, -(CH₂)ₙ-C(O)OR⁷, -(CH₂)ₙ-C(O)NHR⁶, -C(O)-C(O)OR⁸ oder -(CH₂)ₙ-NHR⁶; wobei R⁶, R⁷, R⁸ und n wie in Anspruch 1 definiert sind:
über eine Peptidkopplung, um eine Verbindung der Formel I-d herzustellen: 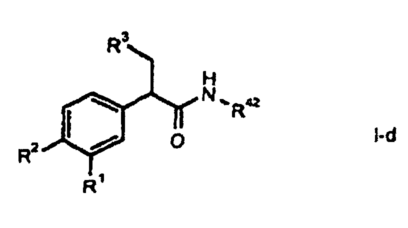 wobei R¹, R², R³ und R⁴² wie vorstehend definiert sind;
gegebenenfalls gefolgt von der Überführung eines oder beider der Substituenten R¹ und/oder R² in einen anderen Substituenten R¹ und/oder R² wie in Anspruch 1 definiert;
(b) die Umsetzung einer Verbindung der Formel IX: 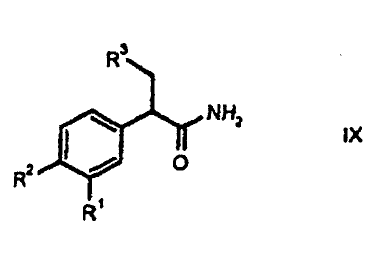 wobei R¹, R² und R³ wie in Anspruch 1 definiert sind;
mit einer Verbindung der Formel X:
R⁴¹-N=C=O X
wobei R⁴¹ Niederalkyl, Niederalkenyl, Hydroxyniederalkyl, Haloniederalkyl oder -(CH₂)ₙ-C(O)OR⁵ ist, wobei R⁵ Wasserstoff oder Niederalkyl ist und n wie vorstehend definiert ist;
um eine Verbindung der Formel I-a herzustellen: 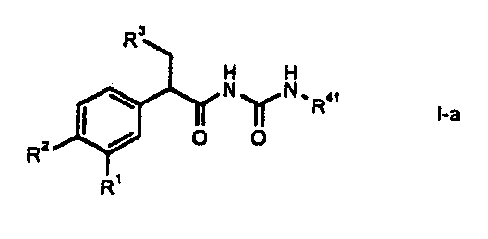 wobei R¹, R²,R³ und R⁴¹ wie vorstehend definiert sind;
gegebenenfalls gefolgt von der Überführung eines oder beider der Substituenten R¹ und/oder R² in einen anderen Substituenten R¹ und/oder R² wie in Anspruch 1 definiert;
(c) die Umsetzung einer Verbindung der Formel XI: 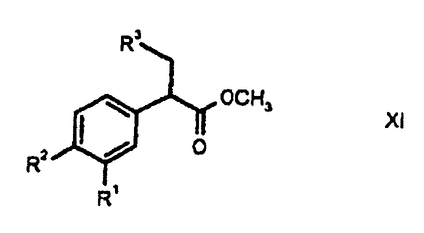 wobei R¹, R² und R³ wie in Anspruch 1 definiert sind;
mit einer Verbindung der Formel:
H₂NCONH₂
um eine Verbindung der Formel I-b herzustellen: 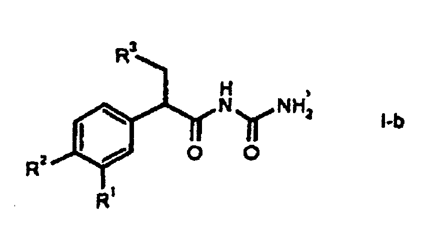 wobei R¹, R² und R³ wie vorstehend definiert sind;
gegebenenfalls gefolgt von der Überführung eines oder beider der Substituenten R¹ und/oder R² in einen anderen Substituenten R¹ und/oder R² wie in Anspruch 1 definiert;
(d) die Umsetzung einer Verbindung der Formel I-b: 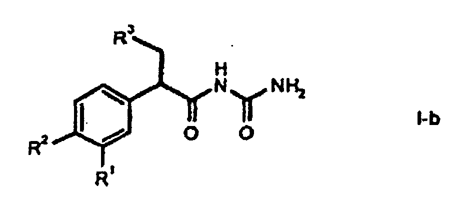
wobei R¹, R² und R³ wie in Anspruch 1 definiert sind;
mit einer Verbindung der Formel XIII:
ClC(O)-(CH₂)ₙ-C(O)OR⁶ XIII
wobei R⁶ Wasserstoff oder Niederalkyl ist und n wie in Anspruch 1 definiert ist;
um eine Verbindung der Formel I-c herzustellen: 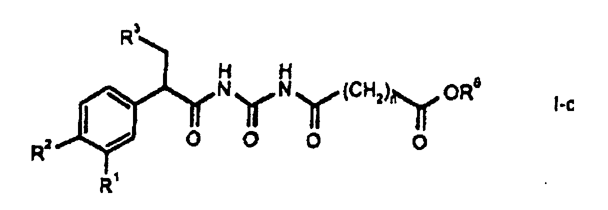 wobei R¹, R², R³, R⁶ und n wie vorstehend definiert sind;
gegebenenfalls gefolgt von der Überführung eines oder beider der Substituenten R¹ und/oder R² in einen anderen Substituenten R¹ und/oder R² wie in Anspruch 1 definiert.

21. 3-Cyclopentyl-2-(4-methansulfonylphenyl)-N-thiazol-2-ylpropionamid.

22. (2R)-3-Cyclopentyl-2-(4-methansulfonylphenyl)-N-thiazol-2-ylpropionamid.

## Revendications

1. Composé comprenant une amide de formule I : 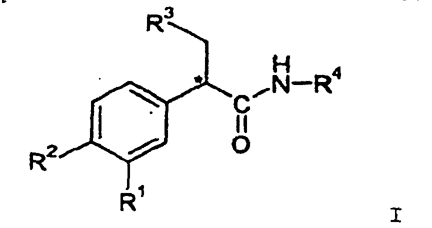 dans laquelle
la * indique un atome de carbone asymétrique ;
R¹ et R² sont indépendamment un hydrogène, un halogène, un amino, un hydroxyamino, un nitro, un cyano, un sulfonamido, un alkyle inférieur, -OR⁵, -C(O)OR⁵, un perfluoroalkyle inférieur, un thioalkyle inférieur, un perfluorothioalkyle inférieur, un sulfonylalkyle inférieur, un perfluorosulfonylalkyle inférieur ou un sulfinylalkyl inférieur ;
R³ est un cycloalkyle ayant de 3 à 7 atomes de carbone ;
R⁴ est -C (O) NHR⁴⁰ ; ou un cycle hétéroaromatique à cinq ou six membres n'étant pas substitué ou monosubstitué, lié par un atome de carbone du cycle au groupement amine représenté, lequel cycle hétéroaromatique à cinq ou six membres contient de 1 à 3 hétéroatomes choisis parmi le soufre, l'oxygène ou l'azote, avec un hétéroatome étant l'azote lequel est adjacent à l'atome de carbone de liaison au cycle ; ledit cycle hétéroaromatique monosubstitué étant monosubstitué à une position sur un atome de carbone du cycle autre que celui adjacent audit atome de carbone de liaison, avec un substituant choisi dans le groupe constitué par un alkyle inférieur, un halogène, un nitro, un cyano, -(CH₂)ₙ-OR⁶, -(CH₂)ₙ-C(O) OR⁷, -(CH₂)ₙ-C (O) NHR⁶, -C(O)-C(O)OR⁸, -(CH₂)ₙ-NHR⁶ ;
R⁴⁰ est un hydrogène, un alkyle inférieur, un alcényle inférieur, un hydroxyalkyle inférieur, un haloalkyle inférieur, -(CH₂)ₙ-C(O)OR⁵ ou -C(O)-(CH₂)ₙ-C(O)OR⁶ ;
R⁵ est un hydrogène, un alkyle inférieur ou un perfluoroalkyle inférieur ;
R⁶, R⁷ et R⁸ sont indépendamment un hydrogène ou un alkyle inférieur ; et
n vaut 0, 1, 2, 3 ou 4 ;
un "alkyle inférieur" et " un alcoxy inférieur " indiquent des groupements avec 1 à 7 atomes de carbone ; et
un "alcényle inférieur» indique des groupements avec 2 à 6 atomes de carbone ; ou un sel pharmaceutiquement acceptable de celui-ci.

2. Composé selon la revendication 1, dans lequel
R¹ et R² sont indépendamment un hydrogène, un halogène, un amino, un hydroxyamino, un cyano, un nitro, un alkyle inférieur, -OR⁵, -C(O)OR⁵, un perfluoroalkyle inférieur, un thioalkyle inférieur, un perfluorothioalkyle inférieur, un sulfonylalkyle inférieur, un perfluorosulfonylalkyle inférieur, un sulfinylalkyle inférieur, ou un sulfonamido ;
R⁴ est un cycle hétéroaromatique à cinq ou six membres n'étant pas substitué ou monosubstitué, lié par un atome de carbone du cycle au groupement amine représenté, lequel cycle hétéroaromatique à cinq ou six membres contient de 1 à 3 hétéroatomes choisis parmi le soufre, l'oxygène ou l'azote, avec un hétéroatome étant l'azote, qui est adjacent à l'atome de carbone de liaison du cycle ; ledit cycle hétéroaromatique monosubstitué étant monosubstitué à une position sur un atome de carbone du cycle autre que celui adjacent audit atome de carbone de liaison, avec un substituant choisi dans le groupe constitué par un alkyle inférieur, un halogène, un nitro, un cyano, - (CH₂)ₙ-OR⁶, -(CH₂)ₙ-C(O)OR⁷, -(CH₂)ₙ-C(O)NHR⁶, -C(O)-C(O)OR⁸ ou -(CH₂)ₙ-NHR⁶ ;
n vaut 0, 1, 2, 3 ou 4 ;
R⁵ est un hydrogène, un alkyle inférieur, ou un perfluoroalkyle inférieur ; et
R⁶, R⁷ et R⁸ sont indépendamment un hydrogène ou un alkyle inférieur ;
ou un sel pharmaceutiquement acceptable de celui-ci.

3. Composé selon l'une quelconque des revendications 1 à 2, dans laquelle R⁴ est un cycle hétéroaromatique à cinq ou six membres n'étant pas substitué ou monosubstitué choisi dans le groupe constitué du thiazolyle, du pyridinyle, de l'imidazolyle, de l'isoxazolyle, de l'oxazolyle, du pyridazinyle, du pyrimidinyle ou du thiadiazolyle.

4. Composé selon l'une quelconque des revendications 1 à 3, dans lequel R⁴ est un thiazolyle non substitué, un pyridinyle non substitué ou un pyridinyle substitué par un halogène, un alkyle inférieur, un hydroxyalkyle inférieur ou -C(O)OR⁵, dans lequel R⁵ est un alkyle inférieur.

5. Composé selon la revendication 1, dans lequel
R¹ et R² sont indépendamment un hydrogène, un halogène, un amino, un nitro, un cyano, un sulfonamido, un alkyle inférieur, un perfluoroalkyle inférieur, un thioalkyle inférieur, un perfluorothioalkyle inférieur, un sulfonylalkyle inférieur ou un perfluorosulfonylalkyle inférieur ;
R⁴ est C(O)NHR⁴⁰ ;
R⁴⁰ est un hydrogène, un alkyle inférieur, un alcényle inférieur, un hydroxyalkyle inférieur, un haloalkyle inférieur, -(CH₂)ₙ-C(O)OR⁵ ou -C(O)-(CH₂)ₙ-C(O)OR⁶ ;
R⁵ et R⁶ sont un hydrogène ou un alkyle inférieur ; et
n vaut 0, 1, 2, 3 ou 4 ;
ou un sel pharmaceutiquement acceptable de celui-ci.

6. Composé selon l'une quelconque des revendication 1 ou 5, dans lequel R⁴ est -C(O)NHR⁴⁰ et R⁴⁰ est un alkyle inférieur ou un alcényle inférieur.

7. Composé selon l'une quelconque des revendications 1 à 6, dans lequel R¹ est un hydrogène, un halogène, un nitro ou un cyano.

8. Composé selon l'une quelconque des revendications 1 à 7, dans lequel R¹ est un hydrogène ou un halogène.

9. Composé selon l'une quelconque des revendications 1 à 8, dans lequel R² est un hydrogène, un sulfonylalkyle inférieur, un perfluoroalkyle inférieur, un perfluorosulfonylalkyle inférieur, un halogène ou -OR⁵, dans lequel R⁵ est un perfluoroalkyle inférieur.

10. Composé selon l'une quelconque des revendications 1 à 9, dans lequel R² est un halogène ou un sulfonylalkyle inférieur.

11. Composé selon l'une quelconque des revendications 1 à 10, dans lequel l'amide est dans la configuration R au carbone asymétrique représenté.

12. Composé selon l'une quelconque des revendications 1 à 11, dans laquelle R³ est un cyclopentyle, un cyclohexyle ou un cycloheptyle.

13. Composé selon l'une quelconque des revendications 1 à 12, dans laquelle R³ est un cyclopentyle.

14. Composé selon l'une quelconque des revendications 1 à 13, choisi dans le groupe constitué par :
la 2-(3-chlorophényl)-3-cyclopentyl-N-thiazol-2-ylpropionamide,
la 2-(4-bromophényl)-3-cyclopentyl-N-thiazol-2-ylpropionamide,
la 2-(4-chlorophényl)-3-cyclopentyl-N-thiazol-2-ylpropionamide,
la 3-cyclopentyl-2-(3,4-dichlorophényl)-N-thiazol-2-ylpropionamide,
la 3-cyclopentyl-N-thiazol-2-yl-2-(4-trifluorométhylphényl) propionamide,
la 3-cyclopentyl-2-(3-fluoro-4-trifluorométhylphényl)-N-thiazol-2-ylpropionamide,
la 3-cyclopentyl-2-(4-fluoro-3-trifluorométhylphényl)-N-thiazol-2-ylpropionamide,
la 3-cyclopentyl-N-thiazol-2-yl-2-(3-trifluorométhylphényl) propionamide,
la 3-cyclopentyl-2(R)-(3,4-dichlorophényl)-N-thiazol-2-ylpropionamide,
la 3-cyclopentyl-2-(4-nitrophényl)-N-thiazol-2-ylpropionamide,
la 2-(4-aminophényl)-3-cyclopentyl-N-thiazol-2-ylpropionamide,
la 2-(3-aminophényl)-3-cyclopentyl-N-thiazol-2-ylpropionamide,
la 2-(4-chloro-3-nitrophényl)-3-cyclopentyl-N-thiazol-2-ylpropionamide,
la 2-(4-cyanophényl)-3-cyclopentyl-N-thiazol-2-ylpropionamide,
la 3-cyclopentyl-N-thiazol-2-yl-2-(4-trifluorométhylsulfanylphényl) propionamide,
la 3-cyclopentyl-2-(4-méthylsulfanyl-phenyl)-N-thiazol-2-ylpropionamide,
la 3-cyclopentyl-2-(4-méthylsulfanyl-3-trifluorométhylphényl)-N-thiazol-2-ylpropionamide,
la 3-cyclopentyl-2-(4-méthane sulfinylphényl)-N-thiazol-2-ylpropionamide,
la 3-cyclopentyl-2-(4-méthane sulfonylphényl)-N-thiazol-2-ylpropionamide,
la 3-cyclopentyl-2-(4-méthane sulfonyl-3-nitrophényl)-N-thiazol-2-ylpropionamide,
la 2-(3-amino-4-méthane sulfonylphényl)-3-cyclopentyl-N-thiazol-2-ylpropionamide,
la 3-cyclopentyl-2-(3-hydroxyamino-4-méthane sulfonylphényl)-N-thiazol-2-ylpropionamide,
la 2-(3-cyano-4-méthane sulfonylphényl)-3-cyclopentyl-N-thiazol-2-ylpropionamide,
la 3-cyclopentyl-2-(4-éthane sulfonylphényl)-N-thiazol-2-ylpropionamide,
la 2-(3,4-bis-méthane sulfonylphényl)-3-cyclopentyl-N-thiazol-2-ylpropionamide,
la 3-cyclopentyl-2-(4-sulfamoylphényl)-N-thiazol-2-ylpropionamide,
la 2-[4-(butane-1-sulfonyl) phényl]-3-cyclopentyl-N-thiazol-2-ylpropionamide,
la 3-cyclopentyl-2-[4-(propane-1-sulfonyl) phényl]-N-thiazol-2-ylpropionamide,
la 3-cyclopentyl-2-(4-méthane sulfonyl-3-trifluorométhylphényl)-N-thiazol-2-ylpropionamide,
la 2(R)-(3-chloro-4-méthane sulfonylphényl)-3-cyclopentyl-N-thiazol-2-ylpropionamide,
la 2-[3-chloro-4-méthane sulfonylphényl]-3-cyclopentyl-N-thiazol-2-ylpropionamide,
la 2-(3-bromo-4-méthane sulfonylphényl)-3-cyclopentyl-N-thiazol-2-ylpropionamide,
la 3-cyclopentyl-N-thiazol-2-yl-2-(4-trifluorométhane sulfonylphényl) propionamide,
la 3-cyclopentyl-N-thiazol-2-yl-2-(3-trifluorométhane sulfonylphényl) propionamide,
la 3-cyclopentyl-N-thiazol-2-yl-2-(4-trifluorométhoxyphényl) propionamide,
la 3-cyclopentyl-2-(3-méthoxyphényl)-N-thiazol-2-ylpropionamide,
la 3-cyclopentyl-2-(3-hydroxyphényl)-N-thiazol-2-ylpropionamide,
la 3-cyclopentyl-2-(3,4-diméthoxyphényl)-N-thiazol-2-ylpropionamide,
la 3-cyclopentyl-2-(3,4-dihydroxyphényl)-N-thiazol-2-ylpropionamide,
la 3-cyclopentyl-2-(4-méthoxyphényl)-N-thiazol-2-ylpropionamide,
la 3-cyclopentyl-2-(4-hydroxyphényl)-N-thiazol-2-ylpropionamide,
l'ester de méthyle d'acide 4-[2-cyclopentyl-1-(thiazol-2-ylcarbamoyl) éthyl] benzoïque,
la 3-cyclopentyl-2-(3-fluoro-4-méthoxyphényl)-N-thiazol-2-ylpropionamide,
la 3-cyclopentyl-2-(3-fluoro-4-hydroxyphényl)-N-thiazol-2-ylpropionamide,
la 3-cyclopentyl-2-(3,4-dichlorophényl)-N-(5-hydroxyméthyl-thiazol-2-yl) propionamide,
la 3-cyclopentyl-2-(3,4-dichlorophényl)-N-[4-(2-hydroxyéthyl) thiazol-2-yl] propionamide,
la 2-(4-chlorophényl)-3-cyclopentyl-N-(5-hydroxyméthylthiazol-2-yl) propionamide,
la 3-cyclopentyl-2-(3,4-dichlorophényl)-N-(4-hydroxyméthylthiazol-2-yl) propionamide,
la 3-cyclopentyl-N-(4-hydroxyméthylthiazol-2-yl)-2-(4-méthane sulfonylphényl) propionamide,
la 3-cyclopentyl-N-[4-(2-hydroxyéthyl) thiazol-2-yl]-2-(4-méthane sulfonylphényl) propionamide,
la 3-cyclopentyl-2-(3,4-dichlorophényl)-N-(4-méthylthiazol-2-yl) propionamide,
la 3-cyclopentyl-2-(3,4-dichlorophényl)-N-(5-méthylthiazol-2-yl) propionamide,
l'ester d'éthyle d'acide {2-[2-(3-chlorophényl)-3-cyclopentylpropionylamino] thiazol-4-yl} acétique,
l'ester de méthyle d'acide {2-[2-(3-chlorophényl)-3-cyclopentylpropionylamino] thiazol-4-yl] acétique,
l'ester de méthyle d'acide 2-[2-(3-chlorophényl)-3-cyclopentylpropionylamino] thiazole-4-carboxylique,
l'ester d'éthyle d'acide 2-[2-(3-chlorophényl)-3-cyclopentylpropionylamino] thiazole-4-carboxylique,
l'ester d'éthyle d'acide {2-[2-(4-chlorophényl)-3-cyclopentylpropionylamino] thiazol-4-yl} acétique,
l'ester de méthyle d'acide 2-[2-(4-chlorophényl)-3-cyclopentylpropionylamino] thiazole-4-carboxylique,
l'ester d'éthyle d'acide 2-[2-(4-chlorophényl)-3-cyclopentylpropionylamino] thiazole-4-carboxylique,
l'ester de méthyle d'acide (2-[2-(4-chlorophényl)-3-cyclopentylpropionylamino] thiazol-4-yl} acétique,
l'acide {2-[3-cyclopentyl-2-(3,4-dichlorophényl) propionylamino] thiazol-4-yl} acétique,
l'ester d'éthyle d'acide (2-[3-cyclopentyl-2-(3,4-dichlorophényl) propionylamino] thiazol-4-yl) acétique,
l'acide 2-[3-cyclopentyl-2-(3,4-dichlorophényl) propionylamino] thiazole-5-carboxylique,
l'acide 2-[3-cyclopentyl-2-(3,4-dichlorophenyl) propionylamino] thiazole-4-carboxylique,
l'ester de méthyle d'acide (2-[3-cyclopentyl-2-(3,4-dichlorophényl) propionylamino] thiazol-4-yl} acétique,
l'ester de méthyle d'acide (2R)-2-[3-cyclopentyl-2-(3,4-dichlorophényl) propionylamino] thiazole-4-carboxylique,
l'ester de méthyle d'acide 2-[3-cyclopentyl-2-(3,4-dichlorophényl) propionylamino] thiazole-5-carboxylique,
l'ester d'éthyle d'acide 2-[3-cyclopentyl-2-(3,4-dichlorophényl) propionylamino] thiazole-5-carboxylique,
l'ester d'éthyle d'acide (2-[3-cyclopentyl-2-(4-nitrophényl) propionylamino] thiazol-4-yl} acétique,
l'ester de méthyle d'acide {2-[3-cyclopentyl-2-(4-nitrophényl) propionylamino] thiazol-4-yl} acétique,
l'ester de méthyle d'acide 2-[3-cyclopentyl-2-(4-nitrophényl) propionylamino] thiazole-4-carboxylique,
l'ester d'éthyle d'acide 2-[3-cyclopentyl-2-(4-nitrophényl) propionylamino] thiazole-4-carboxylique,
l'ester de méthyle d'acide {2-[2-(4-aminophényl)-3-cyclopentylpropionylamino] thiazol-4-yl} acétique,
l'ester de méthyle d'acide 2-[2-(4-aminophényl)-3-cyclopentylpropionylamino] thiazole-4-carboxylique,
l'ester d'éthyle d'acide {2-[3-cyclopentyl-2-(4-méthane sulfonylphényl) propionylamino] thiazolé-4-carboxylique,
l'ester de méthyle d'acide {2-[3-cyclopentyl-2-(4-méthane sulfonylphényl) propionylamino] thiazol-4-yl}-4-acétique,
l'ester de méthyle d'acide {2-[3-cyclopentyl-2-(4-méthane sulfonyl-3-trifluorométhylphényl) propionylamino] thiazol-4-yl} acétique,
l'ester de méthyle d'acide 2-[3-cyclopentyl-2-(4-méthane sulfonyl-3-trifluorométhylphényl) propionylamino] thiazole-4-carboxylique,
l'ester d'éthyle d'acide (2-[3-cyclopentyl-2-(4-nitrophényl) propionylamino] thiazol-4-yl} oxoacétique,
l'ester d'éthyle d'acide (2-[3-cyclopentyl-2-(3,4-dichlorophényl) propionylamino] thiazol-5-yl} oxoacétique,
l'ester d'éthyle d'acide {2-[3-cyclopentyl-2-(4-fluoro-3-trifluorométhylphényl) propionylamino] thiazol-4-yl} oxoacétique,
l'ester d'éthyle d'acide {2-[2-(3-chloro-4-méthane sulfonylphényl)-3-cyclopentylpropionylamino] thiazol-4-yl} oxoacétique,
la 3-cyclopentyl-2-(3,4-dichlorophényl)-N-(5-nitrothiazol-2-yl) propionamide,
la 3-cyclopentyl-2-(3-fluoro-4-trifluorométhylphényl)-N-pyridin-2-ylpropionamide, la 3-cyclopentyl-2-(3,4-dichlorophényl)-N-pyridin-2-ylpropionamide,
la 3-cyclopentyl-N-pyridin-2-yl-2-(4-trifluorométhylphényl)-2-propionamide,
la 3-cyclopentyl-N-thiazol-2-yl-2-(3-trifluorométhylphényl) propionamide,
la 2-(3-chlorophényl)-3-cyclopentyl-N-pyridin-2-ylpropionamide,
la 2-(4-aminophényl)-3-cyclopentyl-N-pyridin-2-ylpropionamide,
la 2-(4-cyanophényl)-3-cyclopentyl-N-pyridin-2-ylpropionamide,
la 2-(4-chlorophényl)-3-cyclopentyl-N-pyridin-2-ylpropionamide,
la 2-(4-chloro-3-nitrophényl)-3-cyclopentyl-N-pyridin-2-yl-propionamide,
la 3-cyclopentyl-2-(4-nitrophényl)-N-pyridin-2-ylpropionamide,
la 2-(4-cyanophényl)-3-cyclopentyl-N-pyridin-2-ylpropionamide,
la 3-cyclopentyl-2-(4-méthylsulfanylphényl)-N-pyridin-2-ylpropionamide,
la 3-cyclopentyl-N-pyridin-2-yl-2-(4-trifluorométhylsulfanylphényl) propionamide,
la 3-cyclopentyl-2-(4-méthane sulfonyl-3-nitrophényl)-N-pyridin-2-ylpropionamide,
la 3-cyclopentyl-2-(4-méthane sulfonylphényl)-N-pyridin-2-ylpropionamide,
la 2-(3-bromo-4-méthane sulfonylphényl)-3-cyclopentyl-N-pyridin-2-ylpropionamide,
la 2-(3-cyano-4-méthane sulfonylphényl)-3-cyclopentyl-N-pyridin-2-ylpropionamide,
la 3-cyclopentyl-2-(4-éthane sulfonylphényl)-N-pyridin-2-ylpropionamide,
la 2-(3,4-bis-méthane sulfonylphényl)-3-cyclopentyl-N-pyridin-2-ylpropionamide,
la 2-(3-chloro-4-méthane sulfonylphényl)-3-cyclopentyl-N-pyridin-2-ylpropionamide,
la 3-cyclopentyl-2-(4-méthane sulfonyl-3-trifluorométhylphényl)-N-pyridin-2-ylpropionamide,
la 3-cyclopentyl-N-pyridin-2-yl-2-(4-trifluorométhane sulfonylphényl) propionamide,
la 3-cyclopentyl-2-(3,4-dichlorophényl)-N-(5-carboxyméthylpyridin)-2-ylpropionamide,
l'ester de méthyle d'acide 6-[3-cyclopentyl-2(R)-(3,4-dichlorophényl) propionylamino] nicotinique,
l'acide 6-[2-(4-chlorophényl)-3-cyclopentylpropionylamino] nicotinique,
l'ester de méthyle d'acide 6-[2-(3-chlorophényl)-3-cyclopentylpropionylamino] nicotinique,
l'ester de méthyle d'acide 6-[2-(4-chlorophényl)-3-cyclopentylpropionylamino] nicotinique,
l'ester de méthyle d'acide 6-[3-cyclopentyl-2-(4-nitrophényl) propionylamino] nicotinique,
l'ester de méthyle d'acide 6-[2-(4-aminophényl)-3-cyclopentylpropionylamino] nicotinique,
l'acide 6-[2-(3-chlorophényl)-3-cyclopentylpropionylamino] nicotinique,
l'acide 6-[2-(4-cyanophényl)-3-cyclopentylpropionylamino] nicotinique,
l'ester de méthyle d'acide 6-[3-cyclopentyl-2-(4-trifluorométhane sulfonylphényl) propionylamino] nicotinique,
l'acide 6-[3-cyclopentyl-2-(4-trifluorométhane sulfonylphényl) propionylamino] nicotinique,
l'ester de méthyle d'acide 6-[3-cyclopentyl-2-(4-méthane sulfonylphényl) propionylamino] nicotinique,
la 3-cyclopentyl-2(R)-(3,4-dichlorophényl)-N-(5-hydroxyméthylpyridin-2-yl) propionamide,
la 3-cyclopentyl-2-(3,4-dichlorophényl)-N-(5-hydroxypyridin-2-yl) propionamide,
la 2-(4-chlorophényl)-3-cyclopentyl-N-(5-hydroxyméthylpyridin-2-yl) propionamide,
la 3-cyclopentyl-N-(5-hydroxyméthylpyridin-2-yl)-2-(4-méthane sulfonylphényl) propionamide,
la N-(5-chloropyridin-2-yl)-3-cyclopentyl-2-(3,4-dichlorophényl) propionamide,
la 3-cyclopentyl-2-(3,4-dichlorophényl)-N-(5-bromopyridin)-2-ylpropionamide,
la 3-cyclopentyl-2(R)-(3,4-dichlorophényl)-N-(5-trifluorométhylpyridin-2-yl) propionamide,
la N-(5-bromopyridin-2-yl)-3-cyclopentyl-2(R)-(3,4-dichlorophényl) propionamide,
la N-(5-chloropyridin-2-yl)-3-cyclopentyl-2-(4-trifluorométhane sulfonylphényl) propionamide,
la N-(5-bromopyridin-2-yl)-3-cyclopentyl-2-(4-trifluorométhane sulfonylphényl) propionamide,
la N-(5-bromopyridin-2-yl)-3-cyclopentyl-2-(4-méthane sulfonyl-3-nitrophényl) propionamide,
la 2-(3-bromo-4-méthane sulfonylphényl)-N-(5-bromopyridin-2-yl)-3-cyclopentylpropionamide,
la N-(5-bromopyridin-2-yl)-2-(3-chloro-4-méthane sulfonylphényl)-3-cyclopentylpropionamide,
la 2-(3-chloro-4-méthane sulfonylphényl)-3-cyclopentyl-N-(5-trifluorométhylpyridin-2-yl) propionamide,
la N-(5-chloropyridin-2-yl)-2-(3-chloro-4-méthane sulfonylphényl)-3-cyclopentylpropionamide,
la 3-cyclopentyl-2-(3,4-dichlorophényl)-N-(5-nitropyridin)-2-ylpropionamide,
la 3-cyclopentyl-2-(3,4-dichlorophényl)-N-(5-méthylpyridin)-2-ylpropionamide,
la 3-cyclopentyl-2-(3,4-dichlorophényl)-N-(4-méthylpyridin)-2-ylpropionamide,
la 3-cyclopentyl-2-(3,4-dichlorophényl)-N-(6-méthylpyridin)-2-ylpropionamide,
la 3-cyclopentyl-N-(5-méthylpyridin-2-yl)-2-(4-trifluorométhane sulfonylphényl) propionamide,
la 3-cyclopentyl-2-(4-fluoro-3-trifluorométhylphényl)-N-(5-méthylpyridin-2-yl) propionamide,
la 6-[3-cyclopentyl-2-(3,4-dichlorophényl) propionylamino]-N-méthylnicotinamide,
la 3-cyclopentyl-2-(3,4-dichlorophényl)-N-(1H-imidazol-2-yl) propionamide,
la 3-cyclopentyl-2-(3,4-dichlorophényl)-N-(5-méthylisoxazol-3-yl) propionamide,
la 3-cyclopentyl-2-(3,4-dichlorophényl)-N-oxazol-2-ylpropionamide,
la 3-cyclopentyl-2-(3,4-dichlorophényl)-N-pyridazin-3-ylpropionamide,
la 3-cyclopentyl-2-(3,4-dichlorophényl)-N-pyrimidin-2-ylpropionamide,
la 3-cyclopentyl-2-(3,4-dichlorophényl)-N-pyrimidin-6-ylpropionamide,
la 3-cyclopentyl-2-(4-nitrophényl)-N-pyrimidin-4-ylpropionamide,
la 3-cyclopentyl-2-(3,4-dichlorophényl)-N-[1,3,4] thiadiazol-2-ylpropionamide,
la 2-[4-méthane sulfonylphényl]-3-cyclohexyl-N-thiazol-2-ylpropionamide, et
la 2-[4-méthane sulfonylphényl]-3-cycloheptyl-N-thiazol-2-ylpropionamide.

15. Composé selon l'une quelconque des revendications 1 à 13, choisi dans le groupe constitué par :
la 1-(3-cyclopentyl-2-phénylpropionyl)-3-méthylurée,
la 1 -[2-(3-chlorophényl)-3 cyclopentylpropionyl]-3-méthylurée,
la 1-[2-(4-chlorophényl)-3 -cyclopentylpropionyl]-3-méthylurée,
la 1-[2-(4-cyanophényl)-3-cyclopentylpropionyl]-3-méthylurée,
la 1-[2-(4-bromophényl)-3-cyclopentylpropionyl]-3-méthylurée,
la [3-cyclopentyl-2-(3,4-dichlorophényl) propionyl] urée,
la 1-[3-cyclopentyl-2-(3,4-dichlorophényl) propionyl]-3-méthylurée,
la 1-[3-cyclopentyl-2(R)-(3,4-dichlorophényl) propionyl]-3-éthylurée,
la 1-allyl-3-[3-cyclopentyl-2-(3,4-dichlorophényl) propionyl] urée,
la 1-allyl-3-[3-cyclopentyl-2(R)-(3,4-dichlorophényl) propionyl] urée,
la 1-[3-cyclopentyl-2-(3,4-dichlorophényl) propionyl]-3-éthylurée,
la 1-[3-cyclopentyl-2(R)-(3,4-dichlorophényl) propionyl]-3-méthylurée,
la 1-[3-cyclopentyl-2-(3,4-dichlorophényl) propionyl]-3-isopropylurée,
la 1-[3-cyclopentyl-2-(3,4-dichlorophényl) propionyl]-3-propylurée,
la 1-[3-cyclopentyl-2-(3,4-difluorophényl) propionyl]-3-méthylurée,
la 1-[2-(4-chloro-3-nitrophényl)-3-cyclopentylpropionyl]-3-méthylurée,
la 1-[3-cyclopentyl-2-(4-nitrophényl) propionyl]-3-méthylurée,
la 1-[3-cyclopentyl-2-(4-trifluorométhylsulfanylphényl) propionyl]-3-méthylurée,
la 1-[3-cyclopentyl-2-(4-méthylsulfanylphényl) propionyl]-3-méthylurée,
la 1-[3-cyclopentyl-2-(4-trifluorométhane sulfonylphényl) propionyl]-3-méthylurée,
la 1-[3-cyclopentyl-2-(3-trifluorométhane sulfonylphényl) propionyl]-3-méthylurée,
la 1-[3-cyclopentyl-2-(4-méthane sulfonylphényl) propionyl]-3-méthylurée,
la 1-{2-[4-(butane-1-sulfonyl) phényl]-3-cyclopentylpropionyl}-3-méthylurée,
la 1-[3-cyclopentyl-2-(4-éthane sulfonylphényl) propionyl]-3-méthylurée,
la 1-[2-(3,4-bis-méthane sulfonylphényl)-3-cyclopentylpropionyl]-3-méthylurée,
la 1-[2-(3-bromo-4-méthane sulfonylphényl)-3-cyclopentylpropionyl]-3-méthylurée,
la 1-[3-cyclopentyl-2-(3-fluoro-4-méthane sulfonylphényl) propionyl]-3-méthylurée,
la 1-[2-(3-chloro-4-méthane sulfonylphényl)-3-cyclopentylpropionyl]-3-méthylurée,
la 1-[2(R)-(3-chloro-4-méthane sulfonylphényl)-3-cyclopentylpropionyl]-3-méthylurée,
la 1-[2-(3-chloro-4-méthane sulfonylphényl)-3-cyclopentylpropionyl]-3-éthylurée,
la 1-[2-(3-cyano-4-méthane sulfonylphényl)-3-cyclopentylpropionyl]-3-méthylurée,
la 1-[3-cyclopentyl-2-(4-méthane sulfonyl-3-trifluorométhylphényl) propionyl]-3-méthylurée,
la 1-[3-cyclopentyl-2-(4-fluoro-3-trifluorométhylphényl) propionyl]-3-méthylurée,
la 1-[3-cyclopentyl-2-(3-fluoro-4-trifluorométhylphényl) propionyl]-3-méthylurée,
la 1-[3-cyclopentyl-2-(4-méthane sulfonyl-3-nitrophényl) propionyl]-3-méthylurée,
la 3-cyclopropyl-2-(3,4-dichlorophényl) propionyl] urée,
la [3-cyclobutyl-2-(3,4-dichlorophényl) propionyl] urée,
la 3-[cyclohexyl-2-(3,4-dichlorophényl) propionyl] urée,
la [3-cyclohexyl-2-(3,4-dichlorophényl) propionyl]-3-méthylurée,
la [3-cycloheptyl-2-(3,4-dichlorophényl) propionyl] urée,
l'ester d'éthyle d'acide 3-{3-[3-cyclopentyl-2-(3,4-dichlorophényl) propionyl] uréido} propionique,
l'ester d'éthyle d'acide {3-[3-cyclopentyl-2-(3,4-dichlorophényl) propionyl] uréido} acétique,
l'ester de méthyle d'acide (3-[3-cyclopentyl-2-(3,4-dichlorophényl) propionyl} uréido) acétique,
l'ester de méthyle d'acide 3-{3-[3-cyclopentyl-2-(3,4-dichlorophényl) propionyl] uréido} propionique,
l'ester d'éthyle d'acide (3-[3-cyclopentyl-2(R)-(3,4-dichlorophényl) propionyl] uréido} acétique,
l'ester d'éthyle d'acide 3-{3-[3-cyclopentyl-2-(3,4-dichlorophényl) propionyl] uréido}-3-oxopropionique,
la 1-[3-cyclopentyl-2-(3,4-dichlorophényl) propionyl]-3-(2-hydroxyéthyl) urée,
la 1-[3-cyclopentyl-2-(3,4-dichlorophényl) propionyl]-3- (2-hydroxypropyl) urée,
la 1-[3-cyclopentyl-2-(3,4-dichlorophényl) propionyl]-3-(3-hydroxypropyl) urée,
la 1-[3-cyclopentyl-2(R)-(3,4-dichlorophényl) propionyl]-3-(2-hydroxypropyl) urée,
la 1-(2-chloroéthyl)-3-[3-cyclopentyl-2-(3,4-dichlorophényl) propionyl] urée, et
la 1- [3-cyclopentyl-2 (R) - (3, 4-dichlorophényl) propionyl]-3-(3-hydroxypropyl) urée.

16. Composé selon l'une quelconque des revendications 1 à 14, choisi dans le groupe constitué par :
la 3-cyclopentyl-2-(4-méthane sulfonylphényl)-N-thiazol-2-ylpropionamide,
la 3-cyclopentyl-N-thiazol-2-yl-2-(4-trifluorométhoxyphényl) propionamide,
la 3-cyclopentyl-N-thiazol-2-yl-2-(4-trifluorométhane sulfonylphényl) propionamide,
la 3-cyclopentyl-2(R)-(3,4-dichlorophényl)-N-pyridin-2-yl-propionamide,
l'ester de méthyle d'acide 6-[3-cyclopentyl-2(R)-(3,4-dichlorophényl) propionylamino] nicotinique,
la N-(5-chloropyridin-2-yl)-3-cyclopentyl-2(R)-(3,4-dichlorophényl) propionamide,
la 3-cyclopentyl-N-pyridin-2-yl-2-(4-trifluorométhane sulfonylphényl) propionamide,
la 3-cyclopentyl-N-(5-méthylpyridin-2-yl)-2-(4-trifluorométhane sulfonylphényl) propionamide,
la 3-cyclopentyl-2(R)-(3,4-dichlorophényl)-N-(5-hydroxyméthylpyridin-2-yl) propionamide,
l'ester de méthyle d'acide 6-[3-cyclopentyl-2-(4-trifluorométhane sulfonylphényl) propionylamino] nicotinique,
la 3-cyclopentyl-2-(3-fluoro-4-trifluorométhylphényl)-N-pyridin-2-ylpropionamide,
la 3-cyclopentyl-2-(4-méthane sulfonyl-3-nitrophényl)-N-pyridin-2-ylpropionamide,
la 2-(3-bromo-4-méthane sulfonylphényl)-3-cyclopentyl-N-pyridin-2-ylpropionamide,
la 2-(3-cyano-4-méthane sulfonylphényl)-3-cyclopentyl-N-pyridin-2-ylpropionamide,
la 2-(4-chloro-3-nitrophényl)-3-cyclopentyl-N-pyridin-2-ylpropionamide),
la 2-(3-chloro-4-méthane sulfonylphényl)-3-cyclopentyl-N-pyridin-2-ylpropionamide,
la N-(5-bromopyridin-2-yl)-2-(3-chloro-9-méthane sulfonylphényl)-3-cyclopentylpropionamide,
la 2-[3-chloro-4-méthane sulfonylphényl]-3-cyclopentyl-N-thiazol-2-ylpropionamide,
la (2R)-3-cyclopentyl-2-(4-méthane sulfonylphényl)-N-thiazol-2-ylpropionamide,
la 2-(3-bromo-4-méthane sulfonylphényl)-3-cyclopentyl-N-thiazol-2-ylpropionamide,
la 2-(3-cyano-4-méthane sulfonylphényl)-3-cyclopentyl-N-thiazol-2-ylpropionamide,
la 3-cyclopentyl-2-(4-éthane sulfonylphényl)-N-thiazol-2-ylpropionamide,
la 3-cyclopentyl-2-(4-méthane sulfonyl-3-trifluorométhylphényl)-N-thiazol-2-ylpropionamide, et
la N-(5-bromopyridin-2-yl)-2(R)-(3-chloro-4-méthane sulfonylphényl)-3-cyclopentyl propionamide.

17. Composé selon l'une quelconque des revendications 1 à 13 et 15, choisi dans le groupe constitué par :
la 1-[3-cyclopentyl-2(R)-(3,9-dichlorophényl) propionyl]-3-éthylurée,
la 1-[3-cyclopentyl-2(R)-(3,4-dichlorophényl) propionyl]-3-méthylurée,
la 1-[3-cyclopentyl-2-(3,4-dichlorophényl) propionyl]-3-méthylurée,
la 1-[3-cyclopentyl-2-(4-méthane sulfonylphényl) propionyl]-3-méthylurée,
la 1-allyl-3-[3-cyclopentyl-2(R)-(3,4-dichlorophényl) propionyl] urée,
la 1-[2-(3-chloro-4-méthane sulfonylphényl)-3-cyclopentylpropionyl]-3-méthylurée,
la 1-[2(R)-(3-chloro-4-méthane sulfonylphényl)-3-cyclopentylpropionyl]-3-méthylurée et
la 1-[2-(3-bromo-4-méthane sulfonylphényl)-3-cyclopentylpropionyl]-3-méthylurée.

18. Composition pharmaceutique comprenant un composé selon l'une quelconque des revendications 1 à 17 et un vecteur et/ou un adjuvant pharmaceutiquement acceptable.

19. Utilisation d'un composé selon l'une quelconque des revendications 1 à 17, pour la préparation de médicaments contenant un composé selon l'une quelconque des revendications 1 à 17 pour le traitement ou la prophylaxie du diabète de type II.

20. Processus pour la préparation des composés de formule I selon l'une quelconque des revendications 1 à 17, 21 et 22, lequel processus comprend:
(a) la condensation d'un composé de formule XI: 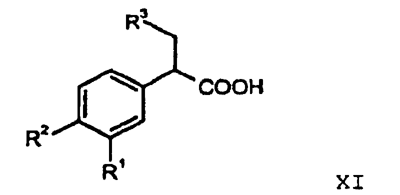 dans laquelle R¹, R² et R³ sont tels que définis dans la revendication 1 ; avec un composé de formule VIII:
R⁴²-NH₂ VIII
dans laquelle R⁴² est un à cycle hétéroaromatique à cinq ou six membres n'étant pas substitué ou monosubstitué, lié par un atome de carbone du cycle au groupement amine représenté, lequel cycle hétéroaromatique à cinq ou six membres contient de 1 à 3 hétéroatomes choisis parmi le soufre, l'oxygène ou l'azote, avec un hétéroatome étant l'azote qui est adjacent à l'atome de carbone du cycle, de liaison ; ledit cycle hétéroaromatique monosubstitué étant monosubstitué à une position sur un atome de carbone du cycle autre que celui adjacent audit atome de carbone de liaison, avec un substituant choisi dans le groupe constitué par un alkyle inférieur, un halogène, un nitro, un cyano, - (CH₂)ₙ-OR⁶, -(CH₂)ₙ-C(O)OR⁷, -(CH₂)ₙ-C(O)NHR⁶, - C(O)-C(O)OR⁸ ou -(CH₂)ₙ-NHR⁶ ; R⁶, R⁷, R⁸ et n sont tels que définis selon la revendication 1 ; via une liaison peptidique pour produire un composé de formule I-d : 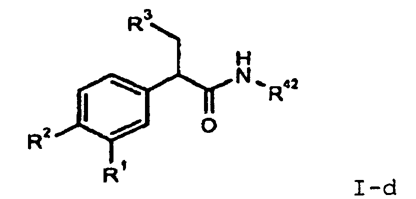 dans laquelle R¹, R², R³ et R⁴² sont tels que définis précédemment ;
facultativement suivi par la conversion de l'un
ou des deux des substituants R¹ et/ou R² en un autre substituant R¹ et/ou R² tels que définis selon la revendication 1.
(b) la réaction d'un composé de formule IX : 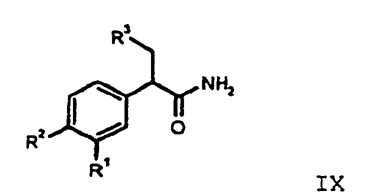 dans laquelle R¹, R² et R³ sont tels que définis selon la revendication 1 ; avec un composé de formule X :
R⁴¹-N=C=O X
dans laquelle R⁴¹ est un alkyle inférieur, un alcényle inférieur, un hydroxyalkyle inférieur, un haloalkyle inférieur ou -(CH₂)ₙ-C(O)OR⁵, dans laquelle R⁵ est un hydrogène ou un alkyle inférieur et n est tel que défini précédemment ; pour produire un composé de formule I-a : 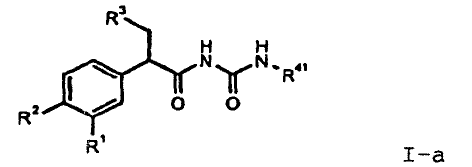 dans laquelle R¹, R², R³ et R⁴¹ sont tels que définis précédemment ;
facultativement suivi par la conversion de l'un ou des deux substituants R¹ et/ou R² en un autre substituant R¹ et/ou R² tels que définis selon la revendication 1.
(c) la réaction d'un composé de formule XI : 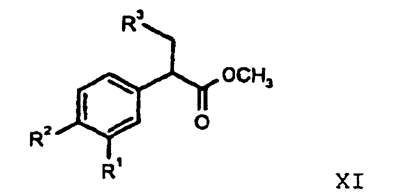 dans laquelle R¹, R² et R³ sont tels que définis selon la revendication 1 ; avec un composé de formule :
H₂NCONH₂
pour produire un composé de formule I-b : 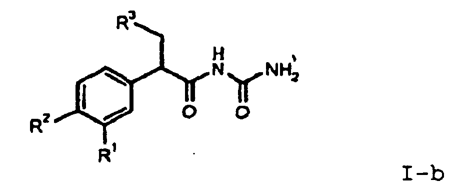 dans laquelle R¹, R² et R³ sont tels que définis précédemment ;
facultativement suivi par la conversion de l'un ou des deux substituants R¹ et/ou R² en un autre substituant R¹ et/ou R² tels que définis selon la revendication 1.
(d) la réaction d'un composé de formule I-b : 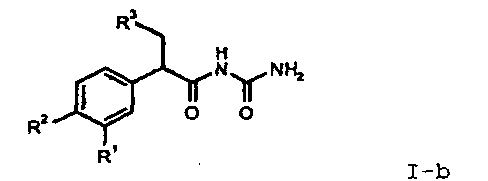
dans laquelle R¹, R² et R³ sont tels que définis selon la revendication 1 ; avec un composé de formule XIII :
ClC(O)-(CH₂)_{*n*}-C(O)OR⁶ XIII
dans laquelle R⁶ est un hydrogène ou un alkyle inférieur et n est tel que défini selon la revendication 1 ;
pour produire un composé de formule I-c : 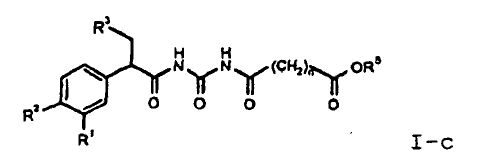 dans laquelle R¹, R², R³, R⁶ et n sont tels que définis précédemment ;
facultativement suivi par la conversion de l'un ou des deux substituants R¹ et/ou R² en un autre substituant R¹ et/ou R² tels que définis selon la revendication 1.

21. 3-cyclopentyl-2-(4-méthane sulfonylphényl)-N-thiazol-2-ylpropionamide.

22. 2R)-3-cyclopentyl-2-(4-méthane sulfonylphényl)-N-thiazol-2-ylpropionamide.
